# EUROPEAN PATENT APPLICATION

(11) **EP 1 195 383 A2**
(43) Date of publication of application: **10.04.2002**
(21) Application number: 01306639.4
(22) Date of filing: 02.08.2001
(51) Int. Cl.: C07K 14/72

(54) **Three-dimensional structure of bovine rhodopsin**

(30) Priority: 03.08.2000 JP 2000236288
(71) Applicant: RIKEN, Wako-shi, Saitama 351-0198 (JP); University of Washington, Seattle, WA 98195 (US)
(72) Inventor: Miyano, Masashi, c/o Riken Harima Institute, Sayou-gun, Hyogo 679-5148 (JP); Palczewski, Krzysztof, Seattle, WA 98195 (US); Kumasaka, Takashi, c/o Riken Harima Institute, Sayou-gun, Hyogo 679-5148 (JP); Hori, Tetsuya, c/o Riken Harima Institute, Sayou-gun, Hyogo 679-5148 (JP); Behnke, Craig A., Seattle, WA 98195 (US); Motoshima, Hiroyuki, c/o Riken Harima Institute, Sayou-gun, Hyogo 679-5148 (JP); Fox, Brain A., Seattle, WA 98195 (US); Le Trong, Isolde, Seattle, WA 98195 (US); Teller, David C., Seattle, WA 98195 (US); Okada, Tetsuji, c/o Riken Harima Institute, Sayou-gun, Hyogo 679-5148 (JP); Stenkamp, Ronald E., Seattle, WA 98195 (US); Yamamoto, Masaki, c/o Riken Harima Institute, Sayou-gun, Hyogo 679-5148 (JP)
(74) Representative: Harrison, David Christopher

(57) **Abstract**

The present invention discloses a three-dimensional structure of bovine rhodopsin. There is provided a G protein-coupled receptor selected from the group consisting of the following (a) and (b):
(a) a G protein-coupled receptor having three-dimensional structure I defined by the atomic coordinates as shown in Table 1;
(b) a G protein-coupled receptor having three-dimensional structure II defined by derived coordinates from the atomic coordinates as shown in Table 1, wherein the mean residual of the discrepancies between the positions of the α carbon atoms in the amino acid residues of seven helix sites H-I (36-64), H-II (71-100); H-III (107-139), H-IV (151-173), H-V (200-225), H-VI (247-277) and H-VII (286-306) in the amino acid sequence as shown in SEQ ID NO: 1 of the three-dimensional structure I and the positions of the corresponding α cabon atoms in the amino acid residues of the corresponding seven helix sites of the three-dimensional structure II is 1.5 Å or less when an image of the three-dimensional structure I obtained by computer-processing the atomic coordinates of Table 1 and an image of the three-dimensional structure II obtained by computer-processing the derived coordinates are superposed.

SEQUENCE LISTING

<110> RIKEN
      UNIVERSITY OF WASHINGTON

<120> THREE-DIMENSIONAL STRUCTURE OF BOVINE RHODOPSIN

<130> PH-1260

<140>
<141>

<150> JP2000-236288
<151> 2000-08-03

<160> 8

<170> PatentIn Ver. 2.0

<210> 1
<211> 348
<212> PRT
<213> Bos sp.

<400> 1
Met Asn Gly Thr Glu Gly Pro Asn Phe Tyr Val Pro Phe Ser Asn Lys
  1               5                  10                  15

Thr Gly Val Val Arg Ser Pro Phe Glu Ala Pro Gln Tyr Tyr Leu Ala

            20                  25                  30

Glu Pro Trp Gln Phe Ser Met Leu Ala Ala Tyr Met Phe Leu Leu Ile
        35                  40                  45

Met Leu Gly Phe Pro Ile Asn Phe Leu Thr Leu Tyr Val Thr Val Gln
    50                  55                  60

His Lys Lys Leu Arg Thr Pro Leu Asn Tyr Ile Leu Leu Asn Leu Ala
65                  70                  75                  80

Val Ala Asp Leu Phe Met Val Phe Gly Gly Phe Thr Thr Thr Leu Tyr
                85                  90                  95

Thr Ser Leu His Gly Tyr Phe Val Phe Gly Pro Thr Gly Cys Asn Leu
            100                 105                 110

Glu Gly Phe Phe Ala Thr Leu Gly Gly Glu Ile Ala Leu Trp Ser Leu
        115                 120                 125

Val Val Leu Ala Ile Glu Arg Tyr Val Val Val Cys Lys Pro Met Ser
    130                 135                 140

Asn Phe Arg Phe Gly Glu Asn His Ala Ile Met Gly Val Ala Phe Thr
145                 150                 155                 160

Trp Val Met Ala Leu Ala Cys Ala Ala Pro Pro Leu Val Gly Trp Ser
                165                 170                 175

2

Arg Tyr Ile Pro Glu Gly Met Gln Cys Ser Cys Gly Ile Asp Tyr Tyr
            180                 185                 190
Thr Pro His Glu Glu Thr Asn Asn Glu Ser Phe Val Ile Tyr Met Phe
        195                 200                 205
Val Val His Phe Ile Ile Pro Leu Ile Val Ile Phe Phe Cys Tyr Gly
    210                 215                 220
Gln Leu Val Phe Thr Val Lys Glu Ala Ala Ala Gln Gln Gln Glu Ser
225                 230                 235                 240
Ala Thr Thr Gln Lys Ala Glu Lys Glu Val Thr Arg Met Val Ile Ile
                245                 250                 255
Met Val Ile Ala Phe Leu Ile Cys Trp Leu Pro Tyr Ala Gly Val Ala
            260                 265                 270
Phe Tyr Ile Phe Thr His Gln Gly Ser Asp Phe Gly Pro Ile Phe Met
        275                 280                 285
Thr Ile Pro Ala Phe Phe Ala Lys Thr Ser Ala Val Tyr Asn Pro Val
    290                 295                 300
Ile Tyr Ile Met Met Asn Lys Gln Phe Arg Asn Cys Met Val Thr Thr
305                 310                 315                 320
Leu Cys Cys Gly Lys Asn Pro Leu Gly Asp Asp Glu Ala Ser Thr Thr
                325                 330                 335
3

Val Ser Lys Thr Glu Thr Ser Gln Val Ala Pro Ala
            340                 345

<210> 2
<211> 29
<212> PRT
<213> Bos sp.

<400> 2
Gln Phe Ser Met Leu Ala Ala Tyr Met Phe Leu Leu Ile Met Leu Gly
1               5                   10                  15

Phe Pro Ile Asn Phe Leu Thr Leu Tyr Val Thr Val Gln
            20                  25

<210> 3
<211> 30
<212> PRT
<213> Bos sp.

<400> 3
Pro Leu Asn Tyr Ile Leu Leu Asn Leu Ala Val Ala Asp Leu Phe Met
1               5                   10                  15

Val Phe Gly Gly Phe Thr Thr Thr Leu Tyr Thr Ser Leu His
            20                  25                  30
4

<210> 4
<211> 33
<212> PRT
<213> Bos sp.

<400> 4
Pro Thr Gly Cys Asn Leu Glu Gly Phe Phe Ala Thr Leu Gly Gly Glu
1               5                   10                  15

Ile Ala Leu Trp Ser Leu Val Val Leu Ala Ile Glu Arg Tyr Val Val
            20                  25                  30

Val

<210> 5
<211> 23
<212> PRT
<213> Bos sp.

<400> 5
Asn His Ala Ile Met Gly Val Ala Phe Thr Trp Val Met Ala Leu Ala
1               5                   10                  15

Cys Ala Ala Pro Pro Leu Val
            20

5

<210> 6
<211> 26
<212> PRT
<213> Bos sp.

<400> 6
Asn Glu Ser Phe Val Ile Tyr Met Phe Val Val His Phe Ile Ile Pro
1               5                   10                  15

Leu Ile Val Ile Phe Phe Cys Tyr Gly Gln
            20                  25

<210> 7
<211> 31
<212> PRT
<213> Bos sp.

<400> 7
Glu Lys Glu Val Thr Arg Met Val Ile Ile Met Val Ile Ala Phe Leu
1               5                   10                  15

Ile Cys Trp Leu Pro Tyr Ala Gly Val Ala Phe Tyr Ile Phe Thr
            20                  25                  30

<210> 8
6

<211> 21
<212> PRT
<213> Bos sp.

<400> 8
Ile Phe Met Thr Ile Pro Ala Phe Phe Ala Lys Thr Ser Ala Val Tyr
1               5                   10                  15

Asn Pro Val Ile Tyr
            20

## Description

### TECHNICAL FIELD OF THE INVENTION

The present invention relates to three-dimensional structures of G protein-coupled receptors, especially rhodopsin, and derived structures therefrom.

### BACKGROUND OF THE INVENTION

Most eukaryotic organisms utilize guanine nucleotide-binding regulatory protein (hereinafter, referred to as "G protein")-coupled receptors (G protein-coupled receptors; hereinafter, frequently referred to as "GPCRs") to respond to a variety of stimuli such as Ca²⁺, amines, hormones, peptides, and even large proteins. Each GPCR is composed of an extracellular region, an intracellular region and a transmembrane bundle of seven α-helices. The binding of specific ligands to the extracellular or transmembrane domain evokes conformational changes that are transmitted to the intracellular region. The conformational switch from inactive to active state in the intracellular region produces a catalytically competent receptor that is responsible for binding and nucleotide exchange (GDP ⇔GTP) on α-subunits of several hundreds of G proteins of a specific subtype. α- and/or A'-subunit of the G proteins activates the next target protein(s) to evoke further intracellular responses. Because all of several hundreds of members in the GPCR family appear to share a common motif in their transmembrane structure and a common topology, it is generally accepted that a similar switching mechanism functions in all of these receptors.

The largest GPCR subfamily constituting about 90% of the total GPCRs include rhodopsin, cone pigment, and adrenergic or other receptors (Y. Shichida et al., Cell. Mol. Life Sci. 54, 1299 (1998)). Rhodopsin is recognized to be a member of the GPCR family because of its importance in the visual excitation (G. Wald et al., Science, 111, 179 (1950)), and its molecular and chemical characterization has been carried out for more than 50 years. The structural feature of rhodopsin is that it is composed of two molecules, i.e., opsin (~40 kDa) and a cofactor 11-*cis*-retinal (a derivative of vitamin A). These two molecules are covalently linked through Lys²⁹⁶ (i.e., lysine at position 296 of the amino acid sequence of rhodopsin) located in the transmembrane region of the receptor (Y. A. Ovchinnikov, FEBS Lett. 148, 179 (1982); P.A. Hargrave et al., Biophys. Struct. Mech. 9, 235 (1983); J. Nathans & T. Hogness, Cell 34, 807 (1983)), and give a characteristic maximum absorption at ∼500 nm. However, when the 11-*cis*-retinylidene group is located in a similar environment in homologous cone opsins, the maximum absorption of these pigments ranges between 380-580 nm. This spectral tuning allows the perception of colors.

Key residues in the binding pocket of the 11-*cis*-retinylidene group appear to be critical in interactions with the chromophore and are responsible for the spectral tuning (T. Yoshizawa, Photochem. Photobiol. 56, 859 (1992); G.G. Kochendoerfer et al., Trends Biochem. Sci. 24, 300 (1999); S.W. Lin et al., J. Biol. Chem. 273, 24583 (1998); A.B. Asenjo et al., Neuron 12, 1131 (1994); G.G. Kochendoerfer et al., Biochemistry 36, 6577 (1997); S.L. Merbs & J. Nathans, Photochem. Photobiol. 56, 869 (1992)). Rhodopsin has also been extensively investigated from a clinical view point since mutations in the human rhodopsin gene lead to retinal pathologies (T.P Dryja et al., Nature 343, 364-366 (1990); P. Humphries et al., Science 256, 804 (1992); A. Ratter et al., Annu. Rev. Genet. 33, 89 (1999)).

Absorption of a photon by the 11-*cis-*retinal chromophore covalently linked to transmembrane helix H-VII ("H" denotes helix, and the subsequent number denotes the order in the primary sequence) of rhodopsin causes its isomerization to all-*trans-*retinal (T. Yoshizawa & G. Wald, Nature 197, 1279 (1963); R.W. Schoenlein et al., Science 254, 412 (1991); Kandori et al., J. Am. Chem. Soc. 118, 1002 (1996)), leading to a conformational change of the protein moiety including the cytoplasmic surface.

The structural change of the chromophore as a result of absorption of photon only transiently activates opsin, and subsequently the all-trans-retinal is hydrolyzed and dissociated from the opsin. Rhodopsin is regenerated by newly synthesized 11-*cis*-retinal from adjacent retinal epithelial cells. This transient photoactivation of rhodopsin is especially remarkable because the absorption of a single photon results in the activaton of 100-1000 G protein molecules, whereas the 11-*cis*-retinal-bound rhodopsin is extremely low in activity (B.K.K. Fung & L Stryer, Proc. Natl. Acad. Sci. USA 77, 2500 (1980); H. Kuhn et al., Proc. Natl. Acad. Sci. USA 78, 6873 (1983)). These two properties are the molecular roots of high sensitivity in the human scotopic visual system and enable detection of as few as five photons (S. Hecht et al., J. Gen. Physiol. 25, 819 (1942)). These features put rhodopsin in a class of its own, as the most sophisticated molecular device among GPCRs.

With respect to three-dimensional structural information on GPCRs, several reports as described below have been made in addition to biochemical approaches and computational prediction studies.

A cryo-electron microscopic study of frog rhodopsin has revealed the organization of this receptor at 7.5 Å in the plane of the membrane and at 16.5 Å in the perpendicular direction to the membrane (V.M. Unger et al., Nature 389, 203 (1997)). This study permitted the prediction of the locations of seven rods of density that correspond to the transmembrane helices. The electron density map also allowed an estimation of the tilt angles for these seven helices. Many additional approaches have been employed to explore the structure of rhodopsin, and have also provided valuable information (J. Klein-Seetharaman et al., Proc. Natl. Acad. Sci. USA 96, 13744 (1999); O.P Ernst et al., J. Biol. Chem. 275, 1937 (2000); C. Altenbach et al., Biochemistry 38, 7945 (1999); J. Hwa et al., Proc. Natl. Acad. Sci. USA 96, 1932 (1999); J.-M. Kim et al., Proc. Natl. Acad. Sci. USA 94, 14273 (1997); Z.T. Farahbakhsh et al., Biochemistry 34, 8812 (1995); H. Yu et al., Biochemistry 38, 12028 (1995)). At present, several theoretical models of rhodopsin are available (T. Shieh et al., J. Mol. Biol. 269, 3737 (1997); P. Herzyk & R.E. Hubbard, J. Mol. Biol. 281, 741 (1998); LD. Pogozheva et al., Biophys. J. 72, 1963 (1997); J. M. Baldwin, EMBO J. 12, 1693, (1993); T.A. Nakayama & H.G. Khoranam, J. Biol. Chem. 266, 4269 (1991)).

Although these studies have provided a starting point for structural studies of the other members in the rhodopsin family, higher resolution three-dimensional accurate experimental models at the atomic level are needed to obtain further insight into the mechanisms of receptor activation, and the source of interactions between specific ligands and G proteins. Atomic models are also vital for understanding how specific mutations of the members of the GPCR superfamily can lead to genetic disorders.

Seven times transmembrane type GPCRs (7TMRs) are a major cellular receptor. They are important targets for developing pharmaceuticals. Many research groups have attempted production and crystallization of 7TMRs and even preparation of heavy atom derivatives from their crystals for the purpose of crystallographic analysis. However, no group has ever succeeded since these attempts have been extremely difficult.

### SUMMARY OF THE INVENTION

It is an object of the invention to elucidate the three-dimensional structure of bovine rhodopsin and to provide the fundamental structure of seven times transmembrane type GPCRs.

As a result of intensive and extensive researches toward the solution of the above problem, the present inventors have succeeded in analyzing the crystal structure of a GPCR at atomic resolution by crystallizing a rhodopsin prepared from bovine eyes, preparing a mercury derivative from the crystal taking a long period of time, and collecting six diffraction data from the single crystal by multi-wavelength anomalous diffraction methods (especially, a method in which the crystal was measured with varied wavelengths using synchrotron radiation and a beamline equipped with a trichrometer at SPring-8 Station). Thus, the present invention has been achieved.

The present invention relates to the following (1) to (6).
(1) A peptide fragment consisting of an amino acid sequence of positions 36-64, positions 71-100, positions 107-139, positions 151-173, positions 200-225, positions 247-277 or positions 286-306 of the amino acid sequence as shown in SEQ ID NO: 1, or a salt of the peptide fragment.
(2) A protein selected from the group consisting of the following (a), (b) and (c):
   (a) an isolated protein consisting of an amino acid sequence of positions 36-306 of the amino acid sequence as shown in SEQ ID NO: 1;
   (b) an isolated protein which consists of a part of an amino acid sequence of positions 36-306 of the amino acid sequence as shown in SEQ ID NO: 1, said part comprising at least positions 107-277, and which has G protein-coupled receptor activity;
   (c) an isolated protein which consists of an amino acid sequence of positions 36-306 or 107-277 of the amino acid sequence as shown in SEQ ID NO: 1 having a deletion(s), substitution(s) or addition(s) of one or more amino acids, and which has G protein-coupled receptor activity.
(3) A G protein-coupled receptor selected from the group consisting of the following (a) and (b):
   (a) a G protein-coupled receptor having three-dimensional structure I defined by the atomic coordinates as shown in Table 1;
   (b) a G protein-coupled receptor having three-dimensional structure II defined by derived coordinates from the atomic coordinates as shown in Table 1, wherein the mean residual of the discrepancies between the positions of the α carbon atoms in the amino acid residues of seven helix sites H-I (36-64), H-II (71-100), H-III (107-139), H-IV (151-173), H-V (200-225), H-VI (247-277) and H-VII (286-306) in the amino acid sequence as shown in SEQ ID NO: 1 of the three-dimensional structure I and the positions of the corresponding α carbon atoms in the amino acid residues of the corresponding seven helix sites of the three-dimensional structure II is 1.5 Ä or less when an image of the three-dimensional structure I obtained by computer-processing the atomic coordinates of Table 1 and an image of the three-dimensional structure II obtained by computer-processing the derived coordinates are superposed.

   As a specific example of the G protein-coupled receptor, bovine rhodopsin may be given. Preferably, this bovine rhodopsin is a metal derivative of its crystal. The metal is, for example, a mercury compound.
(4) A method of virtual screening for drugs, comprising computer-processing the following atomic coordinates (a) or (b), or derived coordinates therefrom, inputting the resultant computer-processed data into a virtual compound library and searching for useful drugs through the library:
   (a) a part of the atomic coordinates as shown in Table 1, the part corresponding to the amino acid residues of at least the three helix sites of H-III (107-139), H-V (200-225) and H-VI (247-277) selected from seven helix sites H-I(36-64), H-II (71-100), H-III (107-139), H-IV (151-173), H-V (200-225), H-VI (247-277) and H-VII (286-306) in the amino acid sequence as shown in SEQ ID NO: 1;
   (b) the atomic coordinates as shown in Table 1.

   In the above-described method, one example of the derived coordinates is derived coordinates generated by homology modeling based on the atomic coordinates as shown in Table 1, the mean residual of the discrepancies between the positions of the α carbon atoms in the amino acid residues of the three helix sites H-III (107-139), H-V (200-225) and H-VI (247-277) in the amino acid sequence as shown in SEQ ID NO: 1 and the positions of the corresponding α carbon atoms in the amino acid residues of the corresponding three helix sites in the derived coordinates being 1.5 Ä or less.
(5) A method of drug design, comprising imaging three-dimensional structures of G protein-coupled receptors using the following atomic coordinates (a) or (b), or derived coordinates therefrom, analyzing the resultant images by computer graphics and designing structures of useful drugs based on the resultant analysis data:
   (a) a part of the atomic coordinates as shown in Table 1, the part corresponding to the amino acid residues of at least the three helix sites of H-III (107-139), H-V (200-225) and H-VI (247-277) selected from seven helix sites H-I(36-64), H-II (71-100), H-III (107-139), H-IV (151-173), H-V (200-225), H-VI (247-277) and H-VII (286-306) in the amino acid sequence as shown in SEQ ID NO: 1;
   (b) the atomic coordinates as shown in Table 1.

   The derived coordinates are as described in (4) above.
(6) A method of screening for target substances that influence the effect of the G protein-coupled receptor described in (3) above, comprising comparing the three-dimensional structure of the receptor with three-dimensional structures of test substances.

The present specification encompasses the contents of the specification and drawings of Japanese Patent Application No. 2000-236288 based on which the present application claims priority.

### BRIEF DESCRIPTION OF THE DRAWINGS

Fig. 1 shows the molecular packing viewed along the x-axis of the crystal (Panel A); the electrostatic potentials of two rhodopsin molecules within the asymmetric unit expressed with MolScript (Panel B); and the crystal packing interface showing interactions between the N-terminal regions of neighboring rhodopsin molecules (Panel C).
Fig. 2 shows ribbon drawings of rhodopsin.
Fig. 3 shows a stereoview of the C α-trace of rhodopsin and residues involved in the hydrogen bonds between transmembrane segments (Panel A) and a ribbon drawing of the structure shadow-coded by average thermal parameters, with the same orientation as Panel A (Panel B). The darker the shadow, the lower the thermal parameter.
Fig. 4A shows the lengths of transmembrane α-helices and an alignment of rhodopsin sequences.
Fig. 4B shows a two-dimensional model of rhodopsin.
Fig. 5 shows stereoviews of the C α-traces on the intradiscal side of the transmembrane helices (Panel A) and on the cytoplasmic side of the molecule (Panel B).
Fig. 6 shows four regions characteristic of rhodopsin. The E-II loop near the disulfide bridge connecting Cys¹¹⁰ and Cys¹⁸⁷, viewed from extracellular side (Panel A). The C-IV cytoplasmic loop from Lys³¹¹ to Leu³²¹ forming a short amphiphilic helix (H-VIII) (Panel B). Interhelical hydrogen bonds mediated by a highly conserved Asn⁵⁵, connecting H-I, H-II, and H-VII, and by Asn⁷⁸ for H-II, H-III, and H-IV (Panel C). The (D/E)R(Y/W) motif region located near the cytoplasmic end of H-III (Panel D).
Fig. 7 shows an electron density map surrounding the 11-*cis*-retinal chromophore using experimental phases (Panel A); an electron density map surrounding the 11-*cis*-retinal chromophore using calculated phases based on the final model (Panel B); a schematic showing the side chains surrounding the 11-*cis*-retinylidene group (Panel C); and a schematic presenting side chains within 4.5 Å distance from the 11-*cis*-retinylidene group (Panel D).
Fig. 8 shows a three-dimensional structure of an AT-II receptor homology model that was created using the atomic coordinates of bovine rhodopsin.

### DETAILED DESCRIPTION OF THE INVENTION

Hereinbelow, the present invention will be described in detail.

G protein-coupled receptors (GPCRs) function in a number of signaling pathways, converting a variety of external stimuli to activate multiple copies of G protein subtypes specific to the relevant G protein-coupled receptor. GPCRs, including the prototypical member of this family, rhodopsin, share many structural features, including a bundle of seven transmembrane α-helices connected by six loops of varying lengths. In the present invention, diffraction data were collected from a crystal of bovine rhodopsin in the ground state at 2.8 Å resolution in order to obtain detailed information on the three-dimensional structure of rhodopsin. X-ray crystallographic analysis of bovine rhodopsin using the multi-wavelength anomalous diffraction method has elucidated for the first tine the structure of GPCR (crystallographic R-factor. 19.98%; R_{free}: 25.55%). The highly organized structure in the extracellular region (including a conserved disulfide bridge) forms a lid for the extracellular moiety of the seven-helix transmembrane motif.

The intrinsic chromophore to bovine rhodopsin, 11-*cis*-retinal, is a key cofactor for holding the transmembrane region of the protein in the inactive conformation. The chromophore also interacts with a cluster of key residues that give the wavelength of the maximum absorption. The site of a set of residues that mediate interactions between the transmembrane helices and the cytoplasmic surface, where G-protein activation is induced, suggests a possible structural change upon photoactivation.

Hereinbelow, the preparation and crystallization of rhodopsin, the structural analysis of the resultant crystal and applications of the obtained structure will be described taking bovine rhodopsin as an example of GPCR.

### 1. Preparation of Bovine Rhodopsin

In the present invention, bovine rhodopsin (hereinafter, sometimes just referred to as "rhodopsin") for use in crystallographic analysis may be collected from bovine eyes. Alternatively, commercial retinas may be used.

For example, rod outer segments (ROSs) are purified from bovine retinas (purchased from Schenk Packing Co., Inc., Stanwood, WA) using alkyl-thio-glycoside and divalent metal ions by conventional methods (e.g., Okada, T., Takeda, K. and Tokuyama, T., Photochem. Photobiol. 67: (5) 495-499 (1998); or Papermaster, D.S. Methods Enzymol. 81, 48-52 (1982)). These ROSs are solubilized with a surfactant nonyl glucoside and zinc acetate, and then concentrated by centrifugation to prepare a sample.

### 2. Crystallization of Bovine Rhodopsin

In the present invention, the crystallization of bovine rhodopsin may be carried out based on commonly used crystallization methods for X-ray crystallographic analysis. For example, a precipitant is added to a bovine rhodopsin solution of a specific concentration to change the concentration. As a result, the solubility of rhodopsin molecules lowers gradually and they are deposited as crystals.

The purity of rhodopsin is important for crystallization. It is essential to purify rhodopsin from highly pure ROSs. The concentration of rhodopsin is 5 mg/ml to 20 mg/ml, preferably ~10 mg/ml. As a precipitant, ammonium sulfate may be used, for example.

A suitable pH range for crystallization is 5.5-7.5, preferably 6.0-6.1. A suitable temperature range is 4-25°C, preferably 4-15°C. In the crystallization of membrane proteins, the type of surfactant and the concentration of the protein are most important. In the crystallization of rhodopsin, nonylglucoside and heptane-1,2,3-triol may be used preferably. In the present invention, PH is also an important parameter. For examining three parameters including pH (e.g., surfactant, pH and protein concentration) efficiently, it is desirable to prepare a phase diagram. Once crystals have begun to deposit, these parameters should be changed further finely to determine the optimal crystallization conditions under which best crystals are generated.

In crystallization, it is preferable to equilibrate a sample solution with a solution of the optimal crystallization conditions. In that case, a technique such as vapor diffusion or dialysis may be used.

Since rhodopsin is unique in structure and has poor resemblance with structures of other proteins, the molecular replacement method cannot be used in the structural analysis in the present invention. Phase information on each diffraction spot must be obtained directly from experiments and used in the analysis. Thus, the present invention has employed a technique called multi-wavelength anomalous diffraction (MAD) method that allows phase determination with only one heavy atom derivative by using variable wavelength synchrotron radiation. According to MAD method, phase information can be obtained using only one heavy atom derivative. Thus, this method is advantageous in a point that structure determination is possible even for those protein crystals, such as bovine rhodopsin crystal, whose lattice changes easily. However, measurement accuracy must be higher than other methods.

For introducing a heavy atom into crystals, the soaking method is generally used. In this method, already grown crystals are soaked in a solution containing a heavy atom reagent, which permeates into crystals spontaneously by diffusion.

Examples of heavy atom reagents useful in the invention include mercury compounds, platinum compounds, uranium compounds and gold compounds. Since bovine rhodopsin crystals are easily destroyed, a sufficient time is needed for soaking. Therefore, soaking for the preparation of a heavy atom derivative of rhodopsin crystal is carried out at 4-25°C, preferably 4-6°C, for two weeks to six months, preferably two to three months.

### 3. Structural Analysis of the Crystal

In the present invention, the structural analysis of the resultant crystal can be performed by the so-called MAD method. In the present invention, X-ray diffraction of the crystal is measured using synchrotron radiation on a beamline equipped with a trichrometer with varied wavelengths. Synchrotron radiation useful in the present invention may be gencrated by RIKEN Beamline I (BL45XU) at SPring-8 station, which is a large scale synchrotron radiation laboratory. In particular, RIKEN Beamline I (BL45XU) is a beamline optimized for the MAD method and suitable for collecting diffraction data for phase determination with varied wavelengths, even from those crystals, such as rhodopsin crystals, that are bad in reproducibility and have little isomorphism among crystals. The range of X-ray wavelengths used is 0.09-0.12 mm, preferably 0.07-0.15 nm. When using synchrotron radiation, it should be noted that the XANES spectrum of the test crystal must be measured in advance in order to determine exactly the X-ray wavelength corresponding to the absorption edge of the heavy atom.

As a result of the above structural analysis, the three-dimensional structure of bovine rhodopsin can be obtained as three-dimensional coordinates. The atomic coordinates obtained by the invention are shown in Table 1 below.

**Table 1**

| Column: | | | | | | | | | | | |
|---|---|---|---|---|---|---|---|---|---|---|---|
| 1 | 2 | 3 | 4 | 5 | 6 | 7 | 8 | 9 | 10 | 11 | 12 |
| ATOM | 1 | CA | ACE | A | 0 | 42.692 | -7.910 | -28.406 | 1.00 | 58.37 | A |
| ATOM | 2 | C | ACE | A | 0 | 43.697 | -7.215 | -27.534 | 1.00 | 58.09 | A |
| ATOM | 3 | O | ACE | A | 0 | 44.356 | -7.856 | -26.737 | 1.00 | 56.65 | A |
| ATOM | 4 | N | MET | A | 1 | 43.789 | -5.895 | -27.702 | 1.00 | 58.70 | A |
| ATOM | 5 | CA | MET | A | 1 | 44.707 | -5.048 | -26.931 | 1.00 | 58.56 | A |
| ATOM | 6 | CB | MET | A | 1 | 44.894 | -3.702 | -27.636 | 1.00 | 59.31 | A |
| ATOM | 7 | CG | MET | A | 1 | 46.319 | -3.408 | -28.061 | 1.00 | 59.50 | A |
| ATOM | 8 | SD | MET | A | 1 | 46.951 | -4.694 | -29.133 | 1.00 | 58.62 | A |
| ATOM | 9 | CE | MET | A | 1 | 48.475 | -5.134 | -28.302 | 1.00 | 59.39 | A |
| ATOM | 10 | C | MET | A | 1 | 44.160 | -4.807 | -25.526 | 1.00 | 57.96 | A |
| ATOM | 11 | O | MET | A | 1 | 42.977 | -4.475 | -25.374 | 1.00 | 58.24 | A |
| ATOM | 12 | N | ASN | A | 2 | 45.029 | -4.949 | -24.518 | 1.00 | 56.79 | A |
| ATOM | 13 | CA | ASN | A | 2 | 44.666 | -4.775 | -23.096 | 1.00 | 54.67 | A |
| ATOM | 14 | CB | ASN | A | 2 | 45.640 | -5.537 | -22.195 | 1.00 | 57.29 | A |
| ATOM | 15 | CG | ASN | A | 2 | 45.749 | -6.998 | -22.546 | 1.00 | 58.49 | A |
| ATOM | 16 | OD1 | ASM | A | 2 | 44.879 | -7.795 | -22.198 | 1.00 | 57.12 | A |
| ATOM | 17 | ND2 | ASN | A | 2 | 46.841 | -7.354 | -23.215 | 1.00 | 62.99 | A |
| ATOM | 18 | C | ASN | A | 2 | 44.657 | -3.320 | -22.645 | 1.00 | 51.53 | A |
| ATOM | 19 | O | ASN | A | 2 | 44.340 | -3.010 | -21.495 | 1.00 | 49.90 | A |
| ATOM | 20 | N | GLY | A | 3 | 45.077 | -2.447 | -23.546 | 1.00 | 49.81 | A |
| ATOM | 21 | CA | GLY | A | 3 | 45.117 | -1.037 | -23.251 | 1.00 | 48.69 | A |
| ATOM | 22 | C | GLY | A | 3 | 44.897 | -0.233 | -24.511 | 1.00 | 48.17 | A |
| ATOM | 23 | O | GLY | A | 3 | 45.179 | -0.680 | -25.628 | 1.00 | 48.57 | A |
| ATOM | 24 | N | THR | A | 4 | 44.357 | 0.958 | -24.328 | 1.00 | 46.74 | A |
| ATOM | 25 | CA | THR | A | 4 | 44.102 | 1.834 | -25.441 | 1.00 | 45.97 | A |
| ATOM | 26 | CB | THR | A | 4 | 42.803 | 2.622 | -25.218 | 1.00 | 44.14 | A |
| ATOM | 27 | OG1 | THR | A | 4 | 41.719 | 1.705 | -25.043 | 1.00 | 43.79 | A |
| ATOM | 28 | CG2 | THR | A | 4 | 42.499 | 3.507 | -26.395 | 1.00 | 42.35 | A |
| ATOM | 29 | C | THR | A | 4 | 45.286 | 2.780 | -25.546 | 1.00 | 47.50 | A |
| ATOM | 30 | O | THR | A | 4 | 45.460 | 3.643 | -24.686 | 1.00 | 48.20 | A |
| ATOM | 31 | N | GLU | A | 5 | 46.146 | 2.554 | -26.542 | 1.00 | 48.98 | A |
| ATOM | 32 | CA | GLU | A | 5 | 47.303 | 3.425 | -26.763 | 1.00 | 50.20 | A |
| ATOM | 33 | CB | GLU | A | 5 | 48.471 | 2.695 | -27.448 | 1.00 | 50.08 | A |
| ATOM | 34 | CG | GLU | A | 5 | 49.682 | 3.624 | -27.697 | 1.00 | 51.17 | A |
| ATOM | 35 | CD | GLU | A | 5 | 50.903 | 2.942 | -28.316 | 1.00 | 53.77 | A |
| ATOM | 36 | OE1 | GLU | A | 5 | 50.953 | 1.692 | -28.362 | 1.00 | 56.13 | A |
| ATOM | 37 | OE2 | GLU | A | 5 | 51.833 | 3.665 | -28.748 | 1.00 | 53.09 | A |
| ATOM | 38 | C | GLU | A | 5 | 46.892 | 4.610 | -27.630 | 1.00 | 51.17 | A |
| ATOM | 39 | O | GLU | A | 5 | 46.112 | 4.457 | -28.579 | 1.00 | 52.54 | A |
| ATOM | 40 | N | GLY | A | 6 | 47.411 | 5.787 | -27.287 | 1.00 | 50.76 | A |
| ATOM | 41 | CA | GLY | A | 6 | 47.120 | 6.994 | -28.037 | 1.00 | 48.54 | A |
| ATOM | 42 | C | GLY | A | 6 | 48.422 | 7.622 | -28.486 | 1.00 | 48.35 | A |
| ATOM | 43 | O | GLY | A | 6 | 49.496 | 7.083 | -28.210 | 1.00 | 47.71 | A |
| ATOM | 44 | N | PRO | A | 7 | 48.365 | 8.779 | -29.159 | 1.00 | 48.51 | A |
| ATOM | 45 | CD | PRO | A | 7 | 47.128 | 9.506 | -29.486 | 1.00 | 49.71 | A |
| ATOM | 46 | CA | PRO | A | 7 | 49.543 | 9.500 | -29.658 | 1.00 | 48.79 | A |
| ATOM | 47 | CB | PRO | A | 7 | 48.927 | 10.664 | -30.435 | 1.00 | 49.17 | A |
| ATOM | 48 | CG | PRO | A | 7 | 47.640 | 10.909 | -29.711 | 1.00 | 51.01 | A |
| ATOM | 49 | C | PRO | A | 7 | 50.486 | 9.989 | -28.566 | 1.00 | 48.79 | A |
| ATOM | 50 | O | PRO | A | 7 | 51.686 | 10.165 | -28.801 | 1.00 | 49.71 | A |
| ATOM | 51 | N | ASN | A | 8 | 49.934 | 10.186 | -27.372 | 1.00 | 48.52 | A |
| ATOM | 52 | CA | ASN | A | 8 | 50.699 | 10.659 | -26.221 | 1.00 | 46.90 | A |
| ATOM | 53 | CB | ASN | A | 8 | 50.780 | 12.191 | -26.237 | 1.00 | 44.50 | A |
| ATOM | 54 | CG | ASN | A | 8 | 49.414 | 12.859 | -26.212 | 1.00 | 42.02 | A |
| ATOM | 55 | OD1 | ASM | A | 8 | 48.373 | 12.214 | -26.354 | 1.00 | 41.82 | A |
| ATOM | 56 | ND2 | ASN | A | 8 | 49.417 | 14.168 | -26.038 | 1.00 | 41.53 | A |
| ATOM | 57 | C | ASN | A | 8 | 50.150 | 10.164 | -24.873 | 1.00 | 46.71 | A |
| ATOM | 58 | O | ASM | A | 8 | 50.259 | 10.858 | -23.860 | 1.00 | 46.87 | A |
| ATOM | 59 | N | PHE | A | 9 | 49.593 | 8.951 | -24.867 | 1.00 | 46.38 | A |
| ATOM | 60 | CA | PHE | A | 9 | 419.039 | 8.351 | -23.656 | 1.00 | 45.07 | A |
| ATOM | 61 | CB | PHE | A | 9 | 47.708 | 9.002 | -23.304 | 1.00 | 43.55 | A |
| ATOM | 62 | CG | PHE | A | 9 | 46.616 | 8.727 | -24.291 | 1.00 | 41.97 | A |
| ATOM | 63 | CD1 | PHE | A | 9 | 45.856 | 7.572 | -24.198 | 1.00 | 42.00 | A |
| ATOM | 64 | CD2 | PHE | A | 9 | 46.307 | 9.653 | -25.275 | 1.00 | 42.21 | A |
| ATOM | 65 | CE1 | PHE | A | 9 | 44.802 | 7.350 | -25.063 | 1.00 | 42.06 | A |
| ATOM | 66 | CE2 | PHE | A | 9 | 45.255 | 9.438 | -26.145 | 1.00 | 41.43 | A |
| ATOM | 67 | CZ | PHE | A | 9 | 44.499 | 8.285 | -26.039 | 1.00 | 40.93 | A |
| ATOM | 68 | C | PHE | A | 9 | 48.853 | 6.837 | -23.732 | 1.00 | 44.99 | A |
| ATOM | 69 | O | PHE | A | 9 | 49.107 | 6.210 | -24.754 | 1.00 | 46.12 | A |
| ATOM | 70 | N | TYR | A | 10 | 48.402 | 6.261 | -22.627 | 1.00 | 44.13 | A |
| ATOM | 71 | CA | TYR | A | 10 | 48.158 | 4.833 | -22.542 | 1.00 | 42.99 | A |
| ATOM | 72 | CB | TYR | A | 10 | 49.473 | 4.063 | -22.300 | 1.00 | 43.47 | A |
| ATOM | 73 | CG | TYR | A | 10 | 49.322 | 2.553 | -22.376 | 1.00 | 45.26 | A |
| ATOM | 74 | CD1 | TYR | A | 10 | 48.954 | 1.808 | -21.256 | 1.00 | 46.30 | A |
| ATOM | 75 | CE1 | TYR | A | 10 | 48.741 | 0.434 | -21.338 | 1.00 | 47.80 | A |
| ATOM | 76 | CD2 | TYR | A | 10 | 49.483 | 1.879 | -23.583 | 1.00 | 47.13 | A |
| ATOM | 77 | CE2 | TYR | A | 10 | 49.272 | 0.503 | -23.678 | 1.00 | 47.87 | A |
| ATOM | 78 | CZ | TYR | A | 10 | 48.900 | -0.211 | -22.554 | 1.00 | 48.99 | A |
| ATOM | 79 | OH | TYR | A | 10 | 48.680 | -1.567 | -22.653 | 1.00 | 50.18 | A |
| ATOM | 80 | C | TYR | A | 10 | 47.168 | 4.589 | -21.402 | 1.00 | 42.43 | A |
| ATOM | 81 | O | TYR | A | 10 | 47.544 | 4.633 | -20.230 | 1.00 | 41.33 | A |
| ATOM | 82 | N | VAL | A | 11 | 45.891 | 4.425 | -21.746 | 1.00 | 41.03 | A |
| ATOM | 83 | CA | VAL | A | 11 | 44.855 | 4.153 | -20.747 | 1.00 | 39.70 | A |
| ATOM | 84 | CB | VAL | A | 11 | 43.451 | 4.588 | -21.236 | 1.00 | 38.18 | A |
| ATOM | 85 | CG1 | VAL | A | 11 | 42.415 | 4.307 | -20.175 | 1.00 | 36.36 | A |
| ATOM | 86 | CG2 | VAL | A | 11 | 43.442 | 6.052 | -21.598 | 1.00 | 36.33 | A |
| ATOM | 87 | C | VAL | A | 11 | 44.860 | 2.638 | -20.561 | 1.00 | 39.64 | A |
| ATOM | 88 | O | VAL | A | 11 | 44.689 | 1.903 | -21.531 | 1.00 | 40.35 | A |
| ATOM | 89 | N | PRO | A | 12 | 45.117 | 2.152 | -19.330 | 1.00 | 39.18 | A |
| ATOM | 90 | CD | PRO | A | 12 | 45.558 | 2.904 | -18.149 | 1.00 | 36.83 | A |
| ATOM | 91 | CA | PRO | A | 12 | 45.146 | 0.711 | -19.047 | 1.00 | 40.46 | A |
| ATOM | 92 | CB | PRO | A | 12 | 45.801 | 0.657 | -17.663 | 1.00 | 38.51 | A |
| ATOM | 93 | CG | PRO | A | 12 | 46.531 | 1.953 | -17.553 | 1.00 | 36.31 | A |
| ATOM | 94 | C | PRO | A | 12 | 43.731 | 0.117 | -19.020 | 1.00 | 42.67 | A |
| ATOM | 95 | O | PRO | A | 12 | 43.347 | -0.559 | -18.064 | 1.00 | 42.99 | A |
| ATOM | 96 | N | PHE | A | 13 | 442.980 | 0.351 | -20.097 | 1.00 | 45.27 | A |
| ATOM | 97 | CA | PHE | A | 13 | 41.590 | -0.096 | -20.237 | 1.00 | 46.73 | A |
| ATOM | 98 | CB | PHE | A | 13 | 40.663 | 1.069 | -19.862 | 1.00 | 46.22 | A |
| ATOM | 99 | CG | PHE | A | 13 | 39.292 | 0.656 | -19.387 | 1.00 | 45.70 | A |
| ATOM | 100 | CD1 | PHE | A | 13 | 38.177 | 0.833 | -20.201 | 1.00 | 43.82 | A |
| ATOM | 101 | CD2 | PHE | A | 13 | 39.106 | 0.164 | -18.093 | 1.00 | 44.88 | A |
| ATOM | 102 | CE1 | PHE | A | 13 | 36.902 | 0.535 | -19.732 | 1.00 | 42.05 | A |
| ATOM | 103 | CE2 | PHE | A | 13 | 37.835 | -0.135 | -17.619 | 1.00 | 42.65 | A |
| ATOM | 104 | CZ | PHE | A | 13 | 36.733 | 0.052 | -18.439 | 1.00 | 42.24 | A |
| ATOM | 105 | C | PHE | A | 13 | 41.365 | -0.436 | -21.706 | 1.00 | 48.19 | A |
| ATOM | 106 | O | PHE | A | 13 | 41.814 | 0.303 | -22.582 | 1.00 | 48.79 | A |
| ATOM | 107 | N | SER | A | 14 | 40.651 | -1.524 | -21.984 | 1.00 | 49.86 | A |
| ATOM | 108 | CA | SER | A | 14 | 40.402 | -1.897 | -23.370 | 1.00 | 51.43 | A |
| ATOM | 109 | CB | SER | A | 14 | 40.085 | -3.382 | -23.499 | 1.00 | 51.26 | A |
| ATOM | 110 | OG | SER | A | 14 | 40.183 | -3.774 | -24.864 | 1.00 | 54.09 | A |
| ATOM | 111 | C | SER | A | 14 | 39.296 | -1.074 | -24.018 | 1.00 | 52.82 | A |
| ATOM | 112 | O | SER | A | 14 | 38.220 | -0.875 | -23.442 | 1.00 | 52.39 | A |
| ATOM | 113 | N | ASN | A | 15 | 39.567 | -0.618 | -25.235 | 1.00 | 54.06 | A |
| ATOM | 114 | CA | ASN | A | 15 | 38.613 | 0.184 | -25.973 | 1.00 | 55.84 | A |
| ATOM | 115 | CB | ASN | A | 15 | 39.342 | 1.225 | -26.794 | 1.00 | 57.15 | A |
| ATOM | 116 | CG | ASN | A | 15 | 368.463 | 2.371 | -27.138 | 1.00 | 58.84 | A |
| ATOM | 117 | OD1 | ASH | A | 15 | 37.585 | 2.722 | -26.354 | 1.00 | 57.21 | A |
| ATOM | 118 | ND2 | ASN | A | 15 | 38.657 | 2.948 | -28.315 | 1.00 | 60.52 | A |
| ATOM | 119 | C | ASN | A | 15 | 37.721 | -0.651 | -26.879 | 1.00 | 57.35 | A |
| ATOM | 120 | O | ASN | A | 15 | 37.147 | -0.153 | -27.852 | 1.00 | 57.21 | A |
| ATOM | 121 | N | LYS | A | 16 | 37.619 | -1.935 | -26.565 | 1.00 | 59.76 | A |
| ATOM | 122 | CA | LYS | A | 16 | 36.783 | -2.839 | -27.337 | 1.00 | 62.14 | A |
| ATOM | 123 | CB | LYS | A | 16 | 347.090 | -4.295 | -26.981 | 1.00 | 65.26 | A |
| ATOM | 124 | CG | LYS | A | 16 | 38.378 | -4.812 | -27.617 | 1.00 | 68.18 | A |
| ATOM | 125 | CD | LYS | A | 16 | 38.259 | -4.804 | -29.132 | 1.00 | 68.88 | A |
| ATOM | 126 | CE | LYS | A | 16 | 39.571 | -5.145 | -29.796 | 1.00 | 69.87 | A |
| ATOM | 127 | NZ | LYS | A | 16 | 39.385 | -5.280 | -31.265 | 1.00 | 70.07 | A |
| ATOM | 128 | C | LYS | A | 16 | 35.325 | -2.520 | -27.076 | 1.00 | 61.59 | A |
| ATOM | 129 | O | LYS | A | 16 | 34.430 | -3.187 | -27.588 | 1.00 | 61.19 | A |
| ATOM | 130 | N | THR | A | 17 | 35.110 | -1.502 | -26.249 | 1.00 | 61.93 | A |
| ATOM | 131 | CA | THR | A | 17 | 33.779 | -1.012 | -25.900 | 1.00 | 61.52 | A |
| ATOM | 132 | CB | THR | A | 17 | 33.549 | -1.028 | -24.373 | 1.00 | 61.66 | A |
| ATOM | 133 | OG1 | THR | A | 17 | 34.154 | -2.197 | -23.805 | 1.00 | 60.58 | A |
| ATOM | 134 | CG2 | THR | A | 17 | 32.047 | -1.024 | -24.064 | 1.00 | 61.50 | A |
| ATOM | 135 | C | THR | A | 17 | 33.691 | 0.444 | -26.377 | 1.00 | 60.86 | A |
| ATOM | 136 | O | THR | A | 17 | 32.635 | 1.074 | -26.289 | 1.00 | 60.20 | A |
| ATOM | 137 | N | GLY | A | 18 | 34.821 | 0.972 | -26.855 | 1.00 | 59.85 | A |
| ATOM | 138 | CA | GLY | A | 18 | 34.875 | 2.340 | -27.333 | 1.00 | 58.66 | A |
| ATOM | 139 | C | GLY | A | 18 | 34.730 | 3.363 | -26.220 | 1.00 | 58.05 | A |
| ATOM | 140 | O | GLY | A | 18 | 34.839 | 4.564 | -26.459 | 1.00 | 58.70 | A |
| ATOM | 141 | N | VAL | A | 19 | 34.527 | 2.882 | -24.997 | 1.00 | 56.90 | A |
| ATOM | 142 | CA | VAL | A | 19 | 34.345 | 3.736 | -23.825 | 1.00 | 55.72 | A |
| ATOM | 143 | CB | VAL | A | 19 | 33.898 | 2.895 | -22.608 | 1.00 | 56.73 | A |
| ATOM | 144 | CG1 | VAL | A | 19 | 35.049 | 2.028 | -22.111 | 1.00 | 58.03 | A |
| ATOM | 145 | CG2 | VAL | A | 19 | 33.353 | 3.788 | -21.502 | 1.00 | 57.66 | A |
| ATOM | 146 | C | VAL | A | 19 | 35.575 | 4.563 | -23.451 | 1.00 | 54.16 | A |
| ATOM | 147 | O | VAL | A | 19 | 35.460 | 5.581 | -22.772 | 1.00 | 55.02 | A |
| ATOM | 148 | N | VAL | A | 20 | 36.749 | 4.125 | -23.889 | 1.00 | 52.88 | A |
| ATOM | 149 | CA | VAL | A | 20 | 37.975 | 4.846 | -23.588 | 1.00 | 51.10 | A |
| ATOM | 150 | CB | VAL | A | 20 | 39.234 | 4.009 | -23.905 | 1.00 | 49.57 | A |
| ATOM | 151 | CG1 | VAL | A | 20 | 40.481 | 4.806 | -23.579 | 1.00 | 48.43 | A |
| ATOM | 152 | CG2 | VAL | A | 20 | 39.220 | 2.710 | -23.122 | 1.00 | 47.78 | A |
| ATOM | 153 | C | VAL | A | 20 | 38.038 | 6.144 | -24.372 | 1.00 | 51.08 | A |
| ATOM | 154 | O | VAL | A | 20 | 37.753 | 6.182 | -25.560 | 1.00 | 50.63 | A |
| ATOM | 155 | N | ARG | A | 21 | 38.394 | 7.217 | -23.685 | 1.00 | 53.23 | A |
| ATOM | 156 | CA | ARG | A | 21 | 38.518 | 8.521 | -24.318 | 1.00 | 55.42 | A |
| ATOM | 157 | CB | ARG | A | 21 | 37.273 | 9.372 | -24.033 | 1.00 | 58.74 | A |
| ATOM | 158 | CG | ARG | A | 21 | 36.974 | 10.418 | -25.107 | 1.00 | 63.20 | A |
| ATOM | 159 | CD | ARG | A | 21 | 37.077 | 9.811 | -26.508 | 1.00 | 67.31 | A |
| ATOM | 160 | NE | ARG | A | 21 | 36.395 | 8.517 | -26.595 | 1.00 | 70.83 | A |
| ATOM | 161 | CZ | ARG | A | 21 | 35.373 | 8.257 | -27.406 | 1.00 | 72.69 | A |
| ATOM | 162 | NH1 | ARG | A | 21 | 34.905 | 9.203 | -28.213 | 1.00 | 74.26 | A |
| ATOM | 163 | MH2 | ARG | A | 21 | 34.811 | 7.053 | -27.408 | 1.00 | 72.56 | A |
| ATOM | 164 | C | ARG | A | 21 | 39.787 | 9.193 | -23.788 | 1.00 | 54.36 | A |
| ATOM | 165 | O | ARG | A | 21 | 40.067 | 9.135 | -22.592 | 1.00 | 54.83 | A |
| ATOM | 166 | N | SER | A | 22 | 40.554 | 9.810 | -24.685 | 1.00 | 53.47 | A |
| ATOM | 167 | CA | SER | A | 22 | 41.808 | 10.468 | -24.326 | 1.00 | 52.57 | A |
| ATOM | 168 | CA | SER | A | 22 | 42.235 | 11.457 | -25.407 | 1.00 | 51.00 | A |
| ATOM | 169 | OG | SER | A | 22 | 43.508 | 12.010 | -25.117 | 1.00 | 47.83 | A |
| ATOM | 170 | C | SER | A | 22 | 41.742 | 11.187 | -22.996 | 1.00 | 54.30 | A |
| ATOM | 171 | O | SER | A | 22 | 40.798 | 11.924 | -22.734 | 1.00 | 53.49 | A |
| ATOM | 172 | N | PRO | A | 23 | 42.723 | 10.928 | -22.113 | 1.00 | 56.74 | A |
| ATOM | 173 | CD | PRO | A | 23 | 43.730 | 9.867 | -22.282 | 1.00 | 57.82 | A |
| ATOM | 174 | CA | PRO | A | 23 | 42.836 | 11.527 | -20.777 | 1.00 | 56.74 | A |
| ATOM | 175 | CB | PRO | A | 23 | 43.970 | 10.726 | -20.132 | 1.00 | 57.71 | A |
| ATOM | 176 | CG | PRO | A | 23 | 43.948 | 9.426 | -20.862 | 1.00 | 58.98 | A |
| ATOM | 177 | C | PRO | A | 23 | 43.205 | 13.006 | -20.850 | 1.00 | 56.91 | A |
| ATOM | 178 | O | PRO | A | 23 | 43.664 | 13.579 | -19.867 | 1.00 | 57.91 | A |
| ATOM | 179 | N | PHE | A | 24 | 43.045 | 13.600 | -22.030 | 1.00 | 56.66 | A |
| ATOM | 180 | CA | PHE | A | 24 | 43.332 | 15.015 | -22.254 | 1.00 | 55.58 | A |
| ATOM | 181 | CB | PHE | A | 24 | 44.502 | 15.169 | -23.231 | 1.00 | 55.30 | A |
| ATOM | 182 | CG | PHE | A | 24 | 45.816 | 14.667 | -22.698 | 1.00 | 54.57 | A |
| ATOM | 183 | CD1 | PHE | A | 24 | 46.464 | 15.332 | -21.665 | 1.00 | 54.43 | A |
| ATOM | 184 | CD2 | PHE | A | 24 | 46.408 | 13.531 | -23.227 | 1.00 | 54.79 | A |
| ATOM | 185 | CE1 | PHE | A | 24 | 47.683 | 14.872 | -21.167 | 1.00 | 53.44 | A |
| ATOM | 186 | CE2 | PHE | A | 24 | 47.628 | 13.066 | -22.731 | 1.00 | 54.53 | A |
| ATOM | 187 | CZ | PHE | A | 24 | 48.262 | 13.740 | -21.700 | 1.00 | 52.84 | A |
| ATOM | 188 | C | PHE | A | 24 | 42.094 | 15.716 | -22.823 | 1.00 | 55.93 | A |
| ATOM | 189 | O | PHE | A | 24 | 42.059 | 16.947 | -22.940 | 1.00 | 55.94 | A |
| ATOM | 190 | N | GLN | A | 25 | 41.075 | 14.920 | -23.149 | 1.00 | 55.86 | A |
| ATOM | 191 | CA | GLN | A | 25 | 39.831 | 15.421 | -23.727 | 1.00 | 56.04 | A |
| ATOM | 192 | CB | GLN | A | 25 | 39.643 | 14.848 | -25.146 | 1.00 | 57.04 | A |
| ATOM | 193 | CG | GLN | A | 25 | 40.805 | 15.044 | -26.129 | 1.00 | 59.85 | A |
| ATOM | 194 | CD | GLN | A | 25 | 40.779 | 14.038 | -27.302 | 1.00 | 62.89 | A |
| ATOM | 195 | OE1 | GLN | A | 25 | 39.872 | 13.199 | -27.413 | 1.00 | 63.17 | A |
| ATOM | 196 | NE2 | GLN | A | 25 | 41.796 | 14.109 | -28.162 | 1.00 | 63.39 | A |
| ATOM | 197 | C | GLN | A | 25 | 38.562 | 15.114 | -22.903 | 1.00 | 54.84 | A |
| ATOM | 198 | O | GLN | A | 25 | 37.546 | 15.791 | -23.081 | 1.00 | 55.15 | A |
| ATOM | 199 | N | ALA | A | 26 | 38.608 | 14.116 | -22.013 | 1.00 | 52.77 | A |
| ATOM | 200 | CA | ALA | A | 26 | 37.420 | 13.741 | -21.226 | 1.00 | 51.75 | A |
| ATOM | 201 | CB | ALA | A | 26 | 36.414 | 13.024 | -22.143 | 1.00 | 52.34 | A |
| ATOM | 202 | C | ALA | A | 26 | 37.653 | 12.892 | -19.958 | 1.00 | 50.19 | A |
| ATOM | 203 | O | ALA | A | 26 | 38.606 | 12.122 | -19.888 | 1.00 | 50.55 | A |
| ATOM | 204 | N | PRO | A | 27 | 36.743 | 12.989 | -18.960 | 1.00 | 48.72 | A |
| ATOM | 205 | CD | PRO | A | 27 | 35.541 | 13.841 | -18.929 | 1.00 | 48.24 | A |
| ATOM | 206 | CA | PRO | A | 27 | 36.846 | 12.235 | -17.705 | 1.00 | 48.18 | A |
| ATOM | 207 | CB | PRO | A | 27 | 35.553 | 12.616 | -16.968 | 1.00 | 47.65 | A |
| ATOM | 208 | CG | PRO | A | 27 | 34.626 | 13.041 | -18.054 | 1.00 | 46.86 | A |
| ATOM | 209 | C | PRO | A | 27 | 36.995 | 10.713 | -17.846 | 1.00 | 47.75 | A |
| ATOM | 210 | O | PRO | A | 27 | 36.341 | 10.079 | -18.684 | 1.00 | 48.12 | A |
| ATOM | 211 | N | GLN | A | 28 | 37.846 | 10.148 | -16.990 | 1.00 | 45.86 | A |
| ATOM | 212 | CA | GLN | A | 28 | 38.136 | 8.722 | -16.965 | 1.00 | 45.53 | A |
| ATOM | 213 | CB | GLN | A | 28 | 39.593 | 8.517 | -16.587 | 1.00 | 42.65 | A |
| ATOM | 214 | CG | GLN | A | 28 | 40.538 | 9.306 | -17.441 | 1.00 | 39.97 | A |
| ATOM | 215 | CD | GLN | A | 28 | 40.440 | 8.928 | -18.896 | 1.00 | 38.43 | A |
| ATOM | 216 | OE1 | GLN | A | 28 | 39.831 | 9.639 | -19.688 | 1.00 | 38.10 | A |
| ATOM | 217 | NE2 | GLN | A | 28 | 41.037 | 7.800 | -19.259 | 1.00 | 37.92 | A |
| ATOM | 218 | C | GLN | A | 28 | 37.247 | 7.956 | -15.992 | 1.00 | 47.57 | A |
| ATOM | 219 | O | GLN | A | 28 | 37.649 | 6.933 | -15.438 | 1.00 | 47.46 | A |
| ATOM | 220 | N | TYR | A | 29 | 36.030 | 8.451 | -15.803 | 1.00 | 50.16 | A |
| ATOM | 221 | CA | TYR | A | 29 | 35.057 | 7.837 | -14.904 | 1.00 | 52.07 | A |
| ATOM | 222 | CB | TYR | A | 29 | 33.796 | 8.696 | -14.841 | 1.00 | 54.49 | A |
| ATOM | 223 | CG | TYR | A | 29 | 34.028 | 10.052 | -14.217 | 1.00 | 56.18 | A |
| ATOM | 224 | CD1 | TYR | A | 29 | 35.109 | 10.272 | -13.365 | 1.00 | 56.34 | A |
| ATOM | 225 | CE1 | TYR | A | 29 | 35.311 | 11.501 | -12.761 | 1.00 | 57.44 | A |
| ATOM | 226 | CD2 | TYR | A | 29 | 33.153 | 11.104 | -14.454 | 1.00 | 58.05 | A |
| ATOM | 227 | CE2 | TYR | A | 29 | 33.345 | 12.340 | -13.853 | 1.00 | 59.89 | A |
| ATOM | 228 | CZ | TYR | A | 29 | 34.425 | 12.529 | -13.006 | 1.00 | 59.52 | A |
| ATOM | 229 | OH | TYR | A | 29 | 34.599 | 13.744 | -12.386 | 1.00 | 61.93 | A |
| ATOM | 230 | C | TYR | A | 29 | 34.694 | 6.421 | -15.312 | 1.00 | 52.01 | A |
| ATOM | 231 | O | TYR | A | 29 | 33.991 | 5.717 | -14.589 | 1.00 | 51.86 | A |
| ATOM | 232 | N | TYR | A | 30 | 35.135 | 6.024 | -16.495 | 1.00 | 52.13 | A |
| ATOM | 233 | CA | TYR | A | 30 | 34.869 | 4.684 | -16.973 | 1.00 | 53.48 | A |
| ATOM | 234 | CB | TYR | A | 30 | 34.857 | 4.670 | -18.503 | 1.00 | 51.86 | A |
| ATOM | 235 | CG | TYR | A | 30 | 36.144 | 5.124 | -19.140 | 1.00 | 49.90 | A |
| ATOM | 236 | CD1 | TYR | A | 30 | 37.206 | 4.243 | -19.288 | 1.00 | 49.78 | A |
| ATOM | 237 | CE1 | TYR | A | 30 | 38.386 | 4.633 | -19.889 | 1.00 | 49.52 | A |
| ATOM | 238 | CD2 | TYR | A | 30 | 36.296 | 6.424 | -19.613 | 1.00 | 49.21 | A |
| ATOM | 239 | CE2 | TYR | A | 30 | 37.482 | 6.829 | -20.225 | 1.00 | 48.45 | A |
| ATOM | 240 | CZ | TYR | A | 30 | 38.524 | 5.920 | -20.357 | 1.00 | 49.12 | A |
| ATOM | 241 | OH | TYR | A | 30 | 39.714 | 6.269 | -20.956 | 1.00 | 49.53 | A |
| ATOM | 242 | C | TYR | A | 30 | 35.926 | 3.731 | -16.396 | 1.00 | 54.93 | A |
| ATOM | 243 | O | TYR | A | 30 | 35.750 | 2.511 | -16.404 | 1.00 | 55.10 | A |
| ATOM | 244 | N | LEU | A | 31 | 37.007 | 4.311 | -15.870 | 1.00 | 56.77 | A |
| ATOM | 245 | CA | LEU | A | 31 | 38.108 | 3.558 | -15.262 | 1.00 | 58.98 | A |
| ATOM | 246 | CB | LEU | A | 31 | 39.399 | 4.383 | -15.288 | 1.00 | 57.98 | A |
| ATOM | 247 | CG | LEU | A | 31 | 40.083 | 4.658 | -16.624 | 1.00 | 57.81 | A |
| ATOM | 248 | CD1 | LEU | A | 31 | 41.409 | 5.356 | -16.386 | 1.00 | 57.24 | A |
| ATOM | 249 | CD2 | LEU | A | 31 | 40.321 | 3.357 | -17.344 | 1.00 | 57.12 | A |
| ATOM | 250 | C | LEU | A | 31 | 37.814 | 3.169 | -13.812 | 1.00 | 60.84 | A |
| ATOM | 251 | O | LEU | A | 31 | 38.395 | 2.213 | -13.282 | 1.00 | 61.51 | A |
| ATOM | 252 | N | ALA | A | 32 | 36.953 | 3.958 | -13.172 | 1.00 | 62.90 | A |
| ATOM | 253 | CA | ALA | A | 32 | 36.537 | 3.764 | -11.781 | 1.00 | 64.61 | A |
| ATOM | 254 | CB | ALA | A | 32 | 37.742 | 3.828 | -10.855 | 1.00 | 64.59 | A |
| ATOM | 255 | C | ALA | A | 32 | 35.517 | 4.851 | -11.407 | 1.00 | 66.21 | A |
| ATOM | 256 | O | ALA | A | 32 | 35.466 | 5.908 | -12.045 | 1.00 | 67.09 | A |
| ATOM | 257 | N | GLU | A | 33 | 34.729 | 4.600 | -10.363 | 1.00 | 66.91 | A |
| ATOM | 258 | CA | GLU | A | 33 | 33.692 | 5.536 | -9.909 | 1.00 | 67.99 | A |
| ATOM | 259 | CB | GLU | A | 33 | 32.953 | 4.952 | -8.697 | 1.00 | 69.11 | A |
| ATOM | 260 | CG | GLU | A | 33 | 31.857 | 3.932 | -9.030 | 1.00 | 70.98 | A |
| ATOM | 261 | CD | GLU | A | 33 | 32.387 | 2.623 | -9.605 | 1.00 | 71.82 | A |
| ATOM | 262 | OE1 | GLU | A | 33 | 31.692 | 2.021 | -10.456 | 1.00 | 71.74 | A |
| ATOM | 263 | OE2 | GLU | A | 33 | 33.488 | 2.189 | -9.198 | 1.00 | 73.40 | A |
| ATOM | 264 | C | GLU | A | 33 | 34.139 | 6.975 | -9.599 | 1.00 | 68.12 | A |
| ATOM | 265 | O | GLU | A | 33 | 35.315 | 7.229 | -9.334 | 1.00 | 69.00 | A |
| ATOM | 266 | N | PRO | A | 34 | 33.198 | 7.941 | -9.668 | 1.00 | 68.22 | A |
| ATOM | 267 | CD | PRO | A | 34 | 31.866 | 7.756 | -10.281 | 1.00 | 69.30 | A |
| ATOM | 268 | CA | PRO | A | 34 | 33.438 | 9.367 | -9.401 | 1.00 | 67.84 | A |
| ATOM | 269 | CB | PRO | A | 34 | 32.071 | 9.997 | -9.687 | 1.00 | 68.34 | A |
| ATOM | 270 | CG | PRO | A | 34 | 31.546 | 9.144 | -10.798 | 1.00 | 68.37 | A |
| ATOM | 271 | C | PRO | A | 34 | 33.930 | 9.714 | -7.987 | 1.00 | 66.46 | A |
| ATOM | 272 | O | PRO | A | 34 | 34.545 | 10.764 | -7.780 | 1.00 | 67.29 | A |
| ATOM | 273 | N | TRP | A | 35 | 33.648 | 8.850 | -7.018 | 1.00 | 64.20 | A |
| ATOM | 274 | CA | TRP | A | 35 | 34.071 | 9.098 | -5.645 | 1.00 | 61.58 | A |
| ATOM | 275 | CB | TRP | A | 35 | 33.171 | 8.347 | -4.661 | 1.00 | 63.00 | A |
| ATOM | 276 | CG | TRP | A | 35 | 33.279 | 6.849 | -4.719 | 1.00 | 63.73 | A |
| ATOM | 277 | CD2 | TRP | A | 35 | 34.129 | 6.022 | -3.915 | 1.00 | 64.23 | A |
| ATOM | 278 | CE2 | TRP | A | 35 | 33.849 | 4.679 | -4.255 | 1.00 | 63.97 | A |
| ATOM | 279 | CE3 | TRP | A | 35 | 35.101 | 6.288 | -2.935 | 1.00 | 64.19 | A |
| ATOM | 280 | CD1 | TRP | A | 35 | 32.546 | 6.000 | -5.501 | 1.00 | 63.86 | A |
| ATOM | 281 | NE1 | TRP | A | 35 | 32.881 | 4.693 | -5.224 | 1.00 | 63.51 | A |
| ATOM | 282 | CZ2 | TRP | A | 35 | 34.504 | 3.601 | -3.649 | 1.00 | 64.18 | A |
| ATOM | 283 | CZ3 | TRP | A | 35 | 35.753 | 5.219 | -2.334 | 1.00 | 63.97 | A |
| ATOM | 284 | CH2 | TRP | A | 35 | 35.450 | 3.890 | -2.694 | 1.00 | 64.70 | A |
| ATOM | 285 | C | TRP | A | 35 | 35.545 | 8.749 | -5.426 | 1.00 | 58.79 | A |
| ATOM | 286 | O | TRP | A | 35 | 36.222 | 9.349 | -4.589 | 1.00 | 58.48 | A |
| ATOM | 287 | N | GLN | A | 36 | 36.037 | 7.767 | -6.172 | 1.00 | 55.09 | A |
| ATOM | 288 | CA | GLN | A | 36 | 37.433 | 7.376 | -6.068 | 1.00 | 52.73 | A |
| ATOM | 289 | CB | GLN | A | 36 | 37.711 | 6.182 | -6.966 | 1.00 | 52.01 | A |
| ATOM | 290 | CG | GLN | A | 36 | 36.674 | 5.109 | -6.819 | 1.00 | 52.45 | A |
| ATOM | 291 | CD | GLN | A | 36 | 37.221 | 3.744 | -7.105 | 1.00 | 53.55 | A |
| ATOM | 292 | OE1 | GLN | A | 36 | 36.829 | 3.094 | -8.075 | 1.00 | 55.20 | A |
| ATOM | 293 | NE2 | GLN | A | 36 | 38.130 | 3.286 | -6.252 | 1.00 | 54.05 | A |
| ATOM | 294 | C | GLN | A | 36 | 38.234 | 8.582 | -6.524 | 1.00 | 51.36 | A |
| ATOM | 295 | O | GLN | A | 36 | 39.209 | 8.969 | -5.881 | 1.00 | 51.76 | A |
| ATOM | 296 | N | PHE | A | 37 | 37.788 | 9.186 | -7.625 | 1.00 | 48.47 | A |
| ATOM | 297 | CA | PHE | A | 37 | 38.414 | 10.385 | -8.159 | 1.00 | 45.29 | A |
| ATOM | 298 | CB | PHE | A | 37 | 37.733 | 10.813 | -9.469 | 1.00 | 44.44 | A |
| ATOM | 299 | CG | PHE | A | 37 | 38.174 | 10.018 | -10.677 | 1.00 | 43.06 | A |
| ATOM | 300 | CD1 | PHE | A | 37 | 39.306 | 10.392 | -11.395 | 1.00 | 40.60 | A |
| ATOM | 301 | CD2 | PHE | A | 37 | 37.478 | 8.880 | -11.077 | 1.00 | 43.60 | A |
| ATOM | 302 | CE1 | PHE | A | 37 | 39.745 | 9.645 | -12.489 | 1.00 | 41.10 | A |
| ATOM | 303 | CE2 | PHE | A | 37 | 37.909 | 8.121 | -12.175 | 1.00 | 43.01 | A |
| ATOM | 304 | CZ | PHE | A | 37 | 39.047 | 8.507 | -12.879 | 1.00 | 42.77 | A |
| ATOM | 305 | C | PHE | A | 37 | 38.270 | 11.478 | -7.104 | 1.00 | 44.14 | A |
| ATOM | 306 | O | PHE | A | 37 | 39.228 | 12.179 | -6.783 | 1.00 | 44.00 | A |
| ATOM | 307 | N | SER | A | 38 | 37.077 | 11.571 | -6.522 | 1.00 | 43.46 | A |
| ATOM | 308 | CA | SER | A | 38 | 36.798 | 12.558 | -5.486 | 1.00 | 43.76 | A |
| ATOM | 309 | CB | SER | A | 38 | 35.313 | 12.530 | -5.098 | 1.00 | 46.04 | A |
| ATOM | 310 | OG | SER | A | 38 | 34.486 | 12.970 | -6.169 | 1.00 | 49.09 | A |
| ATOM | 311 | C | SER | A | 38 | 37.681 | 12.345 | -4.255 | 1.00 | 42.43 | A |
| ATOM | 312 | O | SER | A | 38 | 37.879 | 13.264 | -3.465 | 1.00 | 41.74 | A |
| ATOM | 313 | N | MET | A | 39 | 38.190 | 11.126 | -4.086 | 1.00 | 41.51 | A |
| ATOM | 314 | CA | MET | A | 39 | 39.074 | 10.815 | -2.967 | 1.00 | 41.18 | A |
| ATOM | 315 | CB | MET | A | 39 | 39.029 | 9.329 | -2.623 | 1.00 | 44.09 | A |
| ATOM | 316 | CG | MET | A | 39 | 37.852 | 8.962 | -1.737 | 1.00 | 49.61 | A |
| ATOM | 317 | SD | MET | A | 39 | 37.752 | 9.986 | -0.228 | 1.00 | 57.49 | A |
| ATOM | 318 | CE | MET | A | 39 | 36.203 | 10.897 | -0.537 | 1.00 | 56.18 | A |
| ATOM | 319 | C | MET | A | 39 | 40.504 | 11.269 | -3.249 | 1.00 | 39.31 | A |
| ATOM | 320 | O | MET | A | 39 | 41.224 | 11.689 | -2.335 | 1.00 | 38.29 | A |
| ATOM | 321 | N | LEU | A | 40 | 40.910 | 11.177 | -4.514 | 1.00 | 36.67 | A |
| ATOM | 322 | CA | LEU | A | 40 | 42.233 | 11.621 | -4.927 | 1.00 | 34.61 | A |
| ATOM | 323 | CB | LEU | A | 40 | 42.465 | 11.338 | -6.406 | 1.00 | 30.36 | A |
| ATOM | 324 | CG | LEU | A | 40 | 42.347 | 9.891 | -6.846 | 1.00 | 27.34 | A |
| ATOM | 325 | CD1 | LEU | A | 40 | 42.836 | 9.766 | -8.273 | 1.00 | 23.69 | A |
| ATOM | 326 | CD2 | LEU | A | 40 | 43.160 | 9.015 | -5.921 | 1.00 | 25.37 | A |
| ATOM | 327 | C | LEU | A | 40 | 42.251 | 13.123 | -4.710 | 1.00 | 35.88 | A |
| ATOM | 328 | O | LEU | A | 40 | 43.225 | 13.668 | -4.188 | 1.00 | 35.88 | A |
| ATOM | 329 | N | ALA | A | 41 | 41.152 | 13.773 | -5.106 | 1.00 | 36.04 | A |
| ATOM | 330 | CA | ALA | A | 41 | 40.982 | 15.213 | -4.961 | 1.00 | 36.09 | A |
| ATOM | 331 | CB | ALA | A | 41 | 39.669 | 15.639 | -5.555 | 1.00 | 34.79 | A |
| ATOM | 332 | C | ALA | A | 41 | 41.066 | 15.610 | -3.481 | 1.00 | 38.21 | A |
| ATOM | 333 | O | ALA | A | 41 | 41.877 | 16.464 | -3.123 | 1.00 | 40.99 | A |
| ATOM | 334 | N | ALA | A | 42 | 40.266 | 14.972 | -2.618 | 1.00 | 37.61 | A |
| ATOM | 335 | CA | ALA | A | 42 | 40.288 | 15.255 | -1.176 | 1.00 | 35.01 | A |
| ATOM | 336 | CB | ALA | A | 42 | 39.303 | 14.381 | -0.447 | 1.00 | 32.92 | A |
| ATOM | 337 | C | ALA | A | 42 | 41.694 | 15.003 | -0.649 | 1.00 | 35.26 | A |
| ATOM | 338 | O | ALA | A | 42 | 42.280 | 15.859 | 0.004 | 1.00 | 36.19 | A |
| ATOM | 339 | N | TYR | A | 43 | 42.246 | 13.838 | -0.973 | 1.00 | 34.30 | A |
| ATOM | 340 | CA | TYR | A | 43 | 43.597 | 13.473 | -0.561 | 1.00 | 32.52 | A |
| ATOM | 341 | CB | TYR | A | 43 | 43.986 | 12.171 | -1.242 | 1.00 | 30.14 | A |
| ATOM | 342 | CG | TYR | A | 43 | 45.439 | 11.797 | -1.114 | 1.00 | 27.43 | A |
| ATOM | 343 | CD1 | TYR | A | 43 | 46.227 | 11.630 | -2.245 | 1.00 | 27.24 | A |
| ATOM | 344 | CE1 | TYR | A | 43 | 47.541 | 11.223 | -2.147 | 1.00 | 25.54 | A |
| ATOM | 345 | CD2 | TYR | A | 43 | 46.013 | 11.555 | 0.128 | 1.00 | 26.96 | A |
| ATOM | 346 | CE2 | TYR | A | 43 | 47.329 | 11.148 | 0.237 | 1.00 | 25.16 | A |
| ATOM | 347 | CZ | TYR | A | 43 | 48.080 | 10.980 | -0.908 | 1.00 | 25.93 | A |
| ATOM | 348 | OH | TYR | A | 43 | 49.363 | 10.517 | -0.821 | 1.00 | 31.19 | A |
| ATOM | 349 | C | TYR | A | 43 | 44.613 | 14.560 | -0.917 | 1.00 | 33.46 | A |
| ATOM | 350 | O | TYR | A | 43 | 45.398 | 14.988 | -0.072 | 1.00 | 32.85 | A |
| ATOM | 351 | N | MET | A | 44 | 44.591 | 15.000 | -2.174 | 1.00 | 35.67 | A |
| ATOM | 352 | CA | MET | A | 44 | 45.509 | 16.044 | -2.660 | 1.00 | 35.92 | A |
| ATOM | 353 | CB | MET | A | 44 | 45.381 | 16.231 | -4.182 | 1.00 | 33.16 | A |
| ATOM | 354 | CG | MET | A | 44 | 45.923 | 15.069 | -5.003 | 1.00 | 31.07 | A |
| ATOM | 355 | SD | MET | A | 44 | 47.576 | 14.506 | -4.491 | 1.00 | 29.27 | A |
| ATOM | 356 | CE | MET | A | 44 | 48.506 | 16.034 | -4.521 | 1.00 | 28.34 | A |
| ATOM | 357 | C | MET | A | 44 | 45.296 | 17.377 | -1.952 | 1.00 | 35.20 | A |
| ATOM | 358 | O | MET | A | 44 | 46.244 | 18.094 | -1.663 | 1.00 | 33.71 | A |
| ATOM | 359 | N | PHE | A | 45 | 44.039 | 17.699 | -1.686 | 1.00 | 36.29 | A |
| ATOM | 360 | CA | PHE | A | 45 | 43.693 | 18.921 | -0.993 | 1.00 | 37.83 | A |
| ATOM | 361 | CB | PHE | A | 45 | 42.171 | 19.029 | -0.906 | 1.00 | 41.69 | A |
| ATOM | 362 | CG | PHE | A | 45 | 41.691 | 20.205 | -0.113 | 1.00 | 46.82 | A |
| ATOM | 363 | CD1 | PHE | A | 45 | 40.926 | 20.016 | 1.034 | 1.00 | 49.30 | A |
| ATOM | 364 | CD2 | PHE | A | 45 | 42.024 | 21.503 | -0.494 | 1.00 | 48.89 | A |
| ATOM | 365 | CE1 | PHE | A | 45 | 40.500 | 21.105 | 1.796 | 1.00 | 50.21 | A |
| ATOM | 366 | CE2 | PHE | A | 45 | 41.604 | 22.600 | 0.259 | 1.00 | 49.91 | A |
| ATOM | 367 | CZ | PHE | A | 45 | 40.841 | 22.399 | 1.408 | 1.00 | 50.54 | A |
| ATOM | 368 | C | PHE | A | 45 | 44.334 | 18.910 | 0.403 | 1.00 | 37.13 | A |
| ATOM | 369 | O | PHE | A | 45 | 44.783 | 19.944 | 0.894 | 1.00 | 37.22 | A |
| ATOM | 370 | N | LEU | A | 46 | 44.419 | 17.726 | 1.010 | 1.00 | 36.48 | A |
| ATOM | 371 | CA | LEU | A | 46 | 45.014 | 17.553 | 2.338 | 1.00 | 34.63 | A |
| ATOM | 372 | CB | LEU | A | 46 | 44.572 | 16.225 | 2.954 | 1.00 | 34.19 | A |
| ATOM | 373 | CG | LEU | A | 46 | 43.267 | 16.208 | 3.764 | 1.00 | 35.37 | A |
| ATOM | 374 | CD1 | LEU | A | 46 | 42.486 | 17.503 | 3.575 | 1.00 | 34.98 | A |
| ATOM | 375 | CD2 | LEU | A | 46 | 42.409 | 14.986 | 3.410 | 1.00 | 34.74 | A |
| ATOM | 376 | C | LEU | A | 46 | 46.534 | 17.629 | 2.320 | 1.00 | 34.49 | A |
| ATOM | 377 | O | LEU | A | 46 | 47.142 | 18.015 | 3.311 | 1.00 | 34.21 | A |
| ATOM | 378 | N | LEU | A | 47 | 47.144 | 17.227 | 1.205 | 1.00 | 34.51 | A |
| ATOM | 379 | CA | LEU | A | 47 | 48.604 | 17.270 | 1.059 | 1.00 | 34.31 | A |
| ATOM | 380 | CB | LEU | A | 47 | 49.070 | 16.401 | -0.110 | 1.00 | 32.89 | A |
| ATOM | 381 | CG | LEU | A | 47 | 49.162 | 14.892 | 0.037 | 1.00 | 30.45 | A |
| ATOM | 382 | CD1 | LEU | A | 47 | 49.863 | 14.345 | -1.197 | 1.00 | 29.73 | A |
| ATOM | 383 | CD2 | LEU | A | 47 | 49.942 | 14.551 | 1.284 | 1.00 | 27.83 | A |
| ATOM | 384 | C | LEU | A | 47 | 49.071 | 18.694 | 0.802 | 1.00 | 34.79 | A |
| ATOM | 385 | O | LEU | A | 47 | 50.189 | 19.071 | 1.154 | 1.00 | 34.35 | A |
| ATOM | 386 | N | ILE | A | 48 | 48.230 | 19.448 | 0.101 | 1.00 | 35.66 | A |
| ATOM | 387 | CA | ILE | A | 48 | 48.514 | 20.835 | -0.228 | 1.00 | 34.75 | A |
| ATOM | 388 | CB | ILE | A | 48 | 47.578 | 21.332 | -1.350 | 1.00 | 32.51 | A |
| ATOM | 389 | CG2 | ILE | A | 48 | 47.705 | 22.831 | -1.532 | 1.00 | 32.60 | A |
| ATOM | 390 | CG1 | ILE | A | 48 | 47.909 | 20.591 | -2.650 | 1.00 | 30.84 | A |
| ATOM | 391 | CD1 | ILE | A | 48 | 47.261 | 21.168 | -3.895 | 1.00 | 27.15 | A |
| ATOM | 392 | C | ILE | A | 48 | 48.354 | 21.668 | 1.036 | 1.00 | 35.45 | A |
| ATOM | 393 | O | ILE | A | 48 | 49.064 | 22.657 | 1.242 | 1.00 | 36.90 | A |
| ATOM | 394 | N | MET | A | 49 | 47.468 | 21.214 | 1.914 | 1.00 | 35.64 | A |
| ATOM | 395 | CA | MET | A | 49 | 47.227 | 21.904 | 3.171 | 1.00 | 36.72 | A |
| ATOM | 396 | CB | MET | A | 49 | 45.873 | 21.482 | 3.744 | 1.00 | 36.36 | A |
| ATOM | 397 | CG | MET | A | 49 | 45.282 | 22.480 | 4.708 | 1.00 | 36.93 | A |
| ATOM | 398 | SD | MET | A | 49 | 43.584 | 22.085 | 5.115 | 1.00 | 38.55 | A |
| ATOM | 399 | CE | MET | A | 49 | 42.721 | 23.522 | 4.343 | 1.00 | 36.69 | A |
| ATOM | 400 | C | MET | A | 49 | 48.373 | 21.639 | 4.164 | 1.00 | 36.73 | A |
| ATOM | 401 | O | MET | A | 49 | 48.669 | 22.470 | 5.019 | 1.00 | 38.01 | A |
| ATOM | 402 | N | LEU | A | 50 | 49.026 | 20.490 | 4.032 | 1.00 | 36.47 | A |
| ATOM | 403 | CA | LEU | A | 50 | 50.151 | 20.139 | 4.895 | 1.00 | 37.55 | A |
| ATOM | 404 | CB | LEU | A | 50 | 50.183 | 18.633 | 5.154 | 1.00 | 38.09 | A |
| ATOM | 405 | CG | LEU | A | 50 | 49.177 | 17.996 | 6.104 | 1.00 | 37.57 | A |
| ATOM | 406 | CD1 | LEU | A | 50 | 49.373 | 16.479 | 6.139 | 1.00 | 33.82 | A |
| ATOM | 407 | CD2 | LEU | A | 50 | 49.369 | 18.607 | 7.481 | 1.00 | 37.68 | A |
| ATOM | 408 | C | LEU | A | 50 | 51.492 | 20.552 | 4.271 | 1.00 | 38.43 | A |
| ATOM | 409 | O | LEU | A | 50 | 52.317 | 21.202 | 4.919 | 1.00 | 39.85 | A |
| ATOM | 410 | N | GLY | A | 51 | 51.706 | 20.155 | 3.017 | 1.00 | 36.98 | A |
| ATOM | 411 | CA | GLY | A | 51 | 52.942 | 20.470 | 2.327 | 1.00 | 34.24 | A |
| ATOM | 412 | C | GLY | A | 51 | 53.256 | 21.944 | 2.313 | 1.00 | 32.66 | A |
| ATOM | 413 | O | GLY | A | 51 | 54.415 | 22.332 | 2.278 | 1.00 | 30.63 | A |
| ATOM | 414 | N | PHE | A | 52 | 52.222 | 22.773 | 2.340 | 1.00 | 33.26 | A |
| ATOM | 415 | CA | PHE | A | 52 | 52.449 | 24.204 | 2.329 | 1.00 | 35.51 | A |
| ATOM | 416 | CB | PHE | A | 52 | 51.192 | 24.965 | 1.916 | 1.00 | 35.53 | A |
| ATOM | 417 | CG | PHE | A | 52 | 51.373 | 26.442 | 1.928 | 1.00 | 39.37 | A |
| ATOM | 418 | CD1 | PHE | A | 52 | 52.602 | 27.004 | 1.583 | 1.00 | 40.99 | A |
| ATOM | 419 | CD2 | PHE | A | 52 | 50.341 | 27.278 | 2.324 | 1.00 | 42.26 | A |
| ATOM | 420 | CE1 | PHE | A | 52 | 52.808 | 28.378 | 1.636 | 1.00 | 42.29 | A |
| ATOM | 421 | CE2 | PHE | A | 52 | 50.533 | 28.659 | 2.382 | 1.00 | 43.24 | A |
| ATOM | 422 | CZ | PHE | A | 52 | 51.774 | 29.208 | 2.037 | 1.00 | 42.87 | A |
| ATOM | 423 | C | PHE | A | 52 | 53.064 | 24.754 | 3.636 | 1.00 | 36.49 | A |
| ATOM | 424 | O | PRE | A | 52 | 54.252 | 25.098 | 3.649 | 1.00 | 37.53 | A |
| ATOM | 425 | N | PRO | A | 53 | 52.294 | 24.787 | 4.756 | 1.00 | 35.79 | A |
| ATOM | 426 | CD | PRO | A | 53 | 50.929 | 24.264 | 4.935 | 1.00 | 36.08 | A |
| ATOM | 427 | CA | PRO | A | 53 | 52.794 | 25.292 | 6.038 | 1.00 | 33.97 | A |
| ATOM | 428 | CB | PRO | A | 53 | 51.659 | 24.963 | 6.996 | 1.00 | 33.32 | A |
| ATOM | 429 | CG | PRO | A | 53 | 50.467 | 25.004 | 6.142 | 1.00 | 34.39 | A |
| ATOM | 430 | C | PRO | A | 53 | 54.077 | 24.606 | 6.481 | 1.00 | 34.16 | A |
| ATOM | 431 | O | PRO | A | 53 | 55.122 | 25.235 | 6.535 | 1.00 | 36.65 | A |
| ATOM | 432 | N | ILE | A | 54 | 53.996 | 23.315 | 6.776 | 1.00 | 32.27 | A |
| ATOM | 433 | CA | ILE | A | 54 | 55.151 | 22.546 | 7.222 | 1.00 | 31.91 | A |
| ATOM | 434 | CB | ILE | A | 54 | 54.912 | 21.040 | 7.051 | 1.00 | 31.67 | A |
| ATOM | 435 | CG2 | ILE | A | 54 | 56.101 | 20.262 | 7.552 | 1.00 | 30.45 | A |
| ATOM | 436 | CG1 | ILE | A | 54 | 53.658 | 20.618 | 7.816 | 1.00 | 30.45 | A |
| ATOM | 437 | CD1 | ILE | A | 54 | 53.166 | 19.244 | 7.463 | 1.00 | 29.57 | A |
| ATOM | 438 | C | ILE | A | 54 | 56.413 | 22.915 | 6.463 | 1.00 | 32.63 | A |
| ATOM | 439 | O | ILE | A | 54 | 57.421 | 23.255 | 7.070 | 1.00 | 32.03 | A |
| ATOM | 440 | N | ASN | A | 55 | 56.335 | 22.888 | 5.135 | 1.00 | 34.43 | A |
| ATOM | 441 | CA | ASN | A | 55 | 57.480 | 23.220 | 4.293 | 1.00 | 36.06 | A |
| ATOM | 442 | CB | ASN | A | 55 | 57.237 | 22.816 | 2.838 | 1.00 | 34.75 | A |
| ATOM | 443 | CG | ASN | A | 55 | 57.371 | 21.333 | 2.627 | 1.00 | 33.05 | A |
| ATOM | 444 | OD1 | ASN | A | 55 | 58.465 | 20.785 | 2.705 | 1.00 | 34.94 | A |
| ATOM | 445 | ND2 | ASH | A | 55 | 56.260 | 20.668 | 2.384 | 1.00 | 31.71 | A |
| ATOM | 446 | C | ASN | A | 55 | 57.818 | 24.686 | 4.361 | 1.00 | 36.90 | A |
| ATOM | 447 | O | ASN | A | 55 | 58.986 | 25.049 | 4.462 | 1.00 | 36.68 | A |
| ATOM | 448 | N | PHE | A | 56 | 56.789 | 25.525 | 4.313 | 1.00 | 38.75 | A |
| ATOM | 449 | CA | PHE | A | 56 | 56.982 | 26.968 | 4.376 | 1.00 | 40.68 | A |
| ATOM | 450 | CB | PHE | A | 56 | 55.705 | 27.699 | 3.981 | 1.00 | 43.46 | A |
| ATOM | 451 | CG | PHE | A | 56 | 55.866 | 29.185 | 3.937 | 1.00 | 46.85 | A |
| ATOM | 452 | CD1 | PHE | A | 56 | 56.449 | 29.797 | 2.828 | 1.00 | 48.01 | A |
| ATOM | 453 | CD2 | PHE | A | 56 | 155.489 | 29.970 | 5.024 | 1.00 | 46.41 | A |
| ATOM | 454 | CE1 | PHE | A | 56 | 56.659 | 31.168 | 2.803 | 1.00 | 47.15 | A |
| ATOM | 455 | CE2 | PHE | A | 56 | 55.694 | 31.340 | 5.009 | 1.00 | 46.24 | A |
| ATOM | 456 | CZ | PHE | A | 56 | 56.281 | 31.941 | 3.898 | 1.00 | 47.56 | A |
| ATOM | 457 | C | PHE | A | 56 | 57.465 | 27.461 | 5.749 | 1.00 | 39.97 | A |
| ATOM | 458 | O | PHE | A | 56 | 58.181 | 28.455 | 5.834 | 1.00 | 39.35 | A |
| ATOM | 459 | N | LEU | A | 57 | 57.015 | 26.808 | 6.818 | 1.00 | 39.36 | A |
| ATOM | 460 | CA | PHE | A | 57 | 57.431 | 27.154 | 8.173 | 1.00 | 37.23 | A |
| ATOM | 461 | CB | LEU | A | 57 | 56.573 | 26.426 | 9.201 | 1.00 | 35.80 | A |
| ATOM | 462 | CG | LEU | A | 57 | 57.150 | 26.466 | 10.617 | 1.00 | 35.39 | A |
| ATOM | 463 | CD1 | LEU | A | 57 | 56.976 | 27.862 | 11.157 | 1.00 | 34.37 | A |
| ATOM | 464 | C D2 | LEU | A | 57 | 56.490 | 25.434 | 11.531 | 1.00 | 34.88 | A |
| ATOM | 465 | C | LEU | A | 57 | 58.876 | 26.692 | 8.317 | 1.00 | 38.17 | A |
| ATOM | 466 | O | LEU | A | 57 | 59.684 | 27.341 | 8.970 | 1.00 | 38.21 | A |
| ATOM | 467 | N | THR | A | 58 | 59.180 | 25.539 | 7.729 | 1.00 | 38.57 | A |
| ATOM | 468 | CA | THR | A | 58 | 60.530 | 24.995 | 7.760 | 1.00 | 39.20 | A |
| ATOM | 469 | CB | THR | A | 58 | 60.595 | 23.657 | 6.989 | 1.00 | 36.63 | A |
| ATOM | 470 | OG1 | THR | A | 58 | 59.770 | 22.690 | 7.645 | 1.00 | 35.10 | A |
| ATOM | 471 | CG2 | THR | A | 58 | 62.015 | 23.138 | 6.917 | 1.00 | 33.97 | A |
| ATOM | 472 | C | THR | A | 58 | 61.427 | 26.018 | 7.066 | 1.00 | 42.25 | A |
| ATOM | 473 | O | THR | A | 58 | 62.541 | 26.316 | 7.507 | 1.00 | 42.64 | A |
| ATOM | 474 | N | LEU | A | 59 | 60.887 | 26.587 | 5.996 | 1.00 | 44.91 | A |
| ATOM | 475 | CA | LEU | A | 59 | 61.576 | 27.582 | 5.193 | 1.00 | 46.80 | A |
| ATOM | 476 | CB | LEU | A | 59 | 60.719 | 27.902 | 3.968 | 1.00 | 48.20 | A |
| ATOM | 477 | CG | LEU | A | 59 | 61.397 | 28.570 | 2.783 | 1.00 | 48.78 | A |
| ATOM | 478 | CD1 | LEU | A | 59 | 62.101 | 27.504 | 1.983 | 1.00 | 49.35 | A |
| ATOM | 479 | CD2 | LEU | A | 59 | 60.352 | 29.261 | 1.930 | 1.00 | 49.92 | A |
| ATOM | 480 | C | LEU | A | 59 | 61.859 | 28.865 | 5.986 | 1.00 | 46.86 | A |
| ATOM | 481 | O | LEU | A | 59 | 63.019 | 29.220 | 6.198 | 1.00 | 45.89 | A |
| ATOM | 482 | N | TYR | A | 60 | 60.796 | 29.523 | 6.454 | 1.00 | 47.35 | A |
| ATOM | 483 | CA | TYR | A | 60 | 60.915 | 30.768 | 7.206 | 1.00 | 48.92 | A |
| ATOM | 484 | CB | TYR | A | 60 | 59.536 | 31.317 | 7.611 | 1.00 | 52.88 | A |
| ATOM | 485 | CG | TYR | A | 60 | 59.580 | 32.745 | 8.155 | 1.00 | 58.10 | A |
| ATOM | 486 | CD1 | TYR | A | 60 | 58.726 | 33.162 | 9.186 | 1.00 | 58.93 | A |
| ATOM | 487 | CE1 | TYR | A | 60 | 58.798 | 34.480 | 9.704 | 1.00 | 61.99 | A |
| ATOM | 488 | CD2 | TYR | A | 60 | 60.504 | 33.675 | 7.651 | 1.00 | 61.33 | A |
| ATOM | 489 | CE2 | TYR | A | 60 | 60.589 | 34.985 | 8.159 | 1.00 | 63.16 | A |
| ATOM | 490 | CZ | TYR | A | 60 | 59.738 | 35.384 | 9.182 | 1.00 | 63.60 | A |
| ATOM | 491 | OH | TYR | A | 60 | 59.852 | 36.675 | 9.668 | 1.00 | 63.39 | A |
| ATOM | 492 | C | TYR | A | 60 | 61.817 | 30.674 | 8.432 | 1.00 | 48.21 | A |
| ATOM | 493 | O | TYR | A | 60 | 62.502 | 31.632 | 8.774 | 1.00 | 48.22 | A |
| ATOM | 494 | N | VAL | A | 61 | 621.837 | 29.528 | 9.094 | 1.00 | 47.56 | A |
| ATOM | 495 | CA | VAL | A | 61 | 62.683 | 29.398 | 10.267 | 1.00 | 48.19 | A |
| ATOM | 496 | CB | VAL | A | 61 | 62.394 | 28.087 | 11.047 | 1.00 | 48.28 | A |
| ATOM | 497 | CG1 | VAL | A | 61 | 63.332 | 27.965 | 12.244 | 1.00 | 47.74 | A |
| ATOM | 498 | CG2 | VAL | A | 61 | 60.947 | 28.057 | 11.526 | 1.00 | 46.86 | A |
| ATOM | 499 | C | VAL | A | 61 | 64.166 | 29.478 | 9.885 | 1.00 | 49.97 | A |
| ATOM | 500 | O | VAL | A | 61 | 64.871 | 30.386 | 10.336 | 1.00 | 49.43 | A |
| ATOM | 501 | N | THR | A | 62 | 64.602 | 28.586 | 8.989 | 1.00 | 51.64 | A |
| ATOM | 502 | CA | THR | A | 62 | 66.006 | 28.504 | 8.544 | 1.00 | 51.75 | A |
| ATOM | 503 | CB | THR | A | 62 | 66.199 | 27.429 | 7.412 | 1.00 | 49.98 | A |
| ATOM | 504 | OG1 | THR | A | 62 | 65.582 | 26.190 | 7.793 | 1.00 | 47.83 | A |
| ATOM | 505 | CG2 | THR | A | 62 | 67.677 | 27.163 | 7.169 | 1.00 | 47.16 | A |
| ATOM | 506 | C | THR | A | 62 | 66.624 | 29.843 | 8.100 | 1.00 | 53.25 | A |
| ATOM | 507 | O | THR | A | 62 | 67.791 | 30.129 | 8.400 | 1.00 | 53.15 | A |
| ATOM | 508 | N | VAL | A | 63 | 65.826 | 30.669 | 7.425 | 1.00 | 54.32 | A |
| ATOM | 509 | CA | VAL | A | 63 | 66.282 | 31.969 | 6.928 | 1.00 | 56.47 | A |
| ATOM | 510 | CB | VAL | A | 63 | 65.334 | 32.518 | 5.843 | 1.00 | 55.74 | A |
| ATOM | 511 | CG1 | VAL | A | 63 | 65.972 | 33.709 | 5.150 | 1.00 | 55.35 | A |
| ATOM | 512 | CG2 | VAL | A | 63 | 64.973 | 31.429 | 4.853 | 1.00 | 54.49 | A |
| ATOM | 513 | C | VAL | A | 63 | 66.391 | 33.038 | 8.016 | 1.00 | 58.18 | A |
| ATOM | 514 | O | VAL | A | 63 | 67.326 | 33.846 | 8.014 | 1.00 | 59.55 | A |
| ATOM | 515 | N | GLN | A | 64 | 65.409 | 33.046 | 8.917 | 1.00 | 59.07 | A |
| ATOM | 516 | CA | GLN | A | 64 | 65.322 | 34.004 | 10.020 | 1.00 | 58.87 | A |
| ATOM | 517 | CB | GLN | A | 64 | 63.963 | 33.870 | 10.709 | 1.00 | 59.45 | A |
| ATOM | 518 | CG | GLN | A | 64 | 63.812 | 34.708 | 11.962 | 1.00 | 60.94 | A |
| ATOM | 519 | CD | GLN | A | 64 | 62.433 | 34.594 | 12.581 | 1.00 | 61.16 | A |
| ATOM | 520 | OE1 | GLN | A | 64 | 61.480 | 35.209 | 12.108 | 1.00 | 62.42 | A |
| ATOM | 521 | NE2 | GLN | A | 64 | 62.324 | 33.820 | 13.654 | 1.00 | 60.83 | A |
| ATOM | 522 | C | GLN | A | 64 | 66.420 | 33.856 | 11.058 | 1.00 | 58.59 | A |
| ATOM | 523 | O | GLN | A | 64 | 66.851 | 34.845 | 11.661 | 1.00 | 58.39 | A |
| ATOM | 524 | N | HIS | A | 65 | 66.836 | 32.612 | 11.281 | 1.00 | 58.81 | A |
| ATOM | 525 | CA | HIS | A | 65 | 67.870 | 32.289 | 12.255 | 1.00 | 59.29 | A |
| ATOM | 526 | CB | HIS | A | 65 | 67.462 | 31.033 | 13.022 | 1.00 | 61.19 | A |
| ATOM | 527 | CG | HIS | A | 65 | 66.174 | 31.187 | 13.777 | 1.00 | 63.64 | A |
| ATOM | 528 | CD2 | HIS | A | 65 | 65.455 | 32.289 | 14.099 | 1.00 | 65.30 | A |
| ATOM | 529 | ND1 | HIS | A | 65 | 65.479 | 30.117 | 14.300 | 1.00 | 65.61 | A |
| ATOM | 530 | CE1 | HIS | A | 65 | 64.390 | 30.553 | 14.909 | 1.00 | 63.68 | A |
| ATOM | 531 | NE2 | HIS | A | 65 | 64.352 | 31.867 | 14.803 | 1.00 | 63.66 | A |
| ATOM | 532 | C | HIS | A | 65 | 69.235 | 32.145 | 11.585 | 1.00 | 58.91 | A |
| ATOM | 533 | O | HIS | A | 65 | 69.645 | 31.049 | 11.197 | 1.00 | 58.21 | A |
| ATOM | 534 | N | LYS | A | 66 | 69.936 | 33.279 | 11.506 | 1.00 | 58.73 | A |
| ATOM | 535 | CA | LYS | A | 66 | 71.246 | 33.426 | 10.858 | 1.00 | 58.41 | A |
| ATOM | 536 | CB | LYS | A | 66 | 71.738 | 34.886 | 10.989 | 1.00 | 58.14 | A |
| ATOM | 537 | CG | LYS | A | 66 | 70.894 | 35.910 | 10.205 | 1.00 | 57.41 | A |
| ATOM | 538 | CD | LYS | A | 66 | 71.012 | 35.660 | 8.703 | 1.00 | 57.50 | A |
| ATOM | 539 | CE | LYS | A | 66 | 69.881 | 36.286 | 7.907 | 1.00 | 56.75 | A |
| ATOM | 540 | NZ | LYS | A | 66 | 69.920 | 35.804 | 6.489 | 1.00 | 56.63 | A |
| ATOM | 541 | C | LYS | A | 66 | 72.386 | 32.431 | 11.142 | 1.00 | 57.56 | A |
| ATOM | 542 | O | LYS | A | 66 | 73.302 | 32.298 | 10.317 | 1.00 | 57.85 | A |
| ATOM | 543 | N | LYS | A | 67 | 72.329 | 31.719 | 12.268 | 1.00 | 56.19 | A |
| ATOM | 544 | CA | LYS | A | 67 | 73.376 | 30.747 | 12.601 | 1.00 | 54.53 | A |
| ATOM | 545 | CB | LYS | A | 67 | 73.819 | 30.867 | 14.074 | 1.00 | 55.11 | A |
| ATOM | 546 | CG | LYS | A | 67 | 74.545 | 32.169 | 14.470 | 1.00 | 55.63 | A |
| ATOM | 547 | CD | LYS | A | 67 | 75.470 | 31.952 | 15.684 | 1.00 | 56.05 | A |
| ATOM | 548 | CE | LYS | A | 67 | 74.708 | 31.497 | 16.935 | 1.00 | 55.98 | A |
| ATOM | 549 | NZ | LYS | A | 67 | 75.593 | 31.064 | 18.070 | 1.00 | 55.20 | A |
| ATOM | 550 | C | LYS | A | 67 | 72.954 | 29.304 | 12.310 | 1.00 | 53.05 | A |
| ATOM | 551 | O | LYS | A | 67 | 73.706 | 28.367 | 12.567 | 1.00 | 54.24 | A |
| ATOM | 552 | N | LEU | A | 68 | 71.748 | 29.126 | 11.785 | 1.00 | 51.46 | A |
| ATOM | 553 | CA | LEU | A | 68 | 71.235 | 27.791 | 11.462 | 1.00 | 50.99 | A |
| ATOM | 554 | CB | LEU | A | 68 | 69.698 | 27.809 | 11.577 | 1.00 | 48.86 | A |
| ATOM | 555 | CG | LEU | A | 68 | 68.853 | 26.645 | 12.119 | 1.00 | 44.80 | A |
| ATOM | 556 | CD1 | LEU | A | 68 | 69.327 | 26.196 | 13.482 | 1.00 | 43.96 | A |
| ATOM | 557 | CD2 | LEU | A | 68 | 67.410 | 27.095 | 12.211 | 1.00 | 42.39 | A |
| ATOM | 558 | C | LEU | A | 68 | 71.684 | 27.461 | 10.026 | 1.00 | 51.57 | A |
| ATOM | 559 | O | LEU | A | 68 | 70.859 | 27.187 | 9.147 | 1.00 | 51.25 | A |
| ATOM | 560 | N | ARG | A | 69 | 73.001 | 27.459 | 9.806 | 1.00 | 51.26 | A |
| ATOM | 561 | CA | ARG | A | 69 | 73.551 | 27.225 | 8.476 | 1.00 | 50.50 | A |
| ATOM | 562 | CB | ARG | A | 69 | 74.076 | 28.554 | 7.919 | 1.00 | 49.06 | A |
| ATOM | 563 | CG | ARG | A | 69 | 73.106 | 29.711 | 8.025 | 1.00 | 47.95 | A |
| ATOM | 564 | CD | ARG | A | 69 | 71.860 | 29.450 | 7.208 | 1.00 | 50.65 | A |
| ATOM | 565 | NE | ARG | A | 69 | 70.815 | 30.435 | 7.455 | 1.00 | 53.72 | A |
| ATOM | 566 | CZ | ARG | A | 69 | 70.922 | 31.732 | 7.173 | 1.00 | 57.64 | A |
| ATOM | 567 | MH1 | ARG | A | 69 | 72.040 | 32.216 | 6.629 | 1.00 | 58.63 | A |
| ATOM | 568 | NH2 | ARG | A | 69 | 69.902 | 32.548 | 7.429 | 1.00 | 57.82 | A |
| ATOM | 569 | C | ARG | A | 69 | 74.632 | 26.143 | 8.318 | 1.00 | 51.47 | A |
| ATOM | 570 | O | ARG | A | 69 | 75.779 | 26.449 | 8.003 | 1.00 | 51.90 | A |
| ATOM | 571 | N | THR | A | 70 | 74.268 | 24.879 | 8.498 | 1.00 | 53.05 | A |
| ATOM | 572 | CA | THR | A | 70 | 75.234 | 23.787 | 8.324 | 1.00 | 54.84 | A |
| ATOM | 573 | CB | THR | A | 70 | 75.383 | 22.923 | 9.611 | 1.00 | 54.96 | A |
| ATOM | 574 | OG1 | THR | A | 70 | 74.280 | 22.015 | 9.735 | 1.00 | 54.48 | A |
| ATOM | 575 | CG2 | THR | A | 70 | 75.495 | 23.814 | 10.840 | 1.00 | 56.71 | A |
| ATOM | 576 | C | THR | A | 70 | 74.778 | 22.896 | 7.160 | 1.00 | 55.57 | A |
| ATOM | 577 | O | THR | A | 70 | 73.633 | 23.010 | 6.709 | 1.00 | 55.90 | A |
| ATOM | 578 | N | PRO | A | 71 | 75.674 | 22.029 | 6.630 | 1.00 | 55.72 | A |
| ATOM | 579 | CD | PRO | A | 71 | 77.115 | 21.909 | 6.922 | 1.00 | 55.61 | A |
| ATOM | 580 | CA | PRO | A | 71 | 75.306 | 21.140 | 5.518 | 1.00 | 55.36 | A |
| ATOM | 581 | CB | PRO | A | 71 | 76.479 | 20.174 | 5.464 | 1.00 | 55.09 | A |
| ATOM | 582 | CG | PRO | A | 71 | 77.620 | 21.096 | 5.745 | 1.00 | 55.44 | A |
| ATOM | 583 | C | PRO | A | 71 | 73.968 | 20.429 | 5.728 | 1.00 | 54.31 | A |
| ATOM | 584 | O | PRO | A | 71 | 73.242 | 20.192 | 4.768 | 1.00 | 55.07 | A |
| ATOM | 585 | N | LEU | A | 72 | 73.636 | 20.110 | 6.979 | 1.00 | 52.30 | A |
| ATOM | 586 | CA | LEU | A | 72 | 72.356 | 19.479 | 7.279 | 1.00 | 49.51 | A |
| ATOM | 587 | CB | LEU | A | 72 | 72.350 | 18.859 | 8.671 | 1.00 | 49.57 | A |
| ATOM | 588 | CG | LEU | A | 72 | 70.936 | 18.390 | 9.028 | 1.00 | 49.23 | A |
| ATOM | 589 | CD1 | LEU | A | 72 | 70.600 | 17.176 | 8.190 | 1.00 | 50.64 | A |
| ATOM | 590 | CD2 | LEU | A | 72 | 70.806 | 18.067 | 10.500 | 1.00 | 50.16 | A |
| ATOM | 591 | C | LEU | A | 72 | 71.247 | 20.522 | 7.214 | 1.00 | 47.98 | A |
| ATOM | 592 | O | LEU | A | 72 | 70.209 | 20.297 | 6.606 | 1.00 | 47.58 | A |
| ATOM | 593 | N | ASN | A | 73 | 71.474 | 21.658 | 7.864 | 1.00 | 47.73 | A |
| ATOM | 594 | CA | ASN | A | 73 | 70.498 | 22.742 | 7.901 | 1.00 | 47.16 | A |
| ATOM | 595 | CB | ASN | A | 73 | 70.948 | 23.843 | 8.873 | 1.00 | 45.92 | A |
| ATOM | 596 | CG | ASN | A | 73 | 71.078 | 23.342 | 10.309 | 1.00 | 45.35 | A |
| ATOM | 597 | OD1 | ASN | A | 73 | 72.047 | 22.677 | 10.668 | 1.00 | 45.25 | A |
| ATOM | 598 | ND2 | ASN | A | 73 | 70.095 | 23.654 | 11.130 | 1.00 | 45.34 | A |
| ATOM | 599 | C | ASN | A | 73 | 70.233 | 23.317 | 6.514 | 1.00 | 47.25 | A |
| ATOM | 600 | O | ASN | A | 73 | 69.128 | 23.788 | 6.248 | 1.00 | 48.96 | A |
| ATOM | 601 | N | TYR | A | 74 | 71.235 | 23.252 | 5.636 | 1.00 | 46.18 | A |
| ATOM | 602 | CA | TYR | A | 74 | 71.129 | 23.748 | 4.257 | 1.00 | 46.67 | A |
| ATOM | 603 | CB | TYR | A | 74 | 72.520 | 23.866 | 3.626 | 1.00 | 46.82 | A |
| ATOM | 604 | CG | TYR | A | 74 | 73.050 | 25.271 | 3.540 | 1.00 | 47.01 | A |
| ATOM | 605 | CD1 | TYR | A | 74 | 72.590 | 26.153 | 2.562 | 1.00 | 47.17 | A |
| ATOM | 606 | CE1 | TYR | A | 74 | 73.040 | 27.474 | 2.520 | 1.00 | 47.64 | A |
| ATOM | 607 | CD2 | TYR | A | 74 | 73.979 | 25.738 | 4.469 | 1.00 | 47.79 | A |
| ATOM | 608 | CE2 | TYR | A | 74 | 74.433 | 27.051 | 4.437 | 1.00 | 47.34 | A |
| ATOM | 609 | CZ | TYR | A | 74 | 73.960 | 27.914 | 3.467 | 1.00 | 47.78 | A |
| ATOM | 610 | OH | TYR | A | 74 | 74.394 | 29.219 | 3.467 | 1.00 | 49.58 | A |
| ATOM | 611 | C | TYR | A | 74 | 70.307 | 22.808 | 3.383 | 1.00 | 47.78 | A |
| ATOM | 612 | O | TYR | A | 74 | 69.376 | 23.227 | 2.689 | 1.00 | 47.65 | A |
| ATOM | 613 | N | ILE | A | 75 | 70.722 | 21.540 | 3.380 | 1.00 | 48.75 | A |
| ATOM | 614 | CA | ILE | A | 75 | 70.086 | 20.475 | 2.605 | 1.00 | 47.99 | A |
| ATOM | 615 | CB | ILE | A | 75 | 70.776 | 19.098 | 2.870 | 1.00 | 47.66 | A |
| ATOM | 616 | CG2 | ILE | A | 75 | 69.763 | 17.981 | 2.951 | 1.00 | 46.32 | A |
| ATOM | 617 | CG1 | ILE | A | 75 | 71.796 | 18.793 | 1.772 | 1.00 | 49.02 | A |
| ATOM | 618 | CD1 | ILE | A | 75 | 72.952 | 19.778 | 1.685 | 1.00 | 50.80 | A |
| ATOM | 619 | C | ILE | A | 75 | 68.604 | 20.378 | 2.906 | 1.00 | 47.42 | A |
| ATOM | 620 | O | ILE | A | 75 | 67.823 | 19.962 | 2.055 | 1.00 | 47.68 | A |
| ATOM | 621 | N | LEU | A | 76 | 68.221 | 20.738 | 4.124 | 1.00 | 47.15 | A |
| ATOM | 622 | CA | LEU | A | 76 | 66.818 | 20.679 | 4.485 | 1.00 | 47.88 | A |
| ATOM | 623 | CB | LEU | A | 76 | 66.631 | 20.288 | 5.949 | 1.00 | 46.29 | A |
| ATOM | 624 | CG | LEU | A | 76 | 66.540 | 18.758 | 6.002 | 1.00 | 46.02 | A |
| ATOM | 625 | CD1 | LEU | A | 76 | 67.633 | 18.161 | 6.850 | 1.00 | 45.18 | A |
| ATOM | 626 | CD2 | LEU | A | 76 | 65.164 | 18.329 | 6.468 | 1.00 | 45.07 | A |
| ATOM | 627 | C | LEU | A | 76 | 66.027 | 21.915 | 4.094 | 1.00 | 48.19 | A |
| ATOM | 628 | O | LEU | A | 76 | 64.797 | 21.903 | 4.116 | 1.00 | 49.14 | A |
| ATOM | 629 | N | LEU | A | 77 | 66.735 | 22.970 | 3.699 | 1.00 | 48.02 | A |
| ATOM | 630 | CA | LEU | A | 77 | 66.081 | 24.189 | 3.243 | 1.00 | 47.27 | A |
| ATOM | 631 | CB | LEU | A | 77 | 67.006 | 25.399 | 3.422 | 1.00 | 47.76 | A |
| ATOM | 632 | CG | LEU | A | 77 | 66.379 | 26.791 | 3.288 | 1.00 | 47.63 | A |
| ATOM | 633 | CD1 | LEU | A | 77 | 66.360 | 27.240 | 1.847 | 1.00 | 48.32 | A |
| ATOM | 634 | CD2 | LEU | A | 77 | 64.976 | 26.790 | 3.875 | 1.00 | 48.71 | A |
| ATOM | 635 | C | LEU | A | 77 | 65.802 | 23.925 | 1.768 | 1.00 | 47.10 | A |
| ATOM | 636 | O | LEU | A | 77 | 64.790 | 24.367 | 1.217 | 1.00 | 46.95 | A |
| ATOM | 637 | N | ASN | A | 78 | 66.702 | 23.146 | 1.164 | 1.00 | 46.56 | A |
| ATOM | 638 | CA | ASN | A | 78 | 66.632 | 22.736 | -0.238 | 1.00 | 45.64 | A |
| ATOM | 639 | CB | ASN | A | 78 | 67.860 | 21.861 | -0.574 | 1.00 | 47.42 | A |
| ATOM | 640 | CG | ASN | A | 78 | 67.948 | 21.473 | -2.056 | 1.00 | 47.49 | A |
| ATOM | 641 | OD1 | ASN | A | 78 | 67.505 | 22.213 | -2.938 | 1.00 | 46.97 | A |
| ATOM | 642 | ND2 | ASN | A | 78 | 68.556 | 20.318 | -2.328 | 1.00 | 46.72 | A |
| ATOM | 643 | C | ASN | A | 78 | 65.353 | 21.939 | -0.445 | 1.00 | 44.05 | A |
| ATOM | 644 | O | ASN | A | 78 | 64.573 | 22.220 | -1.350 | 1.00 | 44.31 | A |
| ATOM | 645 | N | LEU | A | 79 | 65.128 | 20.979 | 0.445 | 1.00 | 42.80 | A |
| ATOM | 646 | CA | LEU | A | 79 | 63.965 | 20.113 | 0.385 | 1.00 | 41.37 | A |
| ATOM | 647 | CB | LEU | A | 79 | 64.167 | 18.932 | 1.330 | 1.00 | 40.19 | A |
| ATOM | 648 | CG | LEU | A | 79 | 65.427 | 18.129 | 0.979 | 1.00 | 39.85 | A |
| ATOM | 649 | CD1 | LEU | A | 79 | 65.593 | 16.964 | 1.918 | 1.00 | 38.99 | A |
| ATOM | 650 | CD2 | LEU | A | 79 | 65.357 | 17.636 | -0.457 | 1.00 | 38.87 | A |
| ATOM | 651 | C | LEU | A | 79 | 62.644 | 20.834 | 0.655 | 1.00 | 42.07 | A |
| ATOM | 652 | O | LEU | A | 79 | 61.626 | 20.507 | 0.041 | 1.00 | 43.09 | A |
| ATOM | 653 | N | ALA | A | 80 | 62.661 | 21.830 | 1.541 | 1.00 | 41.12 | A |
| ATOM | 654 | CA | ALA | A | 80 | 61.454 | 22.596 | 1.851 | 1.00 | 39.86 | A |
| ATOM | 655 | CB | ALA | A | 80 | 61.736 | 23.591 | 2.954 | 1.00 | 38.62 | A |
| ATOM | 656 | C | ALA | A | 80 | 60.971 | 23.318 | 0.585 | 1.00 | 40.35 | A |
| ATOM | 657 | O | ALA | A | 80 | 59.768 | 23.401 | 0.320 | 1.00 | 41.58 | A |
| ATOM | 658 | N | VAL | A | 81 | 61.924 | 23.815 | -0.201 | 1.00 | 39.33 | A |
| ATOM | 659 | CA | VAL | A | 81 | 61.641 | 24.510 | -1.459 | 1.00 | 37.70 | A |
| ATOM | 660 | CB | VAL | A | 81 | 62.911 | 25.220 | -1.992 | 1.00 | 36.48 | A |
| ATOM | 661 | CG1 | VAL | A | 81 | 62.694 | 25.724 | -3.413 | 1.00 | 34.73 | A |
| ATOM | 662 | CG2 | VAL | A | 81 | 63.288 | 26.363 | -1.072 | 1.00 | 34.76 | A |
| ATOM | 663 | C | VAL | A | 81 | 61.125 | 23.533 | -2.523 | 1.00 | 36.93 | A |
| ATOM | 664 | O | VAL | A | 81 | 60.098 | 23.783 | -3.158 | 1.00 | 35.47 | A |
| ATOM | 665 | N | ALA | A | 82 | 61.859 | 22.436 | -2.717 | 1.00 | 36.04 | A |
| ATOM | 666 | CA | ALA | A | 82 | 61.497 | 21.408 | -3.686 | 1.00 | 35.47 | A |
| ATOM | 667 | CB | ALA | A | 82 | 62.491 | 20.252 | -3.638 | 1.00 | 33.67 | A |
| ATOM | 668 | C | ALA | A | 82 | 60.087 | 20.918 | -3.382 | 1.00 | 36.09 | A |
| ATOM | 669 | O | ALA | A | 82 | 59.315 | 20.637 | -4.307 | 1.00 | 37.02 | A |
| ATOM | 670 | N | ASP | A | 83 | 59.753 | 20.849 | -2.088 | 1.00 | 35.02 | A |
| ATOM | 671 | CA | ASP | A | 83 | 58.427 | 20.421 | -1.646 | 1.00 | 34.37 | A |
| ATOM | 672 | CB | ASP | A | 83 | 58.433 | 20.018 | -0.168 | 1.00 | 33.07 | A |
| ATOM | 673 | CG | ASP | A | 83 | 59.230 | 18.738 | 0.107 | 1.00 | 34.24 | A |
| ATOM | 674 | OD1 | ASP | A | 83 | 60.058 | 18.319 | -0.735 | 1.00 | 35.14 | A |
| ATOM | 675 | OD2 | ASP | A | 83 | 59.043 | 18.146 | 1.196 | 1.00 | 34.83 | A |
| ATOM | 676 | C | ASP | A | 83 | 57.407 | 21.543 | -1.903 | 1.00 | 35.33 | A |
| ATOM | 677 | O | ASP | A | 83 | 56.245 | 21.270 | -2.198 | 1.00 | 36.97 | A |
| ATOM | 678 | N | LEU | A | 84 | 57.839 | 22.802 | -1.811 | 1.00 | 34.83 | A |
| ATOM | 679 | CA | LEU | A | 84 | 56.941 | 23.925 | -2.085 | 1.00 | 32.53 | A |
| ATOM | 680 | CB | LEU | A | 84 | 57.518 | 25.250 | -1.570 | 1.00 | 32.03 | A |
| ATOM | 681 | CG | LEU | A | 84 | 57.497 | 25.481 | -0.054 | 1.00 | 30.64 | A |
| ATOM | 682 | CD1 | LEU | A | 84 | 58.223 | 26.754 | 0.271 | 1.00 | 29.76 | A |
| ATOM | 683 | CD2 | LEU | A | 84 | 56.077 | 25.544 | 0.463 | 1.00 | 30.26 | A |
| ATOM | 684 | C | LEU | A | 84 | 56.689 | 23.983 | -3.591 | 1.00 | 31.54 | A |
| ATOM | 685 | O | LEU | A | 84 | 55.614 | 24.375 | -4.016 | 1.00 | 31.37 | A |
| ATOM | 686 | N | PHE | A | 85 | 57.680 | 23.580 | -4.386 | 1.00 | 31.79 | A |
| ATOM | 687 | CA | PHE | A | 85 | 57.547 | 23.533 | -5.848 | 1.00 | 32.99 | A |
| ATOM | 688 | CB | PHE | A | 85 | 58.888 | 23.292 | -6.532 | 1.00 | 32.58 | A |
| ATOM | 689 | CG | PHE | A | 85 | 59.610 | 24.542 | -6.867 | 1.00 | 35.24 | A |
| ATOM | 690 | CD1 | PHE | A | 85 | 58.904 | 25.675 | -7.266 | 1.00 | 36.55 | A |
| ATOM | 691 | CD2 | PHE | A | 85 | 60.997 | 24.607 | -6.775 | 1.00 | 36.34 | A |
| ATOM | 692 | CE1 | PHE | A | 85 | 59.568 | 26.863 | -7.569 | 1.00 | 37.42 | A |
| ATOM | 693 | CE2 | PHE | A | 85 | 61.674 | 25.788 | -7.075 | 1.00 | 36.51 | A |
| ATOM | 694 | C2 | PHE | A | 85 | 60.957 | 26.920 | -7.473 | 1.00 | 37.03 | A |
| ATOM | 695 | C | PHE | A | 85 | 56.600 | 22.416 | -6.234 | 1.00 | 32.88 | A |
| ATOM | 696 | O | PHE | A | 85 | 55.899 | 22.495 | -7.240 | 1.00 | 32.01 | A |
| ATOM | 697 | N | MET | A | 86 | 56.626 | 21.345 | -5.458 | 1.00 | 32.13 | A |
| ATOM | 698 | CA | MET | A | 86 | 55.725 | 20.247 | -5.712 | 1.00 | 33.85 | A |
| ATOM | 699 | CB | MET | A | 86 | 56.061 | 19.063 | -4.802 | 1.00 | 33.64 | A |
| ATOM | 700 | CG | MET | A | 86 | 57.468 | 18.522 | -4.962 | 1.00 | 31.95 | A |
| ATOM | 701 | SD | MET | A | 86 | 57.610 | 16.797 | -4.461 | 1.00 | 29.61 | A |
| ATOM | 702 | CE | MET | A | 86 | 58.500 | 16.953 | -3.048 | 1.00 | 32.77 | A |
| ATOM | 703 | C | MET | A | 86 | 54.299 | 20.758 | -5.428 | 1.00 | 34.87 | A |
| ATOM | 704 | O | MET | A | 86 | 53.450 | 20.784 | -6.323 | 1.00 | 34.21 | A |
| ATOM | 705 | N | VAL | A | 87 | 54.087 | 21.247 | -4.204 | 1.00 | 34.00 | A |
| ATOM | 706 | CA | VAL | A | 87 | 52.792 | 21.756 | -3.761 | 1.00 | 33.71 | A |
| ATOM | 707 | CB | VAL | A | 87 | 52.908 | 22.457 | -2.379 | 1.00 | 33.26 | A |
| ATOM | 708 | CG1 | VAL | A | 87 | 51.547 | 22.935 | -1.904 | 1.00 | 31.72 | A |
| ATOM | 709 | CG2 | VAL | A | 87 | 53.503 | 21.527 | -1.357 | 1.00 | 32.17 | A |
| ATOM | 710 | C | VAL | A | 87 | 52.161 | 22.741 | -4.740 | 1.00 | 35.69 | A |
| ATOM | 711 | O | VAL | A | 87 | 50.963 | 22.695 | -4.999 | 1.00 | 35.07 | A |
| ATOM | 712 | N | PHE | A | 88 | 52.976 | 23.631 | -5.287 | 1.00 | 38.23 | A |
| ATOM | 713 | CA | PHE | A | 88 | 52.473 | 24.640 | -6.203 | 1.00 | 39.32 | A |
| ATOM | 714 | CB | PHE | A | 88 | 53.123 | 25.993 | -5.898 | 1.00 | 42.10 | A |
| ATOM | 715 | CG | PHE | A | 88 | 52.998 | 26.418 | -4.461 | 1.00 | 44.23 | A |
| ATOM | 716 | CD1 | PHE | A | 88 | 51.788 | 26.286 | -3.782 | 1.00 | 45.24 | A |
| ATOM | 717 | CD2 | PHE | A | 88 | 54.099 | 26.931 | -3.778 | 1.00 | 46.54 | A |
| ATOM | 718 | CE1 | PHE | A | 88 | 51.672 | 26.656 | -2.438 | 1.00 | 46.03 | A |
| ATOM | 719 | CE2 | PHE | A | 88 | 53.994 | 27.305 | -2.430 | 1.00 | 48.18 | A |
| ATOM | 720 | CZ | PHE | A | 88 | 52.774 | 27.164 | -1.763 | 1.00 | 46.42 | A |
| ATOM | 721 | C | PHE | A | 88 | 52.691 | 24.289 | -7.660 | 1.00 | 38.83 | A |
| ATOM | 722 | O | PHE | A | 88 | 51.797 | 24.474 | -8.477 | 1.00 | 38.64 | A |
| ATOM | 723 | N | GLY | A | 89 | 53.881 | 23.798 | -7.985 | 1.00 | 37.23 | A |
| ATOM | 724 | CA | GLY | A | 89 | 54.181 | 23.459 | -9.363 | 1.00 | 37.90 | A |
| ATOM | 725 | C | GLY | A | 89 | 53.471 | 22.238 | -9.922 | 1.00 | 38.91 | A |
| ATOM | 726 | O | GLY | A | 89 | 53.060 | 22.237 | -11.084 | 1.00 | 38.66 | A |
| ATOM | 727 | N | GLY | A | 90 | 53.333 | 21.195 | -9.108 | 1.00 | 39.29 | A |
| ATOM | 728 | CA | GLY | A | 90 | 52.683 | 19.984 | -9.575 | 1.00 | 39.04 | A |
| ATOM | 729 | C | GLY | A | 90 | 51.375 | 19.628 | -8.899 | 1.00 | 40.35 | A |
| ATOM | 730 | O | GLY | A | 90 | 50.404 | 19.302 | -9.569 | 1.00 | 39.74 | A |
| ATOM | 731 | N | PHE | A | 91 | 51.342 | 19.704 | -7.571 | 1.00 | 42.03 | A |
| ATOM | 732 | CA | PHE | A | 91 | 50.151 | 19.358 | -6.788 | 1.00 | 42.37 | A |
| ATOM | 733 | CB | PHE | A | 91 | 50.468 | 19.367 | -5.284 | 1.00 | 42.92 | A |
| ATOM | 734 | CG | PHE | A | 91 | 51.394 | 18.262 | -4.839 | 1.00 | 43.42 | A |
| ATOM | 735 | CD1 | PHE | A | 91 | 51.612 | 18.033 | -3.488 | 1.00 | 43.83 | A |
| ATOM | 736 | CD2 | PHE | A | 91 | 52.042 | 17.451 | -5.760 | 1.00 | 44.58 | A |
| ATOM | 737 | CE1 | PHE | A | 91 | 52.454 | 17.019 | -3.061 | 1.00 | 43.20 | A |
| ATOM | 738 | CE2 | PHE | A | 91 | 52.888 | 16.434 | -5.341 | 1.00 | 45.02 | A |
| ATOM | 739 | CZ | PHE | A | 91 | 53.092 | 16.219 | -3.986 | 1.00 | 44.77 | A |
| ATOM | 740 | C | PHE | A | 91 | 48.903 | 20.206 | -7.060 | 1.00 | 42.28 | A |
| ATOM | 741 | O | PHE | A | 91 | 47.777 | 19.759 | -6.826 | 1.00 | 41.30 | A |
| ATOM | 742 | N | THR | A | 92 | 49.102 | 21.443 | -7.502 | 1.00 | 41.43 | A |
| ATOM | 743 | CA | THR | A | 92 | 47.982 | 22.324 | -7.807 | 1.00 | 40.22 | A |
| ATOM | 744 | CB | THR | A | 92 | 48.466 | 23.783 | -8.073 | 1.00 | 40.87 | A |
| ATOM | 745 | OG1 | THR | A | 92 | 49.522 | 23.776 | -9.045 | 1.00 | 40.52 | A |
| ATOM | 746 | CG2 | THR | A | 92 | 48.968 | 24.435 | -6.796 | 1.00 | 40.60 | A |
| ATOM | 747 | C | THR | A | 92 | 47.297 | 21.776 | -9.060 | 1.00 | 40.35 | A |
| ATOM | 748 | O | THR | A | 92 | 46.074 | 21.781 | -9.176 | 1.00 | 38.21 | A |
| ATOM | 749 | N | THR | A | 93 | 48.118 | 21.253 | -9.969 | 1.00 | 41.56 | A |
| ATOM | 750 | CA | THR | A | 93 | 47.668 | 20.699 | -11.242 | 1.00 | 42.13 | A |
| ATOM | 751 | CB | THR | A | 93 | 48.814 | 20.782 | -12.315 | 1.00 | 44.14 | A |
| ATOM | 752 | OG1 | THR | A | 93 | 49.403 | 22.100 | -12.311 | 1.00 | 44.71 | A |
| ATOM | 753 | CG2 | THR | A | 93 | 48.276 | 20.486 | -13.707 | 1.00 | 41.76 | A |
| ATOM | 754 | C | THR | A | 93 | 47.159 | 19.252 | -11.100 | 1.00 | 42.29 | A |
| ATOM | 755 | O | THR | A | 93 | 46.405 | 18.767 | -11.949 | 1.00 | 41.39 | A |
| ATOM | 756 | N | THR | A | 94 | 47.570 | 18.564 | -10.030 | 1.00 | 42.43 | A |
| ATOM | 757 | CA | THR | A | 94 | 47.126 | 17.188 | -9.793 | 1.00 | 41.22 | A |
| ATOM | 758 | CB | THR | A | 94 | 48.145 | 16.374 | -8.967 | -1.00 | 40.39 | A |
| ATOM | 759 | OG1 | THR | A | 94 | 47.714 | 15.011 | -8.869 | 1.00 | 40.43 | A |
| ATOM | 760 | CG2 | THR | A | 94 | 48.284 | 16.929 | -7.598 | 1.00 | 41.63 | A |
| ATOM | 761 | C | THR | A | 94 | 45.763 | 17.187 | -9.105 | 1.00 | 41.66 | A |
| ATOM | 762 | O | THR | A | 94 | 44.996 | 16.229 | -9.239 | 1.00 | 41.74 | A |
| ATOM | 763 | N | LEU | A | 95 | 45.464 | 18.274 | -8.391 | 1.00 | 41.76 | A |
| ATOM | 764 | CA | LEU | A | 95 | 44.185 | 18.433 | -7.702 | 1.00 | 40.65 | A |
| ATOM | 765 | CB | LEU | A | 95 | 44.313 | 19.389 | -6.508 | 1.00 | 39.53 | A |
| ATOM | 766 | CG | LEU | A | 95 | 43.054 | 19.829 | -5.744 | 1.00 | 37.83 | A |
| ATOM | 767 | CD1 | LEU | A | 95 | 42.200 | 18.631 | -5.355 | 1.00 | 39.55 | A |
| ATOM | 768 | CD2 | LEU | A | 95 | 43.440 | 20.620 | -4.516 | 1.00 | 34.85 | A |
| ATOM | 769 | C | LEU | A | 95 | 43.094 | 18.917 | -8.654 | 1.00 | 41.21 | A |
| ATOM | 770 | O | LEU | A | 95 | 41.961 | 18.441 | -8.567 | 1.00 | 41.38 | A |
| ATOM | 771 | N | TYR | A | 96 | 43.424 | 19.851 | -9.552 | 1.00 | 41.59 | A |
| ATOM | 772 | CA | TYR | A | 96 | 42.439 | 20.366 | -10.517 | 1.00 | 43.82 | A |
| ATOM | 773 | CB | TYR | A | 96 | 43.038 | 21.514 | -11.361 | 1.00 | 45.90 | A |
| ATOM | 774 | CG | TYR | A | 96 | 42.053 | 22.290 | -12.252 | 1.00 | 49.44 | A |
| ATOM | 775 | CD1 | TYR | A | 96 | 41.608 | 23.573 | -11.899 | 1.00 | 51.05 | A |
| ATOM | 776 | CE1 | TYR | A | 96 | 40.714 | 24.300 | -12.730 | 1.00 | 52.83 | A |
| ATOM | 777 | CD2 | TYR | A | 96 | 41.585 | 21.751 | -13.459 | 1.00 | 51.30 | A |
| ATOM | 778 | CE2 | TYR | A | 96 | 40.700 | 22.465 | -14.293 | 1.00 | 52.69 | A |
| ATOM | 779 | CZ | TYR | A | 96 | 40.265 | 23.736 | -13.925 | 1.00 | 53.45 | A |
| ATOM | 780 | OH | TYR | A | 96 | 39.376 | 24.416 | -14.744 | 1.00 | 53.22 | A |
| ATOM | 781 | C | TYR | A | 96 | 42.016 | 19.196 | -11.408 | 1.00 | 44.11 | A |
| ATOM | 782 | O | TYR | A | 96 | 40.838 | 19.036 | -11.733 | 1.00 | 43.71 | A |
| ATOM | 783 | N | THR | A | 97 | 42.991 | 18.347 | -11.728 | 1.00 | 44.56 | A |
| ATOM | 784 | CA | THR | A | 97 | 42.800 | 17.159 | -12.561 | 1.00 | 44.45 | A |
| ATOM | 785 | CB | THR | A | 97 | 44.175 | 16.537 | -12.897 | 1.00 | 44.67 | A |
| ATOM | 786 | OG1 | THR | A | 97 | 44.880 | 17.414 | -13.784 | 1.00 | 43.79 | A |
| ATOM | 787 | CG2 | THR | A | 97 | 44.033 | 15.163 | -13.529 | 1.00 | 43.06 | A |
| ATOM | 788 | C | THR | A | 97 | 41.877 | 16.107 | -11.919 | 1.00 | 44.37 | A |
| ATOM | 789 | O | THR | A | 97 | 40.955 | 15.612 | -12.565 | 1.00 | 43.89 | A |
| ATOM | 790 | N | SER | A | 98 | 42.141 | 15.764 | -10.657 | 1.00 | 44.39 | A |
| ATOM | 791 | CA | SER | A | 98 | 41.330 | 14.791 | -9.928 | 1.00 | 42.28 | A |
| ATOM | 792 | CB | SER | A | 98 | 41.919 | 14.535 | -8.546 | 1.00 | 40.92 | A |
| ATOM | 793 | OG | SER | A | 98 | 43.126 | 13.809 | -8.649 | 1.00 | 39.71 | A |
| ATOM | 794 | C | SER | A | 98 | 39.912 | 15.312 | -9.786 | 1.00 | 42.68 | A |
| ATOM | 795 | O | SER | A | 98 | 38.951 | 14.550 | -9.902 | 1.00 | 44.27 | A |
| ATOM | 796 | N | LEU | A | 99 | 39.792 | 16.618 | -9.550 | 1.00 | 41.55 | A |
| ATOM | 797 | CA | LEU | A | 99 | 38.496 | 17.269 | -9.408 | 1.00 | 40.28 | A |
| ATOM | 798 | CB | LEU | A | 99 | 38.660 | 18.725 | -8.974 | 1.00 | 36.43 | A |
| ATOM | 799 | CG | LEU | A | 99 | 38.740 | 18.863 | -7.460 | 1.00 | 33.05 | A |
| ATOM | 800 | CD1 | LEU | A | 99 | 39.120 | 20.268 | -7.076 | 1.00 | 31.89 | A |
| ATOM | 801 | CD2 | LEU | A | 99 | 37.412 | 18.468 | -6.859 | 1.00 | 29.01 | A |
| ATOM | 802 | C | LEU | A | 99 | 37.667 | 17.208 | -10.673 | 1.00 | 41.12 | A |
| ATOM | 803 | O | LEU | A | 99 | 36.442 | 17.147 | -10.600 | 1.00 | 42.91 | A |
| ATOM | 804 | N | HIS | A | 100 | 38.333 | 17.192 | -11.824 | 1.00 | 41.46 | A |
| ATOM | 805 | CA | HIS | A | 100 | 37.636 | 17.142 | -13.104 | 1.00 | 42.81 | A |
| ATOM | 806 | CB | HIS | A | 100 | 38.219 | 18.175 | -14.061 | 1.00 | 43.12 | A |
| ATOM | 807 | CG | HIS | A | 100 | 77.953 | 19.586 | -13.644 | 1.00 | 44.38 | A |
| ATOM | 808 | CD2 | HIS | A | 100 | 36.985 | 20.457 | -14.017 | 1.00 | 45.57 | A |
| ATOM | 809 | ND1 | HIS | A | 100 | 38.716 | 20.242 | -12.704 | 1.00 | 44.62 | A |
| ATOM | 810 | CE1 | HIS | A | 100 | 38.230 | 21.456 | -12.515 | 1.00 | 46.31 | A |
| ATOM | 811 | NE2 | HIS | A | 100 | 37.180 | 21.612 | -13.300 | 1.00 | 46.65 | A |
| ATOM | 812 | C | HIS | A | 100 | 37.521 | 15.780 | -13.790 | 1.00 | 43.64 | A |
| ATOM | 813 | O | HIS | A | 100 | 36.628 | 15.594 | -14.628 | 1.00 | 44.29 | A |
| ATOM | 814 | N | GLY | A | 101 | 38.418 | 14.849 | -13.446 | 1.00 | 44.10 | A |
| ATOM | 815 | CA | GLY | A | 101 | 38.401 | 13.506 | -14.024 | 1.00 | 44.28 | A |
| ATOM | 816 | C | GLY | A | 101 | 39.430 | 13.175 | -15.101 | 1.00 | 44.85 | A |
| ATOM | 817 | O | GLY | A | 101 | 39.503 | 12.035 | -15.571 | 1.00 | 44.25 | A |
| ATOM | 818 | N | TYR | A | 102 | 40.230 | 14.168 | -15.486 | 1.00 | 46.41 | A |
| ATOM | 819 | CA | TYR | A | 102 | 41.264 | 14.019 | -16.516 | 1.00 | 47.26 | A |
| ATOM | 820 | CB | TYR | A | 102 | 40.616 | 13.845 | -17.900 | 1.00 | 50.02 | A |
| ATOM | 821 | CG | TYR | A | 102 | 39.918 | 15.093 | -18.433 | 1.00 | 53.62 | A |
| ATOM | 822 | CD1 | TYR | A | 102 | 38.796 | 15.631 | -17.788 | 1.00 | 54.12 | A |
| ATOM | 823 | CE1 | TYR | A | 102 | 38.186 | 16.795 | -18.248 | 1.00 | 54.03 | A |
| ATOM | 824 | CD2 | TYR | A | 102 | 40.404 | 15.756 | -19.562 | 1.00 | 54.85 | A |
| ATOM | 825 | CE2 | TYR | A | 102 | 39.798 | 16.921 | -20.031 | 1.00 | 56.05 | A |
| ATOM | 826 | CZ | TYR | A | 102 | 38.694 | 17.435 | -19.368 | 1.00 | 55.22 | A |
| ATOM | 827 | OH | TYR | A | 102 | 38.117 | 18.596 | -19.825 | 1.00 | 56.48 | A |
| ATOM | 828 | C | TYR | A | 102 | 42.156 | 15.268 | -16.518 | 1.00 | 46.53 | A |
| ATOM | 829 | O | TYR | A | 102 | 41.977 | 16.165 | -15.695 | 1.00 | 46.56 | A |
| ATOM | 830 | N | PHE | A | 103 | 43.107 | 15.329 | -17.444 | 1.00 | 45.77 | A |
| ATOM | 831 | CA | PHE | A | 103 | 44.004 | 16.474 | -17.541 | 1.00 | 46.41 | A |
| ATOM | 832 | CB | PHE | A | 103 | 45.375 | 16.036 | -18.060 | 1.00 | 44.21 | A |
| ATOM | 833 | CG | PHE | A | 103 | 46.234 | 15.362 | -17.018 | 1.00 | 44.01 | A |
| ATOM | 834 | CD1 | PHE | A | 103 | 46.389 | 13.978 | -17.008 | 1.00 | 43.52 | A |
| ATOM | 835 | CD2 | PHE | A | 103 | 46.887 | 16.113 | -16.045 | 1.00 | 42.31 | A |
| ATOM | 836 | CE1 | PHE | A | 103 | 47.181 | 13.356 | -16.043 | 1.00 | 43.72 | A |
| ATOM | 837 | CE2 | PHE | A | 103 | 47.676 | 15.499 | -15.082 | 1.00 | 42.38 | A |
| ATOM | 838 | CZ | PHE | A | 103 | 47.824 | 14.120 | -15.080 | 1.00 | 42.24 | A |
| ATOM | 839 | C | PHE | A | 103 | 43.414 | 17.580 | -18.416 | 1.00 | 48.51 | A |
| ATOM | 840 | O | PHE | A | 103 | 43.589 | 17.588 | -19.631 | 1.00 | 49.24 | A |
| ATOM | 841 | N | VAL | A | 104 | 42.716 | 18.515 | -17.779 | 1.00 | 51.21 | A |
| ATOM | 842 | CA | VAL | A | 104 | 42.071 | 19.632 | -18.471 | 1.00 | 53.34 | A |
| ATOM | 843 | CB | VAL | A | 104 | 41.175 | 20.478 | -17.491 | 1.00 | 52.03 | A |
| ATOM | 844 | CG1 | VAL | A | 104 | 40.562 | 21.681 | -18.200 | 1.00 | 50.45 | A |
| ATOM | 845 | CG2 | VAL | A | 104 | 40.067 | 19.611 | -16.912 | 1.00 | 49.70 | A |
| ATOM | 846 | C | VAL | A | 104 | 43.071 | 20.527 | -19.207 | 1.00 | 56.12 | A |
| ATOM | 847 | O | VAL | A | 104 | 42.859 | 20.849 | -20.378 | 1.00 | 57.69 | A |
| ATOM | 848 | N | PHE | A | 105 | 44.166 | 20.904 | -18.542 | 1.00 | 58.57 | A |
| ATOM | 849 | CA | PHE | A | 105 | 45.186 | 21.763 | -19.164 | 1.00 | 60.74 | A |
| ATOM | 850 | CB | PHE | A | 105 | 46.106 | 22.401 | -18.113 | 1.00 | 63.88 | A |
| ATOM | 851 | CG | PHE | A | 105 | 45.377 | 23.218 | -17.086 | 1.00 | 67.84 | A |
| ATOM | 852 | CD1 | PHE | A | 105 | 44.237 | 23.949 | -17.437 | 1.00 | 69.17 | A |
| ATOM | 853 | CD2 | PHE | A | 105 | 45.805 | 23.230 | -15.760 | 1.00 | 69.65 | A |
| ATOM | 854 | CE1 | PHE | A | 105 | 43.530 | 24.677 | -16.484 | 1.00 | 70.72 | A |
| ATOM | 855 | CE2 | PHE | A | 105 | 45.107 | 23.955 | -14.793 | 1.00 | 71.79 | A |
| ATOM | 856 | CZ | PHE | A | 105 | 43.965 | 24.680 | -15.155 | 1.00 | 71.73 | A |
| ATOM | 857 | C | PHE | A | 105 | 46.018 | 20.998 | -20.186 | 1.00 | 60.31 | A |
| ATOM | 858 | 0 | PHE | A | 105 | 47.000 | 21.524 | -20.716 | 1.00 | 61.32 | A |
| ATOM | 859 | N | GLY | A | 106 | 45.622 | 19.752 | -20.437 | 1.00 | 58.66 | A |
| ATOM | 860 | CA | GLY | A | 106 | 46.301 | 18.912 | -21.403 | 1.00 | 57.21 | A |
| ATOM | 861 | C | GLY | A | 106 | 47.705 | 18.469 | -21.051 | 1.00 | 56.46 | A |
| ATOM | 862 | 0 | GLY | A | 106 | 48.035 | 18.294 | -19.881 | 1.00 | 55.78 | A |
| ATOM | 863 | N | PRO | A | 107 | 48.562 | 18.289 | -22.068 | 1.00 | 56.71 | A |
| ATOM | 864 | CD | PRO | A | 107 | 48.217 | 18.553 | -23.475 | 1.00 | 57.95 | A |
| ATOM | 865 | CA | PRO | A | 107 | 49.959 | 17.861 | -21.960 | 1.00 | 56.58 | A |
| ATOM | 866 | CB | PRO | A | 107 | 50.435 | 17.878 | -23.417 | 1.00 | 57.59 | A |
| ATOM | 867 | CG | PRO | A | 107 | 49.553 | 18.906 | -24.063 | 1.00 | 58.81 | A |
| ATOM | 868 | C | PRO | A | 107 | 50.870 | 18.666 | -21.022 | 1.00 | 55.43 | A |
| ATOM | 869 | O | PRO | A | 107 | 51.751 | 18.077 | -20.389 | 1.00 | 56.43 | A |
| ATOM | 870 | N | THR | A | 108 | 50.688 | 19.989 | -20.938 | 1.00 | 53.46 | A |
| ATOM | 871 | CA | THR | A | 108 | 51.511 | 20.803 | -20.027 | 1.00 | 51.49 | A |
| ATOM | 872 | CB | THR | A | 108 | 51.489 | 22.310 | -20.377 | 1.00 | 49.95 | A |
| ATOM | 873 | OG1 | THR | A | 108 | 50.154 | 22.805 | -20.276 | 1.00 | 50.80 | A |
| ATOM | 874 | CG2 | THR | A | 108 | 52.002 | 22.545 | -21.792 | 1.00 | 49.32 | A |
| ATOM | 875 | C | THR | A | 108 | 51.036 | 20.572 | -18.585 | 1.00 | 50.69 | A |
| ATOM | 876 | O | THR | A | 108 | 51.815 | 20.688 | -17.639 | 1.00 | 50.81 | A |
| ATOM | 877 | N | GLY | A | 109 | 49.757 | 20.212 | -18.440 | 1.00 | 49.99 | A |
| ATOM | 878 | CA | GLY | A | 109 | 49.189 | 19.899 | -17.137 | 1.00 | 48.28 | A |
| ATOM | 879 | C | GLY | A | 109 | 49.767 | 18.570 | -16.668 | 1.00 | 47.15 | A |
| ATOM | 880 | O | GLY | A | 109 | 49.821 | 18.276 | -15.470 | 1.00 | 47.93 | A |
| ATOM | 881 | N | CYS | A | 110 | 50.181 | 17.760 | -17.640 | 1.00 | 45.08 | A |
| ATOM | 882 | CA | CYS | A | 110 | 50.800 | 16.464 | -17.405 | 1.00 | 43.45 | A |
| ATOM | 883 | C | CYS | A | 110 | 52.284 | 16.697 | -17.160 | 1.00 | 43.88 | A |
| ATOM | 884 | O | CYS | A | 110 | 52.908 | 15.997 | -16.365 | 1.00 | 44.64 | A |
| ATOM | 885 | CB | CYS | A | 110 | 50.638 | 15.569 | -18.635 | 1.00 | 43.20 | A |
| ATOM | 886 | SG | CYS | A | 110 | 51.327 | 13.892 | -18.465 | 1.00 | 39.51 | A |
| ATOM | 887 | N | ASN | A | 111 | 52.846 | 17.684 | -17.854 | 1.00 | 42.78 | A |
| ATOM | 888 | CA | ASN | A | 111 | 54.256 | 18.009 | -17.711 | 1.00 | 41.71 | A |
| ATOM | 889 | CB | ASN | A | 111 | 54.730 | 18.915 | -18.861 | 1.00 | 42.08 | A |
| ATOM | 890 | CG | ASN | A | 111 | 54.972 | 18.148 | -20.182 | 1.00 | 41.94 | A |
| ATOM | 891 | OD1 | ASN | A | 111 | 55.127 | 16.923 | -20.205 | 1.00 | 41.75 | A |
| ATOM | 892 | ND2 | ASN | A | 111 | 55.034 | 18.890 | -21.282 | 1.00 | 40.24 | A |
| ATOM | 893 | C | ASN | A | 111 | 54.563 | 18.640 | -16.350 | 1.00 | 41.09 | A |
| ATOM | 894 | O | ASM | A | 111 | 55.552 | 18.278 | -15.721 | 1.00 | 42.02 | A |
| ATOM | 895 | N | LEU | A | 112 | 53.697 | 19.536 | -15.872 | 1.00 | 39.39 | A |
| ATOM | 896 | CA | LEU | A | 112 | 53.908 | 20.195 | -14.577 | 1.00 | 39.01 | A |
| ATOM | 897 | CB | LEU | A | 112 | 52.983 | 21.402 | -14.414 | 1.00 | 38.71 | A |
| ATOM | 898 | CG | LEU | A | 112 | 53.219 | 22.658 | -15.270 | 1.00 | 39.71 | A |
| ATOM | 899 | CD1 | LEU | A | 112 | 51.982 | 23.547 | -15.131 | 1.00 | 37.52 | A |
| ATOM | 900 | CD2 | LEU | A | 112 | 54.529 | 23.407 | -14.897 | 1.00 | 34.31 | A |
| ATOM | 901 | C | LEU | A | 112 | 53.676 | 19.245 | -13.421 | 1.00 | 39.07 | A |
| ATOM | 902 | O | LEU | A | 112 | 54.352 | 19.315 | -12.395 | 1.00 | 39.21 | A |
| ATOM | 903 | N | GLU | A | 113 | 52.681 | 18.383 | -13.598 | 1.00 | 40.42 | A |
| ATOM | 904 | CA | GLU | A | 113 | 52.289 | 17.378 | -12.613 | 1.00 | 40.49 | A |
| ATOM | 905 | CB | GLU | A | 113 | 50.971 | 16.727 | -13.054 | 1.00 | 39.94 | A |
| ATOM | 906 | CG | GLU | A | 113 | 50.087 | 16.224 | -11.931 | 1.00 | 42.39 | A |
| ATOM | 907 | CD | GLU | A | 113 | 50.738 | 15.146 | -11.091 | 1.00 | 43.87 | A |
| ATOM | 908 | OE1 | GLU | A | 113 | 50.944 | 15.381 | -9.877 | 1.00 | 45.43 | A |
| ATOM | 909 | OE2 | GLU | A | 113 | 51.047 | 14.069 | -11.645 | 1.00 | 43.84 | A |
| ATOM | 910 | C | GLU | A | 113 | 53.392 | 16.319 | -12.491 | 1.00 | 40.15 | A |
| ATOM | 911 | O | GLU | A | 113 | 53.760 | 15.914 | -11.384 | 1.00 | 39.65 | A |
| ATOM | 912 | N | GLY | A | 114 | 53.913 | 15.884 | -13.637 | 1.00 | 39.92 | A |
| ATOM | 913 | CA | GLY | A | 114 | 54.973 | 14.891 | -13.658 | 1.00 | 39.76 | A |
| ATOM | 914 | C | GLY | A | 114 | 56.341 | 15.476 | -13.362 | 1.00 | 39.98 | A |
| ATOM | 915 | O | GLY | A | 114 | 57.189 | 14.800 | -12.772 | 1.00 | 38.97 | A |
| ATOM | 916 | N | PHE | A | 115 | 56.547 | 16.738 | -13.745 | 1.00 | 39.24 | A |
| ATOM | 917 | CA | PHE | A | 115 | 57.819 | 17.407 | -13.522 | 1.00 | 38.76 | A |
| ATOM | 918 | CB | PHE | A | 115 | 57.884 | 18.731 | -14.267 | 1.00 | 37.99 | A |
| ATOM | 919 | CG | PHE | A | 115 | 59.234 | 19.361 | -14.230 | 1.00 | 38.11 | A |
| ATOM | 920 | CD1 | PHE | A | 115 | 59.457 | 20.514 | -13.496 | 1.00 | 38.97 | A |
| ATOM | 921 | CD2 | PHE | A | 115 | 60.301 | 18.769 | -14.888 | 1.00 | 38.23 | A |
| ATOM | 922 | CE1 | PHE | A | 115 | 60.729 | 21.068 | -13.412 | 1.00 | 39.54 | A |
| ATOM | 923 | CE2 | PHE | A | 115 | 61.579 | 19.311 | -14.813 | 1.00 | 37.99 | A |
| ATOM | 924 | CZ | PHE | A | 115 | 61.794 | 20.462 | -14.074 | 1.00 | 39.71 | A |
| ATOM | 925 | C | PHE | A | 115 | 58.154 | 17.645 | -12.059 | 1.00 | 39.22 | A |
| ATOM | 926 | O | PHE | A | 115 | 59.126 | 17.096 | -11.547 | 1.00 | 40.44 | A |
| ATOM | 927 | N | PHE | A | 116 | 57.348 | 18.460 | -11.388 | 1.00 | 39.85 | A |
| ATOM | 928 | CA | PHE | A | 116 | 57.577 | 18.786 | -9.978 | 1.00 | 40.68 | A |
| ATOM | 929 | CB | PHE | A | 116 | 56.789 | 20.042 | -9.592 | 1.00 | 39.22 | A |
| ATOM | 930 | CG | PHE | A | 116 | 57.143 | 21.246 | -10.404 | 1.00 | 37.35 | A |
| ATOM | 931 | CD1 | PHE | A | 116 | 58.335 | 21.924 | -10.179 | 1.00 | 37.47 | A |
| ATOM | 932 | CD2 | PHE | A | 116 | 56.285 | 21.705 | -11.397 | 1.00 | 37.45 | A |
| ATOM | 933 | CE1 | PHE | A | 116 | 58.668 | 23.044 | -10.930 | 1.00 | 36.62 | A |
| ATOM | 934 | CE2 | PHE | A | 116 | 56.606 | 22.824 | -12.155 | 1.00 | 36.50 | A |
| ATOM | 935 | CZ | PHE | A | 116 | 57.801 | 23.494 | -11.920 | 1.00 | 36.88 | A |
| ATOM | 936 | C | PHE | A | 116 | 57.313 | 17.659 | -8.966 | 1.00 | 41.90 | A |
| ATOM | 937 | O | PHE | A | 116 | 57.163 | 17.925 | -7.770 | 1.00 | 42.64 | A |
| ATOM | 938 | N | ALA | A | 117 | 57.187 | 16.424 | -9.451 | 1.00 | 41.70 | A |
| ATOM | 939 | CA | ALA | A | 117 | 56.986 | 15.263 | -8.584 | 1.00 | 41.89 | A |
| ATOM | 940 | CB | ALA | A | 117 | 55.775 | 14.468 | -9.006 | 1.00 | 41.09 | A |
| ATOM | 941 | C | ALA | A | 117 | 58.254 | 14.452 | -8.771 | 1.00 | 42.58 | A |
| ATOM | 942 | O | ALA | A | 117 | 58.754 | 13.817 | -7.843 | 1.00 | 42.60 | A |
| ATOM | 943 | N | THR | A | 118 | 58.775 | 14.510 | -9.993 | 1.00 | 44.00 | A |
| ATOM | 944 | CA | THR | A | 118 | 60.016 | 13.838 | -10.352 | 1.00 | 44.87 | A |
| ATOM | 945 | CB | THR | A | 118 | 60.158 | 13.685 | -11.873 | 1.00 | 45.75 | A |
| ATOM | 946 | OG1 | THR | A | 118 | 59.132 | 12.813 | -12.360 | 1.00 | 47.91 | A |
| ATOM | 947 | CG2 | THR | A | 118 | 61.521 | 13.103 | -12.231 | 1.00 | 47.03 | A |
| ATOM | 948 | C | THR | A | 118 | 61.155 | 14.707 | -9.841 | 1.00 | 43.79 | A |
| ATOM | 949 | O | THR | A | 118 | 62.169 | 14.202 | -9.377 | 1.00 | 43.22 | A |
| ATOM | 950 | N | LEU | A | 119 | 60.970 | 16.020 | -9.928 | 1.00 | 43.55 | A |
| ATOM | 951 | CA | LEU | A | 119 | 61.970 | 16.963 | -9.460 | 1.00 | 43.92 | A |
| ATOM | 952 | CB | LEU | A | 119 | 61.580 | 18.398 | -9.821 | 1.00 | 44.11 | A |
| ATOM | 953 | CG | LEU | A | 119 | 62.616 | 19.481 | -9.496 | 1.00 | 44.08 | A |
| ATOM | 954 | CD1 | LEU | A | 119 | 63.861 | 19.242 | -10.321 | 1.00 | 45.02 | A |
| ATOM | 955 | CD2 | LEU | A | 119 | 62.067 | 20.865 | -9.780 | 1.00 | 44.39 | A |
| ATOM | 956 | C | LEU | A | 119 | 62.119 | 16.840 | -7.949 | 1.00 | 44.44 | A |
| ATOM | 957 | O | LEU | A | 119 | 63.222 | 16.651 | -7.456 | 1.00 | 45.27 | A |
| ATOM | 958 | N | GLY | A | 120 | 61.006 | 16.902 | -7.222 | 1.00 | 43.91 | A |
| ATOM | 959 | CA | GLY | A | 120 | 61.058 | 16.812 | -5.770 | 1.00 | 43.51 | A |
| ATOM | 960 | C | GLY | A | 120 | 61.509 | 15.470 | -5.220 | 1.00 | 43.14 | A |
| ATOM | 961 | O | GLY | A | 120 | 62.075 | 15.400 | -4.124 | 1.00 | 42.60 | A |
| ATOM | 962 | N | GLY | A | 121 | 61.220 | 14.405 | -5.963 | 1.00 | 42.33 | A |
| ATOM | 963 | CA | GLY | A | 121 | 61.610 | 13.072 | -5.542 | 1.00 | 42.84 | A |
| ATOM | 964 | C | GLY | A | 121 | 63.094 | 12.855 | -5.733 | 1.00 | 42.59 | A |
| ATOM | 965 | O | GLY | A | 121 | 63.727 | 12.143 | -4.956 | 1.00 | 42.51 | A |
| ATOM | 966 | N | GLU | A | 122 | 63.631 | 13.471 | -6.786 | 1.00 | 43.17 | A |
| ATOM | 967 | CA | GLU | A | 122 | 65.054 | 13.415 | -7.135 | 1.00 | 42.89 | A |
| ATOM | 968 | CB | GLU | A | 122 | 65.231 | 13.571 | -8.649 | 1.00 | 43.42 | A |
| ATOM | 969 | CG | GLU | A | 122 | 64.575 | 12.480 | -9.485 | 1.00 | 44.12 | A |
| ATOM | 970 | CD | GLU | A | 122 | 65.295 | 11.146 | -9.412 | 1.00 | 46.25 | A |
| ATOM | 971 | OE1 | GLU | A | 122 | 66.472 | 11.104 | -8.988 | 1.00 | 45.99 | A |
| ATOM | 972 | OE2 | GLU | A | 122 | 64.681 | 10.129 | -9.798 | 1.00 | 47.00 | A |
| ATOM | 973 | C | GLU | A | 122 | 65.895 | 14.481 | -6.387 | 1.00 | 42.37 | A |
| ATOM | 974 | O | GLU | A | 122 | 67.080 | 14.268 | -6.138 | 1.00 | 42.61 | A |
| ATOM | 975 | N | ILE | A | 123 | 65.308 | 15.643 | -6.084 | 1.00 | 41.53 | A |
| ATOM | 976 | CA | ILE | A | 123 | 66.013 | 16.684 | -5.324 | 1.00 | 39.38 | A |
| ATOM | 977 | CB | ILE | A | 123 | 65.111 | 17.928 | -5.028 | 1.00 | 39.04 | A |
| ATOM | 978 | CG2 | ILE | A | 123 | 65.725 | 18.788 | -3.941 | 1.00 | 38.49 | A |
| ATOM | 979 | CG1 | ILE | A | 123 | 64.855 | 18.769 | -6.283 | 1.00 | 37.46 | A |
| ATOM | 980 | CD1 | ILE | A | 123 | 66.031 | 19.537 | -6.783 | 1.00 | 37.06 | A |
| ATOM | 981 | C | ILE | A | 123 | 66.303 | 16.022 | -3.980 | 1.00 | 40.19 | A |
| ATOM | 982 | O | ILE | A | 123 | 67.350 | 16.244 | -3.379 | 1.00 | 40.05 | A |
| ATOM | 983 | N | ALA | A | 124 | 65.351 | 15.201 | -3.528 | 1.00 | 40.59 | A |
| ATOM | 984 | CA | ALA | A | 124 | 65.454 | 14.480 | -2.265 | 1.00 | 41.56 | A |
| ATOM | 985 | CB | ALA | A | 124 | 64.094 | 13.946 | -1.857 | 1.00 | 41.40 | A |
| ATOM | 986 | C | ALA | A | 124 | 66.477 | 13.344 | -2.325 | 1.00 | 42.51 | A |
| ATOM | 987 | O | ALA | A | 124 | 67.353 | 13.253 | -1.464 | 1.00 | 43.06 | A |
| ATOM | 988 | N | LEU | A | 125 | 66.377 | 12.506 | -3.358 | 1.00 | 42.91 | A |
| ATOM | 989 | CA | LEU | A | 125 | 67.288 | 11.365 | -3.556 | 1.00 | 42.91 | A |
| ATOM | 990 | CB | LEU | A | 125 | 67.018 | 10.700 | -4.915 | 1.00 | 42.56 | A |
| ATOM | 991 | CG | LEU | A | 125 | 67.337 | 9.219 | -5.183 | 1.00 | 42.79 | A |
| ATOM | 992 | CD1 | LEU | A | 125 | 67.796 | 9.078 | -6.629 | 1.00 | 43.04 | A |
| ATOM | 993 | CD2 | LEU | A | 125 | 68.381 | 8.642 | -4.228 | 1.00 | 41.68 | A |
| ATOM | 994 | C | LEU | A | 125 | 68.763 | 11.771 | -3.506 | 1.00 | 42.92 | A |
| ATOM | 995 | O | LEU | A | 125 | 69.556 | 11.177 | -2.778 | 1.00 | 43.01 | A |
| ATOM | 996 | N | TRP | A | 126 | 69.121 | 12.763 | -4.315 | 1.00 | 43.00 | A |
| ATOM | 997 | CA | TRP | A | 126 | 70.486 | 13.250 | -4.393 | 1.00 | 42.68 | A |
| ATOM | 998 | CB | TRP | A | 126 | 70.637 | 14.144 | -5.614 | 1.00 | 43.60 | A |
| ATOM | 999 | CG | TRP | A | 126 | 70.566 | 13.362 | -6.879 | 1.00 | 45.80 | A |
| ATOM | 1000 | CD2 | TRP | A | 126 | 71.590 | 12.521 | -7.422 | 1.00 | 46.59 | A |
| ATOM | 1001 | CE2 | TRP | A | 126 | 71.078 | 11.951 | -8.610 | 1.00 | 47.47 | A |
| ATOM | 1002 | CE3 | TRP | A | 126 | 72.890 | 12.191 | -7.020 | 1.00 | 47.05 | A |
| ATOM | 1003 | CD1 | TRP | A | 126 | 269.507 | 13.275 | -7.736 | 1.00 | 47.93 | A |
| ATOM | 1004 | NE1 | TRP | A | 126 | 69.805 | 12.428 | -8.780 | 1.00 | 47.55 | A |
| ATOM | 1005 | CZ2 | TRP | A | 126 | 71.820 | 11.069 | -9.396 | 1.00 | 47.93 | A |
| ATOM | 1006 | CZ3 | TRP | A | 126 | 73.626 | 11.317 | -7.800 | 1.00 | 48.23 | A |
| ATOM | 1007 | CH2 | TRP | A | 126 | 73.088 | 10.764 | -8.978 | 1.00 | 49.28 | A |
| ATOM | 1008 | C | TRP | A | 126 | 70.938 | 13.957 | -3.128 | 1.00 | 42.98 | A |
| ATOM | 1009 | O | TRP | A | 126 | 72.111 | 13.891 | -2.759 | 1.00 | 41.57 | A |
| ATOM | 1010 | N | SER | A | 127 | 70.003 | 14.624 | -2.457 | 1.00 | 43.61 | A |
| ATOM | 1011 | CA | SER | A | 127 | 70.318 | 15.307 | -1.214 | 1.00 | 44.22 | A |
| ATOM | 1012 | CB | SER | A | 127 | 69.098 | 16.053 | -0.684 | 1.00 | 42.67 | A |
| ATOM | 1013 | OG | SER | A | 127 | 68.883 | 17.245 | -1.417 | 1.00 | 41.49 | A |
| ATOM | 1014 | C | SER | A | 127 | 70.810 | 14.266 | -0.207 | 1.00 | 45.89 | A |
| ATOM | 1015 | O | SER | A | 127 | 71.751 | 14.519 | 0.553 | 1.00 | 46.53 | A |
| ATOM | 1016 | N | LEU | A | 128 | 70.197 | 13.082 | -0.245 | 1.00 | 46.81 | A |
| ATOM | 1017 | CA | LEU | A | 128 | 70.574 | 11.967 | 0.626 | 1.00 | 48.00 | A |
| ATOM | 1018 | CB | LEU | A | 128 | 69.665 | 10.767 | 0.382 | 1.00 | 46.81 | A |
| ATOM | 1019 | CG | LEU | A | 128 | 68.258 | 10.847 | 0.950 | 1.00 | 46.18 | A |
| ATOM | 1020 | CD1 | LEU | A | 128 | 67.507 | 9.580 | 0.597 | 1.00 | 46.62 | A |
| ATOM | 1021 | CD2 | LEU | A | 128 | 68.334 | 11.019 | 2.454 | 1.00 | 46.13 | A |
| ATOM | 1022 | C | LEU | A | 128 | 72.021 | 11.533 | 0.390 | 1.00 | 49.70 | A |
| ATOM | 1023 | O | LEU | A | 128 | 72.674 | 11.019 | 1.298 | 1.00 | 49.91 | A |
| ATOM | 1024 | N | VAL | A | 129 | 72.495 | 11.694 | -0.846 | 1.00 | 50.97 | A |
| ATOM | 1025 | CA | VAL | A | 129 | 73.866 | 11.338 | -1.194 | 1.00 | 51.62 | A |
| ATOM | 1026 | CB | VAL | A | 129 | 74.058 | 11.182 | -2.735 | 1.00 | 50.44 | A |
| ATOM | 1027 | CG1 | VAL | A | 129 | 75.419 | 10.594 | -3.037 | 1.00 | 49.05 | A |
| ATOM | 1028 | CG2 | VAL | A | 129 | 72.978 | 10.289 | -3.322 | 1.00 | 49.45 | A |
| ATOM | 1029 | C | VAL | A | 129 | 74.788 | 12.433 | -0.628 | 1.00 | 52.92 | A |
| ATOM | 1030 | O | VAL | A | 129 | 75.797 | 12.126 | 0.016 | 1.00 | 55.01 | A |
| ATOM | 1031 | N | VAL | A | 130 | 74.418 | 13.701 | -0.822 | 1.00 | 51.50 | A |
| ATOM | 1032 | CA | VAL | A | 130 | 75.207 | 14.817 | -0.295 | 1.00 | 50.32 | A |
| ATOM | 1033 | CB | VAL | A | 130 | 74.590 | 16.175 | -0.684 | 1.00 | 50.38 | A |
| ATOM | 1034 | CG1 | VAL | A | 130 | 75.255 | 17.319 | 0.082 | 1.00 | 49.76 | A |
| ATOM | 1035 | CG2 | VAL | A | 130 | 74.725 | 16.388 | -2.173 | 1.00 | 50.09 | A |
| ATOM | 1036 | C | VAL | A | 130 | 75.261 | 14.706 | 1.228 | 1.00 | 50.42 | A |
| ATOM | 1037 | O | VAL | A | 130 | 76.241 | 15.107 | 1.857 | 1.00 | 50.44 | A |
| ATOM | 1038 | N | LEU | A | 131 | 74.193 | 14.166 | 1.810 | 1.00 | 49.76 | A |
| ATOM | 1039 | CA | LEU | A | 131 | 74.121 | 13.981 | 3.249 | 1.00 | 50.08 | A |
| ATOM | 1040 | CB | LEU | A | 131 | 72.667 | 13.949 | 3.724 | 1.00 | 49.85 | A |
| ATOM | 1041 | CG | LEU | A | 131 | 71.911 | 15.286 | 3.779 | 1.00 | 48.67 | A |
| ATOM | 1042 | CD1 | LEU | A | 131 | 70.462 | 15.024 | 4.144 | 1.00 | 47.91 | A |
| ATOM | 1043 | CD2 | LEU | A | 131 | 72.541 | 16.225 | 4.796 | 1.00 | 47.18 | A |
| ATOM | 1044 | C | LEU | A | 131 | 74.862 | 12.711 | 3.656 | 1.00 | 50.12 | A |
| ATOM | 1045 | O | LEU | A | 131 | 75.578 | 12.709 | 4.652 | 1.00 | 51.40 | A |
| ATOM | 1046 | N | ALA | A | 132 | 74.707 | 11.645 | 2.876 | 1.00 | 49.33 | A |
| ATOM | 1047 | CA | ALA | A | 132 | 75.400 | 10.390 | 3.151 | 1.00 | 49.78 | A |
| ATOM | 1048 | CB | ALA | A | 132 | 75.027 | 9.334 | 2.117 | 1.00 | 49.70 | A |
| ATOM | 1049 | C | ALA | A | 132 | 76.904 | 10.668 | 3.108 | 1.00 | 51.00 | A |
| ATOM | 1050 | O | ALA | A | 132 | 77.673 | 10.103 | 3.891 | 1.00 | 51.90 | A |
| ATOM | 1051 | N | ILE | A | 133 | 77.305 | 11.550 | 2.191 | 1.00 | 50.84 | A |
| ATOM | 1052 | CA | ILE | A | 133 | 78.700 | 11.965 | 2.038 | 1.00 | 50.70 | A |
| ATOM | 1053 | CB | ILE | A | 133 | 78.876 | 12.906 | 0.808 | 1.00 | 49.05 | A |
| ATOM | 1054 | CG2 | ILE | A | 133 | 80.209 | 13.656 | 0.878 | 1.00 | 46.10 | A |
| ATOM | 1055 | CG1 | ILE | A | 133 | 78.752 | 12.110 | -0.492 | 1.00 | 48.51 | A |
| ATOM | 1056 | CD1 | ILE | A | 133 | 78.777 | 12.975 | -1.750 | 1.00 | 48.56 | A |
| ATOM | 1057 | C | ILE | A | 133 | 79.095 | 12.738 | 3.292 | 1.00 | 51.38 | A |
| ATOM | 1058 | O | ILE | A | 133 | 79.979 | 12.328 | 4.043 | 1.00 | 49.06 | A |
| ATOM | 1059 | N | GLU | A | 134 | 78.375 | 13.830 | 3.527 | 1.00 | 54.22 | A |
| ATOM | 1060 | CA | GLU | A | 134 | 78.598 | 14.711 | 4.662 | 1.00 | 57.63 | A |
| ATOM | 1061 | CB | GLU | A | 134 | 77.478 | 15.758 | 4.731 | 1.00 | 59.13 | A |
| ATOM | 1062 | CG | GLU | A | 134 | 77.712 | 16.912 | 5.708 | 1.00 | 60.64 | A |
| ATOM | 1063 | CD | GLU | A | 134 | 77.230 | 16.626 | 7.127 | 1.00 | 61.53 | A |
| ATOM | 1064 | OE1 | GLU | A | 134 | 76.365 | 15.738 | 7.308 | 1.00 | 60.11 | A |
| ATOM | 1065 | OE2 | GLU | A | 134 | 77.711 | 17.305 | 8.063 | 1.00 | 62.89 | A |
| ATOM | 1066 | C | GLU | A | 134 | 78.710 | 13.935 | 5.970 | 1.00 | 59.04 | A |
| ATOM | 1067 | O | GLU | A | 134 | 79.322 | 14.405 | 6.927 | 1.00 | 59.58 | A |
| ATOM | 1068 | N | ARG | A | 135 | 78.135 | 12.741 | 6.014 | 1.00 | 60.21 | A |
| ATOM | 1069 | CA | ARG | A | 135 | 78.234 | 11.945 | 7.220 | 1.00 | 62.70 | A |
| ATOM | 1070 | CB | ARG | A | 135 | 77.147 | 10.870 | 7.277 | 1.00 | 61.49 | A |
| ATOM | 1071 | CG | ARG | A | 135 | 75.731 | 11.414 | 7.305 | 1.00 | 59.73 | A |
| ATOM | 1072 | CD | ARG | A | 135 | 75.633 | 12.731 | 8.079 | 1.00 | 58.36 | A |
| ATOM | 1073 | NE | ARG | A | 135 | 75.896 | 12.583 | 9.507 | 1.00 | 55.30 | A |
| ATOM | 1074 | CZ | ARG | A | 135 | 76.202 | 13.586 | 10.325 | 1.00 | 53.79 | A |
| ATOM | 1075 | NH1 | ARG | A | 135 | 76.293 | 14.829 | 9.870 | 1.00 | 50.17 | A |
| ATOM | 1076 | NH2 | ARG | A | 135 | 76.399 | 13.344 | 11.611 | 1.00 | 53.57 | A |
| ATOM | 1077 | C | ARG | A | 135 | 79.615 | 11.314 | 7.308 | 1.00 | 65.61 | A |
| ATOM | 1078 | O | ARG | A | 135 | 80.272 | 11.410 | 8.339 | 1.00 | 66.32 | A |
| ATOM | 1079 | N | TYR | A | 136 | 80.078 | 10.729 | 6.207 | 1.00 | 68.73 | A |
| ATOM | 1080 | CA | TYR | A | 136 | 81.387 | 10.076 | 6.177 | 1.00 | 72.57 | A |
| ATOM | 1081 | CB | TYR | A | 136 | 81.673 | 9.516 | 4.777 | 1.00 | 73.41 | A |
| ATOM | 1082 | CG | TYR | A | 136 | 82.752 | 8.450 | 4.747 | 1.00 | 75.00 | A |
| ATOM | 1083 | CD1 | TYR | A | 136 | 82.570 | 7.237 | 5.411 | 1.00 | 77.20 | A |
| ATOM | 1084 | CE1 | TYR | A | 136 | 83.555 | 6.244 | 5.392 | 1.00 | 78.60 | A |
| ATOM | 1085 | CD2 | TYR | A | 136 | 83.952 | 8.649 | 4.057 | 1.00 | 75.08 | A |
| ATOM | 1086 | CE2 | TYR | A | 136 | 84.947 | 7.660 | 4.032 | 1.00 | 76.27 | A |
| ATOM | 1087 | CZ | TYR | A | 136 | 84.739 | 6.458 | 4.704 | 1.00 | 77.65 | A |
| ATOM | 1088 | OH | TYR | A | 136 | 85.695 | 5.463 | 4.700 | 1.00 | 77.25 | A |
| ATOM | 1089 | C | TYR | A | 136 | 82.529 | 11.003 | 6.610 | 1.00 | 74.52 | A |
| ATOM | 1090 | O | TYR | A | 136 | 83.316 | 10.657 | 7.500 | 1.00 | 74.04 | A |
| ATOM | 1091 | N | VAL | A | 137 | 82.594 | 12.181 | 5.989 | 1.00 | 77.10 | A |
| ATOM | 1092 | CA | VAL | A | 137 | 83.634 | 13.178 | 6.272 | 1.00 | 79.78 | A |
| ATOM | 1093 | CB | VAL | A | 137 | 83.529 | 14.399 | 5.298 | 1.00 | 79.12 | A |
| ATOM | 1094 | CG1 | VAL | A | 137 | 84.559 | 15.471 | 5.640 | 1.00 | 78.80 | A |
| ATOM | 1095 | CG2 | VAL | A | 137 | 83.730 | 13.941 | 3.860 | 1.00 | 78.73 | A |
| ATOM | 1096 | C | VAL | A | 137 | 83.623 | 13.649 | 7.730 | 1.00 | 81.54 | A |
| ATOM | 1097 | O | VAL | A | 137 | 84.626 | 14.161 | 8.239 | 1.00 | 82.35 | A |
| ATOM | 1098 | N | VAL | A | 138 | 82.495 | 13.456 | 8.406 | 1.00 | 83.08 | A |
| ATOM | 1099 | CA | VAL | A | 138 | 82.376 | 13.849 | 9.802 | 1.00 | 84.53 | A |
| ATOM | 1100 | CB | VAL | A | 138 | 81.020 | 14.529 | 10.069 | 1.00 | 83.25 | A |
| ATOM | 1101 | CG1 | VAL | A | 138 | 80.888 | 14.885 | 11.535 | 1.00 | 83.68 | A |
| ATOM | 1102 | CG2 | VAL | A | 138 | 80.896 | 15.784 | 9.216 | 1.00 | 81.53 | A |
| ATOM | 1103 | C | VAL | A | 138 | 82.587 | 12.644 | 10.731 | 1.00 | 86.63 | A |
| ATOM | 1104 | O | VAL | A | 138 | 83.330 | 12.727 | 11.711 | 1.00 | 86.47 | A |
| ATOM | 1105 | N | VAL | A | 139 | 81.968 | 11.515 | 10.394 | 1.00 | 89.20 | A |
| ATOM | 1106 | CA | VAL | A | 139 | 82.096 | 10.296 | 11.191 | 1.00 | 91.64 | A |
| ATOM | 1107 | CB | VAL | A | 139 | 80.761 | 9.496 | 11.217 | 1.00 | 91.28 | A |
| ATOM | 1108 | CG1 | VAL | A | 139 | 80.757 | 8.500 | 12.368 | 1.00 | 91.26 | A |
| ATOM | 1109 | CG2 | VAL | A | 139 | 79.572 | 10.438 | 11.338 | 1.00 | 91.10 | A |
| ATOM | 1110 | C | VAL | A | 139 | 83.239 | 9.423 | 10.644 | 1.00 | 94.09 | A |
| ATOM | 1111 | O | VAL | A | 139 | 84.199 | 9.952 | 10.081 | 1.00 | 93.34 | A |
| ATOM | 1112 | N | CYS | A | 140 | 83.106 | 8.101 | 10.805 | 1.00 | 97.60 | A |
| ATOM | 1113 | CA | CYS | A | 140 | 84.073 | 7.071 | 10.386 | 1.00 | 100.89 | A |
| ATOM | 1114 | CB | CYS | A | 140 | 83.714 | 6.465 | 9.024 | 1.00 | 98.92 | A |
| ATOM | 1115 | SG | CYS | A | 140 | 84.604 | 4.917 | 8.676 | 1.00 | 95.56 | A |
| ATOM | 1116 | C | CYS | A | 140 | 85.535 | 7.501 | 10.396 | 1.00 | 104.29 | A |
| ATOM | 1117 | O | CYS | A | 140 | 86.334 | 7.003 | 11.199 | 1.00 | 105.15 | A |
| ATOM | 1118 | N | LYS | A | 141 | 85.879 | 8.395 | 9.471 | 1.00 | 107.06 | A |
| ATOM | 1119 | CA | LYS | A | 141 | 87.228 | 8.930 | 9.349 | 1.00 | 109.52 | A |
| ATOM | 1120 | CB | LYS | A | 141 | 88.193 | 7.842 | 8.847 | 1.00 | 109.22 | A |
| ATOM | 1121 | CG | LYS | A | 141 | 89.610 | 7.898 | 9.432 | 1.00 | 108.39 | A |
| ATOM | 1122 | CD | LYS | A | 141 | 89.648 | 7.431 | 10.888 | 1.00 | 107.93 | A |
| ATOM | 1123 | CE | LYS | A | 141 | 91.077 | 7.400 | 11.435 | 1.00 | 107.77 | A |
| ATOM | 1124 | NZ | LYS | A | 141 | 91.151 | 6.961 | 12.865 | 1.00 | 106.81 | A |
| ATOM | 1125 | C | LYS | A | 141 | 87.172 | 10.087 | 8.349 | 1.00 | 111.46 | A |
| ATOM | 1126 | O | LYS | A | 141 | 86.826 | 9.886 | 7.181 | 1.00 | 111.36 | A |
| ATOM | 1127 | N | PRO | A | 142 | 87.413 | 11.327 | 8.820 | 1.00 | 113.63 | A |
| ATOM | 1128 | CD | PRO | A | 142 | 87.485 | 11.691 | 10.247 | 1.00 | 113.92 | A |
| ATOM | 1129 | CA | PRO | A | 142 | 87.403 | 12.534 | 7.977 | 1.00 | 115.39 | A |
| ATOM | 1130 | CB | PRO | A | 142 | 87.686 | 13.652 | 8.984 | 1.00 | 114.68 | A |
| ATOM | 1131 | CG | PRO | A | 142 | 87.042 | 13.137 | 10.231 | 1.00 | 114.39 | A |
| ATOM | 1132 | C | PRO | A | 142 | 88.481 | 12.483 | 6.883 | 1.00 | 116.90 | A |
| ATOM | 1133 | O | PRO | A | 142 | 89.328 | 11.586 | 6.879 | 1.00 | 116.95 | A |
| ATOM | 1134 | N | MET | A | 143 | 88.451 | 13.445 | 5.962 | 1.00 | 118.42 | A |
| ATOM | 1135 | CA | MET | A | 143 | 89.425 | 13.482 | 4.874 | 1.00 | 119.92 | A |
| ATOM | 1136 | CB | MET | A | 143 | 89.073 | 14.586 | 3.873 | 1.00 | 119.73 | A |
| ATOM | 1137 | CG | MET | A | 143 | 89.795 | 14.461 | 2.540 | 1.00 | 119.65 | A |
| ATOM | 1138 | SD | MET | A | 143 | 89.592 | 15.924 | 1.522 | 1.00 | 120.49 | A |
| ATOM | 1139 | CE | MET | A | 143 | 87.879 | 15.761 | 1.023 | 1.00 | 119.00 | A |
| ATOM | 1140 | C | MET | A | 143 | 90.834 | 13.691 | 5.430 | 1.00 | 121.20 | A |
| ATOM | 1141 | O | MET | A | 143 | 91.448 | 12.750 | 5.944 | 1.00 | 121.07 | A |
| ATOM | 1142 | N | SER | A | 144 | 91.352 | 14.913 | 5.303 | 1.00 | 122.66 | A |
| ATOM | 1143 | CA | SER | A | 144 | 92.681 | 15.239 | 5.815 | 1.00 | 123.47 | A |
| ATOM | 1144 | CB | SER | A | 144 | 93.375 | 16.300 | 4.939 | 1.00 | 123.07 | A |
| ATOM | 1145 | OG | SER | A | 144 | 92.584 | 17.466 | 4.772 | 1.00 | 122.96 | A |
| ATOM | 1146 | C | SER | A | 144 | 92.545 | 15.710 | 7.263 | 1.00 | 123.81 | A |
| ATOM | 1147 | O | SER | A | 144 | 93.141 | 15.120 | 8.170 | 1.00 | 123.93 | A |
| ATOM | 1148 | N | ASN | A | 145 | 91.709 | 16.730 | 7.470 | 1.00 | 123.89 | A |
| ATOM | 1149 | CA | ASN | A | 145 | 91.450 | 17.304 | 8.794 | 1.00 | 123.78 | A |
| ATOM | 1150 | CB | ASN | A | 145 | 92.773 | 17.579 | 9.524 | 1.00 | 123.35 | A |
| ATOM | 1151 | CG | ASN | A | 145 | 92.608 | 17.677 | 11.027 | 1.00 | 122.86 | A |
| ATOM | 1152 | OD1 | ASN | A | 145 | 93.166 | 16.873 | 11.775 | 1.00 | 122.86 | A |
| ATOM | 1153 | ND2 | ASN | A | 145 | 91.850 | 18.670 | 11.479 | 1.00 | 122.53 | A |
| ATOM | 1154 | C | ASN | A | 145 | 90.656 | 18.612 | 8.656 | 1.00 | 123.93 | A |
| ATOM | 1155 | O | ASN | A | 145 | 91.176 | 19.691 | 8.948 | 1.00 | 124.33 | A |
| ATOM | 1156 | N | PHE | A | 145 | 89.396 | 18.519 | 8.229 | 1.00 | 123.85 | A |
| ATOM | 1157 | CA | PHE | A | 146 | 88.571 | 19.718 | 8.057 | 1.00 | 122.90 | A |
| ATOM | 1158 | CB | PHE | A | 146 | 88.819 | 20.342 | 6.665 | 1.00 | 123.61 | A |
| ATOM | 1159 | CG | PHE | A | 146 | 87.927 | 19.797 | 5.565 | 1.00 | 123.94 | A |
| ATOM | 1160 | CD1 | PHE | A | 146 | 87.124 | 20.658 | 4.820 | 1.00 | 123.94 | A |
| ATOM | 1161 | CD2 | PHE | A | 146 | 87.881 | 18.432 | 5.282 | 1.00 | 124.13 | A |
| ATOM | 1162 | CE1 | PHE | A | 146 | 86.291 | 20.171 | 3.814 | 1.00 | 123.73 | A |
| ATOM | 1163 | CE2 | PHE | A | 146 | 87.049 | 17.937 | 4.277 | 1.00 | 123.97 | A |
| ATOM | 1164 | CZ | PHE | A | 146 | 86.253 | 18.809 | 3.543 | 1.00 | 123.70 | A |
| ATOM | 1165 | C | PHE | A | 146 | 87.065 | 19.524 | 8.277 | 1.00 | 121.75 | A |
| ATOM | 1166 | O | PHE | A | 146 | 86.509 | 18.454 | 8.003 | 1.00 | 121.89 | A |
| ATOM | 1167 | N | ARG | A | 147 | 86.424 | 20.564 | 8.806 | 1.00 | 119.67 | A |
| ATOM | 1168 | CA | ARG | A | 147 | 84.982 | 20.551 | 9.022 | 1.00 | 117.46 | A |
| ATOM | 1169 | CB | ARG | A | 147 | 84.587 | 21.407 | 10.231 | 1.00 | 119.05 | A |
| ATOM | 1170 | CG | ARG | A | 147 | 85.185 | 21.006 | 11.569 | 1.00 | 120.97 | A |
| ATOM | 1171 | CD | ARG | A | 147 | 84.505 | 21.802 | 12.675 | 1.00 | 122.99 | A |
| ATOM | 1172 | NE | ARG | A | 147 | 85.294 | 21.880 | 13.903 | 1.00 | 125.46 | A |
| ATOM | 1173 | CZ | ARG | A | 147 | 85.157 | 22.833 | 14.825 | 1.00 | 126.61 | A |
| ATOM | 1174 | NH1 | ARG | A | 147 | 84.255 | 23.797 | 14.666 | 1.00 | 126.85 | A |
| ATOM | 1175 | NH2 | ARG | A | 147 | 85.939 | 22.840 | 15.899 | 1.00 | 127.09 | A |
| ATOM | 1176 | C | ARG | A | 147 | 84.379 | 21.176 | 7.766 | 1.00 | 114.90 | A |
| ATOM | 1177 | O | ARG | A | 147 | 85.062 | 21.916 | 7.052 | 1.00 | 114.32 | A |
| ATOM | 1178 | N | PHE | A | 148 | 83.112 | 20.875 | 7.488 | 1.00 | 112.13 | A |
| ATOM | 1179 | CA | PHE | A | 148 | 82.436 | 21.437 | 6.316 | 1.00 | 108.72 | A |
| ATOM | 1180 | CB | PHE | A | 148 | 81.104 | 20.722 | 6.039 | 1.00 | 106.72 | A |
| ATOM | 1181 | CG | PHE | A | 148 | 81.238 | 19.473 | 5.215 | 1.00 | 104.53 | A |
| ATOM | 1182 | CD1 | PHE | A | 148 | 81.301 | 19.544 | 3.832 | 1.00 | 103.69 | A |
| ATOM | 1183 | CD2 | PHE | A | 148 | 81.297 | 18.225 | 5.821 | 1.00 | 104.10 | A |
| ATOM | 1184 | CE1 | PHE | A | 148 | 81.424 | 18.389 | 3.064 | 1.00 | 103.27 | A |
| ATOM | 1185 | CE2 | PHE | A | 148 | 81.420 | 17.068 | 5.058 | 1.00 | 103.21 | A |
| ATOM | 1186 | CZ | PHE | A | 148 | 81.483 | 17.151 | 3.679 | 1.00 | 102.60 | A |
| ATOM | 1187 | C | PHE | A | 148 | 82.183 | 22.931 | 6.500 | 1.00 | 106.83 | A |
| ATOM | 1188 | O | PHE | A | 148 | 81.760 | 23.380 | 7.575 | 1.00 | 107.22 | A |
| ATOM | 1189 | N | GLY | A | 149 | 82.498 | 23.700 | 5.464 | 1.00 | 103.74 | A |
| ATOM | 1190 | CA | GLY | A | 149 | 82.281 | 25.131 | 5.512 | 1.00 | 99.98 | A |
| ATOM | 1191 | C | GLY | A | 149 | 80.896 | 25.410 | 4.977 | 1.00 | 97.19 | A |
| ATOM | 1192 | O | GLY | A | 149 | 80.312 | 24.565 | 4.298 | 1.00 | 96.60 | A |
| ATOM | 1193 | N | GLU | A | 150 | 80.363 | 26.584 | 5.292 | 1.00 | 95.09 | A |
| ATOM | 1194 | CA | GLU | A | 150 | 79.033 | 26.973 | 4.831 | 1.00 | 93.26 | A |
| ATOM | 1195 | CB | GLU | A | 150 | 78.710 | 28.396 | 5.320 | 1.00 | 93.56 | A |
| ATOM | 1196 | CG | GLU | A | 150 | 77.245 | 28.825 | 5.161 | 1.00 | 92.88 | A |
| ATOM | 1197 | CD | GLU | A | 150 | 76.961 | 30.236 | 5.678 | 1.00 | 92.63 | A |
| ATOM | 1198 | OE1 | GLU | A | 150 | 75.793 | 30.686 | 5.579 | 1.00 | 92.03 | A |
| ATOM | 1199 | OE2 | GLU | A | 150 | 77.899 | 30.897 | 6.178 | 1.00 | 92.40 | A |
| ATOM | 1200 | C | GLU | A | 150 | 79.005 | 26.904 | 3.302 | 1.00 | 91.57 | A |
| ATOM | 1201 | O | GLU | A | 150 | 77.973 | 26.632 | 2.692 | 1.00 | 91.98 | A |
| ATOM | 1202 | N | ASN | A | 151 | 80.173 | 27.097 | 2.702 | 1.00 | 89.63 | A |
| ATOM | 1203 | CA | ASN | A | 151 | 80.339 | 27.068 | 1.255 | 1.00 | 87.71 | A |
| ATOM | 1204 | CB | ASN | A | 151 | 81.734 | 27.607 | 0.879 | 1.00 | 88.95 | A |
| ATOM | 1205 | CG | ASN | A | 151 | 82.866 | 26.993 | 1.717 | 1.00 | 89.12 | A |
| ATOM | 1206 | OD1 | ASN | A | 151 | 83.816 | 26.433 | 1.171 | 1.00 | 89.31 | A |
| ATOM | 1207 | ND2 | ASN | A | 151 | 82.782 | 27.131 | 3.040 | 1.00 | 89.04 | A |
| ATOM | 1208 | C | ASN | A | 151 | 80.112 | 25.678 | 0.659 | 1.00 | 85.56 | A |
| ATOM | 1209 | O | ASN | A | 151 | 79.412 | 25.527 | -0.347 | 1.00 | 83.88 | A |
| ATOM | 1210 | N | HIS | A | 152 | 80.690 | 24.671 | 1.312 | 1.00 | 84.19 | A |
| ATOM | 1211 | CA | HIS | A | 152 | 80.589 | 23.272 | 0.896 | 1.00 | 82.65 | A |
| ATOM | 1212 | CB | HIS | A | 152 | 81.307 | 22.371 | 1.909 | 1.00 | 84.90 | A |
| ATOM | 1213 | CG | HIS | A | 152 | 82.739 | 22.742 | 2.157 | 1.00 | 86.92 | A |
| ATOM | 1214 | CD2 | HIS | A | 152 | 83.893 | 22.112 | 1.830 | 1.00 | 87.50 | A |
| ATOM | 1215 | ND1 | HIS | A | 152 | 83.106 | 23.873 | 2.854 | 1.00 | 87.96 | A |
| ATOM | 1216 | CE1 | HIS | A | 152 | 84.424 | 23.922 | 2.948 | 1.00 | 88.79 | A |
| ATOM | 1217 | NE2 | HIS | A | 152 | 84.925 | 22.865 | 2.334 | 1.00 | 88.06 | A |
| ATOM | 1218 | C | HIS | A | 152 | 79.134 | 22.818 | 0.779 | 1.00 | 80.50 | A |
| ATOM | 1219 | O | HIS | A | 152 | 78.801 | 21.980 | -0.059 | 1.00 | 79.40 | A |
| ATOM | 1220 | N | ALA | A | 153 | 78.282 | 23.378 | 1.636 | 1.00 | 77.90 | A |
| ATOM | 1221 | CA | ALA | A | 153 | 76.862 | 23.057 | 1.669 | 1.00 | 74.72 | A |
| ATOM | 1222 | CB | ALA | A | 153 | 76.220 | 23.683 | 2.893 | 1.00 | 73.68 | A |
| ATOM | 1223 | C | ALA | A | 153 | 76.115 | 23.475 | 0.402 | 1.00 | 72.80 | A |
| ATOM | 1224 | O | ALA | A | 153 | 75.218 | 22.762 | -0.052 | 1.00 | 73.35 | A |
| ATOM | 1225 | N | ILE | A | 154 | 76.498 | 24.608 | -0.182 | 1.00 | 69.95 | A |
| ATOM | 1226 | CA | ILE | A | 154 | 75.839 | 25.098 | -1.393 | 1.00 | 68.06 | A |
| ATOM | 1227 | CB | ILE | A | 154 | 76.064 | 26.603 | -1.580 | 1.00 | 67.16 | A |
| ATOM | 1228 | CG2 | ILE | A | 154 | 75.116 | 27.143 | -2.634 | 1.00 | 66.16 | A |
| ATOM | 1229 | CG1 | ILE | A | 154 | 75.780 | 27.321 | -0.263 | 1.00 | 66.81 | A |
| ATOM | 1230 | CD1 | ILE | A | 154 | 75.932 | 28.811 | -0.325 | 1.00 | 67.23 | A |
| ATOM | 1231 | C | ILE | A | 154 | 76.240 | 24.335 | -2.661 | 1.00 | 67.09 | A |
| ATOM | 1232 | O | ILE | A | 154 | 75.600 | 24.459 | -3.709 | 1.00 | 66.43 | A |
| ATOM | 1233 | N | MET | A | 155 | 77.297 | 23.541 | -2.559 | 1.00 | 66.34 | A |
| ATOM | 1234 | CA | MET | A | 155 | 77.747 | 22.736 | -3.685 | 1.00 | 65.89 | A |
| ATOM | 1235 | CB | MET | A | 155 | 79.163 | 22.211 | -3.444 | 1.00 | 69.85 | A |
| ATOM | 1236 | CG | MET | A | 155 | 80.082 | 22.358 | -4.645 | 1.00 | 73.68 | A |
| ATOM | 1237 | SD | MET | A | 155 | 80.377 | 24.104 | -5.041 | 1.00 | 78.00 | A |
| ATOM | 1238 | CE | MET | A | 155 | 79.062 | 24.440 | -6.283 | 1.00 | 78.49 | A |
| ATOM | 1239 | C | MET | A | 155 | 76.784 | 21.565 | -3.807 | 1.00 | 63.00 | A |
| ATOM | 1240 | O | MET | A | 155 | 76.248 | 21.295 | -4.877 | 1.00 | 62.83 | A |
| ATOM | 1241 | N | GLY | A | 156 | 76.563 | 20.892 | -2.682 | 1.00 | 60.05 | A |
| ATOM | 1242 | CA | GLY | A | 156 | 75.654 | 19.765 | -2.633 | 1.00 | 55.80 | A |
| ATOM | 1243 | C | GLY | A | 156 | 74.246 | 20.183 | -2.992 | 1.00 | 53.03 | A |
| ATOM | 1244 | O | GLY | A | 156 | 73.487 | 19.376 | -3.509 | 1.00 | 53.78 | A |
| ATOM | 1245 | N | VAL | A | 157 | 73.895 | 21.436 | -2.709 | 1.00 | 49.41 | A |
| ATOM | 1246 | CA | VAL | A | 157 | 72.577 | 21.956 | -3.038 | 1.00 | 46.20 | A |
| ATOM | 1247 | CB | VAL | A | 157 | 72.272 | 23.259 | -2.282 | 1.00 | 44.97 | A |
| ATOM | 1248 | CG1 | VAL | A | 157 | 71.057 | 23.953 | -2.886 | 1.00 | 43.89 | A |
| ATOM | 1249 | CG2 | VAL | A | 157 | 72.019 | 22.958 | -0.821 | 1.00 | 43.17 | A |
| ATOM | 1250 | C | VAL | A | 157 | 72.497 | 22.211 | -4.534 | 1.00 | 45.95 | A |
| ATOM | 1251 | O | VAL | A | 157 | 71.501 | 21.877 | -5.167 | 1.00 | 46.38 | A |
| ATOM | 1252 | N | ALA | A | 158 | 73.541 | 22.817 | -5.092 | 1.00 | 46.55 | A |
| ATOM | 1253 | CA | ALA | A | 158 | 73.586 | 23.101 | -6.528 | 1.00 | 47.45 | A |
| ATOM | 1254 | CB | ALA | A | 158 | 74.813 | 23.947 | -6.863 | 1.00 | 47.38 | A |
| ATOM | 1255 | C | ALA | A | 158 | 73.629 | 21.775 | -7.285 | 1.00 | 47.57 | A |
| ATOM | 1256 | O | ALA | A | 158 | 72.978 | 21.614 | -8.312 | 1.00 | 45.76 | A |
| ATOM | 1257 | N | PHE | A | 159 | 74.382 | 20.831 | -6.723 | 1.00 | 49.36 | A |
| ATOM | 1258 | CA | PHE | A | 159 | 74.563 | 19.474 | -7.242 | 1.00 | 49.96 | A |
| ATOM | 1259 | CB | PHE | A | 159 | 75.517 | 18.708 | -6.309 | 1.00 | 50.63 | A |
| ATOM | 1260 | CG | PHE | A | 159 | 75.595 | 17.219 | -6.565 | 1.00 | 51.73 | A |
| ATOM | 1261 | CD1 | PHE | A | 159 | 76.404 | 16.708 | -7.579 | 1.00 | 52.40 | A |
| ATOM | 1262 | CD2 | PHE | A | 159 | 74.924 | 16.321 | -5.735 | 1.00 | 51.51 | A |
| ATOM | 1263 | CE1 | PHE | A | 159 | 76.551 | 15.324 | -7.756 | 1.00 | 51.64 | A |
| ATOM | 1264 | CE2 | PHE | A | 159 | 75.067 | 14.939 | -5.906 | 1.00 | 50.81 | A |
| ATOM | 1265 | CZ | PHE | A | 159 | 75.884 | 14.441 | -6.917 | 1.00 | 50.12 | A |
| ATOM | 1266 | C | PHE | A | 159 | 73.219 | 18.751 | -7.334 | 1.00 | 49.29 | A |
| ATOM | 1267 | O | PHE | A | 159 | 72.845 | 18.273 | -8.403 | 1.00 | 50.35 | A |
| ATOM | 1268 | N | THR | A | 160 | 72.497 | 18.674 | -6.218 | 1.00 | 47.51 | A |
| ATOM | 1269 | CA | THR | A | 160 | 71.199 | 18.012 | -6.209 | 1.00 | 45.74 | A |
| ATOM | 1270 | CB | THR | A | 160 | 70.520 | 18.068 | -4.844 | 1.00 | 44.28 | A |
| ATOM | 1271 | OG1 | THR | A | 160 | 70.187 | 19.426 | -4.523 | 1.00 | 42.68 | A |
| ATOM | 1272 | CG2 | THR | A | 160 | 71.411 | 17.459 | -3.785 | 1.00 | 41.22 | A |
| ATOM | 1273 | C | THR | A | 160 | 70.255 | 18.642 | -7.219 | 1.00 | 46.36 | A |
| ATOM | 1274 | O | THR | A | 160 | 69.457 | 17.943 | -7.824 | 1.00 | 46.97 | A |
| ATOM | 1275 | N | TRP | A | 161 | 70.330 | 19.959 | -7.385 | 1.00 | 46.89 | A |
| ATOM | 1276 | CA | TRP | A | 161 | 69.476 | 20.638 | -8.350 | 1.00 | 47.72 | A |
| ATOM | 1277 | CB | TRP | A | 161. | 69.479 | 22.147 | -8.132 | 1.00 | 49.14 | A |
| ATOM | 1278 | CG | TRP | A | 161 | 68.239 | 22.590 | -7.472 | 1.00 | 51.73 | A |
| ATOM | 1279 | CD2 | TRP | A | 161 | 67.023 | 23.012 | -8.112 | 1.00 | 53.47 | A |
| ATOM | 1280 | CE2 | TRP | A | 161 | 66.078 | 23.254 | -7.094 | 1.00 | 53.26 | A |
| ATOM | 1281 | CE3 | TRP | A | 161 | 66.642 | 23.206 | -9.448 | 1.00 | 55.53 | A |
| ATOM | 1282 | CD1 | TRP | A | 161 | 67.992 | 22.602 | -6.136 | 1.00 | 51.81 | A |
| ATOM | 1283 | NB1 | TRP | A | 161 | 66.695 | 22.996 | -5.898 | 1.00 | 53.29 | A |
| ATOM | 1284 | CZ2 | TRP | A | 161 | 64.768 | 23.681 | -7.365 | 1.00 | 53.82 | A |
| ATOM | 1285 | CZ3 | TRP | A | 161 | 65.332 | 23.635 | -9.720 | 1.00 | 55.83 | A |
| ATOM | 1286 | CH2 | TRP | A | 161 | 64.416 | 23.866 | -8.680 | 1.00 | 54.71 | A |
| ATOM | 1287 | C | TRP | A | 161 | 69.862 | 20.319 | -9.783 | 1.00 | 47.65 | A |
| ATOM | 1288 | O | TRP | A | 161 | 69.001 | 20.227 | -10.654 | 1.00 | 47.78 | A |
| ATOM | 1289 | N | VAL | A | 162 | 71.157 | 20.145 | -10.023 | 1.00 | 47.02 | A |
| ATOM | 1290 | CA | VAL | A | 162 | 71.651 | 19.830 | -11.357 | 1.00 | 47.22 | A |
| ATOM | 1291 | CB | VAL | A | 162 | 73.193 | 20.098 | -11.458 | 1.00 | 47.42 | A |
| ATOM | 1292 | CG1 | VAL | A | 162 | 73.730 | 19.737 | -12.837 | 1.00 | 47.67 | A |
| ATOM | 1293 | CG2 | VAL | A | 162 | 73.486 | 21.560 | -11.177 | 1.00 | 47.04 | A |
| ATOM | 1294 | C | VAL | A | 162 | 71.309 | 18.368 | -11.686 | 1.00 | 46.80 | A |
| ATOM | 1295 | O | VAL | A | 162 | 70.728 | 18.073 | -12.732 | 1.00 | 46.36 | A |
| ATOM | 1296 | N | MET | A | 163 | 71.614 | 17.474 | -10.753 | 1.00 | 46.72 | A |
| ATOM | 1297 | CA | MET | A | 163 | 71.358 | 16.043 | -10.912 | 1.00 | 47.40 | A |
| ATOM | 1298 | CB | MET | A | 163 | 72.091 | 15.271 | -9.820 | 1.00 | 49.03 | A |
| ATOM | 1299 | CG | MET | A | 163 | 73.599 | 15.272 | -9.975 | 1.00 | 51.04 | A |
| ATOM | 1300 | SD | MET | A | 163 | 74.118 | 14.209 | -11.327 | 1.00 | 51.92 | A |
| ATOM | 1301 | CE | MET | A | 163 | 74.167 | 15.362 | -12.709 | 1.00 | 52.61 | A |
| ATOM | 1302 | C | MET | A | 163 | 69.883 | 15.631 | -10.937 | 1.00 | 47.21 | A |
| ATOM | 1303 | O | MET | A | 163 | 69.547 | 14.576 | -11.465 | 1.00 | 46.37 | A |
| ATOM | 1304 | N | ALA | A | 164 | 69.017 | 16.440 | -10.331 | 1.00 | 47.66 | A |
| ATOM | 1305 | CA | ALA | A | 164 | 67.582 | 16.161 | -10.301 | 1.00 | 47.42 | A |
| ATOM | 1306 | CB | ALA | A | 164 | 66.943 | 16.763 | -9.068 | 1.00 | 47.89 | A |
| ATOM | 1307 | C | ALA | A | 164 | 66.941 | 16.733 | -11.548 | 1.00 | 47.43 | A |
| ATOM | 1308 | O | ALA | A | 164 | 65.900 | 16.259 | -11.997 | 1.00 | 48.11 | A |
| ATOM | 1309 | N | LEU | A | 165 | 67.544 | 17.797 | -12.065 | 1.00 | 47.64 | A |
| ATOM | 1310 | CA | LEU | A | 165 | 67.072 | 18.434 | -13.285 | 1.00 | 48.45 | A |
| ATOM | 1311 | CB | LEU | A | 165 | 67.616 | 19.861 | -13.383 | 1.00 | 48.08 | A |
| ATOM | 1312 | CG | LEU | A | 165 | 66.815 | 20.977 | -12.716 | 1.00 | 47.09 | A |
| ATOM | 1313 | CD1 | LEU | A | 165 | 67.677 | 22.215 | -12.544 | 1.00 | 45.65 | A |
| ATOM | 1314 | CD2 | LEU | A | 165 | 65.589 | 21.281 | -13.562 | 1.00 | 46.66 | A |
| ATOM | 1315 | C | LEU | A | 165 | 67.535 | 17.603 | -14.488 | 1.00 | 49.77 | A |
| ATOM | 1316 | O | LEU | A | 165 | 66.941 | 17.684 | -15.565 | 1.00 | 49.98 | A |
| ATOM | 1317 | N | ALA | A | 166 | 68.597 | 16.811 | -14.296 | 1.00 | 50.60 | A |
| ATOM | 1318 | CA | ALA | A | 166 | 69.147 | 15.937 | -15.341 | 1.00 | 50.48 | A |
| ATOM | 1319 | CB | ALA | A | 166 | 70.588 | 15.558 | -15.004 | 1.00 | 48.63 | A |
| ATOM | 1320 | C | ALA | A | 166 | 68.262 | 14.672 | -15.517 | 1.00 | 51.04 | A |
| ATOM | 1321 | O | ALA | A | 166 | 68.406 | 13.937 | -16.506 | 1.00 | 51.57 | A |
| ATOM | 1322 | N | CYS | A | 167 | 67.392 | 14.444 | -14.553 | 1.00 | 50.58 | A |
| ATOM | 1323 | CA | CYS | A | 167 | 66.486 | 13.306 | -14.565 | 1.00 | 49.49 | A |
| ATOM | 1324 | CB | CYS | A | 167 | 66.518 | 12.603 | -13.209 | 1.00 | 50.50 | A |
| ATOM | 1325 | SG | CYS | A | 167 | 65.361 | 11.222 | -13.027 | 1.00 | 52.92 | A |
| ATOM | 1326 | C | CYS | A | 167 | 65.061 | 13.757 | -14.844 | 1.00 | 48.44 | A |
| ATOM | 1327 | O | CYS | A | 167 | 64.207 | 12.945 | -15.195 | 1.00 | 49.64 | A |
| ATOM | 1328 | N | ALA | A | 168 | 64.798 | 15.047 | -14.677 | 1.00 | 45.73 | A |
| ATOM | 1329 | CA | ALA | A | 168 | 63.457 | 15.559 | -14.885 | 1.00 | 44.39 | A |
| ATOM | 1330 | CB | ALA | A | 168 | 62.997 | 16.301 | -13.651 | 1.00 | 43.13 | A |
| ATOM | 1331 | C | ALA | A | 168 | 63.286 | 16.431 | -16.121 | 1.00 | 44.36 | A |
| ATOM | 1332 | O | ALA | A | 168 | 62.176 | 16.570 | -16.633 | 1.00 | 45.08 | A |
| ATOM | 1333 | N | ALA | A | 169 | 64.379 | 16.998 | -16.618 | 1.00 | 43.75 | A |
| ATOM | 1334 | CA | ALA | A | 169 | 64.314 | 17.874 | -17.788 | 1.00 | 42.27 | A |
| ATOM | 1335 | CB | ALA | A | 169 | 65.498 | 18.820 | -17.800 | 1.00 | 42.09 | A |
| ATOM | 1336 | C | ALA | A | 169 | 64.149 | 17.196 | -19.154 | 1.00 | 40.36 | A |
| ATOM | 1337 | O | ALA | A | 169 | 63.366 | 17.656 | -19.981 | 1.00 | 40.15 | A |
| ATOM | 1338 | N | PRO | A | 170 | 64.882 | 16.103 | -19.407 | 1.00 | 38.56 | A |
| ATOM | 1339 | CD | PRO | A | 170 | 65.912 | 15.485 | -18.550 | 1.00 | 37.33 | A |
| ATOM | 1340 | CA | PRO | A | 170 | 64.778 | 15.401 | -20.689 | 1.00 | 38.83 | A |
| ATOM | 1341 | CB | PRO | A | 170 | 65.564 | 14.125 | -20.431 | 1.00 | 38.32 | A |
| ATOM | 1342 | CG | PRO | A | 170 | 66.647 | 14.602 | -19.506 | 1.00 | 37.93 | A |
| ATOM | 1343 | C | PRO | A | 170 | 63.360 | 15.115 | -21.210 | 1.00 | 40.49 | A |
| ATOM | 1344 | O | PRO | A | 170 | 63.087 | 15.315 | -22.388 | 1.00 | 40.60 | A |
| ATOM | 1345 | N | PRO | A | 171 | 62.443 | 14.630 | -20.354 | 1.00 | 42.59 | A |
| ATOM | 1346 | CD | PRO | A | 171 | 62.634 | 14.120 | -18.987 | 1.00 | 43.82 | A |
| ATOM | 1347 | CA | PRO | A | 171 | 61.075 | 14.343 | -20.806 | 1.00 | 44.72 | A |
| ATOM | 1348 | CB | PRO | A | 171 | 60.434 | 13.722 | -19.569 | 1.00 | 43.38 | A |
| ATOM | 1349 | CG | PRO | A | 171 | 61.566 | 13.072 | -18.898 | 1.00 | 43.80 | A |
| ATOM | 1350 | C | PRO | A | 171 | 60.288 | 15.574 | -21.271 | 1.00 | 47.48 | A |
| ATOM | 1351 | O | PRO | A | 171 | 59.106 | 15.467 | -21.634 | 1.00 | 48.47 | A |
| ATOM | 1352 | N | LEU | A | 172 | 60.938 | 16.738 | -21.230 | 1.00 | 49.40 | A |
| ATOM | 1353 | CA | LEU | A | 172 | 60.334 | 18.009 | -21.657 | 1.00 | 50.86 | A |
| ATOM | 1354 | CB | LEU | A | 172 | 60.526 | 19.086 | -20.579 | 1.00 | 51.11 | A |
| ATOM | 1355 | CG | LEU | A | 172 | 59.939 | 18.892 | -19.183 | 1.00 | 51.20 | A |
| ATOM | 1356 | CD1 | LEU | A | 172 | 60.412 | 20.015 | -18.288 | 1.00 | 51.60 | A |
| ATOM | 1357 | CD2 | LEU | A | 172 | 58.427 | 18.868 | -19.243 | 1.00 | 51.26 | A |
| ATOM | 1358 | C | LEU | A | 172 | 61.017 | 18.490 | -22.944 | 1.00 | 51.09 | A |
| ATOM | 1359 | O | LEU | A | 172 | 60.568 | 19.450 | -23.588 | 1.00 | 50.50 | A |
| ATOM | 1360 | N | VAL | A | 173 | 62.110 | 17.812 | -23.290 | 1.00 | 49.84 | A |
| ATOM | 1361 | CA | VAL | A | 173 | 62.915 | 18.139 | -24.455 | 1.00 | 49.16 | A |
| ATOM | 1362 | CB | VAL | A | 173 | 64.401 | 18.302 | -24.030 | 1.00 | 49.37 | A |
| ATOM | 1363 | CG1 | VAL | A | 173 | 65.336 | 18.369 | -25.244 | 1.00 | 51.16 | A |
| ATOM | 1364 | CG2 | VAL | A | 173 | 64.544 | 19.557 | -23.193 | 1.00 | 48.19 | A |
| ATOM | 1365 | C | VAL | A | 173 | 62.772 | 17.138 | -25.609 | 1.00 | 49.03 | A |
| ATOM | 1366 | O | VAL | A | 173 | 63.001 | 17.490 | -26.777 | 1.00 | 49.26 | A |
| ATOM | 1367 | N | GLY | A | 174 | 62.381 | 15.904 | -25.291 | 1.00 | 47.76 | A |
| ATOM | 1368 | CA | GLY | A | 174 | 62.215 | 14.903 | -26.329 | 1.00 | 45.26 | A |
| ATOM | 1369 | C | GLY | A | 174 | 62.789 | 13.551 | -25.974 | 1.00 | 44.48 | A |
| ATOM | 1370 | O | GLY | A | 174 | 62.781 | 12.634 | -26.802 | 1.00 | 44.57 | A |
| ATOM | 1371 | N | TRP | A | 175 | 63.360 | 13.447 | -24.777 | 1.00 | 42.82 | A |
| ATOM | 1372 | CA | TRP | A | 175 | 63.916 | 12.184 | -24.321 | 1.00 | 42.15 | A |
| ATOM | 1373 | CB | TRP | A | 175 | 65.268 | 12.386 | -23.631 | 1.00 | 40.91 | A |
| ATOM | 1374 | CG | TRP | A | 175 | 66.054 | 11.110 | -23.537 | 1.00 | 40.16 | A |
| ATOM | 1375 | CD2 | TRP | A | 175 | 67.231 | 10.870 | -22.755 | 1.00 | 39.77 | A |
| ATOM | 1376 | CE2 | TRP | A | 175 | 67.604 | 9.527 | -22.967 | 1.00 | 41.26 | A |
| ATOM | 1377 | CE3 | TRP | A | 175 | 68.005 | 11.656 | -21.896 | 1.00 | 39.85 | A |
| ATOM | 1378 | CD1 | TRP | A | 175 | 65.774 | 9.936 | -24.174 | 1.00 | 39.62 | A |
| ATOM | 1379 | NE1 | TRP | A | 175 | 66.694 | 8.982 | -23.838 | 1.00 | 40.81 | A |
| ATOM | 1380 | CZ2 | TRP | A | 175 | 68.728 | 8.947 | -22.346 | 1.00 | 40.79 | A |
| ATOM | 1381 | CZ3 | TRP | A | 175 | 69.122 | 11.079 | -21.280 | 1.00 | 40.90 | A |
| ATOM | 1382 | CH2 | TRP | A | 175 | 69.469 | 9.738 | -21.511 | 1.00 | 39.10 | A |
| ATOM | 1383 | C | TRP | A | 175 | 62.871 | 11.653 | -23.352 | 1.00 | 42.38 | A |
| ATOM | 1384 | O | TRP | A | 175 | 62.701 | 12.203 | -22.255 | 1.00 | 43.36 | A |
| ATOM | 1385 | N | SER | A | 176 | 62.177 | 10.589 | -23.772 | 1.00 | 40.04 | A |
| ATOM | 1386 | CA | SER | A | 176 | 61.072 | 9.985 | -23.011 | 1.00 | 38.31 | A |
| ATOM | 1387 | CB | SER | A | 176 | 61.500 | 9.469 | -21.626 | 1.00 | 36.67 | A |
| ATOM | 1388 | OG | SER | A | 176 | 60.457 | 8.731 | -20.994 | 1.00 | 32.03 | A |
| ATOM | 1389 | C | SER | A | 176 | 60.006 | 11.076 | -22.880 | 1.00 | 37.83 | A |
| ATOM | 1390 | O | SER | A | 176 | 60.019 | 12.056 | -23.640 | 1.00 | 38.73 | A |
| ATOM | 1391 | N | ARG | A | 177 | 59.101 | 10.935 | -21.917 | 1.00 | 35.55 | A |
| ATOM' | 1392 | CA | ARG | A | 177 | 58.055 | 11.934 | -21.754 | 1.00 | 34.08 | A |
| ATOM | 1393 | CB | ARG | A | 177 | 57.199 | 12.031 | -23.027 | 1.00 | 34.10 | A |
| ATOM | 1394 | CG | ARG | A | 177 | 56.591 | 10.712 | -23.446 | 1.00 | 33.29 | A |
| ATOM | 1395 | CD | ARG | A | 177 | 55.620 | 10.859 | -24.583 | 1.00 | 34.72 | A |
| ATOM | 1396 | NE | ARG | A | 177 | 54.989 | 9.576 | -24.871 | 1.00 | 36.74 | A |
| ATOM | 1397 | CZ | ARG | A | 177 | 54.131 | 9.357 | -25.862 | 1.00 | 39.51 | A |
| ATOM | 1398 | NH1 | ARG | A | 177 | 53.779 | 10.347 | -26.683 | 1.00 | 39.55 | A |
| ATOM | 1399 | NH2 | ARG | A | 177 | 53.647 | 8.133 | -26.052 | 1.00 | 39.43 | A |
| ATOM | 1400 | C | ARG | A | 177 | 57.158 | 11.642 | -20.576 | 1.00 | 32.50 | A |
| ATOM | 1401 | O | ARG | A | 177 | 57.133 | 10.525 | -20.061 | 1.00 | 32.06 | A |
| ATOM | 1402 | N | TYR | A | 178 | 56.435 | 12.670 | -20.148 | 1.00 | 31.30 | A |
| ATOM | 1403 | CA | TYR | A | 178 | 55.513 | 12.544 | -19.039 | 1.00 | 30.90 | A |
| ATOM | 1404 | CB | TYR | A | 178 | 55.405 | 13.862 | -18.270 | 1.00 | 30.96 | A |
| ATOM | 1405 | CG | TYR | A | 178 | 56.649 | 14.181 | -17.451 | 1.00 | 31.46 | A |
| ATOM | 1406 | CD1 | TYR | A | 178 | 57.077 | 13.321 | -16.441 | 1.00 | 32.31 | A |
| ATOM | 1407 | CE1 | TYR | A | 178 | 58.240 | 13.570 | -15.720 | 1.00 | 32.22 | A |
| ATOM | 1408 | CD2 | TYR | A | 178 | 57.421 | 15.314 | -17.714 | 1.00 | 31.39 | A |
| ATOM | 1409 | CE2 | TYR | A | 178 | 58.590 | 15.573 | -16.994 | 1.00 | 32.60 | A |
| ATOM | 1410 | CZ | TYR | A | 178 | 58.992 | 14.690 | -16.000 | 1.00 | 32.97 | A |
| ATOM | 1411 | OH | TYR | A | 178 | 60.153 | 14.905 | -15.294 | 1.00 | 32.41 | A |
| ATOM | 1412 | C | TYR | A | 178 | 54.188 | 12.101 | -19.623 | 1.00 | 31.51 | A |
| ATOM | 1413 | O | TYR | A | 178 | 53.662 | 12.719 | -20.550 | 1.00 | 31.31 | A |
| ATOM | 1414 | N | ILE | A | 179 | 53.692 | 10.984 | -19.104 | 1.00 | 32.28 | A |
| ATOM | 1415 | CA | ILE | A | 179 | 52.455 | 10.373 | -19.567 | 1.00 | 31.67 | A |
| ATOM | 1416 | CB | ILE | A | 179 | 52.805 | 9.072 | -20.344 | 1.00 | 31.38 | A |
| ATOM | 1417 | CG2 | ILE | A | 179 | 53.196 | 7.946 | -19.382 | 1.00 | 31.92 | A |
| ATOM | 1418 | CG1 | ILE | A | 179 | 51.672 | 8.659 | -21.273 | 1.00 | 31.70 | A |
| ATOM | 1419 | CD1 | ILE | A | 179 | 51.977 | 7.414 | -22.075 | 1.00 | 31.95 | A |
| ATOM | 1420 | C | ILE | A | 179 | 51.577 | 10.083 | -18.343 | 1.00 | 32.85 | A |
| ATOM | 1421 | O | ILE | A | 179 | 52.099 | 9.770 | -17.269 | 1.00 | 33.92 | A |
| ATOM | 1422 | N | PRO | A | 180 | 50.238 | 10.241 | -18.468 | 1.00 | 34.04 | A |
| ATOM | 1423 | CD | PRO | A | 180 | 49.488 | 10.772 | -19.620 | 1.00 | 34.76 | A |
| ATOM | 1424 | CA | PRO | A | 180 | 49.318 | 9.988 | -17.351 | 1.00 | 33.89 | A |
| ATOM | 1425 | CB | PRO | A | 180 | 47.956 | 10.361 | -17.941 | 1.00 | 33.18 | A |
| ATOM | 1426 | CG | PRO | A | 180 | 48.288 | 11.382 | -18.954 | 1.00 | 33.09 | A |
| ATOM | 1427 | C | PRO | A | 180 | 49.346 | 8.533 | -16.888 | 1.00 | 33.44 | A |
| ATOM | 1428 | O | PRO | A | 180 | 49.413 | 7.615 | -17.707 | 1.00 | 32.18 | A |
| ATOM | 1429 | N | GLU | A | 181 | 49.270 | 8.336 | -15.573 | 1.00 | 32.79 | A |
| ATOM | 1430 | CA | GLU | A | 181 | 49.309 | 6.997 | -15.000 | 1.00 | 32.85 | A |
| ATOM | 1431 | CB | GLU | A | 181 | 50.595 | 6.815 | -14.185 | 1.00 | 31.95 | A |
| ATOM | 1432 | CG | GLU | A | 181 | 51.889 | 7.192 | -14.883 | 1.00 | 31.11 | A |
| ATOM | 1433 | CD | GLU | A | 181 | 53.085 | 6.986 | -13.985 | 1.00 | 31.85 | A |
| ATOM | 1434 | OE1 | GLU | A | 181 | 53.341 | 7.850 | -13.126 | 1.00 | 36.43 | A |
| ATOM | 1435 | OE2 | GLU | A | 181 | 53.759 | 5.952 | -14.116 | 1.00 | 30.75 | A |
| ATOM | 1436 | C | GLU | A | 181 | 48.115 | 6.670 | -14.100 | 1.00 | 33.13 | A |
| ATOM | 1437 | O | GLU | A | 181 | 47.499 | 7.552 | -13.500 | 1.00 | 32.83 | A |
| ATOM | 1438 | N | GLY | A | 182 | 47.821 | 5.382 | -13.985 | 1.00 | 33.34 | A |
| ATOM | 1439 | CA | GLY | A | 182 | 46.738 | 4.945 | -13.128 | 1.00 | 35.62 | A |
| ATOM | 1440 | C | GLY | A | 182 | 45.374 | 5.411 | -13.568 | 1.00 | 36.98 | A |
| ATOM | 1441 | O | GLY | A | 182 | 44.971 | 5.143 | -14.697 | 1.00 | 37.80 | A |
| ATOM | 1442 | N | MET | A | 183 | 44.655 | 6.083 | -12.667 | 1.00 | 37.89 | A |
| ATOM | 1443 | CA | MET | A | 183 | 43.320 | 6.603 | -12.971 | 1.00 | 38.95 | A |
| ATOM | 1444 | CB | MET | A | 183 | 42.591 | 6.994 | -11.690 | 1.00 | 39.12 | A |
| ATOM | 1445 | CG | MET | A | 183 | 42.039 | 5.804 | -10.946 | 1.00 | 38.93 | A |
| ATOM | 1446 | SD | MET | A | 183 | 41.432 | 6.230 | -9.342 | 1.00 | 37.25 | A |
| ATOM | 1447 | CE | MET | A | 183 | 39.792 | 6.715 | -9.728 | 1.00 | 38.52 | A |
| ATOM | 1448 | C | MET | A | 183 | 43.389 | 7.784 | -13.923 | 1.00 | 39.25 | A |
| ATOM | 1449 | O | MET | A | 183 | 42.370 | 8.418 | -14.222 | 1.00 | 37.95 | A |
| ATOM | 1450 | N | GLN | A | 184 | 44.615 | 8.034 | -14.393 | 1.00 | 40.58 | A |
| ATOM | 1451 | CA | GLN | A | 184 | 44.992 | 9.089 | -15.339 | 1.00 | 41.59 | A |
| ATOM | 1452 | CB | GLN | A | 184 | 44.141 | 9.002 | -16.626 | 1.00 | 41.17 | A |
| ATOM | 1453 | CG | GLN | A | 184 | 44.049 | 7.601 | -17.271 | 1.00 | 41.87 | A |
| ATOM | 1454 | CD | GLN | A | 184 | 45.392 | 7.015 | -17.702 | 1.00 | 42.08 | A |
| ATOM | 1455 | OE1 | GLN | A | 184 | 46.046 | 7.527 | -18.608 | 1.00 | 42.56 | A |
| ATOM | 1456 | NE2 | GLN | A | 184 | 45.788 | 5.918 | -17.068 | 1.00 | 41.79 | A |
| ATOM | 1457 | C | GLN | A | 184 | 44.991 | 10.514 | -14.767 | 1.00 | 41.75 | A |
| ATOM | 1458 | O | GLN | A | 184 | 44.767 | 11.475 | -15.496 | 1.00 | 43.20 | A |
| ATOM | 1459 | N | CYS | A | 185 | 45.301 | 10.649 | -13.478 | 1.00 | 42.12 | A |
| ATOM | 1460 | CA | CYS | A | 185 | 45.319 | 11.955 | -12.815 | 1.00 | 40.96 | A |
| ATOM | 1461 | CB | CYS | A | 185 | 44.265 | 11.984 | -11.724 | 1.00 | 40.54 | A |
| ATOM | 1462 | SG | CYS | A | 185 | 42.637 | 11.796 | -12.442 | 1.00 | 38.22 | A |
| ATOM | 1463 | C | CYS | A | 185 | 46.684 | 12.355 | -12.271 | 1.00 | 41.30 | A |
| ATOM | 1464 | O | CYS | A | 185 | 46.853 | 13.432 | -11.682 | 1.00 | 40.82 | A |
| ATOM | 1465 | N | SER | A | 186 | 47.643 | 11.461 | -12.497 | 1.00 | 40.66 | A |
| ATOM | 1466 | CA | SER | A | 186 | 49.039 | 11.615 | -12.121 | 1.00 | 39.17 | A |
| ATOM | 1467 | CB | SER | A | 186 | 49.450 | 10.531 | -11.119 | 1.00 | 38.90 | A |
| ATOM | 1468 | OG | SER | A | 186 | 50.854 | 10.307 | -11.138 | 1.00 | 41.77 | A |
| ATOM | 1469 | C | SER | A | 186 | 49.801 | 11.426 | -13.426 | 1.00 | 38.67 | A |
| ATOM | 1470 | O | SER | A | 186 | 49.301 | 10.798 | -14.353 | 1.00 | 37.02 | A |
| ATOM | 1471 | N | CYS | A | 187 | 51.003 | 11.976 | -13.502 | 1.00 | 39.71 | A |
| ATOM | 1472 | CA | CYS | A | 187 | 51.810 | 11.850 | -14.701 | 1.00 | 40.37 | A |
| ATOM | 1473 | C | CYS | A | 187 | 53.242 | 11.491 | -14.356 | 1.00 | 40.53 | A |
| ATOM | 1474 | O | CYS | A | 187 | 53.859 | 12.129 | -13.512 | 1.00 | 42.25 | A |
| ATOM | 1475 | CB | CYS | A | 187 | 51.760 | 13.149 | -15.517 | 1.00 | 41.63 | A |
| ATOM | 1476 | SG | CYS | A | 187 | 50.432 | 13.208 | -16.775 | 1.00 | 44.40 | A |
| ATOM | 1477 | N | GLY | A | 188 | 53.757 | 10.452 | -15.000 | 1.00 | 39.54 | A |
| ATOM | 1478 | CA | GLY | A | 188 | 55.121 | 10.037 | -14.747 | 1.00 | 39.80 | A |
| ATOM | 1479 | C | GLY | A | 188 | 55.852 | 9.785 | -16.041 | 1.00 | 40.57 | A |
| ATOM | 1480 | O | GLY | A | 188 | 55.376 | 10.176 | -17.100 | 1.00 | 40.06 | A |
| ATOM | 1481 | N | ILE | A | 189 | 57.009 | 9.136 | -15.957 | 1.00 | 42.24 | A |
| ATOM | 1482 | CA | ILE | A | 189 | 57.807 | 8.831 | -17.141 | 1.00 | 44.75 | A |
| ATOM | 1483 | CB | ILE | A | 189 | 59.211 | 8.336 | -16.750 | 1.00 | 44.19 | A |
| ATOM | 1484 | CG2 | ILE | A | 189 | 60.023 | 8.002 | -17.996 | 1.00 | 45.47 | A |
| ATOM | 1485 | CG1 | ILE | A | 189 | 59.925 | 9.398 | -15.908 | 1.00 | 43.12 | A |
| ATOM | 1486 | CD1 | ILE | A | 189 | 60.185 | 10.692 | -16.634 | 1.00 | 39.74 | A |
| ATOM | 1487 | C | ILE | A | 189 | 57.098 | 7.768 | -17.975 | 1.00 | 47.04 | A |
| ATOM | 1488 | O | ILE | A | 189 | 56.319 | 6.977 | -17.446 | 1.00 | 49.30 | A |
| ATOM | 1489 | N | ASP | A | 190 | 57.357 | 7.748 | -19.277 | 1.00 | 48.68 | A |
| ATOM | 1490 | CA | ASP | A | 190 | 56.705 | 6.780 | -20.143 | 1.00 | 49.33 | A |
| ATOM | 1491 | CB | ASP | A | 190 | 56.621 | 7.313 | -21.572 | 1.00 | 50.38 | A |
| ATOM | 1492 | CG | ASP | A | 190 | 55.509 | 6.657 | -22.372 | 1.00 | 52.84 | A |
| ATOM | 1493 | OD1 | ASP | A | 190 | 55.077 | 5.546 | -21.990 | 1.00 | 55.99 | A |
| ATOM | 1494 | OD2 | ASP | A | 190 | 55.054 | 7.249 | -23.378 | 1.00 | 52.41 | A |
| ATOM | 1495 | C | ASP | A | 190 | 57.369 | 5.407 | -20.133 | 1.00 | 49.97 | A |
| ATOM | 1496 | O | ASP | A | 190 | 58.443 | 5.222 | -20.718 | 1.00 | 50.44 | A |
| ATOM | 1497 | N | TYR | A | 191 | 56.717 | 4.456 | -19.458 | 1.00 | 50.22 | A |
| ATOM | 1498 | CA | TYR | A | 191 | 57.197 | 3.068 | -19.365 | 1.00 | 49.75 | A |
| ATOM | 1499 | CB | TYR | A | 191 | 57.219 | 2.549 | -17.914 | 1.00 | 49.03 | A |
| ATOM | 1500 | CG | TYR | A | 191 | 57.343 | 3.578 | -16.810 | 1.00 | 49.66 | A |
| ATOM | 1501 | CD1 | TYR | A | 191 | 58.592 | 4.007 | -16.365 | 1.00 | 49.00 | A |
| ATOM | 1502 | CE1 | TYR | A | 191 | 58.710 | 4.916 | -15.312 | 1.00 | 48.88 | A |
| ATOM | 1503 | CD2 | TYR | A | 191 | 56.210 | 4.086 | -16.174 | 1.00 | 49.91 | A |
| ATOM | 1504 | CE2 | TYR | A | 191 | 56.319 | 4.992 | -15.122 | 1.00 | 49.92 | A |
| ATOM | 1505 | CZ | TYR | A | 191 | 57.571 | 5.405 | -14.695 | 1.00 | 49.91 | A |
| ATOM | 1506 | OH | TYR | A | 191 | 57.685 | 6.309 | -13.660 | 1.00 | 50.22 | A |
| ATOM | 1507 | C | TYR | A | 191 | 56.218 | 2.180 | -20.115 | 1.00 | 48.96 | A |
| ATOM | 1508 | O | TYR | A | 191 | 56.460 | 0.990 | -20.298 | 1.00 | 47.51 | A |
| ATOM | 1509 | N | TYR | A | 192 | 55.108 | 2.780 | -20.528 | 1.00 | 49.02 | A |
| ATOM | 1510 | CA | TYR | A | 192 | 54.027 | 2.079 | -21.202 | 1.00 | 50.76 | A |
| ATOM | 1511 | CB | TYR | A | 192 | 52.717 | 2.828 | -20.946 | 1.00 | 50.13 | A |
| ATOM | 1512 | CG | TYR | A | 192 | 52.457 | 3.113 | -19.485 | 1.00 | 48.06 | A |
| ATOM | 1513 | CD1 | TYR | A | 192 | 51.737 | 2.219 | -18.703 | 1.00 | 46.82 | A |
| ATOM | 1514 | CE1 | TYR | A | 192 | 51.528 | 2.454 | -17.359 | 1.00 | 45.58 | A |
| ATOM | 1515 | CD2 | TYR | A | 192 | 52.960 | 4.262 | -18.878 | 1.00 | 47.60 | A |
| ATOM | 1516 | CE2 | TYR | A | 192 | 52.757 | 4.505 | -17.534 | 1.00 | 45.89 | A |
| ATOM | 1517 | CZ | TYR | A | 192 | 52.041 | 3.595 | -16.782 | 1.00 | 45.58 | A |
| ATOM | 1518 | OH | TYR | A | 192 | 51.847 | 3.814 | -15.443 | 1.00 | 46.91 | A |
| ATOM | 1519 | C | TYR | A | 192 | 54.160 | 1.798 | -22.697 | 1.00 | 52.85 | A |
| ATOM | 1520 | O | TYR | A | 192 | 53.724 | 0.747 | -23.176 | 1.00 | 53.88 | A |
| ATOM | 1521 | N | THR | A | 193 | 54.740 | 2.731 | -23.441 | 1.00 | 54.04 | A |
| ATOM | 1522 | CA | THR | A | 193 | 54.865 | 2.559 | -24.878 | 1.00 | 55.28 | A |
| ATOM | 1523 | CB | THR | A | 193 | 54.243 | 3.756 | -25.603 | 1.00 | 55.77 | A |
| ATOM | 1524 | OG1 | THR | A | 193 | 54.992 | 4.939 | -25.301 | 1.00 | 54.30 | A |
| ATOM | 1525 | CG2 | THR | A | 193 | 52.796 | 3.958 | -25.137 | 1.00 | 56.17 | A |
| ATOM | 1526 | C | THR | A | 193 | 56.297 | 2.374 | -25.343 | 1.00 | 57.68 | A |
| ATOM | 1527 | O | THR | A | 193 | 57.226 | 2.911 | -24.740 | 1.00 | 57.04 | A |
| ATOM | 1528 | N | PRO | A | 194 | 56.495 | 1.588 | -26.418 | 1.00 | 60.86 | A |
| ATOM | 1529 | CD | PRO | A | 194 | 55.466 | 0.900 | -27.221 | 1.00 | 62.53 | A |
| ATOM | 1530 | CA | PRO | A | 194 | 57.836 | 1.339 | -26.961 | 1.00 | 63.02 | A |
| ATOM | 1531 | CB | PRO | A | 194 | 57.576 | 0.282 | -28.039 | 1.00 | 63.10 | A |
| ATOM | 1532 | CG | PRO | A | 194 | 56.195 | 0.636 | -28.527 | 1.00 | 63.84 | A |
| ATOM | 1533 | C | PRO | A | 194 | 58.384 | 2.633 | -27.556 | 1.00 | 64.36 | A |
| ATOM | 1534 | O | PRO | A | 194 | 59.584 | 2.924 | -27.436 | 1.00 | 64.26 | A |
| ATOM | 1535 | N | HIS | A | 195 | 57.462 | 3.408 | -28.142 | 1.00 | 65.63 | A |
| ATOM | 1536 | CA | HIS | A | 195 | 57.707 | 4.705 | -28.782 | 1.00 | 66.79 | A |
| ATOM | 1537 | CB | HIS | A | 195 | 57.053 | 5.829 | -27.966 | 1.00 | 67.12 | A |
| ATOM | 1538 | CG | HIS | A | 195 | 56.642 | 7.023 | -28.780 | 1.00 | 67.81 | A |
| ATOM | 1539 | CD2 | HIS | A | 195 | 56.103 | 7.117 | -30.020 | 1.00 | 68.35 | A |
| ATOM | 1540 | ND1 | HIS | A | 195 | 56.725 | 8.315 | -28.303 | 1.00 | 67.06 | A |
| ATOM | 1541 | CE1 | HIS | A | 195 | 56.252 | 9.151 | -29.211 | 1.00 | 66.74 | A |
| ATOM | 1542 | NE2 | HIS | A | 195 | 55.869 | 8.450 | -30.262 | 1.00 | 67.55 | A |
| ATOM | 1543 | C | HIS | A | 195 | 59.178 | 5.012 | -29.017 | 1.00 | 67.09 | A |
| ATOM | 1544 | O | HIS | A | 195 | 59.797 | 5.784 | -28.284 | 1.00 | 65.38 | A |
| ATOM | 1545 | N | GLU | A | 196 | 59.737 | 4.351 | -30.023 | 1.00 | 68.86 | A |
| ATOM | 1546 | CA | GLU | A | 196 | 61.131 | 4.524 | -30.392 | 1.00 | 70.93 | A |
| ATOM | 1547 | CB | GLU | A | 196 | 61.496 | 3.520 | -31.491 | 1.00 | 72.72 | A |
| ATOM | 1548 | CG | GLU | A | 196 | 60.458 | 3.439 | -32.621 | 1.00 | 75.55 | A |
| ATOM | 1549 | CD | GLU | A | 196 | 60.988 | 2.756 | -33.875 | 1.00 | 77.32 | A |
| ATOM | 1550 | OE1 | GLU | A | 196 | 61.475 | 3.467 | -34.786 | 1.00 | 77.59 | A |
| ATOM | 1551 | OE2 | GLU | A | 196 | 60.913 | 1.511 | -33.953 | 1.00 | 78.12 | A |
| ATOM | 1552 | C | GLU | A | 196 | 61.387 | 5.949 | -30.881 | 1.00 | 71.56 | A |
| ATOM | 1553 | O | GLU | A | 196 | 62.539 | 6.369 | -30.996 | 1.00 | 71.65 | A |
| ATOM | 1554 | N | GLU | A | 197 | 60.304 | 6.691 | -31.125 | 1.00 | 73.16 | A |
| ATOM | 1555 | CA | GLU | A | 197 | 60.363 | 8.073 | -31.630 | 1.00 | 73.94 | A |
| ATOM | 1556 | CB | GLU | A | 197 | 59.054 | 8.448 | -32.364 | 1.00 | 77.25 | A |
| ATOM | 1557 | CG | GLU | A | 197 | 58.404 | 7.341 | -33.269 | 1.00 | 81.76 | A |
| ATOM | 1558 | CD | GLU | A | 197 | 59.180 | 6.991 | -34.565 | 1.00 | 84.28 | A |
| ATOM | 1559 | OE1 | GLU | A | 197 | 59.365 | 7.872 | -35.437 | 1.00 | 84.37 | A |
| ATOM | 1560 | OE2 | GLU | A | 197 | 59.560 | 5.807 | -34.733 | 1.00 | 85.82 | A |
| ATOM | 1561 | C | GLU | A | 197 | 60.721 | 9.156 | -30.589 | 1.00 | 72.18 | A |
| ATOM | 1562 | O | GLU | A | 197 | 60.971 | 10.300 | -30.963 | 1.00 | 70.61 | A |
| ATOM | 1563 | N | THR | A | 198 | 60.675 | 8.809 | -29.295 | 1.00 | 71.95 | A |
| ATOM | 1564 | CA | THR | A | 198 | 61.046 | 9.721 | -28.186 | 1.00 | 70.16 | A |
| ATOM | 1565 | CB | THR | A | 198 | 59.848 | 10.135 | -27.249 | 1.00 | 69.81 | A |
| ATOM | 1566 | OG1 | THR | A | 198 | 59.136 | 8.977 | -26.791 | 1.00 | 69.65 | A |
| ATOM | 1567 | CG2 | THR | A | 198 | 58.907 | 11.086 | -27.946 | 1.00 | 69.56 | A |
| ATOM | 1568 | C | THR | A | 198 | 62.124 | 9.064 | -27.305 | 1.00 | 69.02 | A |
| ATOM | 1569 | O | THR | A | 198 | 62.450 | 9.568 | -26.223 | 1.00 | 68.29 | A |
| ATOM | 1570 | N | ASN | A | 199 | 62.648 | 7.931 | -27.784 | 1.00 | 67.24 | A |
| ATOM | 1571 | CA | ASN | A | 199 | 63.691 | 7.149 | -27.116 | 1.00 | 65.05 | A |
| ATOM | 1572 | CB | ASN | A | 199 | 65.008 | 7.930 | -27.074 | 1.00 | 66.19 | A |
| ATOM | 1573 | CG | ASN | A | 199 | 65.475 | 8.372 | -28.454 | 1.00 | 66.53 | A |
| ATOM | 1574 | OD1 | ASN | A | 199 | 65.445 | 7.594 | -29.414 | 1.00 | 66.10 | A |
| ATOM | 1575 | ND2 | ASK | A | 199 | 65.907 | 9.630 | -28.558 | 1.00 | 65.44 | A |
| ATOM | 1576 | C | ASN | A | 199 | 63.303 | 6.695 | -25.717 | 1.00 | 64.00 | A |
| ATOM | 1577 | O | ASN | A | 199 | 64.085 | 6.821 | -24.770 | 1.00 | 63.12 | A |
| ATOM | 1578 | N | ASN | A | 200 | 62.097 | 6.140 | -25.608 | 1.00 | 63.43 | A |
| ATOM | 1579 | CA | ASN | A | 200 | 61.565 | 5.654 | -24.338 | 1.00 | 62.35 | A |
| ATOM | 1580 | CB | ASH | A | 200 | 60.107 | 5.188 | -24.491 | 1.00 | 57.91 | A |
| ATOM | 1581 | CG | ASN | A | 200 | 59.109 | 6.331 | -24.443 | 1.00 | 52.45 | A |
| ATOM | 1582 | OD1 | ASN | A | 200 | 57.938 | 6.138 | -24.722 | 1.00 | 48.44 | A |
| ATOM | 1583 | ND2 | ASM | A | 200 | 59.565 | 7.514 | -24.070 | 1.00 | 49.94 | A |
| ATOM | 1584 | C | ASN | A | 200 | 62.394 | 4.547 | -23.684 | 1.00 | 63.76 | A |
| ATOM | 1585 | O | ASN | A | 200 | 62.841 | 4.714 | -22.547 | 1.00 | 64.62 | A |
| ATOM | 1586 | N | GLU | A | 201 | 62.624 | 3.436 | -24.387 | 1.00 | 64.15 | A |
| ATOM | 1587 | CA | GLU | A | 201 | 63.389 | 2.347 | -23.782 | 1.00 | 65.48 | A |
| ATOM | 1588 | CB | GLU | A | 201 | 63.448 | 1.105 | -24.710 | 1.00 | 67.62 | A |
| ATOM | 1589 | CG | GLU | A | 201 | 63.927 | -0.205 | -23.999 | 1.00 | 69.07 | A |
| ATOM | 1590 | CD | GLU | A | 201 | 63.529 | -1.515 | -24.705 | 1.00 | 70.80 | A |
| ATOM | 1591 | OE1 | GLU | A | 201 | 63.416 | -2.557 | -24.012 | 1.00 | 69.56 | A |
| ATOM | 1592 | OE2 | GLU | A | 201 | 63.340 | -1.512 | -25.94 | 1.00 | 72.96 | A |
| ATOM | 1593 | C | GLU | A | 201 | 64.786 | 2.802 | -23.306 | 1.00 | 65.24 | A |
| ATOM | 1594 | O | GLU | A | 201 | 65.357 | 2.220 | -22.380 | 1.00 | 65.67 | A |
| ATOM | 1595 | N | SER | A | 202 | 65.279 | 3.910 | -23.859 | 1.00 | 63.81 | A |
| ATOM | 1596 | CA | SER | A | 202 | 66.597 | 4.432 | -23.494 | 1.00 | 61.85 | A |
| ATOM | 1597 | CB | SER | A | 202 | 67.113 | 5.351 | -24.596 | 1.00 | 61.44 | A |
| ATOM | 1598 | OG | SER | A | 202 | 68.398 | 5.836 | -24.265 | 1.00 | 62.62 | A |
| ATOM | 1599 | C | SER | A | 202 | 66.652 | 5.167 | -22.151 | 1.00 | 60.45 | A |
| ATOM | 1600 | O | SER | A | 202 | 67.478 | 4.850 | -21.291 | 1.00 | 60.38 | A |
| ATOM | 1601 | N | PHE | A | 203 | 65.785 | 6.165 | -22.000 | 1.00 | 58.93 | A |
| ATOM | 1602 | CA | PHE | A | 203 | 65.699 | 6.983 | -20.791 | 1.00 | 57.14 | A |
| ATOM | 1603 | CB | PHE | A | 203 | 64.661 | 8.082 | -20.989 | 1.00 | 58.46 | A |
| ATOM | 1604 | CG | PHE | A | 203 | 64.447 | 8.947 | -19.780 | 1.00 | 59.16 | A |
| ATOM | 1605 | CD1 | PHE | A | 203 | 63.423 | 8.668 | -18.881 | 1.00 | 59.25 | A |
| ATOM | 1606 | CD2 | PHE | A | 203 | 65.249 | 10.057 | -19.553 | 1.00 | 59.46 | A |
| ATOM | 1607 | CE1 | PHE | A | 203 | 63.200 | 9.484 | -17.778 | 1.00 | 58.29 | A |
| ATOM | 1608 | CE2 | PHE | A | 203 | 65.030 | 10.878 | -18.450 | 1.00 | 59.35 | A |
| ATOM | 1609 | CZ | PHE | A | 203 | 64.002 | 10.588 | -17.563 | 1.00 | 58.64 | A |
| ATOM | 1610 | C | PHE | A | 203 | 65.363 | 6.206 | -19.529 | 1.00 | 55.81 | A |
| ATOM | 1611 | O | PHE | A | 203 | 65.951 | 6.446 | -18.483 | 1.00 | 55.22 | A |
| ATOM | 1612 | N | VAL | A | 204 | 64.372 | 5.326 | -19.617 | 1.00 | 54.97 | A |
| ATOM | 1613 | CA | VAL | A | 204 | 63.958 | 4.515 | -18.476 | 1.00 | 54.69 | A |
| ATOM | 1614 | CB | VAL | A | 204 | 62.863 | 3.499 | -18.869 | 1.00 | 54.17 | A |
| ATOM | 1615 | CG1 | VAL | A | 204 | 62.388 | 2.735 | -17.643 | 1.00 | 53.41 | A |
| ATOM | 1616 | CG2 | VAL | A | 204 | 61.699 | 4.203 | -19.543 | 1.00 | 55.09 | A |
| ATOM | 1617 | C | VAL | A | 204 | 65.142 | 3.751 | -17.883 | 1.00 | 55.32 | A |
| ATOM | 1618 | O | VAL | A | 204 | 65.281 | 3.665 | -16.665 | 1.00 | 55.93 | A |
| ATOM | 1619 | N | ILE | A | 205 | 65.981 | 3.185 | -18.751 | 1.00 | 55.80 | A |
| ATOM | 1620 | CA | ILE | A | 205 | 67.159 | 2.437 | -18.320 | 1.00 | 55.29 | A |
| ATOM | 1621 | CB | ILE | A | 205 | 67.841 | 1.703 | -19.506 | 1.00 | 56.85 | A |
| ATOM | 1622 | CG2 | ILE | A | 205 | 69.145 | 1.058 | -19.051 | 1.00 | 57.68 | A |
| ATOM | 1623 | CG1 | ILE | A | 205 | 66.909 | 0.628 | -20.076 | 1.00 | 57.39 | A |
| ATOM | 1624 | CD1 | ILE | A | 205 | 67.488 | -0.148 | -21.250 | 1.00 | 56.19 | A |
| ATOM | 1625 | C | ILE | A | 205 | 68.139 | 3.428 | -17.711 | 1.00 | 54.83 | A |
| ATOM | 1626 | O | ILE | A | 205 | 68.774 | 3.140 | -16.695 | 1.00 | 54.58 | A |
| ATOM | 1627 | N | TYR | A | 206 | 68.247 | 4.597 | -18.340 | 1.00 | 54.28 | A |
| ATOM | 1628 | CA | TYR | A | 206 | 69.123 | 5.664 | -17.864 | 1.00 | 54.24 | A |
| ATOM | 1629 | CB | TYR | A | 206 | 69.098 | 6.841 | -18.866 | 1.00 | 56.12 | A |
| ATOM | 1630 | CG | TYR | A | 206 | 69.473 | 8.221 | -18.315 | 1.00 | 58.72 | A |
| ATOM | 1631 | CD1 | TYR | A | 206 | 68.492 | 9.203 | -18.119 | 1.00 | 59.81 | A |
| ATOM | 1632 | CE1 | TYR | A | 206 | 68.819 | 10.482 | -17.648 | 1.00 | 59.55 | A |
| ATOM | 1633 | CD2 | TYR | A | 206 | 70.802 | 8.556 | -18.023 | 1.00 | 59.50 | A |
| ATOM | 1634 | CE2 | TYR | A | 206 | 71.139 | 9.838 | -17.552 | 1.00 | 60.11 | A |
| ATOM | 1635 | CZ | TYR | A | 206 | 70.138 | 10.791 | -17.369 | 1.00 | 60.22 | A |
| ATOM | 1636 | OH | TYR | A | 206 | 70.449 | 12.050 | -16.908 | 1.00 | 59.94 | A |
| ATOM | 1637 | C | TYR | A | 206 | 68.702 | 6.111 | -16.453 | 1.00 | 52.77 | A |
| ATOM | 1638 | O | TYR | A | 206 | 69.497 | 6.060 | -15.516 | 1.00 | 53.05 | A |
| ATOM | 1639 | N | MET | A | 207 | 67.432 | 6.469 | -16.296 | 1.00 | 50.16 | A |
| ATOM | 1640 | CA | MET | A | 207 | 66.913 | 6.934 | -15.019 | 1.00 | 47.97 | A |
| ATOM | 1641 | CB | MET | A | 207 | 65.451 | 7.321 | -15.165 | 1.00 | 49.19 | A |
| ATOM | 1642 | CG | MET | A | 207 | 64.827 | 7.832 | -13.887 | 1.00 | 50.67 | A |
| ATOM | 1643 | SD | MET | A | 207 | 63.054 | 7.999 | -14.070 | 1.00 | 55.24 | A |
| ATOM | 1644 | CE | MET | A | 207 | 62.596 | 6.274 | -14.424 | 1.00 | 51.51 | A |
| ATOM | 1645 | C | MET | A | 207 | 67.065 | 5.940 | -13.880 | 1.00 | 46.78 | A |
| ATOM | 1646 | O | MET | A | 207 | 67.324 | 6.324 | -12.744 | 1.00 | 47.05 | A |
| ATOM | 1647 | N | PHE | A | 208 | 66.874 | 4.664 | -14.172 | 1.00 | 45.32 | A |
| ATOM | 1648 | CA | PHE | A | 208 | 67.001 | 3.647 | -13.145 | 1.00 | 44.18 | A |
| ATOM | 1649 | CB | PHE | A | 208 | 66.328 | 2.355 | -13.590 | 1.00 | 44.12 | A |
| ATOM | 1650 | CG | PHE | A | 208 | 64.900 | 2.240 | -13.156 | 1.00 | 43.41 | A |
| ATOM | 1651 | CD1 | PHE | A | 208 | 64.574 | 1.592 | -11.971 | 1.00 | 44.18 | A |
| ATOM | 1652 | CD2 | PHE | A | 208 | 63.876 | 2.767 | -13.933 | 1.00 | 44.15 | A |
| ATOM | 1653 | CE1 | PHE | A | 208 | 63.252 | 1.472 | -11.571 | 1.00 | 43.18 | A |
| ATOM | 1654 | CE2 | PHE | A | 208 | 62.550 | 2.652 | -13.541 | 1.00 | 40.59 | A |
| ATOM | 1655 | CZ | PHE | A | 208 | 62.241 | 2.006 | -12.364 | 1.00 | 42.24 | A |
| ATOM | 1656 | C | PHE | A | 208 | 68.442 | 3.378 | -12.730 | 1.00 | 43.64 | A |
| ATOM | 1657 | O | PHE | A | 208 | 68.694 | 2.938 | -11.614 | 1.00 | 43.83 | A |
| ATOM | 1658 | N | VAL | A | 209 | 69.385 | 3.631 | -13.629 | 1.00 | 43.79 | A |
| ATOM | 1659 | CA | VAL | A | 209 | 70.795 | 3.415 | -13.322 | 1.00 | 45.46 | A |
| ATOM | 1660 | CB | VAL | A | 209 | 71.621 | 3.100 | -14.617 | 1.00 | 46.20 | A |
| ATOM | 1661 | CG1 | VAL | A | 209 | 73.103 | 2.972 | -14.282 | 1.00 | 47.29 | A |
| ATOM | 1662 | CG2 | VAL | A | 209 | 71.130 | 1.807 | -15.272 | 1.00 | 45.39 | A |
| ATOM | 1663 | C | VAL | A | 209 | 71.373 | 4.653 | -12.622 | 1.00 | 45.19 | A |
| ATOM | 1664 | O | VAL | A | 209 | 71.730 | 4.618 | -11.442 | 1.00 | 44.77 | A |
| ATOM | 1665 | N | VAL | A | 210 | 71.404 | 5.756 | -13.360 | 1.00 | 45.47 | A |
| ATOM | 1666 | CA | VAL | A | 210 | 71.930 | 7.031 | -12.885 | 1.00 | 46.19 | A |
| ATOM | 1667 | CB | VAL | A | 210 | 72.035 | 8.040 | -14.061 | 1.00 | 45.88 | A |
| ATOM | 1668 | CG1 | VAL | A | 210 | 72.652 | 9.358 | -13.602 | 1.00 | 42.07 | A |
| ATOM | 1669 | CG2 | VAL | A | 210 | 72.823 | 7.421 | -15.215 | 1.00 | 44.93 | A |
| ATOM | 1670 | C | VAL | A | 210 | 71.122 | 7.689 | -11.772 | 1.00 | 47.22 | A |
| ATOM | 1671 | O | VAL | A | 210 | 71.697 | 8.329 | -10.893 | 1.00 | 48.22 | A |
| ATOM | 1672 | N | HIS | A | 211 | 69.802 | 7.514 | -11.793 | 1.00 | 47.64 | A |
| ATOM | 1673 | CA | HIS | A | 211 | 68.932 | 8.149 | -10.803 | 1.00 | 48.22 | A |
| ATOM | 1674 | CB | HIS | A | 211 | 67.981 | 9.121 | -11.509 | 1.00 | 49.09 | A |
| ATOM | 1675 | CG | HIS | A | 211 | 68.680 | 10.151 | -12.338 | 1.00 | 48.24 | A |
| ATOM | 1676 | CD2 | HIS | A | 211 | 68.984 | 10.176 | -13.656 | 1.00 | 49.84 | A |
| ATOM | 1677 | ND1 | HIS | A | 211 | 69.170 | 11.325 | -11.810 | 1.00 | 48.40 | A |
| ATOM | 1678 | CE1 | HIS | A | 211 | 69.749 | 12.027 | -12.766 | 1.00 | 50.69 | A |
| ATOM | 1679 | NE2 | HIS | A | 211 | 69.649 | 11.353 | -13.897 | 1.00 | 51.54 | A |
| ATOM | 1680 | C | HIS | A | 211 | 68.137 | 7.255 | -9.851 | 1.00 | 48.24 | A |
| ATOM | 1681 | O | HIS | A | 211 | 67.027 | 7.612 | -9.440 | 1.00 | 47.64 | A |
| ATOM | 1682 | N | PHE | A | 212 | 68.718 | 6.124 | -9.463 | 1.00 | 48.14 | A |
| ATOM | 1683 | CA | PHE | A | 212 | 68.053 | 5.209 | -8.545 | 1.00 | 47.62 | A |
| ATOM | 1684 | CB | PHE | A | 212 | 66.879 | 4.519 | -9.233 | 1.00 | 48.00 | A |
| ATOM | 1685 | CG | PHE | A | 212 | 66.121 | 3.593 | -8.337 | 1.00 | 49.71 | A |
| ATOM | 1686 | CD1 | PHE | A | 212 | 65.637 | 4.036 | -7.111 | 1.00 | 50.38 | A |
| ATOM | 1687 | CD2 | PHE | A | 212 | 65.881 | 2.276 | -8.720 | 1.00 | 51.02 | A |
| ATOM | 1688 | CE1 | PHE | A | 212 | 64.923 | 3.183 | -6.278 | 1.00 | 51.84 | A |
| ATOM | 1689 | CE2 | PHE | A | 212 | 65.168 | 1.409 | -7.896 | 1.00 | 50.93 | A |
| ATOM | 1690 | CZ | PHE | A | 212 | 64.688 | 1.862 | -6.673 | 1.00 | 51.86 | A |
| ATOM | 1691 | C | PHE | A | 212 | 69.017 | 4.172 | -7.990 | 1.00 | 47.79 | A |
| ATOM | 1692 | O | PHE | A | 212 | 69.334 | 4.186 | -6.804 | 1.00 | 47.09 | A |
| ATOM | 1693 | N | ILE | A | 213 | 69.472 | 3.267 | -8.850 | 1.00 | 48.89 | A |
| ATOM | 1694 | CA | ILE | A | 213 | 70.411 | 2.219 | -8.453 | 1.00 | 49.50 | A |
| ATOM | 1695 | CB | ILE | A | 213 | 70.735 | 1.276 | -9.641 | 1.00 | 50.74 | A |
| ATOM | 1696 | CG2 | ILE | A | 213 | 71.921 | 0.372 | -9.300 | 1.00 | 51.20 | A |
| ATOM | 1697 | CG1 | ILE | A | 213 | 69.499 | 0.449 | -10.015 | 1.00 | 51.21 | A |
| ATOM | 1698 | CD1 | ILE | A | 213 | 69.690 | -0.429 | -11.251 | 1.00 | 51.53 | A |
| ATOM | 1699 | C | ILE | A | 213 | 71.715 | 2.815 | -7.923 | 1.00 | 48.52 | A |
| ATOM | 1700 | O | ILE | A | 213 | 72.127 | 2.517 | -6.803 | 1.00 | 47.53 | A |
| ATOM | 1701 | N | ILE | A | 214 | 72.336 | 3.683 | -8.718 | 1.00 | 47.85 | A |
| ATOM | 1702 | CA | ILE | A | 214 | 73.591 | 4.298 | -8.324 | 1.00 | 47.37 | A |
| ATOM | 1703 | CB | ILE | A | 214 | 74.192 | 5.147 | -9.444 | 1.00 | 46.77 | A |
| ATOM | 1704 | CG2 | ILE | A | 214 | 75.318 | 6.005 | -8.908 | 1.00 | 47.36 | A |
| ATOM | 1705 | CG1 | ILE | A | 214 | 74.734 | 4.223 | -10.532 | 1.00 | 47.27 | A |
| ATOM | 1706 | CD1 | ILE | A | 214 | 75.374 | 4.947 | -11.688 | 1.00 | 48.53 | A |
| ATOM | 1707 | C | ILE | A | 214 | 73.525 | 5.069 | -7.015 | 1.00 | 47.15 | A |
| ATOM | 1708 | O | ILE | A | 214 | 74.192 | 4.690 | -6.053 | 1.00 | 49.27 | A |
| ATOM' | 1709 | N | PRO | A | 215 | 72.728 | 6.149 | -6.945 | 1.00 | 45.44 | A |
| ATOM | 1710 | CD | PRO | A | 215 | 71.879 | 6.799 | -7.960 | 1.00 | 45.26 | A |
| ATOM | 1711 | CA | PRO | A | 215 | 72.673 | 6.877 | -5.674 | 1.00 | 44.93 | A |
| ATOM | 1712 | CB | PRO | A | 215 | 71.503 | 7.828 | -5.886 | 1.00 | 43.60 | A |
| ATOM | 1713 | CG | PRO | A | 215 | 71.612 | 8.149 | -7.340 | 1.00 | 43.62 | A |
| ATOM | 1714 | C | PRO | A | 215 | 72.447 | 5.948 | -4.473 | 1.00 | 45.85 | A |
| ATOM | 1715 | O | PRO | A | 215 | 73.084 | 6.109 | -3.436 | 1.00 | 45.64 | A |
| ATOM | 1716 | N | LEU | A | 216 | 71.606 | 4.929 | -4.648 | 1.00 | 47.60 | A |
| ATOM | 1717 | CA | LEU | A | 216 | 71.315 | 3.981 | -3.574 | 1.00 | 48.84 | A |
| ATOM | 1718 | CB | LEU | A | 216 | 70.124 | 3.111 | -3.927 | 1.00 | 48.33 | A |
| ATOM | 1719 | CG | LEU | A | 216 | 68.797 | 3.845 | -3.848 | 1.00 | 49.44 | A |
| ATOM | 1720 | CD1 | LEU | A | 216 | 67.714 | 2.890 | -4.266 | 1.00 | 51.97 | A |
| ATOM | 1721 | CD2 | LEU | A | 216 | 68.551 | 4.355 | -2.442 | 1.00 | 49.11 | A |
| ATOM | 1722 | C | LEU | A | 216 | 72.494 | 3.101 | -3.227 | 1.00 | 50.75 | A |
| ATOM | 1723 | O | LEU | A | 216 | 72.641 | 2.678 | -2.082 | 1.00 | 52.08 | A |
| ATOM | 1724 | N | ILE | A | 217 | 73.301 | 2.783 | -4.231 | 1.00 | 52.17 | A |
| ATOM | 1725 | CA | ILE | A | 217 | 74.497 | 1.975 | -4.028 | 1.00 | 53.69 | A |
| ATOM | 1726 | CB | ILE | A | 217 | 75.163 | 1.625 | -5.407 | 1.00 | 55.46 | A |
| ATOM | 1727 | CG2 | ILE | A | 217 | 76.677 | 1.802 | -5.361 | 1.00 | 56.63 | A |
| ATOM | 1728 | CG1 | ILE | A | 217 | 74.818 | 0.192 | -5.819 | 1.00 | 55.89 | A |
| ATOM | 1729 | CD1 | ILE | A | 217 | 73.338 | -0.127 | -5.797 | 1.00 | 58.18 | A |
| ATOM | 1730 | C | ILE | A | 217 | 75.446 | 2.784 | -3.127 | 1.00 | 53.47 | A |
| ATOM | 1731 | O | ILE | A | 217 | 75.937 | 2.277 | -2.113 | 1.00 | 53.61 | A |
| ATOM | 1732 | N | VAL | A | 218 | 75.620 | 4.062 | -3.470 | 1.00 | 52.29 | A |
| ATOM | 1733 | CA | VAL | A | 218 | 76.479 | 4.977 | -2.728 | 1.00 | 51.83 | A |
| ATOM | 1734 | CB | VAL | A | 218 | 76.587 | 6.358 | -3.449 | 1.00 | 49.95 | A |
| ATOM | 1735 | CG1 | VAL | A | 218 | 77.363 | 7.359 | -2.606 | 1.00 | 47.74 | A |
| ATOM | 1736 | CG2 | VAL | A | 218 | 77.273 | 6.195 | -4.786 | 1.00 | 48.14 | A |
| ATOM | 1737 | C | VAL | A | 218 | 75.993 | 5.176 | -1.291 | 1.00 | 54.04 | A |
| ATOM | 1738 | O | VAL | A | 218 | 76.795 | 5.152 | -0.359 | 1.00 | 55.49 | A |
| ATOM | 1739 | N | ILE | A | 219 | 74.687 | 5.358 | -1.103 | 1.00 | 55.83 | A |
| ATOM | 1740 | CA | ILE | A | 219 | 74.142 | 5.560 | 0.240 | 1.00 | 57.98 | A |
| ATOM | 1741 | CB | ILE | A | 219 | 72.689 | 6.074 | 0.209 | 1.00 | 56.82 | A |
| ATOM | 1742 | CG2 | ILE | A | 219 | 72.301 | 6.579 | 1.594 | 1.00 | 57.01 | A |
| ATOM | 1743 | CG1 | ILE | A | 219 | 72.552 | 7.228 | -0.785 | 1.00 | 56.44 | A |
| ATOM | 1744 | CD1 | ILE | A | 219 | 71.131 | 7.684 | -1.015 | 1.00 | 54.37 | A |
| ATOM | 1745 | C | ILE | A | 219 | 74.182 | 4.266 | 1.050 | 1.00 | 60.44 | A |
| ATOM | 1746 | O | ILE | A | 219 | 74.228 | 4.285 | 2.282 | 1.00 | 61.30 | A |
| ATOM | 1747 | N | PHE | A | 220 | 74.154 | 3.142 | 0.350 | 1.00 | 62.89 | A |
| ATOM | 1748 | CA | PHE | A | 220 | 74.196 | 1.857 | 1.009 | 1.00 | 66.43 | A |
| ATOM | 1749 | CB | PHE | A | 220 | 73.458 | 0.814 | 0.183 | 1.00 | 67.98 | A |
| ATOM | 1750 | CG | PHE | A | 220 | 71.971 | 0.832 | 0.390 | 1.00 | 69.78 | A |
| ATOM | 1751 | CD1 | PHE | A | 220 | 71.108 | 0.351 | -0.591 | 1.00 | 71.00 | A |
| ATOM | 1752 | CD2 | PHE | A | 220 | 71.430 | 1.311 | 1.580 | 1.00 | 70.17 | A |
| ATOM | 1753 | CE1 | PHE | A | 220 | 69.726 | 0.345 | -0.388 | 1.00 | 70.24 | A |
| ATOM | 1754 | CE2 | PHE | A | 220 | 70.055 | 1.309 | 1.791 | 1.00 | 70.51 | A |
| ATOM | 1755 | CZ | PHE | A | 220 | 69.203 | 0.825 | 0.805 | 1.00 | 69.79 | A |
| ATOM | 1756 | C | PHE | A | 220 | 75.611 | 1.415 | 1.330 | 1.00 | 68.77 | A |
| ATOM | 1757 | O | PHE | A | 220 | 75.815 | 0.525 | 2.162 | 1.00 | 69.58 | A |
| ATOM | 1758 | N | PHE | A | 221 | 76.588 | 2.024 | 0.663 | 1.00 | 71.19 | A |
| ATOM | 1759 | CA | PHE | A | 221 | 77.982 | 1.702 | 0.932 | 1.00 | 73.13 | A |
| ATOM | 1760 | CB | PHE | A | 221 | 78.829 | 1.683 | -0.339 | 1.00 | 74.82 | A |
| ATOM | 1761 | CG | PHE | A | 221 | 80.197 | 1.091 | -0.133 | 1.00 | 77.85 | A |
| ATOM | 1762 | CD1 | PHE | A | 221 | 80.409 | 0.129 | 0.858 | 1.00 | 78.13 | A |
| ATOM | 1763 | CD2 | PHE | A | 221 | 81.280 | 1.514 | -0.897 | 1.00 | 79.33 | A |
| ATOM | 1764 | CE1 | PHE | A | 221 | 81.675 | -0.399 | 1.088 | 1.00 | 79.02 | A |
| ATOM | 1765 | CE2 | PHE | A | 221 | 82.558 | 0.990 | -0.677 | 1.00 | 80.04 | A |
| ATOM | 1766 | CZ | PHE | A | 221 | 82.755 | 0.032 | 0.320 | 1.00 | 80.20 | A |
| ATOM | 1767 | C | PHE | A | 221 | 78.558 | 2.685 | 1.950 | 1.00 | 73.19 | A |
| ATOM | 1768 | O | PHE | A | 221 | 79.504 | 2.359 | 2.659 | 1.00 | 74.57 | A |
| ATOM | 1769 | N | CYS | A | 222 | 78.011 | 3.896 | 2.004 | 1.00 | 73.37 | A |
| ATOM | 1770 | CA | CYS | A | 222 | 78.464 | 4.876 | 2.985 | 1.00 | 73.68 | A |
| ATOM | 1771 | CB | CYS | A | 222 | 77.871 | 6.263 | 2.709 | 1.00 | 72.81 | A |
| ATOM | 1772 | SG | CYS | A | 222 | 77.937 | 7.404 | 4.137 | 1.00 | 67.01 | A |
| ATOM | 1773 | C | CYS | A | 222 | 77.974 | 4.373 | 4.338 | 1.00 | 75.11 | A |
| ATOM | 1774 | O | CYS | A | 222 | 78.747 | 4.292 | 5.288 | 1.00 | 75.31 | A |
| ATOM | 1775 | N | TYR | A | 223 | 76.691 | 4.009 | 4.397 | 1.00 | 76.47 | A |
| ATOM | 1776 | CA | TYR | A | 223 | 76.069 | 3.495 | 5.613 | 1.00 | 77.92 | A |
| ATOM | 1777 | CB | TYR | A | 223 | 74.553 | 3.350 | 5.422 | 1.00 | 77.70 | A |
| ATOM | 1778 | CG | TYR | A | 223 | 73.861 | 2.629 | 6.560 | 1.00 | 78.10 | A |
| ATOM | 1779 | CD1 | TYR | A | 223 | 74.026 | 3.046 | 7.881 | 1.00 | 79.19 | A |
| ATOM | 1780 | CE1 | TYR | A | 223 | 73.441 | 2.344 | 8.943 | 1.00 | 79.08 | A |
| ATOM | 1781 | CD2 | TYR | A | 223 | 73.086 | 1.496 | 6.325 | 1.00 | 78.26 | A |
| ATOM | 1782 | CE2 | TYR | A | 223 | 72.495 | 0.787 | 7.379 | 1.00 | 78.09 | A |
| ATOM | 1783 | CZ | TYR | A | 223 | 72.678 | 1.215 | 8.685 | 1.00 | 78.27 | A |
| ATOM | 1784 | OH | TYR | A | 223 | 72.112 | 0.514 | 9.732 | 1.00 | 77.54 | A |
| ATOM | 1785 | C | TYR | A | 223 | 76.697 | 2.161 | 6.033 | 1.00 | 79.81 | A |
| ATOM | 1786 | O | TYR | A | 223 | 76.542 | 1.726 | 7.172 | 1.00 | 79.33 | A |
| ATOM | 1787 | N | GLY | A | 224 | 77.396 | 1.513 | 5.105 | 1.00 | 82.45 | A |
| ATOM | 1788 | CA | GLY | A | 224 | 78.065 | 0.256 | 5.411 | 1.00 | 85.27 | A |
| ATOM | 1789 | C | GLY | A | 224 | 79.398 | 0.538 | 6.084 | 1.00 | 87.14 | A |
| ATOM | 1790 | O | GLY | A | 224 | 79.954 | -0.312 | 6.784 | 1.00 | 86.59 | A |
| ATOM | 1791 | N | GLN | A | 225 | 79.921 | 1.736 | 5.830 | 1.00 | 89.82 | A |
| ATOM | 1792 | CA | GLN | A | 225 | 81.177 | 2.193 | 6.409 | 1.00 | 93.05 | A |
| ATOM | 1793 | CB | GLN | A | 225 | 81.850 | 3.228 | 5.498 | 1.00 | 92.45 | A |
| ATOM | 1794 | CG | QLM | A | 225 | 82.159 | 2.744 | 4.084 | 1.00 | 91.90 | A |
| ATOM | 1795 | CD | GLN | A | 225 | 83.095 | 1.548 | 4.049 | 1.00 | 92.36 | A |
| ATOM | 1796 | OE1 | GLN | A | 225 | 84.277 | 1.676 | 3.719 | 1.00 | 92.80 | A |
| ATOM | 1797 | NE2 | GLN | A | 225 | 82.565 | 0.371 | 4.372 | 1.00 | 92.21 | A |
| ATOM | 1798 | C | GLN | A | 225 | 80.873 | 2.804 | 7.780 | 1.00 | 95.85 | A |
| ATOM | 1799 | O | GLN | A | 225 | 81.645 | 2.634 | 8.724 | 1.00 | 96.09 | A |
| ATOM | 1800 | N | LEU | A | 226 | 79.757 | 3.530 | 7.882 | 1.00 | 99.41 | A |
| ATOM | 1801 | CA | LEU | A | 226 | 79.343 | 4.128 | 9.154 | 1.00 | 102.99 | A |
| ATOM | 1802 | CB | LEU | A | 226 | 78.371 | 5.300 | 8.967 | 1.00 | 102.22 | A |
| ATOM | 1803 | CG | LEU | A | 226 | 78.910 | 6.624 | 8.427 | 1.00 | 101.91 | A |
| ATOM | 1804 | CD1 | LEU | A | 226 | 77.984 | 7.742 | 8.875 | 1.00 | 101.14 | A |
| ATOM | 1805 | CD2 | LEU | A | 226 | 80.312 | 6.875 | 8.940 | 1.00 | 101.29 | A |
| ATOM | 1806 | C | LEU | A | 226 | 78.686 | 3.057 | 10.011 | 1.00 | 105.94 | A |
| ATOM | 1807 | O | LEU | A | 226 | 77.498 | 3.135 | 10.343 | 1.00 | 105.92 | A |
| ATOM | 1808 | N | VAL | A | 227 | 79.469 | 2.017 | 10.274 | 1.00 | 109.45 | A |
| ATOM | 1809 | CA | VAL | A | 227 | 79.097 | 0.871 | 11.091 | 1.00 | 113.40 | A |
| ATOM | 1810 | CB | VAL | A | 227 | 78.493 | -0.293 | 10.242 | 1.00 | 112.97 | A |
| ATOM | 1811 | CG1 | VAL | A | 227 | 78.420 | -1.572 | 11.059 | 1.00 | 113.09 | A |
| ATOM | 1812 | CG2 | VAL | A | 227 | 77.089 | 0.063 | 9.778 | 1.00 | 112.90 | A |
| ATOM | 1813 | C | VAL | A | 227 | 80.412 | 0.447 | 11.750 | 1.00 | 116.65 | A |
| ATOM | 1814 | O | VAL | A | 227 | 80.411 | -0.112 | 12.846 | 1.00 | 117.73 | A |
| ATOM | 1815 | N | PHE | A | 228 | 81.530 | 0.767 | 11.089 | 1.00 | 120.47 | A |
| ATOM | 1816 | CA | PHE | A | 228 | 82.878 | 0.466 | 11.594 | 1.00 | 123.95 | A |
| ATOM | 1817 | CB | PHE | A | 228 | 83.967 | 0.993 | 10.631 | 1.00 | 123.54 | A |
| ATOM | 1818 | CG | PHE | A | 228 | 84.204 | 0.139 | 9.398 | 1.00 | 123.18 | A |
| ATOM | 1819 | CD1 | PHE | A | 228 | 83.403 | -0.965 | 9.104 | 1.00 | 122.93 | A |
| ATOM | 1820 | CD2 | PHE | A | 228 | 85.241 | 0.464 | 8.520 | 1.00 | 122.50 | A |
| ATOM | 1821 | CE1 | PHE | A | 228 | 83.631 | -1.730 | 7.954 | 1.00 | 122.04 | A |
| ATOM | 1822 | CE2 | PHE | A | 228 | 85.476 | -0.292 | 7.374 | 1.00 | 122.04 | A |
| ATOM | 1823 | CZ | PHE | A | 228 | 84.668 | -1.391 | 7.090 | 1.00 | 122.05 | A |
| ATOM | 1824 | C | PHE | A | 228 | 83.067 | 1.173 | 12.943 | 1.00 | 126.55 | A |
| ATOM | 1825 | O | PHE | A | 228 | 83.707 | 0.637 | 13.858 | 1.00 | 126.84 | A |
| ATOM | 1826 | N | THR | A | 229 | 82.502 | 2.379 | 13.048 | 1.00 | 129.19 | A |
| ATOM | 1827 | CA | THR | A | 229 | 82.610 | 3.196 | 14.256 | 1.00 | 131.49 | A |
| ATOM | 1828 | CB | THR | A | 229 | 83.612 | 4.378 | 14.049 | 1.00 | 131.74 | A |
| ATOM | 1829 | OG1 | THR | A | 229 | 83.289 | 5.086 | 12.844 | 1.00 | 131.60 | A |
| ATOM | 1830 | CG2 | THR | A | 229 | -85.053 | 3.866 | 13.965 | 1.00 | 131.75 | A |
| ATOM | 1831 | C | THR | A | 229 | 81.298 | 3.745 | 14.850 | 1.00 | 132.70 | A |
| ATOM | 1832 | O | THR | A | 229 | 81.228 | 3.969 | 16.060 | 1.00 | 133.27 | A |
| ATOM | 1833 | N | VAL | A | 230 | 80.268 | 3.957 | 14.026 | 1.00 | 133.77 | A |
| ATOM | 1834 | CA | VAL | A | 230 | 78.987 | 4.496 | 14.518 | 1.00 | 134.91 | A |
| ATOM | 1835 | CB | VAL | A | 230 | 78.898 | 6.044 | 14.304 | 1.00 | 135.18 | A |
| ATOM | 1836 | CG1 | VAL | A | 230 | 77.464 | 6.547 | 14.490 | 1.00 | 134.79 | A |
| ATOM | 1837 | CG2 | VAL | A | 230 | 79.814 | 6.764 | 15.285 | 1.00 | 135.77 | A |
| ATOM | 1838 | C | VAL | A | 230 | 77.740 | 3.845 | 13.913 | 1.00 | 135.51 | A |
| ATOM | 1839 | O | VAL | A | 230 | 77.627 | 3.714 | 12.699 | 1.00 | 135.69 | A |
| ATOM | 1840 | N | LYS | A | 231 | 76.783 | 3.495 | 14.774 | 1.00 | 136.36 | A |
| ATOM | 1841 | CA | LYS | A | 231 | 75.528 | 2.868 | 14.348 | 1.00 | 136.93 | A |
| ATOM | 1842 | CB | LYS | A | 231 | 75.414 | 1.456 | 14.944 | 1.00 | 135.70 | A |
| ATOM | 1843 | CG | LYS | A | 231 | 76.509 | 0.502 | 14.497 | 1.00 | 133.84 | A |
| ATOM | 1844 | CD | LYS | A | 231 | 76.330 | -0.877 | 15.097 | 1.00 | 132.66 | A |
| ATOM | 1845 | CE | LYS | A | 231 | 77.413 | -1.819 | 14.604 | 1.00 | 131.92 | A |
| ATOM | 1846 | NZ | LYS | A | 231 | 77.248 | -3.203 | 15.117 | 1.00 | 131.35 | A |
| ATOM | 1847 | C | LYS | A | 231 | 74.286 | 3.713 | 14.706 | 1.00 | 137.88 | A |
| ATOM | 1848 | O | LYS | A | 231 | 74.311 | 4.945 | 14.596 | 1.00 | 137.65 | A |
| ATOM | 1849 | N | GLU | A | 232 | 73.207 | 3.045 | 15.123 | 1.00 | 138.97 | A |
| ATOM | 1850 | CA | GLU | A | 232 | 71.949 | 3.709 | 15.490 | 1.00 | 139.73 | A |
| ATOM | 1851 | CB | GLU | A | 232 | 70.848 | 3.384 | 14.458 | 1.00 | 139.01 | A |
| ATOM | 1852 | CG | GLU | A | 232 | 70.859 | 1.954 | 13.874 | 1.00 | 137.25 | A |
| ATOM | 1853 | CD | GLU | A | 232 | 70.426 | 0.872 | 14.857 | 1.00 | 136.17 | A |
| ATOM | 1854 | OE1 | GLU | A | 232 | 69.454 | 1.090 | 15.613 | 1.00 | 135.56 | A |
| ATOM | 1855 | OE2 | GLU | A | 232 | 71.051 | -0.210 | 14.854 | 1.00 | 135.04 | A |
| ATOM | 1856 | C | GLU | A | 232 | 71.454 | 3.410 | 16.915 | 1.00 | 140.55 | A |
| ATOM | 1857 | O | GLU | A | 232 | 70.247 | 3.285 | 17.158 | 1.00 | 140.57 | A |
| ATOM | 1858 | N | ALA | A | 233 | 72.392 | 3.344 | 17.859 | 1.00 | 141.22 | A |
| ATOM | 1859 | CA | ALA | A | 233 | 72.072 | 3.058 | 19.255 | 1.00 | 142.00 | A |
| ATOM | 1860 | CB | ALA | A | 233 | 73.253 | 2.354 | 19.924 | 1.00 | 141.46 | A |
| ATOM | 1861 | C | ALA | A | 233 | 71.660 | 4.298 | 20.061 | 1.00 | 142.60 | A |
| ATOM | 1862 | O | ALA | A | 233 | 71.626 | 4.261 | 21.294 | 1.00 | 142.89 | A |
| ATOM | 1863 | N | ALA | A | 234 | 71.328 | 5.384 | 19.364 | 1.00 | 142.94 | A |
| ATOM | 1864 | CA | ALA | A | 234 | 70.922 | 6.626 | 20.021 | 1.00 | 143.19 | A |
| ATOM | 1865 | CB | ALA | A | 234 | 70.953 | 7.787 | 19.027 | 1.00 | 142.45 | A |
| ATOM | 1866 | C | ALA | A | 234 | 69.546 | 6.526 | 20.691 | 1.00 | 143.46 | A |
| ATOM | 1867 | O | ALA | A | 234 | 68.571 | 6.065 | 20.087 | 1.00 | 143.62 | A |
| ATOM | 1868 | N | ALA | A | 235 | 69.492 | 6.945 | 21.954 | 1.00 | 143.39 | A |
| ATOM | 1869 | CA | ALA | A | 235 | 68.265 | 6.932 | 22.749 | 1.00 | 142.70 | A |
| ATOM | 1870 | CB | ALA | A | 235 | 68.091 | 5.572 | 23.432 | 1.00 | 142.30 | A |
| ATOM | 1871 | C | ALA | A | 235 | 68.353 | 8.048 | 23.792 | 1.00 | 142.15 | A |
| ATOM | 1872 | O | ALA | A | 235 | 68.669 | 7.798 | 24.957 | 1.00 | 141.88 | A |
| ATOM | 1873 | N | GLN | A | 236 | 68.082 | 9.280 | 23.359 | 1.00 | 141.48 | A |
| ATOM | 1874 | CA | GLN | A | 236 | 68.145 | 10.451 | 24.235 | 1.00 | 140.61 | A |
| ATOM | 1875 | CB | GLN | A | 236 | 69.609 | 10.876 | 24.410 | 1.00 | 139.84 | A |
| ATOM | 1876 | CG | GLN | A | 236 | 69.891 | 11.791 | 25.597 | 1.00 | 138.75 | A |
| ATOM | 1877 | CD | GLN | A | 236 | 70.273 | 11.030 | 26.854 | 1.00 | 137.79 | A |
| ATOM | 1878 | OE1 | GLN | A | 236 | 70.888 | 9.964 | 26.790 | 1.00 | 137.24 | A |
| ATOM | 1879 | NE2 | GLN | A | 236 | 69.929 | 11.588 | 28.008 | 1.00 | 137.25 | A |
| ATOM | 1880 | C | GLN | A | 236 | 67.334 | 11.614 | 23.643 | 1.00 | 140.45 | A |
| ATOM | 1881 | O | GLN | A | 236 | 67.048 | 11.632 | 22.441 | 1.00 | 140.64 | A |
| ATOM | 1882 | N | GLN | A | 237 | 66.960 | 12.574 | 24.490 | 1.00 | 139.98 | A |
| ATOM | 1883 | CA | GLN | A | 237 | 66.191 | 13.744 | 24.056 | 1.00 | 139.46 | A |
| ATOM | 1884 | CB | GLN | A | 237 | 65.053 | 14.046 | 25.054 | 1.00 | 138.16 | A |
| ATOM | 1885 | CG | GLN | A | 237 | 64.406 | 15.447 | 24.949 | 1.00 | 135.77 | A |
| ATOM | 1886 | CD | GLN | A | 237 | 63.721 | 15.741 | 23.613 | 1.00 | 133.96 | A |
| ATOM | 1887 | OE1 | GLN | A | 237 | 63.240 | 16.853 | 23.391 | 1.00 | 131.78 | A |
| ATOM | 1888 | NE2 | GLN | A | 237 | 63.676 | 14.751 | 22.724 | 1.00 | 133.56 | A |
| ATOM | 1889 | C | GLN | A | 237 | 67.044 | 14.997 | 23.787 | 1.00 | 139.78 | A |
| ATOM | 1890 | O | GLN | A | 237 | 66.836 | 15.670 | 22.772 | 1.00 | 139.75 | A |
| ATOM | 1891 | N | GLN | A | 238 | 67.998 | 15.306 | 24.675 | 1.00 | 139.89 | A |
| ATOM | 1892 | CA | GLN | A | 238 | 68.860 | 16.488 | 24.498 | 1.00 | 139.48 | A |
| ATOM | 1893 | CB | GLN | A | 238 | 68.931 | 17.346 | 25.783 | 1.00 | 139.76 | A |
| ATOM | 1894 | CG | GLN | A | 238 | 69.548 | 16.711 | 27.028 | 1.00 | 139.50 | A |
| ATOM | 1895 | CD | GLN | A | 238 | 69.765 | 17.732 | 28.141 | 1.00 | 139.53 | A |
| ATOM | 1896 | OE1 | GLN | A | 238 | 68.821 | 18.138 | 28.822 | 1.00 | 139.44 | A |
| ATOM | 1897 | NE2 | GLN | A | 238 | 71.011 | 18.166 | 28.314 | 1.00 | 139.64 | A |
| ATOM | 1898 | C | GLN | A | 238 | 70.262 | 16.261 | 23.897 | 1.00 | 138.86 | A |
| ATOM | 1899 | O | GLN | A | 238 | 71.222 | 15.925 | 24.604 | 1.00 | 139.00 | A |
| ATOM | 1900 | N | GLU | A | 239 | 70.364 | 16.482 | 22.583 | 1.00 | 137.32 | A |
| ATOM | 1901 | CA | GLU | A | 239 | 71.607 | 16.312 | 21.822 | 1.00 | 135.17 | A |
| ATOM | 1902 | CB | GLU | A | 239 | 71.693 | 14.876 | 21.271 | 1.00 | 135.82 | A |
| ATOM | 1903 | CG | GLU | A | 239 | 71.376 | 13.742 | 22.253 | 1.00 | 136.14 | A |
| ATOM | 1904 | CD | GLU | A | 239 | 70.791 | 12.513 | 21.561 | 1.00 | 136.29 | A |
| ATOM | 1905 | OE1 | GLU | A | 239 | 69.549 | 12.374 | 21.535 | 1.00 | 135.52 | A |
| ATOM | 1906 | OE2 | GLU | A | 239 | 71.567 | 11.688 | 21.037 | 1.00 | 136.72 | A |
| ATOM | 1907 | C | GLU | A | 239 | 71.593 | 17.282 | 20.627 | 1.00 | 133.71 | A |
| ATOM | 1908 | O | GLU | A | 239 | 71.488 | 16.844 | 19.481 | 1.00 | 133.71 | A |
| ATOM | 1909 | N | SER | A | 240 | 71.704 | 18.586 | 20.875 | 1.00 | 131.85 | A |
| ATOM | 1910 | CA | SER | A | 240 | 71.673 | 19.554 | 19.775 | 1.00 | 130.10 | A |
| ATOM | 1911 | CB | SER | A | 240 | 70.499 | 20.526 | 19.943 | 1.00 | 129.95 | A |
| ATOM | 1912 | OG | SER | A | 240 | 70.629 | 21.325 | 21.105 | 1.00 | 129.22 | A |
| ATOM | 1913 | C | SER | A | 240 | 72.968 | 20.328 | 19.540 | 1.00 | 129.21 | A |
| ATOM | 1914 | O | SER | A | 240 | 73.218 | 21.346 | 20.188 | 1.00 | 129.33 | A |
| ATOM | 1915 | N | ALA | A | 241 | 73.767 | 19.845 | 18.588 | 1.00 | 128.28 | A |
| ATOM | 1916 | CA | ALA | A | 241 | 75.053 | 20.443 | 18.208 | 1.00 | 127.54 | A |
| ATOM | 1917 | CB | ALA | A | 241 | 74.832 | 21.578 | 17.202 | 1.00 | 127.46 | A |
| ATOM | 1918 | C | ALA | A | 241 | 75.922 | 20.919 | 19.384 | 1.00 | 127.07 | A |
| ATOM | 1919 | O | ALA | A | 241 | 76.643 | 21.915 | 19.269 | 1.00 | 126.82 | A |
| ATOM | 1920 | N | THR | A | 242 | 75.866 | 20.183 | 20.498 | 1.00 | 126.55 | A |
| ATOM | 1921 | CA | THR | A | 242 | 76.629 | 20.511 | 21.710 | 1.00 | 125.18 | A |
| ATOM | 1922 | CB | THR | A | 242 | 75.724 | 20.551 | 22.988 | 1.00 | 125.22 | A |
| ATOM | 1923 | OG1 | THR | A | 242 | 74.930 | 19.359 | 23.072 | 1.00 | 125.01 | A |
| ATOM | 1924 | CG2 | THR | A | 242 | 74.821 | 21.774 | 22.979 | 1.00 | 125.03 | A |
| ATOM | 1925 | C | THR | A | 242 | 77.876 | 19.655 | 22.020 | 1.00 | 124.04 | A |
| ATOM | 1926 | O | THR | A | 242 | 78.994 | 20.179 | 22.002 | 1.00 | 124.73 | A |
| ATOM | 1927 | N | THR | A | 243 | 77.693 | 18.355 | 22.289 | 1.00 | 121.68 | A |
| ATOM | 1928 | CA | THR | A | 243 | 78.812 | 17.462 | 22.646 | 1.00 | 118.32 | A |
| ATOM | 1929 | CB | THR | A | 243 | 78.538 | 16.751 | 24.003 | 1.00 | 117.71 | A |
| ATOM | 1930 | OG1 | THR | A | 243 | 77.576 | 17.500 | 24.755 | 1.00 | 117.57 | A |
| ATOM | 1931 | CG2 | THR | A | 243 | 79.816 | 16.655 | 24.824 | 1.00 | 117.04 | A |
| ATOM | 1932 | C | THR | A | 243 | 79.272 | 16.408 | 21.610 | 1.00 | 116.36 | A |
| ATOM | 1933 | O | THR | A | 243 | 79.520 | 16.726 | 20.442 | 1.00 | 115.64 | A |
| ATOM | 1934 | N | GLN | A | 244 | 79.426 | 15.164 | 22.072 | 1.00 | 114.26 | A |
| ATOM | 1935 | CA | GLN | A | 244 | 79.882 | 14.031 | 21.250 | 1.00 | 111.63 | A |
| ATOM | 1936 | CB | GLN | A | 244 | 80.613 | 13.006 | 221.139 | 1.00 | 111.67 | A |
| ATOM | 1937 | CG | GLN | A | 244 | 81.260 | 13.562 | 23.421 | 1.00 | 110.44 | A |
| ATOM | 1938 | CD | GLN | A | 244 | 82.484 | 14.422 | 23.154 | 1.00 | 110.49 | A |
| ATOM | 1939 | OE1 | GLN | A | 244 | 83.530 | 13.924 | 22.739 | 1.00 | 109.65 | A |
| ATOM | 1940 | NE2 | GLN | A | 244 | 82.361 | 15.720 | 23.405 | 1.00 | 110.27 | A |
| ATOM | 1941 | C | GLN | A | 244 | 78.730 | 13.314 | 20.522 | 1.00 | 109.26 | A |
| ATOM | 1942 | O | GLN | A | 244 | 78.843 | 12.954 | 19.342 | 1.00 | 107.40 | A |
| ATOM | 1943 | N | LYS | A | 245 | 27.649 | 13.076 | 21.269 | 1.00 | 106.78 | A |
| ATOM | 1944 | CA | LYS | A | 245 | 76.446 | 12.405 | 20.779 | 1.00 | 103.39 | A |
| ATOM | 1945 | CB | LYS | A | 245 | 75.718 | 11.722 | 21.949 | 1.00 | 104.18 | A |
| ATOM | 1946 | CG | LYS | A | 245 | 74.728 | 10.622 | 21.544 | 1.00 | 105.57 | A |
| ATOM | 1947 | CD | LYS | A | 245 | 74.004 | 10.021 | 22.754 | 1.00 | 105.85 | A |
| ATOM | 1948 | CE | LYS | A | 245 | 73.052 | 8.892 | 22.350 | 1.00 | 105.71 | A |
| ATOM | 1949 | MZ | LYS | A | 245 | 72.298 | 8.331 | 23.514 | 1.00 | 105.03 | A |
| ATOM | 1950 | C | LYS | A | 245 | 75.514 | 13.403 | 20.084 | 1.00 | 100.53 | A |
| ATOM | 1951 | O | LYS | A | 245 | 74.309 | 13.176 | 19.973 | 1.00 | 99.89 | A |
| ATOM | 1952 | N | ALA | A | 246 | 76.078 | 14.522 | 19.646 | 1.00 | 97.35 | A |
| ATOM | 1953 | CA | ALA | A | 246 | 75.314 | 15.546 | 18.948 | 1.00 | 94.04 | A |
| ATOM | 1954 | CB | ALA | A | 246 | 75.971 | 16.899 | 19.116 | 1.00 | 94.87 | A |
| ATOM | 1955 | C | ALA | A | 246 | 75.254 | 15.169 | 17.477 | 1.00 | 91.59 | A |
| ATOM | 1956 | O | ALA | A | 246 | 74.184 | 15.156 | 16.878 | 1.00 | 91.12 | A |
| ATOM | 1957 | N | GLU | A | 247 | 76.413 | 14.862 | 16.902 | 1.00 | 89.19 | A |
| ATOM | 1958 | CA | GLU | A | 247 | 76.494 | 14.465 | 15.502 | 1.00 | 87.21 | A |
| ATOM | 1959 | CB | GLU | A | 247 | 77.938 | 14.559 | 14.993 | 1.00 | 86.46 | A |
| ATOM | 1960 | CG | GLU | A | 247 | 78.430 | 15.983 | 14.732 | 1.00 | 86.54 | A |
| ATOM | 1961 | CD | GLU | A | 247 | 77.632 | 16.703 | 13.655 | 1.00 | 86.92 | A |
| ATOM | 1962 | OE1 | GLU | A | 247 | 77.454 | 16.121 | 12.564 | 1.00 | 86.55 | A |
| ATOM | 1963 | OE2 | GLU | A | 247 | 77.187 | 17.851 | 13.896 | 1.00 | 87.90 | A |
| ATOM | 1964 | C | GLU | A | 247 | 75.946 | 13.048 | 15.309 | 1.00 | 86.07 | A |
| ATOM | 1965 | O | GLU | A | 247 | 75.816 | 12.567 | 14.178 | 1.00 | 87.23 | A |
| ATOM | 1966 | N | LYS | A | 248 | 75.602 | 12.397 | 16.418 | 1.00 | 83.11 | A |
| ATOM | 1967 | CA | LYS | A | 248 | 75.056 | 11.044 | 16.391 | 1.00 | 80.27 | A |
| ATOM | 1968 | CB | LYS | A | 248 | 75.527 | 10.279 | 17.633 | 1.00 | 81.81 | A |
| ATOM | 1969 | CG | LYS | A | 248 | 75.405 | 8.757 | 17.561 | 1.00 | 83.89 | A |
| ATOM | 1970 | CD | LYS | A | 248 | 76.243 | 8.086 | 18.657 | 1.00 | 84.52 | A |
| ATOM | 1971 | CE | LYS | A | 248 | 77.736 | 8.390 | 18.481 | 1.00 | 85.16 | A |
| ATOM | 1972 | NZ | LYS | A | 248 | 78.575 | 7.895 | 19.609 | 1.00 | 84.44 | A |
| ATOM | 1973 | C | LYS | A | 248 | 73.521 | 11.072 | 16.297 | 1.00 | 77.87 | A |
| ATOM | 1974 | O | LYS | A | 248 | 72.894 | 10.048 | 16.035 | 1.00 | 77.74 | A |
| ATOM | 1975 | N | GLU | A | 249 | 72.931 | 12.248 | 16.527 | 1.00 | 75.36 | A |
| ATOM | 1976 | CA | GLU | A | 249 | 71.477 | 12.454 | 16.443 | 1.00 | 71.41 | A |
| ATOM | 1977 | CB | GLU | A | 249 | 71.038 | 13.584 | 17.383 | 1.00 | 72.05 | A |
| ATOM | 1978 | CG | GLU | A | 249 | 69.552 | 13.928 | 17.326 | 1.00 | 73.55 | A |
| ATOM | 1979 | CD | GLU | A | 249 | 69.259 | 15.353 | 17.787 | 1.00 | 74.44 | A |
| ATOM | 1980 | OE1 | GLU | A | 249 | 69.826 | 16.302 | 17.201 | 1.00 | 74.49 | A |
| ATOM | 1981 | OE2 | GLU | A | 249 | 68.451 | 15.531 | 18.725 | 1.00 | 74.97 | A |
| ATOM | 1982 | C | GLU | A | 249 | 71.135 | 12.823 | 14.996 | 1.00 | 68.27 | A |
| ATOM | 1983 | O | GLU | A | 249 | 70.009 | 12.611 | 14.538 | 1.00 | 67.49 | A |
| ATOM | 1984 | N | VAL | A | 250 | 72.117 | 13.412 | 14.308 | 1.00 | 64.63 | A |
| ATOM | 1985 | CA | VAL | A | 250 | 71.999 | 13.804 | 12.907 | 1.00 | 61.06 | A |
| ATOM | 1986 | CB | VAL | A | 250 | 73.066 | 14.856 | 12.498 | 1.00 | 59.72 | A |
| ATOM | 1987 | CG1 | VAL | A | 250 | 73.089 | 15.033 | 10.989 | 1.00 | 57.65 | A |
| ATOM | 1988 | CG2 | VAL | A | 250 | 72.775 | 16.191 | 13.158 | 1.00 | 59.55 | A |
| ATOM | 1989 | C | VAL | A | 250 | 72.218 | 12.554 | 12.075 | 1.00 | 59.98 | A |
| ATOM | 1990 | O | VAL | A | 250 | 71.480 | 12.298 | 11.131 | 1.00 | 59.52 | A |
| ATOM | 1991 | N | THR | A | 251 | 73.235 | 11.775 | 12.437 | 1.00 | 59.13 | A |
| ATOM | 1992 | CA | THR | A | 251 | 73.539 | 10.544 | 11.720 | 1.00 | 58.16 | A |
| ATOM | 1993 | CB | THR | A | 251 | 74.818 | 9.856 | 12.250 | 1.00 | 58.64 | A |
| ATOM | 1994 | OG1 | THR | A | 251 | 75.941 | 10.741 | 12.110 | 1.00 | 56.90 | A |
| ATOM | 1995 | CG2 | THR | A | 251 | 75.087 | 8.572 | 11.470 | 1.00 | 57.75 | A |
| ATOM | 1996 | C | THR | A | 251 | 72.359 | 9.582 | 11.797 | 1.00 | 57.48 | A |
| ATOM | 1997 | O | THR | A | 251 | 71.983 | 9.000 | 10.790 | 1.00 | 57.56 | A |
| ATOM | 1998 | N | ARG | A | 252 | 71.769 | 9.428 | 12.981 | 1.00 | 57.31 | A |
| ATOM | 1999 | CA | ARG | A | 252 | 70.612 | 8.548 | 13.135 | 1.00 | 57.64 | A |
| ATOM | 2000 | CB | ARG | A | 252 | 70.183 | 8.390 | 14.612 | 1.00 | 62.22 | A |
| ATOM | 2001 | CG | ARG | A | 252 | 68.918 | 7.499 | 14.821 | 1.00 | 67.65 | A |
| ATOM | 2002 | CD | ARG | A | 252 | 68.565 | 7.178 | 16.311 | 1.00 | 73.56 | A |
| ATOM | 2003 | NE | ARG | A | 252 | 67.751 | 8.192 | 17.014 | 1.00 | 77.86 | A |
| ATOM | 2004 | CZ | ARG | A | 252 | 66.804 | 7.922 | 17.924 | 1.00 | 78.42 | A |
| ATOM | 2005 | NH1 | ARC | A | 252 | 66.523 | 6.664 | 18.263 | 1.00 | 77.83 | A |
| ATOM | 2006 | NH2 | ARG | A | 252 | 66.132 | 8.913 | 18.505 | 1.00 | 77.51 | A |
| ATOM | 2007 | C | ARG | A | 252 | 69.472 | 9.145 | 12.328 | 1.00 | 55.25 | A |
| ATOM | 2008 | O | ARG | A | 252 | 68.638 | 8.422 | 11.808 | 1.00 | 55.54 | A |
| ATOM | 2009 | N | MET | A | 253 | 69.451 | 10.467 | 12.203 | 1.00 | 53.09 | A |
| ATOM | 2010 | CA | MET | A | 253 | 68.397 | 11.125 | 11.444 | 1.00 | 52.02 | A |
| ATOM | 2011 | CB | MET | A | 253 | 68.372 | 12.625 | 11.731 | 1.00 | 52.23 | A |
| ATOM | 2012 | CG | MET | A | 253 | 67.287 | 13.365 | 10.956 | 1.00 | 53.70 | A |
| ATOM | 2013 | SD | MET | A | 253 | 67.037 | 15.084 | 11.449 | 1.00 | 53.84 | A |
| ATOM | 2014 | CE | MET | A | 253 | 67.871 | 15.971 | 10.145 | 1.00 | 53.74 | A |
| ATOM | 2015 | C | MET | A | 253 | 68.551 | 10.888 | 9.946 | 1.00 | 51.09 | A |
| ATOM | 2016 | O | MET | A | 253 | 67.591 | 10.539 | 9.260 | 1.00 | 50.86 | A |
| ATOM | 2017 | N | VAL | A | 254 | 69.767 | 11.081 | 9.448 | 1.00 | 49.96 | A |
| ATOM | 2018 | CA | VAL | A | 254 | 70.064 | 10.890 | 8.036 | 1.00 | 48.21 | A |
| ATOM | 2019 | CB | VAL | A | 254 | 71.521 | 11.309 | 7.727 | 1.00 | 47.73 | A |
| ATOM | 2020 | CG1 | VAL | A | 254 | 71.863 | 11.050 | 6.260 | 1.00 | 48.55 | A |
| ATOM | 2021 | CG2 | VAL | A | 254 | 71.706 | 12.787 | 8.057 | 1.00 | 46.97 | A |
| ATOM | 2022 | C | VAL | A | 254 | 69.798 | 9.445 | 7.603 | 1.00 | 47.41 | A |
| ATOM | 2023 | O | VAL | A | 254 | 69.466 | 9.189 | 6.444 | 1.00 | 46.63 | A |
| ATOM | 2024 | N | ILE | A | 255 | 69.891 | 8.519 | 8.557 | 1.00 | 46.77 | A |
| ATOM | 2025 | CA | ILE | A | 255 | 69.655 | 7.092 | 8.304 | 1.00 | 46.06 | A |
| ATOM | 2026 | CB | ILE | A | 255 | 70.402 | 6.198 | 9.365 | 1.00 | 46.75 | A |
| ATOM | 2027 | CG2 | ILE | A | 255 | 70.213 | 4.721 | 9.066 | 1.00 | 46.22 | A |
| ATOM | 2028 | CG1 | ILE | A | 255 | 71.905 | 6.541 | 9.422 | 1.00 | 47.02 | A |
| ATOM | 2029 | CD1 | ILE | A | 255 | 72.638 | 6.603 | 8.072 | 1.00 | 48.93 | A |
| ATOM | 2030 | C | ILE | A | 255 | 68.144 | 6.758 | 8.262 | 1.00 | 44.05 | A |
| ATOM | 2031 | O | ILE | A | 255 | 67.737 | 5.717 | 7.745 | 1.00 | 42.96 | A |
| ATOM | 2032 | N | ILE | A | 256 | 67.323 | 7.664 | 8.787 | 1.00 | 43.37 | A |
| ATOM | 2033 | CA | ILE | A | 256 | 65.868 | 7.496 | 8.800 | 1.00 | 43.41 | A |
| ATOM | 2034 | CB | ILE | A | 256 | 65.209 | 8.243 | 10.000 | 1.00 | 44.47 | A |
| ATOM | 2035 | CG2 | ILE | A | 256 | 63.694 | 8.360 | 9.801 | 1.00 | 44.70 | A |
| ATOM | 2036 | CG1 | ILE | A | 256 | 65.530 | 7.534 | 11.323 | 1.00 | 43.60 | A |
| ATOM | 2037 | CD1 | ILE | A | 256 | 64.911 | 6.159 | 11.469 | 1.00 | 41.34 | A |
| ATOM | 2038 | C | ILE | A | 256 | 65.285 | 8.036 | 7.499 | 1.00 | 42.61 | A |
| ATOM | 2039 | O | ILE | A | 256 | 64.437 | 7.404 | 6.877 | 1.00 | 42.07 | A |
| ATOM | 2040 | N | MET | A | 257 | 65.754 | 9.211 | 7.096 | 1.00 | 42.41 | A |
| ATOM | 2041 | CA | MET | A | 257 | 65.300 | 9.842 | 5.866 | 1.00 | 43.10 | A |
| ATOM | 2042 | CB | MET | A | 257 | 66.080 | 11.131 | 5.622 | 1.00 | 43.22 | A |
| ATOM | 2043 | CG | MET | A | 257 | 66.141 | 12.077 | 6.798 | 1.00 | 42.93 | A |
| ATOM | 2044 | SD | MET | A | 257 | 67.177 | 13.503 | 6.422 | 1.00 | 41.97 | A |
| ATOM | 2045 | CE | MET | A | 257 | 66.006 | 14.822 | 6.425 | 1.00 | 40.96 | A |
| ATOM | 2046 | C | MET | A | 257 | 65.520 | 8.896 | 4.679 | 1.00 | 44.35 | A |
| ATOM | 2047 | O | MET | A | 257 | 64.909 | 9.056 | 3.620 | 1.00 | 44.44 | A |
| ATOM | 2048 | N | VAL | A | 258 | 66.431 | 7.941 | 4.850 | 1.00 | 44.88 | A |
| ATOM | 2049 | CA | VAL | A | 258 | 66.723 | 6.967 | 3.811 | 1.00 | 45.11 | A |
| ATOM | 2050 | CB | VAL | A | 258 | 68.081 | 6.265 | 4.055 | 1.00 | 46.14 | A |
| ATOM | 2051 | CG1 | VAL | A | 258 | 68.295 | 5.141 | 3.046 | 1.00 | 45.87 | A |
| ATOM | 2052 | CG2 | VAL | A | 258 | 69.215 | 7.283 | 3.936 | 1.00 | 46.82 | A |
| ATOM | 2053 | C | VAL | A | 258 | 65.591 | 5.949 | 3.787 | 1.00 | 44.97 | A |
| ATOM | 2054 | O | VAL | A | 258 | 65.033 | 5.655 | 2.726 | 1.00 | 46.08 | A |
| ATOM | 2055 | N | ILE | A | 259 | 65.226 | 5.441 | 4.960 | 1.00 | 42.72 | A |
| ATOM | 2056 | CA | ILE | A | 259 | 64.144 | 4.473 | 5.043 | 1.00 | 42.09 | A |
| ATOM | 2057 | CB | ILE | A | 259 | 63.944 | 3.976 | 6.470 | 1.00 | 43.29 | A |
| ATOM | 2058 | CG2 | ILE | A | 259 | 62.825 | 2.952 | 6.513 | 1.00 | 42.70 | A |
| ATOM | 2059 | CG1 | ILE | A | 259 | 65.244 | 3.367 | 6.998 | 1.00 | 45.47 | A |
| ATOM | 2060 | CD1 | ILE | A | 259 | 65.214 | 3.075 | 8.485 | 1.00 | 45.00 | A |
| ATOM | 2061 | C | ILE | A | 259 | 62.863 | 5.142 | 4.583 | 1.00 | 41.75 | A |
| ATOM | 2062 | O | ILE | A | 259 | 62.176 | 4.634 | 3.700 | 1.00 | 43.89 | A |
| ATOM | 2063 | N | ALA | A | 260 | 62.570 | 6.308 | 5.153 | 1.00 | 39.70 | A |
| ATOM | 2064 | CA | ALA | A | 260 | 61.374 | 7.057 | 4.799 | 1.00 | 37.88 | A |
| ATOM | 2065 | CB | ALA | A | 260 | 61.382 | 8.400 | 5.477 | 1.00 | 36.09 | A |
| ATOM | 2066 | C | ALA | A | 260 | 61.289 | 7.223 | 3.288 | 1.00 | 37.55 | A |
| ATOM | 2067 | O | ALA | A | 260 | 60.200 | 7.193 | 2.713 | 1.00 | 40.62 | A |
| ATOM | 2068 | N | PHE | A | 261 | 62.445 | 7.345 | 2.646 | 1.00 | 34.91 | A |
| ATOM | 2069 | CA | PHE | A | 261 | 62.511 | 7.494 | 1.198 | 1.00 | 35.03 | A |
| ATOM | 2070 | CB | PHE | A | 261 | 63.944 | 7.827 | 0.765 | 1.00 | 33.51 | A |
| ATOM | 2071 | CG | PHE | A | 261 | 64.139 | 7.878 | -0.730 | 1.00 | 32.58 | A |
| ATOM | 2072 | CD1 | PHE | A | 261 | 64.042 | 9.084 | -1.414 | 1.00 | 30.86 | A |
| ATOM | 2073 | CD2 | PHE | A | 261 | 64.421 | 6.717 | -1.451 | 1.00 | 31.18 | A |
| ATOM | 2074 | CE1 | PHE | A | 261 | 64.221 | 9.131 | -2.787 | 1.00 | 31.54 | A |
| ATOM | 2075 | CE2 | PHE | A | 261 | 64.600 | 6.758 | -2.823 | 1.00 | 29.70 | A |
| ATOM | 2076 | CZ | PHE | A | 261 | 64.500 | 7.964 | -3.494 | 1.00 | 30.18 | A |
| ATOM | 2077 | C | PHE | A | 261 | 62.054 | 6.214 | 0.512 | 1.00 | 36.10 | A |
| ATOM | 2078 | O | PHE | A | 261 | 61.200 | 6.239 | -0.376 | 1.00 | 35.59 | A |
| ATOM | 2079 | N | LEU | A | 262 | 62.656 | 5.102 | 0.925 | 1.00 | 37.17 | A |
| ATOM | 2080 | CA | LEU | A | 262 | 62.350 | 3.787 | 0.376 | 1.00 | 36.67 | A |
| ATOM | 2081 | CB | LEU | A | 262 | 63.166 | 2.714 | 1.093 | 1.00 | 34.55 | A |
| ATOM | 2082 | CG | LEU | A | 262 | 64.682 | 2.867 | 1.119 | 1.00 | 31.36 | A |
| ATOM | 2083 | CD1 | LEU | A | 262 | 65.250 | 1.713 | 1.925 | 1.00 | 30.40 | A |
| ATOM | 2084 | CD2 | LEU | A | 262 | 65.255 | 2.907 | -0.286 | 1.00 | 27.84 | A |
| ATOM | 2085 | C | LEU | A | 262 | 60.866 | 3.448 | 0.476 | 1.00 | 37.31 | A |
| ATOM | 2086 | O | LEU | A | 262 | 60.228 | 3.177 | -0.544 | 1.00 | 38.41 | A |
| ATOM | 2087 | N | ILE | A | 263 | 60.322 | 3.470 | 1.695 | 1.00 | 36.21 | A |
| ATOM | 2088 | CA | ILE | A | 263 | 58.905 | 3.165 | 1.918 | 1.00 | 35.23 | A |
| ATOM | 2089 | CB | ILE | A | 263 | 58.456 | 3.528 | 3.363 | 1.00 | 33.58 | A |
| ATOM | 2090 | CG2 | ILE | A | 263 | 56.977 | 3.199 | 3.567 | 1.00 | 32.44 | A |
| ATOM | 2091 | CG1 | ILE | A | 263 | 59.304 | 2.796 | 4.399 | 1.00 | 31.09 | A |
| ATOM | 2092 | CD1 | ILE | A | 263 | 58.886 | 3.070 | 5.828 | 1.00 | 27.29 | A |
| ATOM | 2093 | C | ILE | A | 263 | 58.045 | 3.989 | 0.963 | 1.00 | 36.17 | A |
| ATOM | 2094 | O | ILE | A | 263 | 57.038 | 3.510 | 0.442 | 1.00 | 37.15 | A |
| ATOM | 2095 | N | CYS | A | 264 | 58.513 | 5.203 | 0.688 | 1.00 | 36.76 | A |
| ATOM | 2096 | CA | CYS | A | 264 | 57.817 | 6.164 | 0.158 | 1.00 | 36.18 | A |
| ATOM | 2097 | CB | CYS | A | 264 | 58.241 | 7.579 | 0.245 | 1.00 | 35.16 | A |
| ATOM | 2098 | SG | CYS | A | 264 | 57.040 | 8.834 | -0.115 | 1.00 | 33.33 | A |
| ATOM | 2099 | C | CYS | A | 264 | 57.988 | 5.990 | -1.657 | 1.00 | 35.31 | A |
| ATOM | 2100 | O | CYS | A | 264 | 57.005 | 5.932 | -2.393 | 1.00 | 36.82 | A |
| ATOM | 2101 | N | TRP | A | 265 | 59.235 | 5.918 | -2.106 | 1.00 | 33.91 | A |
| ATOM | 2102 | CA | TRP | A | 265 | 59.525 | 5.793 | -3.526 | 1.00 | 33.08 | A |
| ATOM | 2103 | CB | TRP | A | 265 | 60.704 | 6.681 | -3.888 | 1.00 | 32.58 | A |
| ATOM | 2104 | CG | TRP | A | 265 | 60.341 | 8.109 | -3.855 | 1.00 | 33.77 | A |
| ATOM | 2105 | CD2 | TRP | A | 265 | 59.481 | 8.792 | -4.780 | 1.00 | 35.96 | A |
| ATOM | 2106 | CE2 | TRP | A | 265 | 59.451 | 10.152 | -4.394 | 1.00 | 34.73 | A |
| ATOM | 2107 | CE3 | TRP | A | 265 | 58.735 | 8.387 | -5.904 | 1.00 | 36.21 | A |
| ATOM | 2108 | CD1 | TRP | A | 265 | 60.775 | 9.046 | -2.967 | 1.00 | 32.57 | A |
| ATOM | 2109 | NB1 | TRP | A | 265 | 60.249 | 10.278 | -3.285 | 1.00 | 34.18 | A |
| ATOM | 2110 | CZ2 | TRP | A | 265 | 58.707 | 11.114 | -5.092 | 1.00 | 33.80 | A |
| ATOM | 2111 | CZ3 | TRP | A | 265 | 57.997 | 9.343 | -6.598 | 1.00 | 35.57 | A |
| ATOM | 2112 | CH2 | TRP | A | 265 | 57.990 | 10.693 | -6.187 | 1.00 | 35.16 | A |
| ATOM | 2113 | C | TRP | A | 265 | 59.723 | 4.404 | -4.112 | 1.00 | 32.53 | A |
| ATOM | 2114 | O | TRP | A | 265 | 59.693 | 4.243 | -5.332 | 1.00 | 33.01 | A |
| ATOM | 2115 | N | LEU | A | 266 | 59.929 | 3.405 | -3.260 | 1.00 | 31.86 | A |
| ATOM | 2116 | CA | LEU | A | 266 | 60.122 | 2.038 | -3.745 | 1.00 | 31.40 | A |
| ATOM | 2117 | CB | LEU | A | 266 | 60.707 | 1.127 | -2.658 | 1.00 | 32.38 | A |
| ATOM | 2118 | CG | LEU | A | 266 | 62.124 | 0.608 | -2.932 | 1.00 | 32.34 | A |
| ATOM | 2119 | CD1 | LEU | A | 266 | 63.060 | 1.777 | -3.253 | 1.00 | 32.05 | A |
| ATOM | 2120 | CD2 | LEU | A | 266 | 62.621 | -0.168 | 1.727 | 1.00 | 30.76 | A |
| ATOM | 2121 | C | LEU | A | 266 | 58.862 | 1.419 | -4.346 | 1.00 | 29.79 | A |
| ATOM | 2122 | O | LEU | A | 266 | 58.924 | 0.854 | -5.440 | 1.00 | 29.51 | A |
| ATOM | 2123 | N | PRO | A | 267 | 57.706 | 1.513 | -3.648 | 1.00 | 26.85 | A |
| ATOM | 2124 | CD | PRO | A | 267 | 57.422 | 2.107 | -2.333 | 1.00 | 24.34 | A |
| ATOM | 2125 | CA | PRO | A | 267 | 56.484 | 0.936 | -4.201 | 1.00 | 25.82 | A |
| ATOM | 2126 | CB | PRO | A | 267 | 55.423 | 1.432 | -3.234 | 1.00 | 23.65 | A |
| ATOM | 2127 | CG | PRO | A | 267 | 56.147 | 1.442 | -1.966 | 1.00 | 22.24 | A |
| ATOM | 2128 | C | PRO | A | 267 | 56.261 | 1.464 | -5.614 | 1.00 | 27.30 | A |
| ATOM | 2129 | O | PRO | A | 267 | 55.949 | 0.694 | -6.525 | 1.00 | 27.01 | A |
| ATOM | 2130 | N | TYR | A | 268 | 56.483 | 2.765 | -5.799 | 1.00 | 27.85 | A |
| ATOM | 2131 | CA | TYR | A | 268 | 56.326 | 3.384 | -7.114 | 1.00 | 28.65 | A |
| ATOM | 2132 | CB | TYR | A | 268 | 56.427 | 4.911 | -7.025 | 1.00 | 26.86 | A |
| ATOM | 2133 | CG | TYR | A | 268 | 56.251 | 5.612 | -8.357 | 1.00 | 23.42 | A |
| ATOM | 2134 | CD1 | TYR | A | 268 | 54.997 | 6.030 | -8.779 | 1.00 | 23.50 | A |
| ATOM | 2135 | CB1 | TYR | A | 268 | 54.822 | 6.666 | -10.005 | 1.00 | 23.96 | A |
| ATOM | 2136 | CD2 | TYR | A | 268 | 57.338 | 5.844 | -9.197 | 1.00 | 21.90 | A |
| ATOM | 2137 | CE2 | TYR | A | 268 | 57.180 | 6.471 | -10.424 | 1.00 | 21.42 | A |
| ATOM | 2138 | CZ | TYR | A | 268 | 55.916 | 6.886 | -10.823 | 1.00 | 23.32 | A |
| ATOM | 2139 | OH | TYR | A | 268 | 55.749 | 7.550 | -12.017 | 1.00 | 19.42 | A |
| ATOM | 2140 | C | TYR | A | 268 | 57.404 | 2.868 | -8.056 | 1.00 | 30.33 | A |
| ATOM | 2141 | O | TYR | A | 268 | 57.112 | 2.474 | -9.177 | 1.00 | 29.92 | A |
| ATOM | 2142 | N | ALA | A | 269 | 58.653 | 2.895 | -7.594 | 1.00 | 33.21 | A |
| ATOM | 2143 | CA | ALA | A | 269 | 59.790 | 2.439 | -8.384 | 1.00 | 33.85 | A |
| ATOM | 2144 | CB | ALA | A | 269 | 61.066 | 2.578 | -7.582 | 1.00 | 33.68 | A |
| ATOM | 2145 | C | ALA | A | 269 | 59.581 | 0.996 | -8.796 | 1.00 | 35.54 | A |
| ATOM | 2146 | O | ALA | A | 269 | 59.842 | 0.620 | -9.940 | 1.00 | 33.94 | A |
| ATOM | 2147 | N | GLY | A | 270 | 59.076 | 0.200 | -7.859 | 1.00 | 38.24 | A |
| ATOM | 2148 | CA | GLY | A | 270 | 58.813 | -1.202 | -8.123 | 1.00 | 42.31 | A |
| ATOM | 2149 | C | GLY | A | 270 | 57.745 | -1.403 | -9.185 | 1.00 | 45.09 | A |
| ATOM | 2150 | 0 | GLY | A | 270 | 58.004 | -2.006 | -10.226 | 1.00 | 46.85 | A |
| ATOM | 2151 | N | VAL | A | 271 | 56.557 | -0.852 | -8.950 | 1.00 | 46.68 | A |
| ATOM | 2152 | CA | VAL | A | 271 | 55.448 | -0.982 | -9.894 | 1.00 | 48.20 | A |
| ATOM | 2153 | CB | VAL | A | 271 | 54.110 | -0.606 | -9.213 | 1.00 | 47.47 | A |
| ATOM | 2154 | CG1 | VAL | A | 271 | 54.053 | 0.875 | -8.920 | 1.00 | 49.70 | A |
| ATOM | 2155 | CG2 | VAL | A | 271 | 52.954 | -1.034 | -10.055 | 1.00 | 46.58 | A |
| ATOM | 2156 | C | VAL | A | 271 | 55.638 | -0.213 | -11.219 | 1.00 | 48.96 | A |
| ATOM | 2157 | O | VAL | A | 271 | 54.820 | -0.338 | -12.127 | 1.00 | 49.07 | A |
| ATOM | 2158 | N | ALA | A | 272 | 56.713 | 0.575 | -11.319 | 1.00 | 50.43 | A |
| ATOM | 2159 | CA | ALA | A | 272 | 57.036 | 1.341 | -12.529 | 1.00 | 50.41 | A |
| ATOM | 2160 | CB | ALA | A | 272 | 57.600 | 2.700 | -12.176 | 1.00 | 48.29 | A |
| ATOM | 2161 | C | ALA | A | 272 | 58.042 | 0.553 | -13.360 | 1.00 | 51.66 | A |
| ATOM | 2162 | O | ALA | A | 272 | 58.082 | 0.680 | -14.582 | 1.00 | 52.48 | A |
| ATOM | 2163 | N | PHE | A | 273 | 58.882 | -0.234 | -12.692 | 1.00 | 53.08 | A |
| ATOM | 2164 | CA | PHE | A | 273 | 59.853 | -1.065 | -13.400 | 1.00 | 55.36 | A |
| ATOM | 2165 | CB | PHE | A | 273 | 61.021 | -1.463 | -12.493 | 1.00 | 54.53 | A |
| ATOM | 2166 | CG | PHE | A | 273 | 62.130 | -2.183 | -13.216 | 1.00 | 53.59 | A |
| ATOM | 2167 | CD1 | PHE | A | 273 | 62.958 | -1.502 | -14.104 | 1.00 | 53.46 | A |
| ATOM | 2168 | CD2 | PHE | A | 273 | 62.349 | -3.541 | -13.009 | 1.00 | 53.41 | A |
| ATOM | 2169 | CE1 | PHE | A | 273 | 63.990 | -2.161 | -14.775 | 1.00 | 52.07 | A |
| ATOM | 2170 | CE2 | PHE | A | 273 | 63.377 | -4.206 | -13.674 | 1.00 | 52.45 | A |
| ATOM | 2171 | CZ | PHE | A | 273 | 64.198 | -3.514 | -14.558 | 1.00 | 52.25 | A |
| ATOM | 2172 | C | PHE | A | 273 | 59.122 | -2.317 | -13.898 | 1.00 | 57.31 | A |
| ATOM | 2173 | O | PHE | A | 273 | 59.519 | -2.923 | -14.898 | 1.00 | 58.22 | A |
| ATOM | 2174 | N | TYR | A | 274 | 58.071 | -2.709 | -13.172 | 1.00 | 57.98 | A |
| ATOM | 2175 | CA | TYR | A | 274 | 57.244 | -3.859 | -13.536 | 1.00 | 57.73 | A |
| ATOM | 2176 | CB | TYR | A | 274 | 56.099 | -4.034 | -12.523 | 1.00 | 58.49 | A |
| ATOM | 2177 | CG | TYR | A | 274 | 55.041 | -5.037 | -12.951 | 1.00 | 60.77 | A |
| ATOM | 2178 | CD1 | TYR | A | 274 | 55.059 | -6.348 | -12.480 | 1.00 | 61.81 | A |
| ATOM | 2179 | CE1 | TYR | A | 274 | 54.120 | -7.288 | -12.923 | 1.00 | 63.12 | A |
| ATOM | 2180 | CD2 | TYR | A | 274 | 54.049 | -4.685 | -13.871 | 1.00 | 61.71 | A |
| ATOM | 2181 | CE2 | TYR | A | 274 | 53.110 | -5.611 | -14.319 | 1.00 | 62.50 | A |
| ATOM | 2182 | CZ | TYR | A | 274 | 53.151 | -6.910 | -13.846 | 1.00 | 63.26 | A |
| ATOM | 2183 | OH | TYR | A | 274 | 52.236 | -7.827 | -14.314 | 1.00 | 63.28 | A |
| ATOM | 2184 | C | TYR | A | 274 | 56.667 | -3.558 | -14.918 | 1.00 | 57.73 | A |
| ATOM | 2185 | O | TYR | A | 274 | 56.841 | -4.322 | -15.867 | 1.00 | 58.39 | A |
| ATOM | 2186 | N | ILE | A | 275 | 55.997 | -2.415 | -15.004 | 1.00 | 56.83 | A |
| ATOM | 2187 | CA | ILE | A | 275 | 55.375 | -1.930 | -16.223 | 1.00 | 56.05 | A |
| ATOM | 2188 | CB | ILE | A | 275 | 154.779 | -0.523 | -15.972 | 1.00 | 54.80 | A |
| ATOM | 2189 | CG2 | ILE | A | 275 | 54.358 | 0.131 | -17.271 | 1.00 | 53.84 | A |
| ATOM | 2190 | CG1 | ILE | A | 275 | 53.611 | -0.635 | -14.991 | 1.00 | 54.03 | A |
| ATOM | 2191 | CD1 | ILE | A | 275 | 53.116 | 0.684 | -14.454 | 1.00 | 55.23 | A |
| ATOM | 2192 | C | ILE | A | 275 | 56.361 | -1.900 | -17.395 | 1.00 | 57.21 | A |
| ATOM | 2193 | O | ILE | A | 275 | 56.117 | -2.524 | -18.429 | 1.00 | 56.89 | A |
| ATOM | 2194 | N | PHE | A | 276 | 57.497 | -1.230 | -17.206 | 1.00 | 58.42 | A |
| ATOM | 2195 | CA | PHE | A | 276 | 58.509 | -1.112 | -18.258 | 1.00 | 59.12 | A |
| ATOM | 2196 | CB | PHE | A | 276 | 59.788 | -0.455 | -17.724 | 1.00 | 59.21 | A |
| ATOM | 2197 | CG | PHE | A | 276 | 60.880 | -0.336 | -18.757 | 1.00 | 59.54 | A |
| ATOM | 2198 | CD1 | PHE | A | 276 | 60.662 | 0.368 | -19.942 | 1.00 | 58.88 | A |
| ATOM | 2199 | CD2 | PHE | A | 276 | 62.117 | -0.952 | -18.559 | 1.00 | 58.95 | A |
| ATOM | 2200 | CE1 | PHE | A | 276 | 61.656 | 0.454 | -20.911 | 1.00 | 58.84 | A |
| ATOM | 2201 | CE2 | PHE | A | 276 | 63.118 | -0.871 | -19.523 | 1.00 | 57.76 | A |
| ATOM | 2202 | CZ | PHE | A | 276 | 62.888 | -0.168 | -20.699 | 1.00 | 58.30 | A |
| ATOM | 2203 | C | PHE | A | 276 | 58.847 | -2.450 | -18.903 | 1.00 | 59.10 | A |
| ATOM | 2204 | O | PHE | A | 276 | 59.073 | -2.527 | -20.114 | 1.00 | 59.36 | A |
| ATOM | 2205 | N | THR | A | 277 | 58.900 | -3.497 | -18.087 | 1.00 | 59.31 | A |
| ATOM | 2206 | CA | THR | A | 277 | 59.203 | -4.823 | -18.599 | 1.00 | 59.55 | A |
| ATOM | 2207 | CB | THR | A | 277 | 59.822 | -5.741 | -17.509 | 1.00 | 59.00 | A |
| ATOM | 2208 | OG1 | THR | A | 277 | 58.936 | -5.844 | -16.390 | 1.00 | 58.43 | A |
| ATOM | 2209 | CG2 | THR | A | 277 | 61.150 | -5.174 | -17.041 | 1.00 | 57.71 | A |
| ATOM | 2210 | C | THR | A | 277 | 57.937 | -5.436 | -19.194 | 1.00 | 59.28 | A |
| ATOM | 2211 | O | THR | A | 277 | 57.881 | -5.718 | -20.397 | 1.00 | 61.80 | A |
| ATOM | 2212 | N | HIS | A | 278 | 56.900 | -5.565 | -18.373 | 1.00 | 56.62 | A |
| ATOM | 2213 | CA | HIS | A | 278 | 55.639 | -6.131 | -18.830 | 1.00 | 54.45 | A |
| ATOM | 2214 | CB | HIS | A | 278 | 54.870 | -6.747 | -17.655 | 1.00 | 54.79 | A |
| ATOM | 2215 | CG | HIS | A | 278 | 55.635 | -7.795 | -16.904 | 1.00 | 57.46 | A |
| ATOM | 2216 | CD2 | HIS | A | 278 | 55.226 | -8.721 | -16.002 | 1.00 | 58.18 | A |
| ATOM | 2217 | ND1 | HIS | A | 278 | 57.000 | -7.954 | -17.019 | 1.00 | 58.58 | A |
| ATOM | 2218 | CE1 | HIS | A | 278 | 57.398 | -8.929 | -16.220 | 1.00 | 58.89 | A |
| ATOM | 2219 | NE2 | HIS | A | 278 | 56.341 | -9.411 | -15.591 | 1.00 | 58.08 | A |
| ATOM | 2220 | C | HIS | A | 278 | 54.764 | -5.080 | -19.525 | 1.00 | 52.89 | A |
| ATOM | 2221 | O | HIS | A | 278 | 53.670 | -4.779 | -19.047 | 1.00 | 53.05 | A |
| ATOM | 2222 | N | GLN | A | 279 | 55.255 | -4.506 | -20.627 | 1.00 | 50.29 | A |
| ATOM | 2223 | CA | GLN | A | 279 | 54.497 | -3.505 | -21.392 | 1.00 | 48.60 | A |
| ATOM | 2224 | CB | GLN | A | 279 | 55.419 | -2.730 | -22.339 | 1.00 | 47.61 | A |
| ATOM | 2225 | CG | GLN | A | 279 | 56.485 | -1.911 | -21.656 | 1.00 | 47.39 | A |
| ATOM | 2226 | CD | GLN | A | 279 | 57.573 | -1.441 | -22.608 | 1.00 | 47.41 | A |
| ATOM | 2227 | OE1 | GLN | A | 279 | 57.623 | -0.267 | -22.982 | 1.00 | 45.83 | A |
| ATOM | 2228 | NE2 | GLN | A | 279 | 58.470 | -2.354 | -22.980 | 1.00 | 47.00 | A |
| ATOM | 2229 | C | GLN | A | 279 | 53.410 | -4.199 | -22.222 | 1.00 | 48.17 | A |
| ATOM | 2230 | O | GLN | A | 279 | 53.588 | -5.337 | -22.652 | 1.00 | 48.61 | A |
| ATOM | 2231 | N | GLY | A | 280 | 52.294 | -3.514 | -22.458 | 1.00 | 47.46 | A |
| ATOM | 2232 | CA | GLY | A | 280 | 51.217 | -4.105 | -23.241 | 1.00 | 46.73 | A |
| ATOM | 2233 | C | GLY | A | 280 | 50.454 | -5.149 | -22.456 | 1.00 | 45.87 | A |
| ATOM | 2234 | O | GLY | A | 280 | 49.437 | -5.676 | -22.906 | 1.00 | 43.90 | A |
| ATOM | 2235 | N | SER | A | 281 | 50.976 | -5.441 | -21.270 | 1.00 | 47.33 | A |
| ATOM | 2236 | CA | SER | A | 281 | 50.396 | -6.398 | -20.340 | 1.00 | 49.09 | A |
| ATOM | 2237 | CB | SER | A | 281 | 51.376 | -6.660 | -19.195 | 1.00 | 49.85 | A |
| ATOM | 2238 | OG | SER | A | 281 | 50.852 | -7.590 | -18.268 | 1.00 | 54.43 | A |
| ATOM | 2239 | C | SER | A | 281 | 49.091 | -5.840 | -19.782 | 1.00 | 49.40 | A |
| ATOM | 2240 | O | SER | A | 281 | 48.747 | -4.676 | -20.018 | 1.00 | 49.09 | A |
| ATOM | 2241 | N | ASP | A | 282 | 48.386 | -6.659 | -19.007 | 1.00 | 50.61 | A |
| ATOM | 2242 | CA | ASP | A | 282 | 47.107 | -6.245 | -18.446 | 1.00 | 50.96 | A |
| ATOM | 2243 | CB | ASP | A | 282 | 46.073 | -7.367 | -18.595 | 1.00 | 54.91 | A |
| ATOM | 2244 | CG | ASP | A | 282 | 44.635 | -6.865 | -18.473 | 1.00 | 59.55 | A |
| ATOM | 2245 | OD1 | ASP | A | 282 | 44.427 | -5.640 | -18.262 | 1.00 | 62.64 | A |
| ATOM | 2246 | OD2 | ASP | A | 282 | 43.709 | -7.703 | -18.603 | 1.00 | 60.36 | A |
| ATOM | 2247 | C | ASP | A | 282 | 47.148 | -5.769 | -17.003 | 1.00 | 48.73 | A |
| ATOM | 2248 | O | ASP | A | 282 | 47.286 | -6.564 | -16.073 | 1.00 | 49.41 | A |
| ATOM | 2249 | N | PHE | A | 283 | 47.031 | -4.459 | -16.834 | 1.00 | 45.70 | A |
| ATOM | 2250 | CA | PHE | A | 283 | 47.006 | -3.848 | -15.514 | 1.00 | 44.46 | A |
| ATOM | 2251 | CB | PHE | A | 283 | 48.349 | -3.193 | -15.124 | 1.00 | 44.55 | A |
| ATOM | 2252 | CG | PHE | A | 283 | 49.094 | -2.546 | -16.267 | 1.00 | 43.56 | A |
| ATOM | 2253 | CD1 | PHE | A | 283 | 48.907 | -1.198 | -16.568 | 1.00 | 43.66 | A |
| ATOM | 2254 | CD2 | PHE | A | 283 | 50.001 | -3.279 | -17.024 | 1.00 | 41.48 | A |
| ATOM | 2255 | CE1 | PHE | A | 283 | 49.612 | -0.595 | -17.603 | 1.00 | 42.01 | A |
| ATOM | 2256 | CE2 | PHE | A | 283 | 50.706 | -2.684 | -18.057 | 1.00 | 40.78 | A |
| ATOM | 2257 | CZ | PHE | A | 283 | 50.512 | -1.341 | -18.348 | 1.00 | 41.31 | A |
| ATOM | 2258 | C | PHE | A | 283 | 45.847 | -2.864 | -15.461 | 1.00 | 43.97 | A |
| ATOM | 2259 | O | PHE | A | 283 | 45.322 | -2.460 | -16.507 | 1.00 | 44.68 | A |
| ATOM | 2260 | N | GLY | A | 284 | 45.387 | -2.563 | -14.247 | 1.00 | 41.85 | A |
| ATOM | 2261 | CA | GLY | A | 284 | 44.268 | -1.660 | -14.094 | 1.00 | 38.42 | A |
| ATOM | 2262 | C | GLY | A | 284 | 44.720 | -0.274 | -13.738 | 1.00 | 38.04 | A |
| ATOM | 2263 | O | GLY | A | 284 | 45.916 | -0.028 | -13.543 | 1.00 | 39.00 | A |
| ATOM | 2264 | N | PRO | A | 285 | 43.783 | 0.682 | -13.716 | 1.00 | 37.28 | A |
| ATOM | 2265 | CD | PRO | A | 285 | 42.395 | 0.535 | -14.182 | 1.00 | 35.40 | A |
| ATOM | 2266 | CA | PRO | A | 285 | 44.083 | 2.080 | -13.376 | 1.00 | 35.62 | A |
| ATOM | 2267 | CB | PRO | A | 285 | 42.728 | 2.765 | -13.542 | 1.00 | 33.73 | A |
| ATOM | 2268 | CG | PRO | A | 285 | 42.066 | 1.945 | -14.591 | 1.00 | 34.90 | A |
| ATOM | 2269 | C | PRO | A | 285 | 44.552 | 2.144 | -11.926 | 1.00 | 35.06 | A |
| ATOM | 2270 | O | PRO | A | 285 | 45.542 | 2.785 | -11.607 | 1.00 | 33.73 | A |
| ATOM | 2271 | N | ILE | A | 286 | 43.851 | 1.411 | -11.069 | 1.00 | 36.06 | A |
| ATOM | 2272 | CA | ILE | A | 286 | 44.151 | 1.348 | -9.651 | 1.00 | 37.31 | A |
| ATOM | 2273 | CB | ILE | A | 286 | 43.287 | 0.270 | -8.942 | 1.00 | 39.02 | A |
| ATOM | 2274 | CG2 | ILE | A | 286 | 43.304 | 0.494 | -7.437 | 1.00 | 39.47 | A |
| ATOM | 2275 | CG1 | ILE | A | 286 | 41.837 | 0.298 | -9.453 | 1.00 | 41.72 | A |
| ATOM | 2276 | CD1 | ILE | A | 286 | 41.506 | -0.773 | -10.527 | 1.00 | 42.91 | A |
| ATOM | 2277 | C | ILE | A | 286 | 45.627 | 1.005 | -9.441 | 1.00 | 37.84 | A |
| ATOM | 2278 | O | ILE | A | 286 | 46.333 | 1.699 | -8.704 | 1.00 | 37.37 | A |
| ATOM | 2279 | N | PHE | A | 287 | 46.086 | -0.037 | -10.135 | 1.00 | 37.55 | A |
| ATOM | 2280 | CA | PHE | A | 287 | 47.470 | -0.526 | -10.059 | 1.00 | 37.12 | A |
| ATOM | 2281 | CB | PHE | A | 287 | 47.866 | -1.195 | -11.386 | 1.00 | 35.42 | A |
| ATOM | 2282 | CG | PHE | A | 287 | 49.133 | -2.013 | -11.314 | 1.00 | 32.85 | A |
| ATOM | 2283 | CD1 | PHE | A | 287 | 49.373 | -2.869 | -10.244 | 1.00 | 31.42 | A |
| ATOM | 2284 | CD2 | PHE | A | 287 | 50.067 | -1.964 | -12.350 | 1.00 | 33.77 | A |
| ATOM | 2285 | CE1 | PHE | A | 287 | 50.522 | -3.673 | -10.202 | 1.00 | 31.08 | A |
| ATOM | 2286 | CE2 | PHE | A | 287 | 51.219 | -2.764 | -12.320 | 1.00 | 32.37 | A |
| ATOM | 2287 | CZ | PHE | A | 287 | 51.444 | -3.621 | -11.239 | 1.00 | 31.41 | A |
| ATOM | 2288 | C | PHE | A | 287 | 48.514 | 0.534 | -9.685 | 1.00 | 37.14 | A |
| ATOM | 2289 | O | PHE | A | 287 | 49.095 | 0.477 | -8.593 | 1.00 | 37.20 | A |
| ATOM | 2290 | N | MET | A | 288 | 48.712 | 1.509 | -10.576 | 1.00 | 37.23 | A |
| ATOM | 2291 | CA | MET | A | 288 | 49.692 | 2.586 | -10.374 | 1.00 | 35.99 | A |
| ATOM | 2292 | CB | MET | A | 288 | 50.290 | 3.017 | -11.718 | 1.00 | 34.12 | A |
| ATOM | 2293 | CG | MET | A | 288 | 51.420 | 4.018 | -11.599 | 1.00 | 35.52 | A |
| ATOM | 2294 | SD | MET | A | 288 | 52.783 | 3.446 | -10.557 | 1.00 | 41.44 | A |
| ATOM | 2295 | CE | MET | A | 288 | 54.071 | 3.254 | -11.755 | 1.00 | 39.91 | A |
| ATOM | 2296 | C | MET | A | 288 | 49.196 | 3.821 | -9.606 | 1.00 | 35.08 | A |
| ATOM | 2297 | O | MET | A | 288 | 50.005 | 4.548 | -9.028 | 1.00 | 34.29 | A |
| ATOM | 2298 | N | THR | A | 289 | 47.882 | 4.041 | -9.570 | 1.00 | 33.54 | A |
| ATOM | 2299 | CA | THR | A | 289 | 47.345 | 5.202 | -8.876 | 1.00 | 33.29 | A |
| ATOM | 2300 | CB | THR | A | 289 | 45.793 | 5.205 | -8.813 | 1.00 | 31.59 | A |
| ATOM | 2301 | OG1 | THR | A | 289 | 45.249 | 4.986 | -10.117 | 1.00 | 32.39 | A |
| ATOM | 2302 | CG2 | THR | A | 289 | 45.294 | 6.547 | -8.331 | 1.00 | 28.33 | A |
| ATOM | 2303 | C | THR | *A* | 289 | 47.883 | 5.255 | -7.455 | 1.00 | 35.26 | A |
| ATOM | 2304 | O | THR | A | 289 | 48.506 | 6.248 | -7.059 | 1.00 | 37.52 | A |
| ATOM | 2305 | N | ILE | A | 290 | 47.722 | 4.151 | -6.728 | 1.00 | 34.25 | A |
| ATOM | 2306 | CA | ILE | A | 290 | 48.150 | 4.058 | -5.335 | 1.00 | 33.46 | A |
| ATOM | 2307 | CB | ILE | A | 290 | 47.692 | 2.710 | -4.724 | 1.00 | 32.86 | A |
| ATOM | 2308 | CG2 | ILE | A | 290 | 48.066 | 2.629 | -3.248 | 1.00 | 35.18 | A |
| ATOM | 2309 | CG1 | ILE | A | 290 | 46.174 | 2.581 | -4.851 | 1.00 | 30.60 | A |
| ATOM | 2310 | CD1 | ILE | A | 290 | 45.601 | 1.418 | -4.100 | 1.00 | 26.49 | A |
| ATOM | 2311 | C | ILE | A | 290 | 49.629 | 4.399 | -4.982 | 1.00 | 33.58 | A |
| ATOM | 2312 | O | ILE | A | 290 | 49.875 | 5.242 | -4.105 | 1.00 | 33.59 | A |
| ATOM | 2313 | N | PRO | A | 291 | 50.620 | 3.773 | -5.655 | 1.00 | 31.46 | A |
| ATOM | 2314 | CD | PRO | A | 291 | 50.546 | 2.704 | -6.659 | 1.00 | 31.92 | A |
| ATOM | 2315 | CA | PRO | A | 291 | 52.022 | 4.078 | -5.342 | 1.00 | 31.65 | A |
| ATOM | 2316 | CB | PRO | A | 291 | 52.797 | 3.122 | -6.248 | 1.00 | 31.69 | A |
| ATOM | 2317 | CG | PRO | A | 291 | 51.861 | 2.003 | -6.455 | 1.00 | 33.20 | A |
| ATOM | 2318 | C | PRO | A | 291 | 52.345 | 5.511 | -5.709 | 1.00 | 31.27 | A |
| ATOM | 2319 | O | PRO | A | 291 | 52.990 | 6.228 | -4.955 | 1.00 | 31.95 | A |
| ATOM | 2320 | N | ALA | A | 292 | 51.886 | 5.915 | -6.885 | 1.00 | 31.58 | A |
| ATOM | 2321 | CA | ALA | A | 292 | 52.112 | 7.260 | -7.381 | 1.00 | 33.06 | A |
| ATOM | 2322 | CB | ALA | A | 292 | 51.373 | 7.459 | -8.714 | 1.00 | 31.25 | A |
| ATOM | 2323 | C | ALA | A | 292 | 51.651 | 8.285 | -6.348 | 1.00 | 34.34 | A |
| ATOM | 2324 | O | ALA | A | 292 | 52.442 | 9.118 | -5.892 | 1.00 | 35.03 | A |
| ATOM | 2325 | N | PHE | A | 293 | 50.392 | 8.167 | -5.932 | 1.00 | 35.51 | A |
| ATOM | 2326 | CA | PHE | A | 293 | 49.811 | 9.081 | -4.953 | 1.00 | 36.82 | A |
| ATOM | 2327 | CB | PHE | A | 293 | 48.287 | 8.938 | -4.899 | 1.00 | 39.63 | A |
| ATOM | 2328 | CG | PHE | A | 293 | 47.571 | 9.785 | -5.915 | 1.00 | 42.47 | A |
| ATOM | 2329 | CD1 | PHE | A | 293 | 46.737 | 10.817 | -5.513 | 1.00 | 43.75 | A |
| ATOM | 2330 | CD2 | PRE | A | 293 | 47.754 | 9.571 | -7.277 | 1.00 | 43.67 | A |
| ATOM | 2331 | CE1 | PHE | A | 293 | 46.097 | 11.628 | -6.454 | 1.00 | 45.19 | A |
| ATOM | 2332 | CE2 | PHE | A | 293 | 47.117 | 10.378 | -8.224 | 1.00 | 44.16 | A |
| ATOM | 2333 | CZ | PHE | A | 293 | 46.290 | 11.407 | -7.813 | 1.00 | 42.98 | A |
| ATOM | 2334 | C | PHE | A | 293 | 50.415 | 8.989 | -3.560 | 1.00 | 35.19 | A |
| ATOM | 2335 | O | PHE | A | 293 | 50.696 | 10.009 | -2.933 | 1.00 | 36.47 | A |
| ATOM | 2336 | N | PHE | A | 294 | 50.625 | 7.775 | -3.075 | 1.00 | 32.15 | A |
| ATOM | 2337 | CA | PHE | A | 294 | 51.220 | 7.616 | -1.764 | 1.00 | 29.30 | A |
| ATOM | 2338 | CB | PHE | A | 294 | 51.427 | 6.138 | -1.466 | 1.00 | 29.77 | A |
| ATOM | 2339 | CG | PHE | A | 294 | 52.338 | 5.887 | -0.313 | 1.00 | 28.91 | A |
| ATOM | 2340 | CD1 | PHE | A | 294 | 52.090 | 6.473 | 0.923 | 1.00 | 27.15 | A |
| ATOM | 2341 | CD2 | PHE | A | 294 | 53.437 | 5.056 | -0.459 | 1.00 | 29.06 | A |
| ATOM | 2342 | CE1 | PHE | A | 294 | 52.918 | 6.235 | 2.005 | 1.00 | 28.12 | A |
| ATOM | 2343 | CE2 | PHE | A | 294 | 54.272 | 4.810 | 0.617 | 1.00 | 31.34 | A |
| ATOM | 2344 | CZ | PHE | A | 294 | 54.011 | 5.401 | 1.858 | 1.00 | 29.82 | A |
| ATOM | 2345 | C | PHE | A | 294 | 52.567 | 8.322 | -1.769 | 1.00 | 27.31 | A |
| ATOM | 2346 | O | PHE | A | 294 | 52.877 | 9.097 | -0.869 | 1.00 | 24.65 | A |
| ATOM | 2347 | N | ALA | A | 295 | 53.345 | 8.035 | -2.813 | 1.00 | 26.84 | A |
| ATOM | 2348 | CA | ALA | A | 295 | 54.671 | 8.594 | -3.025 | 1.00 | 25.76 | A |
| ATOM | 2349 | CB | ALA | A | 295 | 55.230 | 8.097 | -4.346 | 1.00 | 24.47 | A |
| ATOM | 2350 | C | ALA | A | 295 | 54.625 | 10.116 | -3.009 | 1.00 | 27.29 | A |
| ATOM | 2351 | O | ALA | A | 295 | 55.645 | 10.777 | -2.770 | 1.00 | 26.82 | A |
| ATOM | 2352 | N | RET | A | 296 | 53.436 | 10.669 | -3.247 | 1.00 | 26.76 | A |
| ATOM | 2353 | CA | RET | A | 296 | 53.257 | 12.110 | -3.242 | 1.00 | 26.52 | A |
| ATOM | 2354 | C | RET | A | 296 | 53.391 | 12.727 | -1.855 | 1.00 | 27.41 | A |
| ATOM | 2355 | O | RET | A | 296 | 53.444 | 13.951 | -1.727 | 1.00 | 28.93 | A |
| ATOM | 2356 | CB | RET | A | 296 | 51.926 | 12.489 | -3.851 | 1.00 | 25.35 | A |
| ATOM | 2357 | CG | RET | A | 296 | 51.924 | 12.144 | -5.310 | 1.00 | 24.94 | A |
| ATOM | 2358 | CD | RET | A | 296 | 50.750 | 12.863 | -5.939 | 1.00 | 27.60 | A |
| ATOM | 2359 | CE | RET | A | 296 | 50.378 | 12.496 | -7.370 | 1.00 | 26.89 | A |
| ATOM | 2360 | NZ | RET | A | 296 | 51.396 | 12.427 | -8.416 | 1.00 | 26.08 | A |
| ATOM | 2361 | C1 | RET | A | 296 | 62.366 | 6.604 | -9.481 | 1.00 | 29.92 | A |
| ATOM | 2362 | C2 | RET | A | 296 | 63.468 | 6.151 | -8.555 | 1.00 | 31.18 | A |
| ATOM | 2363 | C3 | RET | A | 296 | 63.157 | 6.427 | -7.112 | 1.00 | 31.98 | A |
| ATOM | 2364 | C4 | RET | A | 296 | 62.951 | 7.955 | -6.853 | 1.00 | 33.20 | A |
| ATOM | 2365 | C5 | RET | A | 296 | 62.157 | 8.646 | -7.962 | 1.00 | 32.58 | A |
| ATOM | 2366 | C6 | RET | A | 296 | 61.904 | 8.021 | -9.146 | 1.00 | 31.44 | A |
| ATOM | 2367 | C7 | RET | A | 296 | 61.137 | 8.661 | -10.235 | 1.00 | 31.85 | A |
| ATOM | 2368 | C8 | RET | A | 296 | 59.880 | 9.096 | -9.988 | 1.00 | 29.18 | A |
| ATOM | 2369 | C9 | RET | A | 296 | 58.946 | 9.759 | -10.914 | 1.00 | 29.34 | A |
| ATOM | 2370 | C10 | RET | A | 296 | 57.716 | 10.151 | -10.568 | 1.00 | 29.07 | A |
| ATOM | 2371 | C11 | RET | A | 296 | 56.723 | 10.823 | -11.410 | 1.00 | 26.77 | A |
| ATOM | 2372 | C12 | RET | A | 296 | 55.469 | 11.191 | -11.050 | 1.00 | 24.11 | A |
| ATOM | 2373 | C13 | RET | A | 296 | 54.638 | 11.077 | -9.809 | 1.00 | 21.23 | A |
| ATOM | 2374 | C14 | RET | A | 296 | 53.493 | 11.758 | -9.672 | 1.00 | 19.10 | A |
| ATOM | 2375 | C15 | RET | A | 296 | 52.634 | 11.658 | -8.478 | 1.00 | 23.86 | A |
| ATOM | 2376 | C16 | RET | A | 296 | 62.887 | 6.561 | -10.879 | 1.00 | 28.97 | A |
| ATOM | 2377 | C17 | RET | A | 296 | 61.189 | 5.619 | -9.408 | 1.00 | 30.51 | A |
| ATOM | 2378 | C18 | RET | A | 296 | 61.687 | 10.060 | -7.597 | 1.00 | 31.58 | A |
| ATOM | 2379 | C19 | RET | A | 296 | 59.509 | 9.945 | -12.282 | 1.00 | 28.33 | A |
| ATOM | 2380 | C20 | RET | A | 296 | 55.139 | 10.138 | -8.741 | 1.00 | 15.13 | A |
| ATOM | 2381 | N | THR | A | 297 | 53.435 | 11.893 | -0.815 | 1.00 | 27.21 | A |
| ATOM | 2382 | CA | THR | A | 297 | 53.611 | 12.403 | 0.540 | 1.00 | 26.06 | A |
| ATOM | 2383 | CB | THR | A | 297 | 53.250 | 11.363 | 1.637 | 1.00 | 24.39 | A |
| ATOM | 2384 | OG1 | THR | A | 297 | 54.106 | 10.210 | 1.546 | 1.00 | 22.52 | A |
| ATOM | 2385 | CG2 | THR | A | 297 | 51.803 | 10.965 | 1.515 | 1.00 | 18.34 | A |
| ATOM | 2386 | C | THR | A | 297 | 55.070 | 12.839 | 0.689 | 1.00 | 28.48 | A |
| ATOM | 2387 | O | THR | A | 297 | 55.487 | 13.284 | 1.752 | 1.00 | 30.19 | A |
| ATOM | 2388 | N | SER | A | 298 | 55.835 | 12.705 | -0.393 | 1.00 | 29.14 | A |
| ATOM | 2389 | CA | SER | A | 298 | 57.242 | 13.100 | -0.439 | 1.00 | 30.79 | A |
| ATOM | 2390 | CB | SER | A | 298 | 57.841 | 12.720 | -1.790 | 1.00 | 32.38 | A |
| ATOM | 2391 | OG | SER | A | 298 | 57.047 | 13.218 | -2.857 | 1.00 | 34.08 | A |
| ATOM | 2392 | C | SER | A | 298 | 57.414 | 14.603 | -0.214 | 1.00 | 31.42 | A |
| ATOM | 2393 | O | SER | A | 298 | 58.480 | 15.062 | 0.232 | 1.00 | 31.99 | A |
| ATOM | 2394 | N | ALA | A | 299 | 56.364 | 15.361 | -0.541 | 1.00 | 31.20 | A |
| ATOM | 2395 | CA | ALA | A | 299 | 56.357 | 16.814 | -0.371 | 1.00 | 31.98 | A |
| ATOM | 2396 | CB | ALA | A | 299 | 55.321 | 17.453 | -1.290 | 1.00 | 30.76 | A |
| ATOM | 2397 | C | ALA | A | 299 | 56.055 | 17.187 | 1.071 | 1.00 | 32.13 | A |
| ATOM | 2398 | O | ALA | A | 299 | 56.126 | 18.359 | 1.442 | 1.00 | 30.56 | A |
| ATOM | 2399 | N | VAL | A | 300 | 55.789 | 16.170 | 1.889 | 1.00 | 32.91 | A |
| ATOM | 2400 | CA | VAL | A | 300 | 55.424 | 16.370 | 3.286 | 1.00 | 33.83 | A |
| ATOM | 2401 | CB | VAL | A | 300 | 53.965 | 15.870 | 3.528 | 1.00 | 35.58 | A |
| ATOM | 2402 | CG1 | VAL | A | 300 | 53.660 | 15.753 | 5.034 | 1.00 | 35.20 | A |
| ATOM | 2403 | CG2 | VAL | A | 300 | 52.969 | 16.809 | 2.849 | 1.00 | 35.28 | A |
| ATOM | 2404 | C | VAL | A | 300 | 56.306 | 15.776 | 4.383 | 1.00 | 33.13 | A |
| ATOM | 2405 | O | VAL | A | 300 | 56.658 | 16.474 | 5.337 | 1.00 | 33.86 | A |
| ATOM | 2406 | N | TYR | A | 301 | 56.660 | 14.502 | 4.258 | 1.00 | 31.36 | A |
| ATOM | 2407 | CA | TYR | A | 301 | 57.418 | 13.835 | 5.306 | 1.00 | 31.19 | A |
| ATOM | 2408 | CB | TYR | A | 301 | 57.418 | 12.330 | 5.096 | 1.00 | 29.62 | A |
| ATOM | 2409 | CG | TYR | A | 301 | 58.416 | 11.879 | 4.081 | 1.00 | 28.60 | A |
| ATOM | 2410 | CD1 | TYR | A | 301 | 58.102 | 11.871 | 2.720 | 1.00 | 29.02 | A |
| ATOM | 2411 | CE1 | TYR | A | 301 | 59.035 | 11.448 | 1.766 | 1.00 | 29.21 | A |
| ATOM | 2412 | CD2 | TYR | A | 301 | 59.681 | 11.457 | 4.474 | 1.00 | 28.45 | A |
| ATOM | 2413 | CE2 | TYR | A | 301 | 60.623 | 11.032 | 3.533 | 1.00 | 29.77 | A |
| ATOM | 2414 | CZ | TYR | A | 301 | 60.298 | 11.030 | 2.180 | 1.00 | 28.64 | A |
| ATOM | 2415 | OH | TYR | A | 301 | 61.239 | 10.637 | 1.254 | 1.00 | 25.84 | A |
| ATOM | 2416 | C | TYR | A | 301 | 58.806 | 14.297 | 5.721 | 1.00 | 31.16 | A |
| ATOM | 2417 | O | TYR | A | 301 | 59.202 | 14.027 | 6.847 | 1.00 | 31.86 | A |
| ATOM | 2418 | N | ASN | A | 302 | 59.544 | 14.991 | 4.859 | 1.00 | 33.00 | A |
| ATOM | 2419 | CA | ASN | A | 302 | 60.894 | 15.435 | 5.245 | 1.00 | 33.17 | A |
| ATOM | 2420 | CB | ASN | A | 302 | 61.684 | 15.981 | 4.067 | 1.00 | 34.62 | A |
| ATOM | 2421 | CG | ASN | A | 302 | 62.491 | 14.924 | 3.388 | 1.00 | 33.91 | A |
| ATOM | 2422 | OD1 | ASN | A | 302 | 63.536 | 14.522 | 3.882 | 1.00 | 32.47 | A |
| ATOM | 2423 | ND2 | ASN | A | 302 | 62.007 | 14.455 | 2.246 | 1.00 | 36.38 | A |
| ATOM | 2424 | C | ASN | A | 302 | 60.973 | 16.406 | 6.405 | 1.00 | 33.25 | A |
| ATOM | 2425 | O | ASN | A | 302 | 61.763 | 16.192 | 7.316 | 1.00 | 33.34 | A |
| ATOM | 2426 | N | PRO | A | 303 | 60.245 | 17.539 | 6.342 | 1.00 | 34.12 | A |
| ATOM | 2427 | CD | PRO | A | 303 | 59.565 | 18.175 | 5.199 | 1.00 | 34.64 | A |
| ATOM | 2428 | CA | PRO | A | 303 | 60.320 | 18.466 | 7.479 | 1.00 | 34.54 | A |
| ATOM | 2429 | CB | PRO | A | 303 | 59.607 | 19.717 | 6.952 | 1.00 | 33.95 | A |
| ATOM | 2430 | CG | PRO | A | 303 | 58.713 | 19.198 | 5.873 | 1.00 | 35.15 | A |
| ATOM | 2431 | C | PRO | A | 303 | 59.665 | 17.884 | 8.750 | 1.00 | 34.46 | A |
| ATOM | 2432 | O | PRO | A | 303 | 59.948 | 18.315 | 9.871 | 1.00 | 32.89 | A |
| ATOM | 2433 | N | VAL | A | 304 | 58.845 | 16.855 | 8.565 | 1.00 | 34.02 | A |
| ATOM | 2434 | CA | VAL | A | 304 | 58.173 | 16.205 | 9.674 | 1.00 | 34.69 | A |
| ATOM | 2435 | CB | VAL | A | 304 | 57.054 | 15.272 | 9.162 | 1.00 | 34.55 | A |
| ATOM | 2436 | CG1 | VAL | A | 304 | 56.943 | 14.002 | 9.998 | 1.00 | 34.37 | A |
| ATOM | 2437 | CG2 | VAL | A | 304 | 55.741 | 16.027 | 9.189 | 1.00 | 34.47 | A |
| ATOM | 2438 | C | VAL | A | 304 | 59.183 | 15.473 | 10.540 | 1.00 | 35.91 | A |
| ATOM | 2439 | O | VAL | A | 304 | 59.095 | 15.513 | 11.766 | 1.00 | 37.73 | A |
| ATOM | 2440 | N | ILE | A | 305 | 60.155 | 14.828 | 9.898 | 1.00 | 37.24 | A |
| ATOM | 2441 | CA | ILE | A | 305 | 61.221 | 14.096 | 10.596 | 1.00 | 37.44 | A |
| ATOM | 2442 | CB | ILE | A | 305 | 62.002 | 13.190 | 9.599 | 1.00 | 35.65 | A |
| ATOM | 2443 | CG2 | ILE | A | 305 | 63.115 | 12.447 | 10.305 | 1.00 | 36.59 | A |
| ATOM | 2444 | CG1 | ILE | A | 305 | 61.055 | 12.169 | 8.971 | 1.00 | 35.71 | A |
| ATOM | 2445 | CD1 | ILE | A | 305 | 61.657 | 11.393 | 7.820 | 1.00 | 35.98 | A |
| ATOM | 2446 | C | ILE | A | 305 | 62.171 | 15.121 | 11.253 | 1.00 | 38.05 | A |
| ATOM | 2447 | O | ILE | A | 305 | 62.472 | 15.043 | 12.447 | 1.00 | 38.75 | A |
| ATOM | 2448 | N | TYR | A | 306 | 62.584 | 16.106 | 10.459 | 1.00 | 37.80 | A |
| ATOM | 2449 | CA | TYR | A | 306 | 63.457 | 17.201 | 10.866 | 1.00 | 36.59 | A |
| ATOM | 2450 | CB | TYR | A | 306 | 63.408 | 18.265 | 9.766 | 1.00 | 37.57 | A |
| ATOM | 2451 | CG | TYR | A | 306 | 64.467 | 19.339 | 9.838 | 1.00 | 40.05 | A |
| ATOM | 2452 | CD1 | TYR | A | 306 | 65.664 | 19.124 | 10.516 | 1.00 | 41.13 | A |
| ATOM | 2453 | CE1 | TYR | A | 306 | 66.686 | 20.076 | 10.504 | 1.00 | 41.99 | A |
| ATOM | 2454 | CD2 | TYR | A | 306 | 64.308 | 20.543 | 9.154 | 1.00 | 40.52 | A |
| ATOM | 2455 | CE2 | TYR | A | 306 | 65.323 | 21.502 | 9.135 | 1.00 | 41.90 | A |
| ATOM | 2456 | CZ | TYR | A | 306 | 66.514 | 21.257 | 9.809 | 1.00 | 42.01 | A |
| ATOM | 2457 | OH | TYR | A | 306 | 67.549 | 22.170 | 9.753 | 1.00 | 44.09 | A |
| ATOM | 2458 | C | TYR | A | 306 | 63.006 | 17.840 | 12.182 | 1.00 | 36.04 | A |
| ATOM | 2459 | O | TYR | A | 306 | 63.757 | 17.888 | 13.152 | 1.00 | 34.12 | A |
| ATOM | 2460 | N | ILE | A | 307 | 61.771 | 18.342 | 12.180 | 1.00 | 36.69 | A |
| ATOM | 2461 | CA | ILE | A | 307 | 61.146 | 19.018 | 13.322 | 1.00 | 37.98 | A |
| ATOM | 2462 | CB | ILE | A | 307 | 59.911 | 19.834 | 12.820 | 1.00 | 37.07 | A |
| ATOM | 2463 | CG2 | ILE | A | 307 | 59.415 | 20.787 | 13.891 | 1.00 | 35.40 | A |
| ATOM | 2464 | CG1 | ILE | A | 307 | 60.287 | 20.634 | 11.569 | 1.00 | 36.49 | A |
| ATOM | 2465 | CD1 | ILE | A | 307 | 59.106 | 21.191 | 10.798 | 1.00 | 36.94 | A |
| ATOM | 2466 | C | ILE | A | 307 | 60.696 | 18.020 | 14.410 | 1.00 | 40.01 | A |
| ATOM | 2467 | O | ILE | A | 307 | 59.788 | 18.304 | 15.204 | 1.00 | 40.89 | A |
| ATOM | 2468 | N | MET | A | 308 | 61.380 | 16.878 | 14.477 | 1.00 | 40.63 | A |
| ATOM | 2469 | CA | MET | A | 308 | 61.040 | 15.816 | 15.420 | 1.00 | 40.61 | A |
| ATOM | 2470 | CB | MET | A | 308 | 60.196 | 14.762 | 14.689 | 1.00 | 38.72 | A |
| ATOM | 2471 | CG | MET | A | 308 | 58.828 | 14.458 | 15.292 | 1.00 | 37.70 | A |
| ATOM | 2472 | SD | MET | A | 308 | 57.594 | 15.732 | 15.031 | 1.00 | 36.84 | A |
| ATOM | 2473 | CE | MET | A | 308 | 57.452 | 16.387 | 16.627 | 1.00 | 36.99 | A |
| ATOM | 2474 | C | MET | A | 308 | 62.293 | 15.141 | 15.984 | 1.00 | 42.16 | A |
| ATOM | 2475 | O | MET | A | 308 | 62.295 | 14.640 | 17.116 | 1.00 | 42.35 | A |
| ATOM | 2476 | N | MET | A | 309 | 63.348 | 15.121 | 15.173 | 1.00 | 43.48 | A |
| ATOM | 2477 | CA | MET | A | 309 | 64.610 | 14.490 | 15.540 | 1.00 | 43.93 | A |
| ATOM | 2478 | CB | MET | A | 309 | 64.993 | 13.429 | 14.502 | 1.00 | 43.90 | A |
| ATOM | 2479 | CG | MET | A | 309 | 64.016 | 12.276 | 14.346 | 1.00 | 43.56 | A |
| ATOM | 2480 | SD | MET | A | 309 | 64.734 | 10.975 | 13.319 | 1.00 | 43.66 | A |
| ATOM | 2481 | CE | MET | A | 309 | 65.505 | 9.951 | 14.561 | 1.00 | 42.13 | A |
| ATOM | 2482 | C | MET | A | 309 | 65.775 | 15.460 | 15.680 | 1.00 | 44.03 | A |
| ATOM | 2483 | O | MET | A | 309 | 66.902 | 15.033 | 15.936 | 1.00 | 45.37 | A |
| ATOM | 2484 | N | ASN | A | 310 | 65.533 | 16.746 | 15.449 | 1.00 | 43.52 | A |
| ATOM | 2485 | CA | ASN | A | 310 | 66.604 | 17.734 | 15.566 | 1.00 | 43.78 | A |
| ATOM | 2486 | CB | ASN | A | 310 | 66.866 | 18.446 | 14.236 | 1.00 | 44.61 | A |
| ATOM | 2487 | CG | ASN | A | 310 | 68.269 | 19.034 | 14.157 | 1.00 | 45.88 | A |
| ATOM | 2488 | OD1 | ASN | A | 310 | 68.458 | 20.251 | 14.261 | 1.00 | 47.18 | A |
| ATOM | 2489 | ND2 | ASN | A | 310 | 69.264 | 18.164 | 13.979 | 1.00 | 45.89 | A |
| ATOM | 2490 | C | ASN | A | 310 | 66.224 | 18.752 | 16.614 | 1.00 | 43.64 | A |
| ATOM | 2491 | O | ASN | A | 310 | 65.623 | 19.780 | 16.294 | 1.00 | 44.46 | A |
| ATOM | 2492 | N | LYS | A | 311 | 66.609 | 18.464 | 17.857 | 1.00 | 43.09 | A |
| ATOM | 2493 | CA | LYS | A | 311 | 66.314 | 19.310 | 19.010 | 1.00 | 42.17 | A |
| ATOM | 2494 | CB | LYS | A | 311 | 67.046 | 18.780 | 20.247 | 1.00 | 43.92 | A |
| ATOM | 2495 | CG | LYS | A | 311 | 66.562 | 19.377 | 21.554 | 1.00 | 45.89 | A |
| ATOM | 2496 | CD | LYS | A | 311 | 67.191 | 18.674 | 22.732 | 1.00 | 46.69 | A |
| ATOM | 2497 | CE | LYS | A | 311 | 66.308 | 18.766 | 23.971 | 1.00 | 48.85 | A |
| ATOM | 2498 | NZ | LYS | A | 311 | 66.144 | 20.159 | 24.447 | 1.00 | 49.39 | A |
| ATOM | 2499 | C | LYS | A | 311 | 66.644 | 20.783 | 18.783 | 1.00 | 41.32 | A |
| ATOM | 2500 | O | LYS | A | 311 | 65.940 | 21.668 | 19.284 | 1.00 | 40.00 | A |
| ATOM | 2501 | N | GLN | A | 312 | 67.705 | 21.041 | 18.018 | 1.00 | 40.16 | A |
| ATOM | 2502 | CA | GLN | A | 312 | 68.100 | 22.406 | 17.722 | 1.00 | 39.31 | A |
| ATOM | 2503 | CB | GLN | A | 312 | 69.449 | 22.451 | 17.008 | 1.00 | 41.92 | A |
| ATOM | 2504 | CG | GLN | A | 312 | 70.084 | 23.849 | 16.947 | 1.00 | 44.73 | A |
| ATOM | 2505 | CD | GLN | A | 312 | 71.357 | 23.894 | 16.099 | 1.00 | 47.96 | A |
| ATOM | 2506 | OE1 | GLN | A | 312 | 72.466 | 23.987 | 16.636 | 1.00 | 49.48 | A |
| ATOM | 2507 | NE2 | GLN | A | 312 | 71.201 | 23.829 | 14.767 | 1.00 | 47.92 | A |
| ATOM | 2508 | C | GLN | A | 312 | 67.035 | 23.064 | 16.861 | 1.00 | 37.93 | A |
| ATOM | 2509 | O | GLN | A | 312 | 66.544 | 24.127 | 17.205 | 1.00 | 37.66 | A |
| ATOM | 2510 | N | PHE | A | 313 | 66.623 | 22.405 | 15.784 | 1.00 | 37.01 | A |
| ATOM | 2511 | CA | PHE | A | 313 | 65.619 | 22.991 | 14.905 | 1.00 | 37.62 | A |
| ATOM | 2512 | CB | PHE | A | 313 | 65.527 | 22.238 | 13.578 | 1.00 | 38.44 | A |
| ATOM | 2513 | CG | PHE | A | 313 | 64.726 | 22.974 | 12.530 | 1.00 | 38.61 | A |
| ATOM | 2514 | CD1 | PHE | A | 313 | 63.387 | 22.659 | 12.303 | 1.00 | 38.34 | A |
| ATOM | 2515 | CD2 | PHE | A | 313 | 65.299 | 24.020 | 11.805 | 1.00 | 37.11 | A |
| ATOM | 2516 | CE1 | PHE | A | 313 | 62.633 | 23.377 | 11.376 | 1.00 | 37.13 | A |
| ATOM | 2517 | CE2 | PHE | A | 313 | 64.554 | 24.738 | 10.880 | 1.00 | 36.33 | A |
| ATOM | 2518 | CZ | PHE | A | 313 | 63.218 | 24.416 | 10.667 | 1.00 | 37.08 | A |
| ATOM | 2519 | C | PHE | A | 313 | 64.220 | 23.135 | 15.494 | 1.00 | 38.19 | A |
| ATOM | 2520 | O | PHE | A | 313 | 63.550 | 24.148 | 15.282 | 1.00 | 35.66 | A |
| ATOM | 2521 | N | ARG | A | 314 | 63.759 | 22.106 | 16.194 | 1.00 | 40.70 | A |
| ATOM | 2522 | CA | ARG | A | 314 | 62.426 | 22.147 | 16.782 | 1.00 | 43.32 | A |
| ATOM | 2523 | CB | ARG | A | 314 | 62.109 | 20.840 | 17.517 | 1.00 | 42.19 | A |
| ATOM | 2524 | CG | ARG | A | 314 | 60.786 | 20.853 | 18.262 | 1.00 | 41.53 | A |
| ATOM | 2525 | CD | ARG | A | 314 | 60.446 | 19.488 | 18.823 | 1.00 | 42.32 | A |
| ATOM | 2526 | NE | ARG | A | 314 | 61.443 | 18.944 | 19.750 | 1.00 | 41.37 | A |
| ATOM | 2527 | CZ | ARG | A | 314 | 62.358 | 18.032 | 19.421 | 1.00 | 40.72 | A |
| ATOM | 2528 | NH1 | ARG | A | 314 | 62.426 | 17.564 | 18.182 | 1.00 | 40.64 | A |
| ATOM | 2529 | NH2 | ARG | A | 314 | 63.158 | 17.529 | 20.348 | 1.00 | 41.92 | A |
| ATOM | 2530 | C | ARG | A | 314 | 62.264 | 23.347 | 17.711 | 1.00 | 45.16 | A |
| ATOM | 2531 | O | ARG | A | 314 | 61.387 | 24.160 | 17.500 | 1.00 | 44.33 | A |
| ATOM | 2532 | N | ASH | A | 315 | 63.141 | 23.462 | 18.704 | 1.00 | 47.23 | A |
| ATOM | 2533 | CA | ASN | A | 315 | 63.057 | 24.574 | 19.639 | 1.00 | 48.50 | A |
| ATOM | 2534 | CB | ASN | A | 315 | 64.145 | 24.479 | 20.714 | 1.00 | 48.67 | A |
| ATOM | 2535 | CG | ASN | A | 315 | 64.030 | 23.213 | 21.547 | 1.00 | 50.52 | A |
| ATOM | 2536 | OD1 | ASN | A | 315 | 65.029 | 22.536 | 21.798 | 1.00 | 51.63 | A |
| ATOM | 2537 | ND2 | ASN | A | 315 | 62.806 | 22.876 | 21.962 | 1.00 | 50.43 | A |
| ATOM | 2538 | C | ASN | A | 315 | 63.154 | 25.879 | 18.874 | 1.00 | 49.08 | A |
| ATOM | 2539 | O | ASN | A | 315 | 62.617 | 26.891 | 19.309 | 1.00 | 50.41 | A |
| ATOM | 2540 | N | CYS | A | 316 | 63.783 | 25.832 | 17.699 | 1.00 | 50.36 | A |
| ATOM | 2541 | CA | CYS | A | 316 | 63.932 | 27.017 | 16.857 | 1.00 | 50.62 | A |
| ATOM | 2542 | CB | CYS | A | 316 | 65.042 | 26.843 | 15.810 | 1.00 | 51.43 | A |
| ATOM | 2543 | SG | CYS | A | 316 | 66.663 | 27.579 | 16.272 | 1.00 | 52.48 | A |
| ATOM | 2544 | C | CYS | A | 316 | 62.631 | 27.423 | 16.190 | 1.00 | 50.70 | A |
| ATOM | 2545 | O | CYS | A | 316 | 62.412 | 28.605 | 15.984 | 1.00 | 51.42 | A |
| ATOM | 2546 | N | MET | A | 317 | 61.776 | 26.462 | 15.836 | 1.00 | 51.87 | A |
| ATOM | 2547 | CA | MET | A | 317 | 60.484 | 26.802 | 15.225 | 1.00 | 52.21 | A |
| ATOM | 2548 | CB | MET | A | 317 | 59.891 | 25.638 | 14.412 | 1.00 | 51.62 | A |
| ATOM | 2549 | CG | MET | A | 317 | 59.533 | 24.402 | 15.223 | 1.00 | 50.77 | A |
| ATOM | 2550 | SD | MET | A | 317 | 58.078 | 23.525 | 14.622 | 1.00 | 49.39 | A |
| ATOM | 2551 | CE | MET | A | 317 | 57.464 | 22.810 | 16.157 | 1.00 | 47.19 | A |
| ATOM | 2552 | C | MET | A | 317 | 59.510 | 27.234 | 16.328 | 1.00 | 52.69 | A |
| ATOM | 2553 | O | MET | A | 317 | 58.577 | 27.995 | 16.079 | 1.00 | 53.23 | A |
| ATOM | 2554 | N | VAL | A | 318 | 59.738 | 26.737 | 17.544 | 1.00 | 52.72 | A |
| ATOM | 2555 | CA | VAL | A | 318 | 58.911 | 27.081 | 18.696 | 1.00 | 52.59 | A |
| ATOM | 2556 | CB | VAL | A | 318 | 59.088 | 26.055 | 19.857 | 1.00 | 52.16 | A |
| ATOM | 2557 | CG1 | VAL | A | 318 | 58.276 | 26.465 | 21.072 | 1.00 | 52.18 | A |
| ATOM | 2558 | CG2 | VAL | A | 318 | 58.639 | 24.673 | 19.404 | 1.00 | 52.36 | A |
| ATOM | 2559 | C | VAL | A | 318 | 59.313 | 28.492 | 19.131 | 1.00 | 52.84 | A |
| ATOM | 2560 | O | VAL | A | 318 | 58.512 | 29.226 | 19.709 | 1.00 | 53.74 | A |
| ATOM | 2561 | N | THR | A | 319 | 60.556 | 28.866 | 18.831 | 1.00 | 52.88 | A |
| ATOM | 2562 | CA | THR | A | 319 | 61.068 | 30.199 | 19.145 | 1.00 | 53.14 | A |
| ATOM | 2563 | CB | THR | A | 319 | 62.636 | 30.259 | 19.109 | 1.00 | 51.52 | A |
| ATOM | 2564 | OG1 | THR | A | 319 | 63.178 | 29.654 | 20.289 | 1.00 | 49.93 | A |
| ATOM | 2565 | CG2 | THR | A | 319 | 63.133 | 31.690 | 19.038 | 1.00 | 49.25 | A |
| ATOM | 2566 | C | THR | A | 319 | 60.490 | 31.175 | 18.121 | 1.00 | 54.40 | A |
| ATOM | 2567 | O | THR | A | 319 | 60.211 | 32.329 | 18.442 | 1.00 | 55.98 | A |
| ATOM | 2568 | N | THR | A | 320 | 60.304 | 30.703 | 16.891 | 1.00 | 55.33 | A |
| ATOM | 2569 | CA | THR | A | 320 | 59.751 | 31.533 | 15.830 | 1.00 | 56.69 | A |
| ATOM | 2570 | CB | THR | A | 320 | 60.015 | 30.933 | 14.426 | 1.00 | 57.18 | A |
| ATOM | 2571 | OG1 | THR | A | 320 | 61.409 | 31.037 | 14.116 | 1.00 | 58.92 | A |
| ATOM | 2572 | CG2 | THR | A | 320 | 59.217 | 31.669 | 13.350 | 1.00 | 58.23 | A |
| ATOM | 2573 | C | THR | A | 320 | 58.259 | 31.707 | 16.043 | 1.00 | 58.00 | A |
| ATOM | 2574 | O | THR | A | 320 | 57.772 | 32.834 | 16.072 | 1.00 | 58.61 | A |
| ATOM | 2575 | N | LEU | A | 321 | 57.548 | 30.594 | 16.230 | 1.00 | 59.38 | A |
| ATOM | 2576 | CA | LEU | A | 321 | 56.098 | 30.618 | 16.439 | 1.00 | 60.65 | A |
| ATOM | 2577 | CB | LEU | A | 321 | 55.515 | 29.195 | 16.424 | 1.00 | 58.90 | A |
| ATOM | 2578 | CG | LEU | A | 321 | 55.594 | 28.362 | 15.141 | 1.00 | 56.53 | A |
| ATOM | 2579 | CD1 | LEU | A | 321 | 55.014 | 26.981 | 15.393 | 1.00 | 57.01 | A |
| ATOM | 2580 | CD2 | LEU | A | 321 | 54.853 | 29.044 | 14.020 | 1.00 | 55.19 | A |
| ATOM | 2581 | C | LEU | A | 321 | 55.681 | 31.342 | 17.724 | 1.00 | 62.50 | A |
| ATOM | 2582 | O | LEU | A | 321 | 54.568 | 31.867 | 17.802 | 1.00 | 62.64 | A |
| ATOM | 2583 | N | CYS | A | 322 | 56.563 | 31.360 | 18.727 | 1.00 | 64.96 | A |
| ATOM | 2584 | CA | CYS | A | 322 | 56.269 | 32.033 | 19.994 | 1.00 | 67.26 | A |
| ATOM | 2585 | CB | CYS | A | 322 | 56.788 | 31.223 | 21.195 | 1.00 | 66.18 | A |
| ATOM | 2586 | SG | CYS | A | 322 | 55.649 | 29.910 | 21.776 | 1.00 | 64.95 | A |
| ATOM | 2587 | C | CYS | A | 322 | 56.755 | 33.489 | 20.033 | 1.00 | 69.71 | A |
| ATOM | 2588 | O | CYS | A | 322 | 56.938 | 34.068 | 21.109 | 1.00 | 71.51 | A |
| ATOM | 2589 | N | CYS | A | 323 | 56.948 | 34.062 | 18.839 | 1.00 | 71.03 | A |
| ATOM | 2590 | CA | CYS | A | 323 | 57.364 | 35.458 | 18.615 | 1.00 | 71.18 | A |
| ATOM | 2591 | CB | CYS | A | 323 | 56.293 | 36.416 | 19.148 | 1.00 | 69.51 | A |
| ATOM | 2592 | SG | CYS | A | 323 | 54.657 | 36.152 | 18.429 | 1.00 | 65.90 | A |
| ATOM | 2593 | C | CYS | A | 323 | 58.760 | 35.937 | 19.039 | 1.00 | 72.67 | A |
| ATOM | 2594 | O | CYS | A | 323 | 59.030 | 37.146 | 19.032 | 1.00 | 73.38 | A |
| ATOM | 2595 | N | GLY | A | 324 | 59.649 | 35.005 | 19.375 | 1.00 | 73.68 | A |
| ATOM | 2596 | CA | GLY | A | 324 | 60.993 | 35.387 | 19.774 | 1.00 | 75.65 | A |
| ATOM | 2597 | C | GLY | A | 324 | 61.462 | 34.902 | 21.135 | 1.00 | 77.45 | A |
| ATOM | 2598 | O | GLY | A | 324 | 62.632 | 35.071 | 21.481 | 1.00 | 78.20 | A |
| ATOM | 2599 | N | LYS | A | 325 | 60.552 | 34.335 | 21.924 | 1.00 | 78.51 | A |
| ATOM | 2600 | CA | LYS | A | 325 | 60.888 | 33.823 | 23.251 | 1.00 | 80.08 | A |
| ATOM | 2601 | CB | LYS | A | 325 | 60.303 | 34.738 | 24.343 | 1.00 | 79.40 | A |
| ATOM | 2602 | CG | LYS | A | 325 | 61.067 | 36.075 | 24.555 | 1.00 | 79.38 | A |
| ATOM | 2603 | CD | LYS | A | 325 | 60.705 | 37.191 | 23.550 | 1.00 | 78.34 | A |
| ATOM | 2604 | CE | LYS | A | 325 | 61.701 | 38.363 | 23.621 | 1.00 | 77.80 | A |
| ATOM | 2605 | NZ | LYS | A | 325 | 61.356 | 39.539 | 22.764 | 1.00 | 76.46 | A |
| ATOM | 2606 | C | LYS | A | 325 | 60.405 | 32.365 | 23.385 | 1.00 | 81.61 | A |
| ATOM | 2607 | O | LYS | A | 325 | 59.621 | 31.895 | 22.559 | 1.00 | 81.41 | A |
| ATOM | 2608 | N | ASN | A | 326 | 60.872 | 31.650 | 24.410 | 1.00 | 83.47 | A |
| ATOM | 2609 | CA | ASN | A | 326 | 60.495 | 30.244 | 24.584 | 1.00 | 84.83 | A |
| ATOM | 2610 | CB | ASN | A | 326 | 61.368 | 29.385 | 23.652 | 1.00 | 84.66 | A |
| ATOM | 2611 | CG | ASN | A | 326 | 61.096 | 27.889 | 23.782 | 1.00 | 85.12 | A |
| ATOM | 2612 | OD1 | ASN | A | 326 | 59.946 | 27.457 | 23.914 | 1.00 | 86.02 | A |
| ATOM | 2613 | ND2 | ASN | A | 326 | 62.160 | 27.090 | 23.720 | 1.00 | 83.92 | A |
| ATOM | 2614 | C | ASN | A | 326 | 60.599 | 29.712 | 26.021 | 1.00 | 86.51 | A |
| ATOM | 2615 | O | ASN | A | 326 | 61.703 | 29.550 | 26.548 | 1.00 | 87.06 | A |
| ATOM | 2616 | N | PRO | A | 327 | 59.449 | 29.481 | 26.688 | 1.00 | 88.01 | A |
| ATOM | 2617 | CD | PRO | A | 327 | 58.187 | 30.156 | 26.330 | 1.00 | 87.56 | A |
| ATOM | 2618 | CA | PRO | A | 327 | 59.380 | 28.960 | 28.069 | 1.00 | 89.39 | A |
| ATOM | 2619 | CB | PRO | A | 327 | 57.945 | 29.297 | 28.486 | 1.00 | 88.77 | A |
| ATOM | 2620 | CG | PRO | A | 327 | 57.648 | 30.543 | 27.683 | 1.00 | 87.91 | A |
| ATOM | 2621 | C | PRO | A | 327 | 59.704 | 27.446 | 28.242 | 1.00 | 91.03 | A |
| ATOM | 2622 | O | PRO | A | 327 | 58.804 | 26.609 | 28.410 | 1.00 | 89.48 | A |
| ATOM | 2623 | N | LED | A | 328 | 61.006 | 27.138 | 28.200 | 1.00 | 93.88 | A |
| ATOM | 2624 | CA | LEU | A | 328 | 61.614 | 25.792 | 28.351 | 1.00 | 96.36 | A |
| ATOM | 2625 | CB | LEU | A | 328 | 61.360 | 24.888 | 27.106 | 1.00 | 96.00 | A |
| ATOM | 2626 | CG | LEU | A | 328 | 60.091 | 24.048 | 26.798 | 1.00 | 95.31 | A |
| ATOM | 2627 | CD1 | LEU | A | 328 | 60.096 | 23.602 | 25.325 | 1.00 | 91.86 | A |
| ATOM | 2628 | CD2 | LEU | A | 328 | 59.973 | 22.829 | 27.721 | 1.00 | 93.47 | A |
| ATOM | 2629 | C | LEU | A | 328 | 63.147 | 26.024 | 28.521 | 1.00 | 98.08 | A |
| ATOM | 2630 | O | LEU | A | 328 | 63.635 | 27.141 | 28.301 | 1.00 | 98.35 | A |
| ATOM | 2631 | N | GLY | A | 329 | 63.893 | 24.991 | 28.928 | 1.00 | 99.90 | A |
| ATOM | 2632 | CA | GLY | A | 329 | 65.343 | 25.111 | 29.097 | 1.00 | 101.45 | A |
| ATOM | 2633 | C | GLY | A | 329 | 66.054 | 23.974 | 28.374 | 1.00 | 102.65 | A |
| ATOM | 2634 | 0 | GLY | A | 329 | 65.960 | 22.827 | 28.821 | 1.00 | 103.01 | A |
| ATOM | 2635 | N | ASP | A | 330 | 66.805 | 24.284 | 27.306 | 1.00 | 103.57 | A |
| ATOM | 2636 | CA | ASP | A | 330 | 467.481 | 23.252 | 26.490 | 1.00 | 104.00 | A |
| ATOM | 2637 | CB | ASP | A | 330 | 66.780 | 23.154 | 25.120 | 1.00 | 105.06 | A |
| ATOM | 2638 | CG | ASP | A | 330 | 65.260 | 23.003 | 25.237 | 1.00 | 106.12 | A |
| ATOM | 2639 | OD1 | ASP | A | 330 | 64.787 | 22.050 | 25.897 | 1.00 | 107.06 | A |
| ATOM | 2640 | OD2 | ASP | A | 330 | 64.534 | 23.843 | 24.661 | 1.00 | 107.22 | A |
| ATOM | 2641 | C | ASP | A | 330 | 69.029 | 23.260 | 26.293 | 1.00 | 103.13 | A |
| ATOM | 2642 | O | ASP | A | 330 | 69.786 | 23.197 | 27.272 | 1.00 | 103.16 | A |
| ATOM | 2643 | N | ASP | A | 331 | 69.480 | 23.293 | 25.026 | 1.00 | 101.48 | A |
| ATOM | 2644 | CA | ASP | A | 331 | 70.914 | 23.258 | 24.664 | 1.00 | 98.70 | A |
| ATOM | 2645 | CB | ASP | A | 331 | 71.198 | 22.037 | 23.780 | 1.00 | 98.64 | A |
| ATOM | 2646 | CG | ASP | A | 331 | 71.276 | 20.744 | 24.569 | 1.00 | 98.66 | A |
| ATOM | 2647 | OD1 | ASP | A | 331 | 70.336 | 20.451 | 25.343 | 1.00 | 99.00 | A |
| ATOM | 2648 | OD2 | ASP | A | 331 | 72.281 | 20.018 | 24.410 | 1.00 | 98.02 | A |
| ATOM | 2649 | C | ASP | A | 331 | 71.587 | 24.499 | 24.040 | 1.00 | 96.32 | A |
| ATOM | 2650 | O | ASP | A | 331 | 71.392 | 25.621 | 24.517 | 1.00 | 95.80 | A |
| ATOM | 2651 | N | GLU | A | 332 | 72.409 | 24.276 | 23.005 | 1.00 | 93.22 | A |
| ATOM | 2652 | CA | GLU | A | 332 | 73.152 | 25.350 | 22.331 | 1.00 | 90.42 | A |
| ATOM | 2653 | CB | GLU | A | 332 | 74.310 | 24.802 | 21.486 | 1.00 | 90.52 | A |
| ATOM | 2654 | CG | GLU | A | 332 | 75.274 | 25.912 | 21.027 | 1.00 | 91.65 | A |
| ATOM | 2655 | CD | GLU | A | 332 | 76.031 | 25.615 | 19.728 | 1.00 | 92.83 | A |
| ATOM | 2656 | OE1 | GLU | A | 332 | 375.625 | 24.706 | 18.964 | 1.00 | 91.90 | A |
| ATOM | 2657 | OE2 | GLU | A | 332 | 77.031 | 26.327 | 19.459 | 1.00 | 93.14 | A |
| ATOM | 2658 | C | GLU | A | 332 | 72.328 | 26.287 | 21.459 | 1.00 | 88.35 | A |
| ATOM | 2659 | O | GLU | A | 332 | 71.661 | 25.855 | 20.515 | 1.00 | 87.20 | A |
| ATOM | 2660 | N | ALA | A | 333 | 72.455 | 27.583 | 21.748 | 1.00 | 86.14 | A |
| ATOM | 2661 | CA | ALA | A | 333 | 71.755 | 28.640 | 21.025 | 1.00 | 83.87 | A |
| ATOM | 2662 | CB | ALA | A | 333 | 71.894 | 29.950 | 21.767 | 1.00 | 82.93 | A |
| ATOM | 2663 | C | ALA | A | 333 | 72.296 | 28.777 | 19.607 | 1.00 | 83.07 | A |
| ATOM | 2664 | O | ALA | A | 333 | 73.512 | 28.802 | 19.395 | 1.00 | 83.41 | A |
| ATOM | 2665 | N | SER | A | 334 | 71.380 | 28.892 | 18.646 | 1.00 | 81.91 | A |
| ATOM | 2666 | CA | SER | A | 334 | 71.726 | 29.010 | 17.226 | 1.00 | 80.68 | A |
| ATOM | 2667 | CB | SER | A | 334 | 71.761 | 27.620 | 16.587 | 1.00 | 81.82 | A |
| ATOM | 2668 | OG | SER | A | 334 | 72.541 | 26.724 | 17.365 | 1.00 | 84.81 | A |
| ATOM | 2669 | C | SER | A | 334 | 70.758 | 29.915 | 16.449 | 1.00 | 78.72 | A |
| ATOM | 2670 | O | SER | A | 334 | 71.136 | 30.546 | 15.465 | 1.00 | 78.28 | A |
| ATOM | 2671 | N | THR | A | 335 | 69.497 | 29.929 | 16.862 | 1.00 | 77.24 | A |
| ATOM | 2672 | CA | THR | A | 335 | 68.491 | 30.766 | 16.234 | 1.00 | 75.70 | A |
| ATOM | 2673 | CB | THR | A | 335 | 67.132 | 30.593 | 16.887 | 1.00 | 73.35 | A |
| ATOM | 2674 | OG1 | THR | A | 335 | 66.403 | 31.814 | 16.753 | 1.00 | 70.56 | A |
| ATOM | 2675 | CG2 | THR | A | 335 | 67.271 | 30.271 | 18.367 | 1.00 | 72.63 | A |
| ATOM | 2676 | C | THR | A | 335 | 68.875 | 32.182 | 16.529 | 1.00 | 77.92 | A |
| ATOM | 2677 | O | THR | A | 335 | 68.987 | 32.528 | 17.693 | 1.00 | 78.98 | A |
| ATOM | 2678 | N | THR | A | 336 | 69.040 | 33.017 | 15.511 | 1.00 | 81.56 | A |
| ATOM | 2679 | CA | THR | A | 336 | 69.902 | 34.403 | 15.781 | 1.00 | 85.77 | A |
| ATOM | 2680 | CB | THR | A | 336 | 69.960 | 35.136 | 14.560 | 1.00 | 86.05 | A |
| ATOM | 2681 | OG1 | THR | A | 336 | 69.084 | 34.961 | 13.441 | 1.00 | 86.25 | A |
| ATOM | 2682 | CG2 | THR | A | 336 | 71.351 | 34.623 | 14.243 | 1.00 | 86.82 | A |
| ATOM | 2683 | C | THR | A | 336 | 68.232 | 35.164 | 16.386 | 1.00 | 88.98 | A |
| ATOM | 2684 | O | THR | A | 336 | 67.800 | 36.213 | 15.883 | 1.00 | 89.59 | A |
| ATOM | 2685 | N | VAL | A | 337 | 67.714 | 34.570 | 17.461 | 1.00 | 92.09 | A |
| ATOM | 2686 | CA | VAL | A | 337 | 66.618 | 35.091 | 18.263 | 1.00 | 94.70 | A |
| ATOM | 2687 | CB | VAL | A | 337 | 65.273 | 34.425 | 17.913 | 1.00 | 95.24 | A |
| ATOM | 2688 | CG1 | VAL | A | 337 | 64.172 | 35.013 | 18.774 | 1.00 | 95.76 | A |
| ATOM | 2689 | CG2 | VAL | A | 337 | 64.942 | 34.642 | 16.441 | 1.00 | 96.48 | A |
| ATOM | 2690 | C | VAL | A | 337 | 66.965 | 34.818 | 19.735 | 1.00 | 95.46 | A |
| ATOM | 2691 | O | VAL | A | 337 | 66.960 | 35.748 | 20.557 | 1.00 | 96.74 | A |
| ATOM | 2692 | N | SER | A | 338 | 67.282 | 33.557 | 20.056 | 1.00 | 95.41 | A |
| ATOM | 2693 | CA | SER | A | 338 | 67.660 | 33.175 | 21.426 | 1.00 | 95.50 | A |
| ATOM | 2694 | CB | SER | A | 338 | 67.808 | 31.650 | 21.566 | 1.00 | 94.05 | A |
| ATOM | 2695 | OG | SER | A | 338 | 68.098 | 31.278 | 22.909 | 1.00 | 91.56 | A |
| ATOM | 2696 | C | SER | A | 338 | 68.983 | 33.873 | 21.758 | 1.00 | 96.27 | A |
| ATOM | 2697 | O | SER | A | 338 | 69.099 | 34.534 | 22.797 | 1.00 | 97.75 | A |
| ATOM | 2698 | N | LYS | A | 339 | 69.965 | 33.721 | 20.865 | 1.00 | 95.29 | A |
| ATOM | 2699 | CA | LYS | A | 339 | 71.271 | 34.356 | 21.007 | 1.00 | 93.97 | A |
| ATOM | 2700 | CB | LYS | A | 339 | 72.312 | 33.424 | 21.619 | 1.00 | 92.90 | A |
| ATOM | 2701 | CG | LYS | A | 339 | 72.822 | 33.882 | 22.991 | 1.00 | 91.78 | A |
| ATOM | 2702 | CD | LYS | A | 339 | 73.166 | 35.374 | 23.051 | 1.00 | 90.29 | A |
| ATOM | 2703 | CE | LYS | A | 339 | 74.007 | 35.827 | 21.865 | 1.00 | 91.04 | A |
| ATOM | 2704 | NZ | LYS | A | 339 | 75.095 | 34.863 | 21.544 | 1.00 | 91.11 | A |
| ATOM | 2705 | C | LYS | A | 339 | 71.752 | 34.854 | 19.656 | 1.00 | 94.33 | A |
| ATOM | 2706 | 0 | LYS | A | 339 | 72.322 | 34.105 | 18.859 | 1.00 | 93.20 | A |
| ATOM | 2707 | N | THR | A | 340 | 71.547 | 36.153 | 19.453 | 1.00 | 95.82 | A |
| ATOM | 2708 | CA | THR | A | 340 | 71.893 | 36.870 | 18.232 | 1.00 | 96.50 | A |
| ATOM | 2709 | CB | THR | A | 340 | 71.988 | 38.395 | 18.493 | 1.00 | 95.39 | A |
| ATOM | 2710 | OG1 | THR | A | 340 | 72.738 | 38.630 | 19.694 | 1.00 | 94.13 | A |
| ATOM | 2711 | CG2 | THR | A | 340 | 70.589 | 39.011 | 18.617 | 1.00 | 94.00 | A |
| ATOM | 2712 | C | THR | A | 340 | 73.117 | 36.425 | 17.432 | 1.00 | 97.56 | A |
| ATOM | 2713 | O | THR | A | 340 | 72.989 | 36.156 | 16.232 | 1.00 | 98.37 | A |
| ATOM | 2714 | N | GLU | A | 341 | 74.281 | 36.283 | 18.070 | 1.00 | 97.50 | A |
| ATOM | 2715 | CA | GLU | A | 341 | 75.453 | 35.919 | 17.284 | 1.00 | 98.02 | A |
| ATOM | 2716 | CB | GLU | A | 341 | 76.095 | 37.185 | 16.727 | 1.00 | 100.37 | A |
| ATOM | 2717 | CG | GLU | A | 341 | 75.388 | 37.745 | 15.500 | 1.00 | 102.69 | A |
| ATOM | 2718 | CD | GLU | A | 341 | 75.537 | 39.245 | 15.386 | 1.00 | 104.03 | A |
| ATOM | 2719 | OE1 | GLU | A | 341 | 76.655 | 39.702 | 15.044 | 1.00 | 104.65 | A |
| ATOM | 2720 | OE2 | GLU | A | 341 | 74.536 | 39.959 | 15.648 | 1.00 | 103.84 | A |
| ATOM | 2721 | C | GLU | A | 341 | 76.541 | 34.964 | 17.755 | 1.00 | 97.29 | A |
| ATOM | 2722 | O | GLU | A | 341 | 76.552 | 34.496 | 18.887 | 1.00 | 97.04 | A |
| ATOM | 2723 | N | THR | A | 342 | 77.467 | 34.737 | 16.822 | 1.00 | 97.15 | A |
| ATOM | 2724 | CA | THR | A | 342 | 78.627 | 33.846 | 16.898 | 1.00 | 97.07 | A |
| ATOM | 2725 | CB | THR | A | 342 | 79.606 | 34.150 | 15.737 | 1.00 | 96.63 | A |
| ATOM | 2726 | OG1 | THR | A | 342 | 80.816 | 33.397 | 15.893 | 1.00 | 96.58 | A |
| ATOM | 2727 | CG2 | THR | A | 342 | 79.923 | 35.616 | 15.693 | 1.00 | 95.40 | A |
| ATOM | 2728 | C | THR | A | 342 | 79.435 | 33.620 | 18.170 | 1.00 | 97.91 | A |
| ATOM | 2729 | O | THR | A | 342 | 79.968 | 34.549 | 18.778 | 1.00 | 96.66 | A |
| ATOM | 2730 | N | SER | A | 343 | 79.599 | 32.334 | 18.473 | 1.00 | 99.97 | A |
| ATOM | 2731 | CA | SER | A | 343 | 80.345 | 31.841 | 19.626 | 1.00 | 102.48 | A |
| ATOM | 2732 | CB | SER | A | 343 | 79.402 | 31.082 | 20.576 | 1.00 | 101.74 | A |
| ATOM | 2733 | OG | SER | A | 343 | 78.764 | 29.985 | 19.933 | 1.00 | 101.04 | A |
| ATOM | 2734 | C | SER | A | 343 | 81.427 | 30.894 | 19.102 | 1.00 | 104.70 | A |
| ATOM | 2735 | 0 | SER | A | 343 | 81.966 | 30.067 | 19.842 | 1.00 | 105.05 | A |
| ATOM | 2736 | N | GLN | A | 344 | 81.751 | 31.050 | 17.820 | 1.00 | 107.65 | A |
| ATOM | 2737 | CA | GLN | A | 344 | 82.736 | 30.215 | 17.136 | 1.00 | 110.87 | A |
| ATOM | 2738 | CB | GLN | A | 344 | 82.649 | 30.448 | 15.622 | 1.00 | 111.40 | A |
| ATOM | 2739 | CG | GLN | A | 344 | 83.596 | 29.578 | 14.800 | 1.00 | 112.29 | A |
| ATOM | 2740 | CD | GLN | A | 344 | 83.544 | 29.882 | 13.317 | 1.00 | 112.55 | A |
| ATOM | 2741 | OE1 | GLN | A | 344 | 82.598 | 30.508 | 12.831 | 1.00 | 112.76 | A |
| ATOM | 2742 | NE2 | GLN | A | 344 | 84.563 | 29.431 | 12.584 | 1.00 | 112.24 | A |
| ATOM | 2743 | C | GLN | A | 344 | 84.187 | 30.372 | 17.582 | 1.00 | 112.38 | A |
| ATOM | 2744 | 0 | GLN | A | 344 | 84.638 | 31.472 | 17.909 | 1.00 | 112.63 | A |
| ATOM | 2745 | N | VAL | A | 345 | 84.910 | 29.252 | 17.571 | 1.00 | 114.55 | A |
| ATOM | 2746 | CA | VAL | A | 345 | 86.329 | 29.216 | 17.925 | 1.00 | 116.38 | A |
| ATOM | 2747 | CB | VAL | A | 345 | 86.843 | 27.744 | 18.066 | 1.00 | 116.15 | A |
| ATOM | 2748 | CG1 | VAL | A | 345 | 88.360 | 27.712 | 18.256 | 1.00 | 115.78 | A |
| ATOM | 2749 | CG2 | VAL | A | 345 | 86.160 | 27.055 | 19.240 | 1.00 | 115.57 | A |
| ATOM | 2750 | C | VAL | A | 345 | 87.102 | 29.924 | 16.804 | 1.00 | 117.70 | A |
| ATOM | 2751 | O | VAL | A | 345 | 87.017 | 29.526 | 15.634 | 1.00 | 117.56 | A |
| ATOM | 2752 | N | ALA | A | 346 | 87.828 | 30.982 | 17.172 | 1.00 | 118.99 | A |
| ATOM | 2753 | CA | ALA | A | 346 | 88.626 | 31.780 | 16.235 | 1.00 | 120.61 | A |
| ATOM | 2754 | CB | ALA | A | 346 | 89.536 | 32.750 | 17.012 | 1.00 | 119.21 | A |
| ATOM | 2755 | C | ALA | A | 346 | 89.456 | 30.919 | 15.264 | 1.00 | 121.61 | A |
| ATOM | 2756 | O | ALA | A | 346 | 89.856 | 29.797 | 15.597 | 1.00 | 121.84 | A |
| ATOM | 2757 | N | PRO | A | 347 | 89.711 | 31.435 | 14.044 | 1.00 | 122.73 | A |
| ATOM | 2758 | CD | PRO | A | 347 | 89.215 | 32.740 | 13.565 | 1.00 | 122.99 | A |
| ATOM | 2759 | CA | PRO | A | 347 | 90.479 | 30.759 | 12.986 | 1.00 | 123.78 | A |
| ATOM | 2760 | CB | PRO | A | 347 | 90.598 | 31.841 | 11.912 | 1.00 | 124.02 | A |
| ATOM | 2761 | CG | PRO | A | 347 | 89.312 | 32.587 | 12.066 | 1.00 | 123.26 | A |
| ATOM | 2762 | C | PRO | A | 347 | 91.855 | 30.213 | 13.392 | 1.00 | 124.05 | A |
| ATOM | 2763 | O | PRO | A | 347 | 92.381 | 30.546 | 14.459 | 1.00 | 125.55 | A |
| ATOM | 2764 | N | ALA | A | 348 | 92.413 | 29.363 | 12.526 | 1.00 | 124.08 | A |
| ATOM | 2765 | CA | ALA | A | 348 | 93.721 | 28.728 | 12.729 | 1.00 | 123.47 | A |
| ATOM | 2766 | CB | ALA | A | 348 | 94.858 | 29.722 | 12.424 | 1.00 | 123.92 | A |
| ATOM | 2767 | C | ALA | A | 348 | 93.883 | 28.122 | 14.127 | 1.00 | 123.50 | A |
| ATOM | 2768 | O | ALA | A | 348 | 92.980 | 27.361 | 14.545 | 1.00 | 124.39 | A |
| ATOM | 2769 | OT | ALA | A | 348 | 94.899 | 28.421 | 14.793 | 1.00 | 122.85 | A |
| ATOM | 2770 | CA | ACE | B | 0 | 52.528 | -9.795 | 41.796 | 1.00 | 56.62 | B |
| ATOM | 2771 | C | ACE | B | 0 | 51.609 | -8.901 | 40.990 | 1.00 | 56.17 | B |
| ATOM | 2772 | O | ACE | B | 0 | 50.788 | -9.378 | 40.201 | 1.00 | 54.42 | B |
| ATOM | 2773 | N | MET | B | 1 | 51.771 | -7.593 | 41.176 | 1.00 | 56.29 | B |
| ATOM | 2774 | CA | MET | B | 1 | 50.965 | -6.595 | 40.485 | 1.00 | 56.64 | B |
| ATOM | 2775 | CB | MET | B | 1 | 51.127 | -5.241 | 41.172 | 1.00 | 57.66 | B |
| ATOM | 2776 | CG | MET | B | 1 | 50.729 | -5.255 | 42.636 | 1.00 | 57.54 | B |
| ATOM | 2777 | SD | MET | B | 1 | 49.127 | -6.054 | 42.881 | 1.00 | 57.08 | B |
| ATOM | 2778 | CE | MET | B | 1 | 47.987 | -4.658 | 42.691 | 1.00 | 56.81 | B |
| ATOM | 2779 | C | MET | B | 1 | 51.277 | -6.469 | 38.989 | 1.00 | 56.84 | B |
| ATOM | 2780 | O | MET | B | 1 | 52.426 | -6.239 | 38.600 | 1.00 | 56.03 | B |
| ATOM | 2781 | N | ASN | B | 2 | 50.233 | -6.595 | 38.166 | 1.00 | 56.36 | B |
| ATOM | 2782 | CA | ASN | B | 2 | 50.346 | -6.509 | 36.710 | 1.00 | 54.69 | B |
| ATOM | 2783 | CB | ASN | B | 2 | 49.064 | -6.993 | 36.038 | 1.00 | 55.70 | B |
| ATOM | 2784 | CG | ASN | B | 2 | 48.734 | -8.419 | 36.372 | 1.00 | 57.32 | B |
| ATOM | 2785 | OD1 | ASN | B | 2 | 49.569 | -9.309 | 36.234 | 1.00 | 57.17 | B |
| ATOM | 2786 | ND2 | ASN | B | 2 | 47.498 | -8.628 | 36.811 | 1.00 | 62.05 | B |
| ATOM | 2787 | C | ASN | B | 2 | 50.619 | -5.097 | 36.232 | 1.00 | 53.06 | B |
| ATOM | 2788 | O | ASN | B | 2 | 50.915 | -4.883 | 35.057 | 1.00 | 54.59 | B |
| ATOM | 2789 | N | GLY | B | 3 | 50.464 | -4.132 | 37.127 | 1.00 | 49.84 | B |
| ATOM | 2790 | CA | GLY | B | 3 | 50.705 | -2.754 | 36.760 | 1.00 | 47.67 | B |
| ATOM | 2791 | C | GLY | B | 3 | 51.379 | -2.053 | 37.905 | 1.00 | 46.92 | B |
| ATOM | 2792 | O | GLY | B | 3 | 51.728 | -2.683 | 38.900 | 1.00 | 46.96 | B |
| ATOM | 2793 | N | THR | B | 4 | 51.593 | -0.753 | 37.763 | 1.00 | 46.69 | B |
| ATOM | 2794 | CA | THR | B | 4 | 52.218 | 0.015 | 38.827 | 1.00 | 47.21 | B |
| ATOM | 2795 | CB | THR | B | 4 | 53.689 | 0.330 | 38.504 | 1.00 | 44.95 | B |
| ATOM | 2796 | OG1 | THR | B | 4 | 54.367 | -0.885 | 38.170 | 1.00 | 44.68 | B |
| ATOM | 2797 | CG2 | THR | B | 4 | 54.379 | 0.934 | 39.704 | 1.00 | 44.31 | B |
| ATOM | 2798 | C | THR | B | 4 | 51.420 | 1.295 | 39.117 | 1.00 | 49.22 | B |
| ATOM | 2799 | O | THR | B | 4 | 51.407 | 2.235 | 38.319 | 1.00 | 50.55 | B |
| ATOM | 2800 | N | GLU | B | 5 | 50.706 | 1.291 | 40.242 | 1.00 | 49.95 | B |
| ATOM | 2801 | CA | GLU | B | 5 | 49.899 | 2.433 | 40.656 | 1.00 | 50.04 | B |
| ATOM | 2802 | CB | GLU | B | 5 | 48.656 | 1.966 | 41.433 | 1.00 | 52.37 | B |
| ATOM | 2803 | CG | GLU | B | 5 | 47.690 | 3.097 | 41.851 | 1.00 | 56.21 | B |
| ATOM | 2804 | CD | GLU | B | 5 | 46.372 | 2.608 | 42.482 | 1.00 | 58.67 | B |
| ATOM | 2805 | OE1 | GLU | B | 5 | 46.172 | 1.376 | 42.633 | 1.00 | 58.88 | B |
| ATOM | 2806 | OE2 | GLU | B | 5 | 45.526 | 3.474 | 42.820 | 1.00 | 60.22 | B |
| ATOM | 2807 | C | GLU | B | 5 | 50.689 | 3.454 | 41.481 | 1.00 | 48.61 | B |
| ATOM | 2808 | O | GLU | B | 5 | 51.524 | 3.116 | 42.323 | 1.00 | 47.10 | B |
| ATOM | 2809 | N | GLY | B | 6 | 50.471 | 4.715 | 41.159 | 1.00 | 47.47 | B |
| ATOM | 2810 | CA | GLY | B | 6 | 51.116 | 5.777 | 41.886 | 1.00 | 47.49 | B |
| ATOM | 2811 | C | GLY | B | 6 | 49.991 | 6.556 | 42.522 | 1.00 | 47.93 | B |
| ATOM | 2812 | O | GLY | B | 6 | 48.811 | 6.249 | 42.302 | 1.00 | 48.43 | B |
| ATOM | 2813 | N | PRO | B | 7 | 50.313 | 7.560 | 43.337 | 1.00 | 47.40 | B |
| ATOM | 2814 | CD | PRO | B | 7 | 51.641 | 7.998 | 43.789 | 1.00 | 47.05 | B |
| ATOM | 2815 | CA | PRO | B | 7 | 49.248 | 8.338 | 43.969 | 1.00 | 48.37 | B |
| ATOM | 2816 | CB | PRO | B | 7 | 50.020 | 9.246 | 44.926 | 1.00 | 47.93 | B |
| ATOM | 2817 | CG | PRO | B | 7 | 51.364 | 9.384 | 44.266 | 1.00 | 48.00 | B |
| ATOM | 2818 | C | PRO | B | 7 | 48.418 | 9.125 | 42.946 | 1.00 | 48.85 | B |
| ATOM | 2819 | O | PRO | B | 7 | 47.276 | 9.499 | 43.223 | 1.00 | 49.90 | B |
| ATOM | 2820 | N | ASN | B | 8 | 48.971 | 9.293 | 41.743 | 1.00 | 48.30 | B |
| ATOM | 2821 | CA | ASN | B | 8 | 48.312 | 10.037 | 40.670 | 1.00 | 46.71 | B |
| ATOM | 2822 | CB | ASN | B | 8 | 48.753 | 11.516 | 40.708 | 1.00 | 46.51 | B |
| ATOM | 2823 | CG | ASN | B | 8 | 50.251 | 11.711 | 40.441 | 1.00 | 47.21 | B |
| ATOM | 2824 | OD1 | ASN | B | 8 | 50.653 | 12.714 | 39.853 | 1.00 | 47.27 | B |
| ATOM | 2825 | ND2 | ASN | B | 8 | 51.076 | 10.767 | 40.889 | 1.00 | 48.76 | B |
| ATOM | 2826 | C | ASN | B | 8 | 48.474 | 9.452 | 39.248 | 1.00 | 45.89 | B |
| ATOM | 2827 | O | ASN | B | 8 | 48.033 | 10.057 | 38.266 | 1.00 | 45.00 | B |
| ATOM | 2828 | N | PHE | B | 9 | 49.066 | 8.265 | 39.140 | 1.00 | 44.76 | B |
| ATOM | 2829 | CA | PHE | B | 9 | 49.263 | 7.627 | 37.841 | 1.00 | 42.84 | B |
| ATOM | 2830 | CB | PHE | B | 9 | 50.691 | 7.874 | 37.331 | 1.00 | 41.93 | B |
| ATOM | 2831 | CG | PHE | B | 9 | 51.772 | 7.295 | 38.207 | 1.00 | 41.23 | B |
| ATOM | 2832 | CD1 | PHE | B | 9 | 52.190 | 5.975 | 38.045 | 1.00 | 41.40 | B |
| ATOM | 2833 | CD2 | PHE | B | 9 | 52.375 | 8.068 | 39.194 | 1.00 | 41.08 | B |
| ATOM | 2834 | CE1 | PHE | B | 9 | 53.191 | 5.434 | 38.857 | 1.00 | 42.03 | B |
| ATOM | 2835 | CE2 | PHE | B | 9 | 53.377 | 7.535 | 40.011 | 1.00 | 40.42 | B |
| ATOM | 2836 | CZ | PHE | B | 9 | 53.785 | 6.218 | 39.845 | 1.00 | 40.11 | B |
| ATOM | 2837 | C | PHE | B | 9 | 48.991 | 6.133 | 37.909 | 1.00 | 42.97 | B |
| ATOM | 2838 | O | PHE | B | 9 | 48.629 | 5.619 | 38.960 | 1.00 | 44.24 | B |
| ATOM | 2839 | N | TYR | B | 10 | 49.133 | 5.452 | 36.773 | 1.00 | 43.02 | B |
| ATOM | 2840 | CA | TYR | B | 10 | 48.950 | 4.005 | 36.694 | 1.00 | 42.81 | B |
| ATOM | 2841 | CB | TYR | B | 10 | 47.472 | 3.635 | 36.747 | 1.00 | 42.77 | B |
| ATOM | 2842 | CG | TYR | B | 10 | 47.237 | 2.148 | 36.883 | 1.00 | 46.21 | B |
| ATOM | 2843 | CD1 | TYR | B | 10 | 46.962 | 1.571 | 38.123 | 1.00 | 48.04 | B |
| ATOM | 2844 | CE1 | TYR | B | 10 | 46.752 | 0.196 | 38.253 | 1.00 | 49.11 | B |
| ATOM | 2845 | CD2 | TYR | B | 10 | 47.297 | 1.312 | 35.774 | 1.00 | 48.04 | B |
| ATOM | 2846 | CE2 | TYR | B | 10 | 47.092 | -0.061 | 35.891 | 1.00 | 49.88 | B |
| ATOM | 2847 | CZ | TYR | B | 10 | 46.822 | -0.613 | 37.128 | 1.00 | 50.02 | B |
| ATOM | 2848 | OH | TYR | B | 10 | 46.637 | -1.975 | 37.222 | 1.00 | 50.51 | B |
| ATOM | 2849 | C | TYR | B | 10 | 49.592 | 3.464 | 35.415 | 1.00 | 43.15 | B |
| ATOM | 2850 | O | TYR | B | 10 | 48.971 | 3.483 | 34.356 | 1.00 | 43.52 | B |
| ATOM | 2851 | N | VAL | B | 11 | 50.838 | 2.997 | 35.524 | 1.00 | 42.85 | B |
| ATOM | 2852 | CA | VAL | B | 11 | 51.597 | 2.450 | 34.387 | 1.00 | 44.43 | B |
| ATOM | 2853 | CB | VAL | B | 11 | 53.155 | 2.599 | 34.614 | 1.00 | 43.59 | B |
| ATOM | 2854 | CG1 | VAL | B | 11 | 53.937 | 2.153 | 33.389 | 1.00 | 41.18 | B |
| ATOM | 2855 | CG2 | VAL | B | 11 | 53.518 | 4.031 | 34.957 | 1.00 | 42.85 | B |
| ATOM | 2856 | C | VAL | B | 11 | 51.259 | 0.961 | 34.131 | 1.00 | 45.66 | B |
| ATOM | 2857 | O | VAL | B | 11 | 51.386 | 0.128 | 35.033 | 1.00 | 46.74 | B |
| ATOM | 2858 | N | PRO | B | 12 | 50.829 | 0.613 | 32.893 | 1.00 | 46.01 | B |
| ATOM | 2859 | CD | PRO | B | 12 | 50.611 | 1.536 | 31.767 | 1.00 | 46.47 | B |
| ATOM | 2860 | CA | PRO | B | 12 | 50.472 | -0.757 | 32.493 | 1.00 | 44.73 | B |
| ATOM | 2861 | CB | PRO | B | 12 | 49.850 | -0.561 | 31.109 | 1.00 | 44.56 | B |
| ATOM | 2862 | CG | PRO | B | 12 | 49.476 | 0.879 | 31.063 | 1.00 | 46.00 | B |
| ATOM | 2863 | C | PRO | B | 12 | 51.731 | -1.600 | 32.369 | 1.00 | 44.72 | B |
| ATOM | 2864 | O | PRO | B | 12 | 52.005 | -2.163 | 31.310 | 1.00 | 45.60 | B |
| ATOM | 2865 | N | PHE | B | 13 | 52.506 | -1.666 | 33.443 | 1.00 | 44.06 | B |
| ATOM | 2866 | CA | PHE | B | 13 | 53.751 | -2.414 | 33.431 | 1.00 | 44.50 | B |
| ATOM | 2867 | CB | PHE | B | 13 | 54.861 | -1.552 | 32.808 | 1.00 | 43.88 | B |
| ATOM | 2868 | CG | PHE | B | 13 | 55.895 | -2.329 | 32.022 | 1.00 | 41.83 | B |
| ATOM | 2869 | CD1 | PHE | B | 13 | 57.173 | -2.536 | 32.539 | 1.00 | 41.60 | B |
| ATOM | 2870 | CD2 | PHE | B | 13 | 55.606 | -2.807 | 30.747 | 1.00 | 40.40 | B |
| ATOM | 2871 | CE1 | PHE | B | 13 | 58.149 | -3.205 | 31.795 | 1.00 | 41.21 | B |
| ATOM | 2872 | CE2 | PHE | B | 13 | 56.575 | -3.476 | 29.996 | 1.00 | 40.71 | B |
| ATOM | 2873 | CZ | PHE | B | 13 | 57.849 | -3.674 | 30.523 | 1.00 | 39.93 | B |
| ATOM | 2874 | C | PHE | B | 13 | 54.100 | -2.743 | 34.870 | 1.00 | 45.08 | B |
| ATOM | 2875 | O | PHE | B | 13 | 54.031 | -1.881 | 35.745 | 1.00 | 44.21 | B |
| ATOM | 2876 | N | SER | B | 14 | 54.457 | -4.001 | 35.105 | 1.00 | 46.27 | B |
| ATOM | 2877 | CA | SER | B | 14 | 54.823 | -4.462 | 36.432 | 1.00 | 47.36 | B |
| ATOM | 2878 | CB | SER | B | 14 | 54.866 | -5.982 | 36.477 | 1.00 | 48.14 | B |
| ATOM | 2879 | OG | SER | B | 14 | 55.322 | -6.425 | 37.742 | 1.00 | 50.05 | B |
| ATOM | 2880 | C | SER | B | 14 | 56.167 | -3.919 | 36.862 | 1.00 | 48.44 | B |
| ATOM | 2881 | O | SER | B | 14 | 57.145 | -3.957 | 36.111 | 1.00 | 47.74 | B |
| ATOM | 2882 | N | ASN | B | 15 | 56.213 | -3.444 | 38.096 | 1.00 | 50.73 | B |
| ATOM | 2883 | CA | ASN | B | 15 | 57.438 | -2.904 | 38.635 | 1.00 | 53.70 | B |
| ATOM | 2884 | CB | ASN | B | 15 | 57.141 | -1.751 | 39.564 | 1.00 | 53.94 | B |
| ATOM | 2885 | CG | ASN | B | 15 | 58.340 | -0.922 | 39.798 | 1.00 | 56.30 | B |
| ATOM | 2886 | OD1 | ASN | B | 15 | 59.344 | -1.068 | 39.109 | 1.00 | 55.48 | B |
| ATOM | 2887 | ND2 | ASN | B | 15 | 58.278 | -0.052 | 40.781 | 1.00 | 61.35 | B |
| ATOM | 2888 | C | ASN | B | 15 | 58.253 | -3.974 | 39.357 | 1.00 | 55.76 | B |
| ATOM | 2889 | O | ASN | B | 15 | 58.927 | -3.702 | 40.360 | 1.00 | 56.06 | B |
| ATOM | 2890 | N | LYS | B | 16 | 58.181 | -5.190 | 38.815 | 1.00 | 57.67 | B |
| ATOM | 2891 | CA | LYS | B | 16 | 58.882 | -6.367 | 39.330 | 1.00 | 58.60 | B |
| ATOM | 2892 | CB | LYS | B | 16 | 58.385 | -7.608 | 38.584 | 1.00 | 59.76 | B |
| ATOM | 2893 | CG | LYS | B | 16 | 58.948 | -8.937 | 39.062 | 1.00 | 62.07 | B |
| ATOM | 2894 | CD | LYS | B | 16 | 59.570 | -9.727 | 37.904 | 1.00 | 65.24 | B |
| ATOM | 2895 | CE | LYS | B | 16 | 58.690 | -9.726 | 36.639 | 1.00 | 66.08 | B |
| ATOM | 2896 | NZ | LYS | B | 16 | 57.346 | -10.346 | 36.837 | 1.00 | 66.16 | B |
| ATOM | 2897 | C | LYS | B | 16 | 60.377 | -6.206 | 39.100 | 1.00 | 57.83 | B |
| ATOM | 2898 | O | LYS | B | 16 | 61.200 | -6.799 | 39.795 | 1.00 | 58.83 | B |
| ATOM | 2899 | N | THR | B | 17 | 60.705 | -5.373 | 38.123 | 1.00 | 56.97 | B |
| ATOM | 2900 | CA | THR | B | 17 | 62.074 | -5.096 | 37.734 | 1.00 | 57.19 | B |
| ATOM | 2901 | CB | THR | B | 17 | 62.162 | -5.069 | 36.211 | 1.00 | 58.29 | B |
| ATOM | 2902 | OG1 | THR | B | 17 | 60.932 | -4.552 | 35.675 | 1.00 | 59.73 | B |
| ATOM | 2903 | CG2 | THR | B | 17 | 62.382 | -6.468 | 35.680 | 1.00 | 59.13 | B |
| ATOM | 2904 | C | THR | B | 17 | 62.642 | -3.794 | 38.305 | 1.00 | 57.36 | B |
| ATOM | 2905 | O | THR | B | 17 | 63.814 | -3.469 | 38.068 | 1.00 | 56.18 | B |
| ATOM | 2906 | N | GLY | B | 18 | 61.808 | -3.059 | 39.049 | 1.00 | 57.45 | B |
| ATOM | 2907 | CA | GLY | B | 18 | 62.215 | -1.793 | 39.648 | 1.00 | 55.33 | B |
| ATOM | 2908 | C | GLY | B | 18 | 62.690 | -0.808 | 38.603 | 1.00 | 53.99 | B |
| ATOM | 2909 | O | GLY | B | 18 | 63.593 | -0.011 | 38.852 | 1.00 | 54.42 | B |
| ATOM | 2910 | N | VAL | B | 19 | 62.064 | -0.873 | 37.431 | 1.00 | 52.46 | B |
| ATOM | 2911 | CA | VAL | B | 19 | 62.411 | -0.030 | 36.296 | 1.00 | 50.97 | B |
| ATOM | 2912 | CB | VAL | B | 19 | 62.702 | -0.903 | 35.063 | 1.00 | 50.14 | B |
| ATOM | 2913 | CG1 | VAL | B | 19 | 61.453 | -1.675 | 34.664 | 1.00 | 51.27 | B |
| ATOM | 2914 | CG2 | VAL | B | 19 | 63.190 | -0.059 | 33.914 | 1.00 | 51.15 | B |
| ATOM | 2915 | C | VAL | B | 19 | 61.296 | 0.959 | 35.962 | 1.00 | 50.47 | B |
| ATOM | 2916 | O | VAL | B | 19 | 61.519 | 1.944 | 35.248 | 1.00 | 51.39 | B |
| ATOM | 2917 | N | VAL | B | 20 | 60.096 | 0.687 | 36.472 | 1.00 | 48.86 | B |
| ATOM | 2918 | CA | VAL | B | 20 | 58.941 | 1.553 | 36.232 | 1.00 | 46.66 | B |
| ATOM | 2919 | CB | VAL | B | 20 | 57.616 | 0.881 | 36.665 | 1.00 | 46.82 | B |
| ATOM | 2920 | CG1 | VAL | B | 20 | 56.449 | 1.855 | 36.524 | 1.00 | 44.47 | B |
| ATOM | 2921 | CG2 | VAL | B | 20 | 57.371 | -0.375 | 35.836 | 1.00 | 46.66 | B |
| ATOM | 2922 | C | VAL | B | 20 | 59.078 | 2.876 | 36.964 | 1.00 | 45.36 | B |
| ATOM | 2923 | O | VAL | B | 20 | 59.219 | 2.906 | 38.184 | 1.00 | 44.95 | B |
| ATOM | 2924 | N | ARG | B | 21 | 59.049 | 3.961 | 36.196 | 1.00 | 44.38 | B |
| ATOM | 2925 | CA | ARG | B | 21 | 59.156 | 5.317 | 36.726 | 1.00 | 43.70 | B |
| ATOM | 2926 | CB | ARG | B | 21 | 60.395 | 6.011 | 36.133 | 1.00 | 45.42 | B |
| ATOM | 2927 | CG | ARG | B | 21 | 61.480 | 6.396 | 37.159 | 1.00 | 48.26 | B |
| ATOM | 2928 | CD | ARG | B | 21 | 62.522 | 5.303 | 37.378 | 1.00 | 50.22 | B |
| ATOM | 2929 | NE | ARG | B | 21 | 61.932 | 4.037 | 37.809 | 1.00 | 51.58 | B |
| ATOM | 2930 | CZ | ARG | B | 21 | 62.459 | 3.230 | 38.726 | 1.00 | 51.28 | B |
| ATOM | 2931 | NH1 | ARG | B | 21 | 63.600 | 3.556 | 39.325 | 1.00 | 50.75 | B |
| ATOM | 2932 | NH2 | ARG | B | 21 | 61.852 | 2.086 | 39.026 | 1.00 | 50.33 | B |
| ATOM | 2933 | C | ARG | B | 21 | 57.872 | 6.111 | 36.404 | 1.00 | 42.26 | B |
| ATOM | 2934 | O | ARG | B | 21 | 57.160 | 5.783 | 35.449 | 1.00 | 40.96 | B |
| ATOM | 2935 | N | SER | B | 22 | 57.578 | 7.137 | 37.208 | 1.00 | 40.62 | B |
| ATOM | 2936 | CA | SER | B | 22 | 56.382 | 7.974 | 37.029 | 1.00 | 41.61 | B |
| ATOM | 2937 | CB | SER | B | 22 | 56.277 | 8.994 | 38.169 | 1.00 | 41.89 | B |
| ATOM | 2938 | OG | SER | B | 22 | 55.163 | 9.868 | 38.014 | 1.00 | 40.44 | B |
| ATOM | 2939 | C | SER | B | 22 | 56.315 | 8.714 | 35.689 | 1.00 | 43.45 | B |
| ATOM | 2940 | O | SER | B | 22 | 57.330 | 9.230 | 35.213 | 1.00 | 43.99 | B |
| ATOM | 2941 | N | PRO | B | 23 | 55.106 | 8.797 | 35.075 | 1.00 | 44.21 | B |
| ATOM | 2942 | CD | PRO | B | 23 | 53.844 | 8.207 | 35.551 | 1.00 | 43.55 | B |
| ATOM | 2943 | CA | PRO | B | 23 | 54.885 | 9.477 | 33.791 | 1.00 | 44.13 | B |
| ATOM | 2944 | CB | PRO | B | 23 | 53.411 | 9.189 | 33.495 | 1.00 | 42.97 | B |
| ATOM | 2945 | CG | PRO | B | 23 | 53.117 | 7.966 | 34.267 | 1.00 | 42.67 | B |
| ATOM | 2946 | C | PRO | B | 23 | 55.081 | 10.978 | 33.955 | 1.00 | 45.22 | B |
| ATOM | 2947 | O | PRO | B | 23 | 55.017 | 11.727 | 32.983 | 1.00 | 45.47 | B |
| ATOM | 2948 | N | PHE | B | 24 | 55.306 | 11.401 | 35.199 | 1.00 | 45.50 | B |
| ATOM | 2949 | CA | PHE | B | 24 | 55.490 | 12.801 | 35.537 | 1.00 | 46.93 | B |
| ATOM | 2950 | CB | PHE | B | 24 | 54.558 | 13.159 | 36.692 | 1.00 | 44.61 | B |
| ATOM | 2951 | CG | PHE | B | 24 | 53.094 | 13.010 | 36.362 | 1.00 | 44.39 | B |
| ATOM | 2952 | CD1 | PHE | B | 24 | 52.508 | 13.779 | 35.358 | 1.00 | 43.60 | B |
| ATOM | 2953 | CD2 | PHE | B | 24 | 52.298 | 12.096 | 37.045 | 1.00 | 43.97 | B |
| ATOM | 2954 | CE1 | PHE | B | 24 | 51.149 | 13.638 | 35.039 | 1.00 | 41.94 | B |
| ATOM | 2955 | CE2 | PHE | B | 24 | 50.938 | 11.951 | 36.729 | 1.00 | 42.77 | B |
| ATOM | 2956 | CZ | PHE | B | 24 | 50.368 | 12.724 | 35.724 | 1.00 | 41.56 | B |
| ATOM | 2957 | C | PHE | B | 24 | 56.929 | 13.198 | 35.882 | 1.00 | 50.05 | B |
| ATOM | 2958 | O | PHE | B | 24 | 57.294 | 14.375 | 35.775 | 1.00 | 49.62 | B |
| ATOM | 2959 | N | GLN | B | 25 | 57.751 | 12.217 | 36.261 | 1.00 | 53.32 | B |
| ATOM | 2960 | CA | GLN | B | 25 | 59.149 | 12.467 | 36.646 | 1.00 | 55.67 | B |
| ATOM | 2961 | CB | GLN | B | 25 | 59.462 | 11.798 | 37.996 | 1.00 | 58.60 | B |
| ATOM | 2962 | CG | GLN | B | 25 | 58.972 | 12.555 | 39.229 | 1.00 | 61.68 | B |
| ATOM | 2963 | CD | GLN | B | 25 | 57.958 | 11.769 | 40.043 | 1.00 | 64.32 | B |
| ATOM | 2964 | OE1 | GLN | B | 25 | 56.941 | 12.317 | 40.482 | 1.00 | 65.08 | B |
| ATOM | 2965 | NE2 | GLN | B | 25 | 58.233 | 10.477 | 40.257 | 1.00 | 65.77 | B |
| ATOM | 2966 | C | GLN | B | 25 | 60.231 | 12.062 | 35.643 | 1.00 | 55.07 | B |
| ATOM | 2967 | O | GLN | B | 25 | 61.258 | 12.739 | 35.514 | 1.00 | 55.13 | B |
| ATOM | 2968 | N | ALA | B | 26 | 60.023 | 10.938 | 34.969 | 1.00 | 53.95 | B |
| ATOM | 2969 | CA | ALA | B | 26 | 61.003 | 10.454 | 34.013 | 1.00 | 53.46 | B |
| ATOM | 2970 | CB | ALA | B | 26 | 61.934 | 9.456 | 34.691 | 1.00 | 53.87 | B |
| ATOM | 2971 | C | ALA | B | 26 | 60.341 | 9.813 | 32.808 | 1.00 | 52.28 | B |
| ATOM | 2972 | O | ALA | B | 26 | 59.217 | 9.324 | 32.896 | 1.00 | 52.55 | B |
| ATOM | 2973 | N | PRO | B | 27 | 61.019 | 9.845 | 31.650 | 1.00 | 51.44 | B |
| ATOM | 2974 | CD | PRO | B | 27 | 62.250 | 10.589 | 31.338 | 1.00 | 51.28 | B |
| ATOM | 2975 | CA | PRO | B | 27 | 60.457 | 9.244 | 30.440 | 1.00 | 50.95 | B |
| ATOM | 2976 | CB | PRO | B | 27 | 61.546 | 9.499 | 29.382 | 1.00 | 51.43 | B |
| ATOM | 2977 | CG | PRO | B | 27 | 62.784 | 9.817 | 30.173 | 1.00 | 52.04 | B |
| ATOM | 2978 | C | PRO | B | 27 | 60.096 | 7.762 | 30.583 | 1.00 | 49.53 | B |
| ATOM | 2979 | O | PRO | B | 27 | 60.773 | 7.008 | 31.285 | 1.00 | 48.39 | B |
| ATOM | 2980 | N | GLN | B | 28 | 58.978 | 7.389 | 29.958 | 1.00 | 48.35 | B |
| ATOM | 2981 | CA | GLN | B | 28 | 58.462 | 6.025 | 29.972 | 1.00 | 47.77 | B |
| ATOM | 2982 | CB | GLN | B | 28 | 56.934 | 6.038 | 29.905 | 1.00 | 44.02 | B |
| ATOM | 2983 | CG | GLN | B | 28 | 56.268 | 6.722 | 31.065 | 1.00 | 40.76 | B |
| ATOM | 2984 | CD | GLN | B | 28 | 56.652 | 6.109 | 32.383 | 1.00 | 40.11 | B |
| ATOM | 2985 | OE1 | GLN | B | 28 | 57.779 | 6.256 | 32.837 | 1.00 | 40.38 | B |
| ATOM | 2986 | NE2 | GLN | B | 28 | 55.718 | 5.416 | 33.009 | 1.00 | 41.93 | B |
| ATOM | 2987 | C | GLN | B | 28 | 59.005 | 5.218 | 28.804 | 1.00 | 48.87 | B |
| ATOM | 2988 | O | GLN | B | 28 | 58.287 | 4.401 | 28.224 | 1.00 | 48.93 | B |
| ATOM | 2989 | N | TYR | B | 29 | 60.278 | 5.426 | 28.482 | 1.00 | 50.89 | B |
| ATOM | 2990 | CA | TYR | B | 29 | 60.915 | 4.727 | 27.369 | 1.00 | 53.12 | B |
| ATOM | 2991 | CB | TYR | B | 29 | 62.254 | 5.385 | 26.998 | 1.00 | 55.23 | B |
| ATOM | 2992 | CG | TYR | B | 29 | 62.126 | 6.747 | 26.335 | 1.00 | 57.61 | B |
| ATOM | 2993 | CD1 | TYR | B | 29 | 61.132 | 6.998 | 25.390 | 1.00 | 59.10 | B |
| ATOM | 2994 | CE1 | TYR | B | 29 | 61.002 | 8.254 | 24.778 | 1.00 | 61.35 | B |
| ATOM | 2995 | CD2 | TYR | B | 29 | 62.996 | 7.787 | 26.658 | 1.00 | 59.95 | B |
| ATOM | 2996 | CE2 | TYR | B | 29 | 62.875 | 9.052 | 26.051 | 1.00 | 62.31 | B |
| ATOM | 2997 | CZ | TYR | B | 29 | 61.876 | 9.275 | 25.112 | 1.00 | 62.09 | B |
| ATOM | 2998 | OH | TYR | B | 29 | 61.755 | 10.505 | 24.499 | 1.00 | 62.36 | B |
| ATOM | 2999 | C | TYR | B | 29 | 61.108 | 3.236 | 27.623 | 1.00 | 54.08 | B |
| ATOM | 3000 | O | TYR | B | 29 | 61.603 | 2.520 | 26.748 | 1.00 | 54.62 | B |
| ATOM | 3001 | N | TYR | B | 30 | 60.705 | 2.773 | 28.809 | 1.00 | 54.30 | B |
| ATOM | 3002 | CA | TYR | B | 30 | 60.818 | 1.357 | 29.178 | 1.00 | 54.04 | B |
| ATOM | 3003 | CB | TYR | B | 30 | 61.160 | 1.221 | 30.670 | 1.00 | 52.99 | B |
| ATOM | 3004 | CG | TYR | B | 30 | 60.078 | 1.738 | 31.578 | 1.00 | 50.95 | B |
| ATOM | 3005 | CD1 | TYR | B | 30 | 58.980 | 0.939 | 31.902 | 1.00 | 50.37 | B |
| ATOM | 3006 | CE1 | TYR | B | 30 | 57.941 | 1.431 | 32.666 | 1.00 | 50.74 | B |
| ATOM | 3007 | CD2 | TYR | B | 30 | 60.111 | 3.044 | 32.054 | 1.00 | 49.32 | B |
| ATOM | 3008 | CE2 | TYR | B | 30 | 59.075 | 3.545 | 32.822 | 1.00 | 49.99 | B |
| ATOM | 3009 | CZ | TYR | B | 30 | 57.991 | 2.734 | 33.122 | 1.00 | 50.78 | B |
| ATOM | 3010 | OH | TYR | B | 30 | 56.943 | 3.223 | 33.865 | 1.00 | 52.47 | B |
| ATOM | 3011 | C | TYR | B | 30 | 59.550 | 0.543 | 28.843 | 1.00 | 54.25 | B |
| ATOM | 3012 | O | TYR | B | 30 | 59.496 | -0.662 | 29.096 | 1.00 | 53.09 | B |
| ATOM | 3013 | N | LEU | B | 31 | 58.525 | 1.222 | 28.320 | 1.00 | 55.52 | B |
| ATOM | 3014 | CA | LEU | B | 31 | 57.262 | 0.586 | 27.931 | 1.00 | 56.72 | B |
| ATOM | 3015 | CB | LEU | B | 31 | 56.102 | 1.586 | 28.035 | 1.00 | 54.35 | B |
| ATOM | 3016 | CG | LEU | B | 31 | 55.758 | 2.183 | 29.399 | 1.00 | 52.39 | B |
| ATOM | 3017 | CD1 | LEU | B | 31 | 54.478 | 2.974 | 29.314 | 1.00 | 50.81 | B |
| ATOM | 3018 | CD2 | LEU | B | 31 | 55.586 | 1.080 | 30.398 | 1.00 | 52.70 | B |
| ATOM | 3019 | C | LEU | B | 31 | 57.367 | 0.077 | 26.489 | 1.00 | 58.94 | B |
| ATOM | 3020 | O | LEU | B | 31 | 56.976 | -1.053 | 26.182 | 1.00 | 58.91 | B |
| ATOM | 3021 | N | ALA | B | 32 | 57.914 | 0.939 | 25.628 | 1.00 | 61.55 | B |
| ATOM | 3022 | CA | ALA | B | 32 | 58.131 | 0.679 | 24.201 | 1.00 | 63.06 | B |
| ATOM | 3023 | CB | ALA | B | 32 | 56.906 | 1.087 | 23.415 | 1.00 | 63.21 | B |
| ATOM | 3024 | C | ALA | B | 32 | 59.361 | 1.480 | 23.721 | 1.00 | 64.31 | B |
| ATOM | 3025 | 0 | ALA | B | 32 | 59.738 | 2.472 | 24.352 | 1.00 | 64.84 | B |
| ATOM | 3026 | N | GLU | B | 33 | 59.973 | 1.068 | 22.606 | 1.00 | 64.73 | B |
| ATOM | 3027 | CA | GLU | B | 33 | 61.168 | 1.749 | 22.072 | 1.00 | 65.17 | B |
| ATOM | 3028 | CB | GLU | B | 33 | 61.681 | 1.048 | 20.801 | 1.00 | 67.02 | B |
| ATOM | 3029 | CG | GLU | B | 33 | 62.494 | -0.233 | 21.023 | 1.00 | 69.78 | B |
| ATOM | 3030 | CD | GLU | B | 33 | 61.641 | -1.496 | 21.031 | 1.00 | 72.86 | B |
| ATOM | 3031 | OE1 | GLU | B | 33 | 61.459 | -2.082 | 22.122 | 1.00 | 73.06 | B |
| ATOM | 3032 | OE2 | GLU | B | 33 | 61.166 | -1.911 | 19.943 | 1.00 | 74.78 | B |
| ATOM | 3033 | C | GLU | B | 33 | 60.979 | 3.251 | 21.790 | 1.00 | 63.68 | B |
| ATOM | 3034 | O | GLU | B | 33 | 59.850 | 3.735 | 21.714 | 1.00 | 64.86 | B |
| ATOM | 3035 | N | PRO | B | 34 | 62.086 | 4.017 | 21.693 | 1.00 | 61.73 | B |
| ATOM | 3036 | CD | PRO | B | 34 | 63.442 | 3.664 | 22.150 | 1.00 | 60.81 | B |
| ATOM | 3037 | CA | PRO | B | 34 | 62.001 | 5.460 | 21.422 | 1.00 | 60.44 | B |
| ATOM | 3038 | CB | PRO | B | 34 | 63.446 | 5.933 | 21.625 | 1.00 | 60.44 | B |
| ATOM | 3039 | CG | PRO | B | 34 | 63.961 | 4.991 | 22.659 | 1.00 | 59.98 | B |
| ATOM | 3040 | C | PRO | B | 34 | 61.474 | 5.839 | 20.028 | 1.00 | 58.64 | B |
| ATOM | 3041 | O | PRO | B | 34 | 61.189 | 7.016 | 19.778 | 1.00 | 58.02 | B |
| ATOM | 3042 | N | TRP | B | 35 | 61.367 | 4.860 | 19.124 | 1.00 | 56.22 | B |
| ATOM | 3043 | CA | TRP | B | 35 | 60.860 | 5.132 | 17.775 | 1.00 | 55.17 | B |
| ATOM | 3044 | CB | TRP | B | 35 | 61.328 | 4.076 | 16.737 | 1.00 | 54.01 | B |
| ATOM | 3045 | CG | TRP | B | 35 | 60.612 | 2.726 | 16.753 | 1.00 | 53.08 | B |
| ATOM | 3046 | CD2 | TRP | B | 35 | 59.380 | 2.380 | 16.086 | 1.00 | 52.89 | B |
| ATOM | 3047 | CE2 | TRP | B | 35 | 59.089 | 1.039 | 16.417 | 1.00 | 52.45 | B |
| ATOM | 3048 | CE3 | TRP | B | 35 | 58.494 | 3.077 | 15.248 | 1.00 | 53.64 | B |
| ATOM | 3049 | CD1 | TRP | B | 35 | 61.002 | 1.606 | 17.423 | 1.00 | 52.53 | B |
| ATOM | 3050 | NE1 | TRP | B | 35 | 60.094 | 0.592 | 17.232 | 1.00 | 52.19 | B |
| ATOM | 3051 | CZ2 | TRP | B | 35 | 57.945 | 0.375 | 15.943 | 1.00 | 52.90 | B |
| ATOM | 3052 | CZ3 | TRP | B | 35 | 57.354 | 2.415 | 14.775 | 1.00 | 53.17 | B |
| ATOM | 3053 | CH2 | TRP | B | 35 | 57.095 | 1.078 | 15.126 | 1.00 | 53.05 | B |
| ATOM | 3054 | C | TRP | B | 35 | 59.336 | 5.250 | 17.782 | 1.00 | 54.39 | B |
| ATOM | 3055 | O | TRP | B | 35 | 58.756 | 6.006 | 16.996 | 1.00 | 55.11 | B |
| ATOM | 3056 | N | GLN | B | 36 | 58.698 | 4.526 | 18.698 | 1.00 | 52.03 | B |
| ATOM | 3057 | CA | GLN | B | 36 | 57.247 | 4.529 | 18.818 | 1.00 | 50.05 | B |
| ATOM | 3058 | CB | GLN | B | 36 | 56.790 | 3.330 | 19.664 | 1.00 | 51.17 | B |
| ATOM | 3059 | CG | GLN | B | 36 | 57.586 | 2.055 | 19.354 | 1.00 | 52.08 | B |
| ATOM | 3060 | CD | GLN | B | 36 | 56.811 | 0.769 | 19.560 | 1.00 | 53.58 | B |
| ATOM | 3061 | OE1 | GLN | B | 36 | 57.045 | 0.039 | 20.522 | 1.00 | 54.93 | B |
| ATOM | 3062 | NE2 | GLN | B | 36 | 55.919 | 0.457 | 18.625 | 1.00 | 54.05 | B |
| ATOM | 3063 | C | GLN | B | 36 | 56.748 | 5.861 | 19.385 | 1.00 | 48.52 | B |
| ATOM | 3064 | O | GLN | B | 36 | 55.633 | 6.281 | 19.093 | 1.00 | 47.47 | B |
| ATOM | 3065 | N | PHE | B | 37 | 57.599 | 6.532 | 20.161 | 1.00 | 48.07 | B |
| ATOM | 3066 | CA | PHE | B | 37 | 57.286 | 7.839 | 20.761 | 1.00 | 47.14 | B |
| ATOM | 3067 | CB | PHE | B | 37 | 58.189 | 8.126 | 21.963 | 1.00 | 45.09 | B |
| ATOM | 3068 | CG | PHE | B | 37 | 57.759 | 7.434 | 23.208 | 1.00 | 42.17 | B |
| ATOM | 3069 | CD1 | PHE | B | 37 | 56.976 | 8.092 | 24.140 | 1.00 | 42.47 | B |
| ATOM | 3070 | CD2 | PHE | B | 37 | 58.122 | 6.123 | 23.445 | 1.00 | 41.83 | B |
| ATOM | 3071 | CE1 | PHE | B | 37 | 56.561 | 7.457 | 25.294 | 1.00 | 42.78 | B |
| ATOM | 3072 | CE2 | PHE | B | 37 | 57.712 | 5.475 | 24.598 | 1.00 | 42.89 | B |
| ATOM | 3073 | CZ | PHE | B | 37 | 56.929 | 6.145 | 25.527 | 1.00 | 42.86 | B |
| ATOM | 3074 | C | PHE | B | 37 | 57.438 | 8.968 | 19.753 | 1.00 | 46.95 | B |
| ATOM | 3075 | O | PHE | B | 37 | 56.563 | 9.831 | 19.634 | 1.00 | 47.86 | B |
| ATOM | 3076 | N | SER | B | 38 | 58.576 | 8.987 | 19.066 | 1.00 | 46.08 | B |
| ATOM | 3077 | CA | SER | B | 38 | 58.820 | 10.002 | 18.057 | 1.00 | 45.36 | B |
| ATOM | 3078 | CB | SER | B | 38 | 60.263 | 9.926 | 17.555 | 1.00 | 45.66 | B |
| ATOM | 3079 | OG | SER | B | 38 | 61.139 | 10.589 | 18.453 | 1.00 | 46.91 | B |
| ATOM | 3080 | C | SER | B | 38 | 57.821 | 9.805 | 16.922 | 1.00 | 44.05 | B |
| ATOM | 3081 | O | SER | B | 38 | 57.476 | 10.745 | 16.215 | 1.00 | 43.74 | B |
| ATOM | 3082 | N | MET | B | 39 | 57.317 | 8.583 | 16.796 | 1.00 | 43.22 | B |
| ATOM | 3083 | CA | MET | B | 39 | 56.339 | 8.252 | 15.769 | 1.00 | 44.60 | B |
| ATOM | 3084 | CB | MET | B | 39 | 56.304 | 6.728 | 15.581 | 1.00 | 46.93 | B |
| ATOM | 3085 | CG | MET | B | 39 | 55.816 | 6.241 | 14.226 | 1.00 | 50.25 | B |
| ATOM | 3086 | SD | MET | B | 39 | 56.823 | 6.850 | 12.855 | 1.00 | 53.99 | B |
| ATOM | 3087 | CE | MET | B | 39 | 55.502 | 7.271 | 11.628 | 1.00 | 53.21 | B |
| ATOM | 3088 | C | MET | B | 39 | 54.947 | 8.811 | 16.166 | 1.00 | 43.67 | B |
| ATOM | 3089 | O | MET | B | 39 | 54.100 | 9.074 | 15.304 | 1.00 | 42.24 | B |
| ATOM | 3090 | N | LEU | B | 40 | 54.720 | 8.957 | 17.475 | 1.00 | 42.15 | B |
| ATOM | 3091 | CA | LEU | B | 40 | 53.476 | 9.506 | 18.016 | 1.00 | 39.42 | B |
| ATOM | 3092 | CB | LEU | B | 40 | 53.296 | 9.119 | 19.486 | 1.00 | 37.40 | B |
| ATOM | 3093 | CG | LEU | B | 40 | 53.068 | 7.669 | 19.906 | 1.00 | 35.78 | B |
| ATOM | 3094 | CD1 | LEU | B | 40 | 52.869 | 7.623 | 21.413 | 1.00 | 34.54 | B |
| ATOM | 3095 | CD2 | LEU | B | 40 | 51.857 | 7.087 | 19.200 | 1.00 | 35.18 | B |
| ATOM | 3096 | C | LEU | B | 40 | 53.585 | 11.025 | 17.926 | 1.00 | 39.67 | B |
| ATOM | 3097 | O | LEU | B | 40 | 52.616 | 11.716 | 17.611 | 1.00 | 38.83 | B |
| ATOM | 3098 | N | ALA | B | 41 | 54.776 | 11.534 | 18.231 | 1.00 | 39.27 | B |
| ATOM | 3099 | CA | ALA | B | 41 | 55.046 | 12.960 | 18.167 | 1.00 | 39.60 | B |
| ATOM | 3100 | CB | ALA | B | 41 | 56.433 | 13.243 | 18.718 | 1.00 | 38.44 | B |
| ATOM | 3101 | C | ALA | B | 41 | 54.923 | 13.453 | 16.713 | 1.00 | 40.46 | B |
| ATOM | 3102 | O | ALA | B | 41 | 54.617 | 14.627 | 16.478 | 1.00 | 41.22 | B |
| ATOM | 3103 | N | ALA | B | 42 | 55.152 | 12.545 | 15.753 | 1.00 | 39.53 | B |
| ATOM | 3104 | CA | ALA | B | 42 | 55.066 | 12.837 | 14.315 | 1.00 | 37.70 | B |
| ATOM | 3105 | CB | ALA | B | 42 | 55.744 | 11.736 | 13.510 | 1.00 | 35.72 | B |
| ATOM | 3106 | C | ALA | B | 42 | 53.605 | 12.939 | 13.912 | 1.00 | 38.00 | B |
| ATOM | 3107 | O | ALA | B | 42 | 53.208 | 13.835 | 13.168 | 1.00 | 37.79 | B |
| ATOM | 3108 | N | TYR | B | 43 | 52.820 | 11.988 | 14.408 | 1.00 | 38.90 | B |
| ATOM | 3109 | CA | TYR | B | 43 | 51.383 | 11.912 | 14.164 | 1.00 | 39.12 | B |
| ATOM | 3110 | CB | TYR | B | 43 | 50.821 | 10.682 | 14.900 | 1.00 | 37.93 | B |
| ATOM | 3111 | CG | TYR | B | 43 | 49.312 | 10.472 | 14.881 | 1.00 | 37.01 | B |
| ATOM | 3112 | CD1 | TYR | B | 43 | 48.618 | 10.236 | 16.066 | 1.00 | 36.07 | B |
| ATOM | 3113 | CE1 | TYR | B | 43 | 47.258 | 9.963 | 16.070 | 1.00 | 36.64 | B |
| ATOM | 3114 | CD2 | TYR | B | 43 | 48.592 | 10.438 | 13.684 | 1.00 | 37.92 | B |
| ATOM | 3115 | CE2 | TYR | B | 43 | 47.216 | 10.163 | 13.678 | 1.00 | 38.14 | B |
| ATOM | 3116 | CZ | TYR | B | 43 | 46.561 | 9.925 | 14.880 | 1.00 | 38.00 | B |
| ATOM | 3117 | OH | TYR | B | 43 | 45.211 | 9.645 | 14.899 | 1.00 | 38.37 | B |
| ATOM | 3118 | C | TYR | B | 43 | 50.745 | 13.195 | 14.682 | 1.00 | 39.72 | B |
| ATOM | 3119 | 0 | TYR | B | 43 | 50.052 | 13.901 | 13.943 | 1.00 | 38.83 | B |
| ATOM | 3120 | N | MET | B | 44 | 51.038 | 13.524 | 15.938 | 1.00 | 40.88 | B |
| ATOM | 3121 | CA | MET | B | 44 | 50.483 | 14.721 | 16.552 | 1.00 | 42.90 | B |
| ATOM | 3122 | CB | MET | B | 44 | 50.847 | 14.802 | 18.031 | 1.00 | 42.31 | B |
| ATOM | 3123 | CG | MET | B | 44 | 50.163 | 13.740 | 18.894 | 1.00 | 41.95 | B |
| ATOM | 3124 | SD | MET | B | 44 | 48.393 | 13.488 | 18.578 | 1.00 | 41.47 | B |
| ATOM | 3125 | CE | MET | B | 44 | 47.685 | 14.957 | 19.163 | 1.00 | 39.26 | B |
| ATOM | 3126 | C | MET | B | 44 | 50.884 | 15.996 | 15.828 | 1.00 | 43.78 | B |
| ATOM | 3127 | O | MET | B | 44 | 50.150 | 16.979 | 15.845 | 1.00 | 43.29 | B |
| ATOM | 3128 | N | PHE | B | 45 | 52.038 | 15.971 | 15.171 | 1.00 | 45.28 | B |
| ATOM | 3129 | CA | PHE | B | 45 | 52.492 | 17.135 | 14.428 | 1.00 | 45.81 | B |
| ATOM | 3130 | CB | PHE | B | 45 | 53.967 | 17.000 | 14.042 | 1.00 | 46.23 | B |
| ATOM | 3131 | CG | PHE | B | 45 | 54.516 | 18.214 | 13.361 | 1.00 | 45.63 | B |
| ATOM | 3132 | CD1 | PHE | B | 45 | 54.541 | 18.295 | 11.974 | 1.00 | 45.05 | B |
| ATOM | 3133 | CD2 | PHE | B | 45 | 54.953 | 19.298 | 14.104 | 1.00 | 46.24 | B |
| ATOM | 3134 | CE1 | PHE | B | 45 | 54.985 | 19.438 | 11.339 | 1.00 | 45.91 | B |
| ATOM | 3135 | CE2 | PHE | B | 45 | 55.402 | 20.452 | 13.475 | 1.00 | 48.36 | B |
| ATOM | 3136 | CZ | PHE | B | 45 | 55.417 | 20.522 | 12.087 | 1.00 | 47.45 | B |
| ATOM | 3137 | C | PHE | B | 45 | 51.612 | 17.294 | 13.184 | 1.00 | 46.20 | B |
| ATOM | 3138 | O | PHE | B | 45 | 51.279 | 18.420 | 12.787 | 1.00 | 47.47 | B |
| ATOM | 3139 | N | LEU | B | 46 | 51.239 | 16.163 | 12.582 | 1.00 | 45.25 | B |
| ATOM | 3140 | CA | LEU | B | 46 | 50.372 | 16.154 | 11.404 | 1.00 | 44.05 | B |
| ATOM | 3141 | CB | LEU | B | 46 | 50.307 | 14.752 | 10.792 | 1.00 | 43.08 | B |
| ATOM | 3142 | CG | LEU | B | 46 | 51.638 | 14.282 | 10.221 | 1.00 | 41.43 | B |
| ATOM | 3143 | CD1 | LEU | B | 46 | 51.489 | 12.926 | 9.576 | 1.00 | 40.47 | B |
| ATOM. | 3144 | CD2 | LEU | B | 46 | 52.113 | 15.303 | 9.219 | 1.00 | 40.35 | B |
| ATOM | 3145 | C | LEU | B | 46 | 48.967 | 16.620 | 11.785 | 1.00 | 43.60 | B |
| ATOM | 3146 | O | LEU | B | 46 | 48.358 | 17.427 | 11.074 | 1.00 | 43.76 | B |
| ATOM | 3147 | N | LEU | B | 47 | 48.469 | 16.132 | 12.923 | 1.00 | 42.05 | B |
| ATOM | 3148 | CA | LEU | B | 47 | 47.144 | 16.510 | 13.398 | 1.00 | 39.55 | B |
| ATOM | 3149 | CB | LEU | B | 47 | 46.740 | 15.669 | 14.603 | 1.00 | 36.17 | B |
| ATOM | 3150 | CG | LEU | B | 47 | 46.297 | 14.251 | 14.244 | 1.00 | 34.72 | B |
| ATOM | 3151 | CD1 | LEU | B | 47 | 45.799 | 13.532 | 15.476 | 1.00 | 34.52 | B |
| ATOM | 3152 | CD2 | LEU | B | 47 | 45.204 | 14.311 | 13.198 | 1.00 | 34.00 | B |
| ATOM | 3153 | C | LEU | B | 47 | 47.079 | 17.990 | 13.728 | 1.00 | 39.58 | B |
| ATOM | 3154 | 0 | LEU | B | 47 | 46.041 | 18.621 | 13.570 | 1.00 | 38.78 | B |
| ATOM | 3155 | N | ILE | B | 48 | 48.208 | 18.542 | 14.159 | 1.00 | 41.00 | B |
| ATOM | 3156 | CA | ILE | B | 48 | 48.293 | 19.958 | 14.496 | 1.00 | 42.13 | B |
| ATOM | 3157 | CB | ILE | B | 48 | 49.504 | 20.276 | 15.421 | 1.00 | 43.41 | B |
| ATOM | 3158 | CG2 | ILE | B | 48 | 49.729 | 21.796 | 15.519 | 1.00 | 43.99 | B |
| ATOM | 3159 | CG1 | ILE | B | 48 | 49.272 | 19.699 | 16.824 | 1.00 | 42.88 | B |
| ATOM | 3160 | CD1 | ILE | B | 48 | 50.448 | 19.881 | 17.778 | 1.00 | 40.74 | B |
| ATOM | 3161 | C | ILE | B | 48 | 48.407 | 20.796 | 13.239 | 1.00 | 42.20 | B |
| ATOM | 3162 | O | ILE | B | 48 | 47.734 | 21.816 | 13.113 | 1.00 | 43.54 | B |
| ATOM | 3163 | N | MET | B | 49 | 49.245 | 20.365 | 12.304 | 1.00 | 42.04 | B |
| ATOM | 3164 | CA | MET | B | 49 | 49.427 | 21.119 | 11.073 | 1.00 | 42.55 | B |
| ATOM | 3165 | CB | MET | B | 49 | 50.792 | 20.808 | 10.475 | 1.00 | 43.09 | B |
| ATOM | 3166 | CG | MET | B | 49 | 51.965 | 21.326 | 11.330 | 1.00 | 44.02 | B |
| ATOM | 3167 | SD | MET | B | 49 | 52.289 | 23.128 | 11.199 | 1.00 | 43.54 | B |
| ATOM | 3168 | CE | MET | B | 49 | 51.507 | 23.740 | 12.693 | 1.00 | 43.50 | B |
| ATOM | 3169 | C | MET | B | 49 | 48.287 | 20.987 | 10.050 | 1.00 | 43.25 | B |
| ATOM | 3170 | O | MET | B | 49 | 48.294 | 21.654 | 9.009 | 1.00 | 43.37 | B |
| ATOM | 3171 | N | LEU | B | 50 | 47.289 | 20.161 | 10.374 | 1.00 | 43.51 | B |
| ATOM | 3172 | CA | LEU | B | 50 | 46.110 | 19.980 | 9.516 | 1.00 | 43.62 | B |
| ATOM | 3173 | CB | LEU | B | 50 | 45.916 | 18.508 | 9.123 | 1.00 | 40.80 | B |
| ATOM | 3174 | CG | LEU | B | 50 | 45.360 | 18.176 | 7.725 | 1.00 | 37.18 | B |
| ATOM | 3175 | CD1 | LEU | B | 50 | 44.935 | 16.728 | 7.689 | 1.00 | 35.08 | B |
| ATOM | 3176 | CD2 | LEU | B | 50 | 44.199 | 19.067 | 7.332 | 1.00 | 35.57 | B |
| ATOM | 3177 | C | LEU | B | 50 | 44.864 | 20.461 | 10.281 | 1.00 | 44.86 | B |
| ATOM | 3178 | O | LEU | B | 50 | 44.070 | 21.258 | 9.768 | 1.00 | 44.74 | B |
| ATOM | 3179 | N | GLY | B | 51 | 44.711 | 19.972 | 11.512 | 1.00 | 44.90 | B |
| ATOM | 3180 | CA | GLY | B | 51 | 43.577 | 20.346 | 12.340 | 1.00 | 43.55 | B |
| ATOM | 3181 | C | GLY | B | 51 | 43.424 | 21.843 | 12.494 | 1.00 | 42.29 | B |
| ATOM | 3182 | O | GLY | B | 51 | 42.322 | 22.339 | 12.707 | 1.00 | 41.07 | B |
| ATOM | 3183 | N | PHE | B | 52 | 44.532 | 22.567 | 12.380 | 1.00 | 41.81 | B |
| ATOM | 3184 | CA | PHE | B | 52 | 44.490 | 24.018 | 12.494 | 1.00 | 41.83 | B |
| ATOM | 3185 | CB | PHE | B | 52 | 45.857 | 24.589 | 12.899 | 1.00 | 42.44 | B |
| ATOM | 3186 | CG | PHE | B | 52 | 45.964 | 26.074 | 12.723 | 1.00 | 43.44 | B |
| ATOM | 3187 | CD1 | PHE | B | 52 | 47.035 | 26.624 | 12.046 | 1.00 | 44.68 | B |
| ATOM | 3188 | CD2 | PHE | B | 52 | 44.968 | 26.922 | 13.207 | 1.00 | 45.28 | B |
| ATOM | 3189 | CE1 | PHE | B | 52 | 47.111 | 27.994 | 11.852 | 1.00 | 46.38 | B |
| ATOM | 3190 | CE2 | PHE | B | 52 | 45.034 | 28.291 | 13.021 | 1.00 | 45.02 | B |
| ATOM | 3191 | CZ | PHE | B | 52 | 46.104 | 28.830 | 12.343 | 1.00 | 45.78 | B |
| ATOM | 3192 | C | PHE | B | 52 | 43.983 | 24.706 | 11.225 | 1.00 | 40.74 | B |
| ATOM | 3193 | O | PHE | B | 52 | 42.901 | 25.304 | 11.234 | 1.00 | 41.11 | B |
| ATOM | 3194 | N | PRO | B | 53 | 44.730 | 24.591 | 10.109 | 1.00 | 39.25 | B |
| ATOM | 3195 | CD | PRO | B | 53 | 45.954 | 23.810 | 9.846 | 1.00 | 37.51 | B |
| ATOM | 3196 | CA | PRO | B | 53 | 44.269 | 25.250 | 8.885 | 1.00 | 37.86 | B |
| ATOM | 3197 | CB | PRO | B | 53 | 45.308 | 24.814 | 7.849 | 1.00 | 37.65 | B |
| ATOM | 3198 | CG | PRO | B | 53 | 46.538 | 24.533 | 8.674 | 1.00 | 38.31 | B |
| ATOM | 3199 | C | PRO | B | 53 | 42.859 | 24.858 | 8.468 | 1.00 | 37.26 | B |
| ATOM | 3200 | O | PRO | B | 53 | 42.074 | 25.714 | 8.080 | 1.00 | 36.92 | B |
| ATOM | 3201 | N | ILE | B | 54 | 42.524 | 23.582 | 8.630 | 1.00 | 38.07 | B |
| ATOM | 3202 | CA | ILE | B | 54 | 41.216 | 23.067 | 8.232 | 1.00 | 40.73 | B |
| ATOM | 3203 | CB | ILE | B | 54 | 41.235 | 21.534 | 8.071 | 1.00 | 41.18 | B |
| ATOM | 3204 | CG2 | ILE | B | 54 | 41.127 | 20.854 | 9.430 | 1.00 | 42.67 | B |
| ATOM | 3205 | CG1 | ILE | B | 54 | 40.078 | 21.084 | 7.175 | 1.00 | 41.30 | B |
| ATOM | 3206 | CD1 | ILE | B | 54 | 40.167 | 19.629 | 6.738 | 1.00 | 40.59 | B |
| ATOM | 3207 | C | ILE | B | 54 | 40.035 | 23.451 | 9.115 | 1.00 | 41.79 | B |
| ATOM | 3208 | O | ILE | B | 54 | 38.912 | 23.549 | 8.625 | 1.00 | 42.70 | B |
| ATOM | 3209 | N | ASN | B | 55 | 40.268 | 23.601 | 10.417 | 1.00 | 42.68 | B |
| ATOM | 3210 | CA | ASN | B | 55 | 39.197 | 23.981 | 11.329 | 1.00 | 41.66 | B |
| ATOM | 3211 | CB | ASN | B | 55 | 39.484 | 23.484 | 12.749 | 1.00 | 40.62 | B |
| ATOM | 3212 | CG | ASN | B | 55 | 39.189 | 21.996 | 12.921 | 1.00 | 39.17 | B |
| ATOM | 3213 | OD1 | ASN | B | 55 | 38.061 | 21.546 | 12.742 | 1.00 | 38.79 | B |
| ATOM | 3214 | ND2 | ASN | B | 55 | 40.197 | 21.239 | 13.308 | 1.00 | 38.41 | B |
| ATOM | 3215 | C | ASN | B | 55 | 39.040 | 25.492 | 11.290 | 1.00 | 41.58 | B |
| ATOM | 3216 | O | ASN | B | 55 | 37.924 | 26.016 | 11.301 | 1.00 | 40.80 | B |
| ATOM | 3217 | N | PHE | B | 56 | 40.169 | 26.185 | 11.197 | 1.00 | 42.04 | B |
| ATOM | 3218 | CA | PHE | B | 56 | 40.150 | 27.635 | 11.126 | 1.00 | 43.31 | B |
| ATOM | 3219 | CB | PHE | B | 56 | 41.532 | 28.219 | 11.361 | 1.00 | 43.87 | B |
| ATOM | 3220 | CG | PHE | B | 56 | 41.549 | 29.711 | 11.335 | 1.00 | 44.28 | B |
| ATOM | 3221 | CD1 | PHE | B | 56 | 41.844 | 30.395 | 10.163 | 1.00 | 44.19 | B |
| ATOM | 3222 | CD2 | PHE | B | 56 | 41.251 | 30.438 | 12.482 | 1.00 | 44.80 | B |
| ATOM | 3223 | CE1 | PHE | B | 56 | 41.843 | 31.782 | 10.135 | 1.00 | 43.99 | B |
| ATOM | 3224 | CE2 | PHE | B | 56 | 41.248 | 31.824 | 12.464 | 1.00 | 43.88 | B |
| ATOM | 3225 | CZ | PHE | B | 56 | 41.544 | 32.498 | 11.288 | 1.00 | 43.49 | B |
| ATOM | 3226 | C | PHE | B | 56 | 39.624 | 28.109 | 9.778 | 1.00 | 43.47 | B |
| ATOM | 3227 | O | PHE | B | 56 | 38.994 | 29.161 | 9.696 | 1.00 | 44.96 | B |
| ATOM | 3228 | N | LEU | B | 57 | 39.941 | 27.375 | 8.714 | 1.00 | 42.27 | B |
| ATOM | 3229 | CA | LEU | B | 57 | 39.442 | 27.720 | 7.388 | 1.00 | 41.52 | B |
| ATOM | 3230 | CB | LEU | B | 57 | 39.902 | 26.690 | 6.355 | 1.00 | 39.96 | B |
| ATOM | 3231 | CG | LEU | B | 57 | 39.352 | 26.797 | 4.929 | 1.00 | 38.23 | B |
| ATOM | 3232 | CD1 | LEU | B | 57 | 39.567 | 28.197 | 4.382 | 1.00 | 39.02 | B |
| ATOM | 3233 | CD2 | LEU | B | 57 | 40.016 | 25.756 | 4.037 | 1.00 | 37.04 | B |
| ATOM | 3234 | C | LEU | B | 57 | 37.917 | 27.713 | 7.482 | 1.00 | 42.18 | B |
| ATOM | 3235 | O | LEU | B | 57 | 37.263 | 28.666 | 7.070 | 1.00 | 42.00 | B |
| ATOM | 3236 | N | THR | B | 58 | 37.373 | 26.646 | 8.072 | 1.00 | 43.26 | B |
| ATOM | 3237 | CA | THR | B | 58 | 35.930 | 26.475 | 8.271 | 1.00 | 43.04 | B |
| ATOM | 3238 | CB | THR | B | 58 | 35.621 | 25.182 | 9.091 | 1.00 | 42.28 | B |
| ATOM | 3239 | OG1 | THR | B | 58 | 36.217 | 24.051 | 8.453 | 1.00 | 41.87 | B |
| ATOM | 3240 | CG2 | THR | B | 58 | 34.125 | 24.937 | 9.197 | 1.00 | 39.06 | B |
| ATOM | 3241 | C | THR | B | 58 | 35.400 | 27.680 | 9.052 | 1.00 | 44.38 | B |
| ATOM | 3242 | O | THR | B | 58 | 34.413 | 28.307 | 8.654 | 1.00 | 43.04 | B |
| ATOM | 3243 | N | LEU | B | 59 | 36.076 | 28.005 | 10.154 | 1.00 | 45.70 | B |
| ATOM | 3244 | CA | LEU | B | 59 | 35.689 | 29.135 | 10.988 | 1.00 | 46.53 | B |
| ATOM | 3245 | CB | LEU | B | 59 | 36.628 | 29.258 | 12.198 | 1.00 | 44.18 | B |
| ATOM | 3246 | CG | LEU | B | 59 | 36.442 | 30.443 | 13.158 | 1.00 | 43.38 | B |
| ATOM | 3247 | CD1 | LEU | B | 59 | 36.651 | 29.981 | 14.582 | 1.00 | 44.81 | B |
| ATOM | 3248 | CD2 | LEU | B | 59 | 37.415 | 31.579 | 12.828 | 1.00 | 43.40 | B |
| ATOM | 3249 | C | LEU | B | 59 | 35.662 | 30.432 | 10.175 | 1.00 | 47.73 | B |
| ATOM | 3250 | O | LEU | B | 59 | 34.690 | 31.173 | 10.213 | 1.00 | 47.64 | B |
| ATOM | 3251 | N | TYR | B | 60 | 36.691 | 30.662 | 9.374 | 1.00 | 49.50 | B |
| ATOM | 3252 | CA | TYR | B | 60 | 36.761 | 31.881 | 8.582 | 1.00 | 52.48 | B |
| ATOM | 3253 | CB | TYR | B | 60 | 38.186 | 32.083 | 8.076 | 1.00 | 53.02 | B |
| ATOM | 3254 | CG | TYR | B | 60 | 38.419 | 33.361 | 7.312 | 1.00 | 53.59 | B |
| ATOM | 3255 | CD1 | TYR | B | 60 | 38.377 | 33.375 | 5.920 | 1.00 | 55.22 | B |
| ATOM | 3256 | CE1 | TYR | B | 60 | 38.672 | 34.528 | 5.197 | 1.00 | 56.80 | B |
| ATOM | 3257 | CD2 | TYR | B | 60 | 38.755 | 34.540 | 7.973 | 1.00 | 54.41 | B |
| ATOM | 3258 | CE2 | TYR | B | 60 | 39.054 | 35.703 | 7.262 | 1.00 | 56.19 | B |
| ATOM | 3259 | C2 | TYR | B | 60 | 39.014 | 35.691 | 5.869 | 1.00 | 57.00 | B |
| ATOM | 3260 | OH | TYR | B | 60 | 39.335 | 36.825 | 5.143 | 1.00 | 56.41 | B |
| ATOM | 3261 | C | TYR | B | 60 | 35.765 | 31.937 | 7.426 | 1.00 | 53.99 | B |
| ATOM | 3262 | O | TYR | B | 60 | 35.383 | 33.018 | 6.993 | 1.00 | 53.88 | B |
| ATOM | 3263 | N | VAL | B | 61 | 35.349 | 30.780 | 6.927 | 1.00 | 56.00 | B |
| ATOM | 3264 | CA | VAL | B | 61 | 34.401 | 30.734 | 5.818 | 1.00 | 58.22 | B |
| ATOM | 3265 | CB | VAL | B | 61 | 34.555 | 29.432 | 4.997 | 1.00 | 57.55 | B |
| ATOM | 3266 | CG1 | VAL | B | 61 | 33.450 | 29.311 | 3.958 | 1.00 | 56.49 | B |
| ATOM | 3267 | CG2 | VAL | B | 61 | 35.916 | 29.421 | 4.313 | 1.00 | 56.91 | B |
| ATOM | 3268 | C | VAL | B | 61 | 32.949 | 30.939 | 6.266 | 1.00 | 60.81 | B |
| ATOM | 3269 | O | VAL | B | 61 | 32.246 | 31.758 | 5.674 | 1.00 | 61.97 | B |
| ATOM | 3270 | N | THR | B | 62 | 32.504 | 30.213 | 7.301 | 1.00 | 62.00 | B |
| ATOM | 3271 | CA | THR | B | 62 | 31.131 | 30.360 | 7.818 | 1.00 | 61.97 | B |
| ATOM | 3272 | CB | THR | B | 62 | 30.773 | 29.303 | 8.919 | 1.00 | 60.52 | B |
| ATOM | 3273 | OG1 | THR | B | 62 | 31.877 | 29.135 | 9.809 | 1.00 | 61.43 | B |
| ATOM | 3274 | CG2 | THR | B | 62 | 30.413 | 27.956 | 8.310 | 1.00 | 58.78 | B |
| ATOM | 3275 | C | THR | B | 62 | 30.940 | 31.771 | 8.384 | 1.00 | 63.24 | B |
| ATOM | 3276 | O | THR | B | 62 | 29.884 | 32.385 | 8.220 | 1.00 | 64.75 | B |
| ATOM | 3277 | N | VAL | B | 63 | 31.972 | 32.285 | 9.044 | 1.00 | 64.63 | B |
| ATOM | 3278 | CA | VAL | B | 63 | 31.926 | 33.628 | 9.599 | 1.00 | 66.86 | B |
| ATOM | 3279 | CB | VAL | B | 63 | 33.138 | 33.890 | 10.547 | 1.00 | 66.16 | B |
| ATOM | 3280 | CG1 | VAL | B | 63 | 33.493 | 35.365 | 10.599 | 1.00 | 65.76 | B |
| ATOM | 3281 | CG2 | VAL | B | 63 | 32.814 | 33.407 | 11.950 | 1.00 | 64.33 | B |
| ATOM | 3282 | C | VAL | B | 63 | 31.907 | 34.625 | 8.440 | 1.00 | 70.13 | B |
| ATOM | 3283 | O | VAL | B | 63 | 31.082 | 35.541 | 8.428 | 1.00 | 70.34 | B |
| ATOM | 3284 | N | GLN | B | 64 | 32.762 | 34.392 | 7.440 | 1.00 | 73.56 | B |
| ATOM | 3285 | CA | GLN | B | 64 | 32.864 | 35.264 | 6.264 | 1.00 | 76.86 | B |
| ATOM | 3286 | CB | GLN | B | 64 | 34.053 | 34.868 | 5.384 | 1.00 | 77.18 | B |
| ATOM | 3287 | CG | GLN | B | 64 | 34.280 | 35.797 | 4.188 | 1.00 | 77.73 | B |
| ATOM | 3288 | CD | GLN | B | 64 | 34.848 | 35.075 | 2.969 | 1.00 | 78.53 | B |
| ATOM | 3289 | OE1 | GLN | B | 64 | 34.193 | 34.205 | 2.383 | 1.00 | 77.80 | B |
| ATOM | 3290 | NE2 | GLN | B | 64 | 36.066 | 35.444 | 2.575 | 1.00 | 78.56 | B |
| ATOM | 3291 | C | GLN | B | 64 | 31.614 | 35.311 | 5.386 | 1.00 | 78.58 | B |
| ATOM | 3292 | O | GLN | B | 64 | 31.166 | 36.395 | 5.002 | 1.00 | 79.72 | B |
| ATOM | 3293 | N | HIS | B | 65 | 31.084 | 34.146 | 5.021 | 1.00 | 80.03 | B |
| ATOM | 3294 | CA | HIS | B | 65 | 29.898 | 34.103 | 4.172 | 1.00 | 81.92 | B |
| ATOM | 3295 | CB | HIS | B | 65 | 29.785 | 32.767 | 3.436 | 1.00 | 81.80 | B |
| ATOM | 3296 | CG | HIS | B | 65 | 30.172 | 32.849 | 1.993 | 1.00 | 81.41 | B |
| ATOM | 3297 | CD2 | HIS | B | 65 | 31.385 | 32.926 | 1.399 | 1.00 | 80.70 | B |
| ATOM | 3298 | ND1 | HIS | B | 65 | 29.243 | 32.910 | 0.976 | 1.00 | 82.27 | B |
| ATOM | 3299 | CE1 | HIS | B | 65 | 29.866 | 33.024 | -0.183 | 1.00 | 81.77 | B |
| ATOM | 3300 | NE2 | HIS | B | 65 | 31.167 | 33.037 | 0.047 | 1.00 | 82.15 | B |
| ATOM | 3301 | C | HIS | B | 65 | 28.597 | 34.468 | 4.876 | 1.00 | 83.27 | B |
| ATOM | 3302 | O | HIS | B | 65 | 28.139 | 33.780 | 5.791 | 1.00 | 83.81 | B |
| ATOM | 3303 | N | LYS | B | 66 | 23.025 | 35.579 | 4.420 | 1.00 | 85.13 | B |
| ATOM | 3304 | CA | LYS | B | 66 | 26.785 | 36.156 | 4.935 | 1.00 | 86.34 | B |
| ATOM | 3305 | CB | LYS | B | 66 | 26.504 | 37.490 | 4.214 | 1.00 | 86.15 | B |
| ATOM | 3306 | CG | LYS | B | 66 | 26.328 | 37.383 | 2.680 | 1.00 | 85.39 | B |
| ATOM | 3307 | CD | LYS | B | 66 | 27.605 | 36.955 | 1.958 | 1.00 | 83.02 | B |
| ATOM | 3308 | CE | LYS | B | 66 | 27.316 | 36.469 | 0.554 | 1.00 | 81.72 | B |
| ATOM | 3309 | NZ | LYS | B | 66 | 28.566 | 36.009 | -0.093 | 1.00 | 80.76 | B |
| ATOM | 3310 | C | LYS | B | 66 | 25.561 | 35.240 | 4.842 | 1.00 | 87.16 | B |
| ATOM | 3311 | O | LYS | B | 66 | 24.678 | 35.292 | 5.704 | 1.00 | 87.08 | B |
| ATOM | 3312 | N | LYS | B | 67 | 25.514 | 34.411 | 3.800 | 1.00 | 88.07 | B |
| ATOM | 3313 | CA | LYS | B | 67 | 24.391 | 33.495 | 3.587 | 1.00 | 88.76 | B |
| ATOM | 3314 | CB | LYS | B | 67 | 24.050 | 33.415 | 2.090 | 1.00 | 90.46 | B |
| ATOM | 3315 | CG | LYS | B | 67 | 23.482 | 34.727 | 1.522 | 1.00 | 92.79 | B |
| ATOM | 3316 | CD | LYS | B | 67 | 23.174 | 34.643 | 0.031 | 1.00 | 94.47 | B |
| ATOM | 3317 | CE | LYS | B | 67 | 22.702 | 35.989 | -0.514 | 1.00 | 95.14 | B |
| ATOM | 3318 | NZ | LYS | B | 67 | 22.522 | 35.973 | -1.996 | 1.00 | 95.36 | B |
| ATOM | 3319 | C | LYS | B | 67 | 24.600 | 32.099 | 4.185 | 1.00 | 87.91 | B |
| ATOM | 3320 | O | LYS | B | 67 | 23.756 | 31.214 | 4.025 | 1.00 | 86.53 | B |
| ATOM | 3321 | N | LEU | B | 68 | 25.708 | 31.932 | 4.910 | 1.00 | 87.76 | B |
| ATOM | 3322 | CA | LEU | B | 68 | 26.064 | 30.663 | 5.558 | 1.00 | 87.06 | B |
| ATOM | 3323 | CB | LEU | B | 68 | 27.549 | 30.354 | 5.311 | 1.00 | 85.82 | B |
| ATOM | 3324 | CG | LEU | B | 68 | 27.973 | 29.138 | 4.474 | 1.00 | 84.62 | B |
| ATOM | 3325 | CD1 | LEU | B | 68 | 27.158 | 29.006 | 3.194 | 1.00 | 83.95 | B |
| ATOM | 3326 | CD2 | LEU | B | 68 | 29.451 | 29.258 | 4.154 | 1.00 | 83.53 | B |
| ATOM | 3327 | C | LEU | B | 68 | 25.762 | 30.702 | 7.068 | 1.00 | 87.14 | B |
| ATOM | 3328 | O | LEU | B | 68 | 26.671 | 30.754 | 7.906 | 1.00 | 86.28 | B |
| ATOM | 3329 | N | ARG | B | 69 | 24.473 | 30.676 | 7.401 | 1.00 | 87.03 | B |
| ATOM | 3330 | CA | ARG | B | 69 | 24.026 | 30.715 | 8.791 | 1.00 | 86.91 | B |
| ATOM | 3331 | CB | ARG | B | 69 | 23.561 | 32.139 | 9.172 | 1.00 | 86.74 | B |
| ATOM | 3332 | CG | ARG | B | 69 | 24.524 | 33.304 | 8.848 | 1.00 | 86.72 | B |
| ATOM | 3333 | CD | ARG | B | 69 | 25.694 | 33.446 | 9.831 | 1.00 | 87.85 | B |
| ATOM | 3334 | NE | ARG | B | 69 | 26.365 | 34.748 | 9.711 | 1.00 | 88.87 | B |
| ATOM | 3335 | CZ | ARG | B | 69 | 25.969 | 35.873 | 10.318 | 1.00 | 89.51 | B |
| ATOM | 3336 | NH1 | ARG | B | 69 | 24.898 | 35.884 | 11.108 | 1.00 | 88.97 | B |
| ATOM | 3337 | NH2 | ARG | B | 69 | 26.633 | 37.007 | 10.118 | 1.00 | 88.89 | B |
| ATOM | 3338 | C | ARG | B | 69 | 22.889 | 29.696 | 9.039 | 1.00 | 86.82 | B |
| ATOM | 3339 | O | ARG | B | 69 | 21.909 | 30.000 | 9.720 | 1.00 | 86.89 | B |
| ATOM | 3340 | N | THR | B | 70 | 23.023 | 28.498 | 8.463 | 1.00 | 86.95 | B |
| ATOM | 3341 | CA | THR | B | 70 | 22.046 | 27.401 | 8.618 | 1.00 | 87.04 | B |
| ATOM | 3342 | CB | THR | B | 70 | 22.248 | 26.323 | 7.488 | 1.00 | 86.57 | B |
| ATOM | 3343 | OG1 | THR | B | 70 | 22.616 | 26.967 | 6.263 | 1.00 | 87.13 | B |
| ATOM | 3344 | CG2 | THR | B | 70 | 20.978 | 25.522 | 7.228 | 1.00 | 86.00 | B |
| ATOM | 3345 | C | THR | B | 70 | 22.339 | 26.748 | 9.990 | 1.00 | 87.47 | B |
| ATOM | 3346 | 0 | THR | B | 70 | 23.394 | 27.011 | 10.582 | 1.00 | 88.34 | B |
| ATOM | 3347 | N | PRO | B | 71 | 21.396 | 25.953 | 10.551 | 1.00 | 87.07 | B |
| ATOM | 3348 | CD | PRO | B | 71 | 19.986 | 25.701 | 10.192 | 1.00 | 86.75 | B |
| ATOM | 3349 | CA | PRO | B | 71 | 21.710 | 25.339 | 11.852 | 1.00 | 85.74 | B |
| ATOM | 3350 | CB | PRO | B | 71 | 20.491 | 24.452 | 12.109 | 1.00 | 85.81 | B |
| ATOM | 3351 | CG | PRO | B | 71 | 19.389 | 25.240 | 11.507 | 1.00 | 86.12 | B |
| ATOM | 3352 | C | PRO | B | 71 | 22.991 | 24.505 | 11.749 | 1.00 | 84.08 | B |
| ATOM | 3353 | O | PRO | B | 71 | 23.868 | 24.581 | 12.617 | 1.00 | 84.74 | B |
| ATOM | 3354 | N | LEU | B | 72 | 23.099 | 23.768 | 10.642 | 1.00 | 81.07 | B |
| ATOM | 3355 | CA | LEU | B | 72 | 24.245 | 22.916 | 10.342 | 1.00 | 77.83 | B |
| ATOM | 3356 | CB | LEU | B | 72 | 24.081 | 22.321 | 8.941 | 1.00 | 77.81 | B |
| ATOM | 3357 | CG | LEU | B | 72 | 24.849 | 21.057 | 8.548 | 1.00 | 77.32 | B |
| ATOM | 3358 | CD1 | LEU | B | 72 | 24.131 | 19.828 | 9.088 | 1.00 | 78.03 | B |
| ATOM | 3359 | CD2 | LEU | B | 72 | 24.934 | 20.970 | 7.038 | 1.00 | 76.55 | B |
| ATOM | 3360 | C | LEU | B | 72 | 25.527 | 23.746 | 10.399 | 1.00 | 75.67 | B |
| ATOM | 3361 | O | LEU | B | 72 | 26.525 | 23.318 | 10.975 | 1.00 | 76.00 | B |
| ATOM | 3362 | N | ASN | B | 73 | 25.468 | 24.953 | 9.840 | 1.00 | 72.68 | B |
| ATOM | 3363 | CA | ASN | B | 73 | 26.607 | 25.870 | 9.808 | 1.00 | 69.98 | B |
| ATOM | 3364 | CB | ASN | B | 73 | 26.265 | 27.142 | 9.025 | 1.00 | 71.09 | B |
| ATOM | 3365 | CG | ASN | B | 73 | 25.747 | 26.858 | 7.621 | 1.00 | 72.01 | B |
| ATOM | 3366 | OD1 | ASN | B | 73 | 25.628 | 27.767 | 6.800 | 1.00 | 71.65 | B |
| ATOM | 3367 | ND2 | ASN | B | 73 | 25.409 | 25.602 | 7.349 | 1.00 | 72.97 | B |
| ATOM | 3368 | C | ASN | B | 73 | 27.025 | 26.269 | 11.209 | 1.00 | 67.98 | B |
| ATOM | 3369 | O | ASN | B | 73 | 28.215 | 26.373 | 11.499 | 1.00 | 68.72 | B |
| ATOM | 3370 | N | TYR | B | 74 | 26.041 | 26.524 | 12.066 | 1.00 | 65.42 | B |
| ATOM | 3371 | CA | TYR | B | 74 | 26.313 | 26.912 | 13.441 | 1.00 | 63.38 | B |
| ATOM | 3372 | CB | TYR | B | 74 | 25.025 | 27.296 | 14.157 | 1.00 | 63.75 | B |
| ATOM | 3373 | CG | TYR | B | 74 | 24.668 | 28.750 | 13.976 | 1.00 | 65.64 | B |
| ATOM | 3374 | CD1 | TYR | B | 74 | 25.237 | 29.733 | 14.789 | 1.00 | 65.10 | B |
| ATOM | 3375 | CE1 | TYR | B | 74 | 24.929 | 31.084 | 14.609 | 1.00 | 65.09 | B |
| ATOM | 3376 | CD2 | TYR | B | 74 | 23.778 | 29.153 | 12.977 | 1.00 | 66.04 | B |
| ATOM | 3377 | CE2 | TYR | B | 74 | 23.465 | 30.504 | 12.790 | 1.00 | 65.30 | B |
| ATOM | 3378 | CZ | TYR | B | 74 | 24.044 | 31.460 | 13.608 | 1.00 | 64.62 | B |
| ATOM | 3379 | OH | TYR | B | 74 | 23.736 | 32.786 | 13.422 | 1.00 | 64.45 | B |
| ATOM | 3380 | C | TYR | B | 74 | 27.005 | 25.794 | 14.188 | 1.00 | 61.41 | B |
| ATOM | 3381 | O | TYR | B | 74 | 27.963 | 26.019 | 14.928 | 1.00 | 62.22 | B |
| ATOM | 3382 | N | ILE | B | 75 | 26.524 | 24.579 | 13.975 | 1.00 | 57.72 | B |
| ATOM | 3383 | CA | ILE | B | 75 | 27.103 | 23.432 | 14.628 | 1.00 | 54.65 | B |
| ATOM | 3384 | CB | ILE | B | 75 | 26.154 | 22.228 | 14.547 | 1.00 | 55.46 | B |
| ATOM | 3385 | CG2 | ILE | B | 75 | 296.926 | 20.913 | 14.556 | 1.00 | 56.19 | B |
| ATOM | 3386 | CG1 | ILE | B | 75 | 25.172 | 22.319 | 15.718 | 1.00 | 56.55 | B |
| ATOM | 3387 | CD1 | ILE | B | 75 | 24.104 | 21.288 | 15.714 | 1.00 | 57.78 | B |
| ATOM | 3388 | C | ILE | B | 75 | 28.478 | 23.135 | 14.066 | 1.00 | 51.79 | B |
| ATOM | 3389 | O | ILE | B | 75 | 29.416 | 22.878 | 14.814 | 1.00 | 51.27 | B |
| ATOM | 3390 | N | LEU | B | 76 | 28.613 | 23.231 | 12.751 | 1.00 | 48.94 | B |
| ATOM | 3391 | CA | LEU | B | 76 | 29.896 | 22.976 | 12.124 | 1.00 | 46.79 | B |
| ATOM | 3392 | CB | LEU | B | 76 | 29.747 | 22.798 | 10.616 | 1.00 | 45.52 | B |
| ATOM | 3393 | CG | LEU | B | 76 | 29.599 | 21.352 | 10.112 | 1.00 | 44.53 | B |
| ATOM | 3394 | CD1 | LEU | B | 76 | 30.867 | 20.576 | 10.413 | 1.00 | 44.00 | B |
| ATOM | 3395 | CD2 | LEU | B | 76 | 28.385 | 20.659 | 10.718 | 1.00 | 43.41 | B |
| ATOM | 3396 | C | LEU | B | 76 | 30.910 | 24.060 | 12.464 | 1.00 | 45.93 | B |
| ATOM | 3397 | O | LEU | B | 76 | 32.116 | 23.820 | 12.434 | 1.00 | 46.22 | B |
| ATOM | 3398 | N | LEU | B | 77 | 30.429 | 25.257 | 12.779 | 1.00 | 45.06 | B |
| ATOM | 3399 | CA | LEU | B | 77 | 31.336 | 26.322 | 13.167 | 1.00 | 44.52 | B |
| ATOM | 3400 | CB | LEU | B | 77 | 30.625 | 27.672 | 13.221 | 1.00 | 43.71 | B |
| ATOM | 3401 | CG | LEU | B | 77 | 31.444 | 28.766 | 13.920 | 1.00 | 44.62 | B |
| ATOM | 3402 | CD1 | LEU | B | 77 | 32.735 | 29.027 | 13.172 | 1.00 | 41.87 | B |
| ATOM | 3403 | CD2 | LEU | B | 77 | 30.633 | 30.039 | 14.050 | 1.00 | 45.59 | B |
| ATOM | 3404 | C | LEU | B | 77 | 31.801 | 25.941 | 14.561 | 1.00 | 45.84 | B |
| ATOM | 3405 | O | LEU | B | 77 | 32.986 | 26.041 | 14.876 | 1.00 | 47.84 | B |
| ATOM | 3406 | N | ASN | B | 78 | 30.856 | 25.458 | 15.370 | 1.00 | 45.27 | B |
| ATOM | 3407 | CA | ASN | B | 78 | 31.104 | 25.038 | 16.747 | 1.00 | 42.99 | B |
| ATOM | 3408 | CB | ASN | B | 78 | 29.831 | 24.414 | 17.326 | 1.00 | 41.67 | B |
| ATOM | 3409 | CG | ASN | B | 78 | 29.792 | 24.451 | 18.836 | 1.00 | 41.58 | B |
| ATOM | 3410 | OD1 | ASH | B | 78 | 30.427 | 25.296 | 19.463 | 1.00 | 39.86 | B |
| ATOM | 3411 | ND2 | ASN | B | 78 | 29.025 | 23.541 | 19.432 | 1.00 | 41.05 | B |
| ATOM | 3412 | C | ASN | B | 78 | 32.243 | 24.024 | 16.770 | 1.00 | 42.61 | B |
| ATOM | 3413 | O | ASN | B | 78 | 33.264 | 24.241 | 17.417 | 1.00 | 41.64 | B |
| ATOM | 3414 | N | LEU | B | 79 | 32.071 | 22.943 | 16.017 | 1.00 | 42.92 | B |
| ATOM | 3415 | CA | LEU | B | 79 | 33.065 | 21.878 | 15.911 | 1.00 | 43.77 | B |
| ATOM | 3416 | CB | LEU | B | 79 | 32.584 | 20.820 | 14.920 | 1.00 | 44.31 | B |
| ATOM | 3417 | CG | LEU | B | 79 | 31.327 | 20.103 | 15.412 | 1.00 | 46.33 | B |
| ATOM | 3418 | CD1 | LEU | B | 79 | 30.706 | 19.248 | 14.314 | 1.00 | 46.81 | B |
| ATOM | 3419 | CD2 | LEU | B | 79 | 31.682 | 19.263 | 16.641 | 1.00 | 47.27 | B |
| ATOM | 3420 | C | LEU | B | 79 | 34.432 | 22.411 | 15.490 | 1.00 | 43.75 | B |
| ATOM | 3421 | O | LEU | B | 79 | 35.461 | 21.954 | 15.987 | 1.00 | 44.55 | B |
| ATOM | 3422 | N | ALA | B | 80 | 34.431 | 23.379 | 14.575 | 1.00 | 41.97 | B |
| ATOM | 3423 | CA | ALA | B | 80 | 35.660 | 24.003 | 14.106 | 1.00 | 39.52 | B |
| ATOM | 3424 | CB | ALA | B | 80 | 35.355 | 25.003 | 12.999 | 1.00 | 38.49 | B |
| ATOM | 3425 | C | ALA | B | 80 | 36.312 | 24.714 | 15.285 | 1.00 | 39.01 | B |
| ATOM | 3426 | O | ALA | B | 80 | 37.534 | 24.715 | 15.420 | 1.00 | 39.68 | B |
| ATOM | 3427 | N | VAL | B | 81 | 35.484 | 25.282 | 16.159 | 1.00 | 38.43 | B |
| ATOM | 3428 | CA | VAL | B | 81 | 35.973 | 26.002 | 17.330 | 1.00 | 37.96 | B |
| ATOM | 3429 | CB | VAL | B | 81 | 34.908 | 26.958 | 17.894 | 1.00 | 37.26 | B |
| ATOM | 3430 | CG1 | VAL | B | 81 | 35.529 | 27.883 | 18.925 | 1.00 | 38.55 | B |
| ATOM | 3431 | CG2 | VAL | B | 81 | 34.287 | 27.770 | 16.785 | 1.00 | 36.06 | B |
| ATOM | 3432 | C | VAL | B | 81 | 36.442 | 25.055 | 18.430 | 1.00 | 37.99 | B |
| ATOM | 3433 | O | VAL | B | 81 | 37.423 | 25.343 | 19.113 | 1.00 | 41.24 | B |
| ATOM | 3434 | N | ALA | B | 82 | 35.734 | 23.941 | 18.609 | 1.00 | 36.78 | B |
| ATOM | 3435 | CA | ALA | B | 82 | 36.094 | 22.941 | 19.615 | 1.00 | 35.82 | B |
| ATOM | 3436 | CB | ALA | B | 82 | 34.963 | 21.956 | 19.804 | 1.00 | 33.65 | B |
| ATOM | 3437 | C | ALA | B | 82 | 37.354 | 22.212 | 19.154 | 1.00 | 36.60 | B |
| ATOM | 3438 | O | ALA | B | 62 | 38.198 | 21.815 | 19.976 | 1.00 | 34.42 | B |
| ATOM | 3439 | N | ASP | B | 83 | 37.448 | 22.028 | 17.832 | 1.00 | 37.15 | B |
| ATOM | 3440 | CA | ASP | B | 83 | 38.585 | 21.373 | 17.187 | 1.00 | 38.25 | B |
| ATOM | 3441 | CB | ASP | B | 83 | 38.284 | 21.076 | 15.712 | 1.00 | 38.37 | B |
| ATOM | 3442 | CG | ASP | B | 83 | 37.458 | 19.800 | 15.504 | 1.00 | 42.19 | B |
| ATOM | 3443 | OD1 | ASP | B | 83 | 36.956 | 19.225 | 16.496 | 1.00 | 43.24 | B |
| ATOM | 3444 | OD2 | ASP | B | 83 | 37.307 | 19.364 | 14.334 | 1.00 | 41.93 | B |
| ATOM | 3445 | C | ASP | B | 83 | 39.832 | 22.257 | 17.296 | 1.00 | 38.97 | B |
| ATOM | 3446 | O | ASP | B | 83 | 40.953 | 21.751 | 17.398 | 1.00 | 39.62 | B |
| ATOM | 3447 | N | LEU | B | 84 | 39.645 | 23.575 | 17.251 | 1.00 | 37.82 | B |
| ATOM | 3448 | CA | LEU | B | 84 | 40.774 | 24.481 | 17.385 | 1.00 | 36.67 | B |
| ATOM | 3449 | CB | LEU | B | 84 | 40.399 | 25.913 | 16.997 | 1.00 | 34.69 | B |
| ATOM | 3450 | CG | LEU | B | 84 | 40.415 | 26.120 | 15.477 | 1.00 | 33.30 | B |
| ATOM | 3451 | CD1 | LEU | B | 84 | 39.897 | 27.492 | 15.096 | 1.00 | 31.12 | B |
| ATOM | 3452 | CD2 | LEU | B | 84 | 41.824 | 25.911 | 14.949 | 1.00 | 30.99 | B |
| ATOM | 3453 | C | LEU | B | 84 | 41.262 | 24.392 | 18.821 | 1.00 | 37.98 | B |
| ATOM | 3454 | O | LEU | B | 84 | 42.460 | 24.278 | 19.061 | 1.00 | 38.48 | B |
| ATOM | 3455 | N | PHE | B | 85 | 40.329 | 24.388 | 19.771 | 1.00 | 38.14 | B |
| ATOM | 3456 | CA | PHE | B | 85 | 40.678 | 24.264 | 21.186 | 1.00 | 38.83 | B |
| ATOM | 3457 | CB | PHE | B | 85 | 39.429 | 24.318 | 22.052 | 1.00 | 39.38 | B |
| ATOM | 3458 | CG | PHE | B | 85 | 39.059 | 25.693 | 22.472 | 1.00 | 41.28 | B |
| ATOM | 3459 | CD1 | PHE | B | 85 | 37.759 | 26.152 | 22.319 | 1.00 | 41.19 | B |
| ATOM | 3460 | CD2 | PHE | B | 85 | 40.014 | 26.533 | 23.039 | 1.00 | 41.59 | B |
| ATOM | 3461 | CE1 | PHE | B | 85 | 37.412 | 27.429 | 22.727 | 1.00 | 42.28 | B |
| ATOM | 3462 | CE2 | PHE | B | 85 | 39.680 | 27.811 | 23.451 | 1.00 | 42.23 | B |
| ATOM | 3463 | CZ | PHE | B | 85 | 38.374 | 28.263 | 23.297 | 1.00 | 42.17 | B |
| ATOM | 3464 | C | PHE | B | 85 | 41.387 | 22.941 | 21.438 | 1.00 | 38.84 | B |
| ATOM | 3465 | O | PHE | B | 85 | 42.433 | 22.888 | 22.090 | 1.00 | 39.09 | B |
| ATOM | 3466 | N | MET | B | 86 | 40.789 | 21.874 | 20.917 | 1.00 | 37.28 | B |
| ATOM | 3467 | CA | MET | B | 86 | 41.319 | 20.528 | 21.035 | 1.00 | 33.45 | B |
| ATOM | 3468 | CB | MET | B | 86 | 40.480 | 19.614 | 20.160 | 1.00 | 33.21 | B |
| ATOM | 3469 | CG | MET | B | 86 | 40.600 | 18.147 | 20.454 | 1.00 | 33.98 | B |
| ATOM | 3470 | SD | MET | B | 86 | 39.404 | 17.221 | 19.478 | 1.00 | 31.86 | B |
| ATOM | 3471 | CE | MET | B | 86 | 38.114 | 18.431 | 19.364 | 1.00 | 35.60 | B |
| ATOM | 3472 | C | MET | B | 86 | 42.769 | 20.517 | 20.565 | 1.00 | 31.96 | B |
| ATOM | 3473 | O | MET | B | 86 | 43.649 | 20.018 | 21.254 | 1.00 | 30.77 | B |
| ATOM | 3474 | N | VAL | B | 87 | 43.012 | 21.124 | 19.409 | 1.00 | 31.41 | B |
| ATOM | 3475 | CA | VAL | B | 87 | 44.352 | 21.202 | 18.826 | 1.00 | 33.20 | B |
| ATOM | 3476 | CB | VAL | B | 87 | 44.268 | 21.594 | 17.310 | 1.00 | 33.61 | B |
| ATOM | 3477 | CG1 | VAL | B | 87 | 45.634 | 21.980 | 16.769 | 1.00 | 33.21 | B |
| ATOM | 3478 | CG2 | VAL | B | 87 | 43.690 | 20.444 | 16.492 | 1.00 | 32.78 | B |
| ATOM | 3479 | C | VAL | B | 87 | 55.351 | 22.128 | 19.573 | 1.00 | 34.64 | B |
| ATOM | 3480 | O | VAL | B | 87 | 46.518 | 21.765 | 19.764 | 1.00 | 33.67 | B |
| ATOM | 3481 | N | PHE | B | 88 | 44.903 | 23.317 | 19.987 | 1.00 | 35.55 | B |
| ATOM | 3482 | CA | PHE | B | 88 | 45.774 | 24.269 | 20.693 | 1.00 | 34.44 | B |
| ATOM | 3483 | CB | PHE | B | 88 | 45.318 | 25.704 | 20.448 | 1.00 | 33.91 | B |
| ATOM | 3484 | CG | PHE | B | 88 | 45.674 | 26.206 | 19.093 | 1.00 | 33.91 | B |
| ATOM | 3485 | CD1 | PHE | B | 88 | 44.719 | 26.282 | 18.095 | 1.00 | 32.65 | B |
| ATOM | 3486 | CD2 | PHE | B | 88 | 46.999 | 26.525 | 18.787 | 1.00 | 33.89 | B |
| ATOM | 3487 | CE1 | PHE | B | 88 | 45.081 | 26.658 | 16.814 | 1.00 | 33.49 | B |
| ATOM | 3488 | CE2 | PHE | B | 88 | 47.370 | 26.901 | 17.506 | 1.00 | 29.84 | B |
| ATOM | 3489 | CZ | PHE | B | 88 | 46.418 | 26.967 | 16.520 | 1.00 | 31.73 | B |
| ATOM | 3490 | C | PHE | B | 88 | 45.922 | 24.029 | 22.179 | 1.00 | 34.62 | B |
| ATOM | 3491 | O | PHE | B | 88 | 46.995 | 24.239 | 22.747 | 1.00 | 32.98 | B |
| ATOM | 3492 | N | GLY | B | 89 | 44.830 | 23.616 | 22.806 | 1.00 | 34.86 | B |
| ATOM | 3493 | CA | GLY | B | 89 | 44.862 | 23.353 | 24.224 | 1.00 | 38.01 | B |
| ATOM | 3494 | C | GLY | B | 89 | 45.395 | 21.966 | 24.500 | 1.00 | 39.85 | B |
| ATOM | 3495 | O | GLY | B | 89 | 46.324 | 21.800 | 25.296 | 1.00 | 39.88 | B |
| ATOM | 3496 | N | GLY | B | 90 | 44.841 | 20.985 | 23.790 | 1.00 | 41.36 | B |
| ATOM | 3497 | CA | GLY | B | 90 | 45.233 | 19.595 | 23.973 | 1.00 | 42.56 | B |
| ATOM | 3498 | C | GLY | B | 90 | 46.430 | 19.081 | 23.192 | 1.00 | 43.20 | B |
| ATOM | 3499 | O | GLY | B | 90 | 47.421 | 18.667 | 23.798 | 1.00 | 43.09 | B |
| ATOM | 3500 | N | PHE | B | 91 | 46.347 | 19.111 | 21.860 | 1.00 | 43.31 | B |
| ATOM | 3501 | CA | PHE | B | 91 | 47.424 | 18.619 | 20.986 | 1.00 | 42.80 | B |
| ATOM | 3502 | CB | PHE | B | 91 | 47.016 | 18.695 | 19.512 | 1.00 | 41.13 | B |
| ATOM | 3503 | CG | PHE | B | 91 | 45.850 | 17.822 | 19.150 | 1.00 | 39.60 | B |
| ATOM | 3504 | CD1 | PHE | B | 91 | 45.486 | 17.662 | 17.821 | 1.00 | 39.83 | B |
| ATOM | 3505 | CD2 | PHE | B | 91 | 45.099 | 17.186 | 20.128 | 1.00 | 38.80 | B |
| ATOM | 3506 | CB1 | PHE | B | 91 | 44.390 | 16.884 | 17.473 | 1.00 | 40.71 | B |
| ATOM | 3507 | CE2 | PHE | B | 91 | 44.004 | 16.409 | 19.790 | 1.00 | 39.94 | B |
| ATOM | 3508 | CZ | PHE | B | 91 | 43.647 | 16.256 | 18.461 | 1.00 | 40.14 | B |
| ATOM | 3509 | C | PHE | B | 91 | 48.802 | 19.266 | 21.170 | 1.00 | 43.97 | B |
| ATOM | 3510 | O | PHE | B | 91 | 49.815 | 18.709 | 20.753 | 1.00 | 44.06 | B |
| ATOM | 3511 | N | THR | B | 92 | 48.837 | 20.452 | 21.765 | 1.00 | 45.57 | B |
| ATOM | 3512 | CA | THR | B | 92 | 50.093 | 21.149 | 22.014 | 1.00 | 46.56 | B |
| ATOM | 3513 | CB | THR | B | 92 | 49.819 | 22.575 | 22.576 | 1.00 | 48.44 | B |
| ATOM | 3514 | OG1 | THR | B | 92 | 49.016 | 23.312 | 21.645 | 1.00 | 49.91 | B |
| ATOM | 3515 | CG2 | THR | B | 92 | 51.120 | 23.330 | 22.818 | 1.00 | 50.68 | B |
| ATOM | 3516 | C | THR | B | 92 | 50.894 | 20.330 | 23.040 | 1.00 | 46.47 | B |
| ATOM | 3517 | O | THR | B | 92 | 52.087 | 20.066 | 22.849 | 1.00 | 46.48 | B |
| ATOM | 3518 | N | THR | B | 93 | 50.199 | 19.902 | 24.098 | 1.00 | 45.57 | B |
| ATOM | 3519 | CA | THR | B | 93 | 50.767 | 19.115 | 25.192 | 1.00 | 43.57 | B |
| ATOM | 3520 | CB | THR | B | 93 | 49.779 | 19.033 | 26.369 | 1.00 | 43.67 | B |
| ATOM | 3521 | OG1 | THR | B | 93 | 49.467 | 20.356 | 26.823 | 1.00 | 43.26 | B |
| ATOM | 3522 | CG2 | THR | B | 93 | 50.363 | 18.218 | 27.514 | 1.00 | 42.99 | B |
| ATOM | 3523 | C | THR | B | 93 | 51.125 | 17.697 | 24.769 | 1.00 | 43.06 | B |
| ATOM | 3524 | O | THR | B | 93 | 52.250 | 17.247 | 24.980 | 1.00 | 41.71 | B |
| ATOM | 3525 | N | THR | B | 94 | 50.163 | 16.995 | 24.178 | 1.00 | 42.85 | B |
| ATOM | 3526 | CA | THR | B | 94 | 50.387 | 15.623 | 23.729 | 1.00 | 44.60 | B |
| ATOM | 3527 | CB | THR | B | 94 | 49.069 | 14.947 | 23.210 | 1.00 | 43.94 | B |
| ATOM | 3528 | OG1 | THR | B | 94 | 49.371 | 13.826 | 22.371 | 1.00 | 43.33 | B |
| ATOM | 3529 | CG2 | THR | B | 94 | 48.216 | 15.916 | 22.467 | 1.00 | 44.68 | B |
| ATOM | 3530 | C | THR | B | 94 | 51.549 | 15.497 | 22.735 | 1.00 | 45.24 | B |
| ATOM | 3531 | O | THR | B | 94 | 52.242 | 14.478 | 22.713 | 1.00 | 46.70 | B |
| ATOM | 3532 | N | LEU | B | 95 | 51.797 | 16.552 | 21.961 | 1.00 | 45.71 | B |
| ATOM | 3533 | CA | LEU | B | 95 | 52.907 | 16.566 | 21.005 | 1.00 | 44.84 | B |
| ATOM | 3534 | CB | LEU | B | 95 | 52.877 | 17.834 | 20.146 | 1.00 | 44.93 | B |
| ATOM | 3535 | CG | LEU | B | 95 | 54.191 | 18.167 | 19.428 | 1.00 | 43.41 | B |
| ATOM | 3536 | CD1 | LEU | B | 95 | 54.520 | 17.085 | 18.407 | 1.00 | 44.93 | B |
| ATOM | 3537 | CD2 | LEU | B | 95 | 54.094 | 19.522 | 18.772 | 1.00 | 41.59 | B |
| ATOM | 3538 | C | LEU | B | 95 | 54.221 | 16.542 | 21.766 | 1.00 | 44.83 | B |
| ATOM | 3539 | O | LEU | B | 95 | 55.080 | 15.703 | 21.501 | 1.00 | 44.42 | B |
| ATOM | 3540 | N | TYR | B | 96 | 54.360 | 17.481 | 22.704 | 1.00 | 45.66 | B |
| ATOM | 3541 | CA | TYR | B | 96 | 55.563 | 17.615 | 23.512 | 1.00 | 45.79 | B |
| ATOM | 3542 | CB | TYR | B | 96 | 55.646 | 19.015 | 24.138 | 1.00 | 47.61 | B |
| ATOM | 3543 | CG | TYR | B | 96 | 56.550 | 19.964 | 23.360 | 1.00 | 51.64 | B |
| ATOM | 3544 | CD1 | TYR | B | 96 | 57.584 | 20.661 | 24.001 | 1.00 | 54.86 | B |
| ATOM | 3545 | CE1 | TYR | B | 96 | 58.468 | 21.481 | 23.275 | 1.00 | 56.97 | B |
| ATOM | 3546 | CD2 | TYR | B | 96 | 56.414 | 20.117 | 21.973 | 1.00 | 51.89 | B |
| ATOM | 3547 | CE2 | TYR | B | 96 | 57.288 | 20.930 | 21.238 | 1.00 | 53.88 | B |
| ATOM | 3548 | CZ | TYR | B | 96 | 58.313 | 21.605 | 21.893 | 1.00 | 57.06 | B |
| ATOM | 3549 | OH | TYR | B | 96 | 59.201 | 22.374 | 21.172 | 1.00 | 58.82 | B |
| ATOM | 3550 | C | TYR | B | 96 | 55.767 | 16.513 | 24.548 | 1.00 | 44.75 | B |
| ATOM | 3551 | O | TYR | B | 96 | 56.903 | 16.225 | 24.933 | 1.00 | 45.37 | B |
| ATOM | 3552 | N | THR | B | 97 | 54.680 | 15.866 | 24.961 | 1.00 | 42.84 | B |
| ATOM | 3553 | CA | THR | B | 97 | 54.757 | 14.774 | 25.933 | 1.00 | 41.44 | B |
| ATOM | 3554 | CB | THR | B | 97 | 53.346 | 14.371 | 26.403 | 1.00 | 41.57 | B |
| ATOM | 3555 | OG1 | THR | B | 97 | 52.791 | 15.428 | 27.194 | 1.00 | 40.43 | B |
| ATOM | 3556 | CG2 | THR | B | 97 | 53.380 | 13.093 | 27.218 | 1.00 | 41.81 | B |
| ATOM | 3557 | C | THR | B | 97 | 55.466 | 13.569 | 25.299 | 1.00 | 41.35 | B |
| ATOM | 3558 | O | THR | B | 97 | 56.361 | 12.965 | 25.905 | 1.00 | 39.85 | B |
| ATOM | 3559 | N | SER | B | 98 | 55.060 | 13.256 | 24.066 | 1.00 | 41.41 | B |
| ATOM | 3560 | CA | SER | B | 98 | 55.614 | 12.158 | 23.275 | 1.00 | 40.89 | B |
| ATOM | 3561 | CB | SER | B | 98 | 54.837 | 12.008 | 21.954 | 1.00 | 40.98 | B |
| ATOM | 3562 | OG | SER | B | 98 | 53.510 | 11.545 | 22.159 | 1.00 | 38.11 | B |
| ATOM | 3563 | C | SER | B | 98 | 51.086 | 12.433 | 22.964 | 1.00 | 41.28 | B |
| ATOM | 3564 | O | SER | B | 98 | 57.923 | 11.516 | 22.958 | 1.00 | 41.36 | B |
| ATOM | 3565 | N | LEU | B | 99 | 57.395 | 13.704 | 22.703 | 1.00 | 40.38 | B |
| ATOM | 3566 | CA | LEU | B | 99 | 58.761 | 14.105 | 22.397 | 1.00 | 39.33 | B |
| ATOM | 3567 | CB | LEU | B | 99 | 58.819 | 15.567 | 21.922 | 1.00 | 35.08 | B |
| ATOM | 3568 | CG | LEU | B | 99 | 58.410 | 15.830 | 20.462 | 1.00 | 32.17 | B |
| ATOM | 3569 | CD1 | LEU | B | 99 | 58.381 | 17.308 | 20.191 | 1.00 | 29.93 | B |
| ATOM | 3570 | CD2 | LEU | B | 99 | 59.360 | 15.149 | 19.493 | 1.00 | 30.63 | B |
| ATOM | 3571 | C | LEU | B | 99 | 59.706 | 13.855 | 23.573 | 1.00 | 40.07 | B |
| ATOM | 3572 | O | LEU | B | 99 | 60.883 | 13.555 | 23.368 | 1.00 | 40.86 | B |
| ATOM | 3573 | N | HIS | B | 100 | 59.175 | 13.916 | 24.794 | 1.00 | 41.40 | B |
| ATOM | 3574 | CA | HIS | B | 100 | 59.980 | 13.686 | 25.991 | 1.00 | 41.65 | B |
| ATOM | 3575 | CB | HIS | B | 100 | 59.728 | 14.772 | 27.033 | 1.00 | 44.08 | B |
| ATOM | 3576 | CG | HIS | B | 100 | 60.236 | 16.121 | 26.626 | 1.00 | 47.99 | B |
| ATOM | 3577 | CD2 | HIS | B | 100 | 59.630 | 17.146 | 25.980 | 1.00 | 48.90 | B |
| ATOM | 3578 | ND1 | HIS | B | 100 | 61.534 | 16.526 | 26.856 | 1.00 | 51.04 | B |
| ATOM | 3579 | CE1 | HIS | B | 100 | 61.706 | 17.743 | 26.366 | 1.00 | 52.64 | B |
| ATOM | 3580 | NE2 | HIS | B | 100 | 60.566 | 18.141 | 25.829 | 1.00 | 51.72 | B |
| ATOM | 3581 | C | HIS | B | 100 | 59.750 | 12.311 | 26.585 | 1.00 | 40.40 | B |
| ATOM | 3582 | O | HIS | B | 100 | 60.469 | 11.900 | 27.493 | 1.00 | 40.79 | B |
| ATOM | 3583 | N | GLY | B | 100 | 58.761 | 11.601 | 26.049 | 1.00 | 39.01 | B |
| ATOM | 3584 | CA | GLY | B | 100 | 58.448 | 10.264 | 26.518 | 1.00 | 38.24 | B |
| ATOM | 3585 | C | GLY | B | 100 | 57.658 | 10.187 | 27.811 | 1.00 | 38.92 | B |
| ATOM | 3586 | O | GLY | B | 101 | 57.487 | 9.102 | 28.366 | 1.00 | 38.35 | B |
| ATOM | 3587 | N | TYR | B | 102 | 57.176 | 11.332 | 28.292 | 1.00 | 39.45 | B |
| ATOM | 3588 | CA | TYR | B | 102 | 56.400 | 11.403 | 29.530 | 1.00 | 39.23 | B |
| ATOM | 3589 | CB | TYR | B | 102 | 57.279 | 11.072 | 30.742 | 1.00 | 40.66 | B |
| ATOM | 3590 | CG | TYR | B | 102 | 58.223 | 12.187 | 31.158 | 1.00 | 43.33 | B |
| ATOM | 3591 | CD1 | TYR | B | 102 | 59.399 | 12.447 | 30.446 | 1.00 | 43.99 | B |
| ATOM | 3592 | CE1 | TYR | B | 102 | 60.270 | 13.476 | 30.832 | 1.00 | 44.34 | B |
| ATOM | 3593 | CD2 | TYR | B | 102 | 57.937 | 12.988 | 32.266 | 1.00 | 44.67 | B |
| ATOM | 3594 | CE2 | TYR | B | 102 | 58.795 | 14.020 | 32.657 | 1.00 | 45.20 | B |
| ATOM | 3595 | CZ | TYR | B | 102 | 59.958 | 14.257 | 31.940 | 1.00 | 44.63 | B |
| ATOM | 3596 | OH | TYR | B | 102 | 60.801 | 15.268 | 32.338 | 1.00 | 44.32 | B |
| ATOM | 3597 | C | TYR | B | 102 | 55.865 | 12.819 | 29.684 | 1.00 | 39.26 | B |
| ATOM | 3598 | O | TYR | B | 102 | 56.196 | 13.698 | 28.888 | 1.00 | 38.43 | B |
| ATOM | 3599 | N | PHE | B | 103 | 55.077 | 13.039 | 30.735 | 1.00 | 39.45 | B |
| ATOM | 3600 | CA | PHE | B | 103 | 54.502 | 14.346 | 31.014 | 1.00 | 40.04 | B |
| ATOM | 3601 | CB | PHE | B | 103 | 53.212 | 14.215 | 31.817 | 1.00 | 36.19 | B |
| ATOM | 3602 | CG | PHE | B | 103 | 51.997 | 14.048 | 30.963 | 1.00 | 36.86 | B |
| ATOM | 3603 | CD1 | PHE | B | 103 | 51.498 | 12.786 | 30.674 | 1.00 | 35.01 | B |
| ATOM | 3604 | CD2 | PHE | B | 103 | 51.381 | 15.160 | 30.383 | 1.00 | 37.62 | B |
| ATOM | 3605 | CB1 | PHE | B | 103 | 50.411 | 12.633 | 29.817 | 1.00 | 35.13 | B |
| ATOM | 3606 | CE2 | PHE | B | 103 | 50.287 | 15.014 | 29.518 | 1.00 | 35.82 | B |
| ATOM | 3607 | CZ | PHE | B | 103 | 49.805 | 13.753 | 29.235 | 1.00 | 33.98 | B |
| ATOM | 3608 | C | PHE | B | 103 | 55.459 | 15.306 | 31.701 | 1.00 | 43.64 | B |
| ATOM | 3609 | O | PHE | B | 103 | 55.536 | 15.356 | 32.929 | 1.00 | 44.28 | B |
| ATOM | 3610 | N | VAL | B | 104 | 56.200 | 16.057 | 30.887 | 1.00 | 47.92 | B |
| ATOM | 3611 | CA | VAL | B | 104 | 57.157 | 17.054 | 31.372 | 1.00 | 50.24 | B |
| ATOM | 3612 | CB | VAL | B | 104 | 58.077 | 17.594 | 30.238 | 1.00 | 48.67 | B |
| ATOM | 3613 | CG1 | VAL | B | 104 | 59.050 | 16.539 | 29.807 | 1.00 | 48.46 | B |
| ATOM | 3614 | CG2 | VAL | B | 104 | 57.252 | 18.068 | 29.041 | 1.00 | 46.06 | B |
| ATOM | 3615 | C | VAL | B | 104 | 56.427 | 18.249 | 31.969 | 1.00 | 52.88 | B |
| ATOM | 3616 | O | VAL | B | 104 | 56.982 | 18.960 | 32.809 | 1.00 | 54.89 | B |
| ATOM | 3617 | N | PHE | B | 105 | 55.192 | 18.475 | 31.515 | 1.00 | 54.60 | B |
| ATOM | 3618 | CA | PHE | B | 105 | 54.382 | 19.597 | 31.987 | 1.00 | 56.79 | B |
| ATOM | 3619 | CB | PHE | B | 105 | 53.404 | 20.068 | 30.887 | 1.00 | 60.01 | B |
| ATOM | 3620 | CG | PHE | B | 105 | 54.079 | 20.574 | 29.613 | 1.00 | 62.03 | B |
| ATOM | 3621 | CD1 | PHE | B | 105 | 53.650 | 20.126 | 28.357 | 1.00 | 61.61 | B |
| ATOM | 3622 | CD2 | PHE | B | 105 | 55.136 | 21.490 | 29.667 | 1.00 | 62.54 | B |
| ATOM | 3623 | CE1 | PHE | B | 105 | 54.265 | 20.580 | 27.175 | 1.00 | 62.11 | B |
| ATOM | 3624 | CE2 | PHE | B | 105 | 55.759 | 21.949 | 28.486 | 1.00 | 63.12 | B |
| ATOM | 3625 | CZ | PHE | B | 105 | 55.320 | 21.490 | 27.241 | 1.00 | 62.44 | B |
| ATOM | 3626 | C | PHE | B | 105 | 53.624 | 19.250 | 33.271 | 1.00 | 56.35 | B |
| ATOM | 3627 | O | PHE | B | 105 | 52.946 | 20.104 | 33.850 | 1.00 | 55.85 | B |
| ATOM | 3628 | N | GLY | B | 106 | 53.759 | 17.997 | 33.710 | 1.00 | 56.19 | B |
| ATOM | 3629 | CA | GLY | B | 106 | 53.104 | 17.527 | 34.925 | 1.00 | 55.85 | B |
| ATOM | 3630 | C | GLY | B | 106 | 51.593 | 17.367 | 34.824 | 1.00 | 55.01 | B |
| ATOM | 3631 | O | GLY | B | 106 | 51.070 | 17.163 | 33.723 | 1.00 | 56.20 | B |
| ATOM | 3632 | N | PRO | B | 107 | 50.869 | 17.374 | 35.962 | 1.00 | 53.70 | B |
| ATOM | 3633 | CD | PRO | B | 107 | 51.378 | 17.262 | 37.339 | 1.00 | 53.68 | B |
| ATOM | 3634 | CA | PRO | B | 107 | 49.412 | 17.234 | 35.956 | 1.00 | 52.75 | B |
| ATOM | 3635 | CB | PRO | B | 107 | 49.071 | 17.221 | 37.442 | 1.00 | 52.61 | B |
| ATOM | 3636 | CG | PRO | B | 107 | 50.245 | 16.549 | 38.036 | 1.00 | 51.47 | B |
| ATOM | 3637 | C | PRO | B | 107 | 48.728 | 18.385 | 35.208 | 1.00 | 52.32 | B |
| ATOM | 3638 | O | PRO | B | 107 | 47.710 | 18.169 | 34.561 | 1.00 | 52.99 | B |
| ATOM | 3639 | N | THR | B | 108 | 49.295 | 19.594 | 35.284 | 1.00 | 51.70 | B |
| ATOM | 3640 | CA | THR | B | 108 | 48.755 | 20.761 | 34.572 | 1.00 | 50.34 | B |
| ATOM | 3641 | CB | THR | B | 108 | 49.678 | 22.001 | 34.738 | 1.00 | 49.79 | B |
| ATOM | 3642 | OG1 | THR | B | 108 | 49.704 | 22.400 | 36.111 | 1.00 | 49.11 | B |
| ATOM | 3643 | CG2 | THR | B | 108 | 49.200 | 23.172 | 33.886 | 1.00 | 49.40 | B |
| ATOM | 3644 | C | THR | B | 108 | 48.677 | 20.395 | 33.085 | 1.00 | 50.20 | B |
| ATOM | 3645 | O | THR | B | 108 | 47.711 | 20.731 | 32.398 | 1.00 | 50.87 | B |
| ATOM | 3646 | N | GLY | B | 109 | 49.691 | 19.666 | 32.622 | 1.00 | 50.13 | B |
| ATOM | 3647 | CA | GLY | B | 109 | 49.763 | 19.225 | 31.239 | 1.00 | 49.64 | B |
| ATOM | 3648 | C | GLY | B | 109 | 48.820 | 18.075 | 30.949 | 1.00 | 49.44 | B |
| ATOM | 3649 | O | GLY | B | 109 | 48.169 | 18.058 | 29.903 | 1.00 | 50.17 | B |
| ATOM | 3650 | N | CYS | B | 110 | 48.758 | 17.104 | 31.859 | 1.00 | 48.34 | B |
| ATOM | 3651 | CA | CYS | B | 110 | 47.870 | 15.954 | 31.695 | 1.00 | 47.98 | B |
| ATOM | 3652 | C | CYS | B | 110 | 46.404 | 16.392 | 31.701 | 1.00 | 48.94 | B |
| ATOM | 3653 | O | CYS | B | 110 | 45.553 | 15.771 | 31.050 | 1.00 | 49.22 | B |
| ATOM | 3654 | CB | CYS | B | 110 | 48.113 | 14.919 | 32.793 | 1.00 | 46.09 | B |
| ATOM | 3655 | SG | CYS | B | 110 | 46.765 | 13.711 | 33.007 | 1.00 | 44.69 | B |
| ATOM | 3656 | N | ASN | B | 111 | 46.118 | 17.454 | 32.454 | 1.00 | 48.31 | B |
| ATOM | 3657 | CA | ASN | B | 111 | 44.771 | 18.008 | 32.543 | 1.00 | 46.74 | B |
| ATOM | 3658 | CB | ASN | B | 111 | 44.682 | 19.037 | 33.674 | 1.00 | 46.98 | B |
| ATOM | 3659 | CG | ASN | B | 111 | 44.689 | 18.399 | 35.053 | 1.00 | 49.71 | B |
| ATOM | 3660 | OD1 | ASN | B | 111 | 44.142 | 17.311 | 35.252 | 1.00 | 52.62 | B |
| ATOM | 3661 | ND2 | ASN | B | 111 | 45.298 | 19.082 | 36.015 | 1.00 | 48.23 | B |
| ATOM | 3662 | C | ASN | B | 111 | 44.444 | 18.674 | 31.216 | 1.00 | 45.36 | B |
| ATOM | 3663 | O | ASN | B | 111 | 43.389 | 18.422 | 30.632 | 1.00 | 45.49 | B |
| ATOM | 3664 | N | LEU | B | 112 | 45.362 | 19.518 | 30.744 | 1.00 | 42.47 | B |
| ATOM | 3665 | CA | LEU | B | 112 | 45.198 | 20.210 | 29.475 | 1.00 | 39.90 | B |
| ATOM | 3666 | CB | LEU | B | 112 | 46.467 | 21.008 | 29.148 | 1.00 | 36.99 | B |
| ATOM | 3667 | CG | LEU | B | 112 | 46.816 | 22.245 | 29.972 | 1.00 | 33.75 | B |
| ATOM | 3668 | CD1 | LEU | B | 112 | 48.194 | 22.754 | 29.595 | 1.00 | 33.80 | B |
| ATOM | 3669 | CD2 | LEU | B | 112 | 45.790 | 23.316 | 29.736 | 1.00 | 33.98 | B |
| ATOM | 3670 | C | LEU | B | 112 | 44.904 | 19.193 | 28.358 | 1.00 | 39.79 | B |
| ATOM | 3671 | O | LEU | B | 112 | 43.955 | 19.358 | 27.586 | 1.00 | 39.52 | B |
| ATOM | 3672 | N | GLU | B | 113 | 45.682 | 18.111 | 28.349 | 1.00 | 39.85 | B |
| ATOM | 3673 | CA | GLU | B | 113 | 45.585 | 17.025 | 27.370 | 1.00 | 40.08 | B |
| ATOM | 3674 | CB | GLU | B | 113 | 46.679 | 15.988 | 27.672 | 1.00 | 38.45 | B |
| ATOM | 3675 | CG | GLU | B | 113 | 47.347 | 15.354 | 26.463 | 1.00 | 37.81 | B |
| ATOM | 3676 | CD | GLU | B | 113 | 46.370 | 14.660 | 25.553 | 1.00 | 38.73 | B |
| ATOM | 3677 | OE1 | GLU | B | 113 | 46.172 | 15.127 | 24.411 | 1.00 | 38.16 | B |
| ATOM | 3678 | OE2 | GLU | B | 113 | 45.776 | 13.658 | 25.992 | 1.00 | 40.50 | B |
| ATOM | 3679 | C | GLU | B | 113 | 44.215 | 16.325 | 27.292 | 1.00 | 41.34 | B |
| ATOM | 3680 | O | GLU | B | 113 | 43.651 | 16.175 | 26.205 | 1.00 | 41.79 | B |
| ATOM | 3681 | N | GLY | B | 114 | 43.703 | 15.884 | 28.442 | 1.00 | 43.30 | B |
| ATOM | 3682 | CA | GLY | B | 114 | 42.426 | 15.182 | 28.496 | 1.00 | 44.82 | B |
| ATOM | 3683 | C | GLY | B | 114 | 41.180 | 16.045 | 28.423 | 1.00 | 46.18 | B |
| ATOM | 3684 | O | GLY | B | 114 | 40.206 | 15.676 | 27.758 | 1.00 | 47.23 | B |
| ATOM | 3685 | N | PHE | B | 115 | 41.200 | 17.183 | 29.115 | 1.00 | 45.79 | B |
| ATOM | 3686 | CA | PHE | B | 115 | 40.070 | 18.109 | 29.127 | 1.00 | 45.41 | B |
| ATOM | 3687 | CB | PHE | B | 115 | 40.404 | 19.353 | 29.961 | 1.00 | 45.36 | B |
| ATOM | 3688 | CG | PHE | B | 115 | 39.330 | 20.411 | 29.947 | 1.00 | 46.49 | B |
| ATOM | 3689 | CD1 | PHE | B | 115 | 39.623 | 21.710 | 29.532 | 1.00 | 48.45 | B |
| ATOM | 3690 | CD2 | PHE | B | 115 | 38.032 | 20.115 | 30.339 | 1.00 | 46.60 | B |
| ATOM | 3691 | CE1 | PHE | B | 115 | 38.641 | 22.693 | 29.506 | 1.00 | 48.23 | B |
| ATOM | 3692 | CE2 | PHE | B | 115 | 37.043 | 21.091 | 30.317 | 1.00 | 46.86 | B |
| ATOM | 3693 | CZ | PHE | B | 115 | 37.347 | 22.381 | 29.899 | 1.00 | 48.14 | B |
| ATOM | 3694 | C | PHE | B | 115 | 39.655 | 18.521 | 27.722 | 1.00 | 45.17 | B |
| ATOM | 3695 | O | PHE | B | 115 | 38.512 | 18.309 | 27.322 | 1.00 | 46.00 | B |
| ATOM | 3696 | N | PHE | B | 116 | 40.592 | 19.080 | 26.964 | 1.00 | 44.38 | B |
| ATOM | 3697 | CA | PHE | B | 116 | 40.294 | 19.526 | 25.613 | 1.00 | 44.00 | B |
| ATOM | 3698 | CB | PHE | B | 116 | 41.439 | 20.372 | 25.080 | 1.00 | 42.64 | B |
| ATOM | 3699 | CG | PHE | B | 116 | 41.514 | 21.725 | 25.709 | 1.00 | 42.99 | B |
| ATOM | 3700 | CD1 | PHE | B | 116 | 40.697 | 22.758 | 25.259 | 1.00 | 43.18 | B |
| ATOM | 3701 | CD2 | PHE | B | 116 | 42.368 | 21.965 | 26.773 | 1.00 | 42.55 | B |
| ATOM | 3702 | CE1 | PHE | B | 116 | 40.729 | 24.009 | 25.863 | 1.00 | 42.57 | B |
| ATOM | 3703 | CE2 | PHE | B | 116 | 42.406 | 23.216 | 27.383 | 1.00 | 43.12 | B |
| ATOM | 3704 | CZ | PHE | B | 116 | 41.583 | 24.239 | 26.926 | 1.00 | 42.69 | B |
| ATOM | 3705 | C | PHE | B | 116 | 39.929 | 18.414 | 24.640 | 1.00 | 44.52 | B |
| ATOM | 3706 | O | PHE | B | 116 | 39.290 | 18.672 | 23.614 | 1.00 | 45.35 | B |
| ATOM | 3707 | N | ALA | B | 117 | 40.302 | 17.181 | 24.986 | 1.00 | 43.79 | B |
| ATOM | 3708 | CA | ALA | B | 117 | 40.021 | 16.008 | 24.159 | 1.00 | 43.32 | B |
| ATOM | 3709 | CB | ALA | B | 117 | 41.077 | 14.940 | 24.386 | 1.00 | 43.27 | B |
| ATOM | 3710 | C | ALA | B | 117 | 38.641 | 15.455 | 24.475 | 1.00 | 43.50 | B |
| ATOM | 3711 | O | ALA | B | 117 | 37.953 | 14.934 | 23.596 | 1.00 | 43.60 | B |
| ATOM | 3712 | N | THR | B | 118 | 38.262 | 15.545 | 25.747 | 1.00 | 43.31 | B |
| ATOM | 3713 | CA | THR | B | 118 | 36.960 | 15.078 | 26.212 | 1.00 | 42.88 | B |
| ATOM | 3714 | CB | THR | B | 118 | 36.969 | 14.832 | 27.734 | 1.00 | 41.92 | B |
| ATOM | 3715 | OG1 | THR | B | 118 | 38.063 | 13.971 | 28.064 | 1.00 | 41.86 | B |
| ATOM | 3716 | CG2 | THR | B | 118 | 35.667 | 14.178 | 28.182 | 1.00 | 40.79 | B |
| ATOM | 3717 | C | THR | B | 118 | 35.937 | 16.158 | 25.876 | 1.00 | 42.86 | B |
| ATOM | 3718 | O | THR | B | 118 | 34.790 | 15.860 | 25.528 | 1.00 | 42.47 | B |
| ATOM | 3719 | N | LEU | B | 119 | 36.364 | 17.413 | 26.006 | 1.00 | 41.98 | B |
| ATOM | 3720 | CA | LEU | B | 119 | 35.522 | 18.555 | 25.686 | 1.00 | 41.28 | B |
| ATOM | 3721 | CB | LEU | B | 119 | 36.217 | 19.868 | 26.077 | 1.00 | 39.09 | B |
| ATOM | 3722 | CG | LEU | B | 119 | 35.446 | 21.192 | 25.944 | 1.00 | 37.37 | B |
| ATOM | 3723 | CD1 | LEU | B | 119 | 34.283 | 21.235 | 26.909 | 1.00 | 35.07 | B |
| ATOM | 3724 | CD2 | LEU | B | 119 | 36.379 | 22.364 | 26.207 | 1.00 | 36.70 | B |
| ATOM | 3725 | C | LEU | B | 119 | 35.331 | 18.498 | 24.174 | 1.00 | 42.38 | B |
| ATOM | 3726 | O | LEU | B | 119 | 34.265 | 18.821 | 23.659 | 1.00 | 43.88 | B |
| ATOM | 3727 | N | GLY | B | 120 | 36.359 | 18.033 | 23.473 | 1.00 | 42.14 | B |
| ATOM | 3728 | CA | GLY | B | 120 | 36.280 | 17.940 | 22.032 | 1.00 | 42.32 | B |
| ATOM | 3729 | C | GLY | B | 120 | 35.334 | 16.858 | 21.564 | 1.00 | 42.80 | B |
| ATOM | 3730 | O | GLY | B | 120 | 34.481 | 17.098 | 20.705 | 1.00 | 42.95 | B |
| ATOM | 3731 | N | GLY | B | 121 | 35.485 | 15.663 | 22.127 | 1.00 | 44.01 | B |
| ATOM | 3732 | CA | GLY | B | 121 | 34.636 | 14.544 | 21.753 | 1.00 | 45.43 | B |
| ATOM | 3733 | C | GLY | B | 121 | 33.194 | 14.675 | 22.209 | 1.00 | 45.94 | B |
| ATOM | 3734 | O | GLY | B | 121 | 32.286 | 14.129 | 21.573 | 1.00 | 44.97 | B |
| ATOM | 3735 | N | GLU | B | 122 | 32.986 | 15.365 | 23.328 | 1.00 | 46.14 | B |
| ATOM | 3736 | CA | GLU | B | 122 | 31.642 | 15.566 | 23.853 | 1.00 | 47.59 | B |
| ATOM | 3737 | CB | GLU | B | 122 | 31.667 | 15.827 | 25.361 | 1.00 | 47.31 | B |
| ATOM | 3738 | CG | GLU | B | 122 | 31.875 | 14.565 | 26.192 | 1.00 | 47.02 | B |
| ATOM | 3739 | CD | GLU | B | 122 | 30.825 | 13.492 | 25.912 | 1.00 | 46.61 | B |
| ATOM | 3740 | OE1 | GLU | B | 122 | 29.621 | 13.771 | 26.069 | 1.00 | 48.82 | B |
| ATOM | 3741 | OE2 | GLU | B | 122 | 31.199 | 12.364 | 25.537 | 1.00 | 47.01 | B |
| ATOM | 3742 | C | GLU | B | 122 | 30.884 | 16.663 | 23.106 | 1.00 | 48.47 | B |
| ATOM | 3743 | O | GLU | B | 122 | 29.675 | 16.557 | 22.913 | 1.00 | 49.08 | B |
| ATOM | 3744 | N | ILE | B | 123 | 31.587 | 17.708 | 22.675 | 1.00 | 48.34 | B |
| ATOM | 3745 | CA | ILE | B | 123 | 30.944 | 18.771 | 21.909 | 1.00 | 47.51 | B |
| ATOM | 3746 | CB | ILE | B | 123 | 31.904 | 19.962 | 21.641 | 1.00 | 46.65 | B |
| ATOM | 3747 | CG2 | ILE | B | 123 | 31.362 | 20.843 | 20.519 | 1.00 | 47.07 | B |
| ATOM | 3748 | CG1 | ILE | B | 123 | 32.087 | 20.781 | 22.924 | 1.00 | 44.79 | B |
| ATOM | 3749 | CD1 | ILE | B | 123 | 32.978 | 21.992 | 22.781 | 1.00 | 43.99 | B |
| ATOM | 3750 | C | ILE | B | 123 | 30.492 | 18.137 | 20.594 | 1.00 | 47.83 | B |
| ATOM | 3751 | O | ILE | B | 123 | 29.450 | 18.490 | 20.054 | 1.00 | 48.33 | B |
| ATOM | 3752 | N | ALA | B | 124 | 31.259 | 17.146 | 20.140 | 1.00 | 48.25 | B |
| ATOM | 3753 | CA | ALA | B | 124 | 30.982 | 16.398 | 18.914 | 1.00 | 48.76 | B |
| ATOM | 3754 | CB | ALA | B | 124 | 32.205 | 15.564 | 18.525 | 1.00 | 48.96 | B |
| ATOM | 3755 | C | ALA | B | 124 | 29.759 | 15.491 | 19.073 | 1.00 | 48.88 | B |
| ATOM | 3756 | O | ALA | B | 124 | 28.902 | 15.431 | 18.190 | 1.00 | 47.71 | B |
| ATOM | 3757 | N | LEU | B | 125 | 29.711 | 14.760 | 20.186 | 1.00 | 50.22 | B |
| ATOM | 3758 | CA | LEU | B | 125 | 28.593 | 13.865 | 20.489 | 1.00 | 51.05 | B |
| ATOM | 3759 | CB | LEU | B | 125 | 28.900 | 13.018 | 21.728 | 1.00 | 50.63 | B |
| ATOM | 3760 | CG | LEU | B | 125 | 28.271 | 11.630 | 21.916 | 1.00 | 49.87 | B |
| ATOM | 3761 | CD1 | LEU | B | 125 | 28.113 | 11.364 | 23.401 | 1.00 | 48.75 | B |
| ATOM | 3762 | CD2 | LEU | B | 125 | 26.929 | 11.510 | 21.229 | 1.00 | 50.11 | B |
| ATOM | 3763 | C | LEU | B | 125 | 27.354 | 14.721 | 20.762 | 1.00 | 52.16 | B |
| ATOM | 3764 | O | LEU | B | 125 | 26.252 | 14.362 | 20.347 | 1.00 | 51.60 | B |
| ATOM | 3765 | N | TRP | B | 126 | 27.541 | 15.848 | 21.455 | 1.00 | 53.09 | B |
| ATOM | 3766 | CA | TRP | B | 126 | 26.432 | 16.752 | 21.769 | 1.00 | 54.49 | B |
| ATOM | 3767 | CB | TRP | B | 126 | 26.758 | 17.659 | 22.968 | 1.00 | 55.97 | B |
| ATOM | 3768 | CG | TRP | B | 126 | 26.806 | 16.897 | 24.286 | 1.00 | 58.33 | B |
| ATOM | 3769 | CD2 | TRP | B | 126 | 25.766 | 16.087 | 24.848 | 1.00 | 58.57 | B |
| ATOM | 3770 | CE2 | TRP | B | 126 | 26.276 | 15.506 | 26.028 | 1.00 | 58.63 | B |
| ATOM | 3771 | CE3 | TRP | B | 126 | 24.450 | 15.793 | 24.465 | 1.00 | 59.26 | B |
| ATOM | 3772 | CD1 | TRP | B | 126 | 27.872 | 16.786 | 25.140 | 1.00 | 59.36 | B |
| ATOM | 3773 | NE1 | TRP | B | 126 | 27.562 | 15.949 | 26.185 | 1.00 | 58.29 | B |
| ATOM | 3774 | CZ2 | TRP | B | 126 | 25.519 | 14.649 | 26.823 | 1.00 | 60.10 | B |
| ATOM | 3775 | C23 | TRP | B | 126 | 23.698 | 14.940 | 25.259 | 1.00 | 59.04 | B |
| ATOM | 3776 | CH2 | TRP | B | 126 | 24.234 | 14.379 | 26.424 | 1.00 | 59.35 | B |
| ATOM | 3777 | C | TRP | B | 126 | 25.961 | 17.551 | 20.555 | 1.00 | 54.14 | B |
| ATOM | 3778 | O | TRP | B | 126 | 24.792 | 17.921 | 20.463 | 1.00 | 53.26 | B |
| ATOM | 3779 | N | SER | B | 127 | 26.863 | 17.801 | 19.615 | 1.00 | 54.40 | B |
| ATOM | 3780 | CA | SER | B | 127 | 26.477 | 18.505 | 18.402 | 1.00 | 55.30 | B |
| ATOM | 3781 | CB | SER | B | 127 | 27.688 | 19.112 | 17.689 | 1.00 | 55.22 | B |
| ATOM | 3782 | OG | SER | B | 127 | 28.169 | 20.247 | 18.393 | 1.00 | 56.21 | B |
| ATOM | 3783 | C | SER | B | 127 | 25.753 | 17.514 | 17.496 | 1.00 | 55.23 | B |
| ATOM | 3784 | O | SER | B | 127 | 24.914 | 17.901 | 16.693 | 1.00 | 55.29 | B |
| ATOM | 3785 | N | LEU | B | 128 | 26.078 | 16.231 | 17.629 | 1.00 | 56.20 | B |
| ATOM | 3786 | CA | LEU | B | 128 | 25.413 | 15.203 | 16.837 | 1.00 | 57.61 | B |
| ATOM | 3787 | CB | LEU | B | 128 | 26.094 | 13.844 | 16.985 | 1.00 | 57.29 | B |
| ATOM | 3788 | CG | LEU | B | 128 | 27.477 | 13.607 | 16.392 | 1.00 | 56.44 | B |
| ATOM | 3789 | CD1 | LEU | B | 128 | 27.681 | 12.109 | 16.264 | 1.00 | 55.64 | B |
| ATOM | 3790 | CD2 | LEU | B | 128 | 127.593 | 14.267 | 15.036 | 1.00 | 56.71 | B |
| ATOM | 3791 | C | LEU | B | 128 | 23.972 | 15.083 | 17.305 | 1.00 | 59.21 | B |
| ATOM | 3792 | O | LEU | B | 128 | 23.125 | 14.544 | 16.593 | 1.00 | 58.61 | B |
| ATOM | 3793 | N | VAL | B | 129 | 23.710 | 15.550 | 18.527 | 1.00 | 61.62 | B |
| ATOM | 3794 | CA | VAL | B | 129 | 22.359 | 15.518 | 19.090 | 1.00 | 63.83 | B |
| ATOM | 3795 | CB | VAL | B | 129 | 22.336 | 15.597 | 20.636 | 1.00 | 63.76 | B |
| ATOM | 3796 | CG1 | VAL | B | 129 | 21.162 | 14.798 | 21.177 | 1.00 | 63.03 | B |
| ATOM | 3797 | CG2 | VAL | B | 129 | 23.628 | 15.117 | 21.227 | 1.00 | 64.25 | B |
| ATOM | 3798 | C | VAL | B | 129 | 21.585 | 16.725 | 18.566 | 1.00 | 64.71 | B |
| ATOM | 3799 | O | VAL | B | 129 | 20.517 | 16.571 | 17.968 | 1.00 | 65.71 | B |
| ATOM | 3800 | N | VAL | B | 130 | 22.129 | 17.922 | 18.798 | 1.00 | 64.55 | B |
| ATOM | 3801 | CA | VAL | B | 130 | 21.506 | 19.161 | 18.340 | 1.00 | 63.77 | B |
| ATOM | 3802 | CB | VAL | B | 130 | 22.418 | 20.380 | 18.629 | 1.00 | 62.58 | B |
| ATOM | 3803 | CG1 | VAL | B | 130 | 21.797 | 21.649 | 18.110 | 1.00 | 62.49 | B |
| ATOM | 3804 | CG2 | VAL | B | 130 | 22.639 | 20.511 | 20.121 | 1.00 | 62.69 | B |
| ATOM | 3805 | C | VAL | B | 130 | 21.164 | 19.050 | 16.847 | 1.00 | 64.50 | B |
| ATOM | 3806 | O | VAL | B | 130 | 20.160 | 19.600 | 16.404 | 1.00 | 64.32 | B |
| ATOM | 3807 | N | LEU | B | 131 | 21.968 | 18.282 | 16.103 | 1.00 | 66.15 | B |
| ATOM | 3808 | CA | LEU | B | 131 | 21.755 | 18.043 | 14.666 | 1.00 | 67.53 | B |
| ATOM | 3809 | CB | LEU | B | 131 | 23.065 | 17.628 | 13.968 | 1.00 | 66.53 | B |
| ATOM | 3810 | CG | LEU | B | 131 | 24.114 | 18.691 | 13.606 | 1.00 | 65.91 | B |
| ATOM | 3811 | CD1 | LEU | B | 131 | 25.360 | 18.028 | 13.064 | 1.00 | 64.91 | B |
| ATOM | 3812 | CD2 | LEU | B | 131 | 23.557 | 19.682 | 12.591 | 1.00 | 65.41 | B |
| ATOM | 3813 | C | LEU | B | 131 | 20.700 | 16.954 | 14.439 | 1.00 | 68.71 | B |
| ATOM | 3814 | O | LEU | B | 131 | 19.860 | 17.066 | 13.550 | 1.00 | 67.98 | B |
| ATOM | 3815 | N | ALA | B | 132 | 20.757 | 15.904 | 15.253 | 1.00 | 70.91 | B |
| ATOM | 3816 | CA | ALA | B | 132 | 19.822 | 14.785 | 15.159 | 1.00 | 73.30 | B |
| ATOM | 3817 | CB | ALA | B | 132 | 20.163 | 13.727 | 16.210 | 1.00 | 74.56 | B |
| ATOM | 3818 | C | ALA | B | 132 | 18.370 | 15.223 | 15.314 | 1.00 | 74.21 | B |
| ATOM | 3819 | O | ALA | B | 132 | 17.495 | 14.759 | 14.584 | 1.00 | 73.79 | B |
| ATOM | 3820 | N | ILE | B | 133 | 18.127 | 16.105 | 16.280 | 1.00 | 75.90 | B |
| ATOM | 3821 | CA | ILE | B | 133 | 16.789 | 16.629 | 16.556 | 1.00 | 77.86 | B |
| ATOM | 3822 | CB | ILE | B | 133 | 16.768 | 17.453 | 17.871 | 1.00 | 78.06 | B |
| ATOM | 3823 | CG2 | ILE | B | 133 | 15.447 | 18.210 | 18.014 | 1.00 | 77.94 | B |
| ATOM | 3824 | CG1 | ILE | B | 133 | 17.002 | 16.528 | 19.069 | 1.00 | 77.27 | B |
| ATOM | 3825 | CD1 | ILE | B | 133 | 17.120 | 17.251 | 20.394 | 1.00 | 76.25 | B |
| ATOM | 3826 | C | ILE | B | 133 | 16.268 | 17.490 | 15.406 | 1.00 | 78.62 | B |
| ATOM | 3827 | O | ILE | B | 133 | 15.084 | 17.423 | 15.068 | 1.00 | 77.69 | B |
| ATOM | 3828 | N | GLU | B | 134 | 17.151 | 18.298 | 14.814 | 1.00 | 79.88 | B |
| ATOM | 3829 | CA | GLU | B | 134 | 16.763 | 19.154 | 13.695 | 1.00 | 80.97 | B |
| ATOM | 3830 | CB | GLU | B | 134 | 17.942 | 19.993 | 13.174 | 1.00 | 80.17 | B |
| ATOM | 3831 | CG | GLU | B | 134 | 17.540 | 20.984 | 12.063 | 1.00 | 79.68 | B |
| ATOM | 3832 | CD | GLU | B | 134 | 18.674 | 21.362 | 11.109 | 1.00 | 79.87 | B |
| ATOM | 3833 | OE1 | GLU | B | 134 | 18.461 | 22.267 | 10.272 | 1.00 | 79.09 | B |
| ATOM | 3834 | OE2 | GLU | B | 134 | 19.764 | 20.752 | 11.169 | 1.00 | 80.71 | B |
| ATOM | 3835 | C | GLU | B | 134 | 16.239 | 18.281 | 12.563 | 1.00 | 82.00 | B |
| ATOM | 3836 | O | GLU | B | 134 | 15.226 | 18.599 | 11.949 | 1.00 | 83.60 | B |
| ATOM | 3837 | N | ARG | B | 135 | 16.900 | 17.155 | 12.325 | 1.00 | 82.35 | B |
| ATOM | 3838 | CA | ARG | B | 135 | 16.485 | 16.266 | 11.252 | 1.00 | 83.46 | B |
| ATOM | 3839 | CB | ARG | B | 135 | 17.637 | 15.344 | 10.843 | 1.00 | 81.55 | B |
| ATOM | 3840 | CG | ARG | B | 135 | 18.986 | 16.053 | 10.705 | 1.00 | 79.05 | B |
| ATOM | 3841 | CD | ARG | B | 135 | 18.885 | 17.464 | 10.097 | 1.00 | 77.21 | B |
| ATOM | 3842 | NE | ARG | B | 135 | 18.503 | 17.473 | 8.685 | 1.00 | 74.76 | B |
| ATOM | 3843 | CZ | ARG | B | 135 | 18.590 | 18.535 | 7.887 | 1.00 | 72.21 | B |
| ATOM | 3844 | NH1 | ARG | B | 135 | 19.046 | 19.691 | 8.353 | 1.00 | 70.55 | B |
| ATOM | 3845 | NH2 | ARG | B | 135 | 18.220 | 18.438 | 6.618 | 1.00 | 70.99 | B |
| ATOM | 3846 | C | ARG | B | 135 | 15.206 | 15.480 | 11.567 | 1.00 | 85.85 | B |
| ATOM | 3847 | O | ARG | B | 135 | 14.739 | 14.685 | 10.752 | 1.00 | 86.58 | B |
| ATOM | 3848 | N | TYR | B | 136 | 14.659 | 15.686 | 12.761 | 1.00 | 88.26 | B |
| ATOM | 3849 | CA | TYR | B | 136 | 13.411 | 15.043 | 13.164 | 1.00 | 90.79 | B |
| ATOM | 3850 | CB | TYR | B | 136 | 13.509 | 14.519 | 14.610 | 1.00 | 92.30 | B |
| ATOM | 3851 | CG | TYR | B | 136 | 12.193 | 14.076 | 15.245 | 1.00 | 93.95 | B |
| ATOM | 3852 | CD1 | TYR | B | 136 | 11.568 | 12.882 | 14.866 | 1.00 | 94.13 | B |
| ATOM | 3853 | CE1 | TYR | B | 136 | 10.361 | 12.480 | 15.458 | 1.00 | 95.20 | B |
| ATOM | 3854 | CD2 | TYR | B | 136 | 11.578 | 14.857 | 16.234 | 1.00 | 94.05 | B |
| ATOM | 3855 | CE2 | TYR | B | 136 | 10.379 | 14.465 | 16.830 | 1.00 | 94.61 | B |
| ATOM | 3856 | CZ | TYR | B | 136 | 9.774 | 13.278 | 16.440 | 1.00 | 95.52 | B |
| ATOM | 3857 | OH | TYR | B | 136 | 8.592 | 12.894 | 17.039 | 1.00 | 95.57 | B |
| ATOM | 3858 | C | TYR | B | 136 | 12.346 | 16.131 | 13.047 | 1.00 | 91.94 | B |
| ATOM | 3859 | 0 | TYR | B | 136 | 11.165 | 15.891 | 13.276 | 1.00 | 92.42 | B |
| ATOM | 3860 | N | VAL | B | 137 | 12.786 | 17.331 | 12.675 | 1.00 | 93.53 | B |
| ATOM | 3861 | CA | VAL | B | 137 | 11.902 | 18.482 | 12.518 | 1.00 | 95.78 | B |
| ATOM | 3862 | CB | VAL | B | 137 | 12.339 | 19.647 | 13.443 | 1.00 | 95.49 | B |
| ATOM | 3863 | CG1 | VAL | B | 137 | 11.438 | 20.860 | 13.249 | 1.00 | 95.53 | B |
| ATOM | 3864 | CG2 | VAL | B | 137 | 12.309 | 19.194 | 14.897 | 1.00 | 95.34 | B |
| ATOM | 3865 | C | VAL | B | 137 | 11.833 | 18.956 | 11.060 | 1.00 | 97.47 | B |
| ATOM | 3866 | O | VAL | B | 137 | 10.744 | 19.144 | 10.521 | 1.00 | 97.89 | B |
| ATOM | 3867 | N | VAL | B | 138 | 12.986 | 19.148 | 10.423 | 1.00 | 99.41 | B |
| ATOM | 3868 | CA | VAL | B | 138 | 13.014 | 19.585 | 9.030 | 1.00 | 101.72 | B |
| ATOM | 3869 | CB | VAL | B | 138 | 14.315 | 20.354 | 8.684 | 1.00 | 100.87 | B |
| ATOM | 3870 | CG1 | VAL | B | 138 | 14.385 | 21.657 | 9.463 | 1.00 | 100.20 | B |
| ATOM | 3871 | CG2 | VAL | B | 138 | 15.526 | 19.507 | 8.979 | 1.00 | 100.97 | B |
| ATOM | 3872 | C | VAL | B | 138 | 12.804 | 18.414 | 8.061 | 1.00 | 104.51 | B |
| ATOM | 3873 | O | VAL | B | 138 | 12.824 | 18.593 | 6.843 | 1.00 | 105.14 | B |
| ATOM | 3874 | N | VAL | B | 139 | 12.616 | 17.217 | 8.615 | 1.00 | 108.02 | B |
| ATOM | 3875 | CA | VAL | B | 139 | 12.366 | 16.003 | 7.833 | 1.00 | 111.89 | B |
| ATOM | 3876 | CB | VAL | B | 139 | 13.483 | 14.944 | 8.014 | 1.00 | 112.38 | B |
| ATOM | 3877 | CG1 | VAL | B | 139 | 13.145 | 13.674 | 7.234 | 1.00 | 112.79 | B |
| ATOM | 3878 | CG2 | VAL | B | 139 | 14.822 | 15.504 | 7.544 | 1.00 | 112.54 | B |
| ATOM | 3879 | C | VAL | B | 139 | 11.018 | 15.437 | 8.286 | 1.00 | 114.39 | B |
| ATOM | 3880 | O | VAL | B | 139 | 10.223 | 14.979 | 7.467 | 1.00 | 113.91 | B |
| ATOM | 3881 | N | CYS | B | 140 | 10.791 | 15.423 | 9.599 | 1.00 | 118.58 | B |
| ATOM | 3882 | CA | CYS | B | 140 | 9.513 | 14.983 | 10.157 | 1.00 | 123.05 | B |
| ATOM | 3883 | CB | CYS | B | 140 | 9.702 | 14.224 | 11.474 | 1.00 | 123.02 | B |
| ATOM | 3884 | SG | CYS | B | 140 | 10.525 | 12.627 | 11.304 | 1.00 | 124.41 | B |
| ATOM | 3885 | C | CYS | B | 140 | 8.748 | 16.297 | 10.364 | 1.00 | 125.73 | B |
| ATOM | 3886 | O | CYS | B | 140 | 8.627 | 17.067 | 9.412 | 1.00 | 126.45 | B |
| ATOM | 3887 | N | LYS | B | 141 | 18.280 | 16.590 | 11.579 | 1.00 | 128.80 | B |
| ATOM | 3888 | CA | LYS | B | 141 | 7.554 | 17.848 | 11.827 | 1.00 | 131.99 | B |
| ATOM | 3889 | CB | LYS | B | 141 | 6.251 | 17.889 | 11.016 | 1.00 | 133.36 | B |
| ATOM | 3890 | CG | LYS | B | 141 | 6.338 | 18.774 | 9.772 | 1.00 | 135.18 | B |
| ATOM | 3891 | CD | LYS | B | 141 | 5.181 | 18.535 | 8.817 | 1.00 | 136.68 | B |
| ATOM | 3892 | CE | LYS | B | 141 | 5.349 | 19.341 | 7.536 | 1.00 | 137.18 | B |
| ATOM | 3893 | NZ | LYS | B | 141 | 4.262 | 19.060 | 6.555 | 1.00 | 137.49 | B |
| ATOM | 3894 | C | LYS | B | 141 | 7.267 | 18.188 | 13.295 | 1.00 | 133.51 | B |
| ATOM | 3895 | 0 | LYS | B | 141 | 7.181 | 17.292 | 14.141 | 1.00 | 133.68 | B |
| ATOM | 3896 | N | PRO | B | 142 | 7.119 | 19.498 | 13.611 | 1.00 | 134.82 | B |
| ATOM | 3897 | CD | PRO | B | 142 | 7.290 | 20.627 | 12.673 | 1.00 | 135.03 | B |
| ATOM | 3898 | CA | PRO | B | 142 | 6.841 | 20.002 | 14.966 | 1.00 | 135.59 | B |
| ATOM | 3899 | CB | PRO | B | 142 | 6.624 | 21.501 | 14.734 | 1.00 | 135.60 | B |
| ATOM | 3900 | CG | PRO | B | 142 | 7.545 | 21.796 | 13.604 | 1.00 | 135.30 | B |
| ATOM | 3901 | C | PRO | B | 142 | 5.615 | 19.364 | 15.620 | 1.00 | 136.09 | B |
| ATOM | 3902 | O | PRO | B | 142 | 4.561 | 19.229 | 14.996 | 1.00 | 136.49 | B |
| ATOM | 3903 | N | GLY | B | 149 | 16.541 | 29.203 | 13.561 | 1.00 | 127.68 | B |
| ATOM | 3904 | CA | GLY | B | 149 | 17.040 | 30.543 | 13.308 | 1.00 | 128.21 | B |
| ATOM | 3905 | C | GLY | B | 149 | 18.379 | 30.788 | 13.975 | 1.00 | 128.39 | B |
| ATOM | 3906 | O | GLY | B | 149 | 18.932 | 29.871 | 14.587 | 1.00 | 128.64 | B |
| ATOM | 3907 | N | GLU | B | 150 | 18.895 | 32.017 | 13.866 | 1.00 | 128.57 | B |
| ATOM | 3908 | CA | GLU | B | 150 | 20.182 | 32.397 | 14.469 | 1.00 | 128.11 | B |
| ATOM | 3909 | CB | GLU | B | 150 | 20.574 | 33.851 | 14.106 | 1.00 | 126.77 | B |
| ATOM | 3910 | CG | GLU | B | 150 | 21.117 | 34.074 | 12.679 | 1.00 | 123.79 | B |
| ATOM | 3911 | CD | GLU | B | 150 | 21.715 | 35.470 | 12.469 | 1.00 | 122.23 | B |
| ATOM | 3912 | OE1 | GLU | B | 150 | 22.694 | 35.824 | 13.161 | 1.00 | 120.84 | B |
| ATOM | 3913 | OE2 | GLU | B | 150 | 21.212 | 36.213 | 11.600 | 1.00 | 121.02 | B |
| ATOM | 3914 | C | GLU | B | 150 | 20.194 | 32.218 | 16.000 | 1.00 | 128.37 | B |
| ATOM | 3915 | O | GLU | B | 150 | 21.223 | 32.443 | 16.647 | 1.00 | 129.08 | B |
| ATOM | 3916 | N | ASN | B | 151 | 19.057 | 31.802 | 16.565 | 1.00 | 127.64 | B |
| ATOM | 3917 | CA | ASN | B | 151 | 18.923 | 31.585 | 18.008 | 1.00 | 126.44 | B |
| ATOM | 3918 | CB | ASN | B | 151 | 17.637 | 32.231 | 18.533 | 1.00 | 127.74 | B |
| ATOM | 3919 | CG | ASN | B | 151 | 17.762 | 33.731 | 18.694 | 1.00 | 128.73 | B |
| ATOM | 3920 | OD1 | ASN | B | 151 | 17.802 | 34.245 | 19.814 | 1.00 | 129.41 | B |
| ATOM | 3921 | ND2 | ASN | B | 151 | 17.827 | 34.444 | 17.573 | 1.00 | 129.30 | B |
| ATOM | 3922 | C | ASN | B | 151 | 18.951 | 30.115 | 18.424 | 1.00 | 124.70 | B |
| ATOM | 3923 | O | ASN | B | 151 | 19.809 | 29.700 | 19.206 | 1.00 | 125.42 | B |
| ATOM | 3924 | N | HIS | B | 152 | 18.000 | 29.340 | 17.906 | 1.00 | 121.62 | B |
| ATOM | 3925 | CA | HIS | B | 152 | 17.878 | 27.915 | 18.215 | 1.00 | 118.40 | B |
| ATOM | 3926 | CB | HIS | B | 152 | 16.754 | 27.303 | 17.378 | 1.00 | 119.05 | B |
| ATOM | 3927 | CG | HIS | B | 152 | 15.471 | 28.076 | 17.428 | 1.00 | 119.29 | B |
| ATOM | 3928 | CD2 | HIS | B | 152 | 14.206 | 27.724 | 17.100 | 1.00 | 119.25 | B |
| ATOM | 3929 | ND1 | HIS | B | 152 | 15.407 | 29.389 | 17.846 | 1.00 | 119.47 | B |
| ATOM | 3930 | CE1 | HIS | B | 152 | 14.157 | 29.811 | 17.773 | 1.00 | 119.54 | B |
| ATOM | 3931 | NE2 | HIS | B | 152 | 13.408 | 28.820 | 17.323 | 1.00 | 119.51 | B |
| ATOM | 3932 | C | HIS | B | 152 | 19.188 | 27.168 | 17.966 | 1.00 | 115.80 | B |
| ATOM | 3933 | O | HIS | B | 152 | 19.518 | 26.211 | 18.670 | 1.00 | 115.47 | B |
| ATOM | 3934 | N | ALA | B | 153 | 19.927 | 27.621 | 16.960 | 1.00 | 112.43 | B |
| ATOM | 3935 | CA | ALA | B | 153 | 21.207 | 27.035 | 16.613 | 1.00 | 109.07 | B |
| ATOM | 3936 | CB | ALA | B | 153 | 21.639 | 27.507 | 15.247 | 1.00 | 109.45 | B |
| ATOM | 3937 | C | ALA | B | 153 | 22.239 | 27.425 | 17.664 | 1.00 | 106.85 | B |
| ATOM | 3938 | O | ALA | B | 153 | 23.136 | 26.643 | 17.970 | 1.00 | 107.45 | B |
| ATOM | 3939 | N | ILE | B | 154 | 22.112 | 28.635 | 18.209 | 1.00 | 103.57 | B |
| ATOM | 3940 | CA | ILE | B | 154 | 23.029 | 29.108 | 19.244 | 1.00 | 101.42 | B |
| ATOM | 3941 | CB | ILE | B | 154 | 23.091 | 30.660 | 19.309 | 1.00 | 100.90 | B |
| ATOM | 3942 | CG2 | ILE | B | 154 | 23.486 | 31.147 | 20.711 | 1.00 | 100.24 | B |
| ATOM | 3943 | CG1 | ILE | B | 154 | 24.079 | 31.165 | 18.250 | 1.00 | 100.98 | B |
| ATOM | 3944 | CD1 | ILE | B | 154 | 24.528 | 32.609 | 18.426 | 1.00 | 101.03 | B |
| ATOM | 3945 | C | ILE | B | 154 | 22.724 | 28.493 | 20.612 | 1.00 | 100.15 | B |
| ATOM | 3946 | O | ILE | B | 154 | 23.614 | 28.370 | 21.457 | 1.00 | 100.70 | B |
| ATOM | 3947 | N | MET | B | 155 | 21.471 | 28.101 | 20.826 | 1.00 | 98.04 | B |
| ATOM | 3948 | CA | MET | B | 155 | 21.084 | 27.461 | 22.081 | 1.00 | 95.74 | B |
| ATOM | 3949 | CB | MET | B | 155 | 19.571 | 27.470 | 22.260 | 1.00 | 97.60 | B |
| ATOM | 3950 | CG | MET | B | 155 | 19.035 | 28.781 | 22.787 | 1.00 | 99.64 | B |
| ATOM | 3951 | SD | MET | B | 155 | 17.258 | 28.705 | 23.005 | 1.00 | 102.63 | B |
| ATOM | 3952 | CE | MET | B | 155 | 16.705 | 29.956 | 21.823 | 1.00 | 102.27 | B |
| ATOM | 3953 | C | MET | B | 155 | 21.596 | 26.030 | 22.051 | 1.00 | 92.96 | B |
| ATOM | 3954 | O | MET | B | 155 | 22.054 | 25.505 | 23.064 | 1.00 | 92.82 | B |
| ATOM | 3955 | N | GLY | B | 156 | 21.500 | 25.407 | 20.878 | 1.00 | 89.63 | B |
| ATOM | 3956 | CA | GLY | B | 156 | 21.993 | 24.054 | 20.701 | 1.00 | 85.10 | B |
| ATOM | 3957 | C | GLY | B | 156 | 23.509 | 24.094 | 20.786 | 1.00 | 81.64 | B |
| ATOM | 3958 | O | GLY | B | 156 | 24.136 | 23.149 | 21.262 | 1.00 | 82.85 | B |
| ATOM | 3959 | N | VAL | B | 157 | 24.089 | 25.201 | 20.321 | 1.00 | 76.82 | B |
| ATOM | 3960 | CA | VAL | B | 157 | 25.529 | 25.426 | 20.357 | 1.00 | 71.27 | B |
| ATOM | 3961 | CB | VAL | B | 157 | 25.924 | 26.610 | 19.433 | 1.00 | 69.95 | B |
| ATOM | 3962 | CG1 | VAL | B | 157 | 27.123 | 27.375 | 19.978 | 1.00 | 68.93 | B |
| ATOM | 3963 | CG2 | VAL | B | 157 | 26.222 | 26.087 | 18.039 | 1.00 | 68.69 | B |
| ATOM | 3964 | C | VAL | B | 157 | 25.939 | 25.690 | 21.804 | 1.00 | 69.06 | B |
| ATOM | 3965 | O | VAL | B | 157 | 26.979 | 25.218 | 22.256 | 1.00 | 69.32 | B |
| ATOM | 3966 | N | ALA | B | 158 | 25.097 | 26.412 | 22.538 | 1.00 | 65.63 | B |
| ATOM | 3967 | CA | ALA | B | 158 | 25.373 | 26.713 | 23.937 | 1.00 | 63.38 | B |
| ATOM | 3968 | CB | ALA | B | 158 | 24.526 | 27.878 | 24.405 | 1.00 | 63.05 | B |
| ATOM | 3969 | C | ALA | B | 158 | 25.088 | 25.482 | 24.788 | 1.00 | 61.54 | B |
| ATOM | 3970 | O | ALA | B | 158 | 25.665 | 25.308 | 25.860 | 1.00 | 61.64 | B |
| ATOM | 3971 | N | PHE | B | 159 | 24.191 | 24.636 | 24.297 | 1.00 | 58.78 | B |
| ATOM | 3972 | CA | PHE | B | 159 | 23.809 | 23.409 | 24.981 | 1.00 | 56.72 | B |
| ATOM | 3973 | CB | PHE | B | 159 | 22.503 | 22.880 | 24.388 | 1.00 | 57.14 | B |
| ATOM | 3974 | CG | PHE | B | 159 | 22.168 | 21.483 | 24.802 | 1.00 | 57.52 | B |
| ATOM | 3975 | CD1 | PHE | B | 159 | 21.568 | 21.233 | 26.030 | 1.00 | 58.26 | B |
| ATOM | 3976 | CD2 | PHE | B | 159 | 22.439 | 20.414 | 23.955 | 1.00 | 56.66 | B |
| ATOM | 3977 | CE1 | PHE | B | 159 | 21.242 | 19.934 | 26.406 | 1.00 | 58.36 | B |
| ATOM | 3978 | CE2 | PHE | B | 159 | 22.118 | 19.118 | 24.322 | 1.00 | 56.20 | B |
| ATOM | 3979 | CZ | PHE | B | 159 | 21.519 | 18.874 | 25.547 | 1.00 | 56.88 | B |
| ATOM | 3980 | C | PHE | B | 159 | 24.909 | 22.360 | 24.874 | 1.00 | 55.46 | B |
| ATOM | 3981 | O | PHE | B | 159 | 25.089 | 21.560 | 25.790 | 1.00 | 54.64 | B |
| ATOM | 3982 | N | THR | B | 160 | 25.618 | 22.350 | 23.743 | 1.00 | 54.66 | B |
| ATOM | 3983 | CA | THR | B | 160 | 26.713 | 21.405 | 23.522 | 1.00 | 53.60 | B |
| ATOM | 3984 | CB | THR | B | 160 | 27.272 | 21.456 | 22.082 | 1.00 | 52.79 | B |
| ATOM | 3985 | OG1 | THR | B | 160 | 27.810 | 22.755 | 21.816 | 1.00 | 51.94 | B |
| ATOM | 3986 | CG2 | THR | B | 160 | 26.197 | 21.116 | 21.071 | 1.00 | 50.01 | B |
| ATOM | 3987 | C | THR | B | 160 | 27.845 | 21.723 | 24.482 | 1.00 | 54.09 | B |
| ATOM | 3988 | O | THR | B | 160 | 28.510 | 20.823 | 24.978 | 1.00 | 53.56 | B |
| ATOM | 3989 | N | TRP | B | 161 | 28.072 | 23.013 | 24.718 | 1.00 | 55.27 | B |
| ATOM | 3990 | CA | TRP | B | 161 | 29.105 | 23.462 | 25.643 | 1.00 | 56.72 | B |
| ATOM | 3991 | CB | TRP | B | 161 | 29.354 | 24.952 | 25.479 | 1.00 | 57.70 | B |
| ATOM | 3992 | CG | TRP | B | 161 | 30.131 | 25.218 | 24.283 | 1.00 | 60.02 | B |
| ATOM | 3993 | CD2 | TRP | B | 161 | 31.540 | 25.434 | 24.219 | 1.00 | 61.91 | B |
| ATOM | 3994 | CE2 | TRP | B | 161 | 31.879 | 25.559 | 22.859 | 1.00 | 62.64 | B |
| ATOM | 3995 | CE3 | TRP | B | 161 | 32.553 | 25.532 | 25.181 | 1.00 | 63.01 | B |
| ATOM | 3996 | CD1 | TRP | B | 161 | 29.679 | 25.229 | 23.004 | 1.00 | 60.79 | B |
| ATOM | 3997 | NE1 | TRP | B | 161 | 30.721 | 25.429 | 22.137 | 1.00 | 62.55 | B |
| ATOM | 3998 | CZ2 | TRP | B | 161 | 33.193 | 25.776 | 22.432 | 1.00 | 63.03 | B |
| ATOM | 3999 | CZ3 | TRP | B | 161 | 33.860 | 25.750 | 24.757 | 1.00 | 62.82 | B |
| ATOM | 4000 | CH2 | TRP | B | 161 | 34.166 | 25.868 | 23.393 | 1.00 | 62.68 | B |
| ATOM | 4001 | C | TRP | B | 161 | 28.694 | 23.173 | 27.069 | 1.00 | 57.31 | B |
| ATOM | 4002 | O | TRP | B | 161 | 29.521 | 22.811 | 27.895 | 1.00 | 56.86 | B |
| ATOM | 4003 | N | VAL | B | 162 | 27.407 | 23.354 | 27.351 | 1.00 | 58.66 | B |
| ATOM | 4004 | CA | VAL | B | 162 | 26.854 | 23.097 | 28.676 | 1.00 | 59.63 | B |
| ATOM | 4005 | CB | VAL | B | 162 | 25.346 | 23.409 | 28.719 | 1.00 | 59.58 | B |
| ATOM | 4006 | CG1 | VAL | B | 162 | 24.727 | 22.838 | 29.982 | 1.00 | 60.11 | B |
| ATOM | 4007 | CG2 | VAL | B | 162 | 25.125 | 24.912 | 28.660 | 1.00 | 59.16 | B |
| ATOM | 4008 | C | VAL | B | 162 | 27.062 | 21.633 | 29.022 | 1.00 | 59.85 | B |
| ATOM | 4009 | O | VAL | B | 162 | 27.735 | 21.309 | 30.000 | 1.00 | 59.86 | B |
| ATOM | 4010 | N | MET | B | 163 | 26.494 | 20.762 | 28.192 | 1.00 | 60.26 | B |
| ATOM | 4011 | CA | MET | B | 163 | 26.602 | 19.318 | 28.363 | 1.00 | 60.61 | B |
| ATOM | 4012 | CB | MET | B | 163 | 25.777 | 18.607 | 27.297 | 1.00 | 59.43 | B |
| ATOM | 4013 | CG | MET | B | 163 | 24.340 | 19.008 | 27.320 | 1.00 | 58.01 | B |
| ATOM | 4014 | SD | MET | B | 163 | 23.717 | 18.842 | 28.979 | 1.00 | 58.03 | B |
| ATOM | 4015 | CE | MET | B | 163 | 22.838 | 17.275 | 28.847 | 1.00 | 56.41 | B |
| ATOM | 4016 | C | MET | B | 163 | 28.050 | 18.828 | 28.312 | 1.00 | 61.41 | B |
| ATOM | 4017 | O | MET | B | 163 | 28.517 | 18.193 | 29.252 | 1.00 | 62.39 | B |
| ATOM | 4018 | N | ALA | B | 164 | 28.763 | 19.135 | 27.228 | 1.00 | 61.09 | B |
| ATOM | 4019 | CA | ALA | B | 164 | 30.158 | 18.717 | 27.092 | 1.00 | 59.69 | B |
| ATOM | 4020 | CB | ALA | B | 164 | 30.778 | 19.322 | 25.843 | 1.00 | 58.83 | B |
| ATOM | 4021 | C | ALA | B | 164 | 30.965 | 19.102 | 28.330 | 1.00 | 58.60 | B |
| ATOM | 4022 | O | ALA | B | 164 | 31.760 | 18.304 | 28.819 | 1.00 | 57.87 | B |
| ATOM | 4023 | N | LEU | B | 165 | 30.729 | 20.308 | 28.850 | 1.00 | 58.24 | B |
| ATOM | 4024 | CA | LEU | B | 165 | 31.427 | 20.781 | 30.047 | 1.00 | 58.33 | B |
| ATOM | 4025 | CB | LEU | B | 165 | 31.195 | 22.276 | 30.295 | 1.00 | 57.25 | B |
| ATOM | 4026 | CG | LEU | B | 165 | 32.096 | 23.257 | 29.537 | 1.00 | 56.84 | B |
| ATOM | 4027 | CD1 | LEU | B | 165 | 31.539 | 24.664 | 29.651 | 1.00 | 57.38 | B |
| ATOM | 4028 | CD2 | LEU | B | 165 | 33.518 | 23.199 | 30.064 | 1.00 | 55.20 | B |
| ATOM | 4029 | C | LEU | B | 165 | 31.018 | 19.996 | 31.280 | 1.00 | 58.61 | B |
| ATOM | 4030 | O | LEU | B | 165 | 31.788 | 19.901 | 32.230 | 1.00 | 58.81 | B |
| ATOM | 4031 | N | ALA | B | 166 | 29.812 | 19.431 | 31.258 | 1.00 | 58.81 | B |
| ATOM | 4032 | CA | ALA | B | 166 | 29.307 | 18.630 | 32.380 | 1.00 | 59.28 | B |
| ATOM | 4033 | CB | ALA | B | 166 | 27.793 | 18.433 | 32.254 | 1.00 | 58.78 | B |
| ATOM | 4034 | C | ALA | B | 166 | 30.012 | 17.268 | 32.466 | 1.00 | 59.29 | B |
| ATOM | 4035 | O | ALA | B | 166 | 29.825 | 16.516 | 33.431 | 1.00 | 59.22 | B |
| ATOM | 4036 | N | CYS | B | 167 | 30.822 | 16.963 | 31.450 | 1.00 | 58.64 | B |
| ATOM | 4037 | CA | CYS | B | 167 | 31.562 | 15.706 | 31.383 | 1.00 | 55.37 | B |
| ATOM | 4038 | CB | CYS | B | 167 | 31.273 | 14.982 | 30.074 | 1.00 | 55.43 | B |
| ATOM | 4039 | SG | CYS | B | 167 | 31.838 | 13.283 | 30.097 | 1.00 | 56.58 | B |
| ATOM | 4040 | C | CYS | B | 167 | 33.058 | 15.926 | 31.482 | 1.00 | 52.70 | B |
| ATOM | 4041 | O | CYS | B | 167 | 33.748 | 15.205 | 32.189 | 1.00 | 52.52 | B |
| ATOM | 4042 | N | ALA | B | 168 | 33.551 | 16.924 | 30.761 | 1.00 | 50.34 | B |
| ATOM | 4043 | CA | ALA | B | 168 | 34.971 | 17.233 | 30.749 | 1.00 | 48.72 | B |
| ATOM | 4044 | CB | ALA | B | 168 | 35.322 | 17.980 | 29.470 | 1.00 | 48.38 | B |
| ATOM | 4045 | C | ALA | B | 168 | 35.466 | 18.008 | 31.963 | 1.00 | 47.33 | B |
| ATOM | 4046 | O | ALA | B | 168 | 36.611 | 17.847 | 32.374 | 1.00 | 46.02 | B |
| ATOM | 4047 | N | ALA | B | 168 | 34.591 | 18.807 | 32.564 | 1.00 | 47.60 | B |
| ATOM | 4048 | CA | ALA | B | 169 | 34.972 | 19.643 | 33.709 | 1.00 | 48.37 | B |
| ATOM | 4049 | CB | ALA | B | 169 | 34.081 | 20.887 | 33.764 | 1.00 | 47.42 | B |
| ATOM | 4050 | C | ALA | B | 169 | 35.152 | 19.043 | 35.124 | 1.00 | 48.27 | B |
| ATOM | 4051 | O | ALA | B | 169 | 36.001 | 19.520 | 35.889 | 1.00 | 47.85 | B |
| ATOM | 4052 | N | PRO | B | 170 | 34.342 | 18.032 | 35.509 | 1.00 | 47.76 | B |
| ATOM | 4053 | CD | PRO | B | 170 | 33.096 | 17.575 | 34.863 | 1.00 | 47.21 | B |
| ATOM | 4054 | CA | PRO | B | 170 | 34.475 | 17.428 | 36.843 | 1.00 | 46.74 | B |
| ATOM | 4055 | CB | PRO | B | 170 | 33.372 | 16.373 | 36.842 | 1.00 | 46.38 | B |
| ATOM | 4056 | CG | PRO | B | 170 | 32.38 | 17.026 | 36.031 | 1.00 | 45.54 | B |
| ATOM | 4057 | C | PRO | B | 170 | 35.843 | 16.853 | 37.269 | 1.00 | 46.63 | B |
| ATOM | 4058 | O | PRO | B | 170 | 36.271 | 17.080 | 38.397 | 1.00 | 45.37 | B |
| ATOM | 4059 | N | PRO | B | 171 | 36.537 | 16.097 | 36.391 | 1.00 | 47.67 | B |
| ATOM | 4060 | CD | PRO | B | 171 | 36.104 | 15.603 | 35.071 | 1.00 | 48.29 | B |
| ATOM | 4061 | CA | PRO | B | 171 | 37.848 | 15.521 | 36.740 | 1.00 | 48.14 | B |
| ATOM | 4062 | CB | PRO | B | 171 | 38.245 | 14.788 | 35.464 | 1.00 | 47.84 | B |
| ATOM | 4063 | CG | PRO | B | 171 | 36.931 | 14.349 | 34.914 | 1.00 | 48.53 | B |
| ATOM | 4064 | C | PRO | B | 171 | 38.900 | 16.552 | 37.109 | 1.00 | 49.12 | B |
| ATOM | 4065 | O | PRO | B | 171 | 39.948 | 16.214 | 37.660 | 1.00 | 49.58 | B |
| ATOM | 4066 | N | LEU | B | 172 | 38.609 | 17.804 | 36.772 | 1.00 | 51.01 | B |
| ATOM | 4067 | CA | LEU | B | 172 | 39.491 | 18.939 | 37.032 | 1.00 | 53.26 | B |
| ATOM | 4068 | CB | LEU | B | 172 | 39.300 | 20.002 | 35.942 | 1.00 | 52.30 | B |
| ATOM | 4069 | CG | LEU | B | 172 | 39.620 | 19.648 | 34.487 | 1.00 | 51.06 | B |
| ATOM | 4070 | CD1 | LEU | B | 172 | 39.010 | 20.684 | 33.563 | 1.00 | 50.52 | B |
| ATOM | 4071 | CD2 | LEU | B | 172 | 41.122 | 19.558 | 34.293 | 1.00 | 49.55 | B |
| ATOM | 4072 | C | LEU | B | 172 | 39.190 | 19.570 | 38.388 | 1.00 | 54.85 | B |
| ATOM | 4073 | O | LEU | B | 172 | 39.913 | 20.456 | 38.846 | 1.00 | 55.44 | B |
| ATOM | 4074 | N | VAL | B | 173 | 38.102 | 19.126 | 39.010 | 1.00 | 57.06 | B |
| ATOM | 4075 | CA | VAL | B | 173 | 37.682 | 19.657 | 40.302 | 1.00 | 57.94 | B |
| ATOM | 4076 | CB | VAL | B | 173 | 36.258 | 20.273 | 40.215 | 1.00 | 56.97 | B |
| ATOM | 4077 | CG1 | VAL | B | 173 | 35.769 | 20.691 | 41.587 | 1.00 | 58.52 | B |
| ATOM | 4078 | CG2 | VAL | B | 173 | 36.271 | 21.476 | 39.285 | 1.00 | 56.63 | B |
| ATOM | 4079 | C | VAL | B | 173 | 37.758 | 18.620 | 41.424 | 1.00 | 58.50 | B |
| ATOM | 4080 | O | VAL | B | 173 | 38.498 | 18.817 | 42.390 | 1.00 | 60.34 | B |
| ATOM | 4081 | N | GLY | B | 174 | 37.022 | 17.517 | 41.295 | 1.00 | 57.60 | B |
| ATOM | 4082 | CA | GLY | B | 174 | 37.048 | 16.503 | 42.334 | 1.00 | 56.64 | B |
| ATOM | 4083 | C | GLY | B | 174 | 36.351 | 15.197 | 42.007 | 1.00 | 56.60 | B |
| ATOM | 4084 | O | GLY | B | 174 | 36.396 | 14.248 | 42.792 | 1.00 | 56.67 | B |
| ATOM | 4085 | N | TRP | B | 175 | 35.683 | 15.144 | 40.862 | 1.00 | 56.31 | B |
| ATOM | 4086 | CA | TRP | B | 175 | 34.992 | 13.930 | 40.459 | 1.00 | 56.36 | B |
| ATOM | 4087 | CB | TRP | B | 175 | 33.563 | 14.265 | 40.016 | 1.00 | 57.56 | B |
| ATOM | 4088 | CG | TRP | B | 175 | 32.620 | 13.113 | 40.132 | 1.00 | 59.30 | B |
| ATOM | 4089 | CD2 | TRP | B | 175 | 31.319 | 13.011 | 39.547 | 1.00 | 60.10 | B |
| ATOM | 4090 | CE2 | TRP | B | 175 | 30.813 | 11.732 | 39.876 | 1.00 | 60.83 | B |
| ATOM | 4091 | CE3 | TRP | B | 175 | 30.534 | 13.872 | 38.770 | 1.00 | 59.77 | B |
| ATOM | 4092 | CD1 | TRP | B | 175 | 32.843 | 11.929 | 40.783 | 1.00 | 59.81 | B |
| ATOM | 4093 | NE1 | TRP | B | 175 | 31.764 | 11.094 | 40.630 | 1.00 | 61.38 | B |
| ATOM | 4094 | CZ2 | TRP | B | 175 | 29.553 | 11.293 | 39.454 | 1.00 | 60.54 | B |
| ATOM | 4095 | CZ3 | TRP | B | 175 | 29.287 | 13.435 | 38.349 | 1.00 | 60.67 | B |
| ATOM | 4096 | CH2 | TRP | B | 175 | 28.808 | 12.155 | 38.694 | 1.00 | 60.78 | B |
| ATOM | 4097 | C | TRP | B | 175 | 35.814 | 13.279 | 39.341 | 1.00 | 55.38 | B |
| ATOM | 4098 | O | TRP | B | 175 | 35.861 | 13.778 | 38.220 | 1.00 | 57.12 | B |
| ATOM | 4099 | N | SER | B | 176 | 36.470 | 12.168 | 39.668 | 1.00 | 53.14 | B |
| ATOM | 4100 | CA | SER | B | 176 | 37.350 | 11.450 | 38.747 | 1.00 | 50.42 | B |
| ATOM | 4101 | CB | SER | B | 176 | 36.680 | 11.140 | 37.408 | 1.00 | 50.16 | B |
| ATOM | 4102 | OG | SER | B | 176 | 37.527 | 10.344 | 36.594 | 1.00 | 49.09 | B |
| ATOM | 4103 | C | SER | B | 176 | 38.594 | 12.295 | 38.520 | 1.00 | 49.46 | B |
| ATOM | 4104 | O | SER | B | 176 | 38.694 | 13.425 | 39.004 | 1.00 | 48.70 | B |
| ATOM | 4105 | N | ARG | B | 177 | 39.551 | 11.742 | 37.790 | 1.00 | 49.22 | B |
| ATOM | 4106 | CA | ARG | B | 177 | 40.798 | 12.448 | 37.521 | 1.00 | 48.37 | B |
| ATOM | 4107 | CB | ARG | B | 177 | 41.814 | 12.164 | 38.642 | 1.00 | 46.91 | B |
| ATOM | 4108 | CG | ARG | B | 177 | 42.065 | 10.684 | 38.926 | 1.00 | 46.91 | B |
| ATOM | 4109 | CD | ARG | B | 177 | 43.022 | 10.487 | 40.092 | 1.00 | 47.67 | B |
| ATOM | 4110 | NE | ARG | B | 177 | 43.436 | 9.090 | 40.236 | 1.00 | 48.53 | B |
| ATOM | 4111 | CZ | ARG | B | 177 | 44.368 | 8.666 | 41.086 | 1.00 | 49.45 | B |
| ATOM | 4112 | NH1 | ARG | B | 177 | 44.994 | 9.527 | 41.876 | 1.00 | 50.40 | B |
| ATOM | 4113 | NH2 | ARG | B | 177 | 44.663 | 7.377 | 41.164 | 1.00 | 50.73 | B |
| ATOM | 4114 | C | ARG | B | 177 | 41.370 | 12.038 | 36.174 | 1.00 | 47.50 | B |
| ATOM | 4115 | O | ARG | B | 177 | 41.135 | 10.919 | 35.711 | 1.00 | 48.13 | B |
| ATOM | 4116 | N | TYR | B | 178 | 42.027 | 12.975 | 35.499 | 1.00 | 46.23 | B |
| ATOM | 4117 | CA | TYR | B | 178 | 42.652 | 12.659 | 34.223 | 1.00 | 45.94 | B |
| ATOM | 4118 | CB | TYR | B | 178 | 42.860 | 13.912 | 33.374 | 1.00 | 44.57 | B |
| ATOM | 4119 | CG | TYR | B | 178 | 41.573 | 14.465 | 32.793 | 1.00 | 44.05 | B |
| ATOM | 4120 | CD1 | TYR | B | 178 | 40.736 | 13.667 | 32.016 | 1.00 | 42.98 | B |
| ATOM | 4121 | CE1 | TYR | B | 178 | 39.555 | 14.170 | 31.488 | 1.00 | 42.81 | B |
| ATOM | 4122 | CD2 | TYR | B | 178 | 41.187 | 15.788 | 33.024 | 1.00 | 44.15 | B |
| ATOM | 4123 | CE2 | TYR | B | 178 | 40.003 | 16.296 | 32.499 | 1.00 | 41.93 | B |
| ATOM | 4124 | CZ | TYR | B | 178 | 39.199 | 15.481 | 31.737 | 1.00 | 42.50 | B |
| ATOM | 4125 | OH | TYR | B | 178 | 38.030 | 15.972 | 31.228 | 1.00 | 44.24 | B |
| ATOM | 4126 | C | TYR | B | 178 | 43.967 | 11.995 | 34.615 | 1.00 | 46.85 | B |
| ATOM | 4127 | O | TYR | B | 178 | 44.707 | 12.491 | 35.477 | 1.00 | 47.37 | B |
| ATOM | 4128 | N | ILE | B | 179 | 44.211 | 10.830 | 34.028 | 1.00 | 46.80 | B |
| ATOM | 4129 | CA | ILE | B | 179 | 45.382 | 10.026 | 34.348 | 1.00 | 44.70 | B |
| ATOM | 4130 | CB | ILE | B | 179 | 44.928 | 8.889 | 35.321 | 1.00 | 42.92 | B |
| ATOM | 4131 | CG2 | ILE | B | 179 | 43.920 | 7.971 | 34.639 | 1.00 | 42.83 | B |
| ATOM | 4132 | CG1 | ILE | B | 179 | 46.111 | 8.139 | 35.918 | 1.00 | 41.23 | B |
| ATOM | 4133 | CD1 | ILE | B | 179 | 45.713 | 7.295 | 37.102 | 1.00 | 39.79 | B |
| ATOM | 4134 | C | ILE | B | 179 | 45.992 | 9.495 | 33.045 | 1.00 | 44.54 | B |
| ATOM | 4135 | O | ILE | B | 179 | 45.264 | 9.049 | 32.155 | 1.00 | 43.77 | B |
| ATOM | 4136 | N | PRO | B | 180 | 47.333 | 9.583 | 32.898 | 1.00 | 44.43 | B |
| ATOM | 4137 | CD | PRO | B | 180 | 48.308 | 10.041 | 33.903 | 1.00 | 45.33 | B |
| ATOM | 4138 | CA | PRO | B | 180 | 48.031 | 9.117 | 31.695 | 1.00 | 43.02 | B |
| ATOM | 4139 | CB | PRO | B | 180 | 49.507 | 9.230 | 32.087 | 1.00 | 44.26 | B |
| ATOM | 4140 | CG | PRO | B | 180 | 49.494 | 9.184 | 33.588 | 1.00 | 44.53 | B |
| ATOM | 4141 | C | PRO | B | 180 | 47.634 | 7.716 | 31.258 | 1.00 | 42.16 | B |
| ATOM | 4142 | O | PRO | B | 180 | 47.543 | 6.802 | 32.085 | 1.00 | 40.86 | B |
| ATOM | 4143 | N | GLU | B | 181 | 47.344 | 7.589 | 29.959 | 1.00 | 41.21 | B |
| ATOM | 4144 | CA | GLU | B | 181 | 46.911 | 6.334 | 29.347 | 1.00 | 40.51 | B |
| ATOM | 4145 | CB | GLU | B | 181 | 45.583 | 6.535 | 28.591 | 1.00 | 40.40 | B |
| ATOM | 4146 | CG | GLU | B | 181 | 44.364 | 6.877 | 29.465 | 1.00 | 41.68 | B |
| ATOM | 4147 | CD | GLU | B | 181 | 43.017 | 6.546 | 28.797 | 1.00 | 43.21 | B |
| ATOM | 4148 | OE1 | GLU | B | 181 | 42.499 | 7.370 | 28.000 | 1.00 | 41.17 | B |
| ATOM | 4149 | OE2 | GLU | B | 181 | 42.474 | 5.450 | 29.077 | 1.00 | 43.24 | B |
| ATOM | 4150 | C | GLU | B | 181 | 47.937 | 5.703 | 28.398 | 1.00 | 39.87 | B |
| ATOM | 4151 | O | GLU | B | 181 | 48.829 | 6.389 | 27.883 | 1.00 | 37.55 | B |
| ATOM | 4152 | N | GLY | B | 182 | 47.788 | 4.390 | 28.190 | 1.00 | 39.24 | B |
| ATOM | 4153 | CA | GLY | B | 182 | 48.653 | 3.632 | 27.298 | 1.00 | 38.68 | B |
| ATOM | 4154 | C | GLY | B | 182 | 50.139 | 3.768 | 27.552 | 1.00 | 39.01 | B |
| ATOM | 4155 | O | GLY | B | 182 | 50.660 | 3.242 | 28.532 | 1.00 | 39.43 | B |
| ATOM | 4156 | N | MET | B | 183 | 50.830 | 4.434 | 26.632 | 1.00 | 39.97 | B |
| ATOM | 4157 | CA | MET | B | 183 | 52.266 | 4.666 | 26.758 | 1.00 | 40.34 | B |
| ATOM | 4158 | CB | MET | B | 183 | 52.900 | 4.825 | 25.380 | 1.00 | 39.84 | B |
| ATOM | 4159 | CG | MET | B | 183 | 52.923 | 3.555 | 24.551 | 1.00 | 39.88 | B |
| ATOM | 4160 | SD | MET | B | 183 | 53.539 | 3.897 | 22.885 | 1.00 | 40.41 | B |
| ATOM | 4161 | CE | MET | B | 183 | 53.383 | 2.258 | 22.102 | 1.00 | 40.66 | B |
| ATOM | 4162 | C | MET | B | 183 | 52.558 | 5.910 | 27.609 | 1.00 | 40.79 | B |
| ATOM | 4163 | O | MET | B | 183 | 53.648 | 6.474 | 27.538 | 1.00 | 39.75 | B |
| ATOM | 4164 | N | GLN | B | 184 | 51.566 | 6.319 | 28.405 | 1.00 | 41.35 | B |
| ATOM | 4165 | CA | GLN | B | 184 | 51.645 | 7.481 | 29.297 | 1.00 | 40.52 | B |
| ATOM | 4166 | CB | GLN | B | 184 | 52.667 | 7.231 | 30.409 | 1.00 | 39.42 | B |
| ATOM | 4167 | CG | GLN | B | 184 | 52.609 | 5.842 | 31.019 | 1.00 | 38.43 | B |
| ATOM | 4168 | CD | GLN | B | 184 | 51.247 | 5.491 | 31.569 | 1.00 | 38.46 | B |
| ATOM | 4169 | OE1 | GLN | B | 184 | 50.838 | 5.993 | 32.616 | 1.00 | 40.67 | B |
| ATOM | 4170 | NE2 | GLN | B | 184 | 50.542 | 4.607 | 30.878 | 1.00 | 37.04 | B |
| ATOM | 4171 | C | GLN | B | 184 | 51.982 | 8.770 | 28.557 | 1.00 | 40.33 | B |
| ATOM | 41-72 | O | GLN | B | 184 | 52.861 | 9.518 | 28.973 | 1.00 | 40.26 | B |
| ATOM | 4173 | N | CYS | B | 185 | 51.260 | 9.022 | 27.469 | 1.00 | 42.60 | B |
| ATOM | 4174 | CA | CYS | B | 185 | 51.457 | 10.206 | 26.626 | 1.00 | 44.30 | B |
| ATOM | 4175 | CB | CYS | B | 185 | 52.122 | 9.816 | 25.307 | 1.00 | 45.15 | B |
| ATOM | 4176 | SG | CYS | B | 185 | 53.907 | 9.820 | 25.365 | 1.00 | 50.28 | B |
| ATOM | 4177 | C | CYS | B | 185 | 50.181 | 10.997 | 26.323 | 1.00 | 44.47 | B |
| ATOM | 4178 | O | CYS | B | 185 | 50.252 | 12.085 | 25.747 | 1.00 | 44.04 | B |
| ATOM | 4179 | N | SER | B | 186 | 49.028 | 10.413 | 26.657 | 1.00 | 44.87 | B |
| ATOM | 4180 | CA | SER | B | 186 | 47.705 | 11.028 | 26.472 | 1.00 | 44.16 | B |
| ATOM | 4181 | CB | SER | B | 186 | 46.956 | 10.398 | 25.285 | 1.00 | 41.83 | B |
| ATOM | 4182 | OG | SER | B | 186 | 46.726 | 9.007 | 25.461 | 1.00 | 39.43 | B |
| ATOM | 4183 | C | SER | B | 186 | 46.924 | 10.789 | 27.769 | 1.00 | 45.42 | B |
| ATOM | 4184 | O | SER | B | 186 | 47.147 | 9.773 | 28.437 | 1.00 | 46.77 | B |
| ATOM | 4185 | N | CYS | B | 187 | 46.049 | 11.726 | 28.148 | 1.00 | 45.09 | B |
| ATOM | 4186 | CA | CYS | B | 187 | 45.260 | 11.577 | 29.379 | 1.00 | 44.35 | B |
| ATOM | 4187 | C | CYS | B | 187 | 43.774 | 11.352 | 29.157 | 1.00 | 44.12 | B |
| ATOM | 4188 | O | CYS | B | 187 | 43.229 | 11.744 | 28.121 | 1.00 | 44.48 | B |
| ATOM | 4189 | CB | CYS | B | 187 | 45.493 | 12.747 | 30.352 | 1.00 | 43.77 | B |
| ATOM | 4190 | SG | CYS | B | 187 | 46.945 | 12.465 | 31.418 | 1.00 | 45.34 | B |
| ATOM | 4191 | N | GLY | B | 188 | 43.151 | 10.682 | 30.134 | 1.00 | 43.53 | B |
| ATOM | 4192 | CA | GLY | B | 188 | 41.728 | 10.372 | 30.098 | 1.00 | 42.46 | B |
| ATOM | 4193 | C | GLY | B | 188 | 41.175 | 9.996 | 31.466 | 1.00 | 42.57 | B |
| ATOM | 4194 | O | GLY | B | 188 | 41.933 | 9.856 | 32.424 | 1.00 | 42.76 | B |
| ATOM | 4195 | N | ILE | B | 189 | 39.854 | 9.820 | 31.554 | 1.00 | 43.21 | B |
| ATOM | 4196 | CA | ILE | B | 189 | 39.175 | 9.463 | 32.804 | 1.00 | 41.80 | B |
| ATOM | 4197 | CB | ILE | B | 189 | 37.639 | 9.287 | 32.592 | 1.00 | 41.60 | B |
| ATOM | 4198 | CG2 | ILE | B | 189 | 37.013 | 8.474 | 33.722 | 1.00 | 40.87 | B |
| ATOM | 4199 | CG1 | ILE | B | 189 | 36.954 | 10.658 | 32.538 | 1.00 | 40.52 | B |
| ATOM | 4200 | CD1 | ILE | B | 189 | 37.284 | 11.493 | 31.334 | 1.00 | 39.03 | B |
| ATOM | 4201 | C | ILE | B | 189 | 39.781 | 8.230 | 33.462 | 1.00 | 42.24 | B |
| ATOM | 4202 | O | ILE | B | 189 | 40.234 | 7.303 | 32.789 | 1.00 | 40.73 | B |
| ATOM | 4203 | N | ASP | B | 190 | 39.788 | 8.241 | 34.790 | 1.00 | 44.04 | B |
| ATOM | 4204 | CA | ASP | B | 190 | 40.370 | 7.162 | 35.574 | 1.00 | 46.15 | B |
| ATOM | 4205 | CB | ASP | B | 190 | 40.672 | 7.661 | 36.985 | 1.00 | 44.43 | B |
| ATOM | 4206 | CG | ASP | B | 190 | 41.630 | 6.766 | 37.719 | 1.00 | 45.23 | B |
| ATOM | 4207 | OD1 | ASP | B | 190 | 41.688 | 6.871 | 38.954 | 1.00 | 48.80 | B |
| ATOM | 4208 | OD2 | ASP | B | 190 | 42.331 | 5.958 | 37.076 | 1.00 | 45.03 | B |
| ATOM | 4209 | C | ASP | B | 190 | 39.566 | 5.861 | 35.616 | 1.00 | 47.95 | B |
| ATOM | 4210 | O | ASP | B | 190 | 38.871 | 5.563 | 36.598 | 1.00 | 49.71 | B |
| ATOM | 4211 | N | TYR | B | 191 | 39.680 | 5.082 | 34.543 | 1.00 | 48.65 | B |
| ATOM | 4212 | CA | TYR | B | 191 | 38.987 | 3.802 | 34.436 | 1.00 | 48.64 | B |
| ATOM | 4213 | CB | TYR | B | 191 | 38.917 | 3.330 | 32.967 | 1.00 | 46.89 | B |
| ATOM | 4214 | CG | TYR | B | 191 | 38.662 | 4.404 | 31.923 | 1.00 | 45.79 | B |
| ATOM | 4215 | CD1 | TYR | B | 191 | 37.435 | 5.046 | 31.839 | 1.00 | 46.76 | B |
| ATOM | 4216 | CE1 | TYR | B | 191 | 37.191 | 6.027 | 30.871 | 1.00 | 47.03 | B |
| ATOM | 4217 | CD2 | TYR | B | 191 | 39.648 | 4.766 | 31.008 | 1.00 | 45.22 | B |
| ATOM | 4218 | CE2 | TYR | B | 191 | 39.414 | 5.744 | 30.036 | 1.00 | 45.26 | B |
| ATOM | 4219 | C2 | TYR | B | 191 | 38.182 | 6.371 | 29.974 | 1.00 | 45.44 | B |
| ATOM | 4220 | OH | TYR | B | 191 | 37.929 | 7.346 | 29.031 | 1.00 | 43.89 | B |
| ATOM | 4221 | C | TYR | B | 191 | 39.816 | 2.787 | 35.217 | 1.00 | 50.06 | B |
| ATOM | 4222 | O | TYR | B | 191 | 39.291 | 1.810 | 35.753 | 1.00 | 50.55 | B |
| ATOM | 4223 | N | TYR | B | 192 | 41.111 | 3.080 | 35.309 | 1.00 | 51.23 | B |
| ATOM | 4224 | CA | TYR | B | 192 | 42.123 | 2.235 | 35.945 | 1.00 | 53.52 | B |
| ATOM | 4225 | CB | TYR | B | 192 | 43.508 | 2.812 | 35.617 | 1.00 | 52.90 | B |
| ATOM | 4226 | CG | TYR | B | 192 | 43.713 | 3.051 | 34.127 | 1.00 | 52.17 | B |
| ATOM | 4227 | CD1 | TYR | B | 192 | 44.092 | 2.006 | 33.272 | 1.00 | 50.77 | B |
| ATOM | 4228 | CE1 | TYR | B | 192 | 44.240 | 2.210, | 31.896 | 1.00 | 49.16 | B |
| ATOM | 4229 | CD2 | TYR | B | 192 | 43.491 | 4.309 | 33.566 | 1.00 | 51.18 | B |
| ATOM | 4230 | CE2 | TYR | B | 192 | 43.635 | 4.521 | 32.193 | 1.00 | 50.26 | B |
| ATOM | 4231 | CZ | TYR | B | 192 | 44.007 | 3.469 | 31.365 | 1.00 | 49.81 | B |
| ATOM | 4232 | OH | TYR | B | 192 | 44.139 | 3.681 | 30.010 | 1.00 | 47.02 | B |
| ATOM | 4233 | C | TYR | B | 192 | 42.029 | 1.873 | 37.436 | 1.00 | 55.19 | B |
| ATOM | 4234 | O | TYR | B | 192 | 42.268 | 0.712 | 37.811 | 1.00 | 55.26 | B |
| ATOM | 4235 | N | THR | B | 193 | 41.717 | 2.854 | 38.284 | 1.00 | 56.39 | B |
| ATOM | 4236 | CA | THR | B | 193 | 41.616 | 2.613 | 39.723 | 1.00 | 56.95 | B |
| ATOM | 4237 | CB | THR | B | 193 | 42.753 | 3.304 | 40.489 | 1.00 | 53.89 | B |
| ATOM | 4238 | OG1 | THR | B | 193 | 42.602 | 4.723 | 40.396 | 1.00 | 49.79 | B |
| ATOM | 4239 | CG2 | THR | B | 193 | 44.090 | 2.901 | 39.917 | 1.00 | 52.25 | B |
| ATOM | 4240 | C | THR | B | 193 | 40.287 | 3.050 | 40.342 | 1.00 | 60.85 | B |
| ATOM | 4241 | O | THR | B | 193 | 39.572 | 3.895 | 39.790 | 1.00 | 61.01 | B |
| ATOM | 4242 | N | PRO | B | 194 | 39.915 | 2.429 | 41.480 | 1.00 | 64.06 | B |
| ATOM | 4243 | CD | PRO | B | 194 | 40.555 | 1.251 | 42.100 | 1.00 | 65.86 | B |
| ATOM | 4244 | CA | PRO | B | 194 | 38.668 | 2.760 | 42.176 | 1.00 | 64.52 | B |
| ATOM | 4245 | CB | PRO | B | 194 | 38.528 | 1.623 | 43.202 | 1.00 | 65.79 | B |
| ATOM | 4246 | CG | PRO | B | 194 | 39.367 | 0.502 | 42.643 | 1.00 | 66.14 | B |
| ATOM | 4247 | C | PRO | B | 194 | 38.822 | 4.091 | 42.899 | 1.00 | 64.24 | B |
| ATOM | 4248 | O | PRO | B | 194 | 37.832 | 4.793 | 43.116 | 1.00 | 64.07 | B |
| ATOM | 4249 | N | HIS | B | 195 | 40.074 | 4.410 | 43.254 | 1.00 | 63.80 | B |
| ATOM | 4250 | CA | HIS | B | 195 | 40.468 | 5.619 | 43.990 | 1.00 | 63.97 | B |
| ATOM | 4251 | CB | HIS | B | 195 | 40.964 | 6.726 | 43.046 | 1.00 | 60.74 | B |
| ATOM | 4252 | CG | HIS | B | 195 | 41.792 | 7.780 | 43.726 | 1.00 | 57.89 | B |
| ATOM | 4253 | CD2 | HIS | B | 195 | 42.959 | 7.695 | 44.409 | 1.00 | 57.54 | B |
| ATOM | 4254 | ND1 | HIS | B | 195 | 41.438 | 9.112 | 43.746 | 1.00 | 56.68 | B |
| ATOM | 4255 | CE1 | HIS | B | 195 | 42.347 | 9.801 | 44.413 | 1.00 | 54.90 | B |
| ATOM | 4256 | NE2 | HIS | B | 195 | 43.281 | 8.965 | 44.826 | 1.00 | 55.47 | B |
| ATOM | 4257 | C | HIS | B | 195 | 39.341 | 6.126 | 44.878 | 1.00 | 65.97 | B |
| ATOM | 4258 | O | HIS | B | 195 | 38.519 | 6.936 | 44.445 | 1.00 | 66.06 | B |
| ATOM | 4259 | N | GLU | B | 196 | 39.278 | 5.587 | 46.096 | 1.00 | 68.45 | B |
| ATOM | 4260 | CA | GLU | B | 196 | 38.252 | 5.947 | 47.082 | 1.00 | 69.90 | B |
| ATOM | 4261 | CB | GLU | B | 196 | 38.657 | 5.445 | 48.487 | 1.00 | 71.96 | B |
| ATOM | 4262 | CG | GLU | B | 196 | 38.857 | 3.927 | 48.643 | 1.00 | 73.01 | B |
| ATOM | 4263 | CD | GLU | B | 196 | 37.624 | 3.206 | 49.168 | 1.00 | 73.64 | B |
| ATOM | 4264 | OE1 | GLU | B | 196 | 37.008 | 3.699 | 50.142 | 1.00 | 73.54 | B |
| ATOM | 4265 | OE2 | GLU | B | 196 | 37.284 | 2.134 | 48.617 | 1.00 | 74.06 | B |
| ATOM | 4266 | C | GLU | B | 196 | 38.007 | 7.462 | 47.144 | 1.00 | 69.54 | B |
| ATOM | 4267 | O | GLU | B | 196 | 36.871 | 7.917 | 47.016 | 1.00 | 68.88 | B |
| ATOM | 4268 | N | GLU | B | 197 | 39.097 | 8.224 | 47.270 | 1.00 | 69.83 | B |
| ATOM | 4269 | CA | GLU | B | 197 | 39.087 | 9.690 | 47.386 | 1.00 | 69.78 | B |
| ATOM | 4270 | CB | GLU | B | 197 | 40.530 | 10.213 | 47.481 | 1.00 | 70.97 | B |
| ATOM | 4271 | CG | GLU | B | 197 | 41.253 | 9.826 | 48.777 | 1.00 | 73.85 | B |
| ATOM | 4272 | CD | GLU | B | 197 | 42.538 | 9.037 | 48.545 | 1.00 | 76.18 | B |
| ATOM | 4273 | OE1 | GLU | B | 197 | 43.625 | 9.596 | 48.810 | 1.00 | 77.41 | B |
| ATOM | 4274 | OE2 | GLU | B | 197 | 42.465 | 7.860 | 48.115 | 1.00 | 76.60 | B |
| ATOM | 4275 | C | GLU | B | 197 | 38.282 | 10.539 | 46.385 | 1.00 | 69.42 | B |
| ATOM | 4276 | O | GLU | B | 197 | 37.903 | 11.666 | 46.704 | 1.00 | 68.51 | B |
| ATOM | 4277 | N | THR | B | 198 | 38.025 | 10.017 | 45.187 | 1.00 | 69.76 | B |
| ATOM | 4278 | CA | THR | B | 198 | 37.262 | 10.752 | 44.169 | 1.00 | 68.94 | B |
| ATOM | 4279 | CB | THR | B | 198 | 38.176 | 11.191 | 42.967 | 1.00 | 67.00 | B |
| ATOM | 4280 | OG1 | THR | B | 198 | 38.737 | 10.040 | 42.320 | 1.00 | 66.04 | B |
| ATOM | 4281 | CG2 | THR | B | 198 | 39.314 | 12.074 | 43.443 | 1.00 | 64.47 | B |
| ATOM | 4282 | C | THR | B | 198 | 36.078 | 9.917 | 43.643 | 1.00 | 70.10 | B |
| ATOM | 4283 | O | THR | B | 198 | 35.083 | 10.456 | 43.253 | 1.00 | 70.11 | B |
| ATOM | 4284 | N | ASN | B | 199 | 36.232 | 8.598 | 43.729 | 1.00 | 10.89 | B |
| ATOM | 4285 | CA | ASN | B | 199 | 35.263 | 7.605 | 43.259 | 1.00 | 72.04 | B |
| ATOM | 4286 | CB | ASN | B | 199 | 33.934 | 7.606 | 44.050 | 1.00 | 72.71 | B |
| ATOM | 4287 | CG | ASN | B | 199 | 33.252 | 6.211 | 44.072 | 1.00 | 72.67 | B |
| ATOM | 4288 | OD1 | ASN | B | 199 | 32.023 | 6.098 | 44.129 | 1.00 | 71.02 | B |
| ATOM | 4289 | ND2 | ASN | B | 199 | 34.063 | 5.153 | 44.030 | 1.00 | 72.35 | B |
| ATOM | 4290 | C | ASN | B | 199 | 35.031 | 7.689 | 41.753 | 1.00 | 71.85 | B |
| ATOM | 4291 | O | ASN | B | 199 | 34.133 | 8.381 | 41.257 | 1.00 | 70.00 | B |
| ATOM | 4292 | N | ASN | B | 200 | 35.933 | 7.026 | 41.038 | 1.00 | 72.67 | B |
| ATOM | 4293 | CA | ASN | B | 200 | 35.883 | 6.937 | 39.590 | 1.00 | 73.08 | B |
| ATOM | 4294 | CB | ASN | B | 200 | 37.252 | 6.501 | 39.038 | 1.00 | 70.97 | B |
| ATOM | 4295 | CG | ASN | B | 200 | 38.426 | 7.112 | 39.810 | 1.00 | 67.38 | B |
| ATOM | 4296 | OD1 | ASN | B | 200 | 39.381 | 6.422 | 40.135 | 1.00 | 65.29 | B' |
| ATOM | 4297 | ND2 | ASN | B | 200 | 38.345 | 8.400 | 40.112 | 1.00 | 64.62 | B |
| ATOM | 4298 | C | ASN | B | 200 | 34.817 | 5.867 | 39.317 | 1.00 | 73.80 | B |
| ATOM | 4299 | O | ASN | B | 200 | 34.139 | 5.902 | 38.291 | 1.00 | 74.18 | B |
| ATOM | 4300 | N | GLU | B | 201 | 34.654 | 4.951 | 40.280 | 1.00 | 73.80 | B |
| ATOM | 4301 | CA | GLU | B | 201 | 33.667 | 3.874 | 40.214 | 1.00 | 73.19 | B |
| ATOM | 4302 | CB | GLU | B | 201 | 33.728 | 3.001 | 41.479 | 1.00 | 74.84 | B |
| ATOM | 4303 | CG | GLU | B | 201 | 34.719 | 1.825 | 41.469 | 1.00 | 76.21 | B |
| ATOM | 4304 | CD | GLU | B | 201 | 34.651 | 0.971 | 42.756 | 1.00 | 77.63 | B |
| ATOM | 4305 | OE1 | GLU | B | 201 | 33.708 | 1.147 | 43.561 | 1.00 | 77.96 | B |
| ATOM | 4306 | OE2 | GLU | B | 201 | 35.542 | 0.118 | 42.970 | 1.00 | 77.13 | B |
| ATOM | 4307 | C | GLU | B | 201 | 32.257 | 4.458 | 40.088 | 1.00 | 72.15 | B |
| ATOM | 4308 | O | GLU | B | 201 | 31.298 | 3.726 | 39.851 | 1.00 | 72.61 | B |
| ATOM | 4309 | N | SER | B | 202 | 32.131 | 5.768 | 40.287 | 1.00 | 70.83 | B |
| ATOM | 4310 | CA | SER | B | 202 | 30.839 | 6.440 | 40.187 | 1.00 | 69.39 | B |
| ATOM | 4311 | CB | SER | B | 202 | 30.520 | 7.219 | 41.461 | 1.00 | 70.07 | B |
| ATOM | 4312 | OG | SER | B | 202 | 31.289 | 8.407 | 41.530 | 1.00 | 70.02 | B |
| ATOM | 4313 | C | SER | B | 202 | 30.798 | 7.392 | 39.005 | 1.00 | 67.83 | B |
| ATOM | 4314 | O | SER | B | 202 | 29.732 | 7.611 | 38.431 | 1.00 | 69.25 | B |
| ATOM | 4315 | N | PHE | B | 203 | 31.938 | 7.997 | 38.671 | 1.00 | 65.32 | B |
| ATOM | 4316 | CA | PHE | B | 203 | 31.993 | 8.923 | 37.536 | 1.00 | 62.82 | B |
| ATOM | 4317 | CB | PHE | B | 203 | 33.238 | 9.807 | 37.585 | 1.00 | 60.78 | B |
| ATOM | 4318 | CG | PHE | B | 203 | 33.341 | 10.756 | 36.419 | 1.00 | 58.42 | B |
| ATOM | 4319 | CD1 | PHE | B | 203 | 33.909 | 10.350 | 35.220 | 1.00 | 57.15 | B |
| ATOM | 4320 | CD2 | PHE | B | 203 | 32.827 | 12.039 | 36.507 | 1.00 | 56.92 | B |
| ATOM | 4321 | CE1 | PHE | B | 203 | 33.954 | 11.203 | 34.137 | 1.00 | 56.13 | B |
| ATOM | 4322 | CE2 | PHE | B | 203 | 32.871 | 12.895 | 35.425 | 1.00 | 55.40 | B |
| ATOM | 4323 | CZ | PHE | B | 203 | 33.433 | 12.476 | 34.240 | 1.00 | 55.37 | B |
| ATOM | 4324 | C | PHE | B | 203 | 31.959 | 6.199 | 36.195 | 1.00 | 62.08 | B |
| ATOM | 4325 | O | PHE | B | 203 | 31.323 | 8.657 | 35.249 | 1.00 | 61.06 | B |
| ATOM | 4326 | N | VAL | B | 204 | 32.701 | 7.101 | 36.108 | 1.00 | 61.78 | B |
| ATOM | 4327 | CA | VAL | B | 204 | 32.764 | 6.305 | 34.890 | 1.00 | 61.46 | B |
| ATOM | 4328 | CB | VAL | B | 204 | 33.863 | 5.221 | 35.000 | 1.00 | 59.71 | B |
| ATOM | 4329 | CG1 | VAL | B | 204 | 34.076 | 4.547 | 33.663 | 1.00 | 59.13 | B |
| ATOM | 4330 | CG2 | VAL | B | 204 | 35.162 | 5.840 | 35.500 | 1.00 | 57.48 | B |
| ATOM | 4331 | C | VAL | B | 204 | 31.396 | 5.675 | 34.578 | 1.00 | 62.44 | B |
| ATOM | 4332 | O | VAL | B | 204 | 31.104 | 5.333 | 33.431 | 1.00 | 62.70 | B |
| ATOM | 4333 | N | ILE | B | 205 | 30.557 | 5.530 | 35.600 | 1.00 | 63.14 | B |
| ATOM | 4334 | CA | ILE | B | 205 | 29.222 | 4.976 | 35.404 | 1.00 | 64.37 | B |
| ATOM | 4335 | CB | ILE | B | 205 | 28.697 | 4.278 | 36.679 | 1.00 | 64.39 | B |
| ATOM | 4336 | CG2 | ILE | B | 205 | 27.216 | 3.926 | 36.526 | 1.00 | 64.34 | B |
| ATOM | 4337 | CG1 | ILE | B | 205 | 29.531 | 3.019 | 36.949 | 1.00 | 65.00 | B |
| ATOM | 4338 | CD1 | ILE | B | 205 | 29.058 | 2.172 | 38.119 | 1.00 | 65.93 | B |
| ATOM | 4339 | C | ILE | B | 205 | 28.278 | 6.097 | 34.974 | 1.00 | 65.49 | B |
| ATOM | 4340 | O | ILE | B | 205 | 27.338 | 5.869 | 34.217 | 1.00 | 66.16 | B |
| ATOM | 4341 | N | TYR | B | 206 | 28.560 | 7.312 | 35.441 | 1.00 | 66.08 | B |
| ATOM | 4342 | CA | TYR | B | 206 | 27.766 | 8.495 | 35.110 | 1.00 | 66.83 | B |
| ATOM | 4343 | CB | TYR | B | 206 | 27.994 | 9.589 | 36.165 | 1.00 | 66.69 | B |
| ATOM | 4344 | CG | TYR | B | 206 | 27.996 | 10.996 | 35.607 | 1.00 | 66.80 | B |
| ATOM | 4345 | CD1 | TYR | B | 206 | 29.184 | 11.601 | 35.197 | 1.00 | 66.93 | B |
| ATOM | 4346 | CE1 | TYR | B | 206 | 29.192 | 12.876 | 34.645 | 1.00 | 67.38 | B |
| ATOM | 4347 | CD2 | TYR | B | 206 | 26.811 | 11.708 | 35.455 | 1.00 | 67.66 | B |
| ATOM | 4348 | CE2 | TYR | B | 206 | 26.807 | 12.987 | 34.904 | 1.00 | 67.93 | B |
| ATOM | 4349 | CZ | TYR | B | 206 | 28.001 | 13.564 | 34.500 | 1.00 | 67.51 | B |
| ATOM | 4350 | OH | TYR | B | 206 | 27.999 | 14.823 | 33.944 | 1.00 | 66.55 | B |
| ATOM | 4351 | C | TYR | B | 206 | 28.089 | 9.036 | 33.709 | 1.00 | 67.37 | B |
| ATOM | 4352 | O | TYR | B | 206 | 27.204 | 9.526 | 33.004 | 1.00 | 67.33 | B |
| ATOM | 4353 | N | MET | B | 207 | 29.368 | 8.999 | 33.343 | 1.00 | 67.98 | B |
| ATOM | 4354 | CA | MET | B | 207 | 29.821 | 9.484 | 32.042 | 1.00 | 67.79 | B |
| ATOM | 4355 | CB | MET | B | 207 | 31.349 | 9.602 | 32.013 | 1.00 | 68.72 | B |
| ATOM | 4356 | CG | MET | B | 207 | 31.910 | 10.142 | 30.704 | 1.00 | 69.21 | B |
| ATOM | 4357 | SD | MET | B | 207 | 33.713 | 10.198 | 30.648 | 1.00 | 69.17 | B |
| ATOM | 4358 | CE | MET | B | 207 | 34.094 | 8.447 | 30.351 | 1.00 | 68.41 | B |
| ATOM | 4359 | C | MET | B | 207 | 29.348 | 8.553 | 30.934 | 1.00 | 67.24 | B |
| ATOM | 4360 | O | MET | B | 207 | 28.945 | 9.006 | 29.866 | 1.00 | 67.29 | B |
| ATOM | 4361 | N | PHE | B | 208 | 29.399 | 7.251 | 31.189 | 1.00 | 66.80 | B |
| ATOM | 4362 | CA | PHE | B | 208 | 28.953 | 6.283 | 30.203 | 1.00 | 67.07 | B |
| ATOM | 4363 | CB | PHE | B | 208 | 29.483 | 4.890 | 30.529 | 1.00 | 67.24 | B |
| ATOM | 4364 | CG | PHE | B | 208 | 30.848 | 4.627 | 29.971 | 1.00 | 68.87 | B |
| ATOM | 4365 | CD1 | PHE | B | 208 | 31.039 | 3.651 | 28.997 | 1.00 | 69.66 | B |
| ATOM | 4366 | CD2 | PHE | B | 208 | 31.944 | 5.378 | 30.390 | 1.00 | 69.70 | B |
| ATOM | 4367 | CE1 | PHE | B | 208 | 32.298 | 3.427 | 28.448 | 1.00 | 68.53 | B |
| ATOM | 4368 | CE2 | PHE | B | 208 | 33.208 | 5.160 | 29.846 | 1.00 | 68.94 | B |
| ATOM | 4369 | CZ | PHE | B | 208 | 33.382 | 4.184 | 28.875 | 1.00 | 68.87 | B |
| ATOM | 4370 | C | PHE | B | 208 | 27.437 | 6.264 | 30.045 | 1.00 | 67.34 | B |
| ATOM | 4371 | O | PHE | B | 208 | 26.916 | 5.614 | 29.144 | 1.00 | 66.92 | B |
| ATOM | 4372 | N | VAL | B | 209 | 26.731 | 6.979 | 30.921 | 1.00 | 67.20 | B |
| ATOM | 4373 | CA | VAL | B | 209 | 25.276 | 7.044 | 30.838 | 1.00 | 66.20 | B |
| ATOM | 4374 | CB | VAL | B | 209 | 24.574 | 6.616 | 32.161 | 1.00 | 67.12 | B |
| ATOM | 4375 | CG1 | VAL | B | 209 | 24.915 | 5.167 | 32.500 | 1.00 | 66.84 | B |
| ATOM | 4376 | CG2 | VAL | B | 209 | 24.947 | 7.538 | 33.307 | 1.00 | 66.98 | B |
| ATOM | 4377 | C | VAL | B | 209 | 24.786 | 8.422 | 30.401 | 1.00 | 65.18 | B |
| ATOM | 4378 | O | VAL | B | 209 | 24.033 | 8.529 | 29.439 | 1.00 | 64.88 | B |
| ATOM | 4379 | N | VAL | B | 209 | 25.254 | 9.480 | 31.058 | 1.00 | 64.35 | B |
| ATOM | 4380 | CA | VAL | B | 210 | 24.817 | 10.829 | 30.702 | 1.00 | 64.49 | B |
| ATOM | 4381 | CB | VAL | B | 210 | 24.819 | 11.780 | 31.925 | 1.00 | 64.11 | B |
| ATOM | 4382 | CG1 | VAL | B | 210 | 24.151 | 13.104 | 31.569 | 1.00 | 64.03 | B |
| ATOM | 4383 | CG2 | VAL | B | 210 | 24.085 | 11.142 | 33.087 | 1.00 | 64.99 | B |
| ATOM | 4384 | C | VAL | B | 210 | 25.609 | 11.470 | 29.560 | 1.00 | 63.87 | B |
| ATOM | 4385 | O | VAL | B | 210 | 25.180 | 12.475 | 28.993 | 1.00 | 64.51 | B |
| ATOM | 4386 | N | HIS | B | 211 | 26.741 | 10.878 | 29.194 | 1.00 | 62.79 | B |
| ATOM | 4387 | CA | HIS | B | 211 | 27.558 | 11.441 | 28.121 | 1.00 | 61.21 | B |
| ATOM | 4388 | CB | HIS | B | 211 | 28.770 | 12.167 | 28.712 | 1.00 | 59.84 | B |
| ATOM | 4389 | CG | HIS | B | 211 | 28.422 | 13.438 | 29.422 | 1.00 | 57.51 | B |
| ATOM | 4390 | CD2 | HIS | B | 211 | 28.250 | 13.706 | 30.738 | 1.00 | 56.91 | B |
| ATOM | 4391 | ND1 | HIS | B | 211 | 28.194 | 14.622 | 28.755 | 1.00 | 56.23 | B |
| ATOM | 4392 | CE1 | HIS | B | 211 | 27.892 | 15.563 | 29.631 | 1.00 | 56.04 | B |
| ATOM | 4393 | NE2 | HIS | B | 211 | 27.921 | 15.035 | 30.841 | 1.00 | 54.69 | B |
| ATOM | 4394 | C | HIS | B | 211 | 28.005 | 10.446 | 27.054 | 1.00 | 60.42 | B |
| ATOM | 4395 | O | HIS | B | 211 | 28.984 | 10.693 | 26.344 | 1.00 | 59.14 | B |
| ATOM | 4396 | N | PHE | B | 212 | 27.270 | 9.343 | 26.924 | 1.00 | 59.96 | B |
| ATOM | 4397 | CA | PHE | B | 212 | 27.595 | 8.323 | 25.932 | 1.00 | 60.62 | B |
| ATOM | 4398 | CB | PHE | B | 212 | 28.672 | 7.363 | 26.468 | 1.00 | 58.17 | B |
| ATOM | 4399 | CG | PHE | B | 212 | 29.078 | 6.293 | 25.484 | 1.00 | 55.88 | B |
| ATOM | 4400 | CD1 | PHE | B | 212 | 29.390 | 6.617 | 24.168 | 1.00 | 55.24 | B |
| ATOM | 4401 | CD2 | PHE | B | 212 | 29.115 | 4.957 | 25.867 | 1.00 | 55.65 | B |
| ATOM | 4402 | CE1 | PHE | B | 212 | 29.730 | 5.628 | 23.245 | 1.00 | 55.07 | B |
| ATOM | 4403 | CE2 | PHE | B | 212 | 29.454 | 3.959 | 24.951 | 1.00 | 55.06 | B |
| ATOM | 4404 | CZ | PHE | B | 212 | 29.761 | 4.298 | 23.637 | 1.00 | 54.41 | B |
| ATOM | 4405 | C | PHE | B | 212 | 26.381 | 7.528 | 25.450 | 1.00 | 61.88 | B |
| ATOM | 4406 | O | PHE | B | 212 | 25.938 | 7.679 | 24.307 | 1.00 | 60.63 | B |
| ATOM | 4407 | N | ILE | B | 213 | 25.864 | 6.674 | 26.327 | 1.00 | 64.65 | B |
| ATOM | 4408 | CA | ILE | B | 213 | 24.723 | 5.822 | 26.013 | 1.00 | 67.89 | B |
| ATOM | 4409 | CB | ILE | B | 213 | 24.411 | 4.859 | 27.190 | 1.00 | 69.62 | B |
| ATOM | 4410 | CG2 | ILE | B | 213 | 23.101 | 4.128 | 26.944 | 1.00 | 70.30 | B |
| ATOM | 4411 | CG1 | ILE | B | 213 | 25.550 | 3.842 | 27.351 | 1.00 | 71.80 | B |
| ATOM | 4412 | CD1 | ILE | B | 213 | 25.462 | 2.972 | 28.613 | 1.00 | 73.77 | B |
| ATOM | 4413 | C | ILE | B | 213 | 23.469 | 6.598 | 25.616 | 1.00 | 68.84 | B |
| ATOM | 4414 | O | ILE | B | 213 | 22.976 | 6.441 | 24.499 | 1.00 | 69.55 | B |
| ATOM | 4415 | N | ILE | B | 214 | 22.966 | 7.437 | 26.522 | 1.00 | 69.74 | B |
| ATOM | 4416 | CA | ILE | B | 214 | 21.766 | 8.232 | 26.261 | 1.00 | 70.08 | B |
| ATOM | 4417 | CB | ILE | B | 214 | 21.402 | 9.158 | 27.453 | 1.00 | 71.06 | B |
| ATOM | 4418 | CG2 | ILE | B | 214 | 20.205 | 10.046 | 27.096 | 1.00 | 70.96 | B |
| ATOM | 4419 | CG1 | ILE | B | 214 | 21.090 | 8.324 | 28.696 | 1.00 | 71.62 | B |
| ATOM | 4420 | CD1 | ILE | B | 214 | 20.840 | 9.154 | 29.943 | 1.00 | 72.73 | B |
| ATOM | 4421 | C | ILE | B | 214 | 21.886 | 9.065 | 24.988 | 1.00 | 69.85 | B |
| ATOM | 4422 | O | ILE | B | 214 | 21.049 | 8.944 | 24.100 | 1.00 | 70.12 | B |
| ATOM | 4423 | N | PRO | B | 215 | 22.930 | 9.913 | 24.878 | 1.00 | 69.94 | B |
| ATOM | 4424 | CD | PRO | B | 215 | 24.029 | 10.187 | 25.823 | 1.00 | 70.48 | B |
| ATOM | 4425 | CA | PRO | B | 215 | 23.082 | 10.728 | 23.672 | 1.00 | 70.21 | B |
| ATOM | 4426 | CB | PRO | B | 215 | 24.488 | 11.301 | 23.828 | 1.00 | 69.96 | B |
| ATOM | 4427 | CG | PRO | B | 215 | 24.602 | 11.472 | 25.288 | 1.00 | 70.00 | B |
| ATOM | 4428 | C | PRO | B | 215 | 22.962 | 9.889 | 22.404 | 1.00 | 70.39 | B |
| ATOM | 4429 | O | PRO | B | 215 | 22.239 | 10.257 | 21.479 | 1.00 | 71.18 | B |
| ATOM | 4430 | N | LEU | B | 216 | 23.614 | 8.730 | 22.394 | 1.00 | 69.89 | B |
| ATOM | 4431 | CA | LEU | B | 216 | 23.570 | 7.855 | 21.232 | 1.00 | 69.96 | B |
| ATOM | 4432 | CB | LEU | B | 216 | 24.607 | 6.746 | 21.353 | 1.00 | 69.42 | B |
| ATOM | 4433 | CG | LEU | B | 216 | 26.039 | 7.226 | 21.139 | 1.00 | 68.49 | B |
| ATOM | 4434 | CD1 | LEU | B | 216 | 26.957 | 6.067 | 21.394 | 1.00 | 68.72 | B |
| ATOM | 4435 | CD2 | LEU | B | 216 | 26.228 | 7.767 | 19.724 | 1.00 | 67.13 | B |
| ATOM | 4436 | C | LEU | B | 216 | 22.195 | 7.277 | 20.911 | 1.00 | 70.30 | B |
| ATOM | 4437 | O | LEU | B | 216 | 21.849 | 7.130 | 19.743 | 1.00 | 71.51 | B |
| ATOM | 4438 | N | ILE | B | 217 | 21.402 | 6.964 | 21.930 | 1.00 | 70.58 | B |
| ATOM | 4439 | CA | ILE | B | 217 | 20.068 | 6.423 | 21.684 | 1.00 | 70.87 | B |
| ATOM | 4440 | CB | ILE | B | 217 | 19.420 | 5.863 | 22.973 | 1.00 | 70.86 | B |
| ATOM | 4441 | CG2 | ILE | B | 217 | 18.092 | 5.190 | 22.641 | 1.00 | 70.67 | B |
| ATOM | 4442 | CG1 | ILE | B | 217 | 20.348 | 4.830 | 23.625 | 1.00 | 70.97 | B |
| ATOM | 4443 | CD1 | ILE | B | 217 | 19.835 | 4.273 | 24.941 | 1.00 | 68.75 | B |
| ATOM | 4444 | C | ILE | B | 217 | 19.170 | 7.499 | 21.062 | 1.00 | 71.48 | B |
| ATOM | 4445 | O | ILE | B | 217 | 18.358 | 7.199 | 20.193 | 1.00 | 71.18 | B |
| ATOM | 4446 | N | VAL | B | 218 | 19.344 | 8.749 | 21.496 | 1.00 | 72.73 | B |
| ATOM | 4447 | CA | VAL | B | 218 | 18.577 | 9.888 | 20.978 | 1.00 | 73.59 | B |
| ATOM | 4448 | CB | VAL | B | 218 | 18.894 | 11.185 | 21.760 | 1.00 | 72.18 | B |
| ATOM | 4449 | CG1 | VAL | B | 218 | 18.126 | 12.348 | 21.180 | 1.00 | 71.58 | B |
| ATOM | 4450 | CG2 | VAL | B | 218 | 18.554 | 11.019 | 23.229 | 1.00 | 70.55 | B |
| ATOM | 4451 | C | VAL | B | 218 | 18.930 | 10.091 | 19.500 | 1.00 | 75.95 | B |
| ATOM | 4452 | O | VAL | B | 218 | 18.125 | 10.600 | 18.715 | 1.00 | 75.94 | B |
| ATOM | 4453 | N | ILE | B | 219 | 20.160 | 9.721 | 19.149 | 1.00 | 78.76 | B |
| ATOM | 4454 | CA | ILE | B | 219 | 20.648 | 9.798 | 17.775 | 1.00 | 81.61 | B |
| ATOM | 4455 | CB | ILE | B | 219 | 22.209 | 9.757 | 17.718 | 1.00 | 80.62 | B |
| ATOM | 4456 | CG2 | ILE | B | 219 | 22.705 | 9.294 | 16.355 | 1.00 | 79.82 | B |
| ATOM | 4457 | CG1 | ILE | B | 219 | 22.789 | 11.135 | 18.011 | 1.00 | 80.49 | B |
| ATOM | 4458 | CD1 | ILE | B | 219 | 24.302 | 11.155 | 18.027 | 1.00 | 80.71 | B |
| ATOM | 4459 | C | ILE | B | 219 | 20.086 | 8.593 | 17.020 | 1.00 | 84.09 | B |
| ATOM | 4460 | O | ILE | B | 219 | 19.982 | 8.614 | 15.796 | 1.00 | 85.12 | B |
| ATOM | 4461 | N | PHE | B | 220 | 19.706 | 7.556 | 17.763 | 1.00 | 86.68 | B |
| ATOM | 4462 | CA | PHE | B | 220 | 19.165 | 6.336 | 17.175 | 1.00 | 90.16 | B |
| ATOM | 4463 | CB | PHE | B | 220 | 19.569 | 5.122 | 18.014 | 1.00 | 91.50 | B |
| ATOM | 4464 | CG | PHE | B | 220 | 21.040 | 4.801 | 17.962 | 1.00 | 92.44 | B |
| ATOM | 4465 | CD1 | PHE | B | 220 | 21.672 | 4.208 | 19.057 | 1.00 | 92.69 | B |
| ATOM | 4466 | CD2 | PHE | B | 220 | 21.791 | 5.079 | 16.821 | 1.00 | 92.52 | B |
| ATOM | 4467 | CE1 | PHE | B | 220 | 23.030 | 3.896 | 19.017 | 1.00 | 93.15 | B |
| ATOM | 4468 | CE2 | PHE | B | 220 | 23.149 | 4.772 | 16.768 | 1.00 | 93.21 | B |
| ATOM | 4469 | CZ | PHE | B | 220 | 23.772 | 4.179 | 17.869 | 1.00 | 93.66 | B |
| ATOM | 4470 | C | PHE | B | 220 | 17.653 | 6.315 | 16.932 | 1.00 | 92.20 | B |
| ATOM | 4471 | O | PHE | B | 220 | 17.213 | 5.894 | 15.863 | 1.00 | 92.11 | B |
| ATOM | 4472 | N | PHE | B | 221 | 16.859 | 6.734 | 17.920 | 1.00 | 94.69 | B |
| ATOM | 4473 | CA | PHE | B | 221 | 15.398 | 6.737 | 17.774 | 1.00 | 96.69 | B |
| ATOM | 4474 | CB | PHE | B | 221 | 14.697 | 6.878 | 19.139 | 1.00 | 99.21 | B |
| ATOM | 4475 | CG | PHE | B | 221 | 13.569 | 5.884 | 19.356 | 1.00 | 102.34 | B |
| ATOM | 4476 | CD1 | PHE | B | 221 | 13.762 | 4.517 | 19.119 | 1.00 | 102.92 | B |
| ATOM | 4477 | CD2 | PHE | B | 221 | 12.313 | 6.314 | 19.794 | 1.00 | 103.55 | B |
| ATOM | 4478 | CE1 | PHE | B | 221 | 12.718 | 3.591 | 19.315 | 1.00 | 103.51 | B |
| ATOM | 4479 | CE2 | PHE | B | 221 | 11.262 | 5.396 | 19.993 | 1.00 | 104.06 | B |
| ATOM | 4480 | CZ | PHE | B | 221 | 11.468 | 4.033 | 19.753 | 1.00 | 103.90 | B |
| ATOM | 4481 | C | PHE | B | 221 | 14.944 | 7.826 | 16.803 | 1.00 | 96.41 | B |
| ATOM | 4482 | O | PHE | B | 221 | 13.997 | 7.633 | 16.042 | 1.00 | 96.77 | B |
| ATOM | 4483 | N | CYS | B | 222 | 15.628 | 8.965 | 16.829 | 1.00 | 95.98 | B |
| ATOM | 4484 | CA | CYS | B | 222 | 15.319 | 10.063 | 15.924 | 1.00 | 96.48 | B |
| ATOM | 4485 | CB | CYS | B | 222 | 16.182 | 11.276 | 16.269 | 1.00 | 95.74 | B |
| ATOM | 4486 | SG | CYS | B | 222 | 16.443 | 12.420 | 14.897 | 1.00 | 93.40 | B |
| ATOM | 4487 | C | CYS | B | 222 | 15.632 | 9.590 | 14.508 | 1.00 | 97.75 | B |
| ATOM | 4488 | O | CYS | B | 222 | 15.020 | 10.025 | 13.536 | 1.00 | 97.51 | B |
| ATOM | 4489 | N | TYR | B | 223 | 16.579 | 8.663 | 14.429 | 1.00 | 100.22 | B |
| ATOM | 4490 | CA | TYR | B | 223 | 17.051 | 8.071 | 13.183 | 1.00 | 103.15 | B |
| ATOM | 4491 | CB | TYR | B | 223 | 18.450 | 7.484 | 13.426 | 1.00 | 104.94 | B |
| ATOM | 4492 | CG | TYR | B | 223 | 19.210 | 6.977 | 12.217 | 1.00 | 106.85 | B |
| ATOM | 4493 | CD1 | TYR | B | 223 | 20.503 | 7.432 | 11.947 | 1.00 | 107.64 | B |
| ATOM | 4494 | CE1 | TYR | B | 223 | 21.233 | 6.925 | 10.878 | 1.00 | 108.95 | B |
| ATOM | 4495 | CD2 | TYR | B | 223 | 18.666 | 6.004 | 11.377 | 1.00 | 107.25 | B |
| ATOM | 4496 | CE2 | TYR | B | 223 | 19.384 | 5.493 | 10.307 | 1.00 | 108.27 | B |
| ATOM | 4497 | CZ | TYR | B | 223 | 20.664 | 5.954 | 10.063 | 1.00 | 109.52 | B |
| ATOM | 4498 | OH | TYR | B | 223 | 21.369 | 5.433 | 9.004 | 1.00 | 111.54 | B |
| ATOM | 4499 | C | TYR | B | 223 | 16.092 | 6.976 | 12.724 | 1.00 | 104.22 | B |
| ATOM | 4500 | O | TYR | B | 223 | 15.729 | 6.913 | 11.549 | 1.00 | 104.39 | B |
| ATOM | 4501 | N | GLY | B | 224 | 15.713 | 6.101 | 13.653 | 1.00 | 105.81 | B |
| ATOM | 4502 | CA | GLY | B | 224 | 14.802 | 5.010 | 13.341 | 1.00 | 107.61 | B |
| ATOM | 4503 | C | GLY | B | 224 | 13.440 | 5.507 | 12.898 | 1.00 | 108.64 | B |
| ATOM | 4504 | O | GLY | B | 224 | 12.804 | 4.909 | 12.021 | 1.00 | 108.96 | B |
| ATOM | 4505 | N | GLN | B | 225 | 12.988 | 6.595 | 13.524 | 1.00 | 108.97 | B |
| ATOM | 4506 | CA | GLN | B | 225 | 11.711 | 7.213 | 13.194 | 1.00 | 108.74 | B |
| ATOM | 4507 | CB | GLN | B | 225 | 11.149 | 7.978 | 14.403 | 1.00 | 109.09 | B |
| ATOM | 4508 | CG | GLN | B | 225 | 10.664 | 7.076 | 15.540 | 1.00 | 109.96 | B |
| ATOM | 4509 | CD | GLN | B | 225 | 10.049 | 7.852 | 16.695 | 1.00 | 110.13 | B |
| ATOM | 4510 | OE1 | GLN | B | 225 | 10.453 | 7.692 | 17.848 | 1.00 | 109.55 | B |
| ATOM | 4511 | NE2 | GLN | B | 225 | 9.060 | 8.689 | 16.391 | 1.00 | 110.13 | B |
| ATOM | 4512 | C | GLN | B | 225 | 11.900 | 8.147 | 11.996 | 1.00 | 108.39 | B |
| ATOM | 4513 | O | GLN | B | 225 | 11.876 | 9.372 | 12.139 | 1.00 | 108.54 | B |
| ATOM | 4514 | N | LEU | B | 226 | 12.141 | 7.541 | 10.832 | 1.00 | 107.96 | B |
| ATOM | 4515 | CA | LEU | B | 226 | 12.342 | 8.243 | 9.560 | 1.00 | 107.21 | B |
| ATOM | 4516 | CB | LEU | B | 226 | 13.767 | 8.810 | 9.448 | 1.00 | 106.23 | B |
| ATOM | 4517 | CG | LEU | B | 226 | 14.205 | 9.987 | 10.327 | 1.00 | 105.05 | B |
| ATOM | 4518 | CD1 | LEU | B | 226 | 15.650 | 10.320 | 10.025 | 1.00 | 105.81 | B |
| ATOM | 4519 | CD2 | LEU | B | 226 | 13.327 | 11.199 | 10.089 | 1.00 | 104.20 | B |
| ATOM | 4520 | C | LEU | B | 226 | 12.086 | 7.285 | 8.392 | 1.00 | 107.27 | B |
| ATOM | 4521 | O | LEU | B | 226 | 12.807 | 6.299 | 8.203 | 1.00 | 107.08 | B |
| ATOM | 4522 | N | GLN | B | 244 | 14.004 | 22.695 | -0.662 | 1.00 | 107.22 | B |
| ATOM | 4523 | CA | GLN | B | 244 | 12.922 | 21.827 | -0.213 | 1.00 | 106.85 | B |
| ATOM | 4524 | CB | GLN | B | 244 | 11.647 | 22.093 | -1.027 | 1.00 | 108.05 | B |
| ATOM | 4525 | CG | GLN | B | 244 | 10.483 | 21.152 | -0.691 | 1.00 | 108.58 | B |
| ATOM | 4526 | CD | GLN | B | 244 | 9.281 | 21.335 | -1.599 | 1.00 | 107.84 | B |
| ATOM | 4527 | OE1 | GLN | B | 244 | 9.307 | 20.948 | -2.768 | 1.00 | 107.03 | B |
| ATOM | 4528 | NE2 | GLN | B | 244 | 8.213 | 21.914 | -1.059 | 1.00 | 107.50 | B |
| ATOM | 4529 | C | GLN | B | 244 | 13.292 | 20.348 | -0.314 | 1.00 | 106.00 | B |
| ATOM | 4530 | O | GLN | B | 244 | 13.492 | 19.674 | 0.696 | 1.00 | 106.12 | B |
| ATOM | 4531 | N | LYS | B | 245 | 13.365 | 19.849 | -1.542 | 1.00 | 104.79 | B |
| ATOM | 4532 | CA | LYS | B | 245 | 13.691 | 18.454 | -1.788 | 1.00 | 103.81 | B |
| ATOM | 4533 | CB | LYS | B | 245 | 13.462 | 18.124 | -3.269 | 1.00 | 104.20 | B |
| ATOM | 4534 | CG | LYS | B | 245 | 12.173 | 18.681 | -3.886 | 1.00 | 103.65 | B |
| ATOM | 4535 | CD | LYS | B | 245 | 10.925 | 17.945 | -3.411 | 1.00 | 103.47 | B |
| ATOM | 4536 | CE | LYS | B | 245 | 9.692 | 18.417 | -4.173 | 1.00 | 102.09 | B |
| ATOM | 4537 | NZ | LYS | B | 245 | 8.448 | 17.703 | -3.776 | 1.00 | 101.09 | B |
| ATOM | 4538 | C | LYS | B | 245 | 15.146 | 18.146 | -1.418 | 1.00 | 103.22 | B |
| ATOM | 4539 | O | LYS | B | 245 | 15.459 | 17.031 | -1.002 | 1.00 | 103.31 | B |
| ATOM | 4540 | N | ALA | B | 246 | 16.014 | 19.154 | -1.537 | 1.00 | 102.18 | B |
| ATOM | 4541 | CA | ALA | B | 246 | 17.455 | 19.028 | -1.267 | 1.00 | 100.38 | B |
| ATOM | 4542 | CB | ALA | B | 246 | 18.203 | 20.188 | -1.914 | 1.00 | 100.82 | B |
| ATOM | 4543 | C | ALA | B | 246 | 17.925 | 18.859 | 0.180 | 1.00 | 99.04 | B |
| ATOM | 4544 | O | ALA | B | 246 | 19.133 | 18.828 | 0.435 | 1.00 | 97.64 | B |
| ATOM | 4545 | N | GLU | B | 247 | 16.989 | 18.772 | 1.124 | 1.00 | 98.22 | B |
| ATOM | 4546 | CA | GLU | B | 247 | 17.342 | 18.593 | 2.535 | 1.00 | 97.42 | B |
| ATOM | 4547 | CB | GLU | B | 247 | 16.198 | 19.039 | 3.460 | 1.00 | 97.39 | B |
| ATOM | 4548 | CG | GLU | B | 247 | 16.049 | 20.563 | 3.627 | 1.00 | 99.00 | B |
| ATOM | 4549 | CD | GLU | B | 247 | 17.065 | 21.188 | 4.591 | 1.00 | 99.28 | B |
| ATOM | 4550 | OE1 | GLU | B | 247 | 16.996 | 20.886 | 5.801 | 1.00 | 99.27 | B |
| ATOM | 4551 | OE2 | GLU | B | 247 | 17.911 | 22.003 | 4.148 | 1.00 | 99.06 | B |
| ATOM | 4552 | C | GLU | B | 247 | 17.729 | 17.146 | 2.840 | 1.00 | 96.61 | B |
| ATOM | 4553 | O | GLU | B | 247 | 17.981 | 16.799 | 3.993 | 1.00 | 96.67 | B |
| ATOM | 4554 | N | LYS | B | 248 | 17.769 | 16.310 | 1.802 | 1.00 | 95.75 | B |
| ATOM | 4555 | CA | LYS | B | 248 | 18.136 | 14.899 | 1.941 | 1.00 | 94.90 | B |
| ATOM | 4556 | CB | LYS | B | 248 | 17.579 | 14.075 | 0.768 | 1.00 | 95.46 | B |
| ATOM | 4557 | CG | LYS | B | 248 | 17.933 | 12.570 | 0.783 | 1.00 | 95.73 | B |
| ATOM | 4558 | CD | LYS | B | 248 | 17.272 | 11.817 | 1.942 | 1.00 | 95.88 | B |
| ATOM | 4559 | CE | LYS | B | 248 | 17.537 | 10.318 | 1.872 | 1.00 | 94.99 | B |
| ATOM | 4560 | NZ | LYS | B | 248 | 16.849 | 9.585 | 2.975 | 1.00 | 94.98 | B |
| ATOM | 4561 | C | LYS | B | 248 | 19.657 | 14.736 | 2.016 | 1.00 | 93.98 | B |
| ATOM | 4562 | O | LYS | B | 248 | 20.153 | 13.771 | 2.608 | 1.00 | 94.04 | B |
| ATOM | 4563 | N | GLU | B | 249 | 20.390 | 15.663 | 1.397 | 1.00 | 92.29 | B |
| ATOM | 4564 | CA | GLU | B | 249 | 21.851 | 15.611 | 1.409 | 1.00 | 89.52 | B |
| ATOM | 4565 | CB | GLU | B | 249 | 22.451 | 16.245 | 0.150 | 1.00 | 90.95 | B |
| ATOM | 4566 | CG | GLU | B | 249 | 22.416 | 15.323 | -1.080 | 1.00 | 92.62 | B |
| ATOM | 4567 | CD | GLU | B | 249 | 23.131 | 13.983 | -0.856 | 1.00 | 94.07 | B |
| ATOM | 4568 | OE1 | GLU | B | 249 | 24.321 | 13.990 | -0.460 | 1.00 | 94.34 | B |
| ATOM | 4569 | OE2 | GLU | B | 249 | 22.501 | 12.922 | -1.082 | 1.00 | 94.16 | B |
| ATOM | 4570 | C | GLU | B | 249 | 22.444 | 16.216 | 2.670 | 1.00 | 86.32 | B |
| ATOM | 4571 | O | GLU | B | 249 | 23.555 | 15.873 | 3.059 | 1.00 | 86.14 | B |
| ATOM | 4572 | N | VAL | B | 250 | 21.709 | 17.125 | 3.300 | 1.00 | 82.86 | B |
| ATOM | 4573 | CA | VAL | B | 250 | 22.167 | 17.716 | 4.548 | 1.00 | 80.28 | B |
| ATOM | 4574 | CB | VAL | B | 250 | 21.381 | 18.997 | 4.886 | 1.00 | 80.04 | B |
| ATOM | 4575 | CG1 | VAL | B | 250 | 21.902 | 19.620 | 6.167 | 1.00 | 79.05 | B |
| ATOM | 4576 | CG2 | VAL | B | 250 | 21.483 | 19.989 | 3.737 | 1.00 | 80.30 | B |
| ATOM | 4577 | C | VAL | B | 250 | 21.935 | 16.635 | 5.613 | 1.00 | 79.17 | B |
| ATOM | 4578 | O | VAL | B | 250 | 22.550 | 16.649 | 6.683 | 1.00 | 78.77 | B |
| ATOM | 4579 | N | THR | B | 251 | 21.057 | 15.685 | 5.281 | 1.00 | 77.86 | B |
| ATOM | 4580 | CA | THR | B | 251 | 20.718 | 14.547 | 6.143 | 1.00 | 75.71 | B |
| ATOM | 4581 | CB | THR | B | 251 | 19.321 | 13.934 | 5.788 | 1.00 | 76.54 | B |
| ATOM | 4582 | OG1 | THR | B | 251 | 18.294 | 14.920 | 5.967 | 1.00 | 76.86 | B |
| ATOM | 4583 | CG2 | THR | B | 251 | 19.011 | 12.721 | 6.671 | 1.00 | 75.53 | B |
| ATOM | 4584 | C | THR | B | 251 | 21.788 | 13.477 | 5.945 | 1.00 | 73.36 | B |
| ATOM | 4585 | O | THR | B | 251 | 22.411 | 13.032 | 6.908 | 1.00 | 73.52 | B |
| ATOM | 4586 | N | ARG | B | 252 | 22.012 | 13.095 | 4.685 | 1.00 | 70.86 | B |
| ATOM | 4587 | CA | ARG | B | 252 | 23.015 | 12.092 | 4.328 | 1.00 | 68.07 | B |
| ATOM | 4588 | CB | ARG | B | 252 | 23.072 | 11.887 | 2.808 | 1.00 | 69.51 | B |
| ATOM | 4589 | CG | ARG | B | 252 | 22.420 | 10.587 | 2.333 | 1.00 | 71.95 | B |
| ATOM | 4590 | CD | ARG | B | 252 | 22.733 | 10.267 | 0.866 | 1.00 | 72.64 | B |
| ATOM | 4591 | NE | ARG | B | 252 | 22.496 | 8.852 | 0.568 | 1.00 | 74.53 | B |
| ATOM | 4592 | CZ | ARG | B | 252 | 23.454 | 7.933 | 0.419 | 1.00 | 75.25 | B |
| ATOM | 4593 | NH1 | ARG | B | 252 | 24.741 | 8.259 | 0.525 | 1.00 | 74.61 | B |
| ATOM | 4594 | NH2 | ARG | B | 252 | 23.120 | 6.667 | 0.200 | 1.00 | 75.05 | B |
| ATOM | 4595 | C | ARG | B | 252 | 24.393 | 12.479 | 4.856 | 1.00 | 65.51 | B |
| ATOM | 4596 | O | ARG | B | 252 | 25.345 | 11.711 | 4.761 | 1.00 | 65.17 | B |
| ATOM | 4597 | N | MET | B | 253 | 24.497 | 13.693 | 5.384 | 1.00 | 62.65 | B |
| ATOM | 4598 | CA | MET | B | 253 | 25.741 | 14.162 | 5.952 | 1.00 | 60.05 | B |
| ATOM | 4599 | CB | MET | B | 253 | 25.880 | 15.667 | 5.796 | 1.00 | 58.69 | B |
| ATOM | 4600 | CG | MET | B | 253 | 27.253 | 16.158 | 6.172 | 1.00 | 57.31 | B |
| ATOM | 4601 | SD | MET | B | 253 | 27.435 | 17.899 | 5.897 | 1.00 | 55.71 | B |
| ATOM | 4602 | CE | MET | B | 253 | 28.121 | 18.412 | 7.479 | 1.00 | 57.39 | B |
| ATOM | 4603 | C | MET | B | 253 | 25.675 | 13.811 | 7.419 | 1.00 | 59.38 | B |
| ATOM | 4604 | O | MET | B | 253 | 26.531 | 13.090 | 7.931 | 1.00 | 59.65 | B |
| ATOM | 4605 | N | VAL | B | 254 | 24.629 | 14.301 | 8.082 | 1.00 | 57.66 | B |
| ATOM | 4606 | CA | VAL | B | 254 | 24.419 | 14.034 | 9.497 | 1.00 | 56.92 | B |
| ATOM | 4607 | CB | VAL | B | 254 | 23.023 | 14.505 | 9.948 | 1.00 | 56.63 | B |
| ATOM | 4608 | CG1 | VAL | B | 254 | 22.557 | 13.738 | 11.190 | 1.00 | 57.09 | B |
| ATOM | 4609 | CG2 | VAL | B | 254 | 23.070 | 15.995 | 10.246 | 1.00 | 57.03 | B |
| ATOM | 4610 | C | VAL | B | 254 | 24.603 | 12.551 | 9.786 | 1.00 | 56.69 | B |
| ATOM | 4611 | O | VAL | B | 254 | 25.242 | 12.182 | 10.769 | 1.00 | 56.79 | B |
| ATOM | 4612 | N | ILE | B | 255 | 24.084 | 11.710 | 8.897 | 1.00 | 55.90 | B |
| ATOM | 4613 | CA | ILE | B | 255 | 24.214 | 10.267 | 9.044 | 1.00 | 55.43 | B |
| ATOM | 4614 | CB | ILE | B | 255 | 23.556 | 9.523 | 7.873 | 1.00 | 55.14 | B |
| ATOM | 4615 | CG2 | ILE | B | 2.55 | 23.749 | 8.026 | 8.017 | 1.00 | 53.10 | B |
| ATOM | 4616 | CG1 | ILE | B | 255 | 22.072 | 9.891 | 7.784 | 1.00 | 57.11 | B |
| ATOM | 4617 | CD1 | ILE | B | 255 | 21.282 | 9.699 | 9.072 | 1.00 | 58.84 | B |
| ATOM | 4618 | C | ILE | B | 255 | 25.691 | 9.914 | 9.068 | 1.00 | 55.84 | B |
| ATOM | 4619 | O | ILE | B | 255 | 26.169 | 9.250 | 9.987 | 1.00 | 56.88 | B |
| ATOM | 4620 | N | ILE | B | 256 | 26.413 | 10.417 | 8.076 | 1.00 | 55.46 | B |
| ATOM | 4621 | CA | ILE | B | 256 | 27.838 | 10.169 | 7.950 | 1.00 | 55.70 | B |
| ATOM | 4622 | CB | ILE | B | 256 | 28.369 | 10.759 | 6.618 | 1.00 | 56.49 | B |
| ATOM | 4623 | CG2 | ILE | B | 256 | 29.812 | 11.210 | 6.751 | 1.00 | 56.80 | B |
| ATOM | 4624 | CG1 | ILE | B | 256 | 28.253 | 9.716 | 5.503 | 1.00 | 56.60 | B |
| ATOM | 4625 | CD1 | ILE | B | 256 | 26.848 | 9.219 | 5.234 | 1.00 | 57.63 | B |
| ATOM | 4626 | C | ILE | B | 256 | 28.641 | 10.676 | 9.149 | 1.00 | 55.55 | B |
| ATOM | 4627 | O | ILE | B | 256 | 29.548 | 9.992 | 9.624 | 1.00 | 55.44 | B |
| ATOM | 4628 | N | MET | B | 257 | 28.296 | 11.858 | 9.648 | 1.00 | 55.41 | B |
| ATOM | 4629 | CA | MET | B | 257 | 28.994 | 12.436 | 10.797 | 1.00 | 56.51 | B |
| ATOM | 4630 | CB | MET | B | 257 | 28.450 | 13.829 | 11.106 | 1.00 | 58.66 | B |
| ATOM | 4631 | CG | MET | B | 257 | 28.512 | 14.797 | 9.947 | 1.00 | 61.03 | B |
| ATOM | 4632 | SD | MET | B | 257 | 27.872 | 16.407 | 10.404 | 1.00 | 62.63 | B |
| ATOM | 4633 | CE | MET | B | 257 | 29.270 | 17.047 | 11.335 | 1.00 | 63.44 | B |
| ATOM | 4634 | C | MET | B | 257 | 28.828 | 11.552 | 12.029 | 1.00 | 56.15 | B |
| ATOM | 4635 | 0 | MET | B | 257 | 29.763 | 11.368 | 12.811 | 1.00 | 55.65 | B |
| ATOM | 4636 | N | VAL | B | 258 | 27.616 | 11.032 | 12.205 | 1.00 | 55.72 | B |
| ATOM | 4637 | CA | VAL | B | 258 | 27.310 | 10.160 | 13.327 | 1.00 | 55.06 | B |
| ATOM | 4638 | CB | VAL | B | 258 | 25.811 | 9.805 | 13.374 | 1.00 | 54.66 | B |
| ATOM | 4639 | CG1 | VAL | B | 258 | 25.556 | 8.745 | 14.416 | 1.00 | 54.60 | B |
| ATOM | 4640 | CG2 | VAL | B | 258 | 25.002 | 11.038 | 13.706 | 1.00 | 56.19 | B |
| ATOM | 4641 | C | VAL | B | 258 | 28.131 | 8.890 | 13.200 | 1.00 | 54.58 | B |
| ATOM | 4642 | O | VAL | B | 258 | 28.770 | 8.468 | 14.161 | 1.00 | 54.84 | B |
| ATOM | 4643 | N | ILE | B | 259 | 28.146 | 8.318 | 11.998 | 1.00 | 53.84 | B |
| ATOM | 4644 | CA | ILE | B | 259 | 28.887 | 7.088 | 11.718 | 1.00 | 53.39 | B |
| ATOM | 4645 | CB | ILE | B | 259 | 28.643 | 6.620 | 10.270 | 1.00 | 52.45 | B |
| ATOM | 4646 | CG2 | ILE | B | 259 | 29.398 | 5.334 | 9.994 | 1.00 | 51.47 | B |
| ATOM | 4647 | CG1 | ILE | B | 259 | 27.151 | 6.406 | 10.031 | 1.00 | 50.87 | B |
| ATOM | 4648 | CD1 | ILE | B | 259 | 26.802 | 6.279 | 8.571 | 1.00 | 51.36 | B |
| ATOM | 4649 | C | ILE | B | 259 | 30.397 | 7.240 | 11.952 | 1.00 | 54.21 | B |
| ATOM | 4650 | O | ILE | B | 259 | 31.027 | 6.375 | 12.569 | 1.00 | 54.54 | B |
| ATOM | 4651 | N | ALA | B | 260 | 30.967 | 8.346 | 11.470 | 1.00 | 54.03 | B |
| ATOM | 4652 | CA | ALA | B | 260 | 32.398 | 8.622 | 11.618 | 1.00 | 52.48 | B |
| ATOM | 4653 | CB | ALA | B | 260 | 32.773 | 9.824 | 10.792 | 1.00 | 51.29 | B |
| ATOM | 4654 | C | ALA | B | 260 | 32.818 | 8.824 | 13.076 | 1.00 | 52.34 | B |
| ATOM | 4655 | O | ALA | B | 260 | 33.961 | 8.546 | 13.443 | 1.00 | 52.14 | B |
| ATOM | 4656 | N | PHE | B | 261 | 31.885 | 9.325 | 13.887 | 1.00 | 52.14 | B |
| ATOM | 4657 | CA | PHE | B | 261 | 32.094 | 9.563 | 15.315 | 1.00 | 51.14 | B |
| ATOM | 4658 | CB | PHE | B | 261 | 30.897 | 10.335 | 15.883 | 1.00 | 52.58 | B |
| ATOM | 4659 | CG | PHE | B | 261 | 30.985 | 10.607 | 17.365 | 1.00 | 54.80 | B |
| ATOM | 4660 | CD1 | PHE | B | 261 | 31.524 | 11.806 | 17.837 | 1.00 | 56.01 | B |
| ATOM | 4661 | CD2 | PHE | B | 261 | 30.505 | 9.681 | 18.288 | 1.00 | 55.37 | B |
| ATOM | 4662 | CE1 | PHE | B | 261 | 31.584 | 12.084 | 19.208 | 1.00 | 56.04 | B |
| ATOM | 4663 | CE2 | PHE | B | 261 | 30.561 | 9.946 | 19.661 | 1.00 | 56.73 | B |
| ATOM | 4664 | CZ | PHE | B | 261 | 31.102 | 11.153 | 20.119 | 1.00 | 56.46 | B |
| ATOM | 4665 | C | PHE | B | 261 | 32.221 | 8.229 | 16.037 | 1.00 | 49.83 | B |
| ATOM | 4666 | O | PHE | B | 261 | 33.066 | 8.058 | 16.913 | 1.00 | 47.49 | B |
| ATOM | 4667 | N | LEU | B | 262 | 31.343 | 7.302 | 15.665 | 1.00 | 50.42 | B |
| ATOM | 4668 | CA | LEU | B | 262 | 31.298 | 5.960 | 16.238 | 1.00 | 51.04 | B |
| ATOM | 4669 | CB | LEU | B | 262 | 30.044 | 5.216 | 15.750 | 1.00 | 50.80 | B |
| ATOM | 4670 | CG | LEU | B | 262 | 28.681 | 5.742 | 16.240 | 1.00 | 51.69 | B |
| ATOM | 4671 | CD1 | LEU | B | 262 | 27.550 | 5.198 | 15.388 | 1.00 | 50.52 | B |
| ATOM | 4672 | CD2 | LEU | B | 262 | 28.458 | 5.400 | 17.711 | 1.00 | 51.14 | B |
| ATOM | 4673 | C | LEU | B | 262 | 32.548 | 5.171 | 15.878 | 1.00 | 51.43 | B |
| ATOM | 4674 | O | LEU | B | 262 | 32.811 | 4.115 | 16.450 | 1.00 | 52.83 | B |
| ATOM | 4675 | N | ILE | B | 263 | 33.320 | 5.697 | 14.932 | 1.00 | 50.52 | B |
| ATOM | 4676 | CA | ILE | B | 263 | 34.546 | 5.052 | 14.491 | 1.00 | 48.36 | B |
| ATOM | 4677 | CB | ILE | B | 263 | 34.735 | 5.238 | 12.982 | 1.00 | 48.54 | B |
| ATOM | 4678 | CG2 | ILE | B | 263 | 36.080 | 4.674 | 12.531 | 1.00 | 48.64 | B |
| ATOM | 4679 | CG1 | ILE | B | 263 | 33.576 | 4.548 | 12.253 | 1.00 | 48.46 | B |
| ATOM | 4680 | CD1 | ILE | B | 263 | 33.481 | 4.847 | 10.775 | 1.00 | 47.43 | B |
| ATOM | 4681 | C | ILE | B | 263 | 35.756 | 5.559 | 15.266 | 1.00 | 47.89 | B |
| ATOM | 4682 | O | ILE | B | 263 | 36.720 | 4.820 | 15.461 | 1.00 | 47.32 | B |
| ATOM | 4683 | N | CYS | B | 264 | 35.687 | 6.804 | 15.736 | 1.00 | 48.09 | B |
| ATOM | 4684 | CA | CYS | B | 264 | 36.774 | 7.409 | 16.518 | 1.00 | 47.63 | B |
| ATOM | 4685 | CB | CYS | B | 264 | 36.722 | 8.953 | 16.437 | 1.00 | 47.18 | B |
| ATOM | 4686 | SG | CYS | B | 264 | 37.430 | 9.749 | 14.944 | 1.00 | 49.56 | B |
| ATOM | 4687 | C | CYS | B | 264 | 36.711 | 6.993 | 17.992 | 1.00 | 46.97 | B |
| ATOM | 4688 | O | CYS | B | 264 | 37.716 | 6.588 | 18.588 | 1.00 | 46.20 | B |
| ATOM | 4689 | N | TRP | B | 265 | 35.503 | 7.042 | 18.548 | 1.00 | 46.59 | B |
| ATOM | 4690 | CA | TRP | B | 265 | 35.290 | 6.746 | 19.951 | 1.00 | 45.62 | B |
| ATOM | 4691 | CB | TRP | B | 265 | 34.353 | 7.793 | 20.551 | 1.00 | 46.49 | B |
| ATOM | 4692 | CG | TRP | B | 265 | 34.846 | 9.184 | 20.314 | 1.00 | 46.82 | B |
| ATOM | 4693 | CD2 | TRP | B | 265 | 35.969 | 9.823 | 20.944 | 1.00 | 48.60 | B |
| ATOM | 4694 | CE2 | TRP | B | 265 | 36.094 | 11.108 | 20.370 | 1.00 | 47.79 | B |
| ATOM | 4695 | CE3 | TRP | B | 265 | 36.887 | 9.432 | 21.934 | 1.00 | 50.04 | B |
| ATOM | 4696 | CD1 | TRP | B | 265 | 34.352 | 10.079 | 19.420 | 1.00 | 46.45 | B |
| ATOM | 4697 | NE1 | TRP | B | 265 | 35.093 | 11.238 | 19.444 | 1.00 | 47.86 | B |
| ATOM | 4698 | CZ2 | TRP | B | 265 | 37.100 | 12.009 | 20.750 | 1.00 | 47.37 | B |
| ATOM | 4699 | CZ3 | TRP | B | 265 | 37.893 | 10.332 | 22.313 | 1.00 | 48.88 | B |
| ATOM | 4700 | CH2 | TRP | B | 265 | 37.987 | 11.604 | 21.718 | 1.00 | 47.88 | B |
| ATOM | 4701 | C | TRP | B | 265 | 34.867 | 5.351 | 20.374 | 1.00 | 44.86 | B |
| ATOM | 4702 | O | TRP | B | 265 | 34.968 | 5.032 | 21.550 | 1.00 | 46.27 | B |
| ATOM | 4703 | N | LED | B | 266 | 34.371 | 4.519 | 19.468 | 1.00 | 43.54 | B |
| ATOM | 4704 | CA | LEU | B | 266 | 33.997 | 3.175 | 19.905 | 1.00 | 43.80 | B |
| ATOM | 4705 | CB | LEU | B | 266 | 33.050 | 2.480 | 18.927 | 1.00 | 44.03 | B |
| ATOM | 4706 | CG | LEU | B | 266 | 31.573 | 2.568 | 19.341 | 1.00 | 43.63 | B |
| ATOM | 4707 | CD1 | LEU | B | 266 | 31.189 | 4.006 | 19.715 | 1.00 | 43.73 | B |
| ATOM | 4708 | CD2 | LEU | B | 266 | 30.690 | 2.044 | 18.221 | 1.00 | 42.27 | B |
| ATOM | 4709 | C | LEU | B | 266 | 35.216 | 2.318 | 20.240 | 1.00 | 43.56 | B |
| ATOM | 4710 | O | LEU | B | 266 | 35.174 | 1.537 | 21.190 | 1.00 | 43.08 | B |
| ATOM | 4711 | N | PRO | B | 267 | 36.302 | 2.412 | 19.439 | 1.00 | 42.97 | B |
| ATOM | 4712 | CD | PRO | B | 267 | 36.458 | 2.985 | 18.091 | 1.00 | 42.09 | B |
| ATOM | 4713 | CA | PRO | B | 267 | 37.474 | 1.603 | 19.781 | 1.00 | 42.83 | B |
| ATOM | 4714 | CB | PRO | B | 267 | 38.451 | 1.944 | 18.661 | 1.00 | 41.74 | B |
| ATOM | 4715 | CG | PRO | B | 267 | 37.560 | 2.141 | 17.507 | 1.00 | 40.98 | B |
| ATOM | 4716 | C | PRO | B | 267 | 37.987 | 2.092 | 21.136 | 1.00 | 43.77 | B |
| ATOM | 4717 | O | PRO | B | 267 | 38.384 | 1.297 | 21.984 | 1.00 | 44.27 | B |
| ATOM | 4718 | N | TYR | B | 268 | 37.919 | 3.406 | 21.348 | 1.00 | 43.65 | B |
| ATOM | 4719 | CA | TYR | B | 268 | 38.362 | 4.009 | 22.595 | 1.00 | 43.70 | B |
| ATOM | 4720 | CB | TYR | B | 268 | 38.374 | 5.529 | 22.490 | 1.00 | 44.78 | B |
| ATOM | 4721 | CG | TYR | B | 268 | 38.793 | 6.201 | 23.777 | 1.00 | 46.75 | B |
| ATOM | 4722 | CD1 | TYR | B | 268 | 40.085 | 6.026 | 24.288 | 1.00 | 47.60 | B |
| ATOM | 4723 | CE1 | TYR | B | 268 | 40.473 | 6.613 | 25.494 | 1.00 | 47.29 | B |
| ATOM | 4724 | CD2 | TYR | B | 268 | 37.894 | 6.986 | 24.506 | 1.00 | 46.71 | B |
| ATOM | 4725 | CE2 | TYR | B | 268 | 38.270 | 7.576 | 25.713 | 1.00 | 46.41 | B |
| ATOM | 4726 | CZ | TYR | B | 268 | 39.561 | 7.385 | 26.199 | 1.00 | 47.05 | B |
| ATOM | 4727 | OH | TYR | B | 268 | 39.948 | 7.967 | 27.380 | 1.00 | 45.81 | B |
| ATOM | 4728 | C | TYR | B | 268 | 37.468 | 3.606 | 23.750 | 1.00 | 44.35 | B |
| ATOM | 4729 | O | TYR | B | 268 | 37.929 | 2.996 | 24.705 | 1.00 | 45.00 | B |
| ATOM | 4730 | N | ALA | B | 269 | 36.195 | 3.983 | 23.664 | 1.00 | 45.88 | B |
| ATOM | 4731 | CA | ALA | B | 269 | 35.198 | 3.673 | 24.692 | 1.00 | 46.78 | B |
| ATOM | 4732 | CB | ALA | B | 269 | 33.824 | 4.190 | 24.273 | 1.00 | 45.30 | B |
| ATOM | 4733 | C | ALA | B | 269 | 35.131 | 2.177 | 24.973 | 1.00 | 48.16 | B |
| ATOM | 4734 | O | ALA | B | 269 | 34.894 | 1.768 | 26.110 | 1.00 | 48.24 | B |
| ATOM | 4735 | N | GLY | B | 270 | 35.343 | 1.369 | 23.935 | 1.00 | 49.48 | B |
| ATOM | 4736 | CA | GLY | B | 270 | 35.322 | -0.076 | 24.095 | 1.00 | 50.85 | B |
| ATOM | 4737 | C | GLY | B | 270 | 36.455 | -0.529 | 25.001 | 1.00 | 51.42 | B |
| ATOM | 4738 | O | GLY | B | 270 | 36.253 | -1.356 | 25.898 | 1.00 | 51.44 | B |
| ATOM | 4739 | N | VAL | B | 271 | 37.643 | 0.028 | 24.768 | 1.00 | 50.30 | B |
| ATOM | 4740 | CA | VAL | B | 271 | 38.827 | -0.284 | 25.561 | 1.00 | 50.31 | B |
| ATOM | 4741 | CB | VAL | B | 271 | 40.105 | 0.265 | 24.899 | 1.00 | 51.26 | B |
| ATOM | 4742 | CG1 | VAL | B | 271 | 41.274 | 0.166 | 25.857 | 1.00 | 51.46 | B |
| ATOM | 4743 | CG2 | VAL | B | 271 | 40.414 | -0.505 | 23.621 | 1.00 | 52.05 | B |
| ATOM | 4744 | C | VAL | B | 271 | 38.690 | 0.349 | 26.936 | 1.00 | 50.14 | B |
| ATOM | 4745 | O | VAL | B | 271 | 39.106 | -0.227 | 27.939 | 1.00 | 49.36 | B |
| ATOM | 4746 | N | ALA | B | 272 | 38.106 | 1.542 | 26.963 | 1.00 | 50.37 | B |
| ATOM | 4747 | CA | ALA | B | 272 | 37.889 | 2.281 | 28.200 | 1.00 | 51.65 | B |
| ATOM | 4748 | CB | ALA | B | 272 | 37.309 | 3.650 | 27.893 | 1.00 | 50.79 | B |
| ATOM | 4749 | C | ALA | B | 272 | 36.970 | 1.520 | 29.160 | 1.00 | 52.22 | B |
| ATOM | 4750 | O | ALA | B | 272 | 37.214 | 1.475 | 30.370 | 1.00 | 53.09 | B |
| ATOM | 4751 | N | PHE | B | 273 | 35.930 | 0.899 | 28.617 | 1.00 | 51.65 | B |
| ATOM | 4752 | CA | PHE | B | 273 | 35.001 | 0.162 | 29.451 | 1.00 | 51.42 | B |
| ATOM | 4753 | CB | PHE | B | 273 | 33.678 | -0.050 | 28.737 | 1.00 | 52.75 | B |
| ATOM | 4754 | CG | PHE | B | 273 | 32.566 | -0.410 | 29.657 | 1.00 | 54.23 | B |
| ATOM | 4755 | CD1 | PHE | B | 273 | 32.089 | 0.522 | 30.574 | 1.00 | 55.34 | B |
| ATOM | 4756 | CD2 | PHE | B | 273 | 32.008 | -1.682 | 29.631 | 1.00 | 55.12 | B |
| ATOM | 4757 | CE1 | PHE | B | 273 | 31.069 | 0.196 | 31.456 | 1.00 | 57.07 | B |
| ATOM | 4758 | CE2 | PHE | B | 273 | 30.986 | -2.027 | 30.505 | 1.00 | 56.44 | B |
| ATOM | 4759 | CZ | PHE | B | 273 | 30.512 | -1.086 | 31.423 | 1.00 | 58.11 | B |
| ATOM | 4760 | C | PHE | B | 273 | 35.559 | -1.172 | 29.906 | 1.00 | 50.85 | B |
| ATOM | 4761 | O | PHE | B | 273 | 35.280 | -1.611 | 31.019 | 1.00 | 50.86 | B |
| ATOM | 4762 | N | TYR | B | 274 | 36.313 | -1.831 | 29.031 | 1.00 | 50.42 | B |
| ATOM | 4763 | CA | TYR | B | 274 | 36.919 | -3.114 | 29.366 | 1.00 | 49.91 | B |
| ATOM | 4764 | CB | TYR | B | 274 | 37.756 | -3.652 | 28.196 | 1.00 | 51.66 | B |
| ATOM | 4765 | CG | TYR | B | 274 | 38.635 | -4.858 | 28.525 | 1.00 | 53.90 | B |
| ATOM | 4766 | CD1 | TYR | B | 274 | 38.206 | -6.162 | 28.257 | 1.00 | 54.80 | B |
| ATOM | 4767 | CE1 | TYR | B | 274 | 39.030 | -7.274 | 28.534 | 1.00 | 55.82 | B |
| ATOM | 4768 | CD2 | TYR | B | 274 | 39.909 | -4.691 | 29.085 | 1.00 | 56.28 | B |
| ATOM | 4769 | CE2 | TYR | B | 274 | 40.737 | -5.795 | 29.370 | 1.00 | 56.94 | B |
| ATOM | 4770 | CZ | TYR | B | 274 | 40.291 | -7.079 | 29.089 | 1.00 | 56.53 | B |
| ATOM | 4771 | OH | TYR | B | 274 | 41.113 | -8.153 | 29.348 | 1.00 | 56.33 | B |
| ATOM | 4772 | C | TYR | B | 274 | 37.807 | -2.908 | 30.582 | 1.00 | 49.02 | B |
| ATOM | 4773 | O | TYR | B | 274 | 37.817 | -3.732 | 31.495 | 1.00 | 50.29 | B |
| ATOM | 4774 | N | ILE | B | 275 | 38.537 | -1.797 | 30.603 | 1.00 | 46.15 | B |
| ATOM | 4775 | CA | ILE | B | 275 | 39.421 | -1.515 | 31.720 | 1.00 | 43.11 | B |
| ATOM | 4776 | CB | ILE | B | 275 | 40.395 | -0.387 | 31.395 | 1.00 | 40.55 | B |
| ATOM | 4777 | CG2 | ILE | B | 275 | 41.226 | -0.052 | 32.609 | 1.00 | 39.48 | B |
| ATOM | 4778 | CG1 | ILE | B | 275 | 41.305 | -0.821 | 30.251 | 1.00 | 39.49 | B |
| ATOM | 4779 | CD1 | ILE | B | 275 | 42.262 | 0.242 | 29.794 | 1.00 | 38.31 | B |
| ATOM | 4780 | C | ILE | B | 275 | 38.671 | -1.203 | 33.007 | 1.00 | 43.45 | B |
| ATOM | 4781 | O | ILE | B | 275 | 39.030 | -1.715 | 34.055 | 1.00 | 43.33 | B |
| ATOM | 4782 | N | PHE | B | 276 | 37.614 | -0.402 | 32.935 | 1.00 | 44.12 | B |
| ATOM | 4783 | CA | PHE | B | 276 | 36.865 | -0.064 | 34.139 | 1.00 | 45.20 | B |
| ATOM | 4784 | CB | PHE | B | 276 | 35.715 | 0.895 | 33.824 | 1.00 | 44.26 | B |
| ATOM | 4785 | CG | PHE | B | 276 | 34.901 | 1.281 | 35.032 | 1.00 | 43.72 | B |
| ATOM | 4786 | CD1 | PHE | B | 276 | 35.519 | 1.549 | 36.250 | 1.00 | 42.43 | B |
| ATOM | 4787 | CD2 | PHE | B | 276 | 33.513 | 1.353 | 34.956 | 1.00 | 45.15 | B |
| ATOM | 4788 | CE1 | PHE | B | 276 | 34.773 | 1.877 | 37.372 | 1.00 | 43.75 | B |
| ATOM | 4789 | CE2 | PHE | B | 276 | 32.753 | 1.682 | 36.077 | 1.00 | 44.68 | B |
| ATOM | 4790 | CZ | PHE | B | 276 | 33.385 | 1.943 | 37.287 | 1.00 | 44.38 | B |
| ATOM | 4791 | C | PHE | B | 276 | 36.337 | -1.297 | 34.874 | 1.00 | 47.18 | B |
| ATOM | 4792 | O | PHE | B | 276 | 36.498 | -1.412 | 36.096 | 1.00 | 48.44 | B |
| ATOM | 4793 | N | THR | B | 277 | 35.714 | -2.213 | 34.132 | 1.00 | 48.44 | B |
| ATOM | 4794 | CA | THR | B | 277 | 35.165 | -3.440 | 34.715 | 1.00 | 49.38 | B |
| ATOM | 4795 | CB | THR | B | 277 | 34.302 | -4.206 | 33.686 | 1.00 | 47.25 | B |
| ATOM | 4796 | OG1 | THR | B | 277 | 35.029 | -4.343 | 32.466 | 1.00 | 45.90 | B |
| ATOM | 4797 | CG2 | THR | B | 277 | 33.016 | -3.457 | 33.401 | 1.00 | 44.96 | B |
| ATOM | 4798 | C | THR | B | 277 | 36.271 | -4.351 | 35.283 | 1.00 | 51.81 | B |
| ATOM | 4799 | O | THR | B | 277 | 36.121 | -4.921 | 36.377 | 1.00 | 52.70 | B |
| ATOM | 4800 | N | HIS | B | 278 | 37.393 | -4.439 | 34.560 | 1.00 | 53.17 | B |
| ATOM | 4801 | CA | HIS | B | 278 | 38.554 | -5.250 | 34.967 | 1.00 | 53.25 | B |
| ATOM | 4802 | CB | HIS | B | 278 | 39.083 | -6.091 | 33.797 | 1.00 | 51.92 | B |
| ATOM | 4803 | CG | HIS | B | 278 | 38.026 | -6.642 | 32.894 | 1.00 | 51.18 | B |
| ATOM | 4804 | CD2 | HIS | B | 278 | 38.115 | -7.172 | 31.653 | 1.00 | 49.67 | B |
| ATOM | 4805 | ND1 | HIS | B | 278 | 36.689 | -6.669 | 33.228 | 1.00 | 51.49 | B |
| ATOM | 4806 | CE1 | HIS | B | 278 | 36.001 | -7.191 | 32.228 | 1.00 | 51.51 | B |
| ATOM | 4807 | NE2 | HIS | B | 278 | 36.843 | -7.504 | 31.260 | 1.00 | 49.22 | B |
| ATOM | 4808 | C | HIS | B | 278 | 39.723 | -4.374 | 35.469 | 1.00 | 53.89 | B |
| ATOM | 4809 | O | HIS | B | 278 | 40.783 | -4.321 | 34.831 | 1.00 | 52.31 | B |
| ATOM | 4810 | N | GLN | B | 279 | 39.537 | -3.692 | 36.598 | 1.00 | 54.92 | B |
| ATOM | 4811 | CA | GLN | B | 279 | 40.588 | -2.842 | 37.151 | 1.00 | 56.15 | B |
| ATOM | 4812 | CB | GLN | B | 279 | 40.006 | -1.846 | 38.158 | 1.00 | 56.19 | B |
| ATOM | 4813 | CG | GLN | B | 279 | 39.038 | -0.854 | 37.552 | 1.00 | 56.00 | B |
| ATOM | 4814 | CD | GLN | B | 279 | 38.567 | 0.183 | 38.541 | 1.00 | 57.20 | B |
| ATOM | 4815 | OE1 | GLN | B | 279 | 38.346 | 1.340 | 38.187 | 1.00 | 58.74 | B |
| ATOM | 4816 | NE2 | GLN | B | 279 | 38.405 | -0.224 | 39.790 | 1.00 | 58.47 | B |
| ATOM | 4817 | C | GLN | B | 279 | 41.683 | -3.684 | 37.803 | 1.00 | 57.53 | B |
| ATOM | 4818 | O | GLN | B | 279 | 41.709 | -3.853 | 39.033 | 1.00 | 59.37 | B |
| ATOM | 4819 | N | GLY | B | 280 | 42.564 | -4.230 | 36.965 | 1.00 | 57.13 | B |
| ATOM | 4820 | CA | GLY | B | 280 | 43.663 | -5.054 | 37.451 | 1.00 | 56.74 | B |
| ATOM | 4821 | C | GLY | B | 280 | 44.337 | -5.974 | 36.437 | 1.00 | 56.78 | B |
| ATOM | 4822 | O | GLY | B | 280 | 45.433 | -6.477 | 36.706 | 1.00 | 55.43 | B |
| ATOM | 4823 | N | SER | B | 281 | 43.713 | -6.144 | 35.264 | 1.00 | 57.49 | B |
| ATOM | 4824 | CA | SER | B | 281 | 44.192 | -7.015 | 34.169 | 1.00 | 57.29 | B |
| ATOM | 4825 | CB | SER | B | 281 | 43.311 | -6.839 | 32.924 | 1.00 | 57.42 | B |
| ATOM | 4826 | OG | SER | B | 281 | 41.969 | -6.539 | 33.263 | 1.00 | 56.93 | B |
| ATOM | 4827 | C | SER | B | 281 | 45.662 | -6.866 | 33.751 | 1.00 | 57.69 | B |
| ATOM | 4828 | O | SER | B | 281 | 46.372 | -5.962 | 34.213 | 1.00 | 57.70 | B |
| ATOM | 4829 | N | ASP | B | 282 | 46.095 | -7.739 | 32.839 | 1.00 | 57.55 | B |
| ATOM | 4830 | CA | ASP | B | 282 | 47.475 | -7.733 | 32.355 | 1.00 | 57.97 | B |
| ATOM | 4831 | CB | ASP | B | 282 | 48.063 | -9.157 | 32.422 | 1.00 | 60.97 | B |
| ATOM | 4832 | CG | ASP | B | 282 | 49.604 | -9.179 | 32.405 | 1.00 | 63.90 | B |
| ATOM | 4833 | OD1 | ASP | B | 282 | 50.180 | -10.286 | 32.534 | 1.00 | 64.30 | B |
| ATOM | 4834 | OD2 | ASP | B | 282 | 50.242 | -8.105 | 32.272 | 1.00 | 65.04 | B |
| ATOM | 4835 | C | ASP | B | 282 | 47.605 | -7.167 | 30.939 | 1.00 | 56.61 | B |
| ATOM | 4836 | O | ASP | B | 282 | 48.381 | -7.673 | 30.128 | 1.00 | 56.83 | B |
| ATOM | 4837 | N | PHE | B | 283 | 46.855 | -6.111 | 30.649 | 1.00 | 54.52 | B |
| ATOM | 4838 | CA | PHE | B | 283 | 46.905 | -5.476 | 29.337 | 1.00 | 53.45 | B |
| ATOM | 4839 | CB | PHE | B | 283 | 45.661 | -4.611 | 29.127 | 1.00 | 53.76 | B |
| ATOM | 4840 | CG | PHE | B | 283 | 45.369 | -3.674 | 30.274 | 1.00 | 52.72 | B |
| ATOM | 4841 | CD1 | PHE | B | 283 | 45.833 | -2.367 | 30.261 | 1.00 | 52.65 | B |
| ATOM | 4842 | CD2 | PHE | B | 283 | 44.634 | -4.104 | 31.367 | 1.00 | 52.36 | B |
| ATOM | 4843 | CE1 | PHE | B | 283 | 45.566 | -1.507 | 31.322 | 1.00 | 52.09 | B |
| ATOM | 4844 | CE2 | PHE | B | 283 | 44.363 | -3.247 | 32.432 | 1.00 | 50.64 | B |
| ATOM | 4845 | CZ | PHE | B | 283 | 44.828 | -1.954 | 32.409 | 1.00 | 50.80 | B |
| ATOM | 4846 | C | PHE | B | 283 | 48.169 | -4.627 | 29.243 | 1.00 | 52.65 | B |
| ATOM | 4847 | O | PHE | B | 283 | 48.690 | -4.191 | 30.270 | 1.00 | 51.91 | B |
| ATOM | 4848 | N | GLY | B | 284 | 48.658 | -4.398 | 28.023 | 1.00 | 51.82 | B |
| ATOM | 4849 | CA | GLY | B | 284 | 49.865 | -3.603 | 27.836 | 1.00 | 50.78 | B |
| ATOM | 4850 | C | GLY | B | 284 | 49.629 | -2.123 | 27.584 | 1.00 | 50.42 | B |
| ATOM | 4851 | O | GLY | B | 284 | 48.509 | -1.637 | 27.730 | 1.00 | 50.37 | B |
| ATOM | 4852 | N | PRO | B | 285 | 50.688 | -1.354 | 27.298 | 1.00 | 50.23 | B |
| ATOM | 4853 | CD | PRO | B | 285 | 52.089 | -1.668 | 27.607 | 1.00 | 50.55 | B |
| ATOM | 4854 | CA | PRO | B | 285 | 50.544 | 0.080 | 27.034 | 1.00 | 50.35 | B |
| ATOM | 4855 | CB | PRO | B | 285 | 51.978 | 0.591 | 27.163 | 1.00 | 49.82 | B |
| ATOM | 4856 | CG | PRO | B | 285 | 52.573 | -0.344 | 28.142 | 1.00 | 50.72 | B |
| ATOM | 4857 | C | PRO | B | 285 | 49.981 | 0.344 | 25.632 | 1.00 | 51.07 | B |
| ATOM | 4858 | O | PRO | B | 285 | 49.117 | 1.203 | 25.453 | 1.00 | 52.78 | B |
| ATOM | 4859 | N | ILE | B | 286 | 50.458 | -0.417 | 24.649 | 1.00 | 50.07 | B |
| ATOM | 4860 | CA | ILE | B | 286 | 50.017 | -0.286 | 23.258 | 1.00 | 47.15 | B |
| ATOM | 4861 | CB | ILE | B | 286 | 50.786 | -1.279 | 22.357 | 1.00 | 47.01 | B |
| ATOM | 4862 | CG2 | ILE | B | 286 | 50.477 | -1.022 | 20.901 | 1.00 | 45.93 | B |
| ATOM | 4863 | CG1 | ILE | B | 286 | 52.291 | -1.135 | 22.597 | 1.00 | 48.91 | B |
| ATOM | 4864 | CD1 | ILE | B | 286 | 53.153 | -2.176 | 21.887 | 1.00 | 51.46 | B |
| ATOM | 4865 | C | ILE | B | 286 | 48.515 | -0.559 | 23.137 | 1.00 | 46.30 | B |
| ATOM | 4866 | O | ILE | B | 286 | 47.875 | -0.172 | 22.160 | 1.00 | 45.72 | B |
| ATOM | 4867 | N | PHE | B | 287 | 47.962 | -1.209 | 24.154 | 1.00 | 45.59 | B |
| ATOM | 4868 | CA | PHE | B | 287 | 46.548 | -1.563 | 24.196 | 1.00 | 44.72 | B |
| ATOM | 4869 | CB | PHE | B | 287 | 46.238 | -2.315 | 25.508 | 1.00 | 45.12 | B |
| ATOM | 4870 | CG | PHE | B | 287 | 44.822 | -2.829 | 25.612 | 1.00 | 44.43 | B |
| ATOM | 4871 | CD1 | PHE | B | 287 | 44.305 | -3.703 | 24.657 | 1.00 | 45.49 | B |
| ATOM | 4872 | CD2 | PHE | B | 287 | 44.004 | -2.437 | 26.671 | 1.00 | 43.84 | B |
| ATOM | 4873 | CE1 | PHE | B | 287 | 42.989 | -4.176 | 24.756 | 1.00 | 44.75 | B |
| ATOM | 4874 | CE2 | PHE | B | 287 | 42.691 | -2.903 | 26.779 | 1.00 | 43.63 | B |
| ATOM | 4875 | CZ | PHE | B | 287 | 42.184 | -3.772 | 25.821 | 1.00 | 43.59 | B |
| ATOM | 4876 | C | PHE | B | 287 | 45.619 | -0.357 | 24.013 | 1.00 | 43.27 | B |
| ATOM | 4877 | O | PHE | B | 287 | 44.864 | -0.301 | 23.041 | 1.00 | 44.29 | B |
| ATOM | 4878 | N | MET | B | 288 | 45.684 | 0.608 | 24.927 | 1.00 | 40.94 | B |
| ATOM | 4879 | CA | MET | B | 288 | 44.822 | 1.785 | 24.843 | 1.00 | 39.65 | B |
| ATOM | 4880 | CB | MET | B | 288 | 44.498 | 2.299 | 26.246 | 1.00 | 37.14 | B |
| ATOM | 4881 | CG | MET | B | 288 | 43.752 | 3.628 | 26.300 | 1.00 | 36.40 | B |
| ATOM | 4882 | SD | MET | B | 288 | 42.179 | 3.685 | 25.438 | 1.00 | 36.77 | B |
| ATOM | 4883 | CE | MET | B | 288 | 41.072 | 3.530 | 26.786 | 1.00 | 36.34 | B |
| ATOM | 4884 | C | MET | B | 288 | 45.386 | 2.914 | 23.976 | 1.00 | 40.67 | B |
| ATOM | 4885 | O | MET | B | 288 | 44.637 | 3.801 | 23.547 | 1.00 | 41.58 | B |
| ATOM | 4886 | N | THR | B | 289 | 46.689 | 2.853 | 23.687 | 1.00 | 39.39 | B |
| ATOM | 4887 | CA | THR | B | 289 | 47.377 | 3.867 | 22.881 | 1.00 | 37.66 | B |
| ATOM | 4888 | CB | THR | B | 289 | 48.924 | 3.640 | 22.898 | 1.00 | 39.02 | B |
| ATOM | 4889 | OG1 | THR | B | 289 | 49.479 | 4.230 | 24.086 | 1.00 | 40.69 | B |
| ATOM | 4890 | CG2 | THR | B | 289 | 49.605 | 4.240 | 21.671 | 1.00 | 37.11 | B |
| ATOM | 4891 | C | THR | B | 289 | 46.855 | 4.021 | 21.452 | 1.00 | 36.25 | B |
| ATOM | 4892 | O | THR | B | 289 | 46.904 | 5.112 | 20.891 | 1.00 | 34.96 | B |
| ATOM | 4893 | N | ILE | B | 290 | 46.323 | 2.944 | 20.885 | 1.00 | 36.69 | B |
| ATOM | 4894 | CA | ILE | B | 290 | 45.787 | 2.975 | 19.526 | 1.00 | 36.15 | B |
| ATOM | 4895 | CB | ILE | B | 290 | 45.676 | 1.572 | 18.906 | 1.00 | 37.75 | B |
| ATOM | 4896 | CG2 | ILE | B | 290 | 45.562 | 1.697 | 17.391 | 1.00 | 37.64 | B |
| ATOM | 4897 | CG1 | ILE | B | 290 | 46.860 | 0.697 | 19.333 | 1.00 | 38.40 | B |
| ATOM | 4898 | CD1 | ILE | B | 290 | 48.222 | 1.280 | 18.976 | 1.00 | 40.52 | B |
| ATOM | 4899 | C | ILE | B | 290 | 44.407 | 3.617 | 19.469 | 1.00 | 35.26 | B |
| ATOM | 4900 | O | ILE | B | 290 | 44.217 | 4.580 | 18.734 | 1.00 | 34.65 | B |
| ATOM | 4901 | N | PRO | B | 291 | 43.427 | 3.093 | 20.245 | 1.00 | 34.78 | B |
| ATOM | 4902 | CD | PRO | B | 291 | 43.516 | 1.958 | 21.179 | 1.00 | 34.72 | B |
| ATOM | 4903 | CA | PRO | B | 291 | 42.065 | 3.636 | 20.262 | 1.00 | 34.91 | B |
| ATOM | 4904 | CB | PRO | B | 291 | 41.356 | 2.743 | 21.284 | 1.00 | 34.38 | B |
| ATOM | 4905 | CG | PRO | B | 291 | 42.099 | 1.476 | 21.220 | 1.00 | 35.02 | B |
| ATOM | 4906 | C | PRO | B | 291 | 42.046 | 5.078 | 20.739 | 1.00 | 35.38 | B |
| ATOM | 4907 | O | PRO | B | 291 | 41.185 | 5.872 | 20.336 | 1.00 | 35.33 | B |
| ATOM | 4908 | N | ALA | B | 292 | 43.005 | 5.400 | 21.605 | 1.00 | 35.46 | B |
| ATOM | 4909 | CA | ALA | B | 292 | 43.114 | 6.728 | 22.183 | 1.00 | 34.93 | B |
| ATOM | 4910 | CB | ALA | B | 292 | 43.828 | 6.673 | 23.514 | 1.00 | 32.87 | B |
| ATOM | 4911 | C | ALA | B | 292 | 43.775 | 7.734 | 21.270 | 1.00 | 34.76 | B |
| ATOM | 4912 | O | ALA | B | 292 | 43.408 | 8.898 | 21.293 | 1.00 | 37.53 | B |
| ATOM | 4913 | N | PHE | B | 293 | 44.743 | 7.304 | 20.470 | 1.00 | 34.33 | B |
| ATOM | 4914 | CA | PHE | B | 293 | 45.418 | 8.234 | 19.569 | 1.00 | 34.12 | B |
| ATOM | 4915 | CB | PHE | B | 293 | 46.888 | 7.851 | 19.364 | 1.00 | 33.89 | B |
| ATOM | 4916 | CG | PHE | B | 293 | 47.829 | 8.604 | 20.270 | 1.00 | 37.89 | B |
| ATOM | 4917 | CD1 | PHE | B | 293 | 48.631 | 9.635 | 19.771 | 1.00 | 39.71 | B |
| ATOM | 4918 | CD2 | PHE | B | 293 | 47.865 | 8.342 | 21.643 | 1.00 | 38.32 | B |
| ATOM | 4919 | CE1 | PHE | B | 293 | 49.452 | 10.404 | 20.635 | 1.00 | 38.53 | B |
| ATOM | 4920 | CE2 | PHE | B | 293 | 48.680 | 9.103 | 22.509 | 1.00 | 37.06 | B |
| ATOM | 4921 | CZ | PHE | B | 293 | 49.471 | 10.133 | 22.004 | 1.00 | 35.54 | B |
| ATOM | 4922 | C | PHE | B | 293 | 44.688 | 8.363 | 18.250 | 1.00 | 34.65 | B |
| ATOM | 4923 | O | PHE | B | 293 | 44.676 | 9.427 | 17.622 | 1.00 | 33.35 | B |
| ATOM | 4924 | N | PHE | B | 294 | 44.068 | 7.266 | 17.836 | 1.00 | 34.98 | B |
| ATOM | 4925 | CA | PHE | B | 294 | 43.307 | 7.250 | 16.606 | 1.00 | 34.72 | B |
| ATOM | 4926 | CB | PHE | B | 294 | 42.740 | 5.850 | 16.367 | 1.00 | 33.46 | B |
| ATOM | 4927 | CG | PHE | B | 294 | 41.692 | 5.798 | 15.307 | 1.00 | 33.13 | B |
| ATOM | 4928 | CD1 | PHE | B | 294 | 41.986 | 6.166 | 13.996 | 1.00 | 33.95 | B |
| ATOM | 4929 | CD2 | PHE | B | 294 | 40.405 | 5.392 | 15.614 | 1.00 | 33.03 | B |
| ATOM | 4930 | CE1 | PHE | B | 294 | 41.009 | 6.130 | 13.000 | 1.00 | 33.82 | B |
| ATOM | 4931 | CE2 | PHE | B | 294 | 39.421 | 5.354 | 14.627 | 1.00 | 34.88 | B |
| ATOM | 4932 | CZ | PHE | B | 294 | 39.725 | 5.724 | 13.314 | 1.00 | 34.30 | B |
| ATOM | 4933 | C | PHE | B | 294 | 42.181 | 8.266 | 16.762 | 1.00 | 35.55 | B |
| ATOM | 4934 | O | PHE | B | 294 | 41.957 | 9.092 | 15.879 | 1.00 | 37.03 | B |
| ATOM | 4935 | N | ALA | B | 295 | 41.538 | 8.240 | 17.930 | 1.00 | 35.53 | B |
| ATOM | 4936 | CA | ALA | B | 295 | 40.425 | 9.124 | 16.265 | 1.00 | 34.37 | B |
| ATOM | 4937 | CB | ALA | B | 295 | 39.930 | 8.821 | 19.671 | 1.00 | 33.02 | B |
| ATOM | 4938 | C | ALA | B | 295 | 40.725 | 10.619 | 18.114 | 1.00 | 34.91 | B |
| ATOM | 4939 | O | ALA | B | 295 | 39.803 | 11.426 | 17.939 | 1.00 | 36.92 | B |
| ATOM | 4940 | N | RET | B | 296 | 42.001 | 10.993 | 18.175 | 1.00 | 33.13 | B |
| ATOM | 4941 | CA | RET | B | 296 | 42.382 | 12.394 | 18.030 | 1.00 | 32.32 | B |
| ATOM | 4942 | C | RET | B | 296 | 42.115 | 12.946 | 16.625 | 1.00 | 33.09 | B |
| ATOM | 4943 | O | RET | B | 296 | 42.268 | 14.146 | 16.403 | 1.00 | 34.02 | B |
| ATOM | 4944 | CB | RET | B | 296 | 43.846 | 12.603 | 18.416 | 1.00 | 31.65 | B |
| ATOM | 4945 | CG | RET | B | 296 | 43.997 | 12.746 | 19.926 | 1.00 | 29.35 | B |
| ATOM | 4946 | CD | RET | B | 296 | 45.275 | 12.019 | 20.350 | 1.00 | 29.04 | B |
| ATOM | 4947 | CE | RET | B | 296 | 45.810 | 12.238 | 21.768 | 1.00 | 27.26 | B |
| ATOM | 4948 | NZ | RET | B | 296 | 44.923 | 12.219 | 22.932 | 1.00 | 27.67 | B |
| ATOM | 4949 | C1 | RET | B | 296 | 33.232 | 8.627 | 25.654 | 1.00 | 37.79 | B |
| ATOM | 4950 | C2 | RET | B | 296 | 31.971 | 8.464 | 24.824 | 1.00 | 38.11 | B |
| ATOM | 4951 | C3 | RET | B | 296 | 32.212 | 8.731 | 23.365 | 1.00 | 37.14 | B |
| ATOM | 4952 | C4 | RET | B | 296 | 32.732 | 10.181 | 23.125 | 1.00 | 37.19 | B |
| ATOM | 4953 | C5 | RET | B | 296 | 33.753 | 10.633 | 24.167 | 1.00 | 37.13 | B |
| ATOM | 4954 | C6 | RET | B | 296 | 33.968 | 9.922 | 25.308 | 1.00 | 37.31 | B |
| ATOM | 4955 | C7 | RET | B | 296 | 34.958 | 10.330 | 26.330 | 1.00 | 35.96 | B |
| ATOM | 4956 | C8 | RET | B | 296 | 36.234 | 10.482 | 25.906 | 1.00 | 34.78 | B |
| ATOM | 4957 | C9 | RET | B | 296 | 37.457 | 10.860 | 26.638 | 1.00 | 34.56 | B |
| ATOM | 4958 | C10 | RET | B | 296 | 38.679 | 10.972 | 26.050 | 1.00 | 33.42 | B |
| ATOM | 4959 | C11 | RET | B | 296 | 39.948 | 11.346 | 26.699 | 1.00 | 31.77 | B |
| ATOM | 4960 | C12 | RET | B | 296 | 41.196 | 11.450 | 26.156 | 1.00 | 28.71 | B |
| ATOM | 4961 | C13 | RET | B | 296 | 41.813 | 11.248 | 24.811 | 1.00 | 27.08 | B |
| ATOM | 4962 | C14 | RET | B | 296 | 42.994 | 11.781 | 24.481 | 1.00 | 25.44 | B |
| ATOM | 4963 | C15 | RET | B | 296 | 43.630 | 11.590 | 23.165 | 1.00 | 24.74 | B |
| ATOM | 4964 | C16 | RET | B | 296 | 32.836 | 8.633 | 27.112 | 1.00 | 37.07 | B |
| ATOM | 4965 | C17 | RET | B | 296 | 34.161 | 7.424 | 25.437 | 1.00 | 37.93 | B |
| ATOM | 4966 | C18 | RET | B | 296 | 34.487 | 11.915 | 23.784 | 1.00 | 35.57 | B |
| ATOM | 4967 | C19 | RET | B | 296 | 37.211 | 11.099 | 28.087 | 1.00 | 31.84 | B |
| ATOM | 4968 | C20 | RET | B | 296 | 41.052 | 10.395 | 23.847 | 1.00 | 28.77 | B |
| ATOM | 4969 | N | THR | B | 297 | 41.736 | 12.081 | 15.677 | 1.00 | 33.05 | B |
| ATOM | 4970 | CA | THR | B | 297 | 41.418 | 12.536 | 14.316 | 1.00 | 33.12 | B |
| ATOM | 4971 | CB | THR | B | 297 | 41.347 | 11.374 | 13.258 | 1.00 | 31.86 | B |
| ATOM | 4972 | OG1 | THR | B | 297 | 40.251 | 10.493 | 13.549 | 1.00 | 29.77 | B |
| ATOM | 4973 | CG2 | THR | B | 297 | 42.651 | 10.590 | 13.213 | 1.00 | 28.34 | B |
| ATOM | 4974 | C | THR | B | 297 | 40.059 | 13.248 | 14.368 | 1.00 | 35.30 | B |
| ATOM | 4975 | O | THR | B | 297 | 39.620 | 13.857 | 13.387 | 1.00 | 34.43 | B |
| ATOM | 4976 | N | SER | B | 298 | 39.398 | 13.156 | 15.522 | 1.00 | 35.98 | B |
| ATOM | 4977 | CA | SER | B | 298 | 38.110 | 13.792 | 15.725 | 1.00 | 37.81 | B |
| ATOM | 4978 | CB | SER | B | 298 | 37.567 | 13.468 | 17.124 | 1.00 | 38.78 | B |
| ATOM | 4979 | OG | SER | B | 298 | 36.143 | 13.564 | 17.173 | 1.00 | 40.95 | B |
| ATOM | 4980 | C | SER | B | 298 | 38.322 | 15.294 | 15.562 | 1.00 | 39.39 | B |
| ATOM | 4981 | O | SER | B | 298 | 37.406 | 16.037 | 15.177 | 1.00 | 42.60 | B |
| ATOM | 4982 | N | ALA | B | 299 | 39.557 | 15.722 | 15.806 | 1.00 | 38.93 | B |
| ATOM | 4983 | CA | ALA | B | 299 | 39.927 | 17.123 | 15.696 | 1.00 | 38.71 | B |
| ATOM | 4984 | CB | ALA | B | 299 | 41.219 | 17.388 | 16.441 | 1.00 | 37.76 | B |
| ATOM | 4985 | C | ALA | B | 299 | 40.083 | 17.543 | 14.252 | 1.00 | 39.61 | B |
| ATOM | 4986 | O | ALA | B | 299 | 40.271 | 18.724 | 13.973 | 1.00 | 41.81 | B |
| ATOM | 4987 | N | VAL | B | 300 | 39.993 | 16.590 | 13.328 | 1.00 | 39.77 | B |
| ATOM | 4988 | CA | VAL | B | 300 | 40.168 | 16.915 | 11.918 | 1.00 | 38.43 | B |
| ATOM | 4989 | CB | VAL | B | 300 | 41.570 | 16.468 | 11.405 | 1.00 | 36.46 | B |
| ATOM | 4990 | CG1 | VAL | B | 300 | 41.853 | 17.085 | 10.066 | 1.00 | 37.40 | B |
| ATOM | 4991 | CG2 | VAL | B | 300 | 42.658 | 16.860 | 12.377 | 1.00 | 34.76 | B |
| ATOM | 4992 | C | VAL | B | 300 | 39.102 | 16.357 | 10.981 | 1.00 | 39.08 | B |
| ATOM | 4993 | 0 | VAL | B | 300 | 38.724 | 17.029 | 10.023 | 1.00 | 38.52 | B |
| ATOM | 4994 | N | TYR | B | 301 | 38.599 | 15.156 | 11.265 | 1.00 | 40.35 | B |
| ATOM | 4995 | CA | TYR | B | 301 | 37.598 | 14.504 | 10.403 | 1.00 | 41.51 | B |
| ATOM | 4996 | CB | TYR | B | 301 | 37.294 | 13.078 | 10.890 | 1.00 | 39.56 | B |
| ATOM | 4997 | CG | TYR | B | 301 | 36.110 | 12.959 | 11.832 | 1.00 | 37.52 | B |
| ATOM | 4998 | CD1 | TYR | B | 301 | 36.282 | 13.025 | 13.211 | 1.00 | 37.65 | B |
| ATOM | 4999 | CE1 | TYR | B | 301 | 35.200 | 12.917 | 14.078 | 1.00 | 35.05 | B |
| ATOM | 5000 | CD2 | TYR | B | 301 | 34.819 | 12.782 | 11.343 | 1.00 | 35.63 | B |
| ATOM | 5001 | CE2 | TYR | B | 301 | 33.738 | 12.677 | 12.203 | 1.00 | 34.61 | B |
| ATOM | 5002 | CZ | TYR | B | 301 | 33.938 | 12.744 | 13.565 | 1.00 | 33.47 | B |
| ATOM | 5003 | OH | TYR | B | 301 | 32.875 | 12.628 | 14.415 | 1.00 | 34.10 | B |
| ATOM | 5004 | C | TYR | B | 301 | 36.280 | 15.243 | 10.128 | 1.00 | 43.59 | B |
| ATOM | 5005 | O | TYR | B | 301 | 35.698 | 15.072 | 9.058 | 1.00 | 42.93 | B |
| ATOM | 5006 | N | ASN | B | 302 | 35.787 | 16.012 | 11.100 | 1.00 | 46.84 | B |
| ATOM | 5007 | CA | ASN | B | 302 | 34.534 | 16.756 | 10.934 | 1.00 | 48.26 | B |
| ATOM | 5008 | CB | ASN | B | 302 | 34.198 | 17.544 | 12.196 | 1.00 | 49.62 | B |
| ATOM | 5009 | CG | ASN | B | 302 | 33.383 | 16.749 | 13.160 | 1.00 | 50.79 | B |
| ATOM | 5010 | OD1 | ASN | B | 302 | 32.248 | 16.371 | 12.856 | 1.00 | 49.97 | B |
| ATOM | 5011 | ND2 | ASN | B | 302 | 33.953 | 16.476 | 14.339 | 1.00 | 52.10 | B |
| ATOM | 5012 | C | ASN | B | 302 | 34.540 | 17.685 | 9.725 | 1.00 | 49.34 | B |
| ATOM | 5013 | O | ASN | B | 302 | 33.685 | 17.557 | 8.844 | 1.00 | 47.57 | B |
| ATOM | 5014 | N | PRO | B | 303 | 35.494 | 18.642 | 9.670 | 1.00 | 50.64 | B |
| ATOM | 5015 | CD | PRO | B | 303 | 36.487 | 19.046 | 10.685 | 1.00 | 50.43 | B |
| ATOM | 5016 | CA | PRO | B | 303 | 35.540 | 19.553 | 8.522 | 1.00 | 51.60 | B |
| ATOM | 5017 | CB | PRO | B | 303 | 36.600 | 20.580 | 8.941 | 1.00 | 50.73 | B |
| ATOM | 5018 | CG | PRO | B | 303 | 37.477 | 19.822 | 9.867 | 1.00 | 50.78 | B |
| ATOM | 5019 | C | PRO | B | 303 | 35.838 | 18.904 | 7.149 | 1.00 | 52.83 | B |
| ATOM | 5020 | O | PRO | B | 303 | 35.651 | 19.546 | 6.117 | 1.00 | 53.44 | B |
| ATOM | 5021 | N | VAL | B | 304 | 36.279 | 17.644 | 7.126 | 1.00 | 54.30 | B |
| ATOM | 5022 | CA | VAL | B | 304 | 36.561 | 16.957 | 5.856 | 1.00 | 54.36 | B |
| ATOM | 5023 | CB | VAL | B | 304 | 37.618 | 15.830 | 6.008 | 1.00 | 51.93 | B |
| ATOM | 5024 | CG1 | VAL | B | 304 | 38.025 | 15.310 | 4.645 | 1.00 | 49.40 | B |
| ATOM | 5025 | CG2 | VAL | B | 304 | 38.837 | 16.343 | 6.742 | 1.00 | 50.06 | B |
| ATOM | 5026 | C | VAL | B | 304 | 35.265 | 16.388 | 5.255 | 1.00 | 56.15 | B |
| ATOM | 5027 | O | VAL | B | 304 | 35.105 | 16.332 | 4.034 | 1.00 | 55.72 | B |
| ATOM | 5028 | N | ILE | B | 305 | 34.343 | 15.970 | 6.117 | 1.00 | 58.53 | B |
| ATOM | 5029 | CA | ILE | B | 305 | 33.055 | 15.440 | 5.671 | 1.00 | 61.73 | B |
| ATOM | 5030 | CB | ILE | B | 305 | 32.229 | 14.866 | 6.861 | 1.00 | 61.90 | B |
| ATOM | 5031 | CG2 | ILE | B | 305 | 30.805 | 14.538 | 6.419 | 1.00 | 62.23 | B |
| ATOM | 5032 | CG1 | ILE | B | 305 | 32.904 | 13.619 | 7.436 | 1.00 | 61.89 | B |
| ATOM | 5033 | CD1 | ILB | B | 305 | 32.251 | 13.111 | 8.699 | 1.00 | 61.73 | B |
| ATOM | 5034 | C | ILE | B | 305 | 32.275 | 16.605 | 5.072 | 1.00 | 63.37 | B |
| ATOM | 5035 | 0 | ILE | B | 305 | 31.558 | 16.458 | 4.086 | 1.00 | 63.64 | B |
| ATOM | 5036 | N | TYR | B | 306 | 32.456 | 17.767 | 5.686 | 1.00 | 65.69 | B |
| ATOM | 5037 | CA | TYR | B | 306 | 31.797 | 19.005 | 5.295 | 1.00 | 68.12 | B |
| ATOM | 5038 | CB | TYR | B | 306 | 32.254 | 20.111 | 6.260 | 1.00 | 69.83 | B |
| ATOM | 5039 | CG | TYR | B | 306 | 31.297 | 21.269 | 6.501 | 1.00 | 71.53 | B |
| ATOM | 5040 | CD1 | TYR | B | 306 | 29.961 | 21.224 | 6.091 | 1.00 | 71.63 | B |
| ATOM | 5041 | CE1 | TYR | B | 306 | 29.110 | 22.320 | 6.292 | 1.00 | 72.21 | B |
| ATOM | 5042 | CD2 | TYR | B | 306 | 31.752 | 22.435 | 7.124 | 1.00 | 72.15 | B |
| ATOM | 5043 | CE2 | TYR | B | 306 | 30.916 | 23.525 | 7.327 | 1.00 | 72.24 | B |
| ATOM | 5044 | CZ | TYR | B | 306 | 29.604 | 23.468 | 6.910 | 1.00 | 72.37 | B |
| ATOM | 5045 | OH | TYR | B | 306 | 28.809 | 24.572 | 7.106 | 1.00 | 73.36 | B |
| ATOM | 5046 | C | TYR | B | 306 | 32.067 | 19.374 | 3.822 | 1.00 | 68.58 | B |
| ATOM | 5047 | O | TYR | B | 306 | 31.137 | 19.404 | 3.013 | 1.00 | 69.09 | B |
| ATOM | 5048 | N | ILE | B | 307 | 33.329 | 19.616 | 3.470 | 1.00 | 68.85 | B |
| ATOM | 5049 | CA | ILE | B | 307 | 33.706 | 19.972 | 2.094 | 1.00 | 69.45 | B |
| ATOM | 5050 | CB | ILE | B | 307 | 35.244 | 20.126 | 1.958 | 1.00 | 68.98 | B |
| ATOM | 5051 | CG2 | ILE | B | 307 | 35.634 | 20.416 | 0.519 | 1.00 | 68.67 | B |
| ATOM | 5052 | CG1 | ILE | B | 307 | 35.746 | 21.243 | 2.867 | 1.00 | 68.35 | B |
| ATOM | 5053 | CD1 | ILE | B | 307 | 37.243 | 21.297 | 2.975 | 1.00 | 68.78 | B |
| ATOM | 5054 | C | ILE | B | 307 | 33.242 | 18.894 | 1.115 | 1.00 | 70.48 | B |
| ATOM | 5055 | O | ILE | B | 307 | 32.710 | 19.193 | 0.045 | 1.00 | 70.11 | B |
| ATOM | 5056 | N | MET | B | 308 | 33.417 | 17.642 | 1.528 | 1.00 | 71.82 | B |
| ATOM | 5057 | CA | MET | B | 308 | 33.054 | 16.474 | 0.742 | 1.00 | 72.59 | B |
| ATOM | 5058 | CB | MET | B | 308 | 33.459 | 15.216 | 1.503 | 1.00 | 71.96 | B |
| ATOM | 5059 | CG | MET | B | 308 | 34.216 | 14.206 | 0.680 | 1.00 | 73.14 | B |
| ATOM | 5060 | SD | MET | B | 308 | 35.853 | 14.785 | 0.281 | 1.00 | 73.49 | B |
| ATOM | 5061 | CE | MET | B | 308 | 35.605 | 15.396 | -1.387 | 1.00 | 74.45 | B |
| ATOM | 5062 | C | MET | B | 308 | 31.571 | 16.384 | 0.355 | 1.00 | 74.35 | B |
| ATOM | 5063 | O | MET | B | 308 | 31.246 | 16.260 | -0.828 | 1.00 | 76.03 | B |
| ATOM | 5064 | N | MET | B | 309 | 30.673 | 16.451 | 1.338 | 1.00 | 75.68 | B |
| ATOM | 5065 | CA | MET | B | 309 | 29.242 | 16.344 | 1.050 | 1.00 | 76.73 | B |
| ATOM | 5066 | CB | MET | B | 309 | 28.700 | 14.996 | 1.549 | 1.00 | 76.51 | B |
| ATOM | 5067 | CG | MET | B | 309 | 28.871 | 14.736 | 3.034 | 1.00 | 75.75 | B |
| ATOM | 5068 | SD | MET | B | 309 | 28.720 | 12.987 | 3.411 | 1.00 | 76.52 | B |
| ATOM | 5069 | CE | MET | B | 309 | 27.279 | 12.529 | 2.417 | 1.00 | 77.23 | B |
| ATOM | 5070 | C | MET | B | 309 | 28.318 | 17.504 | 1.458 | 1.00 | 77.66 | B |
| ATOM | 5071 | O | MET | B | 309 | 27.458 | 17.369 | 2.335 | 1.00 | 77.83 | B |
| ATOM | 5072 | N | ASN | B | 310 | 28.507 | 18.639 | 0.788 | 1.00 | 78.49 | B |
| ATOM | 5073 | CA | ASN | B | 310 | 27.702 | 19.846 | 0.977 | 1.00 | 78.75 | B |
| ATOM | 5074 | CB | ASH | B | 310 | 27.799 | 20.401 | 2.398 | 1.00 | 76.72 | B |
| ATOM | 5075 | CG | ASN | B | 310 | 26.851 | 21.564 | 2.621 | 1.00 | 74.96 | B |
| ATOM | 5076 | OD1 | ASN | B | 310 | 27.281 | 22.676 | 2.901 | 1.00 | 73.40 | B |
| ATOM | 5077 | ND2 | ASN | B | 310 | 25.553 | 21.317 | 2.457 | 1.00 | 74.47 | B |
| ATOM | 5078 | C | ASN | B | 310 | 28.098 | 20.921 | -0.037 | 1.00 | 79.70 | B |
| ATOM | 5079 | O | ASN | B | 310 | 29.009 | 21.721 | 0.202 | 1.00 | 77.64 | B |
| ATOM | 5080 | N | LYS | B | 311 | 27.392 | 20.918 | -1.169 | 1.00 | 81.71 | B |
| ATOM | 5081 | CA | LYS | B | 311 | 27.620 | 21.858 | -2.266 | 1.00 | 83.03 | B |
| ATOM | 5082 | CB | LYS | B | 311 | 26.539 | 21.685 | -3.346 | 1.00 | 83.63 | B |
| ATOM | 5083 | CG | LYS | B | 311 | 26.971 | 20.890 | -4.582 | 1.00 | 85.01 | B |
| ATOM | 5084 | CD | LYS | B | 311 | 25.933 | 20.987 | -5.706 | 1.00 | 85.72 | B |
| ATOM | 5085 | CE | LYS | B | 311 | 26.497 | 20.515 | -7.045 | 1.00 | 85.94 | B |
| ATOM | 5086 | NZ | LYS | B | 311 | 27.592 | 21.398 | -7.554 | 1.00 | 85.03 | B |
| ATOM | 5087 | C | LYS | B | 311 | 27.655 | 23.313 | -1.808 | 1.00 | 83.94 | B |
| ATOM | 5088 | O | LYS | B | 311 | 28.477 | 24.094 | -2.284 | 1.00 | 83.79 | B |
| ATOM | 5089 | N | GLN | B | 312 | 26.773 | 23.655 | -0.870 | 1.00 | 85.38 | B |
| ATOM | 5090 | CA | GLN | B | 312 | 26.650 | 25.011 | -0.325 | 1.00 | 86.88 | B |
| ATOM | 5091 | CB | GLN | B | 312 | 25.580 | 25.031 | 0.788 | 1.00 | 89.83 | B |
| ATOM | 5092 | CG | GLN | B | 312 | 24.223 | 24.405 | 0.404 | 1.00 | 93.26 | B |
| ATOM | 5093 | CD | GLN | B | 312 | 23.296 | 24.160 | 1.604 | 1.00 | 95.18 | B |
| ATOM | 5094 | OE1 | GLN | B | 312 | 23.184 | 24.995 | 2.510 | 1.00 | 95.86 | B |
| ATOM | 5095 | NE2 | GLN | B | 312 | 22.622 | 23.008 | 1.603 | 1.00 | 95.28 | B |
| ATOM | 5096 | C | GLN | B | 312 | 27.969 | 25.581 | 0.225 | 1.00 | 86.09 | B |
| ATOM | 5097 | O | GLM | B | 312 | 28.277 | 26.758 | 0.023 | 1.00 | 85.90 | B |
| ATOM | 5098 | N | PHE | B | 313 | 28.746 | 24.723 | 0.885 | 1.00 | 85.16 | B |
| ATOM | 5099 | CA | PHE | B | 313 | 30.017 | 25.094 | 1.512 | 1.00 | 83.26 | B |
| ATOM | 5100 | CB | PHE | B | 313 | 30.235 | 24.216 | 2.746 | 1.00 | 82.11 | B |
| ATOM | 5101 | CG | PHE | B | 313 | 31.363 | 24.662 | 3.620 | 1.00 | 80.91 | B |
| ATOM | 5102 | CD1 | PHE | B | 313 | 32.577 | 23.986 | 3.610 | 1.00 | 80.11 | B |
| ATOM | 5103 | CD2 | PHE | B | 313 | 31.211 | 25.757 | 4.462 | 1.00 | 80.84 | B |
| ATOM | 5104 | CE1 | PHE | B | 313 | 33.625 | 24.394 | 4.424 | 1.00 | 80.12 | B |
| ATOM | 5105 | CE2 | PHE | B | 313 | 32.252 | 26.175 | 5.282 | 1.00 | 80.56 | B |
| ATOM | 5106 | CZ | PHE | B | 313 | 33.463 | 25.492 | 5.264 | 1.00 | 80.63 | B |
| ATOM | 5107 | C | PHE | B | 313 | 31.253 | 25.033 | 0.609 | 1.00 | 83.10 | B |
| ATOM | 5108 | O | PHE | B | 313 | 32.064 | 25.958 | 0.616 | 1.00 | 82.08 | B |
| ATOM | 5109 | N | ARG | B | 314 | 31.405 | 23.928 | -0.128 | 1.00 | 83.78 | B |
| ATOM | 5110 | CA | ARG | B | 314 | 32.540 | 23.708 | -1.042 | 1.00 | 83.58 | B |
| ATOM | 5111 | CB | ARG | B | 314 | 32.363 | 22.364 | -1.797 | 1.00 | 85.54 | B |
| ATOM | 5112 | CG | ARG | B | 314 | 33.656 | 21.699 | -2.335 | 1.00 | 87.80 | B |
| ATOM | 5113 | CD | ARG | B | 314 | 33.423 | 20.268 | -2.933 | 1.00 | 90.32 | B |
| ATOM | 5114 | NE | ARG | B | 314 | 34.682 | 19.507 | -3.096 | 1.00 | 93.13 | B |
| ATOM | 5115 | CZ | ARG | B | 314 | 34.794 | 18.261 | -3.578 | 1.00 | 92.42 | B |
| ATOM | 5116 | NH1 | ARG | B | 314 | 33.725 | 17.577 | -3.975 | 1.00 | 92.23 | B |
| ATOM | 5117 | NH2 | ARG | B | 314 | 35.989 | 17.678 | -3.633 | 1.00 | 90.83 | B |
| ATOM | 5118 | C | ARG | B | 314 | 32.673 | 24.888 | -2.021 | 1.00 | 82.63 | B |
| ATOM | 5119 | O | ARG | B | 314 | 33.783 | 25.331 | -2.313 | 1.00 | 82.49 | B |
| ATOM | 5120 | N | ASN | B | 315 | 31.533 | 25.406 | -2.491 | 1.00 | 81.19 | B |
| ATOM | 5121 | CA | ASN | B | 315 | 31.488 | 26.547 | -3.416 | 1.00 | 78.77 | B |
| ATOM | 5122 | CB | ASN | B | 315 | 30.071 | 26.754 | -3.979 | 1.00 | 79.64 | B |
| ATOM | 5123 | CG | ASN | B | 315 | 29.701 | 25.752 | -5.062 | 1.00 | 79.78 | B |
| ATOM | 5124 | OD1 | ASN | B | 315 | 30.535 | 24.972 | -5.522 | 1.00 | 79.80 | B |
| ATOM | 5125 | ND2 | ASN | B | 315 | 28.438 | 25.782 | -5.485 | 1.00 | 79.84 | B |
| ATOM | 5126 | C | ASN | B | 315 | 31.874 | 27.808 | -2.666 | 1.00 | 76.34 | B |
| ATOM | 5127 | O | ASN | B | 315 | 32.644 | 28.625 | -3.161 | 1.00 | 76.19 | B |
| ATOM | 5128 | N | CYS | B | 316 | 31.307 | 27.958 | -1.473 | 1.00 | 73.90 | B |
| ATOM | 5129 | CA | CYS | B | 316 | 31.556 | 29.116 | -0.629 | 1.00 | 71.70 | B |
| ATOM | 5130 | CB | CYS | B | 316 | 30.441 | 29.252 | 0.419 | 1.00 | 71.82 | B |
| ATOM | 5131 | SG | CYS | B | 316 | 28.779 | 29.594 | -0.309 | 1.00 | 67.40 | B |
| ATOM | 5132 | C | CYS | B | 316 | 32.952 | 29.131 | 0.001 | 1.00 | 70.45 | B |
| ATOM | 5133 | O | CYS | B | 316 | 33.441 | 30.181 | 0.412 | 1.00 | 70.02 | B |
| ATOM | 5134 | N | MET | B | 317 | 33.594 | 27.970 | 0.065 | 1.00 | 69.49 | B |
| ATOM | 5135 | CA | MET | B | 317 | 34.946 | 27.881 | 0.594 | 1.00 | 69.28 | B |
| ATOM | 5136 | CB | MET | B | 317 | 35.290 | 26.443 | 0.982 | 1.00 | 68.48 | B |
| ATOM | 5137 | CG | MET | B | 317 | 36.775 | 26.205 | 1.275 | 1.00 | 66.61 | B |
| ATOM | 5138 | SD | MET | B | 317 | 37.248 | 24.453 | 1.328 | 1.00 | 65.00 | B |
| ATOM | 5139 | CE | MET | B | 317 | 37.840 | 24.183 | -0.345 | 1.00 | 63.53 | B |
| ATOM | 5140 | C | MET | B | 317 | 35.863 | 28.324 | -0.534 | 1.00 | 71.27 | B |
| ATOM | 5141 | O | MET | B | 317 | 36.776 | 29.119 | -0.326 | 1.00 | 71.91 | B |
| ATOM | 5142 | N | VAL | B | 318 | 35.595 | 27.812 | -1.735 | 1.00 | 73.65 | B |
| ATOM | 5143 | CA | VAL | B | 318 | 36.379 | 28.130 | -2.930 | 1.00 | 75.99 | B |
| ATOM | 5144 | CB | VAL | B | 318 | 35.853 | 27.346 | -4.174 | 1.00 | 75.39 | B |
| ATOM | 5145 | CG1 | VAL | B | 318 | 36.488 | 27.862 | -5.455 | 1.00 | 75.23 | B |
| ATOM | 5146 | CG2 | VAL | B | 318 | 36.165 | 25.866 | -4.026 | 1.00 | 75.75 | B |
| ATOM | 5147 | C | VAL | B | 318 | 36.399 | 29.635 | -3.216 | 1.00 | 78.29 | B |
| ATOM | 5148 | O | VAL | B | 318 | 37.426 | 30.180 | -3.632 | 1.00 | 78.85 | B |
| ATOM | 5149 | N | THR | B | 319 | 35.275 | 30.302 | -2.954 | 1.00 | 80.48 | B |
| ATOM | 5150 | CA | THR | B | 319 | 35.150 | 31.745 | -3.171 | 1.00 | 82.50 | B |
| ATOM | 5151 | CB | THR | B | 319 | 33.668 | 32.200 | -3.072 | 1.00 | 82.78 | B |
| ATOM | 5152 | OG1 | THR | B | 319 | 32.860 | 31.403 | -3.949 | 1.00 | 83.02 | B |
| ATOM | 5153 | CG2 | THR | B | 319 | 33.526 | 33.663 | -3.476 | 1.00 | 83.31 | B |
| ATOM | 5154 | C | THR | B | 319 | 36.009 | 32.543 | -2.177 | 1.00 | 83.66 | B |
| ATOM | 5155 | O | THR | B | 319 | 36.381 | 33.691 | -2.445 | 1.00 | 84.48 | B |
| ATOM | 5156 | N | THR | B | 320 | 36.325 | 31.921 | -1.041 | 1.00 | 84.41 | B |
| ATOM | 5157 | CA | THR | B | 320 | 37.148 | 32.536 | 0.002 | 1.00 | 85.04 | B |
| ATOM | 5158 | CB | THR | B | 320 | 36.924 | 31.832 | 1.364 | 1.00 | 84.74 | B |
| ATOM | 5159 | OG1 | THR | B | 320 | 35.544 | 31.938 | 1.734 | 1.00 | 84.79 | B |
| ATOM | 5160 | CG2 | THR | B | 320 | 37.789 | 32.451 | 2.454 | 1.00 | 84.08 | B |
| ATOM | 5161 | C | THR | B | 320 | 38.633 | 32.447 | -0.363 | 1.00 | 85.83 | B |
| ATOM | 5162 | O | THR | B | 320 | 39.427 | 33.327 | -0.016 | 1.00 | 85.51 | B |
| ATOM | 5163 | N | LEU | B | 321 | 38.989 | 31.381 | -1.076 | 1.00 | 86.96 | B |
| ATOM | 5164 | CA | LEU | B | 321 | 40.366 | 31.136 | -1.493 | 1.00 | 88.25 | B |
| ATOM | 5165 | CB | LEU | B | 321 | 40.619 | 29.622 | -1.594 | 1.00 | 85.33 | B |
| ATOM | 5166 | CG | LEU | B | 321 | 40.345 | 28.738 | -0.369 | 1.00 | 82.12 | B |
| ATOM | 5167 | CD1 | LEU | B | 321 | 40.487 | 27.288 | -0.754 | 1.00 | 81.32 | B |
| ATOM | 5168 | CD2 | LEU | B | 321 | 41.284 | 29.066 | 0.771 | 1.00 | 80.53 | B |
| ATOM | 5169 | C | LEU | B | 321 | 40.775 | 31.838 | -2.803 | 1.00 | 90.63 | B |
| ATOM | 5170 | O | LEU | B | 321 | 41.848 | 32.444 | -2.872 | 1.00 | 90.99 | B |
| ATOM | 5171 | N | CYS | B | 322 | 39.925 | 31.767 | -3.829 | 1.00 | 92.98 | B |
| ATOM | 5172 | CA | CYS | B | 322 | 40.227 | 32.386 | -5.121 | 1.00 | 95.39 | B |
| ATOM | 5173 | CB | CYS | B | 322 | 39.528 | 31.645 | -6.252 | 1.00 | 93.92 | B |
| ATOM | 5174 | SG | CYS | B | 322 | 40.449 | 30.197 | -6.772 | 1.00 | 93.30 | B |
| ATOM | 5175 | C | CYS | B | 322 | 39.963 | 33.878 | -5.232 | 1.00 | 98.18 | B |
| ATOM | 5176 | O | CYS | B | 322 | 40.239 | 34.485 | -6.271 | 1.00 | 98.25 | B |
| ATOM | 5178 | N | CYS | B | 323 | 39.425 | 34.459 | -4.162 | 1.00 | 101.67 | B |
| ATOM | 5178 | CA | CYS | B | 323 | 39.130 | 35.891 | -4.091 | 1.00 | 105.54 | B |
| ATOM | 5179 | CB | CYS | B | 323 | 40.441 | 36.687 | -4.031 | 1.00 | 106.50 | B |
| ATOM | 5180 | SG | CYS | B | 323 | 41.611 | 36.129 | -2.753 | 1.00 | 107.31 | B |
| ATOM | 5181 | C | CYS | B | 323 | 38.242 | 36.408 | -5.232 | 1.00 | 107.70 | B |
| ATOM | 5182 | 0 | CYS | B | 323 | 38.528 | 37.453 | -5.836 | 1.00 | 107.52 | B |
| ATOM | 5183 | N | GLY | B | 324 | 37.175 | 35.665 | -5.528 | 1.00 | 110.15 | B |
| ATOM | 5184 | CA | GLY | B | 324 | 36.257 | 36.062 | -6.585 | 1.00 | 112.37 | B |
| ATOM | 5185 | C | GLY | B | 324 | 35.942 | 35.006 | -7.635 | 1.00 | 113.39 | B |
| ATOM | 5186 | O | GLY | B | 324 | 34.772 | 34.685 | -7.863 | 1.00 | 113.56 | B |
| ATOM | 5187 | N | LYS | B | 325 | 36.981 | 34.487 | -8.290 | 1.00 | 114.16 | B |
| ATOM | 5188 | CA | LYS | B | 325 | 36.832 | 33.472 | -9.335 | 1.00 | 114.70 | B |
| ATOM | 5189 | CB | LYS | B | 325 | 38.206 | 33.096 | -9.917 | 1.00 | 114.20 | B |
| ATOM | 5190 | CG | LYS | B | 325 | 38.920 | 34.213 | -10.685 | 1.00 | 113.67 | B |
| ATOM | 5191 | CD | LYS | B | 325 | 39.283 | 35.404 | -9.795 | 1.00 | 113.81 | B |
| ATOM | 5192 | CE | LYS | B | 325 | 39.872 | 36.556 | -10.601 | 1.00 | 114.17 | B |
| ATOM | 5193 | NZ | LYS | B | 325 | 40.069 | 37.778 | -9.772 | 1.00 | 113.16 | B |
| ATOM | 5194 | C | LYS | B | 325 | 36.109 | 32.227 | -8.809 | 1.00 | 115.27 | B |
| ATOM | 5195 | O | LYS | B | 325 | 36.720 | 31.348 | -8.196 | 1.00 | 115.29 | B |
| ATOM | 5196 | N | ASN | B | 326 | 34.800 | 32.172 | -9.053 | 1.00 | 115.86 | B |
| ATOM | 5197 | CA | ASN | B | 326 | 33.951 | 31.065 | -8.607 | 1.00 | 115.98 | B |
| ATOM | 5198 | CB | ASN | B | 326 | 32.481 | 31.517 | -8.574 | 1.00 | 115.47 | B |
| ATOM | 5199 | CG | ASN | B | 326 | 31.624 | 30.678 | -7.636 | 1.00 | 114.92 | B |
| ATOM | 5200 | OD1 | ASN | B | 326 | 32.059 | 29.642 | -7.123 | 1.00 | 114.01 | B |
| ATOM | 5201 | ND2 | ASN | B | 326 | 30.399 | 31.134 | -7.398 | 1.00 | 114.75 | B |
| ATOM | 5202 | C | ASN | B | 326 | 34.110 | 29.819 | -9.486 | 1.00 | 115.97 | B |
| ATOM | 5203 | O | ASN | B | 326 | 34.637 | 28.810 | -8.970 | 1.00 | 115.78 | B |
| ATOM | 5204 | OT | ASN | B | 326 | 33.705 | 29.860 | -10.671 | 1.00 | 116.11 | B |
| ATOM | 5205 | OH2 | WAT | | 953 | 53.913 | 9.916 | -31.259 | 1.00 | 41.73 | |
| ATOM | 5206 | OH2 | WAT | | 956 | 57.696 | -2.053 | 43.265 | 1.00 | 41.22 | |
| ATOM | 5207 | OH2 | WAT | | 957 | 48.693 | 4.852 | -18.060 | 1.00 | 24.01 | |
| ATOM | 5208 | OH2 | WAT | | 960 | 33.888 | 27.069 | -11.307 | 1.00 | 32.67 | |
| ATOM | 5209 | OH2 | WAT | | 961 | 63.709 | 16.988 | 43.214 | 1.00 | 36.42 | |
| ATOM | 5210 | OH2 | WAT | | 962 | 27.271 | 2.233 | 41.823 | 1.00 | 36.67 | |
| ATOM | 5211 | OH2 | WAT | | 964 | 15.610 | 6.952 | 2.044 | 1.00 | 37.53 | |
| ATOM | 5212 | OH2 | WAT | | 965 | 45.976 | 8.381 | -11.066 | 1.00 | 14.67 | |
| ATOM | 5213 | OH2 | WAT | | 966 | 56.871 | -11.644 | -13.182 | 1.00 | 35.46 | |
| ATOM | 5214 | OH2 | WAT | | 967 | 30.304 | 2.564 | 43.209 | 1.00 | 18.38 | |
| ATOM | 5215 | OH2 | WAT | | 969 | 63.220 | 4.609 | -36.622 | 1.00 | 30.11 | |
| ATOM | 5216 | OH2 | WAT | | 970 | 61.784 | 21.128 | 26.096 | 1.00 | 35.05 | |
| ATOM | 5217 | OH2 | WAT | | 971 | 89.670 | 5.459 | 7.567 | 1.00 | 31.75 | |
| ATOM | 5218 | OH2 | WAT | | 972 | 59.969 | -6.175 | -22.347 | 1.00 | 37.13 | |
| ATOM | 5219 | OH2 | WAT | | 974 | 45.137 | 20.260 | -15.703 | 1.00 | 29.76 | |
| ATOM | 5220 | OH2 | WAT | | 975 | 55.915 | -17.781 | -20.267 | 1.00 | 29.16 | |
| ATOM | 5221 | OH2 | WAT | | 976 | 41.975 | -2.817 | -17.030 | 1.00 | 25.63 | |
| ATOM | 5222 | OH2 | WAT | | 980 | 4.811 | 32.025 | 11.115 | 1.00 | 35.45 | |
| ATOM | 5223 | OH2 | WAT | | 982 | 45.771 | -10.820 | -26.055 | 1.00 | 28.68 | |
| ATOM | 5224 | OH2 | WAT | | 986 | 25.963 | 26.533 | -7.731 | 1.00 | 12.74 | |
| ATOM | 5225 | OH2 | WAT | | 987 | 65.137 | 3.207 | -27.249 | 1.00 | 22.76 | |
| ATOM | 5226 | OH2 | WAT | | 988 | 66.257 | 7.500 | 39.600 | 1.00 | 44.93 | |
| ATOM | 5227 | OH2 | WAT | | 989 | 41.842 | 37.561 | -7.728 | 1.00 | 37.92 | |
| ATOM | 5228 | OH2 | WAT | | 992 | 52.765 | -0.772 | -21.390 | 1.00 | 25.73 | |
| ATOM | 5229 | OH2 | WAT | | 993 | 45.447 | -12.557 | -30.728 | 1.00 | 12.66 | |
| ATOM | 5230 | OH2 | WAT | | 997 | 56.459 | 2.341 | -32.514 | 1.00 | 42.07 | |
| ATOM | 5231 | OH2 | WAT | | 998 | 52.227 | 11.940 | -32.300 | 1.00 | 31.62 | |
| ATOM | 5232 | OH2 | WAT | | 999 | 62.677 | 0.656 | -28.418 | 1.00 | 32.09 | |
| ATOM | 5233 | OH2 | WAT | | 1000 | 48.299 | 7.644 | -20.117 | 1.00 | 16.54 | |
| ATOM | 5234 | OH2 | WAT | | 1001 | 55.271 | 4.766 | -3.712 | 1.00 | 20.72 | |
| ATOM | 5235 | OH2 | WAT | | 1002 | 53.890 | -6.325 | -8.895 | 1.00 | 17.25 | |
| ATOM | 5236 | OH2 | WAT | | 1003 | 39.361 | 6.475 | -1.390 | 1.00 | 41.40 | |
| ATOM | 5237 | OH2 | WAT | | 1004 | 45.211 | 7.251 | -2.669 | 1.00 | 11.67 | |
| ATOM | 5238 | OH2 | WAT | | 1005 | 38.939 | -2.110 | -7.622 | 1.00 | 23.05 | |
| ATOM | 5239 | OH2 | WAT | | 1006 | 51.790 | -8.851 | -10.509 | 1.00 | 11.04 | |
| ATOM | 5240 | OH2 | WAT | | 1007 | 48.972 | -6.522 | -11.350 | 1.00 | 23.14 | |
| ATOM | 5241 | OH2 | WAT | | 1011 | 65.749 | -1.044 | -33.726 | 1.00 | 26.43 | |
| ATOM | 5242 | OH2 | WAT | | 1013 | 48.494 | 3.326 | -15.517 | 1.00 | 36.74 | |
| ATOM | 5243 | OH2 | WAT | | 1016 | 24.891 | 4.670 | -0.996 | 1.00 | 35.61 | |
| ATOM | 5244 | OH2 | WAT | | 1017 | 37.931 | 19.049 | -2.376 | 1.00 | 16.88 | |
| ATOM | 5245 | OH2 | WAT | | 1018 | 38.360 | 11.376 | 6.697 | 1.00 | 31.52 | |
| ATOM | 5246 | OH2 | WAT | | 1019 | 33.617 | 8.373 | 7.513 | 1.00 | 8.59 | |
| ATOM | 5247 | OH2 | WAT | | 1020 | 23.737 | 5.532 | 11.924 | 1.00 | 24.20 | |
| ATOM | 5248 | OH2 | WAT | | 1024 | 40.934 | 12.629 | 7.938 | 1.00 | 8.85 | |
| ATOM | 5249 | OH2 | WAT | | 1025 | 40.053 | 11.850 | 2.670 | 1.00 | 23.45 | |
| ATOM | 5250 | OH2 | WAT | | 1026 | 46.429 | 13.194 | 4.378 | 1.00 | 23.75 | |
| ATOM | 5251 | OH2 | WAT | | 1027 | 53.020 | 12.008 | 5.967 | 1.00 | 18.30 | |
| ATOM | 5252 | OH2 | WAT | | 1028 | 51.838 | 1.707 | -1.574 | 1.00 | 23.60 | |
| ATOM | 5253 | OH2 | WAT | | 1029 | 53.174 | -0.673 | 17.026 | 1.00 | 29.69 | |
| ATOM | 5254 | OH2 | WAT | | 1030 | 60.075 | -2.752 | 16.285 | 1.00 | 18.53 | |
| ATOM | 5255 | OH2 | WAT | | 1031 | 57.140 | -3.814 | 15.835 | 1.00 | 19.18 | |
| ATOM | 5256 | OH2 | WAT | | 1032 | 58.304 | 0.394 | 11.516 | 1.00 | 38.89 | |
| ATOM | 5257 | OH2 | WAT | | 1033 | 61.312 | 3.285 | 10.939 | 1.00 | 32.15 | |
| ATOM | 5258 | OH2 | WAT | | 1035 | 63.136 | 15.447 | 29.402 | 1.00 | 34.06 | |
| ATOM | 5259 | OH2 | WAT | | 1036 | 82.241 | 5.815 | 17.976 | 1.00 | 25.42 | |
| ATOM | 5260 | OH2 | WAT | | 1038 | 79.465 | 32.501 | 13.073 | 1.00 | 11.23 | |
| ATOM | 5261 | OH2 | WAT | | 1039 | 79.216 | 27.939 | 13.962 | 1.00 | 20.32 | |
| ATOM | 5262 | OH2 | WAT | | 1040 | 83.625 | 34.117 | 4.154 | 1.00 | 27.91 | |
| ATOM | 5263 | OH2 | WAT | | 1041 | 72.943 | 34.850 | 5.629 | 1.00 | 32.55 | |
| ATOM | 5264 | OH2 | WAT | | 1042 | 70.005 | 32.721 | 3.931 | 1.00 | 33.09 | |
| ATOM | 5265 | OH2 | WAT | | 1043 | 75.035 | 36.861 | 5.711 | 1.00 | 26.45 | |
| ATOM | 5266 | OH2 | WAT | | 1044 | 72.133 | 38.485 | 8.094 | 1.00 | 41.84 | |
| ATOM | 5267 | OH2 | WAT | | 1045 | 77.254 | 22.274 | 14.258 | 1.00 | 31.98 | |
| ATOM | 5268 | OH2 | WAT | | 1046 | 70.808 | 37.464 | 23.933 | 1.00 | 38.59 | |
| ATOM | 5269 | OH2 | WAT | | 1050 | 49.710 | 26.774 | 23.090 | 1.00 | 12.15 | |
| ATOM | 5270 | OH2 | WAT | | 1051 | 54.596 | 23.922 | 21.214 | 1.00 | 32.99 | |
| ATOM | 5271 | OH2 | WAT | | 1052 | 53.984 | 22.762 | 16.617 | 1.00 | 24.07 | |
| ATOM | 5272 | OH2 | WAT | | 1053 | 51.272 | 26.798 | 12.270 | 1.00 | 26.72 | |
| ATOM | 5273 | OH2 | WAT | | 1054 | 43.278 | 22.320 | 32.646 | 1.00 | 10.89 | |
| ATOM | 5274 | OH2 | WAT | | 1055 | 37.413 | 26.489 | 27.956 | 1.00 | 28.32 | |
| ATOM | 5275 | OH2 | WAT | | 1057 | 31.077 | 14.193 | 13.681 | 1.00 | 18.38 | |
| ATOM | 5276 | OH2 | WAT | | 1058 | 23.204 | 12.489 | 20.424 | 1.00 | 44.02 | |
| ATOM | 5277 | OH2 | WAT | | 1059 | 47.668 | 3.781 | 32.197 | 1.00 | 30.06 | |
| ATOM | 5278 | OH2 | WAT | | 1060 | 50.939 | -4.121 | 24.751 | 1.00 | 9.34 | |
| ATOM | 5279 | OH2 | WAT | | 1061 | 47.779 | 5.603 | 16.212 | 1.00 | 17.20 | |
| ATOM | 5280 | OH2 | WAT | | 1062 | 43.163 | -1.845 | 20.479 | 1.00 | 23.74 | |
| ATOM | 5281 | OH2 | WAT | | 1063 | 41.909 | 1.748 | 16.597 | 1.00 | 43.68 | |
| ATOM | 5282 | OH2 | WAT | | 1070 | 42.975 | 3.359 | 44.850 | 1.00 | 30.99 | |
| ATOM | 5283 | OH2 | WAT | | 1073 | 67.697 | 13.092 | -10.446 | 1.00 | 54.00 | |
| ATOM | 5284 | OH2 | WAT | | 1074 | 71.480 | 14.327 | -20.030 | 1.00 | 22.44 | |
| ATOM | 5285 | OH2 | WAT | | 1075 | 77.293 | -2.434 | 0.752 | 1.00 | 31.26 | |
| ATOM | 5286 | OH2 | WAT | | 1076 | 79.159 | -2.050 | 2.806 | 1.00 | 43.11 | |
| ATOM | 5287 | OH2 | WAT | | 1078 | 61.524 | 25.307 | -11.731 | 1.00 | 31.57 | |
| ATOM | 5288 | OH2 | WAT | | 1079 | 79.455 | 18.433 | -5.909 | 1.00 | 31.52 | |
| ATOM | 5289 | OH2 | WAT | | 1080 | 67.684 | 38.913 | 17.286 | 1.00 | 35.93 | |
| ATOM | 5290 | OH2 | WAT | | 1081 | 43.486 | 34.420 | -5.591 | 1.00 | 39.96 | |
| ATOM | 5291 | OH2 | WAT | | 1082 | 64.553 | 23.788 | 32.156 | 1.00 | 27.64 | |
| ATOM | 5292 | OH2 | WAT | | 1083 | 30.762 | 13.635 | -3.241 | 1.00 | 31.60 | |
| ATOM | 5293 | OH2 | WAT | | 1084 | 39.782 | 19.522 | -26.715 | 1.00 | 42.36 | |
| ATOM | 5294 | OH2 | WAT | | 1085 | 37.920 | 21.630 | -29.215 | 1.00 | 28.98 | |
| ATOM | 5295 | OH2 | WAT | | 1086 | 43.431 | -2.928 | -11.223 | 1.00 | 6.22 | |
| ATOM | 5296 | OH2 | WAT | | 1087 | 32.514 | 11.440 | -2.914 | 1.00 | 37.54 | |
| ATOM | 5297 | OH2 | WAT | | 1089 | 77.691 | 22.271 | -9.133 | 1.00 | 49.70 | |
| ATOM | 5298 | OH2 | WAT | | 10890 | 74.297 | 13.373 | -15.590 | 1.00 | 20.65 | |
| ATOM | 5299 | OH2 | WAT | | 1091 | 75.613 | 19.439 | -17.439 | 1.00 | 50.06 | |
| ATOM | 5300 | OH2 | WAT | | 1092 | 67.967 | 15.630 | -24.059 | 1.00 | 11.64 | |
| ATOM | 5301 | OH2 | WAT | | 1093 | 56.494 | 22.470 | -18.207 | 1.00 | 29.66 | |
| ATOM | 5302 | OH2 | WAT | | 1094 | 50.974 | -6.630 | -30.593 | 1.00 | 50.58 | |
| ATOM | 5303 | OH2 | WAT | | 1095 | 55.246 | -3.921 | -26.698 | 1.00 | 48.06 | |
| ATOM | 5304 | OH2 | WAT | | 1096 | 52.411 | -5.738 | -27.300 | 1.00 | 37.70 | |
| ATOM | 5305 | OH2 | WAT, | | 1099 | 54.349 | 0.788 | 2.241 | 1.00 | 31.86 | |
| ATOM | 5306 | OH2 | WAT | | 1101 | 61.950 | -1.675 | -7.346 | 1.00 | 20.67 | |
| ATOM | 5307 | OH2 | WAT | | 1102 | 50.741 | -1.708 | -5.388 | 1.00 | 30.66 | |
| ATOM | 5308 | OH2 | WAT | | 1103 | 47.627 | 6.817 | -0.692 | 1.00 | 30.79 | |
| ATOM | 5309 | OH2 | WAT | | 1104 | 61.714 | 11.915 | -1.183 | 1.00 | 49.70 | |
| ATOM | 5310 | OH2 | WAT | | 1105 | 59.768 | 9.614 | 12.849 | 1.00 | 16.74 | |
| ATOM | 5311 | OH2 | WAT | | 1106 | 65.159 | 12.105 | 18.290 | 1.00 | 17.95 | |
| ATOM | 5312 | OH2 | WAT | | 1107 | 63.693 | 7.802 | 16.545 | 1.00 | 21.10 | |
| ATOM | 5313 | OH2 | WAT | | 1109 | 62.963 | 20.214 | 28.973 | 1.00 | 37.91 | |
| ATOM | 5314 | OH2 | WAT | | 1113 | 73.949 | 25.515 | 26.500 | 1.00 | 38.67 | |
| ATOM | 5315 | OH2 | WAT | | 1114 | 48.747 | -1.771 | 41.770 | 1.00 | 20.02 | |
| ATOM | 5316 | OH2 | WAT | | 1116 | 47.201 | 12.467 | 43.915 | 1.00 | 26.66 | |
| ATOM | 5317 | OH2 | WAT | | 1117 | 56.207 | 10.614 | 9.300 | 1.00 | 20.84 | |
| ATOM | 5318 | OH2 | WAT | | 1118 | 46.498 | 13.040 | 9.645 | 1.00 | 16.94 | |
| ATOM | 5319 | OH2 | WAT | | 1119 | 41.677 | 31.007 | 5.484 | 1.00 | 34.21 | |
| ATOM | 5320 | OH2 | WAT | | 1120 | 33.082 | 33.712 | 15.589 | 1.00 | 31.25 | |
| ATOM | 5321 | OH2 | WAT | | 1125 | 53.578 | 17.029 | 29.102 | 1.00 | 54.39 | |
| ATOM | 5322 | OH2 | WAT | | 1126 | 40.126 | 23.609 | 43.572 | 1.00 | 42.59 | |
| ATOM | 5323 | OH2 | WAT | | 1127 | 43.903 | 22.002 | 43.326 | 1.00 | 37.80 | |
| ATOM | 5324 | OH2 | WAT | | 1128 | 34.165 | 23.338 | 43.252 | 1.00 | 60.20 | |
| ATOM | 5325 | OH2 | WAT | | 1129 | 42.955 | 25.744 | 40.297 | 1.00 | 43.55 | |
| ATOM | 5326 | OH2 | WAT | | 1130 | 40.234 | 27.438 | 42.317 | 1.00 | 49.39 | |
| ATOM | 5327 | OH2 | WAT | | 1132 | 34.831 | 25.934 | 45.691 | 1.00 | 34.67 | |
| ATOM | 5328 | OH2 | WAT | | 1133 | 32.002 | 27.666 | 44.841 | 1.00 | 48.38 | |
| ATOM | 5329 | OH2 | WAT | | 1134 | 33.848 | 23.686 | 47.690 | 1.00 | 29.08 | |
| ATOM | 5330 | OH2 | WAT | | 1135 | 28.991 | 26.854 | 48.167 | 1.00 | 15.30 | |
| ATOM | 5331 | OH2 | WAT | | 1136 | 32.130 | 16.400 | 44.102 | 1.00 | 35.55 | |
| ATOM | 5332 | OH2 | WAT | | 1137 | 30.283 | 13.820 | 43.194 | 1.00 | 9.98 | |
| ATOM | 5333 | OH2 | WAT | | 1138 | 48.321 | 27.265 | 38.177 | 1.00 | 48.00 | |
| ATOM | 5334 | OH2 | WAT | | 1139 | 51.449 | 25.575 | 37.454 | 1.00 | 26.46 | |
| ATOM | 5335 | OH2 | WAT | | 1140 | 44.074 | 22.628 | 35.709 | 1.00 | 38.15 | |
| ATOM | 5336 | OH2 | WAT | | 1141 | 37.862 | 30.118 | 40.823 | 1.00 | 46.99 | |
| ATOM | 5337 | OH2 | WAT | | 1143 | 43.601 | 36.250 | 32.951 | 1.00 | 50.69 | |
| ATOM | 5338 | OH2 | WAT | | 1144 | 48.131 | 27.139 | 33.409 | 1.00 | 45.07 | |
| ATOM | 5339 | OH2 | WAT | | 1145 | 49.374 | 27.310 | 30.343 | 1.00 | 40.90 | |
| ATOM | 5340 | OH2 | WAT | | 1146 | 51.656 | 23.495 | 30.980 | 1.00 | 34.63 | |
| ATOM | 5341 | OH2 | WAT | | 1148 | 40.259 | 31.824 | 33.951 | 1.00 | 32.25 | |
| ATOM | 5342 | OH2 | WAT | | 1149 | 43.303 | 29.383 | 40.123 | 1.00 | 53.84 | |
| ATOM | 5343 | OH2 | WAT | | 1150 | 43.5959 | 32.246 | 38.437 | 1.00 | 57.69 | |
| ATOM | 5344 | OH2 | WAT | | 1151 | 46.556 | 30.993 | 39.694 | 1.00 | 40.86 | |
| ATOM | 5345 | OH2 | WAT | | 1152 | 46.624 | 31.005 | 42.503 | 1.00 | 27.58 | |
| ATOM | 5346 | OH2 | WAT | | 1153 | 51.937 | 33.587 | 36.279 | 1.00 | 72.22 | |
| ATOM | 5347 | OH2 | WAT | | 1154 | 43.315 | 38.652 | 27.775 | 1.00 | 51.33 | |
| ATOM | 5348 | OH2 | WAT | | 1155 | 44.255 | 38.349 | 30.528 | 1.00 | 64.73 | |
| ATOM' | 5349 | OH2 | WAT | | 1156 | 46.784 | 40.192 | 32.331 | 1.00 | 42.28 | |
| ATOM | 5350 | OH2 | WAT | | 1157 | 52.483 | 35.355 | 31.663 | 1.00 | 46.93 | |
| ATOM | 5351 | OH2 | WAT | | 1158 | 40.813 | 42.731 | 28.896 | 1.00 | 49.57 | |
| ATOM | 5352 | OH2 | WAT | | 1160 | 14.320 | 32.912 | 7.669 | 1.00 | 41.93 | |
| ATOM | 5353 | OH2 | WAT | | 1162 | 10.204 | 25.817 | 4.927 | 1.00 | 50.26 | |
| ATOM | 5354 | OH2 | WAT | | 1163 | 13.519 | 26.496 | 4.953 | 1.00 | 37.28 | |
| ATOM | 5355 | OH2 | WAT | | 1164 | 30.958 | -1.556 | 37.591 | 1.00 | 47.66 | |
| ATOM | 5356 | OH2 | WAT | | 1165 | 26.512 | -3.662 | 42.716 | 1.00 | 37.02 | |
| ATOM | 5357 | OH2 | WAT | | 1166 | 26.382 | 9.048 | 45.911 | 1.00 | 52.79 | |
| ATOM | 5358 | OH2 | WAT | | 1169 | 9.761 | 17.242 | -6.985 | 1.00 | 90.24 | |
| ATOM | 5359 | OH2 | WAT | | 1171 | 21.804 | 21.889 | -2.245 | 1.00 | 51.81 | |
| ATOM | 5360 | OH2 | WAT | | 1172 | 31.612 | 6.434 | 6.178 | 1.00 | 41.00 | |
| ATOM | 5361 | OH2 | WAT | | 1173 | 31.787 | 3.027 | 4.544 | 1.00 | 36.50 | |
| ATOM | 5362 | OH2 | WAT | | 1174 | 30.409 | 11.976 | -1.079 | 1.00 | 25.70 | |
| ATOM | 5363 | OH2 | WAT | | 1175 | 37.985 | -4.085 | 22.286 | 1.00 | 22.60 | |
| ATOM | 5364 | OH2 | WAT | | 1176 | 38.657 | -2.558 | 15.728 | 1.00 | 39.58 | |
| ATOM | 5365 | OH2 | WAT | | 1177 | 40.343 | -1.549 | 18.515 | 1.00 | 55.07 | |
| ATOM | 5366 | OH2 | WAT | | 1178 | 37.655 | -0.152 | 13.597 | 1.00 | 25.18 | |
| ATOM | 5367 | OH2 | WAT | | 1179 | 43.735 | -8.994 | 26.965 | 1.00 | 21.79 | |
| ATOM | 5368 | OH2 | WAT | | 1180 | 45.247 | -6.720 | 26.195 | 1.00 | 19.01 | |
| ATOM | 5369 | OH2 | WAT | | 1181 | 40.893 | -7.988 | 25.357 | 1.00 | 31.63 | |
| ATOM | 5370 | OH2 | WAT | | 1183 | 48.295 | -5.377 | 25.111 | 1.00 | 21.86 | |
| ATOM | 5371 | OH2 | WAT | | 1184 | 39.196 | -11.269 | 22.201 | 1.00 | 27.65 | |
| ATOM | 5372 | OH2 | WAT | | 1185 | 45.834 | -3.108 | 21.000 | 1.00 | 27.28 | |
| ATOM | 5373 | OH2 | WAT | | 1187 | 47.336 | -10.925 | 19.269 | 1.00 | 32.21 | |
| ATOM | 5374 | OH2 | WAT | | 1189 | 49.721 | -11.350 | 21.806 | 1.00 | 27.73 | |
| ATOM | 5375 | OH2 | WAT | | 1190 | 45.751 | -14.313 | 20.212 | 1.00 | 32.70 | |
| ATOM | 5376 | OH2 | WAT | | 1191 | 47.280 | -5.311 | 22.044 | 1.00 | 21.49 | |
| ATOM | 5377 | OH2 | WAT | | 1192 | 53.003 | -8.918 | 11.321 | 1.00 | 28.61 | |
| ATOM | 5378 | OH2 | WAT | | 1196 | 50.381 | -17.972 | 20.320 | 1.00 | 27.49 | |
| ATOM | 5379 | OH2 | WAT | | 1197 | 41.828 | -16.171 | 23.949 | 1.00 | 36.78 | |
| ATOM | 5380 | OH2 | WAT | | 1198 | 44.472 | -14.097 | 16.153 | 1.00 | 29.45 | |
| ATOM | 5381 | OH2 | WAT | | 1199 | 43.487 | -19.987 | 16.077 | 1.00 | 39.54 | |
| ATOM | 5382 | OH2 | WAT | | 1200 | 44.561 | -11.587 | 14.584 | 1.00 | 41.08 | |
| ATOM | 5383 | OH2 | WAT | | 1201 | 51.404 | -15.017 | 11.938 | 1.00 | 44.86 | |
| ATOM | 5384 | OH2 | WAT | | 1202 | 47.565 | -6.231 | 13.570 | 1.00 | 35.05 | |
| ATOM | 5385 | OH2 | WAT | | 1203 | 42.589 | -8.089 | 21.774 | 1.00 | 45.49 | |
| ATOM | 5386 | OH2 | WAT | | 1204 | 43.716 | -5.476 | 21.813 | 1.00 | 49.36 | |
| ATOM | 5387 | OH2 | WAT | | 1205 | 31.001 | 15.176 | -7.654 | 1.00 | 41.72 | |
| ATOM | 5388 | OH2 | WAT | | 1206 | 31.965 | 15.881 | -5.171 | 1.00 | 23.88 | |
| ATOM | 5389 | OH2 | WAT | | 1208 | 54.348 | -23.974 | -20.322 | 1.00 | 40.38 | |
| ATOM | 5390 | OH2 | WAT | | 1209 | 52.470 | -22.910 | -17.579 | 1.00 | 35.13 | |
| ATOM | 5391 | OH2 | WAT | | 1210 | 52.411 | -22.520 | -14.464 | 1.00 | 45.37 | |
| ATOM | 5392 | OH2 | WAT | | 1212 | 55.169 | -20.963 | -15.174 | 1.00 | 34.24 | |
| ATOM | 5393 | OH2 | WAT | | 1213 | 28.327 | 4.770 | 1.846 | 1.00 | 42.59 | |
| ATOM | 5394 | OH2 | WAT | | 1214 | 70.425 | -0.584 | -4.634 | 1.00 | 49.45 | |
| ATOM | 5395 | OH2 | WAT | | 1215 | 69.680 | 2.181 | 5.942 | 1.00 | 12.32 | |
| ATOM | 5396 | C1 | NAG | | 504 | 35.535 | 7.769 | -31.651 | 1.00 | 74.79 | |
| ATOM | 5397 | C2 | NAG | | 504 | 34.175 | 8.440 | -31.887 | 1.00 | 76.39 | |
| ATOM | 5398 | N2 | NAG | | 504 | 13.271 | 7.529 | -32.561 | 1.00 | 75.75 | |
| ATOM | 5399 | C7 | NAG | | 504 | 32.003 | 7.449 | -32.170 | 1.00 | 76.55 | |
| ATOM | 5400 | O7 | NAG | | 504 | 31.558 | 6.473 | -31.570 | 1.00 | 77.62 | |
| ATOM | 5401 | C8 | NAG | | 504 | 31.079 | 8.619 | -32.500 | 1.00 | 75.40 | |
| ATOM | 5402 | C3 | NAG | | 504 | 34.310 | 9.721 | -32.718 | 1.00 | 78.14 | |
| ATOM | 5403 | O3 | NAG | | 504 | 33.070 | 10.422 | -32.718 | 1.00 | 79.57 | |
| ATOM | 5404 | C4 | NAG | | 504 | 35.406 | 10.633 | -32.163 | 1.00 | 78.58 | |
| ATOM | 5405 | O4 | NAG | | 504 | 35.661 | 11.688 | -33.086 | 1.00 | 77.72 | |
| ATOM | 5406 | C5 | NAG | | 504 | 36.696 | 9.846 | -31.919 | 1.00 | 78.64 | |
| ATOM | 5407 | O5 | NAG | | 504 | 36.436 | 8.712 | -31.065 | 1.00 | 77.15 | |
| ATOM | 5408 | C6 | NAG | | 504 | 37.758 | 10.691 | -31.223 | 1.00 | 79.38 | |
| ATOM | 5409 | O6 | NAG | | 504 | 38.747 | 11.147 | -32.139 | 1.00 | 80.08 | |
| ATOM | 5410 | C1 | NAG | | 505 | 37.738 | 3.977 | -28.738 | 1.00 | 64.36 | |
| ATOM | 5411 | C2 | NAG | | 505 | 38.503 | 5.203 | -29.193 | 1.00 | 65.62 | |
| ATOM | 5412 | N2 | NAG | | 505 | 39.287 | 5.707 | -28.082 | 1.00 | 64.80 | |
| ATOM | 5413 | C7 | NAG | | 505 | 40.584 | 5.941 | -28.239 | 1.00 | 65.21 | |
| ATOM | 5414 | O7 | NAG | | 505 | 41.386 | 5.060 | -28.544 | 1.00 | 65.51 | |
| ATOM | 5415 | C8 | NAG | | 505 | 41.057 | 7.376 | -28.078 | 1.00 | 65.00 | |
| ATOM | 5416 | C3 | NAG | | 505 | 37.555 | 6.299 | -29.688 | 1.00 | 67.19 | |
| ATOM | 5417 | 03 | NAG | | 505 | 38.324 | 7.256 | -30.400 | 1.00 | 65.07 | |
| ATOM | 5418 | C4 | NAG | | 505 | 36.444 | 5.751 | -30.612 | 1.00 | 69.54 | |
| ATOM | 5419 | O4 | NAG | | 505 | 35.379 | 6.725 | -30.744 | 1.00 | 71.66 | |
| ATOM | 5420 | C5 | NAG | | 505 | 35.848 | 4.448 | -30.057 | 1.00 | 69.13 | |
| ATOM | 5421 | 05 | NAG | | 505 | 36.892 | 3.507 | -29.775 | 1.00 | 67.16 | |
| ATOM | 5422 | C6 | NAG | | 505 | 34.873 | 3.762 | -31.000 | 1.00 | 70.64 | |
| ATOM | 5423 | O6 | NAG | | 505 | 34.978 | 4.265 | -32.327 | 1.00 | 73.21 | |
| ATOM | 5424 | C1 | MAN | | 603 | 64.984 | 8.473 | 43.789 | 1.00 | 99.72 | |
| ATOM | 5425 | C2 | MAN | | 603 | 64.881 | 9.795 | 44.551 | 1.00 | 101.44 | |
| ATOM | 5426 | O2 | MAN | | 603 | 65.242 | 9.606 | 45.914 | 1.00 | 100.71 | |
| ATOM | 5427 | C3 | MAN | | 603 | 65.802 | 10.830 | 43.901 | 1.00 | 103.30 | |
| ATOM | 5428 | O3 | MAN | | 603 | 65.797 | 12.028 | 44.668 | 1.00 | 103.63 | |
| ATOM | 5429 | C4 | MAN | | 603 | 67.235 | 10.276 | 43.784 | 1.00 | 104.23 | |
| ATOM | 5430 | 04 | MAN | | 603 | 68.042 | 11.175 | 43.028 | 1.00 | 105.21 | |
| ATOM | 5431 | C5 | MAN | | 603 | 67.228 | 8.901 | 43.100 | 1.00 | 104.13 | |
| ATOM | 5432 | O5 | MAN | | 603 | 66.338 | 8.001 | 43.794 | 1.00 | 102.02 | |
| ATOM | 5433 | C6 | MAN | | 603 | 68.601 | 8.249 | 43.072 | 1.00 | 105.06 | |
| ATOM | 5434 | O6 | MAN | | 603 | 69.293 | 8.554 | 41.867 | 1.00 | 106.02 | |
| ATOM | 5435 | C1 | NAG | | 604 | 62.911 | 3.631 | 43.526 | 1.00 | 83.09 | |
| ATOM | 5436 | C2 | NAG | | 604 | 64.385 | 3.786 | 43.908 | 1.00 | 85.55 | |
| ATOM | 5437 | N2 | NAG | | 604 | 64.785 | 2.756 | 44.847 | 1.00 | 86.66 | |
| ATOM | 5438 | C7 | NAG | | 604 | 64.558 | 1.474 | 44.588 | 1.00 | 87.52 | |
| ATOM | 5439 | O7 | NAG | | 604 | 65.288 | 0.795 | 43.863 | 1.00 | 87.25 | |
| ATOM | 5440 | C8 | NAG | | 604 | 63.317 | 0.859 | 45.220 | 1.00 | 89.12 | |
| ATOM | 5441 | C3 | NAG | | 604 | 64.637 | 5.144 | 44.545 | 1.00 | 87.70 | |
| ATOM | 5442 | O3 | NAG | | 604 | 66.036 | 5.333 | 44.704 | 1.00 | 87.82 | |
| ATOM | 5443 | C4 | NAG | | 604 | 64.068 | 6.261 | 43.681 | 1.00 | 90.00 | |
| ATOM | 5444 | O4 | NAG | | 604 | 64.178 | 7.514 | 44.384 | 1.00 | 95.14 | |
| ATOM | 5445 | C5 | NAG | | 604 | 62.605 | 5.970 | 43.354 | 1.00 | 88.29 | |
| ATOM | 5446 | O5 | NAG | | 604 | 62.516 | 4.709 | 42.686 | 1.00 | 84.77 | |
| ATOM | 5447 | C6 | NAG | | 604 | 61.966 | 7.004 | 42.438 | 1.00 | 89.08 | |
| ATOM | 5448 | O6 | NAG | | 604 | 60.886 | 6.453 | 41.691 | 1.00 | 90.03 | |
| ATOM | 5449 | C1 | NAG | | 605 | 59.488 | 0.645 | 41.117 | 1.00 | 66.12 | |
| ATOM | 5450 | C2 | NAG | | 605 | 59.183 | 2.025 | 41.622 | 1.00 | 68.77 | |
| ATOM | 5451 | N2 | NAG | | 605 | 518.407 | 2.727 | 40.620 | 1.00 | 67.98 | |
| ATOM | 5452 | C7 | NAG | | 605 | 57.276 | 3.331 | 40.960 | 1.00 | 66.31 | |
| ATOM | 5453 | O7 | NAG | | 605 | 56.309 | 2.728 | 41.419 | 1.00 | 65.03 | |
| ATOM | 5454 | C8 | NAG | | 605 | 57.232 | 4.840 | 40.801 | 1.00 | 66.75 | |
| ATOM | 5455 | C3 | NAG | | 605 | 60.497 | 2.750 | 41.887 | 1.00 | 71.55 | |
| ATOM | 5456 | O3 | NAG | | 605 | 60.217 | 3.984 | 42.528 | 1.00 | 73.03 | |
| ATOM | 5457 | C4 | NAG | | 605 | 61.420 | 1.900 | 42.776 | 1.00 | 73.19 | |
| ATOM | 5458 | O4 | NAG | | 605 | 62.740 | 2.471 | 42.796 | 1.00 | 78.34 | |
| ATOM | 5459 | C5 | NAG | | 605 | 61.522 | 0.482 | 42.237 | 1.00 | 70.64 | |
| ATOM | 5460 | O5 | NAG | | 605 | 60.222 | -0.071 | 42.085 | 1.00 | 69.34 | |
| ATOM | 5461 | C6 | NAG | | 605 | 62.294 | -0.451 | 43.127 | 1.00 | 70.23 | |
| ATOM | 5462 | 06 | NAG | | 605 | 63.445 | -0.932 | 42.457 | 1.00 | 69.84 | |
| ATOM | 5463 | C1 | NAG | | 704 | 50.576 | -12.496 | -21.236 | 1.00 | 82.96 | |
| ATOM | 5464 | C2 | NAG | | 704 | 49.968 | -13.245 | -20.006 | 1.00 | 85.21 | |
| ATOM | 5465 | N2 | NAG | | 704 | 49.668 | -12.280 | -18.950 | 1.00 | 85.80 | |
| ATOM | 5466 | C7 | NAG | | 704 | 50.335 | -12.262 | -17.791 | 1.00 | 84.71 | |
| ATOM | 5467 | O7 | NAG | | 704 | 49.866 | -12.715 | -16.740 | 1.00 | 83.06 | |
| ATOM | 5468 | C8 | NAG | | 704 | 51.697 | -11.577 | -17.774 | 1.00 | 83.45 | |
| ATOM | 5469 | C3 | NAG | | 704 | 50.810 | -14.391 | -19.402 | 1.00 | 86.77 | |
| ATOM | 5470 | O3 | NAG | | 704 | 49.930 | -15.352 | -18.834 | 1.00 | 87.19 | |
| ATOM | 5471 | C4 | NAG | | 704 | 51.703 | -15.079 | -20.427 | 1.00 | 87.23 | |
| ATOM | 5472 | O4 | NAG | | 704 | 52.640 | -15.933 | -19.774 | 1.00 | 88.80 | |
| ATOM | 5473 | C5 | NAG | | 704 | 52.424 | -14.000 | -21.202 | 1.00 | 86.25 | |
| ATOM | 5474 | O5 | NAG | | 704 | 51.464 | -13.315 | -22.012 | 1.00 | 84.41 | |
| ATOM | 5475 | C6 | NAG | | 704 | 53.517 | -14.530 | -22.110 | 1.00 | 86.40 | |
| ATOM | 5476 | O6 | NAG | | 704 | 54.578 | -15.105 | -21.354 | '1.00 | 85.61 | |
| ATOM | 5477 | C1 | NAG | | 705 | 47.363 | -8.699 | -23.082 | 1.00 | 67.64 | |
| ATOM | 5478 | C2 | NAG | | 705 | 48.703 | -8.838 | -23.811 | 1.00 | 70.78 | |
| ATOM | 5479 | N2 | NAG | | 705 | 48.539 | -8.506 | -25.219 | 1.00 | 70.41 | |
| ATOM | 5480 | C7 | NAG | | 705 | 49.031 | -7.366 | -25.701 | 1.00 | 71.09 | |
| ATOM | 5481 | O7 | NAG | | 705 | 48.570 | -6.257 | -25.412 | 1.00 | 71.04 | |
| ATOM | 5482 | C8 | NAG | | 705 | 50.229 | -7.464 | -26.630 | 1.00 | 72.03 | |
| ATOM | 5483 | C3 | NAG | | 705 | 49.224 | -10.276 | -23.653 | 1.00 | 72.74 | |
| ATOM | 5484 | O3 | NAG | | 705 | 50.539 | -10.377 | -24.187 | 1.00 | 74.09 | |
| ATOM | 5485 | C4 | NAG | | 705 | 49.241 | -10.694 | -22.176 | 1.00 | 73.43 | |
| ATOM | 5486 | O4 | NAG | | 705 | 49.543 | -12.105 | -22.083 | 1.00 | 77.77 | |
| ATOM | 5487 | C5 | NAG | | 705 | 47.879 | -10.411 | -21.527 | 1.00 | 70.78 | |
| ATOM | 5488 | O5 | NAG | | 705 | 47.518 | -9.034 | -21.709 | 1.00 | 68.19 | |
| ATOM | 5489 | C6 | NAG | | 705 | 47.849 | -10.676 | -20.032 | 1.00 | 70.46 | |
| ATOM | 5490 | O6 | NAG | | 705 | 48.257 | -9.535 | -19.286 | 1.00 | 68.86 | |
| ATOM | 5491 | C1 | NAG | | 804 | 42.494 | -12.858 | 36.191 | 1.00 | 85.90 | |
| ATOM | 5492 | C2 | NAG | | 804 | 42.005 | -14.295 | 36.051 | 1.00 | 88.19 | |
| ATOM | 5493 | N2 | NAG | | 804 | 42.604 | -14.945 | 34.900 | 1.00 | 87.63 | |
| ATOM | 5494 | C7 | NAG | | 804 | 43.894 | -15.265 | 34.929 | 1.00 | 86.68 | |
| ATOM | 5495 | O7 | NAG | | 804 | 44.659 | -15.041 | 33.994 | 1.00 | 85.64 | |
| ATOM | 5496 | C8 | NAG | | 804 | 44.417 | -15.920 | 36.201 | 1.00 | 86.59 | |
| ATOM | 5497 | C3 | NAG | | 804 | 40.479 | -14.268 | 35.963 | 1.00 | 90.26 | |
| ATOM | 5498 | O3 | NAG | | 804 | 39.963 | -15.582 | 35.768 | 1.00 | 90.69 | |
| ATOM | 5499 | C4 | NAG | | 804 | 39.950 | -13.664 | 37.272 | 1.00 | 91.23 | |
| ATOM | 5500 | O4 | NAG | | 804 | 38.531 | -13.572 | 37.227 | 1.00 | 92.33 | |
| ATOM | 5501 | C5 | NAG | | 804 | 40.560 | -12.265 | 37.503 | 1.00 | 91.44 | |
| ATOM | 5502 | O5 | NAG | | 804 | 42.008 | -12.311 | 37.428 | 1.00 | 89.95 | |
| ATOM | 5503 | C6 | NAG | | 804 | 40.197 | -11.674 | 38.868 | 1.00 | 92.91 | |
| ATOM | 5504 | O6 | NAG | | 804 | 41.167 | -11.984 | 39.868 | 1.00 | 93.63 | |
| ATOM | 5505 | C1 | NAG | | 805 | 46.862 | -9.930 | 36.703 | 1.00 | 66.90 | |
| ATOM | 5506 | C2 | NAG | | 805 | 45.830 | -10.052 | 37.819 | 1.00 | 68.78 | |
| ATOM | 5507 | N2 | NAG | | 805 | 46.466 | -9.913 | 39.112 | 1.00 | 69.64 | |
| ATOM | 5508 | C7 | NAG | | 805 | 46.032 | -8.994 | 39.966 | 1.00 | 70.44 | |
| ATOM | 5509 | O7 | NAG | | 805 | 45.083 | -9.187 | 40.724 | 1.00 | 72.88 | |
| ATOM | 5510 | C8 | NAG | | 805 | 46.750 | -7.653 | 39.972 | 1.00 | 71.03 | |
| ATOM | 5511 | C3 | NAG | | 805 | 45.109 | -11.383 | 37.740 | 1.00 | 70.92 | |
| ATOM | 5512 | 03 | NAG | | 805 | 44.101 | -11.426 | 38.743 | 1.00 | 69.58 | |
| ATOM | 5513 | C4 | NAG | | 805 | 44.488 | -11.534 | 36.349 | 1.00 | 73.05 | |
| ATOM | 5514 | O4 | NAG | | 805 | 43.881 | -12.831 | 36.219 | 1.00 | 78.67 | |
| ATOM | 5515 | C5 | NAG | | 805 | 45.562 | -11.363 | 35.276 | 1.00 | 71.05 | |
| ATOM | 5516 | O5 | NAG | | 805 | 46.218 | -10.081 | 35.424 | 1.00 | 69.47 | |
| ATOM | 5517 | C6 | NAG | | 805 | 45.007 | -11.458 | 33.851 | 1.00 | 70.54 | |
| ATOM | 5518 | O6 | NAG | | 805 | 44.701 | -10.186 | 33.294 | 1.00 | 68.65 | |
| ATOM | 5519 | HG+2 | HG2 | | 901 | 56.595 | 8.520 | 2.461 | 1.00 | 70.48 | |
| ATOM | 5520 | HG+2 | HG2 | | 902 | 37.255 | 9.013 | 12.962 | 0.98 | 74.03 | |
| ATOM | 5521 | HG+2 | HG2 | | 903 | 76.320 | 7.361 | 5.541 | 0.92 | 88.92 | |
| ATOM | 5522 | HG+2 | HG2 | | 904 | 17.925 | 11.864 | 12.861 | 0.93 | 103.54 | |
| ATOM | 5523 | HG+2 | HG2 | | 905 | 67.379 | 27.091 | 18.402 | 0.95 | 116.55 | |
| ATOM | 5524 | HG+2 | HG2 | | 906 | 28.136 | 31.133 | -1.762 | 0.73 | 200.00 | |
| ATOM | 5525 | ZN+2 | ZN2 | | 951 | 60.258 | -0.973 | -24.343 | 1.00 | 109.89 | |
| ATOM | 5526 | ZN+2 | ZN2 | | 952 | 35.103 | -0.702 | 39.316 | 0.89 | 124.85 | |
| ATOM | 5527 | ZN+2 | ZN2 | | 954 | 45.879 | 9.602 | 45.758 | 0.54 | 75.33 | |
| ATOM | 5528 | ZN+2 | ZM2 | | 955 | 62.511 | 19.877 | 23.367 | 0.96 | 60.06 | |
| END | | | | | | | | | | | |

The data shown in Table 1 are expressed based on the Protein Data Bank (PDB) format. The PDB format is a format containing coordinates (X, Y, Z), etc. of individual atoms constituting a protein, and is one of the standard formats in handling coordinates of biopolymers. In Table 1, the "ATOM" appearing in the utmost left column (1st column) denotes each atom of the atomic coordinates. The numbers (1, 2, 3, ....5528) appearing in the next column (2nd column) are serial numbers of individual atoms. Subsequently, in the left to right direction in this Table, there are denoted the type of each atom and its position in the amino acid to which it belongs (e.g., "CB", "CG", "SD") (in the 3rd column); the amino acid residue to which each atom belongs (three-letter abbreviations for amino acids, e.g. "MET", "ASN") (in the 4th column); the protein chain identifier (expressed by "A" or "B") (in the 5th column); the sequence number of the residue counted from the N-terminal (in the 6th column); X-coordinate (in angstrom unit) (in the 7th column); Y-coordinate (in angstrom unit) (in the 8th column); Z-coordinate (in angstrom unit)(in the 9th column); occupancy ratio (e.g., "1.00") (in the 10th column); isotropic thermal factor (e.g., 58.37 for atom No. 1 and 58.09 for atom No. 2) (in the 11th column); and the protein chain identifier (expressed by "A" or "B") (in the 12th column). In bovine rhodopsin, two molecules are present in a non-crystallographic unit. In order to discriminate these two molecules, the protein chain identifiers "A" and "B" are used. "A" denotes one molecule in the non-crystallographic unit and "B" the other.

It should be noted that the entry "ACE" in the 4th column for atom No. 1 to atom No. 3 represents an acetyl group. For atom serial No. 5205 and thereafter, the entries "WAT", "NAG", "MAN","HG2" and "ZN2" in the 4th column represent water, N-acetyl-D-glucosamine, α-D-mannose, divalent mercury ion, and divalent zinc ion, respectively; and the numbers in the 6th column (e.g., 956, 957) represent numbers given to those atoms without a protein chain identifier since they do not belong to any protein chain, in order to discriminate the constituent units of the molecules contained in the crystal.

In addition to the above-described atomic coordinates, the present invention also includes derivatives therefrom, i.e., derivatives from the above-mentioned atomic coordinates (related receptors, etc.) obtained by techniques such as homology modeling on computer. The term "derivatives" means proteins of the GPCR family that have 15% or more, preferably 30% or more, homology to native bovine rhodopsin in the amino acid sequence; or atomic coordinates (derived coordinates) in which a part of the amino acid sequence of native bovine rhodopsin (preferably one or several (e.g., one to ten) amino acids) is deleted, substituted or added and which exhibit a three-dimensional structure of a protein having activity functionally equivalent to native bovine rhodopsin (i.e., the signal transduction activity of GPCRs). Further, in the case of a GPCR family protein that is too low in homology to make an alignment of amino acid sequences with bovine rhodopsin in all regions, modeling may be performed on the seven helix sites alone that can be determined using hydrophobicity or the like as an indicator. In the present invention, whether or not atomic coordinates (derived coordinates) of a GPCR constituting a three-dimensional structure of a mutated amino acid sequence are included in the derivatives of the atomic coordinates constituting the three-dimensional structure of native bovine rhodopsin can be analyzed by comparing the three-dimensional structures of the GPCR in question and native bovine rhodopsin and using as a parameter the discrepancies between the positions of α carbon atoms of specific amino acid residues.

Specifically, an image of the three-dimensional structure composed of the atomic coordinates of native bovine rhodopsin (designated "three-dimensional structure I") and an image of the three-dimensional structure composed of the derived coordinates in question (designated "three-dimensional structure II") are created on computer based on the two sets of atomic coordinates. Subsequently, one image is superposed on the other image on a computer display. The image of three-dimensional structure I contains seven helix sites of bovine rhodopsin. These regions are superposed on the regions of the corresponding helix sites in the image of three-dimensional structure II, or vice versa. This superposing may be processed automatically based on a computer program, or may be carried out manually on a computer display in a manner similar to the one employed in comparing fingerprints.
The seven helix sites (regions) of bovine rhodopsin include the first helix (expressed as "H-I(36-64)") consisting of an amino acid sequence from position 36 to position 64 (SEQ ID NO: 2) of the amino acid sequence as shown in SEQ ID NO: 1 (hereinafter, all the position numbers mentioned in this paragraph refer to position numbers in SEQ ID NO: 1), the second helix (expressed as "H-II (71-100)") consisting of an amino acid sequence from position 71 to position 100 (SEQ ID NO: 3), the third helix (expressed as "H-III (107-139)") consisting of an amino acid sequence from position 107 to position 139 (SEQ ID NO: 4), the fourth helix (expressed as "H-IV (151-173)") consisting of an amino acid sequence from position 151 to position 173 (SEQ ID NO: 5), the fifth helix (expressed as "H-V (200-225)") consisting of an amino acid sequence from position 200 to position 225 (SEQ ID NO: 6), the sixth helix (expressed as "H-VI (247-277)") consisting of an amino acid sequence from position 247 to position 277 (SEQ ID NO: 7), and the seventh helix (expressed as "H-VII (286-306)") consisting of an amino acid sequence from position 286 to position 306 (SEQ ID NO: 8). Then, these helix regions are superposed on the corresponding helix regions of the three-dimensional structure II, or vice versa, to calculate the discrepancies between the positions of the α carbon atoms in the amino acid residues of those helices in the three-dimensional structure I and the positions of the corresponding α carbon atoms in the amino acid residues of the corresponding helices in the three-dimensional structure II. When the mean residual of the discrepancies is 1.5 Å or less, the three-dimensional structure II is regarded as a derivative of the atomic coordinates as shown in Table 1 (derived coordinates). Discrepancies between the positions of corresponding α carbon atoms can be calculated by the so-called least squares method. Generally, such discrepancies can be calculated using a computer software (e.g., LSQKAB, Quanta). The mean residual is calculated as follows. Briefly, individual distances between two corresponding α carbon atoms are squared; the mean value of these squares is calculated; and then the square root of the mean value is calculated to obtain the mean residual.

An active site or protein-binding site is formed by at least one helix selected from the above-described seven helices. Accordingly, a G protein-coupled receptor that has a three-dimensional structure of that active site or protein-binding site is also included in the G protein-coupled receptor of the invention. Preferably, the at least one helix consists of H-III, H-V, H-VI and H-VII.

In addition to the GPCR (bovine rhodopsin) having the three-dimensional structure consisting of the atomic coordinates as shown in Table 1, the present invention also includes a three-dimensional structure consisting of such derived coordinates as the GPCRs of the invention.

In Table 1, atomic coordinates for the amino acid residues of individual helices corresponds to amino acid residues 36-64 for H-I, 71-100 for H-II, 107-139 for H-III, 151-173 for H-IV, 200-225 for H-V, 247-277 for H-VI and 286-306 for H-VII (residue numbers are entered in the 6th column).

### 4. Method of Virtual Screening for Drugs

In the present invention, it is possible to carry out virtual screening for drugs using the above-described atomic coordinates or derived coordinates therefrom.

Briefly, the atomic coordinates of the three-dimensional structure elucidated by the invention are input into a computer so that images of the structure and various parameters are shown on the display. All the information contained in Table 1 may be input, or atomic coordinates corresponding to at least one helix of the seven helices may be input. For example, atomic coordinates corresponding to H-I (107-139), H-V (200-225), H-VI (247-277) and H-V1I (286-306) may be input.

Then, the resultant data are input into a virtual compound library. Since a virtual compound library is contained in a virtual screening software such as DOCK-4 (Kuntz, UCSF), the above-described data may be input into such a software. Candidate drugs may be searched for using a three-dimensional structure database for drug candidate compounds, such as MDDR (Prous Science, Spain).

### 5. Method of Drug Design

In the present invention, it is possible to construct epoch-making and yet highly reliable drug design models by applying the atomic coordinates of rhodopsin as shown in Table 1 to computer drug discovery. As described in the virtual screening method, all the information (atomic coordinates) contained in Table 1 may be used or atomic coordinates corresponding to the seven helices may be appropriately selected and used in this method of drug design. For example, atomic coordinates corresponding to H-III, H-V, H-VI and H-VII may be selected.

When the atomic coordinates as shown in Table 1 or derived coordinates therefrom have been input into a computer graphic program, the structure of rhodopsin can be seen three-dimensionally. Using this information on the three-dimensional structure, various modeling or drug designs can be performed.

Specific examples of computer programs for modeling include "FRODO" or "O" (Program O) for crystallographic analysis, and molecular modeling programs "QUANTA" and "Insight" (MSI, San Diego, USA) for use in creative drug design.

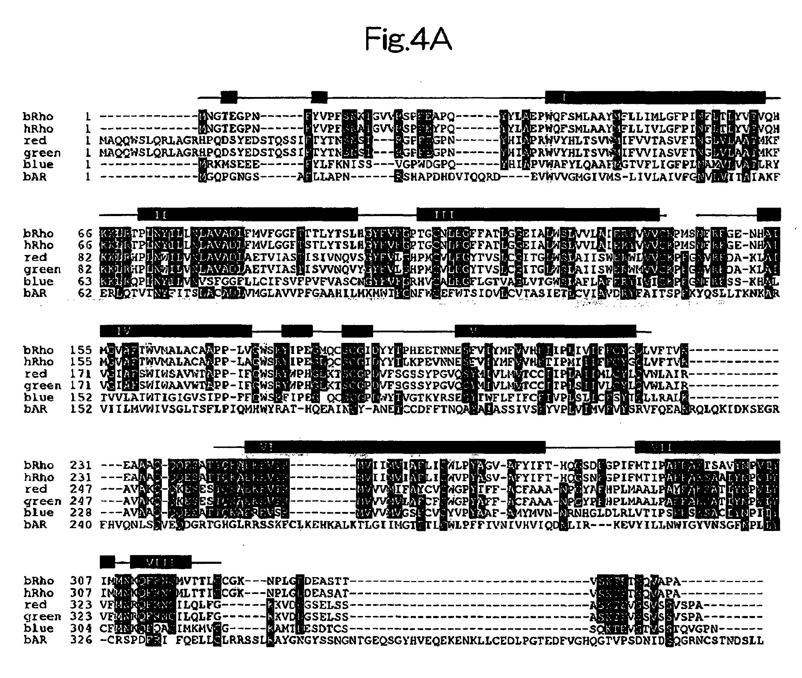

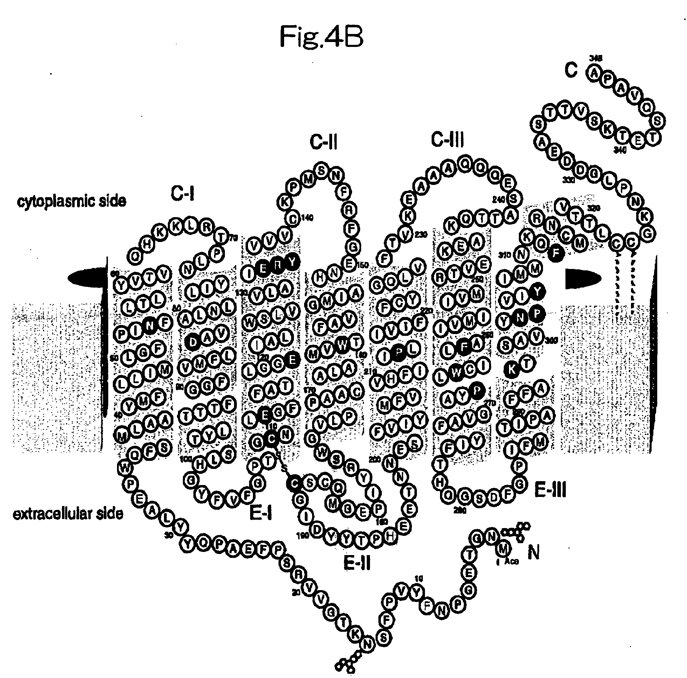

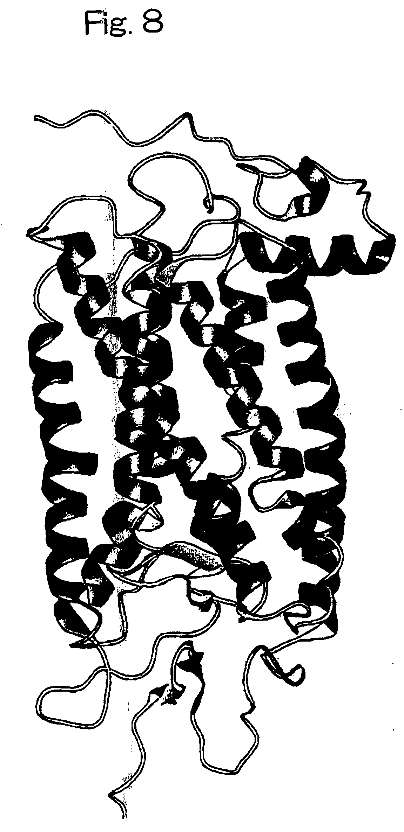

This method will be described below taking as an example angiotensin II receptor (AG22_HUMAN) which is one of the target GPCRs in pharmaceutical development. Briefly, an alignment of the amino acid sequence of angiotensin II receptor with the amino acid sequence of bovine rhodopsin is prepared as shown in Fig. 4 by conventional methods. Then, the atomic coordinates of bovine rhodopsin are input into a molecular design program such as QUANTA. While showing the total image of bovine rhodopsin by computer graphic, the features of the amino acid sequence of angiotensin II (AT-II) receptor are compared with those of rhodopsin to thereby construct anew three-dimensional structure (Fig. 8).

When drugs that act on a target GPCR have been already known as in the case of opioid hormone receptor or angiotensin II receptor, sites of specific interaction can be estimated by superposing the three-dimensional structures of such drugs on the model created as described above on graphics; thus, novel and still improved drugs can be designed (Pogozheva, I.D. et al., Biophys.J. 75, 612-634 (1998)).

### 6. Method of Screening for Substances that Influence the Effect of GPCR

In the present invention, it is possible to screen for target substances that influence the effect of GPCR by comparing the three-dimensional structure defined by the atomic coordinates as shown in Table 1 with three-dimensional structures of test substances that may influence the effect of GPCR. As described in the virtual screening method and drug design method, all the information (atomic coordinates) contained in Table 1 may be used or atomic coordinates corresponding to the seven helices may be appropriately selected and used in this method of screening. For example, atomic coordinates corresponding to H-III, H-V, H-VI and H-VII may be selected.

The expression "influence the effect of GPCR" means that a test substance and GPCR perform specific, chemical interactions to thereby promote or inhibit the function of the GPCR itself to receive molecules and transmit signals. Candidate substances that may influence in such a manner include naturally occurring compounds, artificially synthesized compounds, and synthetic peptides of ten or less amino acid residues. A therapeutic for asthma that is targeted at histamine receptor involved in asthma may also be exemplified. But, candidate substances are not limited to these substances.

Three-dimensional structures may be compared using as a parameter the discrepancies between the positions of α carbon atoms in the above-defined seven helices, or may be compared visually using as a parameter the images of structures displayed by computer graphic. If stereochemical energy conditions (e.g., hydrogen bond distance, Vander Waals contact, electrostatic interaction) between the test substance and the GPCR of the invention are suitable for them to interact with each other, the test substance is selected as a candidate for useful drug. This selection can be made appropriately using a computer software commonly used for this purpose, such as QUANTA, CHARMm, Insight I, or DISCOVER (MSI, USA).

### 7. Peptide Fragments or Proteins Having G protein-Coupled Receptor Activity

### (1) Peptide Fragments or Salts thereof

In the present invention, it is possible to specify active sites of G protein-coupled receptors from the above-described structural analysis of bovine rhodopsin and to prepare peptide fragments that constitute the specified active sites. The peptide fragment consists of an amino acid sequence of positions 36-64 (SEQ ID NO: 2), positions 71-100 (SEQ ID NO: 3), positions 107-139 (SEQ ID NO: 4), positions 151-173 (SEQ ID NO: 5), positions 200-225 (SEQ ID NO: 6), positions 247-277 (SEQ ID NO: 7) or positions 286-306 (SEQ ID NO: 8) of the amino acid sequence as shown in SEQ ID NO: 1. All or part of these peptide fragments can be included in the present invention. These peptide fragments may be synthesized, for example, by conventional peptide synthesis. The peptides of the invention also include their salts.

When the peptides of the invention are synthesized by chemical synthesis, they may be synthesized by conventional means. For example, the azide method, the acid chloride method, the acid anhydride method, the mixed acid anhydride method, the DCC method, the active ester method, the carboimidazolc method, the oxidation-reduction method, or the like may be used. Either solid phase synthesis or liquid phase synthesis may be applied.

Briefly, amino acids that may constitute the peptide of the invention are condensed with each other and, if the resultant product has a protective group, the protective group is removed. Thus, a peptide of interest is synthesized. The condensation and the removal of protective groups may be carried out by any known techniques (see, e.g., Bodanszky, M. and M.A. Ondetti, Peptide Synthesis, Interscience Publishers, New York (1966); Schroeder and Leubke, The Peptide, Academic Press, New York (1965); N. Izumiya et al., Basics and Experiments in Peptide Synthesis, Maruzen Co., Tokyo (1975)). After the reaction, the peptide of the invention may be purified by a combination of conventional purification techniques such as solvent extraction, distillation, column chromatography, liquid chromatography, and recrystallization. Alternatively, the peptide of the invention may be synthesized using a commercial, automated peptide synthesizer (e.g., model PSSM-8 manufactured by Shimadzu Corp. for simultaneous, solid phase synthesis of multiple peptides).

Salts of the peptide of the invention are, preferably, physiologically acceptable acid-added salts or basic salts. Examples of acid-added salts include salts formed by the peptide and an inorganic acid such as hydrochloric acid, phosphoric acid, hydrobromic acid, or sulfuric acid; or salts formed by the peptide and an organic acid such as acetic acid, formic acid, propionic acid, fumaric acid, maleic acid, succinic acid, tartaric acid, citric acid, malic acid, oxalic acid, benzoic acid, methanesulfonic acid, or benzenesulfonic acid. Examples of basic salts include salts formed by the peptide and an inorganic base such as sodium hydroxide, potassium hydroxide, ammonium hydroxide, or magnesium hydroxide; or salts formed by the peptide and an organic base such as caffeine, piperidine, trimethylamine, or pyridine. Salts may be prepared using an appropriate acid such as hydrochloric acid or an appropriate base such as sodium hydroxide.

Although the C-terminal of the peptide of the invention is usually a carboxyl group (-COOH) or carboxylate (-COO⁻), its C-terminal may be an amide (-CONH₂) or ester (-COOR). The R in the ester may be, for example, C₁₋₁₂ alkyl, C₃₋₁₀ cycloalkyl, C₆₋₁₂ aryl, or C₇₋₁₂ aralkyl.

The peptide of the invention further include those peptides whose amino group of the alanine residue at the N-terminal is protected with a protective group, or conjugated peptides such as glycopeptides with sugar chains linked thereto.

The biochemical and physicochemical properties of the peptide of the invention can be analyzed by mass spectrometry, nuclear magnetic resonance, electrophoresis, high performance liquid chromatography, and the like.

### (2) Proteins Having G protein-Coupled Receptor Activity

The G protein-coupled receptor of the invention has the seven helix sites (regions) of H-I (36-64), H-II (71-100), H-III (107-139), H-IV (151-173), H-V (200-225), H-VI (247-277) and H-VII (286-306). Activity of the G protein-coupled receptor arises from these helix sites. In particular, H-III (107-139), H-V (200-225) and H-VI (247-277) form an active core. Therefore, a protein consisting of an amino acid sequence spanning from H-I (36-64) to H-VII (286-306) (positions 36-306) of the amino acid sequence as shown in SEQ ID NO: 1 is included in the protein of the invention having G protein-coupled receptor activity. Also, a protein comprising a region containing H-III (107-139), H-V (200-225) and H-VI (247-277) that form an active core, more specifically, a protein consisting of a part of an amino acid sequence of positions 36-306 of the amino acid sequence as shown in SEQ ID NO: 1, the part comprising at least a region from H-III (107-139) to H-VI (247-277) (positions 107-277), is included in the protein of the invention having G protein-coupled receptor activity.

Further, the above-described protein having G protein-coupled receptor activity may have a mutation(s) such as deletion(s), substitution(s) or addition(s) of one or more amino acids, as long as it retains G protein-coupled receptor activity. Such a protein is also included in the protein of the invention.

Hereinbelow, the present invention will be described more specifically with reference to the following Examples. However, the present invention is not limited to these Examples.

### EXAMPLE 1

### Isolation, Purification and Crystallization of Rhodopsin

Bovine retinas (purchased from Schenk Packing Co., Inc., Stanwood WA, USA) were solubilized with nonyl glycoside (NG), heptane-1,2,3-triol (HPTO) and zinc acetate to prepare a rhodopsin solution with a sufficient purity for crystallization (Okada, T. et al., Photochem. Photobiol. 67, 495-499 (1998)).

Briefly, a rhodopsin solution was concentrated by centrifugation to give a concentration of 10 mg/ml or more. The concentrated solution contained 30-50 mM MES buffer (pH 6.3-6.4), 90-120 mM zinc acetate, 0.5-0.65% (v/v) HPTO, and NG about 2.2 times the amount of rhodopsin molecules in molar ratio.

This rhodopsin solution was mixed with a reservoir solution [3.0-3.4 M ammonium sulfate, 0.1 M MES buffer (pH 6.0-6.1)] at approximately 3:1 to give final concentrations of 30 mM MES, 5-7 mM β-mercaptoethanol, 65-90 mM zinc acetate, 0.55-0.75% (v/v) HPTO, and 0.45-0.55% (w/v) NG, 0.84-0.86 M ammonium sulfate, to thereby prepare a crystallization solution. Then, crystallization was carried out by hanging drop vapor diffusion.

Crystals were obtained after leaving the solution stationary for 45 days to half a year in darkness.

All of the operations, including crystal data measurement, were carried out under dim red light (Fuji Film SC66 or SC68).

### EXAMPLE 2

### X-Ray Analysis of Bovine Rhodopsin

Rhodopsin crystals obtained in Example 1 were transferred in succession to a mother liquor containing 1-5 mM mercury acetate and soaked for 2.5 months to half a year to prepare a mercury derivative.

To this mother liquor of mercury derivative, 10 µl of cryoprotectant solution containing 15% (w/v) sucrose, 0.4% (v/v) HPTO, and 3.4 M ammonium sulfate was added directly. Then, crystals were dipped up with a small nylon loop according to a conventional method. The crystals were immediately placed under a nitrogen gas flow of 100 K (-173°C) in a liquid nitrogen cooling device to perform flash freezing.

Thus, crystals were frozen in succession. Photos of their X-ray diffraction images were taken and good crystals were searched for. Selected crystals were transferred with CryoTong™ (Hampton Research, USA) or the like into a liquid nitrogen container and stored until use in experiments.

### (1) Data Collection

First, in order to estimate X-ray wavelengths to be used in MAD method, the XANES spectrum was measured for experimentally determining the absorption edge wavelength of the mercury atom contained in the crystal.

The selected and stored mercury derivatives of crystals were mounted on the head of a goniometer in an X-ray diffraction device under a nitrogen gas flow of 100 K according to conventional methods. Their images were photographed, and the qualities of these crystals were examined again. For high quality crystals, three wavelength diffraction image data (MAD data) were collected using RIKEN Beamline I (BL45XU) at SPring-8 optimized for MAD measurement (M. Yamamoto, T. Kumasaka, T. Fujisawa and T. Ueki, J. Synchrotron Rad. 5, 222 (1998)).

From the collected MAD data, diffraction intensity data were obtained using ENZO and Scalepack (Z. Otwinowski & W. Minor, Methods Enzymol. 276, 307 (1997)) (programs for calculating diffraction intensities from diffraction image data), and twin ratios were estimated using an integrated crystallographic analysis program CNS (Brunger, A.T. et al., Acta Crystallogr. D54, 905-921 (1998)). Twinned crystals are crystals that have a plurality of segments of different azimuths. They may reduce the anomalous diffraction effect expected in MAD method. A crystal whose twin ratio had been estimated 11% was selected for use in data collection since this ratio was lower than those of other crystals. In order to improve accuracy, MAD data measurement was repeated, and six data sets for MAD phase calculation were collected to 3.3 Å from that crystal. Statistics for crystal parameters and measured data are shown in Table 2.

In addition to the above-mentioned measurement data, diffraction data were collected to 2.8 Å in a similar manner at APS (synchrotron radiation facility), the Argonne Natioanl Laboratory (Chicago, USA). In these measurements, the twin ratio was estimated about 30%.

### (2) Structural Determination and Refinement

For structural determination, the positions of mercury atoms were determined from the diffraction intensity data measured at the beginning, using SOLVE (T.C. Terwilliger and J. Berendzen, Acta Crystallogr. Sect. D 55, 849 (1999) according to conventional methods. The refinement of mercury sites in the crystal lattice, occupancy ratios and thermal factors, as well as the calculation of phases were performed with SHARP (E. de la Fortelle & G. Bricogne, Methods Enzymol. 276, 472 (1997)). In order to improve this experimental phase, the averaging of electron densities by non-crystallographic symmetry utilizing the fact that two identical rhodopsin molecules are present in a non-crystallographic unit, the smoothing of solution regions, and the histogram matching of electron densities were carried out. For these purposes, DM/CCP4 (K. Cowtan, Joint CCP4 and ESF-FACBM Newsletter on Protein Crystallography 31, 34 (1994)) was used. The thus obtained electron density map was input into the program O (T. A. Jones, S. Cowan, J. Y. Zou & M. Kjeltgaard, Acta Crystallogr. Sect. A 47, 110 (1991)). At that time, most of the models for the crystal structure could be constructed. Refinement of models was performed further with CNS, and re-construction of models was repeated with the program O. The R value by the refinement using this 3.3 Å diffraction intensity data set was 23.9% (free R=28%) as a result of calculation using all of the diffraction data measured to 3.3 Ä resolution.

Using the thus obtained structure, model molecules were newly placed in a crystal lattice by the molecular replacement method using a different, 2.8 Å data set in which the length of the crystal lattice is changed. Based on the results, re-construction and refinement of models were performed with the above-mentioned programs CNS and O to obtain the final crystal structure of bovine rhodopsin. During this process, correction of the twin ratio was carried out repeatedly. The results are shown in Table 2.

### (3) Results

The atomic coordinates as shown in Table 1 were obtained. Statistics for data collection, phasing and refinement are shown in Table 2.

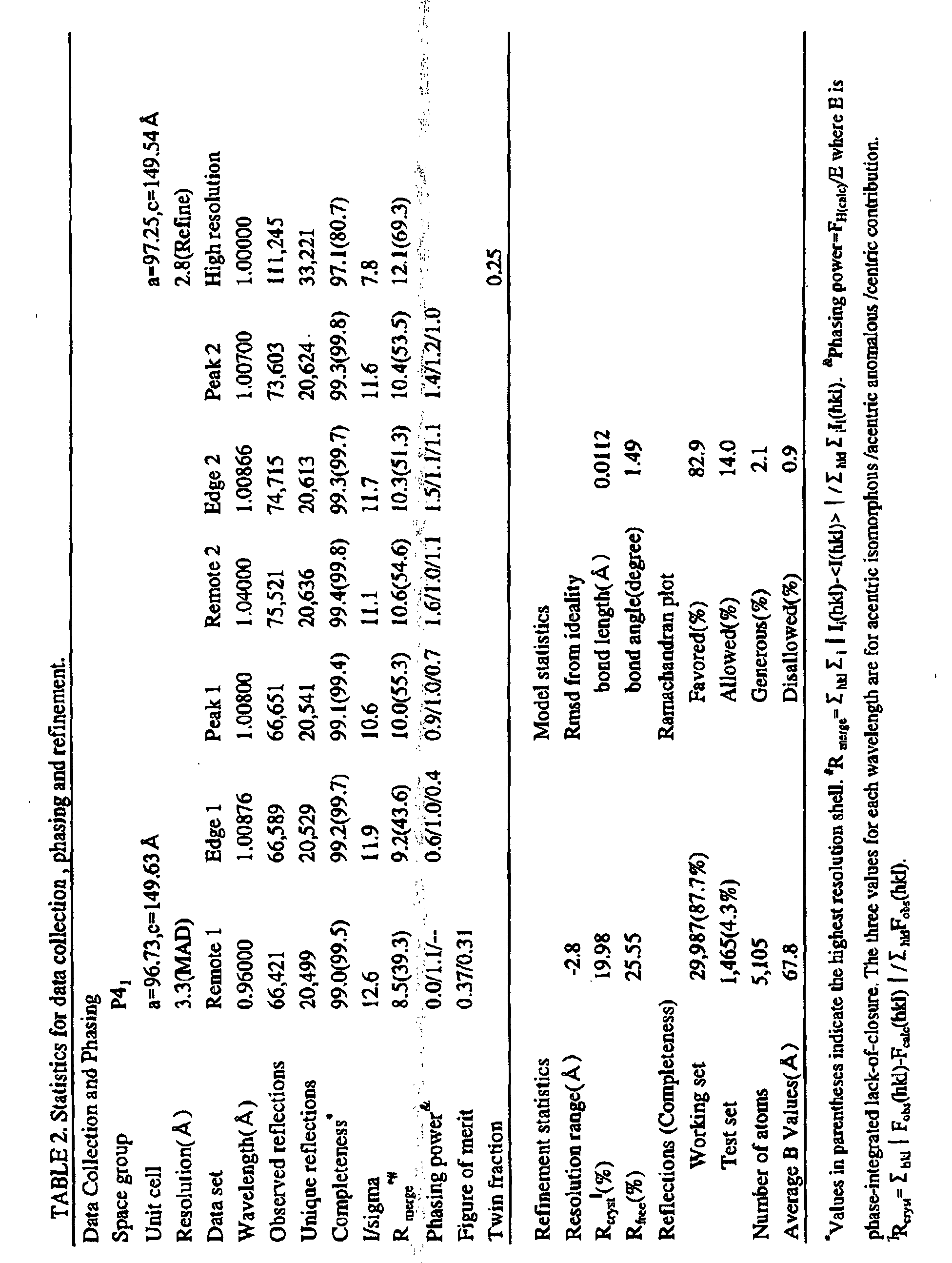

The data obtained by the determination of atomic coordinates (Table 1) were input into the computer graphic program O to create graphic images of the three-dimensional structure from various viewpoints. Hereinbelow, the results will be described based on the images obtained.

### (4-1) Structural Determination: Overall Fold and Molecular Contacts

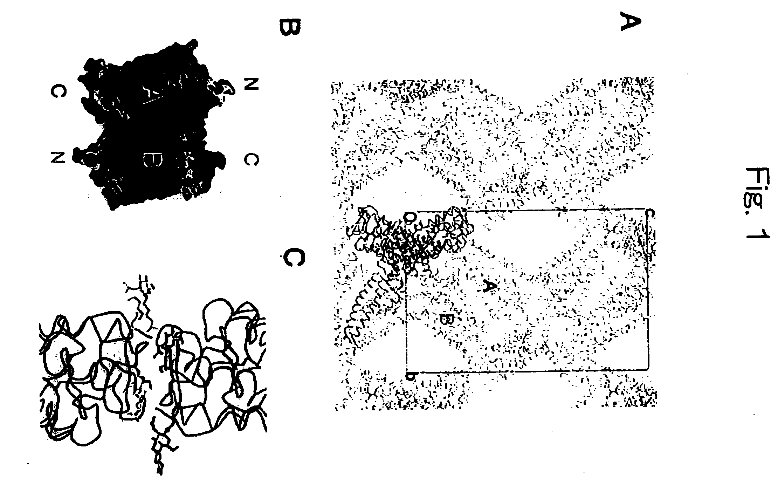

To obtain structural information for rhodopsin in the ground state, diffraction data for bovine rhodopsin crystallized from mixed micelles were collected. Phasing information was obtained using multiwavelength anomalous diffraction (MAD) methods. Rhodopsin molecules are packed in the crystal lattice as shown in Fig. 1A. The two molecules in the asymmetric unit marked with "A" and "B", respectively, are related by a non-crystallographic twofold axis between the two H-I helices. In Fig. 1A, a unit cell in the crystal lattice is shown by a box ("0" denotes the origin of the crystal unit cell; "b" and "c" denotes the crystal axes). These H-I helices run almost antiparallel to each other and are within van der Waals contact distance. Thus, the two rhodopsin molecules are packed in almost opposite directions in this crystal.

This packing allows the electrostatic potentials of the monomers to compensate for each other (Fig. 1B). In Fig. 1B, light gray indicates regions with negative potential and dark gray regions with positive potential.

Due to these electrostatic properties, rhodopsin molecules in native membranes are arranged in the same orientation and present in a monomeric form. Points of contact between protein molecules between non-crystallographic symmetric units are present mainly at the N-terminal regions including the sites where oligosaccharides are attached (the two saccharide regions shown in the stick model in Fig. 1C). On the other hand, the cytoplasmic moiety of rhodopsin appears not to be involved in any specific interactions between molecules in the crystal packing. The direction of the dipole moment of the retinylidene chromophore in the dimer is almost perpendicular to the z-axis of the crystal lattice, which is consistent with the observation that rhodopsin crystals do not exhibit any color upon exposure to light polarized in z-direction (T. Okada, et al., J. Struct. Biol. 130, 73 (2000)).

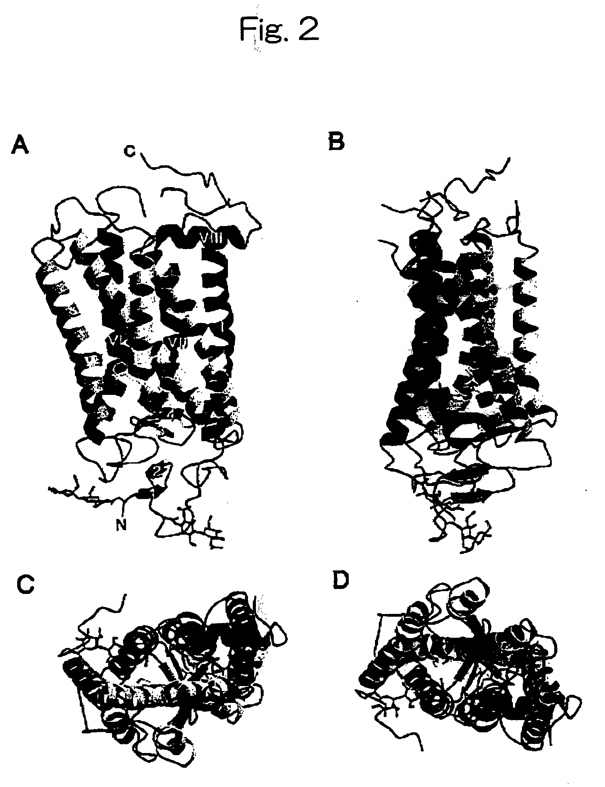

The current model of bovine rhodopsin includes all of the residues that make up seven transmembrane helices; they are 36 to 64 for H-I, 71 to 100 for H-II, 107 to 139 for H-III, 151 to 173 for H-IV, 200 to 225 for H-V, 247 to 277 for H-VI, and 286 to 306 for H-VII in the amino acid sequence of bovine rhodopsin as shown in SEQ ID NO: 1. Ribbon drawings of bovine rhodopsin including models of these helices are shown in Fig. 2. In Fig. 2, Panel A shows the structural model viewed parallel to the plane of the membrane. The upper most part of this drawing corresponds to the outer surface of the disc of rod outer segment. Panel B shows the same model rotated by 90°C around the vertical axis. Panel C and Panel D show the same model viewed from the intradiscal side and the extradiscal side, respectively.

A total of 191 amino acids are present in the transmembrane region, and they amount to 54.9% of the total amino acids (348) in the whole molecule. Further, the intradiscal moiety composed of the N-terminal tail and three inter-helical regions (E-I, E-II and E-III) contains the following residues.

1 to 34 for NH₂-terminal tail, 101 to 106 for E-I, 174 to 199 for E-II, and 278 to 285 for E-III; 74 residues in the total.

The structural model of rhodopsin of the invention includes approximately 60% of the amino acid residues in the cytoplasmic moiety of rhodopsin (C-I, C-II, C-III and C-terminal tail): 65 to 70 for C-I, 140 to 150 for C-II, 226 to 235 and 240 to 246 for C-III, and 307 to 327 and 334 to 348 for the C-terminal region including helix H-VIII.

From the above results, it has been found that a total of 338 amino acids in the current model correspond to 97.1% of the whole rhodopsin molecule. In addition, the 11-*cis*-retinal chromophore connected to Lys²⁹⁶, a part of two oligosaccharides at Asn² and Asn¹⁵ (M..N. Fukuda et al., J. Biol. Chem. 254, 8201 (1979)), one Zn ion and three Hg ions are also included in the current structural model.

It should be noted that the number appearing after each of the amino acids expressed in three-letter abbreviations herein indicates the position of the relevant amino acid in the amino acid sequence of bovine rhodopsin (SEQ ID NO: 1).

The shape of modopsin appears to be very different depending on the viewpoint (Fig. 2A-B). On the plane of the membrane, rhodopsin appears to assume an elliptical shape (Fig. 2C-D). The extracellular domain is folded compactly. Most of the mass of this region comes from the N-terminal and E-II region corresponding to 27 and 35 residues, respectively. The extracellular domain and the transmembrane domain are tightly associated, and a part of E-II appears to be inside the transmembrane domain.

The transmembrane helices are grouped into long helices (H-I, H-II, H-III, H-V and H-VI) and short helices (H-IV and H-VII), with a maximum of 33 residues for H-III and a minimum of 21 residues for H-VII.

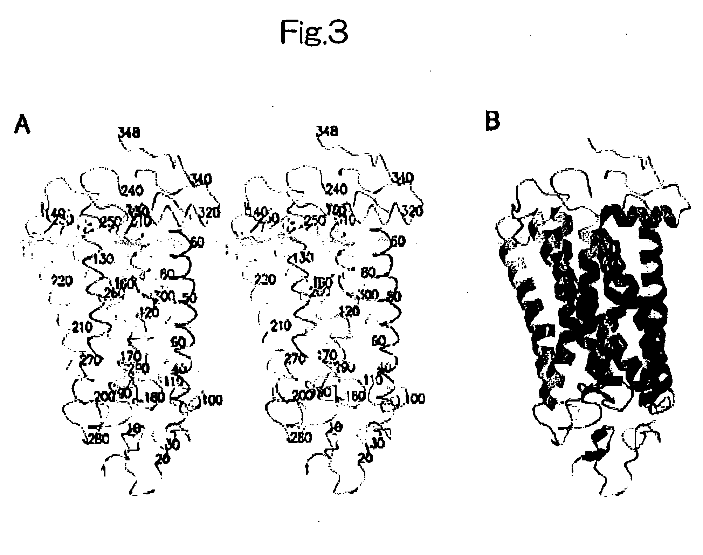

The key residues involved in interhelical hydrogen bonds are presented in Fig. 3A. The position numbers of the residues in the amino acid primary sequence are indicated in this Figure. The cytoplasmic region forms a flat structure with characteristic ruggedness, and most of the C-II loop and the C-terminal region just follows the H-VII helix extending along the expected surface of the membrane.

The 4th loop (C-IV) in this region includes a short helical structure (H-VIII) connected perpendicularly to H-VII. C-III, the largest interhelical loop in the cytoplasmic region, does not seem to be folded over the helix region, but seems to extend the flat surface defined by the edge of the rod of rhodopsin.

In contrast to the compactly folded structure of the extracellular domain, most of the cytoplasmic regions exhibit high thermal factors, and the electron densities of some residues could not be specified (Fig. 3B). In Fig. 3B, light gray indicates high thermal factors. The four regions (C-I to III and C-terminal) do not interact with each other closely. The retinal chromophore is attached to the middle of molecule, is U-shaped in appearance, and is covalently bound to the side chain of Lys²⁹⁶. The electron density is consistent with the 11-*cis*-conformation for the retinal. The dimensions of rhodopsin molecule are approximately 35 Ä x 25 Å x 55 Å, making the total volume ∼48,000Ä³.

### (4-2) Comparison to Other Receptors

The structural model of rhodopsin of the invention offers a structural template for other GPCRs, including the assignment of secondary structural elements and the location of highly conserved amino acids.

The molecular size of bovine rhodopsin, 348 amino acids, is intermediate among the members of the family and thus can feature most of the essential parts of functional importance in G-protein activation. The lengths of the seven transmembrane helices and of the three extracellular loops are expected to be nearly the same for most of the family members, as can be seen in the sequence of β-adrenergic receptor (Fig. 4A). Variation in other regions probably reflects the specificity of each receptor for either its ligand or its G protein. Because most of the vertebrate visual pigments share very similar size distributions for all of the domains, structure-function relationships deduced from the structural model of the invention are directly applicable to the members of this subfamily. The structure of bovine rhodopsin, represented schematically in Fig. 4B, exhibits those features found in most GPCRs, and at the same time demonstrates differences between GPCRs and bacterial retinal-binding proteins (M. Kolbe et al., Science 288, 1390 (2000; H. Luecke et al., J. Mol. Biol. 291, 899 (1999), H. Belrhali et al., Structure 7,909 (1999)).

In Fig. 4A, abbreviations denote the following:
rRho: bovine rhodopsin (P02699)
hRho: human rhodopsin (P08100)
red: human red cone pigment (P04000)
green: human green cone pigment (P04001)
blue: human blue cone pigment (P03999)
bAR: human β-adrenergic receptor (P07550)

The sequences of the above substances were obtained from the GenBank. Identical amino acids in all receptors are shown in white letters on black background. Conservative replacement residues (T=S, E=D=Q=N, M=L=I=V, R=K, and Y=F=W) are shown in white letters on black background. Conservative replacement residues between rhodopsins and cone pigments are shaded in gray. Transmembrane helices (H-I to H-VII) are shown as thick black bars, and encompass residues Gln³⁶-Gln⁶⁴ for H-I, Pro⁷¹-His¹⁰⁰ for H-II, Pro¹⁰⁷-Val¹³⁹ for H-III, Asn¹⁵¹-Val¹⁷³ for H-IV, Asn²⁰⁰-Gln²²⁵ for H-V, Glu²⁴⁷-Thr²⁷⁷ for H-VI and Ile²⁸⁶-Tyr³⁰⁶ for H-VII. Post-translational inodifications are N-terminal acetylation, glycosylation (Asn² and Asn¹⁵, numbers 1 and 2), Cys-Cys bridge (residues 3 and 4), Lys²⁹⁶ that forms the Schiff base with the chromophore (number 5) and two palmitoylation sites (6, 7).

### (4-3) Extracellular (Intradiscal) Regions

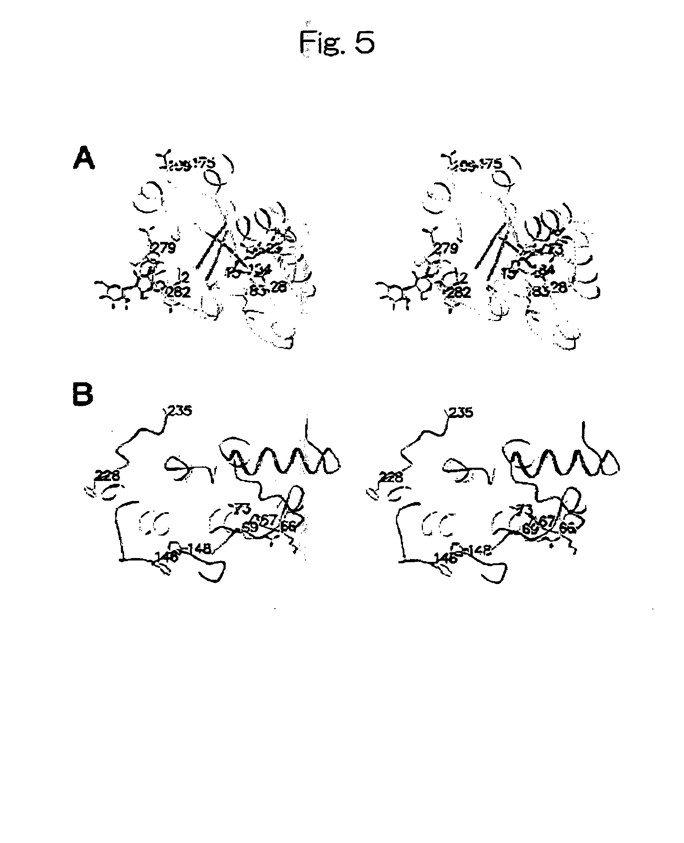

Rcgions in the extracellular domain of rhodopsin (N-terminal and interhelical loops E-I, E-II, and E-III) associate to form a compact structure (Fig. 2 and Fig. 5A). Fig. 5 presents stereoviews of the C α-traces showing the flow of the polypeptide chain, with some key residues, on the inside of the extraccllular domain of the transmembrane helices (Panel A) and on the cytoplasmic side of the molecule (Panel B). The N-terminal tail of rhodopsin contains five distorted β -strand-like structures, forming a substantial domain by itself. The N-terminus is located just below loop E-III, with the side chain of Asp²⁸² close to that of Asn².

The first two antiparallel strands, Gly³ to Pro¹², form a typical β -sheet fold (designated S1 and S2) running almost parallel to the expected plane of the membrane.

The subsequent three strands form a right triangle from Phe¹³ to Pro³⁴, with the third strand running just below E-III, almost parallel to the long axis of rhodopsin viewed from the extracellular direction. S4 connects Ser¹⁴-Asn¹⁵ in the N-terminal region of the molecule with Pro²³, located close to E-I. S5 from Pro²⁷ to Pro³⁴ runs along the surface of the membrane covering the extracellular (intradiscal) space between H-I and H-II. Oligosaccharides at Asn² and Asn¹⁵ extend from the domain and are not involved in any interactions with any parts of the polypeptide.

Mutations of Pro²³ or Gln²⁸ cause the eye disease retinitis pigmentosa (T.P. Dryja et al., Nature 343, 364-366 (1990); P. Humphries et al., Science 256, 804 (1992); A. Rattner et al., Annu. Rev. Genet. 33, 89 (1999)). In the model of the present invention, these side chains are located close together in a space between the 4th and 5th distorted β -strands, and are also close to the side chain of Tyr¹⁰² on E-I loop (His¹⁰⁰-Pro¹⁰⁷).

These findings indicate that the above-mentioned residues maintain the proper orientation between the E-I loop and the N-terminal domain. The N-terminal domain may also contact the E-III loop around Pro¹².

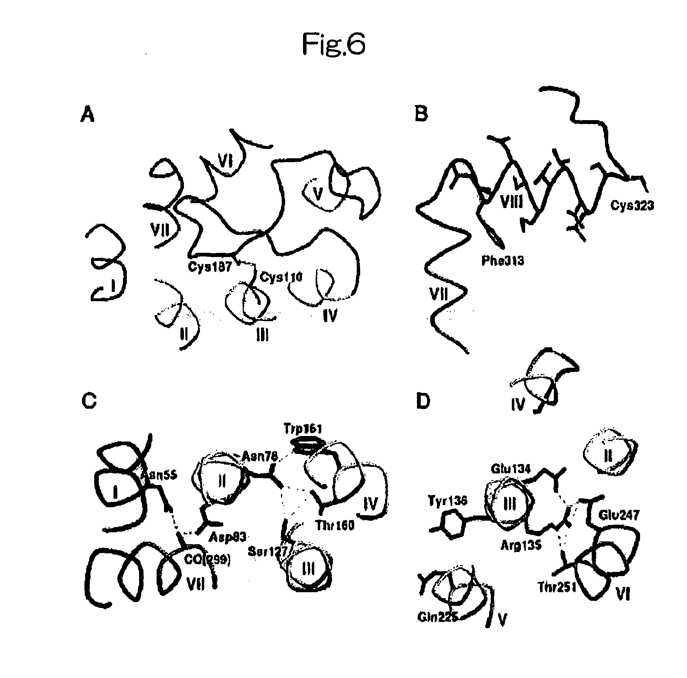

While both the E-I and E-III loops run along the periphery of the molecule, a part of E-II folds deeply into the center of rhodopsin. Just after the extracellular end of H-IV, a long strand from Gly¹⁷⁴ to Met¹⁸³ crosses the molecule along the membrane surface. The side chain of Met¹⁸³ extends toward a hydrophobic pocket around H-I, and the side chain of Gln¹⁸⁴ is surrounded by hydrophilic groups containing bound water. Residues in the middle of this strand, Arg¹⁷⁷ to Glu¹⁸¹(designated β 3), form a typical antiparallel β -sheet with the next region (residues Ser¹⁸⁶ to Asp¹⁹⁰, designated β 4), which is deeper inside the molecule than β3. β 4 is just below the 11-*cis*-retinal and is a part of the chromophore-binding pocket. The side chain of Cys¹⁸⁷ points to that of Cys¹¹⁰ at the extracellular end of H-III, forming a disulfide bridge. This disulfide bridge is conserved in most GPCRs (Fig. 4A and Fig. 6A). Residues Tyr¹⁹¹ to Asn²⁰⁰ from E-II loop form another loop region at the periphery of the molecule, like E-I and E-III. The side-chain amide of Gln²⁷⁹, which is at the beginning of E-III, and the peptide carboxyl of Tyr¹⁹¹ in E-II are close to each other, while Asn¹⁹⁹ is near to Trp¹⁷⁵, which is one of the initial residues of E-II, thus in proximity to the extracellular end of H-IV. This arrangement places E-II in extensive contact with the extracellular regions and also with retinal.

The above-described finings not only well explain the results of many experiments carried out to date, but also prove that the structure of bovine rhodopsin currently determined is correct. Further, it was confirmed this time that important amino acid residues expected by mutation experiments, etc. are forming a hydrogen bond or salt bridge in a hydrophobic environment. Thus, it has become clear that these amino acid residues are key residues in the maintenance of the structure. In particular, the S-S bridge, which is most conserved in GPCRs, forms the lower part of the extracellular domain on the N-terminal side along the *cis*-retinal in charge of photo absorption of rhodopsin. It is suggested that his site plays an important role in the binding of ligand to GPCRs. In fact, in a homology model of angiotensin-II receptor, a site corresponding to the retinal-binding site of rhodopsin has an amino acid side chain supplementing the site, and a specific space is provided on the opposite side of this extended chain. This space runs toward the N-terminal, forming a channel-like shape. This strongly suggests that a ligand (such as hormone)-binding site of GPCRs exists in that space.

This also means that the mechanism of molecular switch (i.e., as retinal changes into all-*trans*-configuration upon absorption of light, this change causes structural change in the protein, switching the receptor to active conformation called metarhodopsin II) is almost directly applicable to GPCRs in general. Thus, various structures of GPCRs can be created by techniques such as homology modeling based on the three-dimensional structure of bovine rhodopsin elucidated by the invention. Further, the structural model of the invention opens up a possibility to create new ligands when applied to drug creation using homology models.

### (4-4) Transmembrane Helices

From a cryo-EM study, the helical bundle of rhodopsin appears to have differently extending faces on the two ends, suggesting that it could be asymmetric along the axis perpendicular to the membrane surface (V.M. Unger et al., Nature 389, 203 (1997)). However, examination of the cross section of the bundle at the two surfaces indicates that these are nearly equal in area irrespective of the difference in shape.

The cytoplasmic ends of H-II and H-IV are near each other, but they diverge in the region of Trp¹⁶¹, one of the residues that are highly conserved among GPCRs. This residue is near the point where H-III penetrates toward H-V between H-II and H-IV. Although H-III has in its central part two consecutive Gly residues (Gly¹²⁰ and Gly¹²¹) that are believed apt to disturb the two-dimensional structure, these residues do not distort H-III. On the other hand, die region of the three residues Glu¹³⁴-Arg¹³⁵-Tyr¹³⁶ that are believed important for activity in H-III does exhibit a slight deviation from typical helical structure.

The cytoplasmic terminal region of H-III is surrounded mostly by hydrophobic residues forming the binding site for a G protein. H-IV and H-V exhibit a deviation from regular helicity in the cytoplasmic region and at His²¹¹, respectively. The regular belicity of H-V is interfered by the side chain of Tyr¹³⁶ at Cys²²². Near this region, the phenolic ring of Tyr²²³, which is also highly conserved among GPCRs, partially covers the interhelical region between H-V and H-VI near the lipid interface. The cytoplasmic end of H-VI extends past the putative membrane surface to Thr²⁴³. This terminal region is slightly distorted. Three basic residues, Lys²⁴⁵, Lys²⁴⁸, and Arg²⁵², located near the cytoplasmic end of H-VI, project from the helical bundle, making this region of C-III highly basic.

In H-VII, two phenyl rings of Phe²⁹³ and Phe²⁹⁴ interact with Leu⁴⁰ of H-I and Cys²⁶⁴ of H-VI, respectively. This interaction with H-VI is particularly important because the region in H-VI is where the direction of helix significantly changes. H-VII is distorted in the region from Ala²⁹⁵ to Tyr³⁰¹. This region includes Ala²⁹⁹, whose peptide carbonyl can hydrogen bond with the side chain NH of Asn⁵⁵ in H-I. A highly conserved NPXXY motif in GPCRs follows this region in a regular helical structure.

The results of structural analysis described so far revealed that bovine rhodopsin has a transmembrane helical structure greatly different from that of bacteriorhodopsin whose structure has already been elucidated though its function is completely different, and that this helical structure of bovine rhodopsin is the first structure ever obtained that can be used as a general model for GPCRs.

### (4-5) 11-cis-Retinal Chromophore

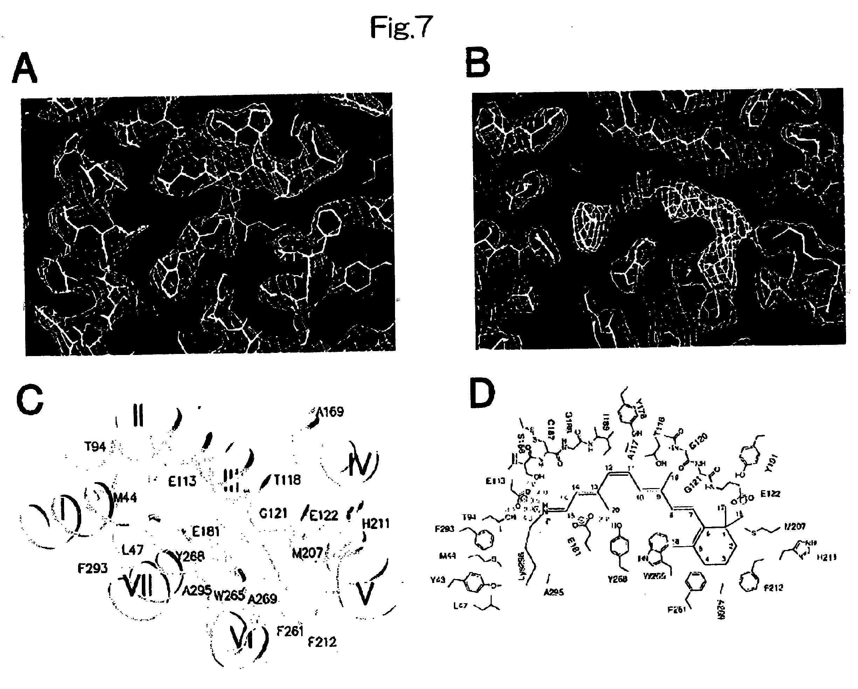

From the experimental electron density, the conformation of the retinal chromophore in the Schiff base linkage with Lys²⁹⁶ is 6s-*cis*, 11-*cis*, 12s-*trans*, *anti* C=N (Fig. 7). Fig. 7A shows an experimental electron density of the retinal chromophore (2Fo-Fc map (1 σ)); and Fig. 7B shows an electron density of the retinal chromophore after structural refinement (2Fo-Fc map (1 σ)). The electron density indicated by a double basket near the retinal molecule shows an omit map (5 σ). Fig. 7C-D presents schematics showing the side chains surrounding the 11-*cis*-retinylidene group.

The electron density for the β-ionone ring exhibits a larger bulge indicating the positions of the two methyl groups connected to C₁ and a smaller bulge for the single methyl at C₅ (Fig. 7B).

Two small bulges along the polyene chain indicate the positions of the C₉- and C₁₃-methyl groups. The refined structure of the retinylidene group is consistent with resonance Raman spectroscopy (R.H. Callender et al., Biochemistry 15, 1621 (1976)), nuclear magnetic resonance (NMR),(P.O. Smith et al., Biochemistry 26, 1606 (1987)) and chemical analysis (P.K. Brown & G. Bald, J. BioL Chem. 222, 865 (1956)). The electron density of the polyene chain merges with that of the side chain of Lys²⁹⁶, indicating the presence of a Schiff base linkage. The retinylidene group is located closer to the extracellular side in the putative lipid bilayer, as suggested previously (D.D. Thomas & L. Stryer, J. Mol. Biol. 154, 145 (1982)).

The position of the β-ionone ring is mainly covered from the cytoplasmic side by the residues in H-III and H-VI, Glu¹²², Phe²⁶¹, and Trp²⁶⁵ (Fig. 7C). The indole ring of Trp²⁶⁵ points down to the retinylidene group near the β-ionone ring, and also comes close to its C₁₃-methyl group with a distance of 3.8 Ä. Because deletion of the C₁₃-methyl group induces partial expression of rhodopsin activity in the dark (T. Ebrcy et al., FEBS Lett. 116, 217 (1980)), loss of the interaction between Trp²⁶⁵ and C₁₃-methyl group may be a possible mechanism of the activation of rhodopsin. From the β-ionone ring to C₁₁, the retinylidene group runs almost parallel to H-III. On H-III, there are amino acid residues that provide many side chains constituting the binding pocket surrounding the polyene chain. These amino acid residues are Glu¹¹³, Gly¹¹⁴, Ala¹¹⁷, Thr¹¹⁸, Gly¹²⁰, and Gly¹²¹, mainly around the polyene chain. The side chain of Thr¹¹⁸, in addition to Tyr²⁶⁸ and IIc¹⁸⁹ from the extracellular side, appears to determine the position of the C₉-methyl of the retinylidene group. Side chains mostly from H-V and H-VI (Met²⁰⁷, Phe²⁰⁸, His²¹¹, Phe²¹², Tyr²⁶⁸, and Ala²⁶⁹) also surround the β-ionone ring. The proximity of Phe²⁶¹ and Ala²⁶⁹ to the retinylidene group is consistent with information showing that these amino acid residues located near the retinal are responsible for the absorption difference between red and green pigments in humans (M. Neitz et al., Science 252, 971 (1991)).

Arrangement of the four residues from H-VI (Phe²⁶¹, Trp²⁶⁵, Tyr²⁶⁸ and Ala²⁶⁹) appears to be determined by a significant bend around Pro²⁶⁷. From H-IV, only Cys¹⁶⁷ participates covering a part of this pocket. Residues from H-I, H-II, and H-VII (Tyr⁴³, Met⁴⁴, Leu⁴⁷, Thr⁹⁴, and Phe²⁹³) are part of the region surrounding the Schiff base.

Finally, the extracellular side of the polyene chain is covered by a part of the E-II loop, β-sheet S4 from Ser¹⁸⁶ to Ile¹⁸⁹. The side chain of Glu¹⁸¹ belonging to S3 of the E-II loop extends toward the retinylidene goup. This supports the previous results demonstrating that the corresponding amino acid in red/green pigments may be the binding site for chloride ion, which is responsible for the red shift in their absorption compared with rhodopsin (Z. Wang et al., Biochemistry 32, 2125 (1993)). Another amino acid from the E-II loop participating in the retinylidene group binding site is Tyr¹⁹¹, whose OH group is also close to that of Tyr²⁶⁸ in H-VI. Since mutation of this residue does not affect the absorption but reduces the ability to activate transducin (T. Doi et al., Proc. Natl. Acad. Sci. USA 87, 4991 (1990)), Tyr¹⁹¹ may participate in the transition to the active form of rhodopsin through interaction with Tyr²⁶⁸.

The arrangement around the Schiff base is of particular interest in terms of understanding the mechanism of the primary process in photoactivation of rhodopsin. The direction of the side chain of Lys²⁹⁶, almost along the long axis of rhodopsin, is supported by two hydrophobic side chains in H-I, Met⁴⁴ and Leu⁴⁷, and by a nearby peptide bond between Phe²⁹³ and Phe²⁹⁴. This region is stabilized through the two phenyl rings interacting with other helices. Since it is difficult to determine exactly from the current structure how the protonated Schiff base linkage is stabilized in the protein environment, the current model is unable to discriminate whether water molecules participate in the formation of a coordinated counterion or not.

Since the distances between the oxygen atoms of the side chain carboxyl group of Glu¹¹³ and the Schiff base nitrogen arc 3.3 Å and 3.5 Å, a salt bridge is formed directly. Also, the OH group of Thr⁹⁴ cornes close to one of the oxygen atoms of Glu¹¹³ with a distance of 3.4 Å. Any other residues, including the nearby Thr⁹² and Thr⁹³, are too far from the Schiff base region to contribute to stabilization of its protonated state.

From the above-described results of structural analysis, the environment surrounding retinal has been clearly shown, and the structural basis for discussing photoactivation has been established. As described previously, the β-strand from Ser¹⁸⁶ to Ile¹⁸⁹ includes the disulfide bridge between Cys¹⁸⁷ and Cys¹¹⁰, which is conserved in most GPCRs. This structural site has been found important for the mechanism of activation switch that is turned on by the binding of a ligand.

### (4-6) Cytoplasmic Surface (Extradiscal Region)

The structure around the C-I loop exhibits a rigid organization (Fig. 5B). Of the three basic side chains in this region, Lys⁶⁷ extends toward the solvent, whereas Lys⁶⁶ and Arg⁶⁹ point toward a lipid-facing region. The peptide carbonyl group of Lys⁶⁷ appears to easily hydrogen-bond with the side chain of Asn⁷³ in H-II. This constraint may be important for maintaining an arrangement of the two exposed basic residues and His⁶⁵ forming a line of positive charges over H-L These make up one of the most conserved amino acid clusters among GPCRs in the rhodopsin subfamily.

The present inventors assign the region from Cys¹⁴⁰ to Glu¹⁵⁰ as the C-II loop. This loop exhibits an L-shaped structure, when viewed parallel to membrane plane, with a barrel (Met¹⁴³ to Phe¹⁴⁶) almost along the main axis of rhodopsin. Four polar side chains in this loop (Lys¹⁴¹, Ser¹⁴⁴, Asn¹⁴⁵, and Arg¹⁴⁷) form a distinct cytoplasmic border from the transmembrane region. The height of these side chains is roughly comparable to that of the cytoplasmic border of C-III loop.

The carboxyl-terminal half of the loop C-II, Asn¹⁴⁵ to Gly¹⁴⁹, stretches to the outside of the molecule, probably along the expected membrane surface. In the C-II loop, the position of Cys¹⁴⁰ is mainly along the membrane surface, forming an external flat domain on the cytoplasmic side of rhodopsin. In contrast to C-I in which basic side chains are exposed to the solvent region, two phenyl rings of Phe¹⁴⁶ and Phe¹⁴⁸ form a part of a cytoplasmic border of rhodopsin. In fact, a continuous curved line of such a border, which is distinct from the rest of the molecule, is formed by the side chains from His⁶⁵ to Phe¹⁴⁶, covering both the C-I and C-II loops (Fig. 5B). This line merges with a part formed by the side chains of C-IV, Arg³¹⁴, Asn³¹⁵ and Thr³¹⁹, although its spatial separation from the transmembrane region becomes obscure around this region.

Thus, the model of the invention can assign a border corresponding to the major cytoplasmic part of rhodopsin.

Several amino acid residues on the cytoplasmic side following H-VI have a helical structure comparable to standard α-helix structure. In contrast, the cytoplasmic extension from H-V exhibits an S-shaped flat loop structure almost along the surface of membrane. The C-III loop connecting from H-V to H-VI reaches close to the lipid-facing side of H-VI at Ala²³⁵, without covering the cytoplasmic surface of the helical bundleof rhodopsin.

The model of the invention shows that this region is of highly flexible nature. Although the four amino acid residues from Gln²³⁶ to Glu²³⁹ are not included in the current model, it is evident that the C-III loop is not folded over the helical region of rhodopsin.

It should also be noted that the C-III loop is known to vary considerably among related GPCRs, so the flexibility and variability of this region may be critical for functionality and specificity in G-protein activation. In particular, the C-terminal part of the C-III loop is drawing a large arc as if covering a part of the cytoplasmic region that has been experimentally demonstrated to interact with G proteins. This relation between the C-terminal part and the site of interaction with G proteins is important when considering structural changes after the activation switch has been turned on. The amphiphilic helical structure of the H-VIII is of particular interest in the cytoplasmic region, considering previous studies of a variety of synthetic peptides and their effects on the activation of G proteins. Direct evidence for interaction of the amphiphilic helical structure region of H-VIII with the G-protein transducin has been provided using a synthetic peptide from Asn³¹⁰ to Leu³²¹ of bovine rhodopsin (B. Konig et al., Proc Natl. Acad. Sci. USA 86, 6878 (1989); E.P. Marin et al., J. Biol. Chem. 275, 1930 (2000)). The short amphiphilic helix of H-VIII is clearly distinct from H-VII and, via the tripeptide Mer³⁰⁹ to Lys³¹¹, lies nearly perpendicular to H-VII. It is also the region that follows the NPXXY motif as a part of a conserved block of residues up to Cys³²². The presence of a short helix structure in this region was demonstrated fora corresponding peptide of turkey β -adrenergic receptor by solution NMR spectroscopy in a nonpolar solvent (H. Jung et al., Biochemistry 35, 6399 (1996)).

It has also been supposed that a group of peptides called mastoparans, which assume an amphiphilic helical structure and have activity on G proteins, mimic the structure of receptors in this region (K. Wakamatsu et al., Biochemistry 31, 5654 (1992)). From the rhodopsin structure, it appears that this short stretch of amino acids is located in a hydrophobic environment, which could induce α-hclical structure. Further, the distribution of side chains along this helix also exhibits an amphiphilic pattern; the charged/polar groups cluster on one side while hydrophobic ones are on the other. This suggests that Phe³¹³, Met³¹⁷ and Leu³²¹ are buried in hydrophobic core of the receptor (Fig. 6B).

In Fig. 6, Panel A shows the E-II loop near the disulfide bridge between Cys¹¹⁰ and Cys¹⁸⁷, viewed from the extracellular side. Panel B shows the C-IV cytoplasmic loop from Lys³¹¹ to Leu³²¹ forming a short amphiphillic helix (H-VIII). Panel C shows interhelical hydrogen bonds mediated by a highly conserved Asn⁵⁵, connecting H-H, H-II and H-VII, and by a highly conserved Asn⁷⁸, connecting H-II, H-III and H-IV. Panel D shows the tripeptide region, Glu¹³⁴-Arg¹³⁵-Tyr¹³⁶ known as (D/E)R(Y/W) motif, located near the cytoplasmic end of H-III.

Phe³¹³ and Arg³¹⁴ are the most conserved residues in this region. Thus, Arg³¹⁴, along with Asn³¹⁵ and Thr³¹⁹, participates in the continuous cytoplasmic border of rhodopsin, suggesting that this short helix in rhodopsin may be functionally important for fixing H-VII to the membrane as an anchor.

Although the model of the invention does not include any lipid-like structure, the side chains of Cys³²² and Cys³²³ to which attachment of palmitic acid has been reported (Y.A. Ovchinnikov et al., FEBS Lett. 230, 1 (1988)) project to the outside of rhodopsin, causing no collision between atoms on the model. Thus, the current model is consistent with the above report.

The helical structure appears to be terminated by Gly³²⁴ and the following C-terminal tail changes the direction (Fig. 6B). The region from Lys325 to Leu328 runs as an elongated structure almost antiparallel to the above-described helix, forming one of the most exposed borders of rhodopsin.

### (4-7) Intramolecular Interactions and Activation

The transmembrane region of rhodopsin is stabilized by a number of interhelical hydrogen bonds and hydrophobic interactions, and most of them are mediated by highly conserved residues in GPCRs (Fig. 4A). One of the residues that exhibit the highest conservation is Asn⁵⁵ in H-I. Its side chain is responsible for two interhelical hydrogen bonds to Asp⁸³ in H-II and to the peptide carbonyl of Ala²⁹⁹ in H-VII (Fig. 6C). Another region that mediates constraints for three helices includes Asn⁷⁸ of H-II, which is hydrogen-bonded to OH groups of Ser¹²⁷ of H-III and Thr¹⁶⁰ of H-IV.

The tripeptide Glu¹³⁴-Arg¹³⁵-Tyr¹³⁶ is part of a highly conserved (D/E)R(Y/W) motif found in GPCRs (Fig. 4A and Fig. 6D). These residues participate in several hydrogen bonds with surrounding residues. The carboxyl group of Glu¹³⁴ forms a salt bridge with the guanidinium group of neighboring Arg¹³⁵. Arg¹³⁵ is also linked to Glu²⁴⁷ and Thr²⁵¹ in H-IV. Also, H-III is close to H-V at the side chain of Tyr¹³⁶, whose OH group can form a hydrogen bond with the side chain of Gln²²⁵. The three Val residues from Val¹³⁷ to Val¹³⁹ are also close enough to partly cover the cytoplasmic side of Glu¹³⁴ and Arg¹³⁵. These hydrophobic residues could be one of the critical constraints keeping rhodopsin in the inactive conformation.

H-VII of most of the GPCRs in the rhodopsin subfamily contains an NPXXY sequence near the cytoplasmic end, but the functional importance of this motif remains unclear. The side chains of the two polar residues in this region, Asn³⁰² and Tyr³⁰⁶ in bovine rhodopsin, project inside the molecule. The OH group of Tyr³⁰⁶ is at 3.2 Å distance from Asn⁷³, which is also highly conserved among GPCRs, suggesting the presence of additional interhelical hydrogen-bonding constraints between H-VII and H-II, Asn⁷³ is also hydrogen-bonded with the peptide carbonyl of Lys⁶⁷ in the C-I loop. Although the distance between Asn³⁰² and Asp⁸³ is too long to form a hydrogen bond, water molecules near Asp83 might possibly interact with the side chain of Asn³⁰². In this case, the water molecules mediate the interhelical contact among H-II, H-III and H-VII.

The energy of light is utilized for photoisomerization of the 11-*cis*-retinal chromophore to an all-*trans*-configuration. This change in conformation would cause multiple effects, including movement of β-ionone toward H-III and/or displacement of Schiff base/C₉/C₁₃-methyl regions, ultimately switching the receptor to active conformation, metarhodopsin II (R.G. Matthews et al., J. Gen. Physiol. 47,215 (1963); CJ. Weitz & J. Nathans, Neuron 8,465 (1992); J. Kibelbek et al., Biochemistry 30, 6761 (1991); Tachibanaki et al., Biochemistry 36, 14173 (1997); L Szundi et al., Biochemistry 37, 14237 (1998); S. Dickopf et al., Biochemistry 37, 16888 (1998)).

The model of bovine rhodopsin of the invention confirms that these effects can change the environment of the salt-bridge between the Schiff base and Glu¹¹³, resulting in its neutralization (K. Fahmy et al., Proc. Natl. Acad. Sci. USA 90, 10206 (1993); F. Jager et al., Biochemistry 33, 10878 (1994)). Displacement of H-III will result in environmental changes in the ERY motif and cause its reorientation. The rhodopsin model of the invention also suggests that interaction between β -ionone ring and H-III occurs at Glu¹²², which is one of the residues that determine the rate of metarhodopsin II decay (H. Imai et al., Proc. Natl. Acad. Sci. USA 94, 2322 (1997)). Because Glu¹²² interacts with His²¹¹ in rhodopsin, the proposed movement of H-III caused by the β-ionone ring can affect the interaction between these residues in the transition to metarhodopsin II. In addition, the change around the Schiff base region can affect the interaction between the C₁₃-methyl group of retinal and Trp²⁶⁵. The photoactivation may also cause breakage of some of the three interhelical constraints mediated by Ala²⁹⁹, Asn³⁰², and Tyr³⁰⁶, and hydrophobic constraints via Phe²⁹⁴ to the highly kinked region in H-VI. As a result, rearrangement of the helical bundle may be triggered, and finally lead to the movements of H-III and/or H-VI (D.L. Farrens et al., Science 274, 768 (1996); S.P. Sheikh et al., Nature 383, 347 (1996)).

### (5) Summary

The GPCR family is one of the largest, most diverse and most interesting groups of proteins encoded by 2 to 5% of the genes present in human genome. They are involved in many physiological processes and are attractive targets for pharmacological intervention to improve these processes in normal and pathological states. The determination of crystal structure of rhodopsin has revealed a comprehensive molecular model and a highly organized heptahelical transmembrane bundle with 11-*cis*-retinal as a key cofactor involved in maintaining rhodopsin in the ground state. Numerous papers have reported many features of rhodopsin found in the structural model of the invention.

A set of residues that interacts with the 11-*cis*-retinal chromophore produce the specific environment that results in an absorption shift of the chromophore to a longer wavelength. However, long distance interactions and electrochemical properties of the chromophore-binding site, as a whole, are responsible for this characteristic maximum absorption. The structure of the invention provides insight into the spectral tuning of related receptors, cone pigments.

Another major issue is what is the molecular mechanism for GPCR activation. The positions of a conserved set of residues on the cytoplasmic surface, where G-protein activation occurs, suggest a possible conformational change upon photoactivation of the chromophore that leads to rhodopsin activation and signal transduction. Therefore, the crystal structure of bovine rhodopsin discloses many fascinating structural features commonly found in GPCRs that will be useful to elucidate the principles that govern receptor activation and spectral tuning. The structure will also lead to molecular explanation of structural changes that occur in mutated receptors as well as several human pathologies as a result of such structural changes. More importantly, the structure of the invention will reveal the nature of the membrane protein bovine rhodopsin in detail.

All the publications, patents and patent applications cited in the present specification are incorporated herein by reference in their entireties.

### EFFECT OF THE INVENTION

According to the present invention, a three-dimensional structure of bovine rhodopsin provided with the basic structure of GPCRs is disclosed. The structural data of the invention provide GPCR-targeting drug design with a structure as an extremely realistic model molecule. Further, the basic structure of bovine rhodopsin of the invention is useful for computer-utilizing development of various drugs targeting GPCR family receptors.

## Claims

1. A peptide fragment consisting of an amino acid sequence of positions 36-64, positions 71-100, positions 107-139, positions 151-173, positions 200-225, positions 247-277 or positions 286-306 of the amino acid sequence as shown in SEQ ID NO: 1, or a salt of said peptide fragment.

2. A protein selected from the group consisting of the following (a), (b) and (c):
(a) an isolated protein consisting of an amino acid sequence of positions 36-306 of the amino acid sequence as shown in SEQ ID NO: 1;
(b) an isolated protein which consists of a part of an amino acid sequence of positions 36-306 of the amino acid sequence as shown in SEQ ID NO: 1, said part comprising at least positions 107-277, and which has G protein-coupled receptor activity;
(c) an isolated protein which consists of an amino acid sequence of positions 36-306 or 107-277 of the amino acid sequence as shown in SEQ ID NO: 1 having a deletion(s), substitution(s) or addition(s) of one or more amino acids, and which has G protein-coupled receptor activity.

3. A G protein-coupled receptor selected from the group consisting of the following (a) and (b):
(a) a G protein-coupled receptor having three-dimensional structure I defined by the atomic coordinates as shown in Table 1;
(b) a G protein-coupled receptor having three-dimensional structure II defined by derived coordinates from the atomic coordinates as shown in Table 1, wherein the mean residual of the discrepancies between the positions of the α carbon atoms in the amino acid residues of seven helix sites H-I (36-64), H-II (71-100), H-III (107-139), H-IV (151-173), H-V (200-225), H-VI (247-277) and H-VII (286-306) in the amino acid sequence as shown in SEQ ID NO: 1 of the three-dimensional structure I and the positions of the corresponding α carbon atoms in the amino acid residues of the corresponding seven helix sites of the three-dimensional structure II is 1.5 Å or less when an image of the three-dimensional structure I obtained by computer-processing the atomic coordinates of Table 1 and an image of the three-dimensional structure II obtained by computer-processing said derived coordinates are superposed.

4. The receptor according to claim 3, wherein the G protein-coupled receptor is bovine rhodopsin.

5. The receptor according to claim 4, wherein the bovine rhodopsin is a metal derivative of its crystal.

6. The receptor according to claim 5, wherein the metal is a mercury compound.

7. A method of virtual screening for drugs, comprising computer-processing the following atomic coordinates (a) or (b), or derived coordinates therefrom, inputting the resultant computer-processed data into a virtual compound library and searching for useful drugs through said library:
(a) a part of the atomic coordinates as shown in Table 1, said part corresponding to the amino acid residues of at least the three helix sites of H-III (107-139), H-V (200-225) and H-VI (247-277) selected from seven helix sites H-I (36-64), H-II (71-100), H-III (107-139), H-IV (151-173), H-V (200-225), H-VI (247-277) and H-VII (286-306) in the amino acid sequence as shown in SEQ ID NO: 1;
(b) the atomic coordinates as shown in Table 1.

8. The method according to claim 7, wherein said derived coordinates are generated by homology modeling based on the atomic coordinates as shown in Table 1, the mean residual of the discrepancies between the positions of the α carbon atoms in the amino acid residues of the three helix sites H-III (107-139), H-V (200-225) and H-VI (247-277) in the amino acid sequence as shown in SEQ ID NO: 1 and the positions of the corresponding α carbon atoms in the amino acid residues of the corresponding three helix sites in said derived coordinates being 1.5 Å or less.

9. A method of drug design, comprising imaging three-dimensional structures of G protein-coupled receptors using the following atomic coordinates (a) or (b), or derived coordinates therefrom, analyzing the resultant images by computer graphics and designing structures of useful drugs based on the resultant analysis data:
(a) a part of the atomic coordinates as shown in Table 1, said part corresponding to the amino acid residues of at least the three helix sites of H-III (107-139), H-V (200-225) and H-VI (247-277) selected from seven helix sites H-I (36-64), H-II (71-100), H-III (107-139), H-IV (151-173), H-V (200-225), H-VI (247-277) and H-VII (286-306) in the amino acid sequence as shown in SEQ ID NO: 1;
(b) the atomic coordinates as shown in Table 1.

10. The method according to claim 9, wherein the derived coordinates are generated by homology modeling based on the atomic coordinates as shown in Table 1, the mean residual of the discrepancies between the positions of the α carbon atoms in the amino acid residues of the three helix sites H-III (107-139), H-V (200-225) and H-VI (247-277) in the amino acid sequence as shown in SEQ ID NO: 1 and the positions of the corresponding α carbon atoms in the amino acid residues of the corresponding three helix sites in said derived coordinates being 1.5 Ä or less.

11. A method of screening for target substances that influence the effect of the G protein-coupled receptor according to any one of claims 3 to 6, comprising comparing the three-dimensional structure of the receptor with three-dimensional structures of test substances.
